# EUROPEAN PATENT APPLICATION

(11) **EP 1 225 224 A1**
(43) Date of publication of application: **24.07.2002**
(21) Application number: 00963041.9
(22) Date of filing: 02.10.2000
(51) Int. Cl.: C12N 15/12, C07K 14/435, C07K 16/18, C12P 21/02, C12Q 1/68, A61K 38/00, A61K 39/395, A61K 48/00, A61P 9/10, G01N 33/50, G01N 33/53

(54) **SHEAR STRESS-RESPONSE DNA**

(30) Priority: 01.10.1999 JP 28097699
(71) Applicant: KYOWA HAKKO KOGYO CO., LTD., Chiyoda-ku, Tokyo 100-8185 (JP); Nojima, Hiroshi, Minoo-shi, Osaka 562-0031 (JP)
(72) Inventor: NOJIMA, Hiroshi, Mino-shi, Osaka 562-0031 (JP); YOSHISUE, Hajime, Kyowa Hakkko Kogyo Co. Ltd., Machida-shi, Tokyo 194-8533 (JP); OBAYASHI, Masaya, Kyowa Hakko Kogyo Co. Ltd., Machida-shi, Tokyo 194-8533 (JP); OTA, Toshio, Kyowa Hakko Kogyo Co. Ltd., Machida-shi, Tokyo 194-8533 (JP); KAWABATA, Ayako, Kyowa Hakko Kogyo Co. Ltd., Machida-shi, Tokyo 194-8533 (JP); SAKURADA, Kazuhiro, Kyowa Hakko Kogyo Co. Ltd., Machida-shi, Tokyo 194-8533 (JP); KUGA, Tetsuro, Kyowa Hakko Kogyo Co. Ltd., Hohu-shi, Yamaguchi 747-8522 (JP); SEKINE, Susumu, Kyowa Hakko Kogyo Co. Ltd., Machida-shi, Tokyo 194-8533 (JP); NAKAMURA, Yusuke, Yokohama-shi, Kanagawa 225-0011 (JP); SUGANO, Sumio, Suginami-ku, Tokyo 167-0052 (JP)
(74) Representative: VOSSIUS & PARTNER
(86) International application number: JP0006840
(87) International publication number: WO0125427

(57) **Abstract**

This invention relates to a novel shear stress-responsive DNA, a protein encoded by the DNA, an antibody against the protein, a method for detecting a shear stress-responsive DNA or protein, a therapeutic agent and a diagnostic agent for vascular diseases caused by arteriosclerosis and a method for screening the therapeutic agent and the diagnostic agent.

## Description

### Field of the Invention

This invention relates to novel DNAs obtained by employing the subtraction method while paying attention to mRNAs which show a shear stress-dependent increase of expression in vascular endothelial cells; and proteins encoded by these DNAs. Moreover, this invention also relates to antibodies against the proteins; methods for detecting the proteins and the DNAs; and the diagnosis and treatment of various vascular diseases caused by arteriosclerosis, such as cardiac insufficiency, restenosis after PTCA (percutaneous transluminal coronary angioplasty) and hypertension, and methods for screening an agent for such treatment or diagnosis.

### Background of the Invention

Conventionally, vascular endothelial cells covering the inner surfaces of blood vessels in the form of a monolayer have been considered to be a mere lining for separating the vascular tissue from blood flowing through the lumen of the blood vessel. However, as a result of the recent progress of research on the vascular endothelium, it has been found that the endothelium has a great diversity of functions which are very important for the living body. These functions include, for example, regulation of the material permeability between blood and tissue, regulation of the tension of the blood vessel, maintenance of an antithrombogenic activity, control of the proliferation of smooth muscle, repair of tissues, inflammatory reaction, and remodeling of the blood vessel. The physical force applied to the vascular wall by a flow of blood is called a shear stress, which is defined by the flow velocity of blood, the viscosity of blood, and the diameter and morphology of the blood vessel. The shear stress acts on the endothelium covering the inner surface of the vascular wall and distorts vascular endothelial cells in the direction of the blood flow. According to investigations made for the last ten years or so, it has been revealed that, similarly to chemical stimuli such as hormones and cytokines, this physical stimulus is closely associated with the morphology of vascular endothelial cells and regulation of the above-described various functions [Cell Technology (in Japanese), 16, 950(1997)].

In industrially advanced countries including Japan, atherosclerosis is one of the major causes of death of adults. It is known that the malfunction of blood vessels caused by hypercholesteremia, hyperhomocysteinemia, diabetes mellitus and the like is closely related to the development of atherosclerosis and the aggravation of the morbid state [Molecular Cardiovascular Medicine, 49-61(1995)]. On the other hand, it is also known that arteriosclerotic lesions are not uniformly distributed over all blood vessels, but are localized in specific regions such as the outside of a bend in a branched part of a blood vessel. Since such local development is also observed in experimental animal having a genetically increased blood cholesterol level, it is considered that the incorporation of cholesterol into the vascular endothelium occurs in two stages, i.e., local changes of vascular endothelial cells and the actual incorporation of cholesterol [Arterioscler. Thromb., 14, 133-140(1994)]. The cause of such local development has scarcely been clarified. However, since incipient lesions occur frequently in places where the intensity and direction of a shear stress are not steady, i.e., places where a low shear stress is produced and the separation or stagnation of a flow or turbulence (e.g., eddies) tends to occur, hemodynamic stresses such as shear stresses are considered to be closely related to the development of atherosclerosis. At present, the molecular mechanism by which a shear stress induces arteriosclerosis locally is not clearly understood. However, genes whose expression is altered by applying a shear stress mechanically to vascular endothelial cells cultured in vitro have been searched until now. Thus, it has been found that a shear stress activates various transcription factors such as AP(activator protein)-1 and NF(nuclear factor)-κB, and thereby causes a change of expression of genes under the control of these transcription factors. Up to now, it has been reported that the proteins encoded by genes exhibiting an alteration of expression in response to a shear stress stimulus include growth factors such as PDGF (platelet-derived growth factor) and TGF(transforming growth factor)-β; adhesion factors such as VCAM(vascular cell adhesion molecule)-1 and ICAM(intercellular adhesion molecule)-1; tension control factors such as ET(endothelin)-1; thrombolysis factors such as t-PA (tissue-type plasminogen activator); enzymes such as NOS (nitric oxide synthase) 3, COX (cyclooxygenase) 2 and SOD (superoxide dismutase); and the like [Molecular Medicine Today, 5, 40(1999)]. Thus, the genes responding to a shear stress in an in vitro reconstituted system are believed to include two groups of molecules having different characteristics, i.e., arteriosclerosis induction factors considered to be expressed in at least low shear stress regions of blood vessels in response to a change of shear stress, and molecules suppressing the development of arteriosclerosis in intravascular places where a high shear stress is produced constitutively. However, among the genes presumed to exhibit an alteration of expression in response to a shear stress only some genes have been specifically identified. In order to understand the cause of arteriosclerosis and develop methods for the prevention and treatment thereof, it is necessary to clarify unknown genes responding to a shear stress. In recent years, unknown genes responding to a shear stress have been searched by employing the differential display method or the like, but it involves several problems in that genes whose alteration of expression is of the order of several times cannot be easily obtained and in that the proportion of false positive clones is high [Nucleic Acids Res., 23, 4520-4523(1995)]. Consequently, the number of genes exhibiting an alteration of expression in response to a shear stress and clarified by the differential display method is not great [Proc. Natl. Acad. Sci. USA, 93, 10417-10422(1996); Proc. Natl. Acad. Sci. USA, 94, 9314-9319(1997); Biochem. Biophys. Res. Comm., 255, 347-351 (1996); Biochem. Biophys. Res. Comm., 246, 881-887(1998); US Patent 5,834,248 (1998); US Patent 5,849,578 (1998); US Patent 5,882,925 (1999)].

As described above, it is recognized that changes of the shear stress applied to vascular endothelial cells are involved in the local development of atherosclerosis, but the fact is that its molecular mechanism is scarcely understood. Nevertheless, it has been reported for long that a shear stress reduces the turnover of endothelial cells in vivo, i.e., a shear stress acts so as to suppress the cell death of the endothelium [Atherosclerosis, 17, 401-417(1973); Circ. Res., 69, 1557-1565(1991)]. Moreover, there are many reports showing that, in vitro, the apoptosis of endothelial cells induced by TNF-α stimulation, hydrogen peroxide stimulation, growth factor depletion or the like is markedly suppressed by the application of a shear stress [J. Exp. Med., 185, 601-607(1997); FEBS Lett., 399, 71-74(1997); Arterioscler. Thromb. Vas. Biol., 17, 3588-3592(1997); Biochem. Biophys. Res. Commun., 231, 586-590(1997)]. That is, it is believed that, in branched or curved parts of arteries where a low shear stress is produced, the character of endothelial cells changes so as to induce apoptosis and this is a cause defining the locality of an incipient arteriosclerotic lesion. At present, however, little is known about genes participating in the molecular mechanism by which the application of a shear stress suppresses the apoptosis of endothelial cells, namely the signal transduction mechanism.

The understanding of the molecular mechanism by which vascular endothelial cells respond to a shear stress leads us to learn the mechanism of development of various vascular diseases caused by arteriosclerosis, and the target for treatment. In order to elucidate the signal transduction mechanism, it is necessary to obtain a group of genes which exhibit a shear stress stimulus-dependent alteration of expression in vascular endothelial cells.

Moreover, the understanding of the molecular mechanism by which the apoptosis of vascular endothelial cells is suppressed in response to a shear stress stimulus leads us to elucidate the mechanism of the local formation of an early lesion of arteriosclerosis and thereby discover remedies for various vascular diseases caused by arteriosclerosis. In order to elucidate the molecular mechanism, it is necessary to obtain genes which exhibit a shear stress stimulus-dependent increase of expression in vascular endothelial cells and have an apoptosis-suppressing activity.

### Summary of the Invention

The present inventors made intensive investigations with a view to solving the above-described problems and have now obtained the following results. Specifically, mRNA derived from cultured vascular endothelial cells having a shear stress applied thereto was used as a template to prepare a cDNA library, and mRNA extracted from endothelial cells having no shear stress applied thereto was subtracted therefrom. Thus, a subtraction library was constructed in which genes exhibiting an increase of expression under shear stress-applied conditions was concentrated. However, since abundance of genes having a low amount of expression are equalized and empty vectors having no inserted fragment are increased in this subtraction library, a reverse subtraction method was newly developed to construct a second-generation subtraction library in which genes exhibiting an alteration of expression in response to a shear stress were concentrated from the subtraction library. Clones present in this second-generation subtraction library were randomly subjected to Northern hybridization, so that a large number of clones exhibiting an increase of expression by the application of a shear stress were obtained. Among these clones, not only the genes already known to exhibit an alteration of expression in response to a shear stress, but also genes presumed to act on the regulation of arteriosclerosis, genes which have not yet been known to be associated with arteriosclerosis, and novel genes were found. Furthermore, peptides encoded by these genes were found. Thus, the present invention has been completed.

Specifically, the present invention provides the following (1) to (76).
(1) A DNA having a nucleotide sequence selected from the nucleotide sequences represented by SEQ ID NO:143, 145, 147, 149, 151, 153, 155, 157, 168, 170 and 172.
(2) A shear stress-responsive DNA capable of hybridizing with a DNA having the nucleotide sequence represented by SEQ ID NO:143, 145, 149, 151, 153, 155, 157, 168, 170 or 172 under stringent conditions.
(3) A shear stress-responsive DNA capable of hybridizing with a DNA having the nucleotide sequence represented by SEQ ID NO:147 under stringent conditions, and having not less than 90% homology with the DNA.
(4) A DNA having the same sequence as 5 to 60 consecutive bases in a nucleotide sequence selected from the nucleotide sequences represented by SEQ ID NO:143, 145, 149, 153, 155, 157, 168, 170 and 172, or a DNA having a sequence complementary to the DNA.
(5) A method for detecting an mRNA for a shear stress-responsive gene using a DNA according to any of (1) to (4).
(6) A diagnostic agent for vascular diseases caused by arteriosclerosis which contains a DNA according to any of (1) to (4).
(7) A method for detecting a gene causative of arteriosclerosis using a DNA according to any of (1) to (4).
(8) A method for screening an agent for regulating the transcription or translation of a shear stress-responsive gene using a DNA according to any of (1) to (4).
(9) A method for screening a therapeutic agent for vascular diseases caused by arteriosclerosis using a DNA according to any of (1) to (4).
(10) A therapeutic agent for vascular diseases caused by arteriosclerosis which contains a DNA according to any of (1) to (4).
(11) A recombinant virus vector containing a DNA according to any of (1) to (4).
(12) A recombinant virus vector containing an RNA comprising a sequence homologous with the sense strand of a DNA according to any of (1) to (4).
(13) A DNA having a nucleotide sequence selected from the nucleotide sequences represented by SEQ ID NO: 111, 113, 115, 116, 117, 119, 121, 123, 125, 127, 129, 130, 131, 132, 133, 134, 135, 137, 139 and 141.
(14) A shear stress-responsive DNA capable of hybridizing with the DNA according to (13) under stringent conditions.
(15) A DNA having the same sequence as 5 to 60 consecutive bases in a nucleotide sequence selected from the nucleotide sequences represented by SEQ ID NO:111, 113, 115, 116, 117, 119, 121, 123, 125, 127, 129, 130, 131, 132, 133, 134, 135, 137, 139 and 141, or a DNA having a sequence complementary to the DNA.
(16) A diagnostic agent for vascular diseases caused by arteriosclerosis which contains a DNA according to any of (13) to (15).
(17) A method for detecting a gene causative of arteriosclerosis using a DNA according to any of (13) to (15).
(18) A method for screening an agent for regulating the transcription or translation of a shear stress-responsive gene using a DNA according to any of (13) to (15).
(19) A method for screening a therapeutic agent for vascular diseases caused by arteriosclerosis using a DNA according to any of (13) to (15).
(20) A method for detecting an mRNA for a shear stress-responsive gene using a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.
(21) A method for identifying the apoptosis sensitivity of cells by detecting the endogenous transcription level of a DNA having the nucleotide sequence represented by SEQ ID NO:7 using a DNA having the nucleotide sequence represented by SEQ ID NO:7 or a DNA having the same sequence as 5 to 60 consecutive bases in the nucleotide sequence represented by SEQ ID NO:7.
(22) A method for suppressing or promoting the apoptosis of cells by regulating the endogenous transcription or translation of a DNA having the nucleotide sequence represented by SEQ ID NO:7 using a DNA having the nucleotide sequence represented by SEQ ID NO:7 or a DNA having the same sequence as 5 to 60 consecutive bases in the nucleotide sequence represented by SEQ ID NO:7, or an antisense DNA having a nucleotide sequence complementary to the nucleotide sequence of each of these DNAs.
(23) A diagnostic agent for vascular diseases caused by arteriosclerosis which contains a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.
(24) An agent for identifying the apoptosis sensitivity of cells which contains a DNA having the nucleotide sequence represented by SEQ ID NO:7, or a DNA having the same sequence as 5 to 60 consecutive bases in the nucleotide sequence represented by SEQ ID NO:7.
(25) A method for screening an agent for regulating the transcription or translation of a shear stress-responsive gene using a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.
(26) A method for screening a therapeutic agent for vascular diseases caused by arteriosclerosis using a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.
(27) A method for screening an agent for suppressing or promoting the apoptosis of cells by regulating the endogenous transcription or translation of a DNA having the nucleotide sequence represented by SEQ ID NO:7 using a DNA having the nucleotide sequence represented by SEQ ID NO:7 or a DNA having the same sequence as 5 to 60 consecutive bases in the nucleotide sequence represented by SEQ ID NO:7.
(28) A therapeutic agent for vascular diseases caused by arteriosclerosis which contains a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.
(29) An agent for suppressing or promoting the apoptosis of cells which contains a DNA having the nucleotide sequence represented by SEQ ID NO:7 or a DNA having the same sequence as 5 to 60 consecutive bases in the nucleotide sequence represented by SEQ ID NO:7, or an antisense DNA having a nucleotide sequence complementary to the nucleotide sequence of each of these DNAs.
(30) A recombinant virus vector containing a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.
(31) A recombinant virus vector containing an RNA comprising a sequence homologous with the sense strand of a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.
(32) A therapeutic agent for vascular diseases caused by arteriosclerosis which contains a recombinant virus vector according to (30) or (31).
(33) A method for suppressing the apoptosis of cells using a recombinant virus vector containing a DNA having the nucleotide sequence represented by SEQ ID NO:7, or a recombinant virus vector containing an RNA comprising a sequence homologous with the sense strand of a DNA having the nucleotide sequence represented by SEQ ID NO:7.
(34) A method for screening an agent for suppressing or promoting the apoptosis of cells using a recombinant virus vector containing a DNA having the nucleotide sequence represented by SEQ ID NO:7, or a recombinant virus vector containing an RNA comprising a sequence homologous with the sense strand of a DNA having the nucleotide sequence represented by SEQ ID NO:7.
(35) A protein having an amino acid sequence selected from the amino acid sequences represented by SEQ ID NO:144, 146, 148, 150, 152, 154, 156, 158, 169, 171 and 173.
(36) A protein comprising an amino acid sequence in which one or more amino acids are deleted, replaced or added as compared with the amino acid sequence possessed by the protein according to (35), and having an activity participating in the formation of an arteriosclerotic lesion.
(37) A DNA encoding a protein according to (35) or (36).
(38) A recombinant DNA obtained by inserting a DNA according to any of (1)-(4) and (37) into a vector.
(39) A transformant obtained by introducing the recombinant DNA according to (38) into a host cell.
(40) A process for the preparation of a protein which comprises culturing the transformant according to (39) in a culture medium, causing a protein according to (35) or (36) to be produced and accumulated in the culture medium, and harvesting the protein from the resulting culture.
(41) A method for screening a therapeutic agent for vascular diseases caused by arteriosclerosis which comprises culturing the transformant according to (39) in a culture medium and using the resulting culture for the screening.
(42) A method for screening a therapeutic agent for vascular diseases caused by arteriosclerosis using a protein according to (35) or (36).
(43) A recombinant virus vector capable of producing a protein according to (35) or (36).
(44) A therapeutic agent for vascular diseases caused by arteriosclerosis which contains the recombinant virus vector of (43).
(45) An antibody capable of recognizing a protein according to (35) or (36).
(46) A method for detecting a protein according to (35) or (36) immunologically using the antibody according to (45).
(47) A method for screening a therapeutic agent for vascular diseases caused by arteriosclerosis using the antibody according to (45).
(48) A method for screening an agent for regulating the transcription or translation of a shear stress-responsive gene using the antibody according to (45).
(49) A diagnostic agent for vascular diseases caused by arteriosclerosis which contains the antibody according to (45).
(50) A therapeutic agent for vascular diseases caused by arteriosclerosis which contains the antibody according to (45).
(51) A drug delivery method which comprises combining the antibody of (45) with a radioactive isotope, a protein or a low-molecular-weight agent, and delivering the resulting conjugated antibody to an arteriosclerotic lesion.
(52) An antibody capable of recognizing a protein having an amino acid sequence represented by SEQ ID NO:112, 114, 118, 120, 122, 124, 126, 128, 136, 138, 140 and 142.
(53) A method for screening a therapeutic agent for vascular diseases caused by arteriosclerosis using the antibody according to (52).
(54) A method for screening an agent for suppressing the transcription or translation of a shear stress-responsive gene using the antibody according to (52).
(55) A diagnostic agent for vascular diseases caused by arteriosclerosis which contains the antibody according to (52).
(56) A therapeutic agent for vascular diseases caused by arteriosclerosis which contains the antibody according to (52).
(57) A drug delivery method which comprises combining the antibody of (52) with a radioactive isotope, a protein or a low-molecular-weight agent, and directing the resulting conjugated antibody to an arteriosclerotic lesion.
(58) A method for screening an agent capable of binding specifically to a protein having the amino acid sequence represented by SEQ ID NO:8 and effective for suppressing or promoting the apoptosis of cells, using a protein having the amino acid sequence represented by SEQ ID NO:8.
(59) A method for screening an agent for suppressing or promoting the apoptosis of cells which comprises inserting a DNA having the nucleotide sequence represented by SEQ ID NO:7 or a DNA encoding a protein having the amino acid sequence represented by SEQ ID NO:8, into a vector; introducing the resulting recombinant DNA into a host cell; culturing the resulting transformant in a culture medium; and using the resulting culture for the screening.
(60) A recombinant virus vector capable of producing a protein having an amino acid sequence selected from the amino acid sequences represented by SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108 and 110.
(61) A therapeutic agent for vascular diseases caused by arteriosclerosis which contains the recombinant virus vector of (60).
(62) A method for suppressing the apoptosis of cells using a recombinant virus vector capable of producing a protein having the amino acid sequence represented by SEQ ID NO:8.
(63) An agent for suppressing the apoptosis of cells which contains a recombinant virus vector capable of producing a protein having the amino acid sequence represented by SEQ ID NO:8.
(64) A method for screening a therapeutic agent for vascular diseases caused by arteriosclerosis using an antibody capable of recognizing a protein having the amino acid sequence represented by SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108 or 110.
(65) A method for screening an agent for suppressing or promoting the transcription or translation of a shear stress-responsive gene using an antibody capable of recognizing a protein having the amino acid sequence represented by SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108 or 110.
(66) A method for regulating the apoptosis of cells using an antibody capable of recognizing a protein having the amino acid sequence represented by SEQ ID NO:8.
(67) A method for screening an agent for regulating the apoptosis of cells using an antibody capable of recognizing a protein having the amino acid sequence represented by SEQ ID NO:8.
(68) A method for identifying the apoptosis sensitivity of cells by detecting the expression level of a protein having the amino acid sequence represented by SEQ ID NO:8 using an antibody capable of recognizing a protein having the amino acid sequence represented by SEQ ID NO:8.
(69) A method according to any of (21), (22), (27), (33), (34), (58), (59), (62), (66), (67) and (68) wherein the cells are vascular endothelial cells.
(70) A diagnostic agent for vascular diseases caused by arteriosclerosis which contains an antibody capable of recognizing a protein having the amino acid sequence represented by SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108 or 110.
(71) An agent for identifying the apoptosis sensitivity of cells which contains an antibody capable of recognizing a protein having the amino acid sequence represented by SEQ ID NO:8.
(72) A therapeutic agent for vascular diseases caused by arteriosclerosis which contains an antibody capable of recognizing a protein having the amino acid sequence represented by SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108 or 110.
(73) An agent for regulating the apoptosis of cells which comprises an antibody capable of recognizing a protein having the amino acid sequence represented by SEQ ID NO:8.
(74) An agent for suppressing or promoting the apoptosis of cells which is obtained by a method according to any of (27), (34), (58), (59) and (67).
(75) An agent according to any of (24), (29), (63), (71), (73) and (74) wherein the cells are vascular endothelial cells.
(76) A drug delivery method which comprises combining an antibody capable of recognizing a protein having the amino acid sequence represented by SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108 or 110, with a radioactive isotope, a protein or a low-molecular-weight agent, and directing the resulting conjugated antibody to an arteriosclerotic lesion.

The term "regulate" as used herein means the action of suppressing or promoting. Moreover, the term "agent" refers to any substances having an arbitrary molecular weight such as proteins and nucleic acids.

The DNA of the present invention is a shear stress-responsive DNA. Examples thereof include a DNA having a nucleotide sequence selected from the nucleotide sequences represented by SEQ ID NO:143, 145, 147, 149, 151, 153, 155, 157, 168, 170 and 172; and a DNA capable of hybridizing with the foregoing DNAs under stringent conditions and showing an alteration in the expression level in response to the application of a shear stress.

The above-described DNA capable of hybridizing with a nucleotide sequence selected from the nucleotide sequences represented by SEQ ID NO:143, 145, 147, 149, 151, 153, 155, 157, 168, 170 and 172 under stringent conditions is a DNA obtained by carrying out colony hybridization, plaque hybridization or Southern blot hybridization while using a DNA having a nucleotide sequence selected from the nucleotide sequences represented by SEQ ID NO:143, 145, 147, 149, 151, 153, 155, 157, 168, 170 and 172 as a probe. Specifically, it includes DNA which can be identified by using a filter having colony- or plaque-derived DNAs immobilized thereon to carry out hybridization at 65°C in the presence of 0.7-1.0 M NaCl, and then washing the filter with an SSC solution having a 0.1 - to 2-fold concentration (an SSC solution having a one-fold concentration is composed of 150 mM sodium chloride and 15 mM sodium citrate) under 65°C conditions.

Hybridization may be carried out according to the methods described in Molecular Cloning, A Laboratory Manual, Second Edition, Cold Spring Harbor Laboratory Press (1989) (hereinafter referred to briefly as "Molecular Cloning, Second Edition"); Current Protocols in Molecular Biology, John Wiley & Sons (1987-1997) (hereinafter referred to briefly as "Current Protocols in Molecular Biology"); DNA Cloning 1: Core Techniques, A Practical Approach, Second Edition, Oxford University (1995); and the like. Specific examples of the hybridizable DNAs include DNAs having not less than 60% homology, preferably not less than 80% homology, more preferably not less than 90% homology, and most preferably not less than 95% homology with a nucleotide sequence selected from the nucleotide sequences represented by SEQ ID NO:143, 145, 147, 149, 151, 153, 155, 157, 168, 170 and 172.

Furthermore, the DNA of the present invention also includes oligonucleotides and antisense oligonucleotide having a sequence of a part of the DNA of the present invention. The oligonucleotide includes, for example, an oligonucleotide having the same sequence as the nucleotide sequence of 5 to 60 residues, preferably 10 to 40 residues, in a nucleotide sequence selected from the nucleotide sequences represented by SEQ ID NO:143, 145, 147, 149, 151, 153, 155, 157, 168, 170 and 172. The antisense oligonucleotide includes, for example, an antisense oligonucleotide of the foregoing oligonucleotide.

The protein of the present invention includes a protein having an activity associated with arteriosclerosis. Specific examples thereof include a protein having an amino acid sequence selected from the amino acid sequences represented by SEQ ID NO:144, 146, 148, 150, 152, 154, 156, 158, 169, 171 and 173, and a protein comprising amino acid sequences in which one or more amino acids are deleted, replaced or added as compared with the amino acid sequence possessed by the foregoing protein, and having an activity involved in the formation of an arteriosclerotic lesion.

The protein comprising amino acid sequence in which one or more amino acids are deleted, replaced or added as compared with the amino acid sequence of protein having an amino acid sequence selected from the amino acid sequences represented by SEQ ID NO:144, 146, 148, 150, 152, 154, 156, 158, 169, 171 and 173, and having an activity involved in the formation of an arteriosclerotic lesion may be prepared according to the methods described in Molecular Cloning, Second Edition; Current Protocols in Molecular Biology; Nucleic Acids Research, 10, 6487(1982); Proc. Natl. Acad. Sci. USA, 79, 6409(1982); Gene, 34, 315(1985); Nucleic Acids Research, 13, 4431(1985); Proc. Natl. Acad. Sci. USA, 82, 488(1985); and the like.

Moreover, among the acquired large number of genes exhibiting an increase of expression by the application of a shear stress in vascular endothelial cells, the present inventors have found A4RS-041 having homology with LFG (lifeguard), a brain-specific gene which has been reported to suppress Fas-mediated apoptosis [Proc. Natl, Acad. Sci. USA, 22, 12673-12678(1999)]. First of all, according to an analysis of the nucleotide sequence of A4RS-041, the present inventors have found that A4RS-041 is a gene entirely different from LFG because A4RS-041 has about 50% identity to LFG, but about one-third thereof on the amino-terminal side has little homology. Moreover, the present inventors have also found that the expression profiles of A4RS-041 and LFG in tissues are substantially different because A4RS-041 is widely expressed in a variety of tissues including vascular endothelial cells, whereas LFG is highly expressed in the brain but not in vascular endothelial cells. Furthermore, by constructing a transformed cell which permits A4RS-041 to be stably and highly expressed, the present inventors have also found that A4RS-041 suppresses Fas-mediated apoptosis, thus ascertaining that A4RS-041 is a key molecule for the suppression of the apoptosis of vascular endothelial cells by a shear stress. Thus, the present invention has been completed.

### Brief Description of the Drawings

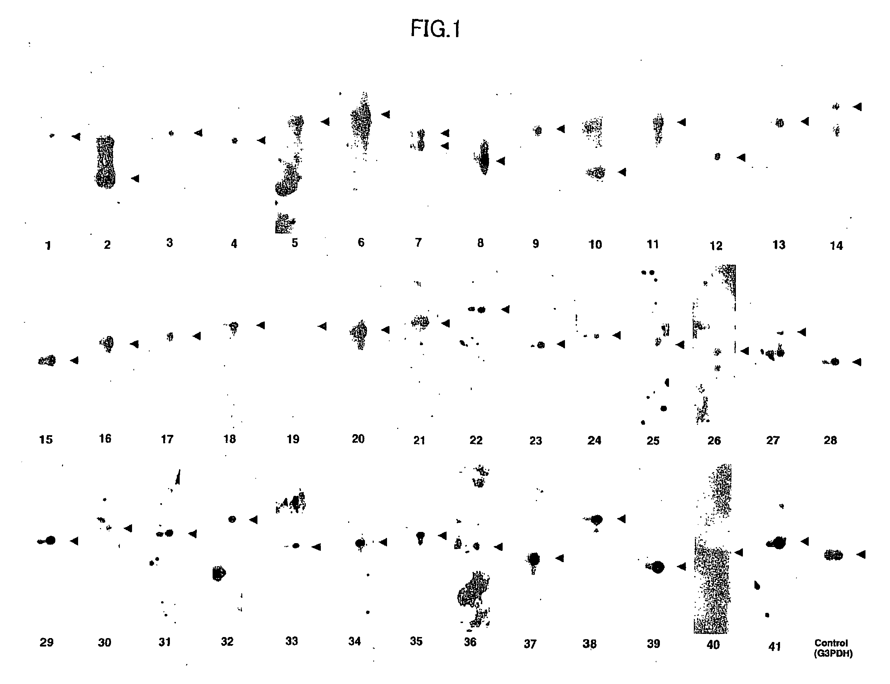

FIG. 1 illustrates the results of Northern analysis of genes exhibiting an increase of expression in response to a shear stress stimulus. Lanes 1-41 show shear stress-dependent increases of expression for A4RS-016, -026, -040, -041, -063, -096, -116, -126, -131, -148, -154, -174, -175, -194, -197, -260, -271, -307, -355, -389, -391, -423, -431, -453, -492, -507, -514, -523, -544, -547, -557, -577, -588, -602, -608, -612, -625, -666, -668, -674 and -682, respectively. In each blot, 4 µg of total RNA derived from HUVEC having no shear stress applied thereto (with a stimulation time of 0) was electrophoresed in the left-hand lane, and 4 µg of total RNA derived from HUVEC. having a shear stress applied thereto (a mixture of equal amounts of total RNA samples derived from HUVEC stimulated for 0.5, 1, 1.5, 2, 3, 4, 6, 10 and 20 hours) was electrophoresed in the right-hand lane.

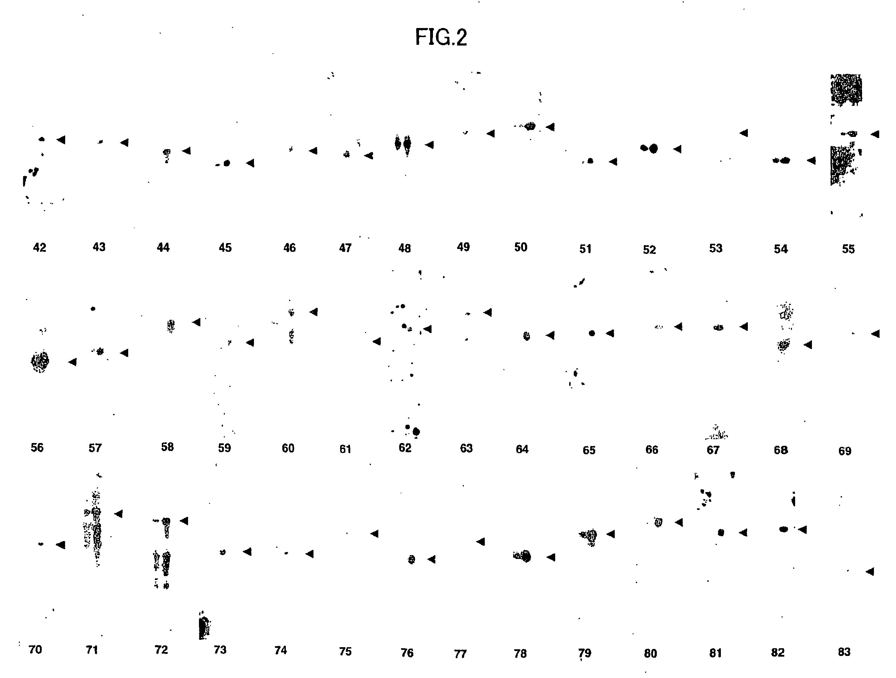

FIG. 2 illustrates the results of Northern analysis of genes exhibiting an increase of expression in response to a shear stress stimulus. Lanes 42-83 show shear stress-dependent increases of expression for A4RS-751, -781, -784, -817, -818, -914, -929, -935, -938, -939, -945, -947, -948, -949, -011, -115, -143, -171, -193, -280, -402, -533, -604, -615, -619, -626, -676, -679, -737, -780, -826, -916, -933, -943, -002, -049, -230, -239, -242, -491, -578 and -829, respectively. In each blot, 4 µg of total RNA derived from HUVEC having no shear stress applied thereto (with a stimulation time of 0) was electrophoresed in the left-hand lane, and 4 µg of total RNA derived from HUVEC having a shear stress applied thereto (a mixture of equal amounts of total RNA samples derived from HUVEC stimulated for 0.5, 1, 1.5, 2, 3, 4, 6, 10 and 20 hours) was electrophoresed in the right-hand lane.

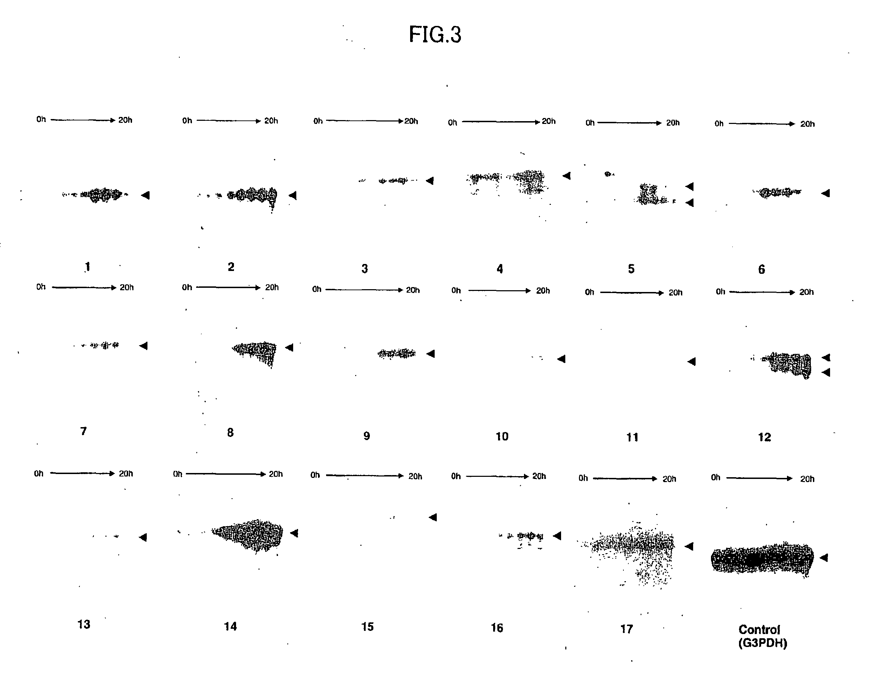

FIG. 3 illustrates the results of Northern blotting analysis of genes expressed in response to a shear stress stimulus, showing their changes of expression with time. Lanes 1-17 show shear stress-dependent increases of expression for A4RS-016, -041, -063, -096, -116, -260, -271, -307, -389, -391, -602, -784, -115, -143, -193, -280 and -402, respectively. In each blot, 4 µg of total RNA samples derived from HUVEC having shear stress application times of 0, 0.5, 1, 1.5, 2, 3, 4, 6, 10 and 20 hours respectively were electrophoresed as viewed from left to right.

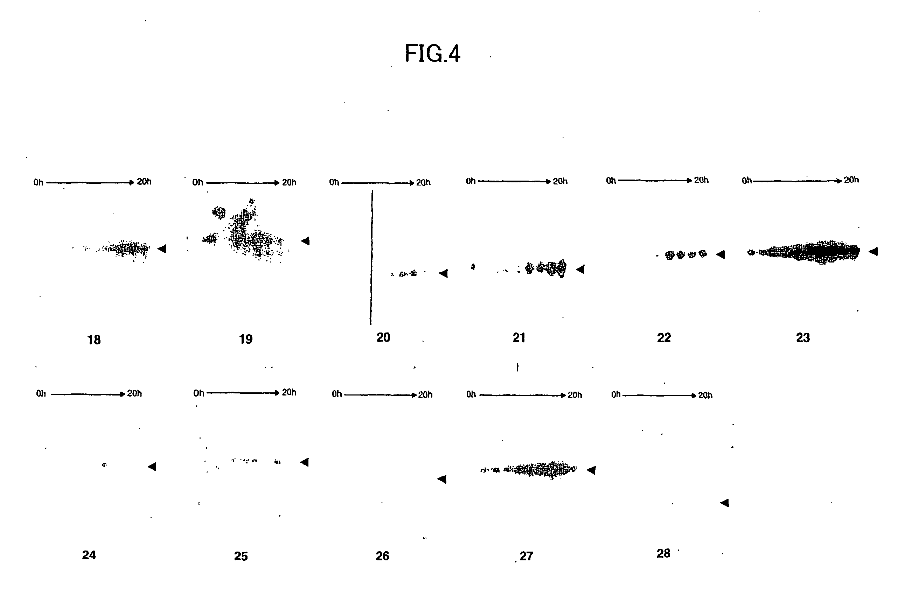

FIG. 4 illustrates the results of Northern blotting analysis of genes expressed in response to a shear stress stimulus, showing their changes of expression with time. Lanes 18-28 show shear stress-dependent increases of expression for A4RS-604, -626, -916, -002, -049, -230, -239, -242, -491, -578 and -829, respectively. In each blot, 4 µg of total RNA samples derived from HUVEC having shear stress application times of 0, 0.5, 1, 1.5, 2, 3, 4, 6, 10 and 20 hours respectively were electrophoresed as viewed from left to right.

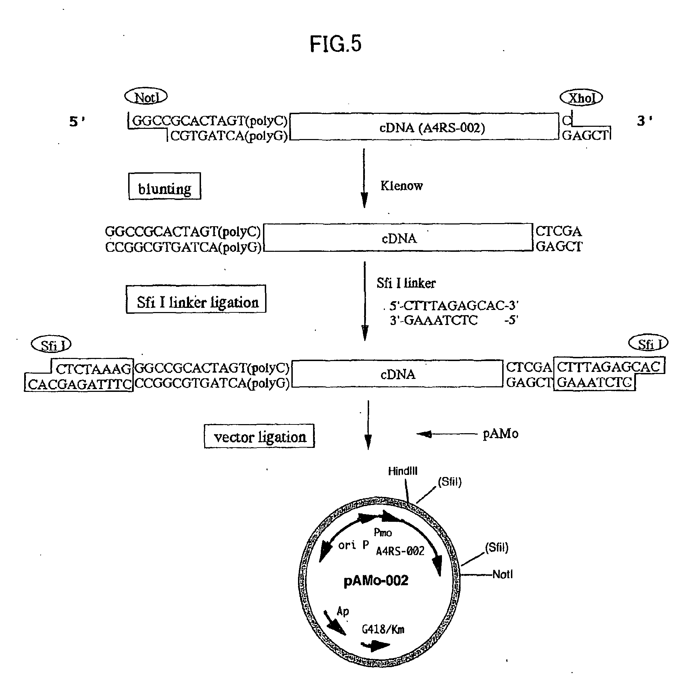

FIG. 5 illustrates the construction of the plasmid pAMo-002 for the expression in animal cells.

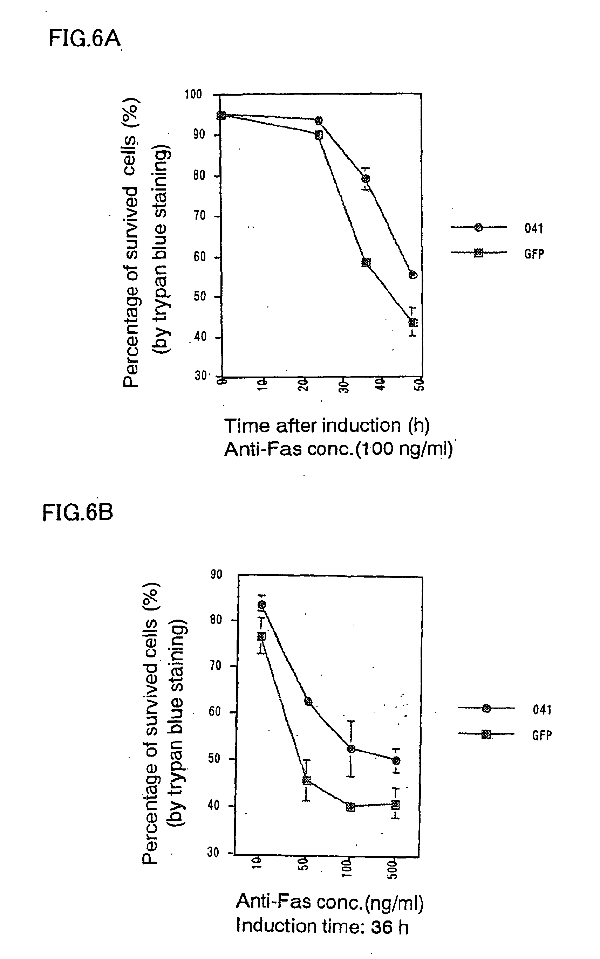

FIG. 6A and FIG. 6B are diagrams showing the apoptosis suppressing activity of A4RS-041. FIG. 6A shows changes with time when the anti-Fas monoclonal antibody concentration was fixed at 100 ng/ml, and FIG. 6B shows dependence on the anti-Fas monoclonal antibody concentration when the stimulation time was fixed at 36 hours. ● represents HeLa cells into which A4RS-041 was introduced, and ■ represents HeLa cells into which GFP was introduced.

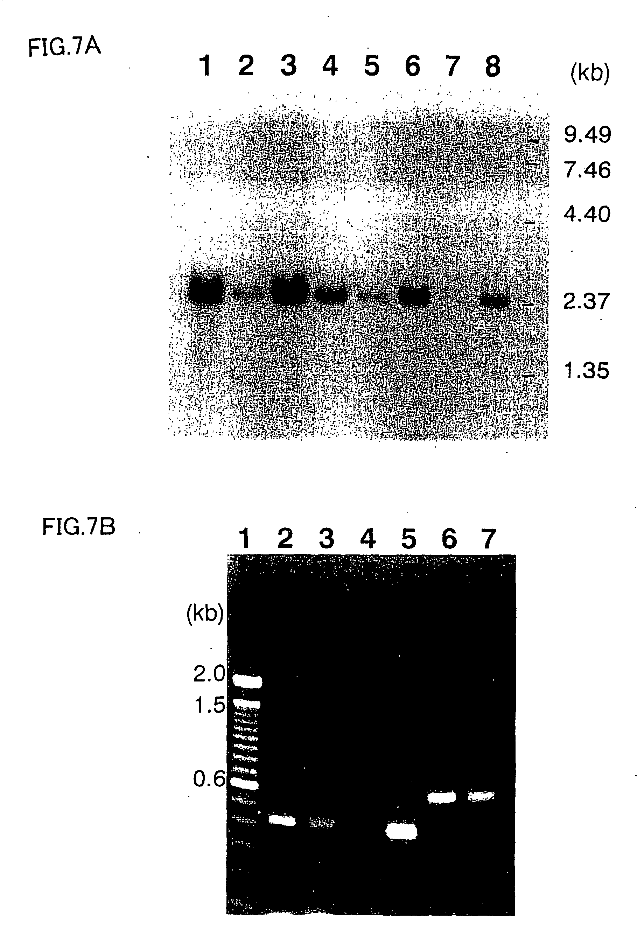

FIG. 7A and FIG. 7B are diagrams showing the distribution of expression of A4RS-041. FIG. 7A is a diagram showing the results obtained by analyzing the expression of A4RS-041 in human normal tissues by Northern blotting. FIG. 7B is a diagram showing the results obtained by analyzing the expression of A4RS-041 and LFG in human vascular endothelial cells and human brain by RT-PCR.

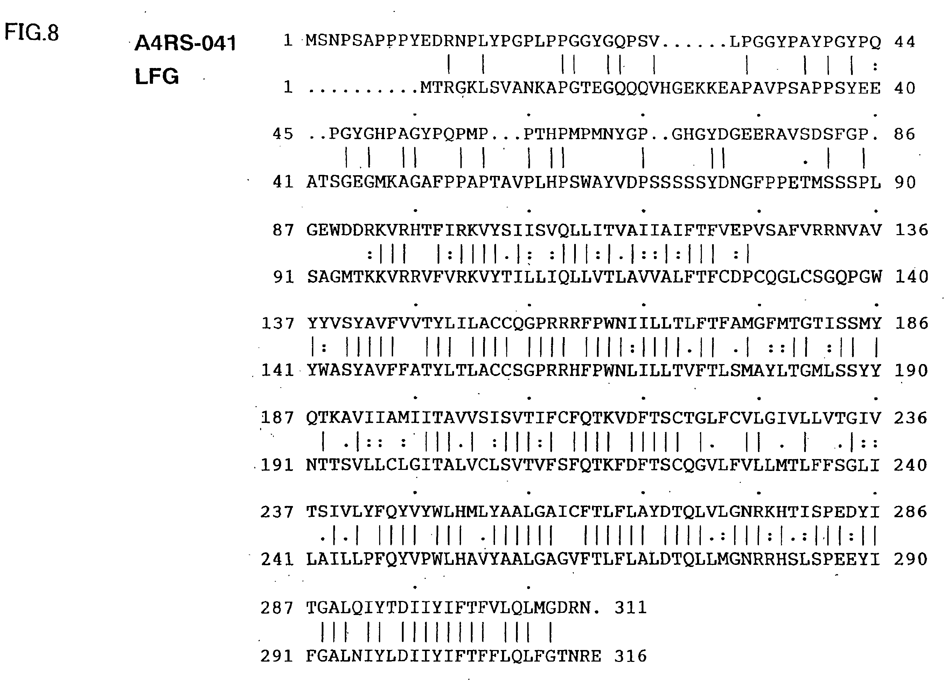

FIG. 8 is a diagram showing the amino acid sequence homology of A4RS-041 and LFG.

### Detailed Description of the Invention

The present invention will be more specifically described hereinbelow. No particular limitation is placed on the type of cells used to prepare the DNA of the present invention, so long as they exhibit responsiveness to the application of a shear stress. However, adhesion type cells are preferred. Examples thereof include vascular endothelial cells, and human vascular endothelial cells are especially preferred. More preferred are human umbilical vein endothelial cells (HUVECs). These vascular endothelial cells can be easily separated from a human umbilical cord according to the method described in Cell (in Japanese), 20, 329(1988) or Human Cell, 1, 188(1988). It is also possible to obtain and use secondary cultured cells having been separated. The passage number of vascular endothelial cells is not critical, provided that they retain properties as vascular endothelial cells. However, vascular endothelial cells having a passage number of 20 or less are preferred.

The culture medium used for cell culture may have a conventionally known composition. In the case, for example, of vascular endothelial cells, it is preferable to use a cell culture medium to which 0 to 20% of the blood serum of an animal such as cattle is added. More preferred is E-GM medium (containing 2% fetal calf serum; manufactured by Kurabo Industries, Ltd.) or M199 medium having 20% fetal calf serum added thereto. In order to promote the growth of cells, a cell growth factor such as ECGS (endothelial cell growth supplement), EGF (epidermal growth factor) or basic FGF (fibroblast growth factor) may be added to the culture medium. A high shear stress can be applied to the cultured cells by adding dextran or the like to the culture medium and thereby increasing the viscosity of the culture medium.

As the culture apparatus permitting the application of a shear stress, there may be used an apparatus of the micro-carrier type [Am. J. Physiol., 259, H804(1990)], the rotary disc type [Biorheology, 25, 461(1988)], the parallel plate type [Biotechnol. Bioeng., 27, 1021(1985)] or the like.

In the application of a shear stress, no particular limitation is placed on the method for the culture of vascular endothelial cells. One exemplary method is as follows. Vascular endothelial cells are allowed to adhere to micro-carriers and suspended in a culture medium within a spinner flask. Although the incubation temperature may be any desired temperature that permit the culture of the cells, it preferable to use a temperature of 37°C. The incubation is preferably carried out in an incubator filled with 5% carbon dioxide gas. No particular limitation is placed on the number of cells harvested, so long as RNA can be extracted therefrom. A typical example thereof is a number of that order which can be obtained by ordinary culture techniques, and a number of not less than 1 x 10⁶ cells is preferred. Although the incubation time is not specified, it is preferable to use an incubation time at which the expression of a gene is distinctly changed as compared with a culture without the application of a shear stress. Especially preferred is an incubation time which provides good viability of the cells. Specifically, an incubation time in the range of 4 to 24 hours is useful.

As the method for preparing total RNA from vascular endothelial cells having a shear stress applied thereto, the guanidine thiocyanate-cesium trifluoroacetate method [Methods in Enzymol., 154, 3(1987)] or the like may be employed.

As the method for preparing poly(A)⁺ RNA from total RNA, the oligo(dT)-immobilized cellulose column method (Molecular Cloning, Second Edition) or the like may be employed.

Furthermore, mRNA may be prepared by using a kit such as Fast Track mRNA Isolation Kit (manufactured by Invitrogen) or Quick Prep mRNA Purification Kit (manufactured by Amersham Pharmacia Biotech).

Now, the method for the construction of a cDNA library is described below. Usable methods for the construction of a cDNA library include the methods described in Molecular Cloning, Second Edition, Current Protocols in Molecular Biology, DNA Cloning 1: Core Techniques, A Practical Approach, Second Edition, Oxford University Press (1995), and the like; and methods using a commercially available kit such as Superscript Plasmid System for cDNA Synthesis and Plasmid Cloning (manufactured by Life Technologies) or ZAP-cDNA Synthesis Kit (manufactured by Stratagene).

As a cloning vector for the construction of a cDNA library, there may be used any of phage vectors, plasmid vectors and the like, provided that they can replicate autonomously in Escherichia coli K12 strain. Specific examples thereof include ZAP Express [manufactured by Stratagene; Strategies, 5, 58(1992)], pBluescript II SK(+) [Nucleic Acids Res., 17, 9494(1989)], λ zap II (manufactured by Stratagene), λgt10, λgt11 [DNA Cloning, A Practical Approach, 1, 49(1985)], λBlueMid (manufactured by Clontech), λExCell (manufactured by Amersham Pharmacia Biotech), pT7T318U (manufactured by Amersham Pharmacia Biotech), pcD2 [Mol. Cell. Biol., 3, 280(1983)] and pUC18 [Gene, 33, 103(1985)].

As a Escherichia coli for introducing vectors having cDNAs integrated thereinto, there may be used any microorganism that belongs to Escherichia coli. Specifically, Escherichia coli XL1-Blue MRF' [manufactured by Stratagene; Strategies, 5, 81(1992)], Escherichia coli C600 [Genetics, 39, 440(1954)], Escherichia coli Y1088 [Science, 222, 778(1983)], Escherichia coli Y1090 [Science, 222, 778(1983)], Escherichia coli NM522 [J. Mol. Biol., 166, 1(1983)], Escherichia coli K802 [J. Mol. Biol., 16, 118(1966)], Escherichia coli JM105 [Gene, 38, 275(1985)] and the like may be used.

Since this cDNA library has the characteristics of vascular endothelial cells having a shear stress applied thereto, it is useful, for example, in cloning a gene associated with a lesion occurring in vital vascular regions undergoing a change of shear stress (specifically, the formation of arteriosclerotic lesions, or the like) and in developing pharmaceuticals by controlling the expression of the gene. Moreover, this cDNA library is different in the types and quantities of genes contained therein, from a cDNA library constructed by using mRNA derived from another type of cells (specifically, standing-cultured vascular endothelial cells having no shear stress applied thereto) as a template. Accordingly, it is possible to isolate the above-described gene associated with the formation of arteriosclerotic lesions or a protein encoded by the gene while using the difference as an index.

As the method for concentrating genes exhibiting an increase of expression by the application of a shear stress from the cDNA library so constructed, there may be employed a method such as the subtraction method [Proc. Natl. Acad. Sci. USA, 88, 2825(1991)] or differential hybridization [J. Biol. Chem., 265, 2973(1990)].

As the method for selecting clones having expression specificity (i.e., exhibiting an increase of expression by the application of a shear stress) from the subtraction library in which such genes are concentrated in the above-described manner, there may be employed Northern hybridization [Molecular Cloning, Second Edition], RT(reverse-transcribed)-PCR [Current Protocols in Molecular Biology] or the like.

With respect to the shear stress-responsive clone selected in the above-described manner, the nucleotide sequence of the DNA can be determined by analyzing it according to a commonly employed nucleotide sequence analysis method such as the dideoxy method of Sanger et al. [Proc. Natl. Acad. Sci. USA, 74, 5463(1977)], or by means of a nucleotide sequence analyzer such as 373A DNA Sequencer (manufactured by Perkin Elmer).

The novelty of the nucleotide sequence determined in the above-described manner can be confirmed by using a homology search program (e.g., blast) to search the nucleotide sequence in nucleotide sequence databases such as GenBank, EMBL and DDBJ, and thereby ascertaining that the databases do not include any nucleotide sequence having a distinct identity to the aforesaid nucleotide sequence and hence considered to be identical thereto.

When the DNA obtained in the above-described manner is a partial DNA of the cDNA corresponding to a shear stress-related mRNA, a clone containing the full-length cDNA may be selected again from the cDNA library by using the DNA obtained in the above-described manner as a probe.

The selection of a cDNA clone from the cDNA library may be carried out by colony hybridization or plaque hybridization using a probe labeled with an isotope or digoxigenin [Sambrook et al., Molecular Cloning, Second Edition (1989)].

Examples of the full-length cDNA of the shear stress-responsive gene having a novel nucleotide sequence, which is obtained in the above-described manner, include DNAs having the nucleotide sequences represented by SEQ ID NO:143, 145, 147, 149, 151, 153, 155, 157, 168, 170 and 172.

Once the full-length cDNA of a shear stress-related gene is obtained and its nucleotide sequence is determined as described above, the desired DNA can be obtained by preparing primers based on the nucleotide sequence and carrying out PCR [PCR Protocols, Academic Press (1990)] while using cDNA synthesized from mRNA or a cDNA library as a template. Moreover, the desired DNA may also be prepared by using a DNA synthesizer to synthesize it chemically on the basis of the determined nucleotide sequence of the DNA. Usable DNA synthesizers include Model 392 DNA Synthesizer (manufactured by Perkin Elmer) using the phosphoamidite method, and the like.

On the basis of the nucleotide sequence information of the aforesaid DNA and DNA fragment, an oligonucleotide and an antisense oligonucleotide each having a partial sequence of the DNA of the present invention may be prepared according to a conventional method or by means of a DNA synthesizer.

Examples of the oligonucleotide or antisense oligonucleotide include a sense primer corresponding to a nucleotide sequence on the 5'-terminal side and an antisense primer corresponding to a nucleotide sequence on the 3'-terminal side, both in a partial nucleotide sequence of mRNA to be detected. However, the base corresponding to uracil in mRNA is thymidine in oligonucleotide primers. Preferably, the sense primer and antisense primer are oligonucleotides whose melting temperatures (Tm) and numbers of bases are not extremely different from each other and which consist of 10 to 40 bases.

Moreover, in the present invention, there may be used derivatives of the nucleotides. Examples thereof include methyl derivatives and phosphothioate derivatives of the nucleotides.

Now, the method for the preparation of a protein having an activity involved in the formation of an arteriosclerotic lesion is described below.

The cDNA of the shear stress-responsive gene, which was obtained in the above-described manner, encodes a protein having an activity involved in the formation of an arteriosclerotic lesion.

The activity involved in the formation of an arteriosclerotic lesion means an activity regulating the development of arteriosclerosis, and preferably an activity preventing the development of arteriosclerosis. Examples thereof include, but are not limited to, the following activities.

They include regulation of the incorporation of low-density lipoprotein (LDL) into the vascular endothelium; regulation of the incorporation of oxidized LDL into the vascular endothelium; regulation of the expression of LDL receptors in vascular endothelial cells; regulation of the production of oxidized LDL in vascular endothelial cells; regulation of the expression of scavenger receptors in the vascular endothelium; regulation of the infiltration of lymphocytes into blood vessels; regulation of the expression of a cell surface adhesion molecule promoting the infiltration of lymphocytes into blood vessels in vascular endothelial cells; regulation of the proliferation of vascular smooth muscle produced in vascular endothelial cells; regulation of the apoptosis of vascular endothelial cells; and the like.

The DNAs and proteins of the present invention have been found on the basis of their shear stress-dependent increase of expression in vascular endothelial cells. As described in the Background of the Invention, it is generally known that arteriosclerosis occurs frequently in places where a low shear stress is produced and the separation or stagnation of a flow or turbulence (e.g., eddies) tends to occur. Accordingly, the DNAs and proteins of the present invention are especially useful for the treatment or prevention of arteriosclerosis or various vascular diseases caused thereby, including non-limitative examples such as cardiac insufficiency, restenosis after PTCA, and hypertension.

If necessary, a DNA fragment of appropriate length containing a portion encoding the protein is prepared on the basis of the full-length cDNA.

An expression plasmid for the protein is created by inserting the DNA fragment or the full-length cDNA into an expression vector on the downstream side of a promoter.

The expression plasmid is introduced into a host cell suited to the expression vector.

As the host cell, there may be used any host cell that enables the expression of the desired DNA. For example, there may be used bacteria belonging to the genera Escherichia, Serratia, Corynebacterium, Brevibacterium, Pseudomonas, Bacillus and Microbacterium; yeasts belonging to the genera Kluyveromyces, Saccharomyces, Shizosaccharomyces, Trichosporon and Schawnniomyces; animal cells; and insect cells.

As the expression vector, there is used a vector which can be autonomously replicated or incorporated into a chromosome in the aforesaid host cell and which contains a promoter at a position capable of transcribing the shear stress-responsive DNA.

When a bacterium or the like is used as the host cell, the shear stress-responsive DNA expression vector is preferably a recombinant vector which can be autonomously replicated in the bacterium and which consists of a promoter, a ribosome-binding sequence, the shear stress-responsive DNA and a transcription termination sequence. A gene controlling the promoter may be contained therein.

Examples of such expression vectors include pBTrp2, pBTac1, pBTac2 (all commercially available from Boehringer Mannheim), pKK233-2 (manufactured by Amersham Pharmacia Biotech), pSE280 (manufactured by Invitrogen), pGEMEX-1 (manufactured by Promega), pQE-8 (manufactured by QIAGEN), pKYP10 (Japanese Published Unexamined Patent application No. 110600/83), pKYP200 [Agricultural Biological Chemistry, 48, 669 (1984)], pLSA1 [Agric. Biol. Chem., 53, 277(1989)], pGEL1 [Proc. Natl. Acad. Sci. USA, 82, 4306(1985)], pBluescript II SK(-) (manufactured by Stratagene), pGEX (manufactured by Amersham Pharmacia Biotech), pET-3 (manufactured by Novagen), pTerm2 (USP 4686191, USP 4939094, USP 5160735), pSupex, pUB110, pTP5, pC194, pEG400 [J. Bacteriol., 172, 2392(1990)].

The promoter may be any promoter that can express a gene in the host cell. Examples thereof include promoters derived from Escherichia coli and phages, such as trp promoter (Ptrp), lac promoter (Plac), P_{L} promoter, P_{R} promoter and T7 promoter; and SP01 promoter, SP02 promoter and penP promoter. Moreover, there may also be used artificially designed or modified promoters and the like, such as a promoter comprising two Ptrp's connected in series (Ptrpx2), tac promoter, letI promoter [Gene, 44, 29(1986)] and lacT7 promoter.

The ribosome-binding sequence may be any ribosome-binding sequence that can be expressed in the host cell. However, it is preferable to use a plasmid in which the distance between the Shine-Dargarno sequence and the initiation codon is adjusted to a suitable length (e.g., 6-18 bases).

In the nucleotide sequence of the protein-encoding part of the shear stress-responsive DNA of the present invention, some residues may be replaced so as to give the codons most suitable for its expression in the host. Thus, the production rate of the desired protein can be improved.

A transcription termination sequence is not necessarily required for the expression of the shear stress-responsive DNA of the present invention. However, it is desirable to dispose a transcription termination sequence just downstream of the structural gene.

Examples of the host cell include microorganisms belonging to the genera Escherichia, Serratia, Corynebacterium, Brevibacterium, Pseudomonas, Bacillus and the like, such as Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No.49, Escherichia coli W3110, Escherichia coli NY49, Bacillus subtilis, Bacillus amyloliquefaciens, Brevibacterium ammoniagenes, Brevibacterium immariophilum ATCC14068, Brevibacterium saccharolyticum ATCC14066, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilum ATCC15354, and Pseudomonas sp. D-0110.

As the method for introducing the recombinant vector, there may be employed any method that can introduce DNA into the aforesaid host cell. Examples thereof include the calcium ion method [Proc. Natl. Acad. Sci. USA, 69, 2110(1972)], the protoplast method (Japanese Published Unexamined Patent Application No. 248397/88), and the methods described in Gene, 17, 107(1982) and Molecular & General Genetics, 168, 111(1979).

Where a yeast is used as the host cell, usable expression vectors include, for example, YEp13 (ATCC37115), YEp24 (ATCC37051), YCp50 (ATCC37419), pHS19 and pHS15.

The promoter may be any promoter that can express a gene in the yeast. Examples thereof include PHO5 promoter, PGK promoter, GAP promoter, ADH promoter, gal 1 promoter, gal 10 promoter, heat shock protein promoter, MFα1 promoter and CUP 1 promoter.

Examples of the host cell include Saccharomyces cerevisae, Shizosaccharomyces pombe, Kluyveromyces lactis, Trichosporon pullulans and Schwanniomyces alluvius.

As the method for introducing the recombinant vector, there may be employed any method that can introduce DNA into yeasts. Examples thereof include the electroporation method [Methods. Enzymol., 194, 182(1990)], the spheroplast method [Proc. Natl. Acad. Sci. USA, 75, 1929(1978)], the lithium acetate method [J. Bacteriol., 153, 163(1983)], and the methods described in Proc. Natl. Acad. Sci. USA, 75, 1929(1978).

Where an animal cell is used as the host cell, usable expression vectors include, for example, pcDNAI, pcDM8 (manufactured by Funakoshi), pAGE107 [Japanese Published Unexamined Patent Application No. 22979/90; Cytotechnology, 3, 133(1990)], pAS3-3 (Japanese Published Unexamined Patent Application No. 227075/90), pCDM8 [Nature, 329, 840(1987)], pcDNAI/Amp (manufactured by Invitrogen), pREP4 (manufactured by Invitrogen), pAGE103 [J. Biochem., 101,1307(1987)] and pAGE210.

The promoter may be any promoter that can be expressed in the animal cell. Examples thereof include the promoter of the IE (immediate early) gene of cytomegalovirus (human CMV), the early promoter of SV40, the promoter of retroviruses, metallothionein promoter, heat shock protein promoter, and SRα promoter. The enhancer of the IE gene of human CMV may be used in conjunction with the promoter.

Examples of the host cell include Namalwa cell derived from a human, COS cell derived from a monkey, CHO cell derived from a Chinese hamster, and HBT5637 (Japanese Published Unexamined Patent Application No. 299/88).

As the method for introducing the recombinant vector into the animal cell, there may be employed any method that can introduce DNA into animal cells. Examples thereof include the electroporation method [Cytotechnology, 3, 133(1990)], the calcium phosphate method (Japanese Published Unexamined Patent Application No. 227075/90), the lipofection method [Proc. Natl. Acad. Sci. USA, 84, 7413(1987)], and the methods described in Virology, 52, 456(1973). Transformants may be harvested and cultured according to the method described in Japanese Published Unexamined Patent Application No. 227075/90 or 257891/90.

Where an insect cell is used as the host, the protein may be expressed according to the method described, for example, in Baculovirus Expression Vectors, A Laboratory Manual, Current Protocols in Molecular Biology Supplement 1-38 (1987-1997), or Bio/Technology, 6, 47(1988).

Specifically, a recombinant gene transfer vector and a baculovirus are co-introduced into an insect cell. After a recombinant virus is obtained in the culture supernatant of the insect cell, an insect cell is further infected with the recombinant virus to express the protein.

Examples of the gene transfer vector used in this method include pVL1392, pVL1393 and pBlueBacIII (all manufactured by Invitrogen).

As the baculovirus, there may be used, for example, autographa californica nuclear polyhedrosis virus that is a virus infecting insects belonging to Noctuidae.

As the insect cell, there may be used Sf9 and Sf21 that are ovarian cells of Spodoptera frugiperda [Baculovirus Expression Vectors, A Laboratory Manual, W.H. Freeman and Company, New York (1992)], High 5 that is an ovarian cell of Trichoplusia ni (manufactured by Invitrogen), and the like.

The methods which may be employed for co-introducing the aforesaid recombinant gene transfer vector and the aforesaid baculovirus into an insect cell in order to prepare a recombinant virus include, for example, the calcium phosphate method (Japanese Published Unexamined Patent Application No. 227075/90) and the lipofection method [Proc. Natl. Acad. Sci. USA, 84, 7413(1987)].

The expression of the gene may be effected not only by direct expression, but also by secretory production, fusion protein expression or the like, for example, according to the methods described in Molecular Cloning, Second Edition.

When the gene is expressed by means of a yeast, an animal cell or an insect cell, the protein having a sugar or sugar chain added thereto may be obtained.

A shear stress-responsive protein may be prepared by culturing a transformant containing a recombinant DNA having the shear stress-responsive DNA integrated thereinto in a culture, causing a shear stress-responsive protein to be produced and accumulated in the culture, and harvesting the protein from the resulting culture.

In order to culture the transformant of the present invention for the preparation of the shear stress-responsive protein in a culture medium, there may be employed a common method for the culture of the host.

When the transformant of the present invention is a procaryote (e.g., Escherichia coli) or a eucaryote (e.g., yeast), the culture medium for the culture of such microorganisms may be a natural medium or a synthetic medium, provided that this medium contains a carbon source, a nitrogen source, minerals and other nutrients which can be assimilated by the microorganism and that this medium permits the transformant to be efficiently cultured.

The carbon source may be any carbon source that can be assimilated by the respective microorganisms. There may be used carbohydrates such as glucose, fructose, sucrose, molasses containing them, starch and starch hydrolyzate; organic acids such as acetic acid and propionic acid; and alcohols such as ethanol and propanol.

As the nitrogen source, there may be used ammonia; ammonium salts of various inorganic acids or organic acids, such as ammonium chloride, ammonium sulfate, ammonium acetate and ammonium phosphate; and other nitrogen-containing compounds, as well as peptone, meat extract, yeast extract, corn steep liquor, casein hydrolyzate, soybean meal and soybean meal hydrolyzate, various fermented bacterial cells and their digestion products, and the like.

As the minerals, there may be used potassium dihydrogen phosphate, dipotassium hydrogen phosphate, magnesium phosphate, magnesium sulfate, sodium chloride, ferrous sulfate, manganese sulfate, copper sulfate, calcium carbonate and the like.

The cultivation is carried out under aerobic conditions, for example, according to a shaking culture or deep aerated spinner culture technique. The incubation temperature should be in the range of 15 to 40°C and the incubation time usually ranges from 16 hours to 7 days. During cultivation, pH is maintained at 3.0 to 9.0. The adjustment of pH is made with an inorganic or organic acid, an alkaline solution, urea, calcium carbonate, ammonia or the like.

During cultivation, an antibiotic such as ampicillin or tetracycline may be added to the culture medium, if necessary.

When a microorganism transformed with an expression vector using an inducible promoter is cultured, an inducer may be added to the culture medium, if necessary. For example, when a microorganism transformed with an expression vector using lac promoter is cultured, isopropyl-β-D-thiogalactopyranoside (IPTG) or the like may be added to the culture medium, and when a microorganism transformed with an expression vector using trp promoter is cultured, indoleacrylic acid (IAA) or the like may be added to the culture medium.

As the culture medium for culturing a transformant obtained by using an animal cell as the host cell, there may be used any of commonly used culture media such as RPMI1640 medium [The Journal of the American Medical Association, 199, 519(1967)], Eagle's MEM [Science, 122, 501(1952)], Dulbecco-modified MEM [Virology, 8, 396(1959)], 199 medium [Proceeding of the Society for the Biological Medicine, 73, 1(1950)], and culture media prepared by adding fetal calf serum or the like to the foregoing media.

The cultivation is usually carried out for 1 to 7 days under conditions including a pH of 6 to 8, a temperature of 30 to 40°C, and the presence of 5% CO₂.

During cultivation, an antibiotic such as kanamycin or penicillin may be added to the culture medium, if necessary.

As the culture medium for culturing a transformant obtained by using an insect cell as the host cell, there may be used any of commonly used culture media such as TNM-FH medium (manufactured by Pharmingen), Sf-900 II SFM medium (manufactured by Life Technologies), ExCel1400, ExCel1405 (both manufactured by JRH Biosciences), and Grace's Insect Medium [Nature, 195, 788(1962)].

The cultivation is usually carried out for 1 to 5 days under conditions including a pH of 6 to 7 and a temperature of 25 to 30°C.

During cultivation, an antibiotic such as gentamicin may be added to the culture medium, if necessary.

In order to isolate and purify a protein having an activity associated with arteriosclerosis in accordance with the present invention, from the culture of the transformant of the present invention, there may be employed common techniques for the isolation and purification of enzymes.

For example, where the protein of the present invention is expressed in a dissolved state within cells, the cells are collected by centrifugation after completion of the incubation, suspended in an aqueous buffer, and disrupted with a sonicator, French press, Manton Gaulin homogenizer, Dynomill or the like to obtain a cell-free extract. From the supernatant obtained by centrifuging the cell-free extract, a purified preparation may be obtained by employing common techniques for the isolation and purification of enzymes, either alone or in combination. These techniques include, for example, solvent extraction, salting-out with ammonium sulfate or the like, desalting, precipitation with an organic solvent, anion-exchange chromatography using a resin such as diethylaminoethyl (DEAE)-Sepharose or DIAION HPA-75 (manufactured by Mitsubishi Chemical Corp.), cation-exchange chromatography using a resin such as S-Sepharose FF (manufactured by Amersham Pharmacia Biotech), hydrophobic chromatography using a resin such as butyl Sepharose or phenyl Sepharose, gel filtration with a molecular sieve, affinity chromatography, chromatofocusing, and electrophoresis such as isoelectric focusing.

Where the protein is expressed in the form of an insoluble material within cells, the cells are collected, disrupted and centrifuged. Thus, the insoluble protein is recovered as a precipitate fraction.

The insoluble protein so recovered is solubilized with a protein denaturing agent.

The solubilized solution is diluted or dialyzed to reduce the concentration of the protein denaturing agent in the solubilized solution and thereby refold the protein to a normal stereostructure. Thereafter, a purified preparation of the protein may be obtained according to the same isolation and purification techniques as described above.

Where the protein of the present invention or a derivative thereof (e.g., a glycosylated product) is secreted out of cells, the protein or a derivative thereof (e.g., a glycosylated product) may be recovered from the culture supernatant. Specifically, the culture supernatant is recovered from the resulting culture according to a technique such as centrifugation. Then, a purified preparation may be obtained from the culture supernatant by employing the same isolation and purification techniques as described above.

Examples of the protein thus obtained include proteins having the amino acid sequences represented by SEQ ID NO:144, 146, 148, 150, 152, 154, 156, 158, 169, 171, 173 and the like.

The protein expressed in the above-described manner may also be prepared by chemical synthesis processes such as Fmoc method (fluorenylmethyloxycarbonyl method) and tBoc method (t-butyloxycarbonyl method). Alternatively, they may also be synthesized by means of a peptide synthesizer manufactured by Sowa Trading Co. (Advanced ChemTech, USA), Perkin Elmer, Amersham Pharmacia Biotech, Aloka (Protein Technology Instrument, USA), Kurabo (Synthecell-Vega, USA), PerSeptive Japan, Ltd. (PerSeptive, USA), Shimadzu Corporation or the like.

Now, the methods for the preparation of antibodies recognizing the protein of the present invention are described below.

### (i) Preparation of a polyclonal antibody

A purified full-length or partial fragment of the protein obtained in the above-described manner, or a peptide having a partial amino acid sequence of the protein of the present invention is used as an antigen. A polyclonal antibody may be prepared by administering this antigen to an animal.

As the animal to which the antigen is administered, there may be used a rabbit, goat, mouse, hamster or the like. The dose of the antigen is preferably in the range of 50 to 100 µg per animal. When a peptide is used, it desirably used after being covalently bonded to carrier protein such as keyhole limpet haemocyanin or bovine thyroglobulin. The peptide used as an antigen may be synthesized by means of a peptide synthesizer.

After the first administration, the antigen is administered 3 to 10 times at intervals of 1 to 2 weeks. After each administration, blood is collected from the venous plexus of fundus oculi on the 3rd to 7th day, and the reaction of the serum with the antigen used for immunization is confirmed by enzyme immunoassay [Enzyme-Linked Immunosorbent assay (ELISA) (in Japanese), Igaku Shoin, 1976; Antibodies-A Laboratory Manual, Cold Spring Harbor Laboratory (1988)] or the like.

Serum is obtained from a nonhuman mammal whose serum exhibits a sufficient antibody titer against the antigen used for immunization. Then, a polyclonal antibody can be obtained by separating and purifying the serum.

The techniques which can be employed for the purpose of separation and purification include centrifugation, salting-out with 40-50% saturated ammonium sulfate, caprylic acid precipitation [Antibodies, A Laboratory Manual, Cold Spring Harbor Laboratory (1988)], chromatography using a DEAE-Sepharose column, anion-exchange column, protein A or G column, or gel filtration column, and the like. These techniques may be used either alone or in combination.

### (ii) Preparation of a monoclonal antibody

### (a) Preparation of antibody-producing cells

A rat whose serum exhibits a sufficient antibody titer against the partial fragment polypeptide of the protein of the present invention used for immunization is used as a source of antibody-producing cells.

After the antigenic substance is finally administered to the rat exhibiting the aforesaid antibody titer, the spleen is excised on the 3rd to 7th day. The spleen is minced in MEM (manufactured by Nissui Seiyaku Co., Ltd.) and loosened with a pincette. After this suspension is centrifuged at 1,200 rpm for 5 minutes, the supernatant is discarded. The spleen cells in the resulting precipitate fraction are treated with a Tris-ammonium chloride buffer (pH 7.65) for 1-2 minutes to remove erythrocytes, and washed three times with MEM. The spleen cells thus obtained are used as antibody-producing cells.

### (b) Preparation of myeloma cells

As the myeloma cells, an established cell line obtained from a mouse or rat is used.

Usable cell lines include, for example, the 8-azaguanine-resistant mouse (BALB/c-derived) myeloma cell line P3-X63Ag8-U1 (hereinafter abbreviated as P3-U1) [Curr. Topics. Microbiol. Immunol., 81, 1(1978); Europ. J. Immunol., 6, 511(1976)], SP2/0-Ag14(SP-2) [Nature, 276, 269(1978)], P3-X63-Ag8653(653) [J. Immunol., 123, 1548(1979)], and P3-X63-Ag8(X63) [Nature, 256, 495(1975)].

Such a cell line is subcultured in 8-azaguanine medium [a culture medium prepared by adding glutamine (1.5 mmol/l), 2-mercaptoethanol (5 x 10⁻⁵ M), gentamicin (10 µg/ml) and fetal calf serum (FCS) (manufactured by CSL, 10%) to RPMI-1640 medium (the resulting medium is hereinafter referred to as the normal medium) and further adding 8-azaguanine (15 µg/ml) thereto]. Three or four days before cell fusion, the cell line is cultured in the normal medium, and not less than 2 x 10⁷ cells are used for the purpose of cell fusion.

### (c) Formation of hybridomas

The antibody-producing cells obtained in (a) and the myeloma cells obtained in (b) are thoroughly washed with MEM or PBS (1.83 g disodium phosphate, 0.21 g monopotassium phosphate, 7.65 g sodium chloride, 1 liter distilled water, pH 7.2), and mixed so that the antibody-producing cells and the myeloma cells are present in a ratio of 5-10:1. After this mixture was centrifuged at 1,200 rpm for 5 minutes, the supernatant is discarded.

The mass of cells in the resulting precipitate fraction is thoroughly loosened, and 0.2 to 1 ml (per 10⁸ antibody-producing cells) of a solution prepared by mixing 2 g of polyethylene glycol-1000 (PEG 1000), 2 ml of MEM, and 0.7 ml of dimethyl sulfoxide (DMSO) is added to the mass of cells at 37°C with stirring. Moreover, 1 to 2 ml of MEM is added thereto several times at intervals of 1 to 2 minutes. After completion of the addition, MEM is added to make a total volume of 50 ml.

After the suspension so prepared is centrifuged at 900 rpm for 5 minutes, the supernatant is discarded. The cells of the resulting precipitate fraction are gently loosened, and gently suspended in 100 ml of HAT medium [a culture medium prepared by adding hypoxanthine (10⁻⁴ M), thymidine (1.5 x 10⁻⁵ M) and aminopterin (4 x 10⁻⁷ M) to the normal medium] by repeated sucking and blowing with a measuring pipette.

This suspension is pipetted into the wells of a 96-well culture plate in an amount of 100 µl per well, and incubated at 37°C in a 5% CO₂ incubator for 7 to 14 days. After incubation, a portion of the culture supernatant is taken, and hybridomas reacting specifically with the partial fragment polypeptide of the protein of the present invention are selected according to enzyme immunoassay as described in Antibodies, A Laboratory Manual, Cold Spring Harbor Laboratory, Chapter 14 (1988) or the like.

An exemplary procedure for enzyme immunoassay is described below.

The partial fragment polypeptide of the protein of the present invention, which was used as an antigen at the time of immunization, is coated on a suitable plate and reacted with a first antibody comprising the culture supernatant of a hybridoma or the purified antibody obtained in (d) below, further reacted with a second antibody comprising an anti-rat or anti-mouse immunoglobulin antibody labeled with biotin, an enzyme, a chemiluminescent substance, a radioactive compound or the like, and then subjected to a reaction depending on the labeling material. Thus, the hybridomas reacting specifically with the protein of the present invention are selected as hybridomas for producing a monoclonal antibody against the protein of the present invention.

Using these hybridomas, cloning is repeated twice according to the limiting dilution method [HT medium (HAT medium freed of aminopterin) was used for the first time and the normal medium for the second time]. A hybridoma exhibiting a high antibody titer stably is selected as a hybridoma strain capable of producing an antibody against the polypeptide of the protein of the present invention.

### (d) Preparation of a monoclonal antibody

5 x 10⁶ to 20 x 10⁶ cells per animal of the hybridoma capable of producing a monoclonal antibody against the protein of the present invention, which was obtained in (c), is intraperitoneally injected into 8- to 10-weeks-old mice or nude mice having been subjected to a pristane treatment (i.e., having been treated by administering 0.5 ml of 2,6,10,14-tetramethylpentadecane (pristane) intraperitoneally and kept for 2 weeks]. After 10 to 21 days, the hybridoma develops into an ascites tumor. Ascites is collected from a mouse having developed an ascites tumor, and centrifuged at 3,000 rpm for 5 minutes to remove any solid matter. From the resulting supernatant, a monoclonal antibody may be purified and harvested in the same manner as described above in connection with a polyclonal antibody.

The subclass of the antibody may be determined by means of a mouse monoclonal antibody typing kit or a rat monoclonal antibody typing kit. The amount of protein may be determined by the Lowry method or calculated from the absorbance at 280 nm.

Now, the method for preparing a recombinant virus vector useful for producing the protein of the present invention in a specific human tissue is described below.

The cDNA of the shear stress-responsive gene, which was obtained in the above-described manner, encodes a protein having an activity involved in the formation of an arteriosclerotic lesion.

If necessary, a DNA fragment of appropriate length containing a portion encoding the protein is prepared from the full-length cDNA.

A recombinant virus vector is created by inserting the DNA fragment or the full-length cDNA into a virus vector on the downstream side of a promoter.

This recombinant virus vector is introduced into a packaging cell suited to the vector.

As the packaging cell, there may be used any cell that, when the recombinant virus vector lacks any of the genes encoding proteins necessary for the packaging of the virus, can supplied the deficient proteins. For example, there may be used human kidney-derived HEK293 cell or mouse fibroblast cell NIH3T3. The proteins supplied by the packaging cell include mouse retrovirus-derived proteins such as gag, pol and env for a retrovirus vector; HIV virus-derived proteins such as gag, pol, env, vpr, vpu, vif, tat, rev and nef for a lentivirus vector; adenovirus-derived proteins such as E1A and E1B for an adenovirus vector; and proteins such as Rep(p5, p19, p40) and Vp(Cap) for an adeno-associated virus.

As the virus vector, there is used a virus vector which can produce the recombinant virus in the aforesaid packaging cell and which contains a promoter at a position permitting the shear stress-responsive DNA to be transcribed in a target cell. As the plasmid vector, there may be used MFG [Proc. Natl. Acad. Sci. USA, 92, 6733-6737 (1995)], pBabePuro [Nucleic Acids Res., 18, 3587-3596(1990)], LL-CG, CL-CG, CS-CG, CLG [Journal of Virology, 72, 8150-8157(1998)] and pAdexl [Nucleic Acids Res., 23, 3816-3821(1995)]. The promoter may be any promoter that can be expressed in human tissues. Examples thereof include the promoter of the IE (immediate early) gene of cytomegalovirus (human CMV), the early promoter of SV40, the promoter of retroviruses, metallothionein promoter, heat shock protein promoter, and SRα promoter. The enhancer of the IE gene of human CMV may be used in conjunction with the promoter.

Examples of the method for introducing the aforesaid recombinant virus vector into the aforesaid packaging cell include the calcium phosphate method (Japanese Published Unexamined Patent Application No. 227075/90) and the lipofection method [Proc. Natl. Acad. Sci. USA, 84, 7413(1987)].

Now, the method for detecting a shear stress-responsive mRNA using the shear stress-responsive DNA of the present invention is described below.

The DNAs which can be used in this method include, for example, DNAs having the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 116, 117, 119, 121, 123, 125, 127, 129, 130, 131, 132, 133, 134, 135, 137, 139, 141, 143, 145, 147, 149, 151, 153, 155, 157, 168, 170, 172 and the like. Moreover, they include a DNA having a sequence of part of the foregoing DNA, an oligonucleotide DNA having the nucleotide sequence of 5 to 60 consecutive bases in the foregoing DNA, and preferably an oligonucleotide DNA having the nucleotide sequence of 10 to 40 consecutive bases therein. Furthermore, they include a shear stress-responsive DNA capable of hybridizing with the foregoing DNA or a fragment thereof under stringent conditions.

The identification of a change in the expression level of a shear stress-responsive mRNA and a structural change of the expressed mRNA in human biological specimens and human primary cultured cells is useful in knowing the risk of developing arteriosclerosis in the future or the cause of an already developed vascular disease.

Examples of the method for detecting the expression level of a shear stress-responsive mRNA and a structural change thereof include (1) Northern blotting, (2) in situ hybridization, (3) quantitative PCR, (4) differential hybridization, (5) the DNA chip method, and (6) RNase protection assay.

The materials which can be analyzed by the aforesaid methods include mRNA or total RNA which is obtained from biological specimens (e.g., vascular endothelium, blood serum and saliva) collected from arteriosclerotic patients and healthy subjects, or a primary cultured cell sample prepared by isolating cells from such a biological specimen and culturing them in a suitable culture medium in vitro (the mRNA and total RNA are hereinafter referred to as the specimen-derived RNA). Alternatively, isolated paraffin or cryostat sections of tissues obtained from biological specimens may also be used.

Northern blotting is a technique in which the specimen-derived RNA is separated by gel electrophoresis, transferred to a support such as a nylon filter, hybridized with a labeled probe prepared from the DNA of the present invention, and then washed to detect a band bound specifically to a shear stress-responsive mRNA. Thus, the expression level of a shear stress-responsive mRNA and a structural change thereof can be detected. The hybridization is carried out by incubating the support under such conditions as a stable hybrid is formed between the probe and a shear stress-responsive mRNA in the specimen-derived RNA. In order to prevent a false positive reaction, it is desirable to carry out the hybridization and washing steps under highly stringent conditions. They are determined according to a large number of factors such as temperature, ionic strength, base composition, probe length and formamide concentration. These factors are described, for example, in Molecular Cloning, Second Edition (as mentioned above).

The labeled probe used for Northern blotting may be prepared, for example, by incorporating a radioactive isotope, biotin, a fluorescent group, a chemiluminescent group or the like into the DNA of the present invention or an oligonucleotide designed from the sequence of the DNA, according to a well-known technique (nick translation, random priming or kinasing). Since the amount of labeled probe hybridized reflects the expression level of the shear stress-responsive mRNA, the expression level of the shear stress-responsive mRNA can be determined by determining the amount of labeled probe hybridized. Moreover, a structural change of the shear stress-responsive mRNA can be detected by analyzing the binding site of the labeled probe.

The expression level of a shear stress-responsive mRNA can also be detected by in situ hybridization in which the hybridization and washing steps are carried out by using the aforesaid labeled probe and isolated paraffin or cryostat sections of tissues obtained from the living body. In order to prevent a false positive reaction in in situ hybridization, it is desirable to carry out the hybridization and washing steps under highly stringent conditions. They are determined according to a large number of factors such as temperature, ionic strength, base composition, probe length and formamide concentration. These factors are described, for example, in Current Protocols in Molecular Biology.

Some methods for detecting a shear stress-responsive mRNA, such as quantitative PCR, differential hybridization, and the DNA chip method, may be carried out on the basis of the synthesis of cDNA by using the specimen-derived RNA, an oligo-dT primer or random primer, and reverse transcriptase (the resulting cDNA is hereinafter referred to as the specimen-derived cDNA). When the specimen-derived RNA is mRNA, both of the aforesaid primers may be used. However, when the specimen-derived RNA is total RNA, it is necessary to use an oligo-dT primer.

In quantitative PCR, DNA fragments derived from the shear stress-responsive mRNA are amplified by carrying out PCR while using a template comprising the specimen-derived cDNA and primers designed on the basis of the nucleotide sequence possessed by the DNA of the present invention. Since the amount of the amplified DNA fragments reflects the expression level of the shear stress-responsive mRNA, the amount of the shear stress-responsive mRNA can be determined by using, as an internal control, a DNA encoding actin or G3PDH (glyceraldehyde 3-phosphate dehydrogenase) not responding to a shear stress. Moreover, a structural change of the shear stress-responsive mRNA can be detected by separating the amplified DNA fragments by gel electrophoresis. In this detection method, it is desirable to use suitable primers capable of amplifying a target sequence specifically and efficiently. Such suitable primers can be designed on the basis of the conditions that they do not hybridize between primers or within primers and that they hybridize specifically with the target cDNA at the annealing temperature and separate from the target under denaturing conditions. The quantitative determination of the amplified DNA fragments must be carried out within the range of the number of cycles of PCR in which the amplification product is increasing exponentially. Such a number of cycles of PCR can be known by recovering the amplified DNA fragment produced at each number of cycles of PCR and analyzing it by gel electrophoresis.

An alteration of the expression level of the shear stress-responsive mRNA can be detected by hybridizing and washing the DNA of the present invention immobilized on a filter or a substrate (e.g., slide glass or silicon) while using a probe comprising the specimen-derived cDNA synthesized from the specimen-derived RNA with the aid of dNTP. The methods based on this principle include methods called differential hybridization [Trends in Genetics, 7, 314-317(1991)] and the DNA chip method [Genome Research, 6, 639-645(1996)]. In both methods, the difference in the expression of the shear stress-responsive mRNA between a control specimen and a target specimen can be accurately detected by immobilizing an internal control (e.g., actin or G3PDH) on the filter or substrate. Moreover, the accurate expression level of the shear stress-responsive mRNA can be determined by synthesizing cDNAs from a control specimen and the specimen-derived RNA using different labeled dNTP and carrying out hybridization with the two labeled cDNA probes simultaneously on one filter or one substrate.

In RNase protection assay, a promoter sequence (e.g., T7 promoter or SP6 promoter) is first linked to the 3'-terminus of the DNA of the present invention. Then, in an in vitro transcription system using RNA polymerase, a labeled antisense RNA is synthesized using labeled rNTP. After this labeled antisense RNA is combined with the specimen-derived RNA to form an RNA-RNA hybrid, it is digested with RNase and a band protected from digestion is detected by gel electrophoresis. The expression level of the shear stress-responsive mRNA can be determined by assaying the protected band.

Now, the method for detecting a gene causative of arteriosclerosis using the shear stress-responsive DNA of the present invention is described below.

The DNAs which can be used in this method include, for example, DNAs having the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 116, 117, 119, 121, 123, 125, 127, 129, 130, 131, 132, 133, 134, 135, 137, 139, 141, 143, 145, 147, 149, 151, 153, 155, 157, 168, 170, 172 and the like. Moreover, they include a DNA having a sequence of part of the foregoing DNA, an oligonucleotide DNA having the nucleotide sequence of 5 to 60 consecutive bases in the foregoing DNA, and preferably an oligonucleotide DNA having the nucleotide sequence of 10 to 40 consecutive bases therein. Furthermore, they include a shear stress-responsive DNA capable of hybridizing with the foregoing DNA or a fragment thereof under stringent conditions.

The most accurate test for evaluating the presence or absence of a mutation causative of arteriosclerosis in the locus of a shear stress-responsive gene is a direct comparison of the genes from a control population with the genes from arteriosclerotic patients.

Specifically, human biological specimens such as vascular endothelium, blood serum or saliva, or specimens derived from primary cultured cells established from the biological specimens, are collected from 10 to 100 arteriosclerotic patients and healthy subjects. Then, DNA is extracted from each of the biological specimens or the primary cultured cell-derived specimens (this DNA is hereinafter referred to as the specimen-derived DNA). This specimen-derived DNA may be used directly, or may be used by amplifying a shear stress-responsive DNA using primers designed on the basis of the nucleotide sequence possessed by the DNA of the present invention. Alternatively, PCR may be carried out by using a template comprising the specimen-derived cDNA and primers designed on the basis of the nucleotide sequence possessed by the DNA of the present invention. Thus, a DNA fragment comprising a shear stress-responsive DNA sequence can be amplified and used.

In order to determine whether the DNA of the present invention has a mutation causative of arteriosclerosis, a method for detecting a heteroduplex formed by hybridization between a DNA strand having a wild type allele and a DNA strand having a mutated allele can be used.

The methods which can be used to detect a heteroduplex include (1) the detection of a heteroduplex by polyacrylamide electrophoresis [Trends Genet., 7, 5(1991)], (2) single strand conformation polymorphism analysis [Genomics, 16, 325-332(1993)], (3) chemical cleavage of mismatches (CCM), (4) enzymatic cleavage of mismatches [Nature Genetics, 9, 103-104(1996)], (5) denaturing gradient gel electrophoresis [Mutat. Res., 288, 103-112(1993)], and the like.

Using a template comprising the specimen-derived DNA or specimen-derived cDNA and primers designed on the basis of the sequence possessed by the DNA of the present invention, a shear stress-responsive DNA is amplified as a fragment smaller than 200 bp, and then subjected to polyacrylamide electrophoresis. If a heteroduplex is formed owing to a mutation of the shear stress-responsive DNA, it has lower mobility than a homoduplex having no mutation, and can hence be detected as extra bands. The use of a specially made gel (Hydro-link, MDE or the like) provides a higher degree of separation. The analysis of a fragment smaller than 200 bp makes it possible to detect an insertion, a deletion, and most one-base substitutions. It is desirable to carry out this heteroduplex analysis on one sheet of gel in combination with single strand conformation polymorphism analysis as described below.

In single strand conformation polymorphism analysis (SSCP analysis), a shear stress-responsive DNA is amplified as a fragment smaller than 200 bp by using a template comprising the specimen-derived DNA or specimen-derived cDNA and primers designed on the basis of the sequence possessed by the DNA of the present invention. The amplified shear stress-responsive DNA fragment is denatured and then electrophoresed through native polyacrylamide gel. During DNA amplification, the primers are labeled with an isotope or fluorochrome, or the unlabeled amplification product is stained with silver. Thus, the amplified shear stress-responsive DNA fragment can be detected as bands. In order to clarify the difference from a wild type pattern, a control specimen may be electrophoresed at the same time. Thus, fragments having a mutation can be detected owing to their difference in mobility.

In chemical cleavage of mismatches (CCM), the shear stress-responsive DNA is amplified by using a template comprising the specimen-derived DNA or specimen-derived cDNA and primers designed on the basis of the sequence possessed by the DNA of the present invention. The amplified DNA fragment is hybridized with a labeled DNA prepared by incorporating an isotope or fluorescent label into the DNA of the present invention, and treated with osmium tetroxide to cleave one strand of the DNA at a mismatching site. Thus, a mutation can be detected. CCM is one of the most sensitive detection methods and can be applied even to specimens of kilobase length.

In place of the aforesaid osmium tetroxide, a combination of an enzyme involved in the repair of mismatches in cells (e.g., T4 phage resolvase or endonuclease VII) and RNase A may be used. Thus, a mismatch can be cleaved enzymatically.

In denaturing gradient gel electrophoresis (DGGE), the shear stress-responsive DNA is amplified by using a template comprising the specimen-derived DNA or specimen-derived cDNA and primers designed on the basis of the sequence possessed by the DNA of the present invention. The amplified DNA fragment is electrophoresed through a gel having a concentration gradient of a chemical denaturing agent or a temperature gradient. The amplified DNA fragment moves through the gel up to a position where it is denatured into single strands, and cease to move after denaturation. Since the amplified DNA fragments move through the gel differently according to the presence or absence of a mutation in the shear stress-responsive DNA, the presence of a mutation can be detected. In order to enhance detection sensitivity, a poly(G:C) terminus may be attached to each primer.

Another method for determining whether the DNA of the present invention has a mutation causative of arteriosclerosis is a protein truncation test (PTT) [Genomics, 20, 1-4(1994)]. This test can specifically detect a frame shift mutation, splice site mutation or nonsense mutation which develops a deletion in protein. In PTT, a special primer is designed by linking a T7 promoter sequence and a eucaryotic translation initiation sequence to the 5'-terminus of the DNA of the present invention. Using this primer, cDNA is prepared from specimen-derived RNA according to the reverse-transcribed PCR (RT-PCR) technique. When this cDNA is reacted in an in vitro transcription/translation system, it is transcribed into mRNA by the action of T7 promoter and translated by the action of the translation initiation sequence, so that a protein is produced. When this protein is electrophoresed through a gel, there will be no mutation that develops a deletion if the position of the migrated protein corresponds to that of the full-length protein. If the protein has a deletion, it will be migrated over a shorter distance than the full-length protein. Thus, the degree of deletion can be estimated from that position.

In order to determine the nucleotide sequences of the specimen-derived DNA and the specimen-derived cDNA, it is possible to use primers designed on the basis of the nucleotide sequence of the DNA of the present invention. An analysis of the determined nucleotide sequences makes it possible to judge whether or not the specimen-derived DNA or the specimen-derived cDNA has a mutation causative of arteriosclerosis.

A mutation outside the coding region of a shear stress-responsive gene can be detected by testing non-coding regions such as introns and control sequences near or within the gene. An arteriosclerotic disease caused by a mutation in a non-coding region can be ascertained by comparing the test specimen with a control specimen according to the above-described method and detecting an abnormal size, or abnormal production, of mRNA in the arteriosclerotic patient.

For the gene suggesting the presence of a mutation in a non-coding region, the DNA of the non-coding region can be cloned by using the DNA of the present invention as a probe for hybridization. A mutation in a non-coding region may be searched according to any of the above-described methods.

By subjecting to a statistical analysis according to the method described in Handbook of Human Genetics Linkage, The John Hopkins University Press, Baltimore (1994), the mutations so found may be identified as single nucleotide polymorphisms (SNPs) having a linkage with arteriosclerosis. Moreover, a gene causative of arteriosclerosis may be identified by obtaining DNA from a family having a history of arteriosclerosis according to the previously described method, and detecting a mutation therefrom.

Now, the method for diagnosing vascular diseases caused by arteriosclerosis using the shear stress-responsive DNA of the present invention is described below.

The DNAs which can be used in this method include, for example, DNAs having the nucleotide sequences represented by SEQ ID NO:1,3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 116, 117, 119, 121, 123, 125, 127, 129, 130, 131, 132, 133, 134, 135, 137, 139, 141, 143, 145, 147, 149, 151, 153, 155, 157, 168, 170, 172 and the like. Moreover, they include a DNA having a sequence of part of the foregoing DNA, an oligonucleotide DNA having the nucleotide sequence of 5 to 60 consecutive bases in the foregoing DNA, and preferably an oligonucleotide DNA having the nucleotide sequence of 10 to 40 consecutive bases therein. Furthermore, they include a shear stress-responsive DNA capable of hybridizing with the foregoing DNA or a fragment thereof under stringent conditions.

The cause of arteriosclerosis can be ascertained by detecting a gene mutation in any tissue of a human subject. For example, where a mutation is present in the germ cell system, an individual having inherited the mutation may tend to develop arteriosclerosis. The mutation can be identified by testing DNA obtained from any tissue of the body of the individual. For example, a diagnosis of arteriosclerosis can be made by collecting blood from a subject, extracting DNA from cells of the blood, and using this DNA to perform a test for a gene mutation. Moreover, a prenatal diagnosis may be made by using fetal cells, placental cells or amniotic cells to perform a test for a gene mutation.

Furthermore, by obtaining a biological tissue from the lesion of a patient having developed a vascular disease and testing its DNA, the type of the vascular disease can be diagnosed and the results thus obtained can be utilized to select a drug to be administered. In order to detect a mutation of a gene in the tissue, it is useful to isolate the lesional tissue segregated from the surrounding normal tissues. An arteriosclerotic lesion may be obtained, for example, by a bypass operation for replacing the lesion of arteriosclerosis with a normal blood vessel. The tissue thus obtained is treated with trypsin or the like, and the resulting cells are cultured in a suitable culture medium. Then, chromosomal DNA and mRNA can be extracted from the cultured cells.

The DNA obtained from a human specimen for diagnostic purposes according to any of the aforesaid methods is hereinafter referred to as the diagnostic specimen-derived DNA. Moreover, the cDNA synthesized from the RNA obtained from a human specimen for diagnostic purposes according to any of the aforesaid methods is hereinafter referred to as the diagnostic specimen-derived cDNA.

Using the shear stress-responsive DNA of the present invention and the diagnostic specimen-derived DNA or the diagnostic specimen-derived cDNA, a diagnosis of arteriosclerosis may be made according to the above-described method for detecting a gene causative of arteriosclerosis.

Moreover, in order to make a diagnosis of arteriosclerosis by using the shear stress-responsive DNA of the present invention and the diagnostic specimen-derived DNA or the diagnostic specimen-derived cDNA, there may also be employed a method such as (1) the detection of a restriction enzyme site, (2) the utilization of an allele-specific oligonucleotide probe [allele-specific oligonucleotide hybridization (ASO)], (3) PCR using an allele-specific oligonucleotide [amplification refractory mutation system (ARMS)], (4) oligonucleotide ligation assay (OLA), (5) PCR-preferential homoduplex formation assay (PCR-PHFA), or (6) oligo-DNA array [Protein-Nucleic Acid-Enzyme (in Japanese), 43, 2004-2011(1998)].

Where a restriction enzyme site disappears or appears as a result of a single base change, the mutation may be easily detected by amplifying the diagnostic specimen-derived DNA or the diagnostic specimen-derived cDNA with primers designed on the basis of the sequence possessed by the DNA of the present invention, digesting it with the restriction enzyme, and comparing the resulting restriction enzyme-cleaved DNA fragments with those obtained from healthy subjects. However, the occurrence of such a mutation is rare. For diagnostic purposes, a mismatch exerting no influence on annealing is introduced into PCR primers designed on the basis of the sequence possessed by the DNA of the present invention. Thus, for a mutation not accompanied by the disappearance or appearance of a restriction enzyme site, a restriction enzyme site is artificially introduced.

A short synthetic DNA probe hybridizes only with a perfectly base pairing sequence alone. Taking advantage of this characteristic, a single-base mutation can be easily detected by preparing an allele-specific oligonucleotide probe (ASO). For diagnostic purposes, reverse dot blotting is often employed in which an oligonucleotide designed on the basis of the sequence possessed by the DNA of the present invention and an identified mutation is attached to a filter and hybridization is carried out with a probe prepared from the diagnostic specimen-derived DNA or the diagnostic specimen-derived cDNA by PCR using primers designed on the basis of the sequence possessed by the DNA of the present invention and labeled dNTP. In the DNA chip method, an oligonucleotide designed on the basis of the sequence possessed by the DNA of the present invention and the mutation is synthesized directly on a substrate (e.g., slide glass or silicon) to form a highly dense array. This DNA chip method is a mutation detection method suitable for large-scale diagnostic purposes because various mutations can be more conveniently detected by using a small amount of the diagnostic specimen-derived DNA or the diagnostic specimen-derived cDNA.

Nucleotide mutations can also be detected by the following oligonucleotide ligation assay (OLA).

Two oligonucleotides consisting of about 20 bases, which are designed from the sequence possessed by the DNA of the present invention and are capable of hybridizing on both sides of a mutation site, are prepared. Using a template comprising the diagnostic specimen-derived DNA or the diagnostic specimen-derived cDNA and primers designed from the sequence possessed by the DNA of the present invention, shear stress-responsive DNA fragment is amplified by PCR. The amplified fragment is hybridized with the aforesaid polynucleotide. After hybridization, the two oligonucleotides are ligated by means of DNA ligase. For example, by labeling one oligonucleotide with biotin and the other oligonucleotide with a different label such as digoxigenin, it is possible to detect rapidly whether the ligation has occurred or not. OLA is a mutation detection method suitable for large-scale diagnostic purposes because it does not require electrophoresis or centrifugation.

A very small amount of a mutated gene can also be quantitatively and easily detected by the following PCR-PHFA.

PCR-PHFA is a combination of three techniques including gene amplification (PCR), liquid-phase hybridization exhibiting very high specificity, and enzymatic detection of PCR product (ED-PCR) detecting the PCR product in the same manner as ELISA. Using a dinitrophenyl(DNP)-labeled and biotin-labeled primer set, an amplification product labeled at both ends is prepared by carrying out PCR amplification while using the DNA of the present invention as a template. This amplification product is mixed with a large excess (20- to 100-fold) of an unlabeled amplification product obtained by using an unlabeled primer set having the same sequences and a template comprising the diagnostic specimen-derived DNA or the diagnostic specimen-derived cDNA. After thermal denaturation, this mixture is cooled with a gentle temperature gradient of the order of 1°C/5-10 minutes to form perfect complementary strands preferentially. The labeled DNA so reformed is captured and adsorbed to a streptavidin-immobilized well via biotin. On the other hand, an enzyme-labeled anti-DNP antibody is bound thereto via DNP. Thus, the labeled DNA can be detected by a color-developing reaction based on the enzyme. If a gene having the same sequence as the labeled DNA is not present in the specimen, the original double-strand labeled DNA is preferentially reformed to develop a color. In contrast, if a gene having the same sequence is present in the specimen, complementary strands are randomly replaced to decrease the reformation of the labeled DNA, resulting in a marked reduction in color development. Thus, known mutated polymorphic genes can be detected and quantitatively determined.

Now, the methods for detection and quantitative determination of the shear stress-responsive protein of the present invention immunologically using the antibody of the present invention are described below.

The methods by which a microorganism, an animal cell or an insect cell, or a tissue, expressing the protein of the present invention intracellularly or extracellularly can be immunologically detected and determined quantitatively using the antibody (polyclonal antibody or monoclonal antibody) of the present invention include fluorescent antibody technique, enzyme-linked immunosorbent assay (ELISA), radioimmunoassay (RIA), immunohistochemical staining techniques (e.g., ABC method and CSA method) such as tissue immunostaining and cell immunostaining, western blotting, dot blotting, immunoprecipitation, sandwich ELISA [Experimental Manual for Monoclonal Antibodies (in Japanese), Kodansha Scientific (1987); Biochemical Experimental Lectures (Second Series) 5, Methods of Immunobiochemical Research (in Japanese), Tokyo-Kagaku-Dojin (1986)], and the like.

The fluorescent antibody technique is a technique in which, after a microorganism, an animal cell or an insect cell, or a tissue, expressing the protein of the present invention intracellularly or extracellularly is reacted with the antibody of the present invention and further reacted with an anti-mouse IgG antibody, or a fragment thereof, labeled with a fluorescent material such as fluorescein isothiocyanate (FITC), fluorescence is measured with a flow cytometer.

Enzyme-linked immunosorbent assay (ELISA) is a technique in which, after a microorganism, an animal cell or an insect cell expressing the protein of the present invention intracellularly or extracellularly is reacted with the antibody of the present invention and further reacted with an anti-mouse IgG antibody, or a binding fragment thereof, labeled with an enzyme such as peroxidase or biotin, the developed color is measured with a spectrophotometer.

Radioimmunoassay (RIA) is a technique in which, after a microorganism, an animal cell or an insect cell, or a tissue, expressing the protein of the present invention intracellularly or extracellularly is reacted with the antibody of the present invention and further reacted with an anti-mouse IgG antibody, or a fragment thereof, labeled with a radioactive substance, radioactivity is measured with a scintillation counter or the like.

Cell immunostaining and tissue immunostaining are in which, after a microorganism, an animal cell or an insect cell, or a tissue, expressing the protein of the present invention intracellularly or extracellularly is reacted with the antibody of the present invention and further reacted with an anti-mouse IgG antibody, or a fragment thereof, labeled with a fluorescent material (e.g., FITC) or an enzyme (e.g., peroxidase or biotin), it is observed under the microscope.

Western blotting is a technique in which, after an extract of a microorganism, an animal cell or an insect cell, or a tissue, expressing the protein of the present invention intracellularly or extracellularly is fractionated by SDS-polyacrylamide gel electrophoresis [Antibodies-A Laboratory Manual, Cold Spring Harbor Laboratory (1988)], this gel is blotted to a PVDF membrane or a nitrocellulose membrane, then the membrane is reacted with the antibody of the present invention and further reacted with an anti-mouse IgG antibody, or a fragment thereof, labeled with a fluorescent material (e.g., FITC) or an enzyme (e.g., peroxidase or biotin). Thus, the protein of the present invention can be detected.

Dot blotting is a technique in which, after an extract of a microorganism, an animal cell or an insect cell, or a tissue, expressing the protein of the present invention intracellularly or extracellularly is blotted to a nitrocellulose membrane, this membrane is reacted with the antibody of the present invention and further reacted with an anti-mouse IgG antibody, or a binding fragment thereof, labeled with a fluorescent material (e.g., FITC) or an enzyme (e.g., peroxidase or biotin). Thus, the protein of the present invention can be detected.

Immunoprecipitation is a technique in which, after an extract of a microorganism, an animal cell or an insect cell, or a tissue, expressing the protein of the present invention intracellularly or extracellularly is extracted, the resulting extract is reacted with the antibody of the present invention, a carrier having the ability to bind specifically to immunoglobulin (e.g., protein G-Sepharose) is added thereto so as to precipitate the resulting antigen-antibody complex.

In sandwich ELISA, two antibodies of the present invention having different antigen recognition sites are provided. In advance, one of the antibodies is adsorbed to a plate, and the other is labeled with a fluorescent material (e.g., FITC) or an enzyme (e.g., peroxidase or biotin). After an extract of a microorganism, an animal cell or an insect cell, or a tissue, expressing the protein of the present invention intracellularly or extracellularly is extracted, the resulting extract is reacted with the antibody-adsorbed plate, the plate is reacted with the labeled antibody and subjected to a reaction depending on the labeling material.

Now, the method for diagnosing vascular diseases caused by arteriosclerosis using the antibody of the present invention is described below.

The identification of an alteration of the expression level of a shear stress-responsive protein and a structural change of the expressed protein in human biological specimens and human primary cultured cells is useful in knowing the risk of developing arteriosclerosis in the future or the cause of an already developed vascular disease.

The methods which can be employed to make a diagnosis by detecting the expression level of a shear stress-responsive protein and a structural change thereof include the above-described fluorescent antibody technique, enzyme-linked immunosorbent assay (ELISA), radioimmunoassay (RIA), immunohistochemical staining techniques (e.g., ABC method and CSA method) such as tissue immunostaining and cell immunostaining, western blotting, dot blotting, immunoprecipitation, sandwich ELISA and the like.

The materials which can be diagnosed by the aforesaid methods include biological specimens themselves (e.g., blood vessels in the lesion, blood, serum, urine, feces and saliva) collected from human subjects, and cells or cell extracts obtained from the foregoing biological specimens. Alternatively, an isolated paraffin or cryostat section of a tissue obtained from biological specimens may also be used.

Now, the methods for screening a therapeutic agent for vascular diseases caused by arteriosclerosis using the shear stress-responsive DNA of the present invention, a protein encoded by the DNA, or an antibody capable of recognizing the protein are described below.

The DNAs which can be used in these screening methods include, for example, DNAs having the nucleotide sequences represented by SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 116, 117, 119, 121, 123, 125, 127, 129, 130, 131, 132, 133, 134, 135, 137, 139, 141, 143, 145, 147, 149, 151, 153, 155, 157, 168, 170, 172 and the like; and a shear stress-responsive DNA capable of hybridizing with the foregoing DNA or a fragment thereof under stringent conditions. The protein which can be used therein include a protein encoded by the foregoing DNA (e.g., a protein having an amino acid sequence selected from the amino acid sequences represented by SEQ ID NO:144, 146, 148, 150, 152, 154, 156, 158, 169, 171 and 173); and a protein comprising an amino acid sequence in which one or more amino acids are deleted, replaced or added as compared with the amino acid sequence possessed by the foregoing protein, and having an activity involved in the formation of an arteriosclerotic lesion. The antibody which can be used therein include an antibody capable of recognizing the foregoing protein.

A microorganism, an animal cell or an insect cell transformed by introducing the DNA of the present invention so as to produce the protein of the present invention or a partial polypeptide of the protein, and the protein or polypeptide in purified form, are useful for the purpose of screening an agent acting specifically on a shear stress-responsive protein. The agent obtained by this screening is useful for the treatment of vascular diseases caused by arteriosclerosis.

One of the aforesaid screening methods comprises selecting a labeled compound binding specifically to a microorganism, an animal cell or an insect cell transformed so as to produce the protein of the present invention or a partial polypeptide of the protein (hereinafter referred to as the transformant for screening). The specific binding of a labeled compound may be detected by comparison with a control comprising an untransformed microorganism, animal cell or insect cell. Alternatively, an unlabeled compound may be selected by competitive screening in which its inhibitory effect on the binding to the transformant for screening of a compound or protein binding specifically to the transformant for screening is used as an index.

The purified protein of the present invention or the purified partial polypeptide of the protein may be used to select a labeled compound binding specifically to a shear stress-responsive protein. The binding of the labeled compound may be determined quantitatively according to the above-described immunological method using the antibody of the present invention. Alternatively, an unlabeled compound may be selected by competitive screening in which its inhibitory effect on the binding to the protein or polypeptide of a labeled compound binding to the protein or polypeptide is used as an index.

In the other of the aforesaid screening methods, a large number of partial peptides of the protein are densely synthesized on plastic pins or a certain solid support. Thus, a compound or protein binding selectively to the peptides can be efficiently screened (WO 84/03564).

An expression regulating agent capable of regulating the expression of a shear stress-responsive mRNA or protein in vascular endothelial cells is also useful for the treatment of vascular diseases caused by arteriosclerosis.

An agent for regulating the transcription or translation of a shear stress-responsive gene can be screened by adding various compounds to a vascular endothelial cell line and assaying an increase or decrease in the expression of a shear stress-responsive mRNA using the DNA of the present invention. An increase or decrease in the expression of a shear stress-responsive mRNA may be detected by the above-described PCR, Northern blotting, and RNase protection assay.

An agent for regulating the transcription or translation of a shear stress-responsive gene can also be screened by adding various compounds to a vascular endothelial cell line and assaying an increase or decrease in the expression of a shear stress-responsive protein using the antibody of the present invention. An increase or decrease in the expression of a shear stress-responsive protein may be detected by the above-described fluorescent antibody technique, enzyme-linked immunosorbent assay (ELISA), radioimmunoassay (RIA), immunohistochemical staining techniques (e.g., ABC method and CSA method) such as tissue immunostaining and cell immunostaining, western blotting, dot blotting, immunoprecipitation, and sandwich ELISA.

The compound obtained by the aforesaid methods may be administered, as an agent, to model animals for arteriosclerosis, such as ApoE knockout mice and rabbits fed with a high cholesterol diet. By measuring the incorporation of oxidized LDL and cholesterol into the vascular endothelium and the formation of an arteriosclerotic lesion in these animals, the therapeutic effect of the compound on the vascular disease caused by arteriosclerosis can be evaluated.

Now, the drug delivery method using the antibody of the present invention is described below.

The antibody used for this drug delivery may be any antibody in accordance with the present invention. However, it is particularly desirable to use a humanized antibody.

Usable humanized antibodies include a human chimeric antibody, a complementary determining region (hereinafter referred to as CDR) grafted humanized antibody, and the like.

The human chimeric antibody means an antibody consisting of a heavy-chain variable region (the heavy chain may hereinafter be referred to as H chain, the variable region as V region, and the heavy-chain variable region as HV or VH) and a light-chain variable region (the light chain may hereinafter be referred to as L chain, and the light-chain variable region as LV or VL) of an antibody of an animal other than human, and a heavy-chain constant region (the constant region may hereinafter be referred to as C region, and the heavy-chain constant region as CH) and a light-chain constant region (the light-chain constant region may hereinafter be referred to as CL) of a human antibody. As the animal other than human, there may be used any of various animals that permit the generation of hybridomas, such as mice, rats, hamsters and rabbits.

The human chimeric antibody of the present invention may be produced by obtaining cDNAs encoding VH and VL from a hybridoma capable of producing a monoclonal antibody which binds to the protein of the present invention and neutralizes the action of the protein of the present invention; inserting them into an expression vector for mammalian cells having genes encoding human antibody CH and human antibody CL, respectively, to construct a human chimeric antibody expression vector; and introducing the vector into mammalian cells to express the antibody.

The CH of the human chimeric antibody may be any CH belonging to human immunoglobulin (hereinafter abbreviated as hIg). However, the CH of hIgG class is preferred. Furthermore, there may be used any of various subclasses (e.g., hIgG1, hIgG2, hIgG3 and hIgG4) belonging to hIgG class. The CL of the human chimeric antibody may be any CL belonging to hIg, and the CL of κ or λ class may be used.

The CDR-grafted humanized antibody means an antibody in which the amino acid sequences of CDRs of VH and VL of an antibody of an animal other than human are transplanted into appropriate positions of VH and VL of a human antibody.

The CDR-grafted humanized antibody of the present invention may be produced by constructing cDNAs encoding V regions in which the CDR sequences of VH and VL of any human antibody are replaced by the CDR sequences of VH and VL, respectively, of an antibody of an animal other than human that reacts with the protein of the present invention, binds to the protein of the present invention, and neutralizes the action of the protein of the present invention; inserting them into an expression vector for mammalian cells having genes encoding human antibody CH and human antibody CL, respectively, to construct a CDR-grafted humanized antibody expression vector; and introducing the vector into animal cells to express the antibody.

The CH of the CDR-grafted humanized antibody may be any CH belonging to hIg. However, the CH of hIgG class is preferred. Furthermore, there may be used any of various subclasses (e.g., hIgG1, hIgG2, hIgG3 and hIgG4) belonging to hIgG class. The CL of the CDR-grafted humanized antibody may be any CL belonging to hIg, and the CL of κ or λ class may be used.

Originally, human antibodies mean antibodies existing naturally in the human body. However, they also include antibodies obtained from a human antibody phage library and a human antibody-producing transgenic animal which have been created on the basis of the recent progress of genetic engineering, cell technology and embryological engineering.

An antibody existing in the human body may be obtained, for example, according to the following method.

Lymphocytes are isolated from human peripheral blood, immortalized by infection with EB virus or the like, and then cloned. After the selected lymphocyte producing a desired antibody is cultured, the antibody can be obtained from the resulting culture.

The human antibody phage library is a library in which an antibody gene prepared from human B cells is inserted into a phage gene so as to express antibody fragments (e.g., Fab and single-chain antibody) on the phage surface. From this library, a phage expressing an antibody fragment having a desired antigen-binding activity can be recovered by using its binding activity for a substrate having the antigen immobilized thereon as an index. This antibody fragment can further be converted into a complete human antibody according to genetic engineering techniques.

The human antibody-producing transgenic animal means an animal in which a human antibody gene is introduced into the cells. Specifically, a human antibody-producing transgenic animal may be created by introducing a human antibody gene into a mouse ES cell, transplanting the ES cell into an early embryo of another mouse, and developing the embryo. In order to prepare a human antibody from the human antibody-producing transgenic animal, there may be employed a method which comprises obtaining a human antibody-producing hybridoma according to the common method for the formation of hybridomas in mammals other than human, and culturing the hybridoma to produce and accumulate the human antibody in the resulting culture.

The antibody fragments include Fab, Fab', F(ab')₂, single-chain antibody, dsFv, CDR-containing peptides, and the like.

Among the fragments obtained by treating IgG with the proteolytic enzyme papain (IgG is cleaved at the 224th amino acid residue of each H chain), Fab is an antibody fragment with a molecular weight of about 50,000 which has an antigen-binding activity and consists of about a half of an H chain on the N-terminal side and a whole L chain which are linked together via a disulfide bond.

The Fab of the present invention may be obtained from an antibody reacting specifically with the protein of the present invention, by treating it with the proteolytic enzyme papain. Alternatively, Fab may also be obtained by inserting DNA encoding the Fab of the antibody into a procaryotic expression vector or a eucaryotic expression vector, and introducing the resulting vector into a procaryote or eucaryote to express the DNA.

Among the fragments obtained by treating IgG with the proteolytic enzyme pepsin (IgG is cleaved at the 234th amino acid residue of each H chain), F(ab')₂ is an antibody fragment with a molecular weight of about 100,000 which has an antigen-binding activity and is slightly larger than two Fab molecules linked together via disulfide bonds in the hinge area.

The F(ab')₂ of the present invention may be obtained from an antibody reacting specifically with the protein of the present invention, by treating it with the proteolytic enzyme pepsin. Alternatively, F(ab')₂ may also be obtained by inserting DNA encoding the F(ab')₂ of the antibody into a procaryotic expression vector or an eucaryotic expression vector, and introducing the resulting vector into a procaryote or eucaryote to express the DNA.

Fab' is an antibody fragment with a molecular weight of about 50,000 which has an antigen-binding activity and is obtained by breaking the disulfide bonds in the hinge area of the aforesaid F(ab')₂.

The Fab' of the present invention may be obtained from an antibody reacting specifically with the protein of the present invention, by treating it with the reducing agent dithiothreitol. Alternatively, Fab' may also be obtained by inserting DNA encoding the Fab' fragment of the antibody into a procaryotic expression vector or an eucaryotic expression vector, and introducing the resulting vector into a procaryote or eucaryote to express the DNA.

A single-chain antibody (hereinafter also referred to as scFv) is a VH-P-VL or VL-P-VH polypeptide consisting of one VH and one VL linked together by a suitable peptide linker (hereinafter referred to as P). The VH and VL contained in the scFv used in the present invention may be those of any antibody (e.g., humanized antibody or human antibody) reacting specifically with the protein of the present invention.

The single-chain antibody of the present invention may be obtained according to the following method.

After cDNAs encoding the VH and VL of an antibody reacting specifically with the protein of the present invention are obtained, a DNA encoding the single-chain antibody is constructed. Then, the single-chain antibody may be obtained by inserting the DNA into a procaryotic expression vector or an eucaryotic expression vector, and introducing the resulting vector into a procaryote or eucaryote to express the DNA.

The disulfide-stabilized V region fragment (hereinafter referred to as dsFv) is a fragment obtained by replacing one amino acid residue of each of VH and VL with a cysteine residue and linking these polypeptides together by a disulfide bond extending between the cysteine residues. The amino acid residues to be replaced with cysteine residues can be selected on the basis of the predicted stereostructure of the antibody, according to the method shown by Reiter et al. [Protein Engineering, 7, 697(1994)]. The VH and VL contained in the disulfide-stabilized V region fragment used in the present invention may be those of any antibody (e.g., humanized antibody or human antibody) reacting specifically with the protein of the present invention.

The disulfide-stabilized V region fragment of the present invention may be obtained according to the following method.

After cDNAs encoding the VH and VL of an antibody reacting specifically with the protein of the present invention are obtained, a DNA encoding the disulfide-stabilized V region fragment is constructed. Then, the disulfide-stabilized V region fragment may be obtained by inserting the DNA into a procaryotic expression vector or an eucaryotic expression vector, and introducing the resulting vector into a procaryote or eucaryote to express the DNA.

The CDR-containing peptides may be prepared by chemical synthesis processes such as the Fmoc method and the tBoc method.

A conjugated antibody prepared from the antibody of the present invention as described below may be used as a drug delivery system to deliver an agent or protein specifically to a lesion of arteriosclerosis.

The conjugated antibody is an antibody obtained by linking a radioactive isotope, protein, low-molecular-weight agent or the like to an antibody reacting specifically with the protein of the present invention (e.g., a humanized antibody, a human antibody, or a fragment of these antibodies) by chemical means or genetic engineering means.

The conjugated antibody of the present invention may be prepared by linking a radioactive isotope, protein, low-molecular-weight agent or the like to N-terminal or C-terminal side of an H chain or L chain of an antibody or antibody fragment reacting specifically with the protein of the present invention, to a suitable substituent group or side chain of the antibody or antibody fragment, or to a sugar chain of the antibody or antibody fragment, by chemical means or genetic engineering means.

Usable radioactive isotopes include ¹³¹I, ¹²⁵I and the like. They can be linked to an antibody or an antibody fragment, for example, according to the chloramine T method.

Usable low-molecular-weight agents are anticancer drugs including, for example, alkylating agents such as nitrogen mustard and cyclophosphamide; antimetabolites such as 5-fluorouracil and methotrexate; antibiotics such as daunomycin, bleomycin, mitomycin C, daunorubicin and doxorubicin; plant alkaloids such as vincristine, vinblastine and vindesine; and hormones such as tamoxifen and dexamethasone [Clinical Oncology (in Japanese), edited by the Japanese Society for the Research of Clinical Oncology, 1996, Gan-to-Kagakuryoho Sha]; anti-inflammatory drugs including, for example, steroids such as hydrocortisone and prednisone; nonsteroidal anti-inflammatory drugs such as aspirin and indomethacin; immunomodulators such as gold sodium thiomalate and penicillamine; immunosuppressants such as cyclophosphamide and azathioprine; and antihistamines such as chlorpheniramine maleate and clemastine [Inflammation and Anti-inflammatory Therapy (in Japanese), 1982, Ishiyaku Shuppan Kabushiki Kaisha]; and the like.

A low-molecular-weight agent may be linked to the aforesaid antibody in the usual manner. For example, daunomycin may be linked to the antibody, for example, by linking daunomycin to an amino group of the antibody via glutaraldehyde, or by linking the amino group of daunomycin to a carboxyl group of the antibody via water-soluble carbodiimide.

Suitable proteins include cytokines activating immunocompetent cells, and growth controlling factors for vascular endothelium, vascular smooth muscle and the like. Examples thereof include human interleukin 2, human granulocyte macrophage colony-stimulating factor, human macrophage colony-stimulating factor, human interleukin 12, fibroblast growth factor 2 (FGF-2) and platelet-derived growth factor (PDGF). Moreover, in order to damage directly with proliferative vascular smooth muscle cells of an arteriosclerotic lesion, there may be used a toxin such as ricin or diphtheria toxin.

The conjugated antibody having a protein linked thereto may be prepared according to the following method.

A DNA encoding the conjugated antibody is constructed by linking cDNA encoding the protein to cDNA encoding the antibody or antibody fragment. After this DNA is inserted into a prokaryotic or eucaryotic expression vector, the resulting expression vector is introduced into a procaryote or eucaryote to express the DNA. Thus, the desired conjugated antibody can be obtained.

Now, the method of gene therapy using a virus vector containing the shear stress-responsive DNA of the present invention is described below.

A therapeutic agent may be prepared from the above-described recombinant virus vector and a base for gene therapeutic agents [Nature Genet., 8, 42(1994)].

The base for gene therapeutic agents may be any base that is commonly used for injections. Examples thereof include distilled water; a solution of a salt such as sodium chloride or a mixture of sodium chloride and an inorganic salt; a solution of mannitol, lactose, dextran, glucose or the like; a solution of an amino acid such as glycine or arginine; and a mixture of an organic acid solution or a salt solution and a glucose solution. Moreover, injections in the form of solutions, suspensions or dispersions may be prepared in the usual manner, by using auxiliaries such as osmotic pressure regulators, pH regulators, vegetable oils (e.g., sesame oil and soybean oil) and surfactants (e.g., lecithin and nonionic surfactants), in combination with the aforesaid bases. These injections may also be prepared as preparations to be dissolved at the time of use, according to a technique such as powdering or freeze-drying. Where the gene therapeutic agent of the present invention is a liquid, it may be used directly for therapeutic purposes, as required. Where it is a solid, it may be dissolved immediately before gene therapy in the aforesaid base having been sterilized as required, and used for therapeutic purposes. In order to administer the gene therapeutic agent of the present invention, there may be employed a local administration method using a double balloon catheter or the like so that the gene therapeutic agent will be absorbed into the vascular endothelium of the treated site of the patient.

As a method for carrying a virus vector more specifically to an arteriosclerotic lesion, Somia et al. have reported a method using a fusion protein consisting of a single-chain antibody capable of recognizing specifically the LDL receptor and the Env protein of a retrovirus vector [Proc. Natl. Acad. Sci. USA, 92, 7570-7574(1995)]. This system is not limited to retrovirus vectors, but may also be applied to lentivirus vectors and the like.

The nonviral gene transfer techniques which are known in this field include calcium phosphate coprecipitation [Virology, 52, 456-467(1973); Science, 209, 1414-1422(1980)], microinjection [Proc. Natl. Acad. Sci. USA, 77, 5399-5403(1980); Proc. Natl. Acad. Sci. USA, 77, 7380-7384(1980); Cell, 27, 223-231(1981); Nature, 294, 92-94(1981)], membrane fusion-mediated transfer method using liposomes [Proc. Natl. Acad. Sci. USA, 84, 7413-7417(1987); Biochemistry, 28, 9508-9514(1989); J. Biol. Chem., 264, 12126-12129(1989); Hum. Gene Ther., 3, 267-275(1992); Science, 249, 1285-1288(1990); Circulation, 83, 2007-2011(1992)], direct DNA incorporation and receptor-mediated DNA transfer method [Science, 247, 1465-1468(1990); J. Biol. Chem., 266, 14338-14342(1991); Proc. Natl. Acad. Sci. USA, 87, 3655-3659(1991); J. Biol. Chem., 264, 16985-16987(1989); BioTechniques, 11, 474-485(1991); Proc. Natl. Acad. Sci. USA, 87, 3410-3414(1990); Proc. Natl. Acad. Sci. USA, 88, 4255-4259(1991); Proc. Natl. Acad. Sci. USA, 87, 4033-4037(1990); Proc. Natl. Acad. Sci. USA, 88, 8850-8854(1991); Hum. Gene Ther., 3, 147-154(1991)], and the like.

When gene transfer using a virus vector is combined with direct in vivo gene transfer using liposome delivery, the virus vector can be directed to an arteriosclerotic lesion.

In addition, a virus vector may also be prepared by combining a DNA of appropriate size in accordance with the present invention with a polylysine-conjugated antibody specific for adenovirus hexon protein to form a complex, and linking the resulting complex to an adenovirus vector. This virus vector attains target cells stably, is incorporated into the cells by the action of endosomes, and is decomposed in the cells. Thus, the gene can be expressed efficiently.

In an investigation on tumors, it has been reported that membrane fusion-mediated transfer method using liposomes permits a liposome preparation to be administered directly to a target tissue, and the tissue can hence incorporate and express the gene locally [Hum. Gene Ther., 3, 399-410(1992)]. Accordingly, it may be expected that a similar effect is produced in the case of an arteriosclerotic lesion. In order to deliver DNA directly to an arteriosclerotic lesion, it is preferable to employ a gene transfer technique. Receptor-mediated DNA transfer is carried out, for example, by conjugating DNA (usually taking the form of a covalently closed supercoiled plasmid) to a protein ligand via polylysine. The ligand is selected on the basis of the presence of the corresponding ligand receptor on the cell surface of the target cells or tissue. Examples of the combination of the receptor and the ligand include the combination of LDL receptor and LDL, and the combination of scavenger receptor and oxidized LDL. If desired, this ligand-DNA conjugate may be directly injected into the blood and thereby delivered to a target tissue where it binds to the receptor and the DNA-protein complex is internalized. In order to prevent the intracellular degradation of DNA, the target tissue may be simultaneously infected with an adenovirus to disrupt the endosome function.

Now, the method of treatment using an antibody capable of recognizing specifically the shear stress-responsive DNA of the present invention is described below.

A pharmaceutical containing the antibody of the present invention may be administered alone as a therapeutic agent. However, it is usually desirable to provide as pharmaceutical preparations produced by blending the antibody with one or more pharmacologically acceptable carriers and working up the resulting blend according to any of various techniques known well in the technical field of pharmaceutics.

It is desirable to use the route of administration which is most effective for the purpose of treatment. Examples thereof include oral administration and parenteral administration such as buccal, intratracheal, intrarectal, subcutaneous, intramuscular and intravenous administration. In the case of antibody preparations, intravenous administration is desirable.

Examples of dosage forms include sprays, capsules, tablets, granules, medicated syrups, emulsions, suppositories, injections, ointments, tapes, and the like.

Pharmaceutical preparations suitable for oral administration include emulsions, medicated syrups, capsules, tablets, powders, granules and the like.

Liquid preparations such as emulsions and medicated syrups may be produced using additives including water; sugars such as sucrose, sorbitol and fructose; glycols such as polyethylene glycol and propylene glycol; oils such as sesame oil, olive oil and soybean oil; antiseptics such as p-hydroxybenzoic acid esters; flavors such as strawberry flavor and peppermint; and the like.

Capsules, tablets, powders, granules and the like may be produced using additives including excipients such as lactose, glucose, sucrose and mannitol; disintegrators such as starch and sodium alginate; lubricants such as magnesium stearate and talc; binders such as polyvinyl alcohol, hydroxypropylcellulose and gelatin; surfactants such as fatty acid esters; plasticizers such as glycerin; and the like.

Pharmaceutical preparations suitable for parenteral administration include injection, suppository, spray and the like. Injection is prepared using a carrier comprising a salt solution, a glucose solution or a mixture thereof, and the like. Alternatively, a powder for injection may be prepared by freeze-drying the antibody of the present invention in the usual manner and adding sodium chloride thereto. Suppository is prepared using a carrier such as cacao butter, hydrogenated fats and carboxylic acids.

Spray is prepared by using the antibody of the present invention itself, or using a carrier which does not irritate the mucous membranes of the oral cavity and respiratory tract of the patient and enables the antibody of the present invention to be dispersed as fine particles and easily absorbed, and the like.

Specific examples of the carrier include lactose and glycerin. Depending on the antibody of the present invention and the nature of the carrier used, aerosols, dry powders and the like may be prepared. Also in these parenteral preparations, the ingredients described as additive in connection with oral preparations may be added.

The dosage and the frequency of administration may vary according to the desired therapeutic effect, the method of administration, the period of treatment, the age and body weight of the patient, and the like. However, the drugs of the present invention are usually administered to adults in a daily dose of 10 µg/kg to 20 mg/kg.

One of the activities involved in an arteriosclerotic lesion (i.e. the activities regulating the development of arteriosclerosis) is the promotion or suppression of the apoptosis of vascular endothelial cells. Since it is known that, in vascular endothelial cells, the application of a shear stress tends to suppress the apoptosis of endothelial cells, the shear stress-responsive DNA of the present invention is considered to contain a gene and protein which exhibits a shear stress-dependent increase of expression in vascular endothelial cells and has an apoptosis-suppressing activity. Accordingly, by using this DNA containing a gene having an apoptosis-suppressing activity, a protein encoded by the DNA, a recombinant virus vector constructed by inserting the DNA into a vector, an antibody against the protein encoded by the DNA, and the like, the following applications can be made: (1) identification of the apoptosis sensitivity of cells, (2) regulation of the apoptosis of cells, and (3) screening of an agent for regulating the apoptosis of cells. These applications (1), (2) and (3) are described below in greater detail.

### (1) Identification of the apoptosis sensitivity of cells

Now, the method for identifying the apoptosis sensitivity of cells using the shear stress-responsive DNA of the present invention or a protein encoded by the DNA is described below.

Apoptosis sensitivity means the degree of ease with which cells undergo apoptosis in response to an exogenous apoptotic stimulus, i.e. the degree of susceptibility of cells to the influence of an apoptotic stimulus. It is believed that this apoptosis sensitivity is defined according to whether the apoptotic signal in the cells is accompanied by a suppressive or promotive signal. The molecular entity thereof comprises a group of proteins involved in the suppression or promotion of apoptosis (e.g., apoptosis signal transduction molecule), i.e., the so-called apoptosis-related proteins. These apoptosis-related proteins include, for example, a protein encoded by the DNA (A4RS-041) having the nucleotide sequence represented by SEQ ID NO:7 of the present invention, and a protein having the amino acid sequence represented by SEQ ID NO:8.

Hemodynamic physical forces applied to vascular endothelial cells include a shear stress resulting from a flow of blood with fixed directionality (i.e., a laminar flow) and applied in parallel with the direction of the blood flow, and a normal stress caused by a blood pressure and applied perpendicularly to the endothelium. Vascular endothelial cells are always subjected to both forces. Generally, the development of arteriosclerosis is suppressed in regions where the shear stress is greater than the normal stress. Conversely, the development of arteriosclerosis tends to occur in regions where the normal stress is greater than the shear stress. In fact, it has been reported that the apoptosis of vascular endothelial cells is suppressed by a shear stress resulting from a laminar flow. In the culture system (i.e., the micro carrier/spinner flask system) used to obtain the DNAs of the present invention, not only a shear stress due to a flow, but also a normal stress due to a centrifugal force caused by rotation is applied to endothelial cells. Some of the genes responding to a shear stress are modified by a normal stress, and others are not modified thereby. This difference in reactivity can be clarified by confirming the presence or absence of an increase of expression in HUVECs cultured in a parallel plate type culture apparatus or other apparatus which applies only a shear stress thereto. It is believed that at least the group of shear stress-responsive genes not modified by a normal stress act protectively against arteriosclerosis, and this gene group includes a gene and protein having an apoptosis-suppressing activity.

The endogenous transcription level of the DNA of the present invention having an apoptosis-suppressing activity, or the expression level of the protein of the present invention having an apoptosis-suppressing activity, or a structural change of the expressed protein may be detected using the DNA of the present invention having an apoptosis-suppressing activity, a DNA having the same sequence as 5 to 60 consecutive bases in the nucleotide sequence of the DNA, an antibody capable of recognizing the protein of the present invention having an apoptosis-suppressing activity, or the like. Thus, the apoptosis sensitivity of cells can be identified.

Examples of the DNA used in the method for identifying apoptosis sensitivity, and an antibody capable of recognizing a protein encoded by the DNA include a DNA having the nucleotide sequence represented by SEQ ID NO:7, a DNA having the same sequence as 5 to 60 consecutive bases in the nucleotide sequence represented by SEQ ID NO:7, and an antibody capable of recognizing a protein having the amino acid sequence represented by SEQ ID NO:8.

The DNA of the present invention, a DNA having the same sequence as 5 to 60 consecutive bases in the nucleotide sequence of the DNA, and an antibody capable of recognizing the protein of the present invention having an apoptosis-suppressing activity, which are used in the above-described method, are effective as agents for identifying the apoptosis sensitivity of cells.

Since the apoptosis of vascular endothelial cells is promoted in an arteriosclerotic lesion, these agents can also be utilized as diagnostic agents for vascular diseases caused by arteriosclerosis with a view, for example, to identifying the arteriosclerotic lesion or predicting the risk of developing arteriosclerosis in the future.

The agent for identifying the apoptosis sensitivity of cells include, for example, an agent containing a DNA having the nucleotide sequence represented by SEQ ID NO:7, a DNA having the same sequence as 5 to 60 consecutive bases in the nucleotide sequence represented by SEQ ID NO:7, an antibody capable of recognizing a protein having the amino acid sequence represented by SEQ ID NO:8, or the like.

Since the DNAs of the present invention were obtained from human umbilical vein endothelial cells (HUVECs) as shear stress-responsive DNAs, it is desirable that the objective cells used for the identification of apoptosis sensitivity are vascular endothelial cells such as human primary cultured vascular endothelial cells and human umbilical vein endothelial cells (HUVECs). However, since apoptosis is a phenomenon universally observed in all types of cells of the living body including vascular endothelial cells, the objective cells are not limited to vascular endothelial cells.

### (2) Regulation of the apoptosis of cells

Since the DNA of the present invention is a shear stress-responsive gene which is known to exhibit an increase of expression in response to a shear stress and lead to the suppression of apoptosis, the DNA of the present invention or a DNA having the same sequence as 5 to 60 consecutive bases in the DNA may be involved in the suppression of apoptosis. On the other hand, when an antisense DNA having a nucleotide sequence complementary to the nucleotide sequence of each of these DNAs is used, the apoptosis of cells is promoted because the endogenous transcription or translation of the DNA is suppressed.

Similarly to the DNA of the present invention, the apoptosis of cells may also be regulated using a protein encoded by the DNA of the present invention or an antibody capable of recognizing the protein. Specifically, a protein having an apoptosis-suppressing activity is selected from various proteins encoded by the DNAs of the present invention, and the DNA encoding this protein is integrated into a virus vector to create a recombinant virus vector. Then, the apoptosis of cells or a tissue may be suppressed by introducing the recombinant virus vector into the cells or tissue and expressing the protein having an apoptosis-suppressing activity.

Moreover, the apoptosis of cells may be regulated by using an antibody capable of recognizing the aforesaid protein and thereby giving a positive or negative apoptosis-regulating signal to the cells.

Examples of the method for suppressing or promoting apoptosis include a method for promoting the apoptosis of cells by suppressing the endogenous transcription or translation of the DNA using a DNA having the nucleotide sequence represented by SEQ ID NO:7, a DNA having the same sequence as 5 to 60 consecutive bases in the nucleotide sequence represented by SEQ ID NO:7, or an antisense DNA having a nucleotide sequence complementary to the nucleotide sequence of each of these DNAs, for example, according to the antisense technique; and a method for suppressing the apoptosis of cells by introducing the DNA into the cells and thereby accelerating the transcription of the DNA.

Moreover, they also include a method for suppressing the apoptosis of cells by increasing the intracellular expression level of a protein having the amino acid sequence represented by SEQ ID NO:8, using a recombinant virus vector containing a DNA having the nucleotide sequence represented by SEQ ID NO:7, a recombinant virus vector containing an RNA comprising a sequence homologous with the sense strand of the DNA having the nucleotide sequence represented by SEQ ID NO:7, or a recombinant virus vector capable of producing a protein having the amino acid sequence represented by SEQ ID NO:8.

Furthermore, since the amino acid sequence represented by SEQ ID NO:8 is considered to be a membrane protein on the basis of its structure, they also include a method for regulating the apoptosis of cells by subjecting the cells to the action of an antibody capable of recognizing a protein having the amino acid sequence represented by SEQ ID NO:8, stimulating the protein expressed on the cell surface, and thereby transducing a positive or negative apoptosis-regulating signal in the cells.

The DNA of the present invention, a DNA having the same sequence as 5 to 60 consecutive bases in the nucleotide sequence of the DNA, a recombinant virus vector capable of expressing the protein of the present invention having an apoptosis-suppressing activity, and an antibody capable of recognizing the protein of the present invention, which are used in the above-described methods, are effective as agents for regulating the apoptosis of cells. These agents can also be utilized as therapeutic agents for vascular diseases caused by arteriosclerosis.

The agents for regulating apoptosis include, for example, an agent containing a DNA having the nucleotide sequence represented by SEQ ID NO:7, a DNA having the same sequence as 5 to 60 consecutive bases in the nucleotide sequence represented by SEQ ID NO:7, an antisense DNA having a nucleotide sequence complementary to the nucleotide sequence of each of these DNAs, a recombinant virus vector containing a DNA having the nucleotide sequence represented by SEQ ID NO:7, a recombinant virus vector containing an RNA comprising a sequence homologous with the sense strand of the DNA having the nucleotide sequence represented by SEQ ID NO:7, a recombinant virus vector capable of producing a protein having the amino acid sequence represented by SEQ ID NO:8, or an antibody capable of recognizing a protein having the amino acid sequence represented by SEQ ID NO:8.

Since the DNAs of the present invention were obtained from human umbilical vein endothelial cells (HUVECs) as shear stress-responsive DNAs, it is desirable that the objective cells used for the regulation of apoptosis are vascular endothelial cells such as human primary cultured vascular endothelial cells and human umbilical vein endothelial cells (HUVECs). However, since apoptosis is a phenomenon universally observed in all types of cells of the living body including vascular endothelial cells, the objective cells are not limited to vascular endothelial cells.

### (3) Screening of an agent for regulating the apoptosis of cells

The methods for screening an agent for regulating the apoptosis of cells using the shear stress-responsive DNA of the present invention or a protein encoded by the DNA are described below.

One of the aforesaid screening methods is such that, when apoptosis is induced in an animal cell line exhibiting the Fas-dependent induction of apoptosis, a compound or protein which can suppress or promote apoptosis by regulating the endogenous transcription or translation of the DNA of the present invention is selected.

In particular, a compound or protein which can suppress apoptosis by promoting the endogenous transcription or translation of the DNA of the present invention is effective for the treatment of vascular diseases caused by arteriosclerosis. On the other hand, a compound or protein which can promote apoptosis by suppressing the endogenous transcription or translation of the DNA of the present invention is effective for the treatment of diseases based on abnormal proliferation of cells, such as cancer.

According to one exemplary method for screening an agent for regulating the apoptosis of cells using the DNA of the present invention, after a test material is made to act on cells, an increase or decrease of the endogenous transcription level of a DNA having the nucleotide sequence represented by SEQ ID NO:7 is assayed using the DNA having the nucleotide sequence represented by SEQ ID NO:7, or a DNA having the same sequence as 5 to 60 consecutive bases in the nucleotide sequence represented by SEQ ID NO:7. Thus, an agent for suppressing or promoting the apoptosis of the cells can be screened.

Another of the aforesaid screening methods is such that, when an animal cell which has been transformed by introducing the DNA of the present invention so as to produce the protein of the present invention or a partial polypeptide of the protein is used, a compound or protein which can suppress the apoptosis of the cell by binding specifically to the cell is selected. In this method, the specific binding of a compound or protein can be detected by using an untransformed cell as a control. The agent obtained by this screening is also effective for the treatment of vascular diseases caused by arteriosclerosis.

According to one exemplary screening method using the protein of the present invention, a DNA having the nucleotide sequence represented by SEQ ID NO:7 is introduced into cells using a recombinant virus vector containing the DNA having the nucleotide sequence represented by SEQ ID NO:7, or a recombinant virus vector containing an RNA comprising a sequence homologous with the sense strand of the DNA having the nucleotide sequence represented by SEQ ID NO:7, to express a protein having the amino acid sequence represented by SEQ ID NO:8. By exposing the cells to a test material so as to contact the test material with the protein, an agent which binds specifically to the protein to change the activity of the protein is selected. Thus, an agent for suppressing or promoting the apoptosis of cells can be screened.

Alternatively, a DNA having the nucleotide sequence represented by SEQ ID NO:7 or a DNA encoding a protein having the amino acid sequence represented by SEQ ID NO:8 is inserted into a vector to construct a recombinant DNA. This recombinant DNA is introduced into a host cell, and the resulting transformant is cultured in a culture medium. By using the resulting culture to contact the protein in the culture with a test material, an agent which binds specifically to the protein to change the activity of the protein is selected. Thus, an agent for suppressing or promoting the apoptosis of cells can be screened.

Alternatively, an isolated and purified protein having the amino acid sequence represented by SEQ ID NO:8 or a partial peptide of the protein having the amino acid sequence represented by SEQ ID NO:8 is used in an in vitro system. By contacting a test material with the protein or the peptide, an agent which binds specifically to the protein or peptide to cause a change in the activity of the protein is selected. Thus, an agent for suppressing or promoting the apoptosis of cells can be screened.

When an agent for suppressing or promoting apoptosis is screened by using an increase or decrease of the transcription level of a DNA having the nucleotide sequence represented by SEQ ID NO:7 in the cells as an index, the transcription level of the DNA may be analyzed according to a technique such as Northern hybridization, in situ hybridization, RNase protection assay or RT-PCR, by using a probe or primer comprising the DNA having the nucleotide sequence represented by SEQ ID NO:7, or a DNA having the same sequence as 5 to 60 consecutive bases in the nucleotide sequence represented by SEQ ID NO:7.

When an agent for suppressing or promoting apoptosis is screened by using the expression level of a protein having the amino acid sequence represented by SEQ ID NO:8 in the cells as an index, the expression level of the protein may be analyzed according to an immunological detection technique using an antibody capable of recognizing the protein having the amino acid sequence represented by SEQ ID NO:8.

The agents obtained by the above-described screening methods can be utilized as an agent for suppressing or promoting the apoptosis of cells.

Since the DNAs of the present invention were obtained from human umbilical vein endothelial cells (HUVECs) as shear stress-responsive DNAs, it is desirable that the objective cells used for the regulation of apoptosis are vascular endothelial cells such as human primary cultured vascular endothelial cells and human umbilical vein endothelial cells (HUVECs). However, since apoptosis is a phenomenon universally observed in all types of cells of the living body including vascular endothelial cells, the objective cells are not limited to vascular endothelial cells.

As the vector used to express the DNA of the present invention in an animal cell and the method for introducing a recombinant vector, there may be employed any of the previously described methods.

As the immunological detection technique for assaying an increase or decrease of the expression level of the protein of the present invention using an antibody, there may be employed any of the previously described techniques.

As the host cell required in a screening system for detecting the suppression or promotion of apoptosis, there may be used any animal cell that exhibits the Fas-dependent induction of apoptosis. Examples thereof include suspension type cells such as Jurkat [J. Exp. Med., 152,1709-19(1980)], HPB-ALL [Int. J. Cancer, 21, 166-170(1978)] and SKW6.4[Immunol. Lett., 7, 17-23(1983)]; and adhesion type cells such as HeLa and A673 [Arch. Biochem. Biophys., 230, 93-102(1984)].

One example of a substance for inducing Fas-dependent cell death in the aforesaid cell lines is the anti-human Fas monoclonal antibody CH-11 [J. Exp. Med., 169, 1747-1756(1989)]. Exemplary methods for inducing cell death are as follows. In the case of a suspension type cell, a cell suspension is diluted with a culture medium so as to have a density of about 10⁶ cells/ml and added to a 24-well plate or a 96-well microtiter plate for the culture of animal cells. After the anti-human Fas monoclonal antibody is added to a concentration of 1 to 500 ng/ml, the plate is incubated in a CO₂ incubator at 37°C for several hours to 2 day and culture is carried out. In the case of an adhesion type cell, cells are inoculated onto a plate in advance. When cell death is to be induced, the culture medium is replaced by a culture medium containing the anti-human Fas monoclonal antibody, and the culture is continued in a CO₂ incubator at 37°C.

As the method for detecting the suppression or promotion of apoptosis, there may be employed, for example, a detection method in which the cells are stained with trypan blue, Giemsa stain or the like and observed under an optical microscope. In the case of adhesion type cells, apoptosis causes cells to detach from the plate and float. Accordingly, the occurrence of apoptosis can be easily detected without staining. Also known is a detection method in which the cells are stained with a fluorochrome such as Hoechst 33342, Hoechst 33258 or propidium iodide and observed under a fluorescence microscope [Biomanual UP Series, New Experimental Methods for the Research of Apoptosis (in Japanese), Second Edition]. Moreover, there may also be employed biochemical methods such as a method involving the measurement of the activity of caspase activated in the process of apoptosis [J. Exp. Med., 183, 1957-1964(1996)], and MTT assay involving the measurement of mitochondrial dehydrogenase activity in living cells [J. Immunol. Methods, 16, 55-63(1983)]. Furthermore, a method for detecting a structural change of cell membrane using Annexin V [J. Exp. Med., 182, 1545-1556(1995)], and detection methods based on DNA fragmentation such as TUNEL method and Burton's method [Biomanual UP Series, New Experimental Methods for the Research of Apoptosis (in Japanese), Second Edition] are also known.

### Examples

The present invention is more specifically described hereinbelow with reference to the following examples. However, the present invention is in no way to be limited to these examples.

### Example 1

### Construction of a cDNA library from HUVECs having a shear stress applied thereto

### (1) Culture of HUVECs

Using F-12K medium (manufactured by Dainippon Pharmaceutical Co., Ltd.) containing 10% fetal calf serum, 1% penicillin (5,000 units/ml)/streptomycin (5 mg/ml) solution (manufactured by Life Technologies), 0.003% Endothelial Cell Growth Supplement (manufactured by Becton Dickinson), 0.01% heparin (manufactured by Wako Pure Chemical Industries Ltd.) and 0.14% NaHCO₃ (manufactured by Life Technologies), HUVECs were cultured and subcultured under the condition of 5% CO₂ and 37°C. The HUVECs used were purchased from Clonetics.

### (2) Application of a shear stress to HUVECs

A suspension of 0.2 g of micro-carriers (Cytodex 3; manufactured by Amersham Pharmacia Biotech) in 10 ml of PBS buffer was transferred to a sterilized 50 ml tube, and centrifuged at 1,000 rpm for 3 minutes at room temperature. After the supernatant was removed, F12K medium was added. After the resulting suspension was centrifuged again and the supernatant was removed, the medium was added to make up to about 10 ml.

After the HUVECs obtained in the above culture step (1) were dissociated with trypsin/EDTA, about 2 x 10⁶ HUVECs were suspended in 10 ml of the medium and mixed with the above-described micro-carriers. This mixture was transferred to a 200 ml spinner flask, and 15 ml of the medium was added to make a total volume of about 35 ml. The mixture was stirred at 50-60 rpm for 30 seconds and then allowed to stand for one hour. By repeating this stirring/standing procedure four times, HUVECs were made to adhere to the micro-carriers. Thereafter, a shear stress was applied to the cells by stirring the mixture at 160 rpm for a selected period of time.

### (3) Preparation of RNA

Samples of 1.6 x 10⁷ HUVECs having a shear stress applied thereto for 0.5 hour, 1 hour, 1.5 hours, 2 hours, 3 hours, 4 hours, 6 hours, 10 hours, and 20 hours respectively were prepared in the manner described in the above step (2). From each of the aforesaid nine samples of cells having different shear stress application times, total RNAs were prepared by the guanidine thiocyanate-cesium trifluoroacetate method [Methods in Enzymology, 154, 3(1987)]. 100 µg each of these total RNAs from the nine samples were mixed to obtain 900 µg of total RNA. This 900 µg of total RNA was passed through an oligo-dT cellulose column (manufactured by Collaborative Research) to obtain 30.9 µg of mRNA as poly(A)⁺ RNA.

### (4) Construction of a cDNA library

Using 3.0 µg of mRNA obtained in the above step (3), the synthesis of cDNA, the addition of BamHI adapter, and cleavage reaction with NotI were carried out according to the linker primer method [Hiroshi Nojima ed., "Methods for the Construction of Gene Libraries" (in Japanese)]. The resulting double-stranded cDNAs were ligated between BglII/NotI of the plasmid vector pAP3neo [Genes to Cells, 3, 459(1998)] so that 5'-terminus of the cDNAs was always located on the BglII site of the vector. Using the resulting ligation reaction solution, the plasmid was introduced into Escherichia coli MC1061A (Molecular Cloning, Second Edition) by electroporation. Thus, a cDNA library was constructed.

### Example 2

### Construction of a subtraction library

### (I) Preparation of single-strand DNA

2 µg of the plasmid of the cDNA library obtained in Example 1 by amplification in MC1061A was introduced into Escherichia coli XL1-Blue MRF' (manufactured by Stratagene) by electroporation. After this Escherichia coli was suspended in 4.5 ml of SOC medium (Molecular Cloning, Second Edition) and incubated at 37°C for 1 hour with vigorous shaking, all of the resulting culture was added to 5.5 ml of LB medium (Molecular Cloning, Second Edition) containing 50 µg/ml ampicillin. After being incubated at 37°C for 5 hours with vigorous shaking, 5 ml of the resulting culture was inoculated into 45 ml of 2·YT medium (Molecular Cloning, Second Edition) containing ampicillin, and 1 x 10¹¹ pfu of helper phage R408 [Gene, 45, 333(1986)] was added thereto. After being incubated at 37°C for 12 hours with vigorous shaking, the resulting culture was transferred to a sterilized tube and centrifuged at 10,000 rpm for 10 minutes at 4°C to precipitate the Escherichia coli. The phage-containing supernatant was transferred to a new sterilized tube and centrifuged again. The supernatant was passed through a sterilizing filter (manufactured by Millipore) having a pore diameter of 0.22 µm to remove the Escherichia coli completely. 2.5 ml of 10·DNase buffer [100 mM Tris-HCl (pH 7.5), 100 mM MgCl₂], 1 µl of 20 units/µl DNase I (manufactured by Nippon Gene Co., Ltd.) were added to 25 ml of the phage solution, and this mixture was reacted at 37°C for 30 minutes. Then, 1/4 volume of 20% polyethylene glycol (molecular weight 6,000)/2.5 M NaCl was added thereto and mixed well, following by standing at room temperature for 20 minutes. After this mixture was centrifuged at 10,000 rpm for 10 minutes at 4°C, the supernatant was removed completely. The resulting precipitate of phage was dissolved in 400 µl of TE [10 mM Tris-HCl (pH 8.0), 1 mM EDTA (pH 8.0)], and 25 µl of 25 mg/ml Proteinase K and 4 µl of 10% SDS were added thereto, following by reaction at 42°C for 1 hour. The reaction mixture was subjected to a phenol treatment, a phenol-chloroform treatment and a chloroform treatment, and then precipitated with ethanol. The resulting precipitate of single-strand phage DNA was dissolved in 30 µl of TE.

### (2) Biotinylation of RNA

In the same manner as in Example 1, poly(A)⁺ RNA was prepared from HUVECs having no shear stress applied thereto (i.e., HUVECs made only to adhere to micro carriers). To 30 µg of this RNA was added distilled water so as to make a volume of 20 µl. Then, 30 µl of 1 µg/µl PHOTOPROBE biotin (manufactured by Vector Laboratories) was added thereto in the dark. After the tube was uncapped and placed on ice, the mixture was irradiated with light from a mercury vapor lamp disposed about 10 cm above the tube for 20 minutes to biotinylate the RNA, followed by the addition of 50 µl of 100 mM Tris·HCl (pH 9.5)/1 mM EDTA (pH 8.0). Then, 100 µl of water-saturated butanol was added thereto, followed by vigorous stirring. After this mixture was centrifuged at 14,000 rpm for 5 minutes at 4°C, the upper butanol layer was removed. This procedure was repeated two more times. 100 µl of chloroform was added to the aqueous layer, followed by vigorous stirring. After this mixture was centrifuged at 14,000 rpm for 5 minutes at 4°C, the aqueous layer was transferred to a new tube. After this procedure was repeated again, RNA was precipitated with ethanol. The recovered precipitate of RNA was dissolved in 20 µl of distilled water, and subjected again to the procedure for biotinylation. The biotinylated RNA was preserved at -80°C in the ethanol-precipitated state till use for hybridization.

### (3) Hybridization of single-strand DNA with RNA

20 µg of the biotinylated RNA prepared in step (2) was recovered by centrifugation at 14,000 rpm for 15 minutes at 4°C, and dissolved in 8 µl of distilled water. To this solution, 12.5 µl of 2 x reaction buffer [80% formamide, 100 mM HEPES (pH 7.5), 2 mM EDTA (pH 8.0), 0.2% SDS], 2.5 µl of 2.5 M NaCl, 1 µl of 1 µg/µl poly(A) (manufactured by Amersham Pharmacia Biotech), and 1 µl (0.5 µg/µl) of the single-strand DNA prepared in step (1) from the cDNA library derived from HUVECs having a shear stress applied thereto were added so as to make a total volume of 25 µl. After this mixture was heated at 65°C for 10 minutes, it was quickly transferred to a heat block warmed at 42°C and incubated at 42°C for two nights to effect hybridization.

### (4) Subtraction and rehybridization

After completion of the hybridization, 400 µl of a buffer [500 mM NaCl, 50 mM HEPES (pH 7.5), 2 mM EDTA (pH 8.0)] was added to the reaction mixture. Then, 5 µl of 2 µg/µl streptavidin (manufactured by Life Technologies) was added thereto and mixed therewith. After this mixture was allowed to stand at room temperature for 5 minutes, it was subjected to a phenol-chloroform treatment. The aqueous layer was transferred to a new tube, and 5 µl of fresh streptavidin was added thereto. After this mixture was allowed to stand at room temperature for 5 minutes, subtraction was carried out by subjecting it twice to a phenol-chloroform treatment and once to a chloroform treatment. The aqueous layer was placed in the upper chamber of a Millipore Filter UFCP3TK50 (manufactured by Millipore) and centrifuged at 10,000 rpm at 4°C until all of the solution passed into the lower chamber. After the solution was removed from the lower chamber, the filter was washed by adding 300 µl of TE to the upper chamber and centrifuging the filter. After this procedure was repeated, single-strand DNA captured on the filter was recovered with 30 µl of 1/10 TE. This single-strand DNA was dried under vacuum and dissolved in distilled water to make up to 9 µl. After 10 µg of the biotinylated RNA prepared in step (2) was precipitated with ethanol and recovered by centrifugation, 9 µl of the above single-strand DNA solution was added to the precipitate. After the addition of 12.5 µl of 2 x reaction buffer, 2.5 µl of 2.5 M NaCI, and 1 µl of poly(A), a second hybridization step was carried out in the same manner as in step (3), and subtraction was carried out in the above-described manner. Thereafter, single-strand DNA was recovered in a similar manner and subjected to a third subtraction step by hybridization with 10 µg of the biotinylated RNA and a fourth subtraction step by using 5 µg of the biotinylated RNA.

### (5) Synthesis of double-strand DNA and its introduction into Escherichia coli

After four subtraction steps were successively carried out as described above, the resulting single-strand DNA was recovered in 30 µl of 1/10 TE. To a 15 µl portion thereof, 14 µl of distilled water and 1 µl of a 2 µg/µl primer extension primer having the nucleotide sequence represented by SEQ ID NO: 159 were added, followed by heating at 65°C for 10 minutes. After this mixture was allowed to stand at room temperature for 5 minutes so as to anneal the primer to single-strand DNA, 5 µl of 10 x reaction buffer (attached to BcaBEST Dideoxy Sequencing Kit; manufactured by Takara Shuzo Co., Ltd.), 10 µl of a 1 mM dNTP mixture, 0.5 µl of 3 µg/µl single-strand DNA-binding protein (manufactured by USB), 2 µl of 2 units/µl BcaBEST DNA polymerase (manufactured by Takara Shuzo Co., Ltd.), and 2.5 µl of distilled water were added thereto. This mixture was reacted at 65°C for 1 hour to synthesize double-strand DNA. After the addition of 50 µl of distilled water, the reaction mixture was subjected to a phenol-chloroform treatment and a chloroform treatment. The resulting solution was concentrated by means of a Millipore Filter UFCP3TK50, and the double-strand DNA was finally dissolved in 20 µl of TE. Using 1/5 volume of this solution, the double-strand DNA was introduced into Escherichia coli MC1061A by electroporation.

### (6) Reverse subtraction

Escherichia coli MC1061A having the double-strand DNA introduced thereinto, which was obtained in step (5), was cultured, and plasmid DNA was prepared from the Escherichia coli. In the same manner as in step (1), this plasmid DNA was introduced into Escherichia coli XL1-Blue MRF' to prepare single-strand DNA. Two µg of mRNA derived from HUVECs having a shear stress applied thereto was biotinylated in the manner described in step (2), and mixed with 2 µg of the aforesaid single-strand DNA. To this mixture, 12.5 µl of 2 x reaction buffer, 2.5 µl of 2.5 M NaCI, 1 µl of 1 µg/µl poly(A), and 1 µl of distilled water were added so as to make a total volume of 25 µl. In the same manner as in step (3), this mixture was incubated at 42°C for two nights to carry out hybridization. Four hundred µl of a buffer [500 mM NaCI, 50 mM HEPES (pH 7.5), 2 mM EDTA (pH 8.0)] was added to the reaction mixture. Then, 7 µl of 2 µg/µl streptavidin was added thereto and mixed therewith. After this mixture was allowed to stand at room temperature for 5 minutes, phenol-chloroform was added thereto with vigorous mixing. After this mixture was centrifuged at 14,000 rpm for 7 minutes at room temperature, the aqueous layer was removed. Then, 400 µl of fresh TE was added thereto with vigorous mixing. After this mixture was centrifuged at 14,000 rpm for 7 minutes at room temperature, the aqueous layer was removed. This procedure was repeated two more times, so that single-strand DNA which did not hybridize with the biotinylated RNA was removed. After 400 µl of TE was added without mixing, the tube was heated at 95°C for 5 minutes in the uncapped state. Thereafter, by placing the tube on ice for 5 minutes to denature the DNA, the single-strand DNA having hybridized with the biotinylated RNA and present in the phenol-chloroform layer was separated from the biotinylated RNA. After the reaction mixture was vigorously mixed and centrifuged at 14,000 rpm for 7 minutes at room temperature, the aqueous layer was transferred to a new tube. The aqueous layer was subjected again to a phenol-chloroform treatment and then to a chloroform treatment. The aqueous layer containing the single-strand DNA was concentrated by means of a Millipore Filter UFCP3TK50, and the single-strand DNA was finally recovered in 30 µl of 1/10 TE. Fifteen µl of this solution was dried under vacuum, and dissolved in distilled water to make up to 9 µl. Five µg of mRNA derived from HUVECs having no shear stress applied thereto was biotinylated and recovered by precipitation with ethanol. To the precipitate was added 9 µl of the aforesaid single-strand DNA solution. Then, 12.5 µl of 2 x reaction buffer, 2.5 µl of 2.5 M NaCl, and 1 µl of poly(A) were added thereto, and normal subtraction was carried out in the same manner as steps (3) and (4).

That is, a subtraction library in which genes exhibiting an increase of expression in response to the application of a shear stress in HUVECs were concentrated was prepared by carrying out four successive subtraction steps, one reverse subtraction step, and one normal subtraction step.

### Example 3

### Obtaining of clones exhibiting an alteration of expression by Northern hybridization

Northern hybridization was carried out in order to select clones which are included in the subtraction library obtained in Example 2 and exhibit a shear stress-dependent increase of expression.

### (1) Transfer of RNA to a membrane

According to the same procedure as in Example 1, total RNAs were obtained from HUVECs having a shear stress applied thereto and HUVECs having no shear stress applied thereto, respectively. To 4 µg of each total RNA was added distilled waster so as to make a volume of 1.8 µl. Then, 0.8 µl of 10 x MOPS buffer [80 mM sodium acetate, 197 mM MOPS, 10 mM EDTA (pH 8.0)], 1.4 µl of a 35% formaldehyde solution (manufactured by Nacalai Tesque), and 4 µl of deionized formamide were added thereto. After this mixture was heated at 65°C for 15 minutes and then cooled rapidly by placing it on ice for 5 minutes, the total amount thereof was electrophoresed through 1 x MOPS/2% formaldehyde/1% agarose gel. After completion of the electrophoresis, the gel was washed with distilled water for 20 minutes, and this washing step was repeated three times to remove any formaldehyde from the gel. After the gel was soaked in 20xSSC (3 M NaCl, 0.3 M sodium citrate) for 30 minutes, RNA in the gel was transferred to a nylon membrane Biodyne A (manufactured by Pall BioSupport) according to a capillary transfer method using 20xSSC. After completion of the transfer, the RNA was fixed to the membrane by allowing the membrane to stand at 80°C for 2 hours.

### (2) Labeling of probes

In the subtraction library obtained in Example 2, clones having an inserted DNA fragment of not less than 0.4 kb size were treated by cleaving the plasmid with SmaI and NotI to excise the inserted DNA fragment. The fragments thus obtained were purified using a QIAquick Gel Extraction Kit (manufactured by QIAGEN), and the procedure therefor was carried out according to the manual attached to the kit. Using about 50 ng of the purified DNA fragments as templates, the DNA fragments were labeled using a Random Primer DNA Labeling Kit Ver. 2 (manufactured by Takara Shuzo Co., Ltd.) and [α-³²P]dCTP (110 TBq/mmol; Amersham Pharmacia Biotech), and used as probes. The procedure therefor was carried out according to the manual attached to the kit.

### (3) Hybridization and autoradiography

The membrane prepared in step (1) was placed in a hybridization bag, and a freshly prepared hybridization solution [50% formamide, 5 x Denhardt's, 5 x SSC, 0.1% SDS, denatured salmon DNA (0.1 mg/ml)] was added thereto. The hybridization bag was incubated at 42°C for 2 hours or more to carry out prehybridization. The probes prepared in step (2) were denatured by heating them at 95°C for 5 minutes and cooling them rapidly. These probes were mixed with a hybridization solution and added to the prehybridized membrane. The hybridization bag was incubated at 42°C for 24 hours or more to carry out hybridization. The membrane was taken out of the hybridization bag, placed in 2 x SSC/0.1% SDS, and slowly shaken at room temperature for 10 minutes to remove the hybridization solution as much as possible. Then, the membrane was washed in 0.15 x SSC/0.1% SDS at 42°C for 30 minutes, and this washing step was repeated twice. After completion of the washing steps, autoradiography was carried out by exposing an X-ray film to the membrane. A total of 1,026 clones were named A4RS-1 to A4RS-1026, respectively, and each of them was subjected to Northern hybridization. Thus, there were obtained 107 clones exhibiting a shear stress-dependent increase of expression.

### Example 4

### Identification of clones exhibiting alteration of expression

### (1) Determination of nucleotide sequences

With respect to the clones which were ascertained to exhibit an increase of expression in response to the application of a shear stress in Example 3, their nucleotide sequences were determined by means of a 377 DNA Sequencer (manufactured by Perkin Elmer). For the determination of the nucleotide sequences, a Dye Primer Cycle Sequencing Kit (manufactured by Perkin Elmer) was used. The procedure therefor was carried out according to the attached manual. The clones exhibiting alteration of expression were identified by comparing the resulting nucleotide sequences with the database GenBank. As a result, the 107 clones were classified into 88 types of genes. In these 88 genes, 5 genes which have been reported to exhibit the induction of expression by a shear stress stimulus in vascular endothelial cells, i.e. the genes encoding endothelin 1, monocyte chemotactic protein 1, heparin-binding EGF-like growth factor, thrombomodulin, and transforming growth factor β, are included. Accordingly, 83 genes with which the induction of expression by a shear stress stimulus in vascular endothelial cells had not yet been reported could be identified. These genes included 55 known genes and 28 novel genes. With respect to genes whose sequences are not identical with any of the full-length cDNAs included in the known sequences, but are identical only with expressed sequence tags (ESTs) alone, and genes whose sequences are not identical with any of the known sequences (i.e., novel genes), all ESTs included in the corresponding UniGene are joined together to construct as long sequences as possible on a computer. With respect to eight of the novel genes, full-length cDNAs were cloned from a cDNA library prepared with a λ phage vector in Example 5 that will be given later.

### (2) Known genes exhibiting a shear stress-dependent increase of expression

When the nucleotide sequence of A4RS-016 was determined, this was identical with the sequence of thioredoxin reductase [Accession: X91247] (SEQ ID NO:1). The amino acid sequence encoded by this gene is shown as SEQ ID NO:2. Thioredoxin reductase is an enzyme reducing thioredoxin using NADPH, and participates in various physiological reactions such as control of intracellular antioxidation, signal transduction, and NO production. Its Northern blots exhibiting a shear stress-dependent increase of expression are shown in lane 1 of FIG. 1 and lane 1 of FIG. 3.

When the nucleotide sequence of A4RS-026 was determined, this was identical with the sequence of lipopolysaccharide-induced protein gene [Accession: Q51544] (SEQ ID NO:3). The amino acid sequence encoded by this gene is shown as SEQ ID NO:4. The protein encoded by this gene does not show substantial homology with other known proteins, and its function is unknown. Its Northern blot exhibiting a shear stress-dependent increase of expression is shown in lane 2 of FIG. 1.

When the nucleotide sequence of A4RS-040 was determined, this was identical with the sequence of spliceosome-associated protein (SAP145) [Accession: U41371] (SEQ ID NO:5). The amino acid sequence encoded by this gene is shown as SEQ ID NO:6. Its Northern blot exhibiting a shear stress-dependent increase of expression is shown in lane 3 of FIG. 1.

When the nucleotide sequence of A4RS-041 was determined, this was identical with the sequence of human proline-rich membrane protein (PRMP) [Accession: V50494] (SEQ ID NO:7). The amino acid sequence encoded by this gene is shown as SEQ ID NO:8. Only the sequence of PRMP is registered in a database, and its function is unknown. However, PRMP has substantial homology with rat neural membrane protein 35 (NMP35) [Molecular and Cellular Neuroscience, 11, 260(1998)] and the glutamate-binding subunit of NMDA receptor [Accession: W62612]. Although the function of NMP35 is not clearly known, it is expressed specifically in the brain, like the glutamate-binding subunit of NMDA receptor. From an analysis of hydrophilicity on the basis of its amino acid sequence, NMP35 is presumed to be a membrane protein. RPMP also has an extremely high degree of hydrophobicity and hence functions as a membrane protein. Its Northern blots exhibiting a shear stress-dependent increase of expression are shown in lane 4 of FIG. 1 and lane 2 of FIG. 3.

When the nucleotide sequence of A4RS-063 was determined, this was identical with the sequence of puromycin-sensitive aminopeptidase [Accession: AJ132583] (SEQ ID NO:9). The amino acid sequence encoded by this gene is shown as SEQ ID NO: 10. Its Northern blots exhibiting a shear stress-dependent increase of expression are shown in lane 5 of FIG. 1 and lane 3 of FIG. 3.

When the nucleotide sequence of A4RS-096 was determined, this was identical with the sequence of human secreted protein gene 125 clone HSPAG15 [Accession: V59635] (SEQ ID NO:11). The amino acid sequence encoded by this gene is shown as SEQ ID NO:12. Only the sequence of this gene is registered in a bank, and its function is unknown. The protein encoded by this gene does not show substantial homology with other known proteins. Its Northern blots exhibiting a shear stress-dependent increase of expression are shown in lane 6 of FIG. 1 and lane 4 of FIG. 3.

When the nucleotide sequence of A4RS-116 was determined, this was identical with the sequence of lamin C [Accession: M13451] (SEQ ID NO:13). The amino acid sequence encoded by this gene is shown as SEQ ID NO:14. Lamin C is a lining protein for the nuclear membrane and is one of the cytoskeleton forming factors. Its Northern blots exhibiting a shear stress-dependent increase of expression are shown in lane 7 of FIG. 1 and lane 5 of FIG. 3.

When the nucleotide sequence of A4RS-126 was determined, this was identical with the sequence of cytokine-response gene CR8 [Accession: T43383] (SEQ ID NO:15). The amino acid sequence encoded by this gene is shown as SEQ ID NO:16. Cytokine-response gene CR8, which is also called DEC1, is a transcription factor having a basic helix-loop-helix motif. In particular, it has high homology with a HES family of transcription factors participating in nerve differentiation. Its Northern blot exhibiting a shear stress-dependent increase of expression is shown in lane 8 of FIG. 1.

When the nucleotide sequence of A4RS-131 was determined, this was identical with the sequence of human enhancer of filamentation (HEF1) [Accession: L43821] (SEQ ID NO:17). The amino acid sequence encoded by this gene is shown as SEQ ID NO:18. HEF1 is a signal transduction molecule having an SH3 domain, having a FAK-binding activity, and participating in regulation of the cytoskeleton. Its Northern blot exhibiting a shear stress-dependent increase of expression is shown in lane 9 of FIG. 1.

When the nucleotide sequence of A4RS-148 was determined, this was identical with the sequence of interferon-induced 15-kDa protein gene [Accession: M21786] (SEQ ID NO:19). The amino acid sequence encoded by this gene is shown as SEQ ID NO:20. The protein encoded by this gene does not show substantial homology with other known proteins, and its function is unknown. Its Northern blot exhibiting a shear stress-dependent increase of expression is shown in lane 10 of FIG. 1.

When the nucleotide sequence of A4RS-154 was determined, this was identical with the sequence of LDL receptor [Accession: N60388] (SEQ ID NO:21). The amino acid sequence encoded by this gene is shown as SEQ ID NO:22. LDL receptor incorporates LDL, which is one of the causes for the formation of an arteriosclerotic lesion, under the endothelium. It has been reported that, when a shear stress is applied to cultured bovine aortic endothelial cells, the binding and incorporation of LDL via LDL receptor increases [Circulation, 76, 648(1987)]. Its Northern blot exhibiting a shear stress-dependent increase of expression is shown in lane 11 of FIG. 1.

When the nucleotide sequence of A4RS-174 was determined, this was identical with the sequence of peripheral myelin protein (PMP)-22 [Accession: Q32869] (SEQ ID NO:23). The amino acid sequence encoded by this gene is shown as SEQ ID NO:24. PMP-22 is a component of myelin present in the peripheral nervous system, and is a membrane protein having four transmembrane domains. Its Northern blot exhibiting a shear stress-dependent increase of expression is shown in lane 12 of FIG. 1.

When the nucleotide sequence of A4RS-175 was determined, this was identical with the sequence of tyrosine kinase receptor UFO/ArK [Accession: S65125) (SEQ ID NO:25). The amino acid sequence encoded by this gene is shown as SEQ ID NO:26. Its Northern blot exhibiting a shear stress-dependent increase of expression is shown in lane 13 of FIG. 1.

When the nucleotide sequence of A4RS-194 was determined, this was identical with the sequence of calcium-ATPase HK2 [Accession: M23115] (SEQ ID NO:27). The amino acid sequence encoded by this gene is shown as SEQ ID NO:28. Calcium-ATPase HK2 is present in the membranes of the endoplasmic reticulum within cells. Its Northern blot exhibiting a shear stress-dependent increase of expression is shown in lane 14 of FIG. 1.

When the nucleotide sequence of A4RS-197 was determined, this was identical with the sequence of human arginine-rich protein [Accession: M83751] (SEQ ID NO:29). The amino acid sequence encoded by this gene is shown as SEQ ID NO:30. The amino acid sequence encoded by this gene does not show substantial homology with other known proteins, and its function is unknown. However, it is suggested that this gene may be a kind of proto-oncogene. Its Northern blot exhibiting a shear stress-dependent increase of expression is shown in lane 15 of FIG. 1.

When the nucleotide sequence of A4RS-260 was determined, this was identical with the sequence of KIAA0025 [Accession: D14695] (SEQ ID NO:31). The amino acid sequence encoded by this gene is shown as SEQ ID NO:32. The protein encoded by this gene does not show substantial homology with other known proteins, and its function is unknown. Its Northern blots exhibiting a shear stress-dependent increase of expression are shown in lane 16 of FIG. 1 and lane 6 of FIG. 3.

When the nucleotide sequence of A4RS-271 was determined, this was identical with the sequence of human high-mobility group phosphoprotein isoform I-C (HMGI-C) [Accession: U28749] (SEQ ID NO:33). The amino acid sequence encoded by this gene is shown as SEQ ID NO:34. Judging from its structure, HMGI-C is a transcription factor. Its Northern blots exhibiting a shear stress-dependent increase of expression are shown in lane 17 of FIG. 1 and lane 7 of FIG. 3.

When the nucleotide sequence of A4RS-307 was determined, this was identical with the sequence of PRAD1 [Accession: X59798] (SEQ ID NO:35). The amino acid sequence encoded by this gene is shown as SEQ ID NO:36. PRAD1 is a member of the cyclin family and is also called cyclin D1. Its Northern blots exhibiting a shear stress-dependent increase of expression are shown in lane 18 of FIG. 1 and lane 8 of FIG. 3.

When the nucleotide sequence of A4RS-355 was determined, this was identical with the sequence of KIAA0964 [Accession: AB023181] (SEQ ID NO:37). The amino acid sequence encoded by this gene is shown as SEQ ID NO:38. The protein encoded by this gene is judged to be the human ortholog of rat PSD-95/SAP90-associated protein-4 (SAPAP-4). SAPAP-4 is present in membranes and is considered to participate in the clustering of NMDA receptor. Its Northern blot exhibiting a shear stress-dependent increase of expression is shown in lane 19 of FIG. 1.

When the nucleotide sequence of A4RS-389 was determined, this was identical with the sequence of lamin A [Accession: M13452] (SEQ ID NO:39). The amino acid sequence encoded by this gene is shown as SEQ ID NO:40. Lamin A is a lining protein for the nuclear membrane and is one of the cytoskeleton forming factors. Its Northern blots exhibiting a shear stress-dependent increase of expression are shown in panel 20 of FIG. 1 and panel 9 of FIG. 3.

When the nucleotide sequence of A4RS-391 was determined, this was identical with the sequence of non-muscle alpha actinin [Accession: U48734] (SEQ ID NO:41). The amino acid sequence encoded by this gene is shown as SEQ ID NO:42. Alpha actinin is one of the cytoskeleton forming factors. Its Northern blots exhibiting a shear stress-dependent increase of expression are shown in lane 21 of FIG. 1 and lane 10 of FIG. 3.

When the nucleotide sequence of A4RS-423 was determined, this was identical with the sequence of gamma-filamin [Accession: AF089841] (SEQ ID NO:43). The amino acid sequence encoded by this gene is shown as SEQ ID NO:44. Gamma-filamin is an actin filament crosslinking protein, and participates in filopodia formation by binding to low-molecular-weight GTP-binding proteins such as rac and rho. Its Northern blot exhibiting a shear stress-dependent increase of expression is shown in lane 22 of FIG. 1.

When the nucleotide sequence of A4RS-431 was determined, this was identical with the sequence of growth factor inducible immediate early gene product CYR61 [Accession: U62015] (SEQ ID NO:45). The amino acid sequence encoded by this gene is shown as SEQ ID NO:46. CYR61 is also called gigl, monocyte mature differentiation factor, or connective tissue growth factor-2, and is a secreted factor having a signal sequence at the amino-terminus. Its Northern blot exhibiting a shear stress-dependent increase of expression is shown in lane 23 of FIG. 1.

When the nucleotide sequence of A4RS-453 was determined, this was identical with the sequence of nuclear factor of activated T cells (NF-ATc) [Accession: U08015] (SEQ ID NO:47). The amino acid sequence encoded by this gene is shown as SEQ ID NO:48. NF-ATc is one of the components of a transcription factor. Its Northern blot exhibiting a shear stress-dependent increase of expression is shown in lane 24 of FIG. 1.

When the nucleotide sequence of A4RS-492 was determined, this was identical with the sequence of GLI Krupple-related protein [Accession: M77698] (SEQ ID NO:49). The amino acid sequence encoded by this gene is shown as SEQ ID NO:50. GLI Krupple-related protein, which is also called YY1, is a suppressively functioning transcription factor. Its Northern blot exhibiting a shear stress-dependent increase of expression is shown in lane 25 of FIG. 1.

When the nucleotide sequence of A4RS-507 was determined, this was identical with the sequence of human mRNA homologous to the p64 bovine chloride channel [Accession: Y12696] (SEQ ID NO:51). The amino acid sequence encoded by this gene is shown as SEQ ID NO:52. Only the sequence of this gene is reported, and its function is not clearly known. Its Northern blot exhibiting a shear stress-dependent increase of expression is shown in lane 26 of FIG. 1.

When the nucleotide sequence of A4RS-514 was determined, this was identical with the sequence of KIAA0080 [Accession: D38522] (SEQ ID NO:53). The amino acid sequence encoded by this gene is shown as SEQ ID NO:54. The protein encoded by this gene is judged to be the human ortholog of rat synaptotagmin XI that is a membrane protein. Its Northern blot exhibiting a shear stress-dependent increase of expression is shown in lane 27 of FIG. 1.

When the nucleotide sequence of A4RS-523 was determined, this was identical with the sequence of nicotinamide N-methyltransferase [Accession: U08021] (SEQ ID NO:55). The amino acid sequence encoded by this gene is shown as SEQ ID NO:56. Its Northern blot exhibiting a shear stress-dependent increase of expression is shown in lane 28 of FIG. 1.

When the nucleotide sequence of A4RS-544 was determined, this was identical with the sequence of H. sapiens mRNA for surface glycoprotein [Accession: Z50022] (SEQ ID NO:57). The amino acid sequence encoded by this gene is shown as SEQ ID NO:58. The protein encoded by this gene is a type I membrane protein. Its Northern blot exhibiting a shear stress-dependent increase of expression is shown in lane 29 of FIG. 1.

When the nucleotide sequence of A4RS-547 was determined, this was identical with the sequence of early growth response gene alpha(EGR-alpha) [Accession: S81439] (SEQ ID NO:59). The amino acid sequence encoded by this gene is shown as SEQ ID NO:60. EGR-alpha is a transcription factor, and it has been reported that its homologue, EGR-1, is activated by a shear stress in endothelial cells [Arterioscler. Thromb. Vasc. Biol., 17, 2280(1997)]. Its Northern blot exhibiting a shear stress-dependent increase of expression is shown in lane 30 of FIG. 1.

When the nucleotide sequence of A4RS-557 was determined, this was identical with the sequence of SF2p33 [Accession: M69040] (SEQ ID NO:61). The amino acid sequence encoded by this gene is shown as SEQ ID NO:62. SF2p33 is a nuclear factor and is indispensable for the splicing of pre-mRNA. Its Northern blot exhibiting a shear stress-dependent increase of expression is shown in lane 31 of FIG. 1.

When the nucleotide sequence of A4RS-577 was determined, this was identical with the sequence of p66 shc [Accession: U73377] (SEQ ID NO:63). The amino acid sequence encoded by this gene is shown as SEQ ID NO:64. shc is a signal transduction molecule which transduces a stimulus from tyrosine kinase to ras. Its Northern blot exhibiting a shear stress-dependent increase of expression is shown in lane 32 of FIG. 1.

When the nucleotide sequence of A4RS-588 was determined, this was identical with the sequence of lysosomal acid lipase (LAL) [Accession: M74775] (SEQ ID NO:65). The amino acid sequence encoded by this gene is shown as SEQ ID NO:66. LAL, which is also called cholesteryl esterase, is an enzyme hydrolyzing cholesteryl esters incorporated into cells. If this gene is deficient, cholesteryl ester storage disease may be induced to cause arteriosclerosis. Its Northern blot exhibiting a shear stress-dependent increase of expression is shown in lane 33 of FIG. 1.

When the nucleotide sequence of A4RS-602 was determined, this was identical with the sequence of N^{G},N^{G}-dimethylarginine dimethylaminohydrolase (DDAH) [Accession: AB001915] (SEQ ID NO:67). The amino acid sequence encoded by this gene is shown as SEQ ID NO:68. DDAH hydrolyzes N^{G}-monomethyl-L-arginine (MMA) and N^{G},N^{G}-dimethyl-L-arginine (DMA) to citrullin. Since MMA and DMA are substrate analogs for NO synthase, they inhibit the synthesis of NO. That is, DDAH induces NO synthesis indirectly. Its Northern blots exhibiting a shear stress-dependent increase of expression are shown in lane 34 of FIG. 1 and lane 11 of FIG. 3.

When the nucleotide sequence of A4RS-608 was determined, this was identical with the sequence of serum deprivation response (SDPR) [Accession: AF085481] (SEQ ID NO:69). The amino acid sequence encoded by this gene is shown as SEQ ID NO:70. For human SDPR, only its sequence is registered. It has been reported that the expression of its mouse ortholog, sdr, is induced by serum deprivation in NIH3T3. However, its function is unknown. Its Northern blot exhibiting a shear stress-dependent increase of expression is shown in lane 35 of FIG. 1.

When the nucleotide sequence of A4RS-612 was determined, this was identical with the sequence of regulator of G protein signaling (RGS3) [Accession: U27655] (SEQ ID NO:71). The amino acid sequence encoded by this gene is shown as SEQ ID NO:72. Its Northern blot exhibiting a shear stress-dependent increase of expression is shown in panel 36 of FIG. 1.

When the nucleotide sequence of A4RS-625 was determined, this was identical with the sequence of cytokine-inducible nuclear protein C-193 [Accession: X83703] (SEQ ID NO:73). The amino acid sequence encoded by this gene is shown as SEQ ID NO:74. In endothelial cells, this gene is expressed in response to inflammatory stimuli such as TNF-α and LPS. The amino acid sequence encoded by this gene does not show substantial homology with other known proteins, but has been proved to be a nuclear factor. Its Northern blot exhibiting a shear stress-dependent increase of expression is shown in lane 37 of FIG. 1.

When the nucleotide sequence of A4RS-666 was determined, this was identical with the sequence of laminin B1 chain [Accession: M61916] (SEQ ID NO:75). The amino acid sequence encoded by this gene is shown as SEQ ID NO:76. Laminin B1 chain is a glycoprotein and is a kind of extracellular matrix. It has been reported that, in bovine arterial endothelial cells, laminin protein is increased by the application of a shear stress [Laboratory Investigation, 73, 565(1995)]. Its Northern blot exhibiting a shear stress-dependent increase of expression is shown in lane 38 of FIG. 1.

When the nucleotide sequence of A4RS-668 was determined, this was identical with the sequence of Matrix Gla protein (MGP) [Accession: M58549] (SEQ ID NO:77). The amino acid sequence encoded by this gene is shown as SEQ ID NO:78. MGP is a kind of extracellular matrix. It has been reported that, in knockout mice of this gene, calcification occurs in arteries and cartilages and results in death [Nature, 386, 78(1997)]. Its Northern blot exhibiting a shear stress-dependent increase of expression is shown in lane 39 of FIG. 1.

When the nucleotide sequence of A4RS-674 was determined, this was identical with the sequence of PTX3 (SEQ ID NO:79). The amino acid sequence encoded by this gene is shown as SEQ ID NO:80. PTX3 is a member of the pentraxin family, and is a secreted factor having a signal sequence at the amino-terminus. It has been reported that, in vascular endothelial cells and monocytes, this gene is expressed in response to inflammatory stimuli such as IL-1 and TNF-α. Its Northern blot exhibiting a shear stress-dependent increase of expression is shown in lane 40 of FIG. 1.

When the nucleotide sequence of A4RS-682 was determined, this was identical with the sequence of connective tissue growth factor [Accession: X78947] (SEQ ID NO:81). The amino acid sequence encoded by this gene is shown as SEQ ID NO:82. Connective tissue growth factor is a secreted factor having a signal sequence at the amino-terminus, and its expression in developed arteriosclerotic lesions has been reported [Circulation, 95, 831(1997)]. Its Northern blot exhibiting a shear stress-dependent increase of expression is shown in lane 41 of FIG. 1.

When the nucleotide sequence of A4RS-751 was determined, this was identical with the sequence of FLI-1 [Accession: Q50644] (SEQ ID NO:83). The amino acid sequence encoded by this gene is shown as SEQ ID NO:84. FLI-1, which is also called ERGB, is a transcription factor belonging to the ETS family. Its Northern blot exhibiting a shear stress-dependent increase of expression is shown in lane 42 of FIG. 2.

When the nucleotide sequence of A4RS-781 was determined, this was identical with the sequence of HLA-E [Accession: X56841] (SEQ ID NO:85). The amino acid sequence encoded by this gene is shown as SEQ ID NO:86. HLA-E is a kind of MHC class I protein. Its Northern blot exhibiting a shear stress-dependent increase of expression is shown in lane 43 of FIG. 2.

When the nucleotide sequence of A4RS-784 was determined, this was identical with the sequence of plasminogen activator inhibitor (PAI) [Accession: M16006] (SEQ ID NO:87). The amino acid sequence encoded by this gene is shown as SEQ ID NO:88. PAI acts antagonistically against plasminogen activator. It has been reported that its expression is decreased by the application of a shear stress [Blood, 87, 2314(1996)]. Its Northern blots exhibiting a shear stress-dependent increase of expression are shown in lane 44 of FIG. 2 and lane 12 of FIG. 3.

When the nucleotide sequence of A4RS-817 was determined, this was identical with the sequence of keratin 18 [Accession: M26326] (SEQ ID NO:89). The amino acid sequence encoded by this gene is shown as SEQ ID NO:90. Keratin 18 is a kind of intermediate filament. Its Northern blot exhibiting a shear stress-dependent increase of expression is shown in lane 45 of FIG. 2.

When the nucleotide sequence of A4RS-818 was determined, this was identical with the sequence of human secreted protein gene 5 clone HELDY41 [Accession: V34315] (SEQ ID NO:91). The amino acid sequence encoded by this gene is shown as SEQ ID NO:92. The amino acid sequence encoded by this gene coincides with a partial sequence of human hedgehog interacting protein [Accession: W56538]. Its Northern blot exhibiting a shear stress-dependent increase of expression is shown in lane 46 of FIG. 2.

When the nucleotide sequence of A4RS-914 was determined, this was identical with the sequence of monocyte-derived neutrophil-activating protein (MONAP) [Accession: M26383] (SEQ ID NO:93). The amino acid sequence encoded by this gene is shown as SEQ ID NO:94. MONAP is also called interleukin 8 (IL-8), and its relation with the development of arteriosclerosis is strongly suggested. In fact, its strong expression in an mRNA level and in a protein level has been reported in macrophages derived from arteriosclerotic plaques [Arterioscler. Thromb. Vascul. Biol., 16, 1007(1996)]. Its Northern blot exhibiting a shear stress-dependent increase of expression is shown in lane 47 of FIG. 2.

When the nucleotide sequence of A4RS-929 was determined, this was identical with the sequence of MUC18 glycoprotein [Accession: M28882] (SEQ ID NO:95). The amino acid sequence encoded by this gene is shown as SEQ ID NO:96. MUC18, which is also called Mel-CAM or CD146, is a cell adhesion factor having an immunoglobulin-like domain. Its Northern blot exhibiting a shear stress-dependent increase of expression is shown in lane 48 of FIG. 2.

When the nucleotide sequence of A4RS-935 was determined, this was identical with the sequence of nuclear speckle-type protein (SPOP) [Accession: AJ000644] (SEQ ID NO:97). The amino acid sequence encoded by this gene is shown as SEQ ID NO:98. SPOP is a nuclear factor which is considered to interact with splicing factors. Its Northern blot exhibiting a shear stress-dependent increase of expression is shown in lane 49 of FIG. 2.

When the nucleotide sequence of A4RS-938 was determined, this was identical with the sequence of thrombospondin (TSP) [Accession: X14787] (SEQ ID NO:99). The amino acid sequence encoded by this gene is shown as SEQ ID NO:100. TSP is a glycoprotein functioning as an extracellular matrix, and has an inhibitory effect on carcinogenesis and angiogenesis. Its Northern blot exhibiting a shear stress-dependent increase of expression is shown in lane 50 of FIG. 2.

When the nucleotide sequence of A4RS-939 was determined, this was identical with the sequence of caveolin [Accession: Z18951] (SEQ ID NO:101). The amino acid sequence encoded by this gene is shown as SEQ ID NO:102. Caveolin is a principal component of caveolae present in the cell membrane. It has been reported that caveolin participates in the control of NO production by interacting with nitric oxide (NO) synthase [J. Biol. Chem., 273, 34724(1998)]. Its Northern blot exhibiting a shear stress-dependent increase of expression is shown in lane 51 of FIG. 2.

When the nucleotide sequence of A4RS-945 was determined, this was identical with the sequence of human BENE mRNA [Accession: U17077] (SEQ ID NO:103). The amino acid sequence encoded by this gene is shown as SEQ ID NO:104. BENE is a membrane protein having homology with T cell surface glycoprotein MAL. Since its expression in endothelial cells is increased by lysophosphatidyl choline (lysoPC) that is a component of oxidized lipoproteins, its relation with arteriosclerosis is suggested [J. Biochemistry, 123, 1119(1998)]. Its Northern blot exhibiting a shear stress-dependent increase of expression is shown in lane 52 of FIG. 2.

When the nucleotide sequence of A4RS-947 was determined, this was identical with the sequence of 1,4-alpha-glucan branching enzyme [Accession: L07956] (SEQ ID NO:105). The amino acid sequence encoded by this gene is shown as SEQ ID NO:106. Its Northern blot exhibiting a shear stress-dependent increase of expression is shown in lane 53 of FIG. 2.

When the nucleotide sequence of A4RS-948 was determined, this was identical with the sequence of ferritin H [Accession: M11146] (SEQ ID NO:107). The amino acid sequence encoded by this gene is shown as SEQ ID NO:108. Its Northern blot exhibiting a shear stress-dependent increase of expression is shown in lane 54 of FIG. 2.

When the nucleotide sequence of A4RS-949 was determined, this was identical with the sequence of human PAST (HPAST) [Accession: AF001434] (SEQ ID NO:109). The amino acid sequence encoded by this gene is shown as SEQ ID NO:110. HPAST has homology with PAST-1 that is a fly-derived glycoprotein. Its Northern blot exhibiting a shear stress-dependent increase of expression is shown in lane 55 of FIG. 2.

### (3) Novel partial-length genes exhibiting a shear stress-dependent increase of expression

When the nucleotide sequence of A4RS-011 was determined, this was identical with a group of ESTs included in UniGene Hs. 71475. The sequence represented by SEQ ID NO: 111 could be obtained by joining the corresponding ESTs together. The amino acid sequence encoded by this sequence is shown as SEQ ID NO:112. The amino acid sequence encoded by this sequence does not show substantial homology with other known proteins. Its Northern blot exhibiting a shear stress-dependent increase of expression is shown in lane 56 of FIG. 2.

When the nucleotide sequence of A4RS-115 was determined, this was identical with a group of ESTs included in UniGene Hs. 3742. The sequence represented by SEQ ID NO:113 could be obtained by joining the corresponding ESTs together. The amino acid sequence encoded by this sequence is shown as SEQ ID NO:114. This gene has very high homology with rat SEC61 [Accession: M96630] and is considered to be the human ortholog thereof. Its Northern blots exhibiting a shear stress-dependent increase of expression are shown in lane 57 of FIG. 2 and lane 13 of FIG. 3.

When the nucleotide sequence of A4RS-143 was determined, this was identical with a group of ESTs included in UniGene Hs. 5307. The sequence represented by SEQ ID NO:115 could be obtained by joining the corresponding ESTs together. This sequence does not have an ORF consisting of 50 or more amino acids and is hence considered to be a non-translational region. Its Northern blots exhibiting a shear stress-dependent increase of expression are shown in lane 58 of FIG. 2 and lane 14 of FIG. 3.

When the nucleotide sequence of A4RS-171 was determined, no sequence identical exactly with it was found in the data banks. The nucleotide sequence is shown as SEQ ID NO: 116. This sequence does not have an ORF consisting of 50 or more amino acids and is hence considered to be a non-translational region. Its Northern blot exhibiting a shear stress-dependent increase of expression is shown in lane 59 of FIG. 2.

When the nucleotide sequence of A4RS-193 was determined, this was identical with a group of ESTs included in UniGene Hs. 112157. The sequence represented by SEQ ID NO:117 could be obtained by joining the corresponding ESTs together. The amino acid sequence encoded by this sequence is shown as SEQ ID NO: 118. The protein encoded by this sequence does not show substantial homology with other known proteins. Its Northern blots exhibiting a shear stress-dependent increase of expression are shown in lane 60 of FIG. 2 and lane 15 of FIG. 3.

When the nucleotide sequence of A4RS-280 was determined, this was identical with a group of ESTs included in UniGene Hs. 109017. The sequence represented by SEQ ID NO:119 could be obtained by joining the corresponding ESTs together. The amino acid sequence encoded by this sequence is shown as SEQ ID NO:120. This gene has as high as 87% homology with human ras-like protein TC10 [Accession: M31470] and is considered to be a novel human low-molecular-weight GTP-binding protein. Its Northern blots exhibiting a shear stress-dependent increase of expression are shown in lane 61 of FIG. 2 and lane 16 of FIG. 3.

When the nucleotide sequence of A4RS-402 was determined, this was identical with a group of ESTs included in UniGene Hs. 181077. The sequence represented by SEQ ID NO:121 could be obtained by joining the corresponding ESTs together. The amino acid sequence encoded by this sequence is shown as SEQ ID NO:122. Its Northern blots exhibiting a shear stress-dependent increase of expression are shown in lane 62 of FIG. 2 and lane 17 of FIG. 3.

When the nucleotide sequence of A4RS-533 was determined, this was identical with EST clones R07925 and T86046. The sequence represented by SEQ ID NO:123 could be obtained by joining the corresponding ESTs together. The amino acid sequence encoded by this sequence is shown as SEQ ID NO:124. The amino acid sequence encoded by this sequence does not show substantial homology with other known proteins. Its Northern blot exhibiting a shear stress-dependent increase of expression is shown in lane 63 of FIG. 2.

When the nucleotide sequence of A4RS-604 was determined, this was identical with a group of ESTs included in UniGene Hs. 34160. The sequence represented by SEQ ID NO:125 could be obtained by joining the corresponding ESTs together. The amino acid sequence encoded by this sequence is shown as SEQ ID NO:126. The protein encoded by this sequence does not show substantial homology with other known proteins. Its Northern blots exhibiting a shear stress-dependent increase of expression are shown in lane 64 of FIG. 2 and lane 18 of FIG. 4.

When the nucleotide sequence of A4RS-615. was determined, this was identical with a group of ESTs included in UniGene Hs. 193974. The sequence represented by SEQ ID NO:127 could be obtained by joining the corresponding ESTs together. The amino acid sequence encoded by this sequence is shown as SEQ ID NO:128. The protein encoded by this sequence does not show significant homology with other known proteins. Its Northern blot exhibiting a shear stress-dependent increase of expression is shown in lane 65 of FIG. 2.

When the nucleotide sequence of A4RS-619 was determined, this was identical with a group of ESTs included in UniGene Hs. 14512. The sequence represented by SEQ ID NO:129 could be obtained by joining the corresponding ESTs together. This sequence does not have an ORF consisting of 50 or more amino acids and is hence considered to be a non-translational region. Its Northern blot exhibiting a shear stress-dependent increase of expression is shown in lane 66 of FIG. 2.

When the nucleotide sequence of A4RS-626 was determined, no sequence identical exactly with it was found in the data banks. The nucleotide sequence is shown as SEQ ID NO:130. This sequence does not have an ORF consisting of 50 or more amino acids and is hence considered to be a non-translational region. Its Northern blots exhibiting a shear stress-dependent increase of expression are shown in lane 67 of FIG. 2 and lane 19 of FIG. 4.

When the nucleotide sequence of A4RS-676 was determined, this was identical with a group of ESTs included in UniGene Hs. 8881. The sequence represented by SEQ ID NO:131 could be obtained by joining the corresponding ESTs together. This sequence does not have an ORF consisting of 50 or more amino acids and is hence considered to be a non-translational region. Its Northern blot exhibiting a shear stress-dependent increase of expression is shown in lane 68 of FIG. 2.

When the nucleotide sequence of A4RS-679 was determined, no sequence identical exactly with it was found in the data banks. The nucleotide sequence is shown as SEQ ID NO: 132. This sequence does not have an ORF consisting of 50 or more amino acids and is hence considered to be a non-translational region. Its Northern blot exhibiting a shear stress-dependent increase of expression is shown in lane 69 of FIG. 2.

When the nucleotide sequence of A4RS-737 was determined, no sequence identical exactly with it was found in the data banks. The nucleotide sequence is shown as SEQ ID NO:133. This sequence does not have an ORF consisting of 50 or more amino acids and is hence considered to be a non-translational region. Its Northern blot exhibiting a shear stress-dependent increase of expression is shown in lane 70 of FIG. 2.

When the nucleotide sequence of A4RS-780 was determined, this was identical with a group of ESTs included in UniGene Hs. 34489. The sequence represented by SEQ ID NO:134 could be obtained by joining the corresponding ESTs together. This sequence does not have an ORF consisting of 50 or more amino acids and is hence considered to be a non-translational region. Its Northern blot exhibiting a shear stress-dependent increase of expression is shown in lane 71 of FIG. 2.

When the nucleotide sequence of A4RS-826 was determined, this was identical with a group of ESTs included in UniGene Hs. 7348. The sequence represented by SEQ ID NO:135 could be obtained by joining the corresponding ESTs together. The amino acid sequence encoded by this sequence is shown as SEQ ID NO:136. The protein encoded by this sequence does not show substantial homology with other known proteins. Its Northern blot exhibiting a shear stress-dependent increase of expression is shown in lane 72 of FIG. 2.

When the nucleotide sequence of A4RS-916 was determined, this was identical with a group of ESTs included in UniGene Hs. 105695. The sequence represented by SEQ ID NO:137 could be obtained by joining the corresponding ESTs together. The amino acid sequence encoded by this sequence is shown as SEQ ID NO:138. The protein encoded by this sequence does not show substantial homology with other known proteins. Its Northern blots exhibiting a shear stress-dependent increase of expression are shown in lane 73 of FIG. 2 and lane 20 of FIG. 4.

When the nucleotide sequence of A4RS-933 was determined, this was identical with EST clone AI391599. The sequence represented by SEQ ID NO:139 could be obtained by joining the corresponding ESTs together. The amino acid sequence encoded by this sequence is shown as SEQ ID NO:140. The protein encoded by this sequence does not show substantial homology with other known proteins. Its Northern blot exhibiting a shear stress-dependent increase of expression is shown in lane 74 of FIG. 2.

When the nucleotide sequence of A4RS-943 was determined, this was identical with a group of ESTs included in UniGene Hs. 186838. The sequence represented by SEQ ID NO:141 could be obtained by joining the corresponding ESTs together. The amino acid sequence encoded by this sequence is shown as SEQ ID NO:142. The amino acid sequence encoded by this sequence has a zinc finger motif and shows 67% homology with bird-derived zinc finger 5 protein [Accession: U51640]. Its Northern blot exhibiting a shear stress-dependent increase of expression is shown in lane 75 of FIG. 2.

### Example 5

### Cloning of full-length cDNAs

With respect to the novel DNAs exhibiting a shear stress-dependent increase of expression which were obtained in Example 3, the length of the insert was significantly shorter than the size of mRNA detected by Northern blotting in most cases. That is, the clones obtained from the subtraction library were judged to be partial cDNA fragments and not full-length cDNAs. Accordingly, with respect to eight of the novel DNAs, their full-length cDNAs were obtained again from a cDNA library.

### (1) Construction of a cDNA library using a λ phage vector

Three point two µg of oligo(dT)-XhoI primer (SEQ ID NO:160) was added to 4.8 µg of the HUVEC-derived poly(A)⁺ RNA obtained in Example 1. Then, distilled water was added thereto so as to make a volume of 6.8 µl. This solution was heated at 70°C for 10 minutes and then cooled rapidly by placing it on ice. To this solution, 4 µl of 5 x reverse transcriptase reaction buffer (attached to the enzyme), 2 µl of 100 mM DTT, 1.2 µl of a mixed dNTP solution [10 mM dATP, 10 mM dGTP, 10 mM dTTP, 5 mM 5-methyl dCTP], and 1 µl of [α-³²P]dATP (110 TBq/mmol; manufactured by Amersham Pharmacia Biotech) as a tracer were added on ice. After this mixture was kept at 37°C for 2 minutes, 5 µl of Superscript II RNase H⁻ Reverse Transcriptase (1,000 units; manufactured by Life Technologies) was added thereto and reacted at 44°C for 1 hour to synthesize cDNA. After the reaction was stopped by the addition of 0.8 µl of 0.5 M EDTA (pH 8.0), the reaction mixture was subjected to a phenol-chloroform treatment and a chloroform treatment, and precipitated with ethanol to recover a cDNA-mRNA hybrid. After the precipitate was dissolved in 17 µl of distilled water, 5 µl of 5 x reaction buffer (attached to the enzyme), 2.5 µl of 100 µM dGTP, and 0.5 µl of 15 units/µl terminal deoxynucleotidyl transferase (manufactured by Life Technologies) were added thereto. This mixture was reacted at 37°C for 30 minutes to add oligo-dG to the 3'-terminus of cDNA. After the reaction was stopped by the addition of 5 µl of 0.5 M EDTA (pH 8.0), the reaction mixture was subjected to a phenol-chloroform treatment and a chloroform treatment, and precipitated with ethanol. After the resulting precipitate was dissolved in 20.7 µl of distilled water, 1.5 µl of reaction buffer A [200 mM Tris-HCl (pH 8.75), 100 mM KCl, 100 mM (NH₄)₂SO₄, 20 mM MgSO₄, 1% Triton X-100, 1 mg/ml BSA], 1.5 µl of reaction buffer B [200 mM Tris·HCl (pH 9.2), 600 mM KCl, 20 mM MgCl₂], 0.3 µg of oligo(dC) NotI primer (SEQ ID NO:161), 0.75 µl of a 10 mM mixed dNTP solution, and 1.5 µl of 10 mM β-NAD were added thereto so as to make a total volume of 27.45 µl. After this mixture was kept at 55°C for 5 minutes, 1.5 µl of 5 units/µl ExTaq DNA polymerase (manufactured by Takara Shuzo Co., Ltd.), 0.75 µl of 100 units/µl Ampligase (manufactured by Epicentre), and 0.3 µl of 5 units/µl Hybridase (manufactured by Epicentre) were added thereto. Using a Thermal Cycler DNA Engine (manufactured by MJ Research), the temperature of this mixture was slowly reduced from 55°C to 35°C at a rate of 0.3°C per minute. Thereafter, the mixture was kept at 35°C for 15 minutes to anneal the primer to the template single-stranded cDNA. Thereafter, the mixture was kept at 72°C for 15 minutes to carry out the extension reaction of second-strand DNA. By repeating this annealing/extension cycle three more times, mRNA was degraded to make double-stranded cDNA. To the reaction mixture, 0.5 µl of 0.5 M EDTA (pH 8.0), 0.5 µl of 10% SDS, and 0.5 µl of 20 µg/µl Proteinase K were added. Then, the reaction mixture was kept at 45°C for 15 minutes to stop the reaction and inactivate the enzyme. Thereafter, the reaction mixture was subjected to a phenol-chloroform treatment and a chloroform treatment, and precipitated with ethanol. The resulting precipitate was dissolved in 44 µl of distilled water. Then, 5 µl of 10 x reaction buffer (attached to the enzyme) and 1 µl of XhoI (10 units/µl; manufactured by Takara Shuzo Co., Ltd.) were added thereto and reacted at 37°C for 2 hours to cleave the Xhol site in the oligo(dT)-XhoI primer. Then, 0.5 µl of 5 M NaCl and 1 µl of NotI (10 units/µl; manufactured by Takara Shuzo Co., Ltd.) were added to the reaction mixture and reacted at 37°C for 2 hours to cleave the NotI site in the oligo(dC)-NotI primer. In order to remove short cDNA fragments of not greater than 400 bp size, and unreacted primers and nucleotides, the reaction mixture was placed on a SizeSep-400 spun column (manufactured by Amersham Pharmacia Biotech) equilibrated with TE buffer, and centrifuged at 400 x g for 2 minutes. The resulting eluate was purified by subjecting it to a phenol-chloroform treatment and a chloroform treatment. Eight µl of 10 x reaction buffer (manufactured by Takara Shuzo Co., Ltd.), 62 µl of distilled water, and 50 units (5 µl) of XhoI were added to 5 µg (5 µl) of cloning vector λZAPII (manufactured by Stratagene), and reacted at 37°C for 4 hours. Then, 1 µl of 5 M NaCl and 50 units (5 µl) of NotI were added to the reaction mixture, and further reacted at 37°C for 4 hours. Thus, the XhoI and NotI sites of the vector were cleaved. Then, 9 µl of 10 x reaction buffer (attached to the enzyme) and 0.025 unit of temperature-sensitive alkaline phosphatase (manufactured by Life Technologies) were added to the reaction mixture and reacted at 65°C for 15 minutes to dephosphorylate the 5'-termini of the XhoI-cleaved end and NotI-cleaved end of the vector. After the reaction was stopped by the addition of 10 µl of a reaction stopper (attached to the enzyme), the reaction mixture was subjected to a phenol-chloroform treatment and a chloroform treatment, and the vector was recovered by ethanol precipitation. The aforesaid purified cDNA was added to 0.25 µg of the vector, followed by ethanol precipitation. The recovered vector DNA and cDNA were dissolved in 4 µl of a ligase buffer [100 mM Tris-HCl (pH 7.6), 5 mM MgCl₂, 300 mM NaCI], and 4 µl of solution B in a Ligation Kit Ver. 1 (manufactured by Takara Shuzo Co., Ltd.). This mixture was reacted at 26°C for 10 minutes to ligate cDNA to vector DNA. Using 4 µl portions of the reaction mixture, packaging was carried out using a λ Phage Packaging Extract Gigapack β Gold (manufactured by Stratagene). Specifically, reagents were used and the procedure for packaging was carried out according to the manual attached to the kit. Escherichia coli XL1-Blue MRF' strain was infected with the resulting phage, and its titer was measured. Moreover, the phage was multiplied on a plate and recovered in SM buffer (its composition is described in the manual of Stratagene). Thus, the cDNA library was amplified once to obtain a final cDNA library. Specifically, the procedures for titer measurement and library amplification were carried out according to the manual attached to the λ phage packaging kit.

### (2) Obtaining of full-length cDNAs by plaque hybridization

With respect to the library constructed in step (1), plaque DNAs were blotted to a nylon membrane Hybond N+ (manufactured by Amersham Pharmacia Biotech). The plasmids derived from the subtraction library obtained in Example 2 were used as templates, and primers specific for each genes were synthesized and used. After a PCR DIG labeling mix (manufactured by Boehringer Mannheim) was added, PCR was carried out to amplify and label each gene-specific fragments. Using these DNA fragments as probes, hybridization and the detection of positive plaques were carried out according to the manual of Boehringer Mannheim. Positive plaques were amplified in SM buffer and formed into plasmids using helper phage ExAssist (manufactured by Stratagene). Specifically, the procedure for plasmid formation was carried out according to the manual of Stratagene.

### (2) Determination of nucleotide sequence

The nucleotide sequence of each of the cDNA clones thus obtained was determined by means of a 377 DNA Sequencer (manufactured by Perkin Elmer). Specifically, the determination of the nucleotide sequence was carried out with a Dye Primer Cycle Sequencing FS Ready Reaction Kit according to the manual attached to the Kit (manufactured by Perkin Elmer). Moreover, this nucleotide sequence was translated into an amino acid sequence on a three-frame basis and examined for the presence of an open reading frame (ORF).

### (3) Homology analysis of full-length cDNAs

### ①A4RS-002

With respect to the full-length cDNA clone pfA4RS-002-1 obtained as a result of plaque hybridization, the whole nucleotide sequence of cDNA was determined and the resulting nucleotide sequence is shown as SEQ ID NO:143. Escherichia coli DH5α strain having clone pfA4RS-002-1 introduced thereinto (Escherichia coli DH5α/pfA4RS-002-1) was internationally deposited on August 5, 1999 with the National Institute of Bioscience and Human-Technology, the Agency of Industrial Science and Technology (Higashi 1-1-3, Tsukuba City, Ibaraki Prefecture, Japan) under the Budapest Treaty, and assigned the accession number FERM BP-6822. In the nucleotide sequence of A4RS-002, an ORF consisting of 390 amino acids was observed (its amino acid sequence is shown as SEQ ID NO:144). As a result of homology analysis, this amino acid sequence was found to show significant homology with proteins belonging to the immunoglobulin family. Among others, this amino acid sequence show high homology with A33 antigen that is a specific marker for human colon carcinoma [Proc. Natl. Acad. Sci. USA, 94, 469(1997)] and CAR (coxackie and adenovirus receptor) that is a virus receptor protein [Science, 275, 1320(1997)]. Judging from their primary structure, these factors are presumed to be a type I membrane protein. According to a hydrophilicity analysis on the basis of the amino acid sequence, 29 residues at the amino-terminus of A4RS-002 is estimated to be a secretion signal, and a sequence extending from the 249th to 270th amino acid is considered to be a highly hydrophobic transmembrane region. Since ICAM-1 and VCAM-1 belonging to the immunoglobulin family exhibit a shear stress-dependent alteration of expression, A4RS-002 is presumed to belong to the immunoglobulin family and function as a membrane protein. Its Northern blots exhibiting a shear stress-dependent increase of expression are shown in lane 76 of FIG. 2 and lane 21 of FIG. 4.

### ② A4RS-049

With respect to the full-length cDNA clone pfA4RS-049-1 obtained as a result of plaque hybridization, the whole nucleotide sequence of cDNA was determined and the resulting nucleotide sequence is shown as SEQ ID NO:145. Escherichia coli DH5α strain having clone pfA4RS-049-1 introduced thereinto (Escherichia coli DH5α/pfA4RS-049-1) was internationally deposited on August 5, 1999 with the National Institute of Bioscience and Human-Technology, the Agency of Industrial Science and Technology (Higashi 1-1-3, Tsukuba City, Ibaraki Prefecture, Japan) under the Budapest Treaty, and assigned the accession number FERM BP-6823. In the nucleotide sequence of A4RS-049, an ORF consisting of 881 amino acids was observed (its amino acid sequence is shown as SEQ ID NO:146). As a result of homology analysis, the protein encoded by A4RS-049 showed significant homology not only with mouse-derived 3BP-1 (SH3 domain binding protein) [EMBO, J. 14, 3127(1995)], but also with various GTPase-activating protein (GAPs) such as rhoGAP and Abr. GAPs are a family of proteins controlling the GTPase activity of low-molecular-weight GTP-binding proteins such as ras and rab, and A4RS-049 shows homology with GAPs (e.g., rho and rac) specific for a subfamily considered to participate in regulation of cytoskeleton. In the amino acid sequence encoded by A4RS-049, the GTPase-activating domain conserved among known GAPs is present. Accordingly, A4RS-049 is presumed to function as a GAP. Moreover, a nematode-derived gene [Accession: Z73425] and a yeast-derived gene [Accession: Z97210], which are registered in databases but have an unknown function, show significant homology with the protein encoded by A4RS-049. Thus, A4RS-049 is expected to be a gene conserved well in the process of evolution. Its Northern blots exhibiting a shear stress-dependent increase of expression are shown in lane 77 of FIG. 2 and lane 22 of FIG. 4.

### ③ A4RS-230

With respect to the full-length cDNA clone pfA4RS-230-2 obtained as a result of plaque hybridization, the whole nucleotide sequence of cDNA was determined and the resulting nucleotide sequence is shown as SEQ ID NO:147. Escherichia coli DH5α strain having clone pfA4RS-230-2 introduced thereinto (Escherichia coli DH5α/pfA4RS-230-2) was internationally deposited on August 5, 1999 with the National Institute of Bioscience and Human-Technology, the Agency of Industrial Science and Technology (Higashi 1-1-3, Tsukuba City, Ibaraki Prefecture, Japan) under the Budapest Treaty, and assigned the accession number FERM BP-6824.

In the nucleotide sequence of A4RS-230, an ORF consisting of 322 amino acids was observed (its amino acid sequence is shown as SEQ ID NO:148). As a result of homology analysis, the protein encoded by A4RS-230 shows as high as 83% homology with mouse myeloid upregulated protein [Accession: 035682] and is considered to be a human counterpart thereof. However, its part on the C-terminal side is substantially different. As to mouse myeloid upregulated protein, only the sequence is registered in a database and its function is unknown. According to a hydrophilicity analysis on the basis of the amino acid sequence, the protein encoded by A4RS-230 has very high hydrophobicity and may hence function as a membrane protein. However, a sequence judged to be a signal sequence is not present at the N-terminus. Its Northern blots exhibiting a shear stress-dependent increase of expression are shown in lane 78 of FIG. 2 and lane-23 of FIG. 4.

### ④ A4RS-239

With respect to the full-length cDNA clone pfA4RS-239-2 obtained as a result of plaque hybridization, the whole nucleotide sequence of cDNA was determined and the resulting nucleotide sequence is shown as SEQ ID NO:149. Escherichia coli DH5α strain having clone pfA4RS-239-2 introduced thereinto (Escherichia coli DH5α/pfA4RS-239-2) was internationally deposited on August 5, 1999 with the National Institute of Bioscience and Human-Technology, the Agency of Industrial Science and Technology (Higashi 1-1-3, Tsukuba City, Ibaraki Prefecture, Japan) under the Budapest Treaty, and assigned the accession number FERM BP-6825.

In the nucleotide sequence of A4RS-239, an ORF consisting of 663 amino acids was observed (its amino acid sequence is shown as SEQ ID NO:150). As a result of homology analysis, the protein encoded by A4RS-239 showed low but significant homology with various GAPs such as rhoGAP and Abr, similarly to the above-described A4RS-049. However, A4RS-239 and A4RS-049 are different DNAs. In the amino acid sequence encoded by A4RS-239, the GTPase-activating domain conserved among known GAPs is present. Accordingly, A4RS-239 is presumed to function as a GAP. Its Northern blots exhibiting a shear stress-dependent increase of expression are shown in lane 79 of FIG. 2 and lane 24 of FIG. 3.

### ⑤ A4RS-242

With respect to the full-length cDNA clone pfA4RS-242-1 obtained as a result of plaque hybridization, the whole nucleotide sequence of cDNA was determined and the resulting nucleotide sequence is shown as SEQ ID NO:151. Escherichia coli DH5α strain having clone pfA4RS-242-1 introduced thereinto (Escherichia coli DH5α/pfA4RS-242-1) was internationally deposited on August 5, 1999 with the National Institute of Bioscience and Human-Technology, the Agency of Industrial Science and Technology (Higashi 1-1-3, Tsukuba City, Ibaraki Prefecture, Japan) under the Budapest Treaty, and assigned the accession number FERM BP-6826. In the nucleotide sequence of A4RS-242, an ORF consisting of 863 amino acids was observed (its amino acid sequence is shown as SEQ ID NO:152). As a result of homology analysis, the amino-terminal half of the protein encoded by A4RS-242 is identical with approximately the full length of the product of the gene ehb10. However, a part of A4RS-242 corresponding to the half on the carboxyl-terminal side is not present in ehb10. That is, they are considered to be splicing variants. ehb10 is one of the proteins obtained by expression cloning, as factors binding to the EH domain considered to participate in the protein interaction of Eps15 (the substrate for EGF receptor) [Genes & Dev., 11, 2239(1997)], but its function is unknown. However, the motif required for binding to the EH domain is also present in A4RS-242. Its Northern blots exhibiting a shear stress-dependent increase of expression are shown in lane 80 of FIG. 2 and lane 25 of FIG. 4.

### ⑥ A4RS-491

With respect to the full-length cDNA clone pfA4RS-491-1 obtained as a result of plaque hybridization, the whole nucleotide sequence of cDNA was determined and the resulting nucleotide sequence is shown as SEQ ID NO:153. Escherichia coli DH5α strain having clone pfA4RS-491-1 introduced thereinto (Escherichia coli DH5α/pfA4RS-491-1) was internationally deposited on August 5, 1999 with the National Institute of Bioscience and Human-Technology, the Agency of Industrial Science and Technology (Higashi 1-1-3, Tsukuba City, Ibaraki Prefecture, Japan) under the Budapest Treaty, and assigned the accession number FERM BP-6827. In the nucleotide sequence of A4RS-491, an ORF consisting of 331 amino acids was observed (its amino acid sequence is shown as SEQ ID NO:154). As a result of homology analysis, the protein encoded by A4RS-491 was identical with an amino acid sequence [Accession: 043334] registered in a database as a human hypothetical protein over a wide range. However, this hypothetical protein consists of 393 amino acids, and it has been found that its 88th to 148th amino acids are not contained in the amino acid sequence encoded by A4RS-491. That is, they are considered to be splicing variants. The protein encoded by A4RS-491 shows substantial homology with nematode-derived glycerophosphodiester phosphodiesterase [Accession: Z78198] and bacterium-derived glycerophosphodiester phosphodiesterase [Accession: E69827], and has been found to be a gene conserved well in the process of evolution. It is known that bacterium-derived glycerophosphodiester phosphodiesterase is present on membranes. According to a hydrophilicity analysis on the basis of the amino acid sequence encoded by A4RS-491, an amino acid sequence extending from the 1st to 26th amino acid in SEQ ID NO:154 is presumed to be a signal peptide. Its Northern blots exhibiting a shear stress-dependent increase of expression are shown in lane 81 of FIG. 2 and lane 26 of FIG. 4.

### ⑦ A4RS-578

With respect to the full-length cDNA clone pfA4RS-578-1 obtained as a result of plaque hybridization, the whole nucleotide sequence of cDNA was determined and the resulting nucleotide sequence is shown as SEQ ID NO:155. Escherichia coli DH5α strain having clone pfA4RS-578-1 introduced thereinto (Escherichia coli DH5α/pfA4RS-578-1) was internationally deposited on August 5, 1999 with the National Institute of Bioscience and Human-Technology, the Agency of Industrial Science and Technology (Higashi 1-1-3, Tsukuba City, Ibaraki Prefecture, Japan) under the Budapest Treaty, and assigned the accession number FERM BP-6828. In the nucleotide sequence of A4RS-578, an ORF consisting of 541 amino acids was observed (its amino acid sequence is shown as SEQ ID NO:156). As a result of homology analysis, the protein encoded by A4RS-578 showed the highest homology with the amino acid sequence [Accession: Z95559] of a protein of unknown function registered as a nematode-derived hypothetical protein, and then showed significant homology with rat brain finger protein (BFP) [Biochem. Biophys. Res. Commun., 240, 8(1997)]. Rat BFP was cloned as a novel gene having the RING finger motif as a kind of zinc finger motif, and it has been reported that this gene is expressed brain-specifically and that the expression of this gene may be induced at the stage of differentiation into nerve cells. However, a sequence judged to be the RING finger motif is not present in the amino acid sequence encoded by A4RS-578. The protein encoded by A4RS-578 also shows significant homology with various GTP-binding proteins, and has two of the three motifs possessed in common by many GTP-binding proteins. Since the existence of GTP-binding proteins having only two motifs has bee reported, there is a possibility that the protein encoded by A4RS-578 functions as a GTP-binding protein. Its Northern blots exhibiting a shear stress-dependent increase of expression are shown in lane 82 of FIG. 2 and lane 27 of FIG. 4.

### ⑧ A4RS-829

With respect to the full-length cDNA clone pfA4RS-829-1 obtained as a result of plaque hybridization, the whole nucleotide sequence of cDNA was determined and the resulting nucleotide sequence is shown as SEQ ID NO:157. Escherichia coli DH5α strain having clone pfA4RS-829-1 introduced thereinto (Escherichia coli DH5α/pfA4RS-829-1) was internationally deposited on August 5, 1999 with the National Institute of Bioscience and Human-Technology, the Agency of Industrial Science and Technology (Higashi 1-1-3, Tsukuba City, Ibaraki Prefecture, Japan) under the Budapest Treaty, and assigned the accession number FERM BP-6829. In the nucleotide sequence of A4RS-829, an ORF consisting of 173 amino acids was observed (its amino acid sequence is shown as SEQ ID NO:158). As a result of homology analysis, the protein encoded by A4RS-829 showed substantial homology with the amino acid sequences of proteins of unknown functions registered as hypothetical proteins, such as an arabidopsis-derived protein [Accession: 048707], a nematode-derived protein [Accession: Q20340] and a yeast-derived protein [Accession: Q03677], and was found to be a gene conserved well in the process of evolution. Its Northern blots exhibiting a shear stress-dependent increase of expression are shown in lane 83 of FIG. 2 and lane 28 of FIG. 4.

### Example 6

### Production of a recombinant protein of A4RS-002

### (1) Construction of an expression plasmid

To 2 µg of pfA4RS-002-1 obtained in Example 5, 5 µl of 10·reaction buffer (attached to the enzyme), 1 µl of XhoI (10 units/µl; manufactured by Takara Shuzo Co., Ltd.), and distilled water were added so as to make a total volume of 50 µl. This mixture incubated at 37°C for 2 hours to digest the cDNA completely. Then, 0.5 µl of 5 M NaCl and 1 µl of NotI (10 units/µl; manufactured by Takara Shuzo Co., Ltd.) were added to the reaction mixture. This mixture incubated at 37°C for 2 hours to digest the cDNA completely. The reaction mixture was subjected to a phenol-chloroform treatment and a chloroform treatment, and then precipitated with ethanol. After the resulting precipitate was dissolved in 20 µl of distilled water, 3 µl of 10 x blunting buffer (attached to the enzyme), 6 µl of a 2.5 mM mixed dNTP solution, and 1 µl of Klenow fragment (manufactured by Takara Shuzo Co., Ltd.) were added thereto. This mixture was incubated at 37°C for 1 hour to carry out the blunting of restriction enzyme-treated ends. The reaction mixture was subjected to a phenol-chloroform treatment and a chloroform treatment, and then precipitated with ethanol. After the resulting precipitate was dissolved in 5 µl of distilled water, 0.4 µg and 0.3 µg, respectively, of SfiI linkers (5'-CTTTAGAGCAC-3', 5'-CTCTAAAG-3') were added thereto so as to make a volume of 6 µl. Twelve µl of solution I and 6 µl of solution II of a Ligation Kit Ver. 2 (manufactured by Takara Shuzo Co., Ltd.) were added thereto, and the resulting mixture was incubated at 16°C overnight to carry out linker ligation. The total amount of the reaction mixture was electrophoresed through 0.8% agarose gel, and the desired fragments were recovered using a QIAEX II Gel Extraction Kit (manufactured by QIAGEN). The procedure therefor was carried out according to the manual attached to the kit. The recovered DNA fragments were dissolved in 10 µl of distilled water. To this insert DNA, an animal cell expression plasmid vector pAMo [J. Biol. Chem., 268, 22782(1993); also called pAMoPRC3Sc (Japanese Published Unexamined Patent Application No. 336963/93)], which had been linearized with SfiI and similarly recovered from an agarose gel, was added in a molar amount equal to 1/5 of that of the insert, and Ligation High (manufactured by Toyobo Co., Ltd.) in a volume equal to that of the solution. This mixture was incubated at 16°C for 3 hours to ligate the insert with linkers to the vector, and then introduced into competent-cell Escherichia coli MW294. After introduction, LB agar medium containing 50 µg/ml ampicillin was inoculated with the bacterial suspension and incubated at 37°C overnight to form colonies. The resulting colonies were randomly picked up, and the plasmid was obtained from each colony and examined for the presence or absence of the insert by a restriction enzyme treatment. With respect to the colonies having the insert, the direction of the insert was examined. Using one clone, pAMo-002, having the desired directivity, the plasmid was mass-prepared using a QIAGEN Plasmid Midi Kit (manufactured by QIAGEN). The procedure therefor was carried out according to the manual attached to the kit. This plasmid was sterilely precipitated with ethanol and then dissolved in distilled water to a concentration of 1 µg/µl. The above-described construction of pAMo-002 is illustrated in FIG. 5.

### (2) Introduction of the recombinant plasmid into cultured animal cells

Namalwa KJM-1 [Cytotechnology, 1, 151(1988)], which is a host cell for gene expression, was collected by centrifugation, washed with 10 ml of K-PBS [13.7 mM KCI, 0.27 mM NaCl, 0.81 mM Na₂HPO₄, 0.15 mM KH₂PO₄, 0.4 mM MgCl₂], and suspended in cooled K-PBS so as to have a density of 8 x 10⁶ cells/ml. Two hundred µl (1.6 x 10⁶ cells) of this cell suspension was mixed with 4 µl (4 µg) of the plasmid DNA prepared in step (1), and this mixture was quickly transferred to a chamber (manufactured by BIO-RAD) which had previously been cooled on ice. Then, using a Gene Pulser (manufactured by BIO-RAD), electroporation was carried out by the application of a voltage of 0.35 kV, 125 µF. Thereafter, the chamber was quickly placed on ice, and-the electroporated cells were transferred to a flask containing 8 ml of RPMI1640 medium (manufactured by Nissui Seiyaku Co., Ltd.). After the flask was incubated for 24 hours under conditions include 37°C and 5% CO₂, G-418 as an agent for selection was added thereto so as to give a final concentration of 0.5 mg/ml. Gene-introduced cells were selected by continuing the incubation for one more week. As a control, KJM-1 cells into which only pAMo vector having no insert was introduced were also prepared.

### Example 7

### Cloning of full-length cDNAs (2)

Similarly to Example 5, with respect to three novel partial cDNA fragments obtained from the subtraction library, full-length cDNAs were obtained from full-length cDNA libraries derived from human adipose tissue or Kato III.

### (1) Construction of full-length cDNA libraries derived from human adipose tissue or KatoIII cells

mRNA was extracted from human adipose tissue according to the method described in the paper (J. Sambrook, E.F. Fritsch & T. Maniatis, Molecular Cloning, Second Edition, Cold Spring Harbor Laboratory Press, 1989). Moreover, poly(A)⁺ RNA was purified with oligo-dT cellulose.

Similarly, mRNA was extracted from KatoIII cells according to the method described in the paper (J. Sambrook, E.F. Fritsch & T. Maniatis, Molecular Cloning, Second Edition, Cold Spring Harbor Laboratory Press, 1989). Moreover, poly(A)⁺ RNA was purified with oligo-dT cellulose.

From each poly(A)⁺ RNA, a cDNA library was constructed according to an oligo-cap method [M. Maruyama and S. Sugano, Gene, 138, 171-174(1994)]. Using an oligo-cap linker (SEQ ID NO:162) and an oligo-dT primer (SEQ ID NO:163), BAP (Bacterial Alkaline Phosphatase) treatment, TAP (Tabacco Acid Phosphatase) treatment, RNA ligation, the synthesis of first-strand cDNA, and the removal of RNA were carried out according to the methods described in the paper [Suzuki & Kanno, Tanpakushitsu-Kakusan-Koso (in Japanese), 41:197-201(1996); Y. Suzuki, Gene, 200, 149-156(1997)]. Then, the resulting cDNA was converted to double-stranded cDNA by PCR using two primers [a 5'-terminal sense primer (SEQ ID NO:164) and a 3'-terminal antisense primer (SEQ ID NO:165)], and then cleaved with SfiI. This PCR was carried out in such a way that, using a commercially available GeneAmp XL PCR Kit (manufactured by Perkin Elmer), the reaction mixture was heat-treated at 95°C for 5 minutes, subjected 12 times to a reaction cycle comprising heating at 95°C for 1 minute, 58°C for 1 minute, and at 72°C for 10 minutes, and then kept at 4°C. Thereafter, a cDNA library was constructed by cloning the cDNA into a DraIII-cleaved pME18SFL3 vector [Accession: AB009864; expression vector, 3392 bp] with the fixed directivity of the cDNA.

### (2) Determination of the full-length cDNA sequences

With respect to the plasmid DNAs of clones obtained from the cDNA libraries prepared in step (1), each cDNA clone was subjected to an in vitro transposon (hereinafter abbreviated as Tn) transposition reaction by using a GSP-1 Genome Priming System (manufactured by NEB). pGPS1.1 (manufactured by NEB) was used as the Tn donor. After completion of the Tn transposition reaction, a portion of the DNA sample was taken and used to transform Escherichia coli. Typically, 16 Tn-inserted clones were picked up for each cDNA clone. With respect to the plasmid DNAs of clones thus obtained, the full-length cDNA sequences were determined in the same manner as in Example 5, by using Primer N (SEQ ID NO:166) and Primer S (SEQ ID NO:167) as primers.

### (3) Novel full-length genes exhibiting a shear stress-dependent increase of expression

Using the sequence of A4RS-011 obtained from the subtraction library in Example 3 as a query, the cDNA sequences obtained in step (2) were searched by means of BLAST program [Altschul, Stephen F., Thomas L. Madden, Alejandro A. Schaffer, Jinghui Zhang, Zheng Zhang, Webb Miller, and David j. Lipman, Nucleic Acids Res., 25, 3389-3402(1997)]. As a result, the sequence of A4RS-011 was identical with C-KAT07969 (SEQ ID NO:168). Among the ORFs of this cDNA sequence, the longest translated amino acid sequence was regarded as the amino acid sequence (121-1062; SEQ ID NO:169) encoded by the cDNA sequence of C-KAT07969. This amino acid sequence does not show substantial homology with other known proteins. Its Northern blot exhibiting a shear stress-dependent increase of expression is shown in panel 56 of FIG. 2.

Using the sequence of A4RS-604 obtained from the subtraction library in Example 3 as a query, the cDNA sequences obtained in step (2) were searched by means of BLAST program. As a result, the sequence of A4RS-604 was identical with the sequence of C-ADKA02341 (SEQ ID NO:170). This sequence is identical with a portion of the sequence of H. sapiens mRNA for myosin-I beta [Accession: X98507]. Among the ORFs of this cDNA sequence, the longest translated amino acid sequence was regarded as the amino acid sequence (SEQ ID NO:171) encoded by the cDNA sequence of C-ADKA02341. Its Northern blots exhibiting a shear stress-dependent increase of expression are shown in panel 64 of FIG. 2 and panel 18 of FIG. 4.

Using the sequence of A4RS-619 obtained from the subtraction library in Example 3 as a query, the cDNA sequences obtained in step (2) were searched by means of BLAST program. As a result, the sequence of A4RS-619 was identical with the sequence of C-hep01279 (SEQ ID NO:172). Among the ORFs of this cDNA sequence, the longest translated amino acid sequence was regarded as the amino acid sequence (SEQ ID NO:173) encoded by the cDNA sequence of C-hep01279. This amino acid sequence does not show substantial homology with other known proteins. Its Northern blot exhibiting a shear stress-dependent increase of expression is shown in panel 66 of FIG. 2.

### Example 8

### Detection of the apoptosis-suppressing activity of A4RS-041

In order to investigate the function of genes exhibiting a shear stress-dependent increase of expression which were obtained from the subtraction library, the following experiments were carried out with respect to a gene of unknown function, A4RS-041, having homology with the gene LFG capable of suppressing Fas-mediated apotosis.

### (1) Construction of a recombinant virus vector

Using a plasmid having the full-length A4RS-041 (SEQ ID NO:7) as a template, the part of the cDNA sequence which encodes the protein of A4RS-041 was specifically amplified by PCR. That is, in a PCR tube, 20 ng of the template plasmid DNA, 25 pmol of a 5'-terminal sense primer having the HindIII site added thereto (SEQ ID NO: 174), 25 pmol of a 3'-terminal antisense primer having the ClaI site added thereto (SEQ ID NO: 175), 5 µl of 10 x reaction buffer (attached to the enzyme), 5 µl of a 2 mM dNTP solution, and 0.5 µl of KOD DNA polymerase (2.5 units/µl; manufactured by Toyobo Co, Ltd.) were mixed, and sterilized water was added thereto so as to make a volume of 50 µl. This mixture was heated at 98°C for 15 seconds, at 65°C for 2 seconds, and at 74°C for 30 seconds. This cycle was repeated 25 times to amplify the cDNA. The resulting amplified fragment of the full-length A4RS-041 was purified by cleaving its ends with HindIII and ClaI, and ligated to virus vector pCLNCX (manufactured by IMGENEX) which had previously been cleaved with HindIII and ClaI. As a result, there was constructed a recombinant virus vector pCLNC041 with which the expression of A4RS-041 is induced by CMV promoter. With respect to the resulting recombinant virus vector pCLNC041, the nucleotide sequence of its inserted fragment part was determined. Thus, it was confirmed that no nucleotide substitution was caused by PCR. As a control, pCLNCGFP was constructed by inserting EFGP (enhanced green fluorescent protein; manufactured by Clontech) into the HindIII and ClaI sites of pCLNCX in the same manner.

### (2) Preparation of HeLa cells expressing A4RS-041 to be highly and stably

Recombinant viruses were produced by introducing each of the recombinant virus vectors constructed in step (1) into 293 cells for virus production. For the transfection of 293 cells with pCLNC041 or pCLNCGFP, a TransFast (manufactured by Promega) was used. The procedure therefor was carried out according to the attached manual. The procedures for virus production and for the infection of HeLa cells were carried out according to the manual attached to the virus vector (manufactured by IMGENEX) used.

Two days after infection, 300 µg/ml of G418 (manufactured by life Technologies) was added to the HeLa cells and the incubation was continued, so that uninfected cells were selectively eliminated. According to this procedure, there were obtained transformants expressing A4RS-041 or GFP to be highly and stably.

### (3) Detection of an apotosis-suppressing activity

Apoptosis was induced by adding 100 ng/ml of anti-Fas monoclonal antibody CH-11 (manufactured by MBL) to the stable transformants of HeLa cells obtained in step (2) (i.e., the stable transformants of HeLa cells expressing the expression of A4RS-041 or GFP as a control). Twenty-four (24), 36 and 48 hours after the start of induction, the survival rate of the cells was measured by staining with trypan blue. For this purpose, the survival rate was measured for both suspended cells and adhering cells. All experiments were carried out in duplicate, and averages and standard deviations were obtained. The results are shown in FIG. 6A. Moreover, when the antibody concentration was altered to 10, 50, 100 and 500 ng/ml, the survival rate after 36 hours was measured. The results are shown in FIG. 6B. In the HeLa cells having A4RS-041 introduced thereinto (represented by ● in FIG. 6), a significant increase in survival rate was observed at all points as compared with the HeLa cells having GFP (control) introduced thereinto (represented by ■ in FIG. 6). Thus, it has been found that, at least in HeLa cells, A4RS-041 has an activity for suppressing Fas-mediated apotosis.

### Example 9

### Analysis of the distribution of expression of A4RS-041

With respect to A4RS-041 that was found to have an apoptosis-suppressing activity in Example 8, the following experiments were carried out in order to examine its sites of expression in human tissues.

### (1) Analysis of the expression of A4RS-041 in human normal tissues

Using primers specific for A4RS-041 (SEQ ID NO:176,177) and a PCR DIG Labeling Mix (manufactured by Boehringer Mannheim), a template comprising the A4RS-041-containing plasmid obtained in Example 2 was subjected to PCR. Thus, a DIG-labeled A4RS-041 specific fragment was prepared. Using this DNA fragment as a probe, hybridization was carried out with a Human Multiple Tissue Northern Blot (manufactured by Clontech) on which RNAs derived from 8 human tissues had been blotted. After washing, chemiluminescence signals were detected using a DIG luminescence detection kit (manufactured by Boehringer Mannheim). The procedure therefor was carried out according to the manual attached to the kit. As shown in panel A of FIG. 7, signals specific for A4RS-041 were detected in the vicinity of about 2.5 kb. In Lanes 1 to 8, 2 µg each of poly(A)⁺ RNAs derived from spleen, kidney, skeletal muscle, liver, lung, placenta, brain and heart had been electrophoresed. Although signals were observed in all lanes, the signal in lane 7 (brain) was weak. Thus, it has been found that the expression of A4RS-041 is relatively low in the brain. On the other hand, it has been reported that the expression of LFG is very high in the brain and low in the periphery [Proc. Natl. Acad. Sci. USA, 22, 12673-12678(1999)]. This suggests that A4RS-041 and LFG function tissue-specifically.

### (2) Investigation on the expression of A4RS-041 and LFG in human vascular endothelial cells and brain

Using a template comprising 1 µg of the HUVEC (having no shear stress applied thereto)-derived poly(A)⁺ RNA obtained in Example 2 or 1 µg of human brain-derived poly(A)⁺ RNA (manufactured by Clontech), single-stranded cDNA was synthesized using a Superscript Preamplification System (manufactured by Life Technologies). The procedure therefor was carried out according to the manual attached to the kit. The finally obtained cDNA solution was diluted to 5 ml and used for PCR. Using these cDNAs as templates, PCR was carried out using primers specific for A4RS-041 (SEQ ID NO:176,177), LFG (SEQ ID NO:178,179) and G3PDH (SEQ ID NO:180,181). The reaction mixture contained 5 µl of a cDNA solution, 2 µl of 10 x reaction buffer (attached to the enzyme), 1.6 µl of a 2.5 mM dNTP solution, 1 µl of dimethyl sulfoxide, 10 pmol each of sense and antisense primers, and 0.1 µl of GeneTaq DNA polymerase (5 units/µl; manufactured by Nippon Gene Co., Ltd.), and sterilized water was added thereto so as to make a total volume of 20 µl. After the template and the primers were denatured by heating at 94°C for 1 minute, a cycle comprising heating at 94°C for 1 minute, at 60°C for 1 minute, and at 72°C for 1 minute was repeated. The number of cycles was 33 for A4RS-041 and LFG, and 24 for G3PDH. The reaction mixture was kept at 72°C for 10 minutes and then cooled to 4°C. One-half of the resulting PCR product was subjected to 1.8% agarose electrophoresis. The results are shown in panel B of FIG. 7. In lane 1, a 100 bp ladder (manufactured by Life Technologies) was electrophoresed as a size marker. Lanes 2, 4 and 6 show the PCR products obtained with HUVEC-derived cDNA, and Lanes 3, 5 and 7 show the PCR products obtained with human brain-derived cDNA. Moreover, lanes 2 and 3 show the PCR products obtained with A4RS-041-specific primers, lanes 4 and 5 show the PCR products obtained with LFG-specific primers, and lanes 6 and 7 show the PCR products obtained with G3PDH-specific primers.

The band of A4RS-041 is amplified in both HUVECs (lane 2) and the brain (lane 3), indicating that A4RS-041 is expressed in both of them. Its expression level in the brain tends to be lower than in HUVECs. On the other hand, LFG is very strongly expressed in the brain(lane 5), but the band of LFG is not amplified at all in HUVECs (lane 4), and this indicates that LFG is not expressed in HUVECs.

From the above-described results, it is believed that the factor involved in the suppression of apoptosis in endothelial cells is not LFG but A4RS-041.

The homology between the amino acid sequences of A4RS-041 and LFG (human-derived) is shown in FIG. 8. They are judged to be homologous proteins having 48.9% (152/311) identity. However, it has been found that a portion corresponding to about one-third on the N-tenninal side has considerably low homology.

### FREE TEXT FOR SEQUENCE LISTING

SEQ ID NO:159 - Description of artificial sequence: Artificial synthetic primer sequence
SEQ ID NO:160- Description of artificial sequence: Artificial synthetic primer sequence
SEQ ID NO:161 - Description of artificial sequence: Artificial synthetic primer sequence
SEQ ID NO:162 - Description of artificial sequence: Oligo-cap linker sequence
SEQ ID NO:163 - Description of artificial sequence: Oligo-dT primer sequence
SEQ ID NO:164 - Description of artificial sequence: Artificial synthetic primer sequence
SEQ ID NO:165 - Description of artificial sequence: Artificial synthetic primer sequence
SEQ ID NO:166 - Description of artificial sequence: Artificial synthetic primer sequence
SEQ ID NO:167 - Description of artificial sequence: Artificial synthetic primer sequence
SEQ ID NO:174 - Description of artificial sequence: Synthetic DNA
SEQ ID NO:175 - Description of artificial sequence: Synthetic DNA
SEQ ID NO:176 - Description of artificial sequence: Synthetic DNA
SEQ ID NO:177 - Description of artificial sequence: Synthetic DNA
SEQ ID NO:178 - Description of artificial sequence: Synthetic DNA
SEQ ID NO:179 - Description of artificial sequence: Synthetic DNA
SEQ ID NO:180 - Description of artificial sequence: Synthetic DNA
SEQ ID NO:181 - Description of artificial sequence: Synthetic DNA

SEQUENCE LISTING

<110> KYOWA HAKKO KOGYO CO., LTD.

<120> Shear stress-responsive genes

<130> PH-1064-PCT

<140>

<141>

<150> JP 1999-280976

<151> 1999-10-01

<160> 181

<170> PatentIn Ver. 2.0

<210> 1

<211> 3817

<212> DNA

<213> Homo sapiens

<220>

<221> CDS

<222> (440)..(1930)

<400> 1
gaattcgggt ggagtcctga aggagggcct gatgtcttca tcattctcaa attcttgtaa 60
gctctgcgtc gggtgaaacc agacaaagcc gcgagcccag ggatgggagc acgcggggga 120
cggcctgccg gcggggacga cagcattgcg cctgggtgca gcagtgtgcg tctcggggaa 180
gggaagatat tttaaggcgt gtctgagcag acggggaggc ttttccaaac ccaggcagct 240
tcgtggcgtg tgcggtttcg acccggtcac acaaagcttc agcatgtcat gtgaggacgg 300
tcgggccctg aaaggaacgc tctcggaatt ggccgcggaa accgatctgc ccgttgtgtt 360
tgtgaaacag agaaagatag gcggccatgg tccaaccttg aaggcttatc aggagggcag 420
acttcaaaag ctactaaaa atg aac ggc cct gaa gat ctt ccc aag tcc tat 472
gac tat gac ctt atc atc att gga ggt ggc tca gga ggt ctg gca gct   520
gct aag gag gca gcc caa tat ggc aag aag gtg atg gtc ctg gac ttt   568
gtc act ccc acc cct ctt gga act aga tgg ggt ctt gga gga aca tgt   616
gtg aat gtg ggt tgc ata cct aaa aaa ctg atg cat caa gca gct ttg   664
tta gga caa gcc ctg caa gac tct cga aat tat gga tgg aaa gtc gag   712
gag aca gtt aag cat gat tgg gac aga atg ata gaa gct gta cag aat   760
cac att ggc tct ttg aat tgg ggc tac cga gta gct ctg cgg gag aaa   808
aaa gtc gtc tat gag aat gct tat ggg caa ttt att ggt cct cac agg   856
att aag gca aca aat aat aaa ggc aaa gaa aaa att tat tca gca gag   904
agt ttt ctc att gcc act ggt gaa aga cca cgt tac ttg ggc atc cct 952
ggt gac aaa gaa tac tgc atc agc agt gat gat ctt ttc tcc ttg cct 1000
tac tgc ccg ggt aag acc ctg gtt gtt gga gca tcc tat gtc gct ttg 1048
gag tgc gct gga ttt ctt gct ggt att ggt tta ggc gtc act gtt atg 1096
gtt agg tcc att ctt ctt aga gga ttt gac cag gac atg gcc aac aaa 1144
att ggt gaa cac atg gaa gaa cat ggc atc aag ttt ata aga cag ttc 1192
gta cca att aaa gtt gaa caa att gaa gca ggg aca cca ggc cga ctc 1240
aga gta gta gct cag tcc acc aat agt gag gaa atc att gaa gga gaa 1288
tat aat acg gtg atg ctg gca ata gga aga gat gct tgc aca aga aaa 1336
att ggc tta gaa acc gta ggg gtg aag ata aat gaa aag act gga aaa 1384
ata cct gtc aca gat gaa gaa cag acc aat gtg cct tac atc tat gcc 1432
att ggc gat ata ttg gag gat aag gtg gag ctc acc cca gtt gca atc 1480
cag gca gga aga ttg ctg gct cag agg ctc tat gca ggt tcc act gtc 1528
aag tgt gac tat gaa aat gtt cca acc act gta ttt act cct ttg gaa 1576
tat ggt gct tgt ggc ctt tct gag gag aaa gct gtg gag aag ttt ggg 1624
gaa gaa aat att gag gtt tac cat agt tac ttt tgg cca ttg gaa tgg 1672
acg att ccg tca aga gat aac aac aaa tgt tat gca aaa ata atc tgt 1720
aat act aaa gac aat gaa cgt gtt gtg ggc ttt cac gta ctg ggt cca 1768
aat gct gga gaa gtt aca caa ggc ttt gca gct gcg ctc aaa tgt gga 1816
ctg acc aaa aag cag ctg gac agc aca att gga atc cac cct gtc tgt 1864
gca gag gta ttc aca aca ttg tct gtg acc aag cgc tct ggg gca agc 1912
atc ctc cag gct ggc tgc tgaggttaag ccccagtgtg gatgctgttg 1960
ccaagactgc aaaccactgg ctcgtttccg tgcccaaatc caaggcgaag ttttctagag 2020
ggttcttggg ctcttggcac ctgcgtgtcc tgtgcttacc accgcccaag gcccccttgg 2080
atctcttgga taggagttgg tgaatagaag gcaggcagca tcacactggg gtcactgaca 2140
gacttgaagc tgacatttgg cagggcatcg aagggatgca tccatgaagt caccagtctc 2200
aagcccatgt ggtaggcggt gatggaacaa ctgtcaaatc agttttagca tgacctttcc 2260
ttgtggattt tcttattctc gttgtcaagt tttctagggt tgaatttttt tcttttttct 2320
ccatggtgtt aatgatatta gagatgaaaa acgttagcag ttgatttttg tccaaaagca 2380
agtcatggct agagtatcca tgcaaggtgt cttgttgcat ggaagggata gtttggctcc 2440
cttggaggct atgtaggctt gtcccgggaa agagaactgt cctgcagctg aaatggactg 2500
ttctttactg acctgctcag cagtttcttc tctcatatat tcccaaaaca agtacatctg 2560
cgatcaactc tagccaaatt tgcccctgtg tgctacatga tggatgatta ttattttaag 2620
gtctgtttag gaagggaaat ggctacttgg ccagccattg cctggcattt ggtagtatag 2680
tatgattctc accattattt gtcatggagg cagacataca ccagaaatgg gggagaaaca 2740
gtacatatct ttctgtcttt agtttattgt gtgctggtct aagcaagctg agatcatttg 2800
caatggaaaa cacgtaactt gtttaaaagt ttttctggta gctttagctt tatgctaaaa 2860
aaaataatga cattgggtat ctatttcttt ctaagacata cattagtagg aaaataagtc 2920
ttttcatgct tatgatttag ctgttttgtg gtaattgctt tttaaaggaa gttattaata 2980
tcataagtta ttattaatat tttgaacaca ggtggatgtg aaggattttc atttaaaaac 3040
caagtggttt tgactttttc tgttgaatga acaactgtgc cttgtggaat ttttgcagaa 3100
gtgtttatgc tttgttagca tttcaacttg cattattata aagaggtatt aatgcctcag 3160
ttatgtgttt gtcaatgtac tggctgagga ttctatctca gctgtctttt ctaactgtgt 3220
aggttgagtt ttgaacacgt gcttgtggac atcagcctcc tgccagcagt tcttgaagct 3280
tctttttcat tcctgctact ctacctgtat ttctcagttg cagcactgag tggtcaaaat 3340
acatttctgg gccacctcag ggaacccatg catctgcctg gcatttaggc agcagagccc 3400
ctgaccgtcc cccacaggct ctgcctcacg tcctcatctc atttggctgt gtaaagaaat 3460
gggaaaaggg aaaaggagag agcaattgag gcagttgacc atattcagtt ttatttattt 3520
atttttaatt tgtttttttc tccaagtcca ccagtctctg aaattagaac agtaggcggt 3580
atgagataat caggcctaat catgttgtga ttctcttttc ttagtggagt ggaatgttct 3640
atccccacaa gaaggattat atcttataga cttgtcttgt tcagattctg tatttaccca 3700
ttttattgaa acatatacta agttccatgt attttgttta caaatcttct gaaaaaaaac 3760
aaaacaatgt gaaacattaa aattaaaagg cattaataat aaaaaaaaaa aaaaaaa 3817

<210> 2

<211> 497

<212> PRT

<213> Homo sapiens

<400> 2

<210> 3
<211> 1487
<212> DNA
<213> Homo sapiens
<220>
<221> CDS
<222> (6)..(938)
<400> 3
ggagc atg cgg ggc gcg gcg cgg gcg gca tgg ggg cgc gcg ggg cag ccg 50
Met Arg Gly Ala Ala Arg Ala Ala Trp Gly Arg Ala Gly Gln Pro
1               5                   10                  15
tgg ccg cga ccc ccc gcc ccg ggc ccg ccc ccg ccg ccg ctc ccg ctg 98
Trp Pro Arg Pro Pro Ala Pro Gly Pro Pro Pro Pro Pro Leu Pro Leu
                20                  25                  30
ctg ctc ctg ctc ctg gcc ggg ctg ctg ggc ggc gcg ggc gcg cag tac 146
Leu Leu Leu Leu Leu Ala Gly Leu Leu Gly Gly Ala Gly Ala Gln Tyr
            35                  40                  45

tcc agc gac cgg tgc agc tgg aag ggg agc ggg ctg acg cac gag gca 194
Ser Ser Asp Arg Cys Ser Trp Lys Gly Ser Gly Leu Thr His Glu Ala
        50                  55                  60
cac agg aag gag gtg gag cag gtg tat ctg cgc tgt gcg gcg ggt gcc 242
His Arg Lys Glu Val Glu Gln Val Tyr Leu Arg Cys Ala Ala Gly Ala
    65                  70                  75
gtg gag tgg atg tac cca aca ggt gct ctc atc gtt aac ctg cgg ccc 290
Val Glu Trp Met Tyr Pro Thr Gly Ala Leu Ile Val Asn Leu Arg Pro
80                  85                  90                  95
aac acc ttc tcg cct gcc cgg cac ctg acc gtg tgc atc agg tcc ttc 338
Asn Thr Phe Ser Pro Ala Arg His Leu Thr Val Cys Ile Arg Ser Phe
                100                 105                 110
acg gac tcc tcg ggg gcc aat att tat ttg gaa aaa act gga gaa ctg 386
Thr Asp Ser Ser Gly Ala Asn Ile Tyr Leu Glu Lys Thr Gly Glu Leu
            115                 120                 125
aga ctg ctg gta ccg gac ggg gac ggc agg ccc ggc cgg gtg cag tgt 434
Arg Leu Leu Val Pro Asp Gly Asp Gly Arg Pro Gly Arg Val Gln Cys
        130                 135                 140
ttt ggc ctg gag cag ggc ggc ctg ttc gtg gag gcc acg ccg cag cag 482
Phe Gly Leu Glu Gln Gly Gly Leu Phe Val Glu Ala Thr Pro Gln Gln
    145                 150                 155
gat atc ggc cgg agg acc aca ggc ttc cag tac gag ctg gtt agg agg 530
Asp Ile Gly Arg Arg Thr Thr Gly Phe Gln Tyr Glu Leu Val Arg Arg
160                 165                 170                 175
cac agg gcg tcg gac ctg cac gag ctg tct gcg ccg tgc cgt ccc tgc 578
His Arg Ala Ser Asp Leu His Glu Leu Ser Ala Pro Cys Arg Pro Cys
                180                 185                 190

agt gac acc gag gtg ctc cta gcc gtc tgc acc agc gac ttc gcc gtt 626
Ser Asp Thr Glu Val Leu Leu Ala Val Cys Thr Ser Asp Phe Ala Val
            195                 200                 205
cga ggc tcc atc cag caa gtt acc cac gag cct gag cgg cag gac tca 674
Arg Gly Ser Ile Gln Gln Val Thr His Glu Pro Glu Arg Gln Asp Ser
        210                 215                 220
gcc atc cac ctg cgc gtg agc aga ctc tat cgg cag aaa agc agg gtc 722
Ala Ile His Leu Arg Val Ser Arg Leu Tyr Arg Gln Lys Ser Arg Val
    225                 230                 235
ttc gag ccg gtg ccc gag ggt gac ggc cac tgg cag ggg cgc gtc agg 770
Phe Glu Pro Val Pro Glu Gly Asp Gly His Trp Gln Gly Arg Val Arg
240                 245                 250                 255
acg ctg ctg gag tgt ggc gtg cgg ccg ggg cat ggc gac ttc ctc ttc 818
Thr Leu Leu Glu Cys Gly Val Arg Pro Gly His Gly Asp Phe Leu Phe
                260                 265                 270
act ggc cac atg cac ttc ggg gag gcg cgg ctc ggc tgt gcc cca cgc 866
Thr Gly His Met His Phe Gly Glu Ala Arg Leu Gly Cys Ala Pro Arg
            275                 280                 285
ttc aag gac ttc cag agg atg tac agg gat gcc cag gag agg ggg ctg 914
Phe Lys Asp Phe Gln Arg Met Tyr Arg Asp Ala Gln Glu Arg Gly Leu
        290                 295                 300
aac cct tgt gag gtt ggc acg gac tgactccgtg ggccgctgcc cttcctctcc 968
Asn Pro Cys Glu Val Gly Thr Asp
    305                 310
tgatgagtca caggctgcgg tgggcgctgc ggtcctggtg ggccgtgcgg gtgagggcca 1028
cgcgctggga gccgcgtgcc ctgggcccag tcctgaccct ggtaccgaag ctgtggacgt 1088

tctcgccaca ctcaacccca tgagcttcca gccaaggatg ccctggccga ttggaaatgc 1148

tgtaaaatgc aaactaagtt attatatttt tttttggtaa aaaagaaatg tccataggaa 1208

acaaattcct gtgtcttaaa acgccttggt gtgccgtctg atactgttct ctaaagacgt 1268

taggagtcac ggcatctggc ctgcggttgg gtgaagcact ggccgttggg cacagtggat 1328

gtgtgaaaag gtgccattca gagttgttat tctcatgacg gaagttttgg agccaaataa 1388

tacgtttttt attttcattt tatttttaaa ggatgagctt tggtcctttt caggccgccg 1448

gttgtttccg ttcccgagaa taaagacgag gatccgacc 1487

<210> 4

<211> 311

<212> PRT

<213> Homo sapiens

<400> 4

Met Arg Gly Ala Ala Arg Ala Ala Trp Gly Arg Ala Gly Gln Pro Trp

1               5                   10                  15

Pro Arg Pro Pro Ala Pro Gly Pro Pro Pro Pro Pro Leu Pro Leu Leu

            20                  25                  30

Leu Leu Leu Leu Ala Gly Leu Leu Gly Gly Ala Gly Ala Gln Tyr Ser

        35                  40                  45

Ser Asp Arg Cys Ser Trp Lys Gly Ser Gly Leu Thr His Glu Ala His

    50                  55                  60

Arg Lys Glu Val Glu Gln Val Tyr Leu Arg Cys Ala Ala Gly Ala Val

65                  70                  75                  80

Glu Trp Met Tyr Pro Thr Gly Ala Leu Ile Val Asn Leu Arg Pro Asn

                85                  90                  95

Thr Phe Ser Pro Ala Arg His Leu Thr Val Cys Ile Arg Ser Phe Thr

            100                 105                 110

Asp Ser Ser Gly Ala Asn Ile Tyr Leu Glu Lys Thr Gly Glu Leu Arg
        115                 120                 125
Leu Leu Val Pro Asp Gly Asp Gly Arg Pro Gly Arg Val Gln Cys Phe
    130                 135                 140
Gly Leu Glu Gln Gly Gly Leu Phe Val Glu Ala Thr Pro Gln Gln Asp
145                 150                 155                 160
Ile Gly Arg Arg Thr Thr Gly Phe Gln Tyr Glu Leu Val Arg Arg His
                165                 170                 175
Arg Ala Ser Asp Leu His Glu Leu Ser Ala Pro Cys Arg Pro Cys Ser
            180                 185                 190
Asp Thr Glu Val Leu Leu Ala Val Cys Thr Ser Asp Phe Ala Val Arg
        195                 200                 205
Gly Ser Ile Gln Gln Val Thr His Glu Pro Glu Arg Gln Asp Ser Ala
    210                 215                 220
Ile His Leu Arg Val Ser Arg Leu Tyr Arg Gln Lys Ser Arg Val Phe
225                 230                 235                 240
Glu Pro Val Pro Glu Gly Asp Gly His Trp Gln Gly Arg Val Arg Thr
                245                 250                 255
Leu Leu Glu Cys Gly Val Arg Pro Gly His Gly Asp Phe Leu Phe Thr
            260                 265                 270
Gly His Met His Phe Gly Glu Ala Arg Leu Gly Cys Ala Pro Arg Phe
        275                 280                 285
Lys Asp Phe Gln Arg Met Tyr Arg Asp Ala Gln Glu Arg Gly Leu Asn
    290                 295                 300
Pro Cys Glu Val Gly Thr Asp
305                 310

<210> 5

<211> 2820

<212> DNA

<213> Homo sapiens

<220>

<221> CDS

<222> (49)..(2664)

<400> 5

ctcccaaagc agaattgcag ctgccgccgc cgccacctcc aggccact atg gcg cct 57

Met Ala Pro

1

ggg gct gcc cag gag ctt cag gcc aag ttg gca gag atc gga gct ccg 105

Gly Ala Ala Gln Glu Leu Gln Ala Lys Leu Ala Glu Ile Gly Ala Pro

    5                   10                  15

atc cag ggt aat cgc gag gag ctg gtg gag cgg ctg cag agc tac acc 153

Ile Gln Gly Asn Arg Glu Glu Leu Val Glu Arg Leu Gln Ser Tyr Thr

20                  25                  30                  35

cgc cag act ggc atc gtg ctg aat cgg ccg gtt ttg aga ggg gaa gat 201

Arg Gln Thr Gly Ile Val Leu Asn Arg Pro Val Leu Arg Gly Glu Asp

                40                  45                  50

ggg gac aaa gcc gct cca cct ccc atg tcg gca cag ctc cct gga att 249

Gly Asp Lys Ala Ala Pro Pro Pro Met Ser Ala Gln Leu Pro Gly Ile

            55                  60                  65

ccc atg cca cca cca cct ttg gga ctc ccc cct ctg cag cct cct ccg 297

Pro Met Pro Pro Pro Pro Leu Gly Leu Pro Pro Leu Gln Pro Pro Pro

        70                  75                  80

cca ccc cca cca cct cca cca ggc ctt ggc ctt ggc ttt cct atg gcc 345

Pro Pro Pro Pro Pro Pro Pro Gly Leu Gly Leu Gly Phe Pro Met Ala
    85                  90                  95
cac cca cca aat ttg ggg ccc ccg cct cct ctc cgt gtg ggt gag cca 393
His Pro Pro Asn Leu Gly Pro Pro Pro Pro Leu Arg Val Gly Glu Pro
100                 105                 110                 115
gtg gca ctg tca gag gag gag cgg ctg aag ttg gct cag cag cag gcg 441
Val Ala Leu Ser Glu Glu Glu Arg Leu Lys Leu Ala Gln Gln Gln Ala
                120                 125                 130
gca ttg ctg atg cag cag gag gag cgt gcc aag cag cag gga gat cat 489
Ala Leu Leu Met Gln Gln Glu Glu Arg Ala Lys Gln Gln Gly Asp His
            135                 140                 145
tcg ctg aag gaa cat gag ctc ttg gag cag cag aag cgg gca gct gtg 537
Ser Leu Lys Glu His Glu Leu Leu Glu Gln Gln Lys Arg Ala Ala Val
        150                 155                 160
tta ctg gag cag gaa cga cag cag gag att gcc aag atg ggc acc cca 585
Leu Leu Glu Gln Glu Arg Gln Gln Glu Ile Ala Lys Met Gly Thr Pro
    165                 170                 175
gtc cct cgg ccc cca caa gac atg ggc cag att ggt gtg cgc act cct 633
Val Pro Arg Pro Pro Gln Asp Met Gly Gln Ile Gly Val Arg Thr Pro
180                 185                 190                 195
ctg ggt cct cga gta gct gct cca gtg ggc cca gtg ggc ccc act cct 681
Leu Gly Pro Arg Val Ala Ala Pro Val Gly Pro Val Gly Pro Thr Pro
                200                 205                 210
aca gtt ttg ccc atg gga gcc cct gtt ccc cgg cct cgt ggt ccc cca 729
Thr Val Leu Pro Met Gly Ala Pro Val Pro Arg Pro Arg Gly Pro Pro
            215                 220                 225

ccg ccc cct gga gat gag aac aga gag atg gat gac ccc tct gtg ggc 777
Pro Pro Pro Gly Asp Glu Asn Arg Glu Met Asp Asp Pro Ser Val Gly
        230                 235                 240
ccc aag atc ccc cag gct ttg gag aag atc ctg cag ctg aag gag agc 825
Pro Lys Ile Pro Gln Ala Leu Glu Lys Ile Leu Gln Leu Lys Glu Ser
    245                 250                 255
cgc cag gaa gag atg aat tct cag cag gag gaa gag gaa atg gaa aca 873
Arg Gln Glu Glu Met Asn Ser Gln Gln Glu Glu Glu Glu Met Glu Thr
260                 265                 270                 275
gat gct cgc tcg tcc ctg ggc cag tca gcg tca gag act gag gag gac 921
Asp Ala Arg Ser Ser Leu Gly Gln Ser Ala Ser Glu Thr Glu Glu Asp
                280                 285                 290
aca gtg tcc gta tct aaa aag gag aaa aac cgg aag cgt agg aac cga 969
Thr Val Ser Val Ser Lys Lys Glu Lys Asn Arg Lys Arg Arg Asn Arg
            295                 300                 305
aag aag aag aaa aag ccc cag cgg gtg cga ggg gtg tcc tct gag agc 1017
Lys Lys Lys Lys Lys Pro Gln Arg Val Arg Gly Val Ser Ser Glu Ser
        310                 315                 320
tct ggg gac cgg gag aaa gac tca acc cgg tcc cgt ggc tct gat tcc 1065
Ser Gly Asp Arg Glu Lys Asp Ser Thr Arg Ser Arg Gly Ser Asp Ser
    325                 330                 335
cca gca gct gat gtt gag att gag tat gtg act gaa gaa cct gaa att 1113
Pro Ala Ala Asp Val Glu Ile Glu Tyr Val Thr Glu Glu Pro Glu Ile
340                 345                 350                 355
tac gag ccc aac ttt atc ttc ttt aag agg atc ttt gag gct ttt aag 1161
Tyr Glu Pro Asn Phe Ile Phe Phe Lys Arg Ile Phe Glu Ala Phe Lys
                360                 365                 370

ctc act gat gat gtg aag aag gag aaa gag aaa gag cca gag aaa ctt 1209
Leu Thr Asp Asp Val Lys Lys Glu Lys Glu Lys Glu Pro Glu Lys Leu
            375                 380                 385
gac aaa ctg gag aac tct gca gcc ccc aag aag aag gga ttt gaa gag 1257
Asp Lys Leu Glu Asn Ser Ala Ala Pro Lys Lys Lys Gly Phe Glu Glu
        390                 395                 400
gag cac aag gac agt gat gat gac agc agt gat gac gag cag gaa aag 1305
Glu His Lys Asp Ser Asp Asp Asp Ser Ser Asp Asp Glu Gln Glu Lys
    405                 410                 415
aag cca gaa gcc ccc aag ctg tcc aag aag aag ttg cgc cga atg aac 1353
Lys Pro Glu Ala Pro Lys Leu Ser Lys Lys Lys Leu Arg Arg Met Asn
420                 425                 430                 435
cgc ttc act gtg gct gaa ctc aag cag ctg gtg gct cgg ccc gat gtc 1401
Arg Phe Thr Val Ala Glu Leu Lys Gln Leu Val Ala Arg Pro Asp Val
                440                 445                 450
gtg gag atg cac gat gtg aca gcg cag gac cct aag ctc ttg gtt cac 1449
Val Glu Met His Asp Val Thr Ala Gln Asp Pro Lys Leu Leu Val His
            455                 460                 465
ctc aag gcc act cgg aac tct gtg cct gtg cca cgc cac tgg tgt ttt 1497
Leu Lys Ala Thr Arg Asn Ser Val Pro Val Pro Arg His Trp Cys Phe
        470                 475                 480
aag cgc aaa tac ctg cag ggc aaa cgg ggc att gag aag ccc ccc ttc 1545
Lys Arg Lys Tyr Leu Gln Gly Lys Arg Gly Ile Glu Lys Pro Pro Phe
    485                 490                 495
gag ctg cca gac ttc atc aaa cgc aca ggc atc cag gag atg cga gag 1593
Glu Leu Pro Asp Phe Ile Lys Arg Thr Gly Ile Gln Glu Met Arg Glu

500                 505                 510                 515

gcc ctg cag gag aag gaa gaa cag aag acc atg aag tca aaa atg cga 1641

Ala Leu Gln Glu Lys Glu Glu Gln Lys Thr Met Lys Ser Lys Met Arg

                520                 525                 530

gag aaa gtt cgg cct aag atg ggc aaa att gac atc gac tac cag aaa 1689

Glu Lys Val Arg Pro Lys Met Gly Lys Ile Asp Ile Asp Tyr Gln Lys

            535                 540                 545

ctg cat gat gcc ttc ttc aag tgg cag acc aag cca aag ctg acc atc 1737

Leu His Asp Ala Phe Phe Lys Trp Gln Thr Lys Pro Lys Leu Thr Ile

        550                 555                 560

cat ggg gac ctg tac tat gag ggg aag gag ttc gag aca cga ctg aag 1785

His Gly Asp Leu Tyr Tyr Glu Gly Lys Glu Phe Glu Thr Arg Leu Lys

    565                 570                 575

gag aag aag cca gga gat ctg tct gat gag cta agg att tcc ttg ggg 1833

Glu Lys Lys Pro Gly Asp Leu Ser Asp Glu Leu Arg Ile Ser Leu Gly

580                 585                 590                 595

atg cca gta gga cca aat gcc cac aag gtc cct ccc cca tgg ctg att 1881

Met Pro Val Gly Pro Asn Ala His Lys Val Pro Pro Pro Trp Leu Ile

                600                 605                 610

gcc atg cag cga tat gga cca ccc cca tcg tat ccc aac ctg aaa atc 1929

Ala Met Gln Arg Tyr Gly Pro Pro Pro Ser Tyr Pro Asn Leu Lys Ile

            615                 620                 625

cct ggg ctg aac tcg ccc atc cct gag agc tgt tcc ttt ggg tac cat 1977

Pro Gly Leu Asn Ser Pro Ile Pro Glu Ser Cys Ser Phe Gly Tyr His

        630                 635                 640

gct ggt ggc tgg ggc aaa cct cca gtg gat gag act ggg aaa ccg ctc 2025

Ala Gly Gly Trp Gly Lys Pro Pro Val Asp Glu Thr Gly Lys Pro Leu

    645                 650                 655

tat ggg gac gtg ttt gga acc aat gct gct gaa ttt cag acc aag act 2073
Tyr Gly Asp Val Phe Gly Thr Asn Ala Ala Glu Phe Gln Thr Lys Thr
660                 665                 670                 675

gag gaa gaa gag att gat cgg acc cct tgg ggg gaa ctg gaa cca tct 2121
Glu Glu Glu Glu Ile Asp Arg Thr Pro Trp Gly Glu Leu Glu Pro Ser
                680                 685                 690

gat gaa gaa tcc tca gaa gaa gag gaa gag gaa gaa agt gat gaa gac 2169
Asp Glu Glu Ser Ser Glu Glu Glu Glu Glu Glu Glu Ser Asp Glu Asp
            695                 700                 705

aaa cca gat gag aca ggc ttt att acc cct gca gac agt ggc ctt atc 2217
Lys Pro Asp Glu Thr Gly Phe Ile Thr Pro Ala Asp Ser Gly Leu Ile
        710                 715                 720

act cct gga ggc ttt tca tca gtg cct gct gga atg gag acc cct gaa 2265
Thr Pro Gly Gly Phe Ser Ser Val Pro Ala Gly Met Glu Thr Pro Glu
    725                 730                 735

ctc att gag ctg agg aag aag aag att gag gag gcg atg gac gga agt 2313
Leu Ile Glu Leu Arg Lys Lys Lys Ile Glu Glu Ala Met Asp Gly Ser
740                 745                 750                 755

gag aca cct cag ctc ttc act gtg ttg cca gag aag aga aca gcc act 2361
Glu Thr Pro Gln Leu Phe Thr Val Leu Pro Glu Lys Arg Thr Ala Thr
                760                 765                 770

gtt gga ggg gcc atg atg gga tca acc cac att tat gac atg tcc acg 2409
Val Gly Gly Ala Met Met Gly Ser Thr His Ile Tyr Asp Met Ser Thr
            775                 780                 785

gtt atg agc cgg aag ggc ccg gct cct gag ctg caa ggt gtg gaa gtg 2457

Val Met Ser Arg Lys Gly Pro Ala Pro Glu Leu Gln Gly Val Glu Val
        790                 795                 800
gcg ctg gcg cct gaa gag ttg gag ctg gat cct atg gcc atg acc cag 2505
Ala Leu Ala Pro Glu Glu Leu Glu Leu Asp Pro Met Ala Met Thr Gln
    805                 810                 815
aag tat gag gag cat gtg cgg gag cag cag gct caa gta gag aag gag 2553
Lys Tyr Glu Glu His Val Arg Glu Gln Gln Ala Gln Val Glu Lys Glu
820                 825                 830                 835
gac ttc agt gac atg gtg gct gag cac gct gcc aaa cag aag caa aaa 2601
Asp Phe Ser Asp Met Val Ala Glu His Ala Ala Lys Gln Lys Gln Lys
                840                 845                 850
aaa cgg aaa gct cag ccc cag gac agc cgt ggg ggc agc aag aaa tat 2649
Lys Arg Lys Ala Gln Pro Gln Asp Ser Arg Gly Gly Ser Lys Lys Tyr
            855                 860                 865
aag gag ttc aag ttt taggtcccct cacactagcc ctttttttgg ccctacgtct 2704
Lys Glu Phe Lys Phe
        870
ggatgcctgg gcttcacaca agaaccacct ctcccgcagt tcccaaggac ttgtcatttc 2764
atgttcttat tttagacctg ttttgtaaat aaagctgttt cccaaggaaa gagatg 2820
<210> 6
<211> 872
<212> PRT
<213> Homo sapiens
<400> 6
Met Ala Pro Gly Ala Ala Gln Glu Leu Gln Ala Lys Leu Ala Glu Ile
1               5                   10                  15

Gly Ala Pro Ile Gln Gly Asn Arg Glu Glu Leu Val Glu Arg Leu Gln
            20                  25                  30
Ser Tyr Thr Arg Gln Thr Gly Ile Val Leu Asn Arg Pro Val Leu Arg
        35                  40                  45
Gly Glu Asp Gly Asp Lys Ala Ala Pro Pro Pro Met Ser Ala Gln Leu
    50                  55                  60
Pro Gly Ile Pro Met Pro Pro Pro Pro Leu Gly Leu Pro Pro Leu Gln
65                  70                  75                  80
Pro Pro Pro Pro Pro Pro Pro Pro Pro Pro Gly Leu Gly Leu Gly Phe
                85                  90                  95
Pro Met Ala His Pro Pro Asn Leu Gly Pro Pro Pro Pro Leu Arg Val
            100                 105                 110
Gly Glu Pro Val Ala Leu Ser Glu Glu Glu Arg Leu Lys Leu Ala Gln
        115                 120                 125
Gln Gln Ala Ala Leu Leu Met Gln Gln Glu Glu Arg Ala Lys Gln Gln
    130                 135                 140
Gly Asp His Ser Leu Lys Glu His Glu Leu Leu Glu Gln Gln Lys Arg
145                 150                 155                 160
Ala Ala Val Leu Leu Glu Gln Glu Arg Gln Gln Glu Ile Ala Lys Met
                165                 170                 175
Gly Thr Pro Val Pro Arg Pro Pro Gln Asp Met Gly Gln Ile Gly Val
            180                 185                 190
Arg Thr Pro Leu Gly Pro Arg Val Ala Ala Pro Val Gly Pro Val Gly
        195                 200                 205
Pro Thr Pro Thr Val Leu Pro Met Gly Ala Pro Val Pro Arg Pro Arg
    210                 215                 220
Gly Pro Pro Pro Pro Pro Gly Asp Glu Asn Arg Glu Met Asp Asp Pro

225                 230                 235                 240

Ser Val Gly Pro Lys Ile Pro Gln Ala Leu Glu Lys Ile Leu Gln Leu

                245                 250                 255

Lys Glu Ser Arg Gln Glu Glu Met Asn Ser Gln Gln Glu Glu Glu Glu

            260                 265                 270

Met Glu Thr Asp Ala Arg Ser Ser Leu Gly Gln Ser Ala Ser Glu Thr

        275                 280                 285

Glu Glu Asp Thr Val Ser Val Ser Lys Lys Glu Lys Asn Arg Lys Arg

    290                 295                 300

Arg Asn Arg Lys Lys Lys Lys Lys Pro Gln Arg Val Arg Gly Val Ser

305                 310                 315                 320

Ser Glu Ser Ser Gly Asp Arg Glu Lys Asp Ser Thr Arg Ser Arg Gly

                325                 330                 335

Ser Asp Ser Pro Ala Ala Asp Val Glu Ile Glu Tyr Val Thr Glu Glu

            340                 345                 350

Pro Glu Ile Tyr Glu Pro Asn Phe Ile Phe Phe Lys Arg Ile Phe Glu

        355                 360                 365

Ala Phe Lys Leu Thr Asp Asp Val Lys Lys Glu Lys Glu Lys Glu Pro

    370                 375                 380

Glu Lys Leu Asp Lys Leu Glu Asn Ser Ala Ala Pro Lys Lys Lys Gly

385                 390                 395                 400

Phe Glu Glu Glu His Lys Asp Ser Asp Asp Asp Ser Ser Asp Asp Glu

                405                 410                 415

Gln Glu Lys Lys Pro Glu Ala Pro Lys Leu Ser Lys Lys Lys Leu Arg

            420                 425                 430

Arg Met Asn Arg Phe Thr Val Ala Glu Leu Lys Gln Leu Val Ala Arg

        435                 440                 445

Pro Asp Val Val Glu Met His Asp Val Thr Ala Gln Asp Pro Lys Leu

    450                 455                 460

Leu Val His Leu Lys Ala Thr Arg Asn Ser Val Pro Val Pro Arg His

465                 470                 475                 480

Trp Cys Phe Lys Arg Lys Tyr Leu Gln Gly Lys Arg Gly Ile Glu Lys

                485                 490                 495

Pro Pro Phe Glu Leu Pro Asp Phe Ile Lys Arg Thr Gly Ile Gln Glu

            500                 505                 510

Met Arg Glu Ala Leu Gln Glu Lys Glu Glu Gln Lys Thr Met Lys Ser

        515                 520                 525

Lys Met Arg Glu Lys Val Arg Pro Lys Met Gly Lys Ile Asp Ile Asp

    530                 535                 540

Tyr Gln Lys Leu His Asp Ala Phe Phe Lys Trp Gln Thr Lys Pro Lys

545                 550                 555                 560

Leu Thr Ile His Gly Asp Leu Tyr Tyr Glu Gly Lys Glu Phe Glu Thr

                565                 570                 575

Arg Leu Lys Glu Lys Lys Pro Gly Asp Leu Ser Asp Glu Leu Arg Ile

            580                 585                 590

Ser Leu Gly Met Pro Val Gly Pro Asn Ala His Lys Val Pro Pro Pro

        595                 600                 605

Trp Leu Ile Ala Met Gln Arg Tyr Gly Pro Pro Pro Ser Tyr Pro Asn

    610                 615                 620

Leu Lys Ile Pro Gly Leu Asn Ser Pro Ile Pro Glu Ser Cys Ser Phe

625                 630                 635                 640

Gly Tyr His Ala Gly Gly Trp Gly Lys Pro Pro Val Asp Glu Thr Gly

                645                 650                 655

Lys Pro Leu Tyr Gly Asp Val Phe Gly Thr Asn Ala Ala Glu Phe Gln
            660                 665                 670
Thr Lys Thr Glu Glu Glu Glu Ile Asp Arg Thr Pro Trp Gly Glu Leu
        675                 680                 685
Glu Pro Ser Asp Glu Glu Ser Ser Glu Glu Glu Glu Glu Glu Glu Ser
    690                 695                 700
Asp Glu Asp Lys Pro Asp Glu Thr Gly Phe Ile Thr Pro Ala Asp Ser
705                 710                 715                 720
Gly Leu Ile Thr Pro Gly Gly Phe Ser Ser Val Pro Ala Gly Met Glu
                725                 730                 735
Thr Pro Glu Leu Ile Glu Leu Arg Lys Lys Lys Ile Glu Glu Ala Met
            740                 745                 750
Asp Gly Ser Glu Thr Pro Gln Leu Phe Thr Val Leu Pro Glu Lys Arg
        755                 760                 765
Thr Ala Thr Val Gly Gly Ala Met Met Gly Ser Thr His Ile Tyr Asp
    770                 775                 780
Met Ser Thr Val Met Ser Arg Lys Gly Pro Ala Pro Glu Leu Gln Gly
785                 790                 795                 800
Val Glu Val Ala Leu Ala Pro Glu Glu Leu Glu Leu Asp Pro Met Ala
                805                 810                 815
Met Thr Gln Lys Tyr Glu Glu His Val Arg Glu Gln Gln Ala Gln Val
            820                 825                 830
Glu Lys Glu Asp Phe Ser Asp Met Val Ala Glu His Ala Ala Lys Gln
        835                 840                 845
Lys Gln Lys Lys Arg Lys Ala Gln Pro Gln Asp Ser Arg Gly Gly Ser
    850                 855                 860

Lys Lys Tyr Lys Glu Phe Lys Phe

865                 870

<210> 7

<211> 2433

<212> DNA

<213> Homo sapiens

<220>

<221> CDS

<222> (214)..(1146)

<400> 7

atgccagccc caaacctcat ccctagtgga ggccttgctg atgtggaagt ggccagggcc 60

ctcatggtag gctgggcaga agcccaagaa caggctctaa agctgctaaa cccggcagtc 120

ctggtccccg gaggctcttg ccagtctgac agtgttcttg gcactgctca aaggtcccag 180

cagctggggt tccccgtcag cccgtgagcg gcc atg tcc aac ccc agc gcc cca 234

Met Ser Asn Pro Ser Ala Pro

1               5

cca cca tat gaa gac cgc aac ccc ctg tac cca ggc cct ccg ccc cct 282

Pro Pro Tyr Glu Asp Arg Asn Pro Leu Tyr Pro Gly Pro Pro Pro Pro

        10                  15                  20

ggg ggc tat ggg cag cca tct gtc ctg cca gga ggg tat cct gcc tac 330

Gly Gly Tyr Gly Gln Pro Ser Val Leu Pro Gly Gly Tyr Pro Ala Tyr

    25                  30                  35

cct ggc tac ccg cag cct ggc tac ggt cac cct gct ggc tac cca cag 378

Pro Gly Tyr Pro Gln Pro Gly Tyr Gly His Pro Ala Gly Tyr Pro Gln

40                  45                  50                  55

ccc atg ccc ccc acc cac ccg atg ccc atg aac tac ggc cca ggc cat 426

Pro Met Pro Pro Thr His Pro Met Pro Met Asn Tyr Gly Pro Gly His
                60                  65                  70
ggc tat gat ggg gag gag aga gcg gtg agt gat agc ttc ggg cct gga 474
Gly Tyr Asp Gly Glu Glu Arg Ala Val Ser Asp Ser Phe Gly Pro Gly
            75                  80                  85
gag tgg gat gac cgg aaa gtg cga cac act ttt atc cga aag gtt tac 522
Glu Trp Asp Asp Arg Lys Val Arg His Thr Phe Ile Arg Lys Val Tyr
        90                  95                  100
tcc atc atc tcc gtg cag ctg ctc atc act gtg gcc atc att gct atc 570
Ser Ile Ile Ser Val Gln Leu Leu Ile Thr Val Ala Ile Ile Ala Ile
    105                 110                 115
ttc acc ttt gtg gaa cct gtc agc gcc ttt gtg agg aga aat gtg gct 618
Phe Thr Phe Val Glu Pro Val Ser Ala Phe Val Arg Arg Asn Val Ala
120                 125                 130                 135
gtc tac tac gtg tcc tat gct gtc ttc gtt gtc acc tac ctg atc ctt 666
Val Tyr Tyr Val Ser Tyr Ala Val Phe Val Val Thr Tyr Leu Ile Leu
                140                 145                 150
gcc tgc tgc cag gga ccc aga cgc cgt ttc cca tgg aac atc att ctg 714
Ala Cys Cys Gln Gly Pro Arg Arg Arg Phe Pro Trp Asn Ile Ile Leu
            155                 160                 165
ctg acc ctt ttt act ttt gcc atg ggc ttc atg acg ggc acc att tcc 762
Leu Thr Leu Phe Thr Phe Ala Met Gly Phe Met Thr Gly Thr Ile Ser
        170                 175                 180
agt atg tac caa acc aaa gcc gtc atc att gca atg atc atc act gcg 810
Ser Met Tyr Gln Thr Lys Ala Val Ile Ile Ala Met Ile Ile Thr Ala
    185                 190                 195
gtg gta tcc att tca gtc acc atc ttc tgc ttt cag acc aag gtg gac 858

Val Val Ser Ile Ser Val Thr Ile Phe Cys Phe Gln Thr Lys Val Asp
200                 205                 210                 215
ttc acc tcg tgc aca ggc ctc ttc tgt gtc ctg gga att gtg ctc ctg 906
Phe Thr Ser Cys Thr Gly Leu Phe Cys Val Leu Gly Ile Val Leu Leu
                220                 225                 230
gtg act ggg att gtc act agc att gtg ctc tac ttc caa tac gtt tac 954
Val Thr Gly Ile Val Thr Ser Ile Val Leu Tyr Phe Gln Tyr Val Tyr
            235                 240                 245
tgg ctc cac atg ctc tat gct gct ctg ggg gcc att tgt ttc acc ctg 1002
Trp Leu His Met Leu Tyr Ala Ala Leu Gly Ala Ile Cys Phe Thr Leu
        250                 255                 260
ttc ctg gct tac gac aca cag ctg gtc ctg ggg aac cgg aag cac acc 1050
Phe Leu Ala Tyr Asp Thr Gln Leu Val Leu Gly Asn Arg Lys His Thr
    265                 270                 275
atc agc ccc gag gac tac atc act ggc gcc ctg cag att tac aca gac 1098
Ile Ser Pro Glu Asp Tyr Ile Thr Gly Ala Leu Gln Ile Tyr Thr Asp
280                 285                 290                 295
atc atc tac atc ttc acc ttt gtg ctg cag ctg atg ggg gat cgc aat 1146
Ile Ile Tyr Ile Phe Thr Phe Val Leu Gln Leu Met Gly Asp Arg Asn
                300                 305                 310
taaggagcaa gcccccattt tcacccgatc ctgggctctc ccttccaagc tagagggctg 1206
ggccctatga ctgtggtctg ggctttaggc ccctttcctt ccccttgagt aacatgccca 1266
gtttcctttc tgtcctggag acaggtggcc tctctggcta tggatgtgtg ggtacttggt 1326
ggggacggag gagctaggga ctaactgttg ctcttggtgg gcttggcagg gactaggctg 1386
aagatgtgtc ttctccccgc cacctactgt atgacaccac attcttccta acagctgggg 1446
ttgtgaggaa tatgaaaaga gcctattcga tagctagaag ggaatatgaa aggtagaagt 1506

gacttcaagg tcacgaggtt cccctcccac ctctgtcaca ggcttcttga ctacgtagtt 1566

ggagctattt cttcccccag caaagccaga gagctttgtc cccggcctcc tggacacata 1626

ggccattatc ctgtattcct ttggcttggc atcttttagc tcaggaaggt agaagagatc 1686

tgtgcccatg ggtctccttg cttcaatccc ttcttgtttc agtgacatat gtattgttta 1746

tctgggttag ggatggggga cagataatag aacgagcaaa gtaacctata caggccagca 1806

tggaacagca tctcccctgg gcttgctcct ggcttgtgac gctataagac agagcaggcc 1866

acatgtggcc atctgctccc cattcttgaa agctgctggg gcctccttgc aggcttctgg 1926

atctctggtc agagtgaact cttgcttcct gtattcaggc agctcagagc agaaagtaag 1986

gggcagagtc atacgtgtgg ccaggaagta gccagggtga agagagactc ggtgcgggca 2046

gggagaatgc ctgggggtcc ctcacctggc tagggagata ccgaagccta ctgtggtact 2106

gaagacttct gggttctttc cttctgctaa cccagggagg gtcctaagag gaaggtgact 2166

tctctctgtt tgtcttaagt tgcactgggg gatttctgac ttgaggccca tctctccagc 2226

cagccactgc cttctttgta atattaagtg ccttgagctg gaatggggaa gggggacaag 2286

ggtcagtctg tcgggtgggg gcagaaatca aatcagccca aggatatagt taggattaat 2346

tacttaatag agaaatccta actatatcac acaaaaggat acaactataa atgtaataaa 2406

atttatgtct agaagtt 2423

<210> 8

<211> 311

<212> PRT

<213> Homo sapiens

<400> 8

Met Ser Asn Pro Ser Ala Pro Pro Pro Tyr Glu Asp Arg Asn Pro Leu

1               5                   10                  15

Tyr Pro Gly Pro Pro Pro Pro Gly Gly Tyr Gly Gln Pro Ser Val Leu

            20                  25                  30

Pro Gly Gly Tyr Pro Ala Tyr Pro Gly Tyr Pro Gln Pro Gly Tyr Gly

        35                  40                  45
His Pro Ala Gly Tyr Pro Gln Pro Met Pro Pro Thr His Pro Met Pro
    50                  55                  60
Met Asn Tyr Gly Pro Gly His Gly Tyr Asp Gly Glu Glu Arg Ala Val
65                  70                  75                  80
Ser Asp Ser Phe Gly Pro Gly Glu Trp Asp Asp Arg Lys Val Arg His
                85                  90                  95
Thr Phe Ile Arg Lys Val Tyr Ser Ile Ile Ser Val Gln Leu Leu Ile
            100                 105                 110
Thr Val Ala Ile Ile Ala Ile Phe Thr Phe Val Glu Pro Val Ser Ala
        115                 120                 125
Phe Val Arg Arg Asn Val Ala Val Tyr Tyr Val Ser Tyr Ala Val Phe
    130                 135                 140
Val Val Thr Tyr Leu Ile Leu Ala Cys Cys Gln Gly Pro Arg Arg Arg
145                 150                 155                 160
Phe Pro Trp Asn Ile Ile Leu Leu Thr Leu Phe Thr Phe Ala Met Gly
                165                 170                 175
Phe Met Thr Gly Thr Ile Ser Ser Met Tyr Gln Thr Lys Ala Val Ile
            180                 185                 190
Ile Ala Met Ile Ile Thr Ala Val Val Ser Ile Ser Val Thr Ile Phe
        195                 200                 205
Cys Phe Gln Thr Lys Val Asp Phe Thr Ser Cys Thr Gly Leu Phe Cys
    210                 215                 220
Val Leu Gly Ile Val Leu Leu Val Thr Gly Ile Val Thr Ser Ile Val
225                 230                 235                 240
Leu Tyr Phe Gln Tyr Val Tyr Trp Leu His Met Leu Tyr Ala Ala Leu
                245                 250                 255

Gly Ala Ile Cys Phe Thr Leu Phe Leu Ala Tyr Asp Thr Gln Leu Val
            260                 265                 270
Leu Gly Asn Arg Lys His Thr Ile Ser Pro Glu Asp Tyr Ile Thr Gly
        275                 280                 285
Ala Leu Gln Ile Tyr Thr Asp Ile Ile Tyr Ile Phe Thr Phe Val Leu
    290                 295                 300
Gln Leu Met Gly Asp Arg Asn
305                 310
<210> 9
<211> 4049
<212> DNA
<213> Homo sapiens
<220>
<221> CDS
<222> (86)..(2710)
<400> 9
cctcggccct ccgcctcctc ccctcctcct tctcgtcttc agccgctcct ctcgccgccg 60
cctccacagc ctgggcctcg ccgcg atg ccg gag aag agg ccc ttc gag cgg 112
Met Pro Glu Lys Arg Pro Phe Glu Arg
1               5
ctg cct gcc gat gtc tcc ccc atc aac tac agc ctt tgc ctc aag ccc 160
Leu Pro Ala Asp Val Ser Pro Ile Asn Tyr Ser Leu Cys Leu Lys Pro
10                  15                  20                  25
gac ttg ctg gac ttc acc ttc gag ggc aag ctg gag gcc gcc gcc cag 208
Asp Leu Leu Asp Phe Thr Phe Glu Gly Lys Leu Glu Ala Ala Ala Gln
                30                  35                  40

gtg agg cag gcg act aat cag att gtg atg aat tgt gct gat att gat 256
Val Arg Gln Ala Thr Asn Gln Ile Val Met Asn Cys Ala Asp Ile Asp
            45                  50                  55
att att aca gct tca tat gca cca gaa gga gat gaa gaa ata cat gct 304
Ile Ile Thr Ala Ser Tyr Ala Pro Glu Gly Asp Glu Glu Ile His Ala
        60                  65                  70
aca gga ttt aac tat cag aat gaa gat gaa aaa gtc acc ttg tct ttc 352
Thr Gly Phe Asn Tyr Gln Asn Glu Asp Glu Lys Val Thr Leu Ser Phe
    75                  80                  85
cct agt act ctg caa aca ggt acg gga acc tta aag ata gat ttt gtt 400
Pro Ser Thr Leu Gln Thr Gly Thr Gly Thr Leu Lys Ile Asp Phe Val
90                  95                  100                 105
gga gag ctg aat gac aaa atg aaa ggt ttc tat aga agt aaa tat act 448
Gly Glu Leu Asn Asp Lys Met Lys Gly Phe Tyr Arg Ser Lys Tyr Thr
                110                 115                 120
acc cct tct gga gag gtg cgc tat gct gct gta aca cag ttt gag gct 496
Thr Pro Ser Gly Glu Val Arg Tyr Ala Ala Val Thr Gln Phe Glu Ala
            125                 130                 135
act gat gcc cga agg gct ttt cct tgc tgg gat gag cct gct atc aaa 544
Thr Asp Ala Arg Arg Ala Phe Pro Cys Trp Asp Glu Pro Ala Ile Lys
        140                 145                 150
gca act ttt gat atc tca ttg gtt gtt cct aaa gac aga gta gct tta 592
Ala Thr Phe Asp Ile Ser Leu Val Val Pro Lys Asp Arg Val Ala Leu
    155                 160                 165
tca aac atg aat gta att gac cgg aaa cca tac cct gat gat gaa aat 640
Ser Asn Met Asn Val Ile Asp Arg Lys Pro Tyr Pro Asp Asp Glu Asn

170                 175                 180                 185

tta gtg gaa gtg aag ttt gcc cgc aca cct gtt atg tct aca tat ctg 688

Leu Val Glu Val Lys Phe Ala Arg Thr Pro Val Met Ser Thr Tyr Leu

                190                 195                 200

gtg gca ttt gtt gtg ggt gaa tat gac ttt gta gaa aca agg tca aaa 736

Val Ala Phe Val Val Gly Glu Tyr Asp Phe Val Glu Thr Arg Ser Lys

            205                 210                 215

gat ggt gtg tgt gtc cgt gtt tac act cct gtt ggc aaa gca gag caa 784

Asp Gly Val Cys Val Arg Val Tyr Thr Pro Val Gly Lys Ala Glu Gln

        220                 225                 230

gga aaa ttt gcg tta gag gtt gct gct aaa acc ttg cct ttt tat aag 832

Gly Lys Phe Ala Leu Glu Val Ala Ala Lys Thr Leu Pro Phe Tyr Lys

    235                 240                 245

gac tac ttc aat gtt cct tat cct cta cct aaa att gat ctc att gct 880

Asp Tyr Phe Asn Val Pro Tyr Pro Leu Pro Lys Ile Asp Leu Ile Ala

250                 255                 260                 265

att gca gac ttt gca gct ggt gcc atg gag aac tgg ggc ctt gtt act 928

Ile Ala Asp Phe Ala Ala Gly Ala Met Glu Asn Trp Gly Leu Val Thr

                270                 275                 280

tat agg gag act gca ttg ctt att gat cca aaa aat tcc tgt tct tca 976

Tyr Arg Glu Thr Ala Leu Leu Ile Asp Pro Lys Asn Ser Cys Ser Ser

            285                 290                 295

tcc cgc cag tgg gtt gct ctg gtt gtg gga cat gaa ctc gcc cat caa 1024

Ser Arg Gln Trp Val Ala Leu Val Val Gly His Glu Leu Ala His Gln

        300                 305                 310

tgg ttt gga aat ctt gtt act atg gaa tgg tgg act cat ctt tgg tta 1072

Trp Phe Gly Asn Leu Val Thr Met Glu Trp Trp Thr His Leu Trp Leu

    315                 320                 325

aat gaa ggt ttt gca tcc tgg att gaa tat ctg tgt gta gac cac tgc 1120

Asn Glu Gly Phe Ala Ser Trp Ile Glu Tyr Leu Cys Val Asp His Cys

330                 335                 340                 345

ttc cca gag tat gat att tgg act cag ttt gtt tct gct gat tac acc 1168

Phe Pro Glu Tyr Asp Ile Trp Thr Gln Phe Val Ser Ala Asp Tyr Thr

                350                 355                 360

cgt gcc cag gag ctt gac gcc tta gat aac agc cat cct att gaa gtc 1216

Arg Ala Gln Glu Leu Asp Ala Leu Asp Asn Ser His Pro Ile Glu Val

            365                 370                 375

agt gtg ggc cat cca tct gag gtt gat gag ata ttt gat gct ata tca 1264

Ser Val Gly His Pro Ser Glu Val Asp Glu Ile Phe Asp Ala Ile Ser

        380                 385                 390

tat agc aaa ggt gca tct gtc atc cga atg ctg cat gac tac att ggg 1312

Tyr Ser Lys Gly Ala Ser Val Ile Arg Met Leu His Asp Tyr Ile Gly

    395                 400                 405

gat aag gac ttt aag aaa gga atg aac atg tat tta acc aag ttc caa 1360

Asp Lys Asp Phe Lys Lys Gly Met Asn Met Tyr Leu Thr Lys Phe Gln

410                 415                 420                 425

caa aag aat gct gcc aca gag gat ctc tgg gaa agt tta gaa aat gct 1408

Gln Lys Asn Ala Ala Thr Glu Asp Leu Trp Glu Ser Leu Glu Asn Ala

                430                 435                 440

agt ggt aaa cct ata gca gct gtg atg aat acc tgg acc aaa caa atg 1456

Ser Gly Lys Pro Ile Ala Ala Val Met Asn Thr Trp Thr Lys Gln Met

            445                 450                 455

gga ttt ccc ctc att tat gtg gaa gct gaa cag gta gaa gat gac aga 1504

Gly Phe Pro Leu Ile Tyr Val Glu Ala Glu Gln Val Glu Asp Asp Arg
        460                 465                 470
tta ttg agg ttg tcc caa aag aag ttc tgt gct ggt ggg tca tat gtt 1552
Leu Leu Arg Leu Ser Gln Lys Lys Phe Cys Ala Gly Gly Ser Tyr Val
    475                 480                 485
ggt gaa gat tgt ccc cag tgg atg gtc cct atc aca atc tct act agt 1600
Gly Glu Asp Cys Pro Gln Trp Met Val Pro Ile Thr Ile Ser Thr Ser
490                 495                 500                 505
gaa gac ccc aac cag gcc aaa cta aaa att cta atg gac aag cca gag 1648
Glu Asp Pro Asn Gln Ala Lys Leu Lys Ile Leu Met Asp Lys Pro Glu
                510                 515                 520
atg aat gtg gtt ttg aaa aat gtc aaa cca gac caa tgg gtg aag tta 1696
Met Asn Val Val Leu Lys Asn Val Lys Pro Asp Gln Trp Val Lys Leu
            525                 530                 535
aac tta gga aca gtt ggg ttt tat cgg acc cag tac agc tct gcc atg 1744
Asn Leu Gly Thr Val Gly Phe Tyr Arg Thr Gln Tyr Ser Ser Ala Met
        540                 545                 550
ctg gaa agt tta tta cca ggc att cgt gac ctt tct ctg ccc cct gtg 1792
Leu Glu Ser Leu Leu Pro Gly Ile Arg Asp Leu Ser Leu Pro Pro Val
    555                 560                 565
gat cga ctt gga tta cag aat gac ctc ttc tcc ttg gct cga gct gga 1840
Asp Arg Leu Gly Leu Gln Asn Asp Leu Phe Ser Leu Ala Arg Ala Gly
570                 575                 580                 585
atc att agc act gta gag gtt cta aaa gtc atg gag gct ttt gtg aat 1888
Ile Ile Ser Thr Val Glu Val Leu Lys Val Met Glu Ala Phe Val Asn
                590                 595                 600
gag ccc aat tat act gta tgg agc gac ctg agc tgt aac ctg ggg att 1936

Glu Pro Asn Tyr Thr Val Trp Ser Asp Leu Ser Cys Asn Leu Gly Ile
            605                 610                 615
ctc tca act ctc ttg tcc cac aca gac ttc tat gag gaa atc cag gag 1984
Leu Ser Thr Leu Leu Ser His Thr Asp Phe Tyr Glu Glu Ile Gln Glu
        620                 625                 630
ttt gtg aaa gat gtc ttt tca cct ata ggg gag aga ctg ggc tgg gac 2032
Phe Val Lys Asp Val Phe Ser Pro Ile Gly Glu Arg Leu Gly Trp Asp
    635                 640                 645
ccc aaa cct gga gaa ggt cat ctc gat gca ctc ctg agg ggc ttg gtt 2080
Pro Lys Pro Gly Glu Gly His Leu Asp Ala Leu Leu Arg Gly Leu Val
650                 655                 660                 665
ctg gga aaa cta gga aaa gca gga cat aag gca acg tta gaa gaa gcc 2128
Leu Gly Lys Leu Gly Lys Ala Gly His Lys Ala Thr Leu Glu Glu Ala
                670                 675                 680
cgt cgt cgg ttt aag gac cac gtg gaa gga aaa cag att ctc tcc gct 2176
Arg Arg Arg Phe Lys Asp His Val Glu Gly Lys Gln Ile Leu Ser Ala
            685                 690                 695
gat ctg agg agt cct gtc tat ctg act gtt ttg aag cat ggt gat ggc 2224
Asp Leu Arg Ser Pro Val Tyr Leu Thr Val Leu Lys His Gly Asp Gly
        700                 705                 710
act act tta gat att atg tta aaa ctt cat aaa caa gca gat atg caa 2272
Thr Thr Leu Asp Ile Met Leu Lys Leu His Lys Gln Ala Asp Met Gln
    715                 720                 725
gaa gag aaa aac cga atc gaa aga gtc ctt ggc gct act ctt ttg cct 2320
Glu Glu Lys Asn Arg Ile Glu Arg Val Leu Gly Ala Thr Leu Leu Pro
730                 735                 740                 745

gac ctg att caa aaa gtc ctc acg ttt gca ctt tca gaa gag gta cgt 2368
Asp Leu Ile Gln Lys Val Leu Thr Phe Ala Leu Ser Glu Glu Val Arg
                750                 755                 760
cca cag gac act gta tcg gta att ggt gga gta gct gga ggc agc aag 2416
Pro Gln Asp Thr Val Ser Val Ile Gly Gly Val Ala Gly Gly Ser Lys
            765                 770                 775
cat ggt agg aaa gct gct tgg aaa ttc ata aag gac aac tgg gaa gaa 2464
His Gly Arg Lys Ala Ala Trp Lys Phe Ile Lys Asp Asn Trp Glu Glu
        780                 785                 790
ctt tat aac cga tac cag gga gga ttc tta ata tcc aga cta ata aag 2512
Leu Tyr Asn Arg Tyr Gln Gly Gly Phe Leu Ile Ser Arg Leu Ile Lys
    795                 800                 805
cta tca gtt gag gga ttt gca gtt gat aaa atg gct gga gag gtt aag 2560
Leu Ser Val Glu Gly Phe Ala Val Asp Lys Met Ala Gly Glu Val Lys
810                 815                 820                 825
gct ttc ttc gag agt cac cca gct cct tca gct gag cgt acc atc cag 2608
Ala Phe Phe Glu Ser His Pro Ala Pro Ser Ala Glu Arg Thr Ile Gln
                830                 835                 840
cag tgt tgt gaa aat att ctg ctg aat gct gcc tgg cta aag cga gat 2656
Gln Cys Cys Glu Asn Ile Leu Leu Asn Ala Ala Trp Leu Lys Arg Asp
            845                 850                 855
gct gag agc atc cac cag tac ctc ctt cag cgg aag gcc tca cca ccc 2704
Ala Glu Ser Ile His Gln Tyr Leu Leu Gln Arg Lys Ala Ser Pro Pro
        860                 865                 870
aca gtg tgaatcctga ggtgccgcca ttggcggttc tgctgcttcg ctgcagggat 2760
Thr Val
    875

aaggtggagc taccgaacag ctgattcata tgccaagaat ttggagtctt ctttcaaacc 2820
agtgggggtt ggacaatgaa tgtagttaac tggttcctgc tcacactcca gaattaaatt 2880
ctattgaaaa aggaaaatca gcaattcagc aaaaaaataa ataaaaaata aaaatgtaaa 2940
tatgatagta ataaaataga gcataacgaa actgtgaaac tttctgaagc cttgtcagtg 3000
gttaaaagta tttaacactc tactgttaat gacagatgtt ctgtttttat aacctaccaa 3060
aaggaaacta gaggcttctt ggtgaagagc atttttgtga agtgggttct gcaaggagcc 3120
tataaagcca agggtggtgt ccatttctgg gaatggttaa acacaaaagg ctgatagctg 3180
gtatcacata gttggagtca gtgcataatt ccaagtggct tttttttttt ttggcacggg 3240
gactgatcag gaagatatat tcctgcataa ctcaatctga accaaggatt gtagtttagt 3300
tttcctcctt gccttccctt ctgtgtgacc gaccccttgg ccaaaaaaaa aacaaaaagc 3360
aaaaaacaaa aacctaccct gttctggttt ttttcctccc tttagttcca cccccaaccc 3420
ccattccctg gtgtccttct tagagatgaa gaaataataa ggaaacatct ttcatagcca 3480
cattaaataa gagaaactga tatacattat tttttctttt ttaaagatga cttataagaa 3540
ccctgaaatt tatataggtg agacaataga aataaaaaga tcttcagcca ggcctttctg 3600
aaggagttat tctgctaaaa atggtcttag ttgtctgaaa agccagctct tgaacctctt 3660
cacaacagta tcaacactgg cttctcccgg ttcattttat gcgtgcgaga agtcagtggt 3720
aactgctgca gggcttaata cattagtggt aactggttta aaaaacaaag actgtaagcc 3780
tgtgtgtgcc actgtttgct tcaacagtat atcctactaa taagcctcac ctatttaatc 3840
caatgagttt taaatctaaa tctcattccc ttcttctttc cctacctttt ttttcttttt 3900
ttcttaaaaa aatattttgt gttattaaca gaaattcata tttggtgtgg cttaacggta 3960
tttcagaagg tcatcagatt gtgagactgc ttccttgaaa catttttgtg ctattgtttt 4020
aaaaaaataa ttaaaaaaca gttggcgtt 4049

<210> 10
<211> 875
<212> PRT
<213> Homo sapiens

<400> 10

Met Pro Glu Lys Arg Pro Phe Glu Arg Leu Pro Ala Asp Val Ser Pro

1               5                   10                  15

Ile Asn Tyr Ser Leu Cys Leu Lys Pro Asp Leu Leu Asp Phe Thr Phe

            20                  25                  30

Glu Gly Lys Leu Glu Ala Ala Ala Gln Val Arg Gln Ala Thr Asn Gln

        35                  40                  45

Ile Val Met Asn Cys Ala Asp Ile Asp Ile Ile Thr Ala Ser Tyr Ala

    50                  55                  60

Pro Glu Gly Asp Glu Glu Ile His Ala Thr Gly Phe Asn Tyr Gln Asn

65                  70                  75                  80

Glu Asp Glu Lys Val Thr Leu Ser Phe Pro Ser Thr Leu Gln Thr Gly

                85                  90                  95

Thr Gly Thr Leu Lys Ile Asp Phe Val Gly Glu Leu Asn Asp Lys Met

            100                 105                 110

Lys Gly Phe Tyr Arg Ser Lys Tyr Thr Thr Pro Ser Gly Glu Val Arg

        115                 120                 125

Tyr Ala Ala Val Thr Gln Phe Glu Ala Thr Asp Ala Arg Arg Ala Phe

    130                 135                 140

Pro Cys Trp Asp Glu Pro Ala Ile Lys Ala Thr Phe Asp Ile Ser Leu

145                 150                 155                 160

Val Val Pro Lys Asp Arg Val Ala Leu Ser Asn Met Asn Val Ile Asp

                165                 170                 175

Arg Lys Pro Tyr Pro Asp Asp Glu Asn Leu Val Glu Val Lys Phe Ala

            180                 185                 190

Arg Thr Pro Val Met Ser Thr Tyr Leu Val Ala Phe Val Val Gly Glu

        195                 200                 205

Tyr Asp Phe Val Glu Thr Arg Ser Lys Asp Gly Val Cys Val Arg Val

    210                 215                 220

Tyr Thr Pro Val Gly Lys Ala Glu Gln Gly Lys Phe Ala Leu Glu Val

225                 230                 235                 240

Ala Ala Lys Thr Leu Pro Phe Tyr Lys Asp Tyr Phe Asn Val Pro Tyr

                245                 250                 255

Pro Leu Pro Lys Ile Asp Leu Ile Ala Ile Ala Asp Phe Ala Ala Gly

            260                 265                 270

Ala Met Glu Asn Trp Gly Leu Val Thr Tyr Arg Glu Thr Ala Leu Leu

        275                 280                 285

Ile Asp Pro Lys Asn Ser Cys Ser Ser Ser Arg Gln Trp Val Ala Leu

    290                 295                 300

Val Val Gly His Glu Leu Ala His Gln Trp Phe Gly Asn Leu Val Thr

305                 310                 315                 320

Met Glu Trp Trp Thr His Leu Trp Leu Asn Glu Gly Phe Ala Ser Trp

                325                 330                 335

Ile Glu Tyr Leu Cys Val Asp His Cys Phe Pro Glu Tyr Asp Ile Trp

            340                 345                 350

Thr Gln Phe Val Ser Ala Asp Tyr Thr Arg Ala Gln Glu Leu Asp Ala

        355                 360                 365

Leu Asp Asn Ser His Pro Ile Glu Val Ser Val Gly His Pro Ser Glu

    370                 375                 380

Val Asp Glu Ile Phe Asp Ala Ile Ser Tyr Ser Lys Gly Ala Ser Val

385                 390                 395                 400

Ile Arg Met Leu His Asp Tyr Ile Gly Asp Lys Asp Phe Lys Lys Gly

                405                 410                 415

Met Asn Met Tyr Leu Thr Lys Phe Gln Gln Lys Asn Ala Ala Thr Glu
            420                 425                 430
Asp Leu Trp Glu Ser Leu Glu Asn Ala Ser Gly Lys Pro Ile Ala Ala
        435                 440                 445
Val Met Asn Thr Trp Thr Lys Gln Met Gly Phe Pro Leu Ile Tyr Val
    450                 455                 460
Glu Ala Glu Gln Val Glu Asp Asp Arg Leu Leu Arg Leu Ser Gln Lys
465                 470                 475                 480
Lys Phe Cys Ala Gly Gly Ser Tyr Val Gly Glu Asp Cys Pro Gln Trp
                485                 490                 495
Met Val Pro Ile Thr Ile Ser Thr Ser Glu Asp Pro Asn Gln Ala Lys
            500                 505                 510
Leu Lys Ile Leu Met Asp Lys Pro Glu Met Asn Val Val Leu Lys Asn
        515                 520                 525
Val Lys Pro Asp Gln Trp Val Lys Leu Asn Leu Gly Thr Val Gly Phe
    530                 535                 540
Tyr Arg Thr Gln Tyr Ser Ser Ala Met Leu Glu Ser Leu Leu Pro Gly
545                 550                 555                 560
Ile Arg Asp Leu Ser Leu Pro Pro Val Asp Arg Leu Gly Leu Gln Asn
                565                 570                 575
Asp Leu Phe Ser Leu Ala Arg Ala Gly Ile Ile Ser Thr Val Glu Val
            580                 585                 590
Leu Lys Val Met Glu Ala Phe Val Asn Glu Pro Asn Tyr Thr Val Trp
        595                 600                 605
Ser Asp Leu Ser Cys Asn Leu Gly Ile Leu Ser Thr Leu Leu Ser His
    610                 615                 620

Thr Asp Phe Tyr Glu Glu Ile Gln Glu Phe Val Lys Asp Val Phe Ser
625                 630                 635                 640
Pro Ile Gly Glu Arg Leu Gly Trp Asp Pro Lys Pro Gly Glu Gly His
                645                 650                 655
Leu Asp Ala Leu Leu Arg Gly Leu Val Leu Gly Lys Leu Gly Lys Ala
            660                 665                 670
Gly His Lys Ala Thr Leu Glu Glu Ala Arg Arg Arg Phe Lys Asp His
        675                 680                 685
Val Glu Gly Lys Gln Ile Leu Ser Ala Asp Leu Arg Ser Pro Val Tyr
    690                 695                 700
Leu Thr Val Leu Lys His Gly Asp Gly Thr Thr Leu Asp Ile Met Leu
705                 710                 715                 720
Lys Leu His Lys Gln Ala Asp Met Gln Glu Glu Lys Asn Arg Ile Glu
                725                 730                 735
Arg Val Leu Gly Ala Thr Leu Leu Pro Asp Leu Ile Gln Lys Val Leu
            740                 745                 750
Thr Phe Ala Leu Ser Glu Glu Val Arg Pro Gln Asp Thr Val Ser Val
        755                 760                 765
Ile Gly Gly Val Ala Gly Gly Ser Lys His Gly Arg Lys Ala Ala Trp
    770                 775                 780
Lys Phe Ile Lys Asp Asn Trp Glu Glu Leu Tyr Asn Arg Tyr Gln Gly
785                 790                 795                 800
Gly Phe Leu Ile Ser Arg Leu Ile Lys Leu Ser Val Glu Gly Phe Ala
                805                 810                 815
Val Asp Lys Met Ala Gly Glu Val Lys Ala Phe Phe Glu Ser His Pro
            820                 825                 830
Ala Pro Ser Ala Glu Arg Thr Ile Gln Gln Cys Cys Glu Asn Ile Leu

835                 840                 845

Leu Asn Ala Ala Trp Leu Lys Arg Asp Ala Glu Ser Ile His Gln Tyr

    850                 855                 860

Leu Leu Gln Arg Lys Ala Ser Pro Pro Thr Val

865                 870                 875

<210> 11

<211> 2007

<212> DNA

<213> Homo sapiens

<220>

<221> CDS

<222> (1124)..(1330)

<400> 11

tctaaaagcc cccttatacc ccactttgtg cagcaaagat ccccgtgcag gtcacagcct 60

gatttgtggc caggctggac aaattcctga ggcacaactt ggcttcagtt cagatttcaa 120

gctgtgttgg tgttgggacc agcagaaggc aaacgtccag ccaacacaca ggactgtaag 180

aggactctga gctacgtgcc ctgtgaagac ccccaggctt tgtcatagga ggtcgttcag 240

cttccccaaa gtcagaggtg atttgatttg gggaagactg aatattcaca cctaagtcgt 300

gagcatatcc tgagttttac ttccttatgg cttgccctcc aagttctctc tctcatacac 360

acacacaccc ttgctccaga atcaccagac acctccatgg ctccagctat gggaacagct 420

gcattggggc tgcctttctg tttggcttag gaacttctgt gcttcttgtg gctccactcg 480

cgaggcagct cggaggtgtg gactccgatt ggctgcagg cagctctggg acggcacagg 540

gcgggcgctc tgatcagctc gtgtaaaaca caccgtcttc ttggcctcct ggcagttctt 600

tctgcgaata gtcctctccc tggccagttg aatgggggaa gctgctggca caggaaggag 660

aggcgatccc ggctgaggct taggaaattg ctggagccgg ctccaagcag ataattcact 720

ggggaggttt tcagagtcaa acatcattct gcctgtkttg ggggccaggt gtgtcacaca 780

agcatctcaa agtcaaaagc catctggggc tgctgcttct ctttctcagg ctctggggaa 840

aggaatctcc ctctcctctc acttgattcc aagtgtggtt gaattgtctg gagcactggg 900

actttttttc tcttttcctt gatggaccaa cagtgcaaat gcaatctcgc catttaactt 960

tcaggtcgat ttcctttcct gatcagacat ctttgtgccc cctttaggaa ggaaaagaat 1020

acacctacga tgtgccaggc actgtgttag gcgcttttat atagatcctc gttaggatga 1080

gactaaggga tgaggacatc tctttataaa aggcccctaa gta atg gat aaa cag 1135



1

aaa cac tta gag gtg aga agg tct gtc ttc aag atc caa ggt aag att 1183



5                   10                  15                  20

gcc ttc agt ctg atg ttt gtt ctc aag gac tta tcc cct aca ata ttc 1231



                25                  30                  35

tcc cac tcc ata ctt ctc ctt cta ccc cac cat gtg ctc ccg tgc act 1279



            40                  45                  50

cct cag atg gtc aga ggg gta acc caa gtc ctt aga gaa ttt ggg gac 1327



        55                  60                  65

caa tagaatatgt gatgtgtgaa ttttctttaa aaaacttaag gagtctttgc 1380



taccttctgc ttgttgagtt gttttggcat tcatattaaa agccagcatc tcactattta 1440

ttgacaggtt gggctgtgtg tgtgcgcatg tgtgtataca tttccaggcg tgcctgtgtc 1500

ctgtagcttt ttaaaaggaa acccagtcat cccactatga atctggcatc ttcttatgct 1560

tctagtgttt tggccataca tcaaccaagg ggtttaattt atccaatgct tgacgacatg 1620

ttcaggaggg gctggatcaa attttgagag ggttatggga aagggagggg gagaagaaat 1680

tgacatttat tttattattt attttaaatg tttacatctt ctttatgttg tatcaagcct 1740

gaatagaaac tgatagcatt aaaatactcc gttcctctct ctcttctcgc ttcctttttt 1800

ttttttttta aatttaggat aacacatttt tgtttctaaa gtgatttgtg atttgtgctg 1860

tataaactgt ataaaaggtt ctgtttttaa aggtggattt tcattcctct ggggacagtg 1920

gtcgccaaga catctacatt gtaagagaac acagtggaag atcctgtcct gattctcaaa 1980

aattattttc tctgtatgat taaaagt 2007

<210> 12

<211> 69

<212> PRT

<213> Homo sapiens

<400> 12

<210> 13

<211> 1953

<212> DNA

<213> Homo sapiens

<220>

<221> CDS

<222> (135)..(1850)

<400> 13
acgcctgcca ggagcaagcc gaagagccag ccggccggcg cactccgact ccgagcagtc 60
tctgtccttc gacccgagcc ccgcgccctt tccgggaccc ctgccccgcg ggcagcgctg 120
ccaacctgcc ggcc atg gag acc ccg tcc cag cgg cgc gcc acc cgc agc 170
ggg gcg cag gcc agc tcc act ccg ctg tcg ccc acc cgc atc acc cgg 218
ctg cag gag aag gag gac ctg cag gag ctc aat gat cgc ttg gcg gtc 266
tac atc gac cgt gtg cgc tcg ctg gaa acg gag aac gca ggg ctg cgc 314
ctt cgc atc acc gag tct gaa gag gtg gtc agc cgc gag gtg tcc ggc 362
atc aag gcc gcc tac gag gcc gag ctc ggg gat gcc cgc aag acc ctt 410
gac tca gta gcc aag gag cgc gcc cgc ctg cag ctg gag ctg agc aaa 458
gtg cgt gag gag ttt aag gag ctg aaa gcg cgc aat acc aag aag gag 506
ggt gac ctg ata gct gct cag gct cgg ctg aag gac ctg gag gct ctg 554
ctg aac tcc aag gag gcc gca ctg agc act gct ctc agt gag aag cgc 602
acg ctg gag ggc gag ctg cat gat ctg cgg ggc cag gtg gcc aag ctt 650
gag gca gcc cta ggt gag gcc aag aag caa ctt cag gat gag atg ctg 698
cgg cgg gtg gat gct gag aac agg ctg cag acc atg aag gag gaa ctg 746
gac ttc cag aag aac atc tac agt gag gag ctg cgt gag acc aag cgc 794
cgt cat gag acc cga ctg gtg gag att gac aat ggg aag cag cgt gag 842
ttt gag agc cgg ctg gcg gat gcg ctg cag gaa ctg cgg gcc cag cat 890
gag gac cag gtg gag cag tat aag aag gag ctg gag aag act tat tct 938
gcc aag ctg gac aat gcc agg cag tct gct gag agg aac agc aac ctg 986
gtg ggg gct gcc cac gag gag ctg cag cag tcg cgc atc cgc atc gac 1034
agc ctc tct gcc cag ctc agc cag ctc cag aag cag ctg gca gcc aag 1082
gag gcg aag ctt cga gac ctg gag gac tca ctg gcc cgt gag cgg gac 1130
acc agc cgg cgg ctg ctg gcg gaa aag gag cgg gag atg gcc gag atg 1178
cgg gca agg atg cag cag cag ctg gac gag tac cag gag ctt ctg gac 1226
atc aag ctg gcc ctg gac atg gag atc cac gcc tac cgc aag ctc ttg 1274
gag ggc gag gag gag agg cta cgc ctg tcc ccc agc cct acc tcg cag 1322
cgc agc cgt ggc cgt gct tcc tct cac tca tcc cag aca cag ggt ggg 1370
ggc agc gtc acc aaa aag cgc aaa ctg gag tcc act gag agc cgc agc 1418
agc ttc tca cag cac gca cgc act agc ggg cgc gtg gcc gtg gag gag 1466
gtg gat gag gag ggc aag ttt gtc cgg ctg cgc aac aag tcc aat gag 1514
gac cag tcc atg ggc aat tgg cag atc aag cgc cag aat gga gat gat 1562
ccc ttg ctg act tac cgg ttc cca cca aag ttc acc ctg aag gct ggg 1610
cag gtg gtg acg atc tgg gct gca gga gct ggg gcc acc cac agc ccc 1658
cct acc gac ctg gtg tgg aag gca cag aac acc tgg ggc tgc ggg aac 1706
agc ctg cgt acg gct ctc atc aac tcc act ggg gaa gaa gtg gcc atg 1754
cgc aag ctg gtg cgc tca gtg act gtg gtt gag gac gac gag gat gag    1802
gat gga gat gac ctg ctc cat cac cac cac gtg agt ggt agc cgc cgc    1850
tgaggccgag cctgcactgg ggccaccagc caggcctggg ggcagcctct ccccagcctc 1910
cccgtgccaa aaatcttttc attaaagaat gttttggaac ttt                   1953

<210> 14
<211> 572
<212> PRT
<213> Homo sapiens
<400> 14

<210> 15
<211> 2865
<212> DNA
<213> Homo sapiens
<220>
<221> CDS
<222> (240)..(1475)
<400> 15
   cacaccgcca gtctgtgcgc tgagtcggag ccagaggccg cggggacacc gggccatgca 60
   cgcccccaac tgaagctgca tctcaaagcc gaagattcca gcagcccagg ggatttcaaa 120
   gagctcagac tcagaggaac atctgcggag agacccccga agccctctcc agggcagtcc 180
   tcatccagac gctccgttag tgcagacagg agcgcgcagt ggccccggct cgccgcgcc 239
   atg gag cgg atc ccc agc gcg caa cca ccc ccc gcc tgc ctg ccc aaa 287
   Met Glu Arg Ile Pro Ser Ala Gln Pro Pro Pro Ala Cys Leu Pro Lys
   1 5 10 15
   gca ccg gga ctg gag cac cga gac cta cca ggg atg tac cct gcc cac 335
   Ala Pro Gly Leu Glu His Arg Asp Leu Pro Gly Met Tyr Pro Ala His
   20 25 30
   atg tac caa gtg tac aag tca aga cgg gga ata aag cgg agc gag gac 383
   Met Tyr Gln Val Tyr Lys Ser Arg Arg Gly Ile Lys Arg Ser Glu Asp
   35 40 45
   agc aag gag acc tac aaa ttg ccg cac cgg ctc ttc gag aaa aag aga 431
   Ser Lys Glu Thr Tyr Lys Leu Pro His Arg Leu Phe Glu Lys Lys Arg
   50 55 60
   cgt gac cgg att aac gag tgc atc gcc cag ctg aag gat ctc cta ccc 479
   Arg Asp Arg Ile Asn Glu Cys Ile Ala Gln Leu Lys Asp Leu Leu Pro
   65 70 75 80
   gaa cat ctc aaa ctt aca act ttg ggt cac ttg gaa aaa gca gtg gtt 527
   Glu His Leu Lys Leu Thr Thr Leu Gly His Leu Glu Lys Ala Val Val
   85 90 95
   ctt gaa ctt acc ttg aag cat gtg aaa gca cta aca aac cta att gat 575
   Leu Glu Leu Thr Leu Lys His Val Lys Ala Leu Thr Asn Leu Ile Asp
   100 105 110
   cag cag cag cag aaa atc att gcc ctg cag agt ggt tta caa gct ggt 623
   Gln Gln Gln Gln Lys Ile Ile Ala Leu Gln Ser Gly Leu Gln Ala Gly
   115 120 125
   gag ctg tca ggg aga aat gtc gaa aca ggt caa gag atg ttc tgc tca 671
   Glu Leu Ser Gly Arg Asn Val Glu Thr Gly Gln Glu Met Phe Cys Ser
   130 135 140
   ggt ttc cag aca tgt gcc cgg gag gtg ctt cag tat ctg gcc aag cac 719
   Gly Phe Gln Thr Cys Ala Arg Glu Val Leu Gln Tyr Leu Ala Lys His
   145 150 155 160
   gag aac act cgg gac ctg aag tct tcg cag ctt gtc acc cac ctc cac 767
   Glu Asn Thr Arg Asp Leu Lys Ser Ser Gln Leu Val Thr His Leu His
   165 170 175
   cgg gtg gtc tcg gag ctg ctg cag ggt ggt acc tcc agg aag cca tca 815
   Arg Val Val Ser Glu Leu Leu Gln Gly Gly Thr Ser Arg Lys Pro Ser
   180 185 190
   gac cca gct ccc aaa gtg atg gac ttc aag gaa aaa ccc agc tct ccg 863
   Asp Pro Ala Pro Lys Val Met Asp Phe Lys Glu Lys Pro Ser Ser Pro
   195 200 205
   gcc aaa ggt tcg gaa ggt cct ggg aaa aac tgc gtg cca gtc atc cag 911
   Ala Lys Gly Ser Glu Gly Pro Gly Lys Asn Cys Val Pro Val Ile Gln
   210 215 220
   cgg act ttc gct cac tcg agt ggg gag cag agc ggc agc gac acg gac 959
   Arg Thr Phe Ala His Ser Ser Gly Glu Gln Ser Gly Ser Asp Thr Asp
   225 230 235 240
   aca gac agt ggc tat gga gga gat tcg gag aag ggc gac ttg cgc agt 1007
   Thr Asp Ser Gly Tyr Gly Gly Asp Ser Glu Lys Gly Asp Leu Arg Ser
   245 250 255
   gag cag ccg tgc ttc aaa agt gac cac gga cgc agg ttc acg atg gga 1055
   Glu Gln Pro Cys Phe Lys Ser Asp His Gly Arg Arg Phe Thr Met Gly
   260 265 270
   gaa agg atc ggc gca att aag caa gag tcc gaa gaa ccc ccc aca aaa 1103
   Glu Arg Ile Gly Ala Ile Lys Gln Glu Ser Glu Glu Pro Pro Thr Lys
   275 280 285
   aag aac cgg atg cag ctt tcg gat gat gaa ggc cat ttc act agc agt 1151
   Lys Asn Arg Met Gln Leu Ser Asp Asp Glu Gly His Phe Thr Ser Ser
   290 295 300
   gac ctg atc agc tcc ccg ttc ctg ggc cca cac cca cac cag cct cct 1199
   Asp Leu Ile Ser Ser Pro Phe Leu Gly Pro His Pro His Gln Pro Pro
   305 310 315 320
   ttc tgc ctg ccc ttc tac ctg atc cca cct tca gcg act gcc tac ctg 1247
   Phe Cys Leu Pro Phe Tyr Leu Ile Pro Pro Ser Ala Thr Ala Tyr Leu
   325 330 335
   ccc atg ctg gag aag tgc tgg tat ccc acc tca gtg cca gtg cta tac 1295
   Pro Met Leu Glu Lys Cys Trp Tyr Pro Thr Ser Val Pro Val Leu Tyr
   340 345 350
   cca ggc ctc aac gcc tct gcc gca gcc ctc tct agc ttc atg aac cca 1343
   Pro Gly Leu Asn Ala Ser Ala Ala Ala Leu Ser Ser Phe Met Asn Pro
   355 360 365
   gac aag atc tcg gct ccc ttg ctc atg ccc cag aga ctc cct tct ccc 1391
   Asp Lys Ile Ser Ala Pro Leu Leu Met Pro Gln Arg Leu Pro Ser Pro
   370 375 380
   ttg cca gct cat ccg tcc gtc gac tct tct gtc ttg ctc caa gct ctg 1439
   Leu Pro Ala His Pro Ser Val Asp Ser Ser Val Leu Leu Gln Ala Leu
   385 390 395 400
   aag cca atc ccc cct tta aac tta gaa acc aaa gac taaactctct 1485
   Lys Pro Ile Pro Pro Leu Asn Leu Glu Thr Lys Asp
   405 410
   aggggatcct gctgcttngc tttcctncct cgctacttcc taaaaagcaa ccnnaaagnt 1545
   ttngtgaatg ctgnnagant gttgcattgt gtatactgag ataatctgag gcatggagag 1605
   caganncagg gtgtgtgtgt gtgtgtgtgt gtgtgtgtgt gtatgtgcgt gtgcgtgcac 1665
   atgtgtgcct gcgtgttggt ataggacttt anngctcctt nnggcatagg gaagtcacga 1725
   aggattgctn gacatcagga gactnggggg ggattgtagc agacgtctgg gcttnncccc 1785
   acccagagaa tagcccccnn cnanacanat cagctggatt tacaaaagct tcaaagtctt 1845
   ggtctgtgag tcactcttca gtttgggagc tgggtctgtg gctttgatca gaaggtactt 1905
   tcaaaagagg gctttccagg gctcagctcc caaccagctg ttaggacccc acccttttgc 1965
   ctttattgtc gacgtgactc accagacgtc ggggagagag agcagtcaga ccgagctttt 2025
   ctgctaacat ggggagggta gcagacactg gcatagcacg gtagtggttt gggggagggt 2085
   ttccgcaggt ctgctcccca cccctgcctc ggaagaataa agagaatgta gttccctact 2145
   caggctttcg tagtgattag cttactaagg aactgaaaat gggccccttg tacaagctga 2205
   gctgccccgg agggagggag gagttccctg ggcttctggc acctgtttct aggcctaacc 2265
   attagtactt actgtgcagg gaaccaaacc aaggtctgag aaatgcggac ancccgagcg 2325
   agcaccccaa agtgcacaaa gctgagtaaa aagctgcccc cttcaaacag aactagactc 2385
   agttttcaat tccatcctaa aactcctttt aaccaagctt agcttctcaa agggctaacc 2445
   aagccttgga accgccagat cctttctgta ggctaattcc tcttggccaa cggcatatgg 2505
   agtgtcctta ttgctaaaaa ggattccgnc tccttcaaag aagttttatt tttggtccag 2565
   agtacttgtt ttcccgatgt gtccagccag ctccgcagca gcttttcaaa atgcactatg 2625
   cctgattgct gatcgtgttt taactttttc ttttcctgtt tttattttgg tattaagtcg 2685
   ctggctttat ttgtaaagct gttataaata tatattatat naantatatt aaaaaggaaa 2745
   ntgttncaga tgtttatttg tataattact tgattcacan agngagaaaa antgantgta 2805
   ttcctgtntt ngaagagaag annaattttt tttttctcta gggagaggta cagngttnnt 2865

<210> 16

<211> 412

<212> PRT

<213> Homo sapiens

<400> 16

Met Glu Arg Ile Pro Ser Ala Gln Pro Pro Pro Ala Cys Leu Pro Lys
1               5                   10                  15

Ala Pro Gly Leu Glu His Arg Asp Leu Pro Gly Met Tyr Pro Ala His
            20                  25                  30

Met Tyr Gln Val Tyr Lys Ser Arg Arg Gly Ile Lys Arg Ser Glu Asp

        35                  40                  45
Ser Lys Glu Thr Tyr Lys Leu Pro His Arg Leu Phe Glu Lys Lys Arg
    50                  55                  60
Arg Asp Arg Ile Asn Glu Cys Ile Ala Gln Leu Lys Asp Leu Leu Pro
65                  70                  75                  80
Glu His Leu Lys Leu Thr Thr Leu Gly His Leu Glu Lys Ala Val Val
                85                  90                  95
Leu Glu Leu Thr Leu Lys His Val Lys Ala Leu Thr Asn Leu Ile Asp
            100                 105                 110
Gln Gln Gln Gln Lys Ile Ile Ala Leu Gln Ser Gly Leu Gln Ala Gly
        115                 120                 125
Glu Leu Ser Gly Arg Asn Val Glu Thr Gly Gln Glu Met Phe Cys Ser
    130                 135                 140
Gly Phe Gln Thr Cys Ala Arg Glu Val Leu Gln Tyr Leu Ala Lys His
145                 150                 155                 160
Glu Asn Thr Arg Asp Leu Lys Ser Ser Gln Leu Val Thr His Leu His
                165                 170                 175
Arg Val Val Ser Glu Leu Leu Gln Gly Gly Thr Ser Arg Lys Pro Ser
            180                 185                 190
Asp Pro Ala Pro Lys Val Met Asp Phe Lys Glu Lys Pro Ser Ser Pro
        195                 200                 205
Ala Lys Gly Ser Glu Gly Pro Gly Lys Asn Cys Val Pro Val Ile Gln
    210                 215                 220
Arg Thr Phe Ala His Ser Ser Gly Glu Gln Ser Gly Ser Asp Thr Asp
225                 230                 235                 240
Thr Asp Ser Gly Tyr Gly Gly Asp Ser Glu Lys Gly Asp Leu Arg Ser
                245                 250                 255

Glu Gln Pro Cys Phe Lys Ser Asp His Gly Arg Arg Phe Thr Met Gly
            260                 265                 270
Glu Arg Ile Gly Ala Ile Lys Gln Glu Ser Glu Glu Pro Pro Thr Lys
        275                 280                 285
Lys Asn Arg Met Gln Leu Ser Asp Asp Glu Gly His Phe Thr Ser Ser
    290                 295                 300
Asp Leu Ile Ser Ser Pro Phe Leu Gly Pro His Pro His Gln Pro Pro
305                 310                 315                 320
Phe Cys Leu Pro Phe Tyr Leu Ile Pro Pro Ser Ala Thr Ala Tyr Leu
                325                 330                 335
Pro Met Leu Glu Lys Cys Trp Tyr Pro Thr Ser Val Pro Val Leu Tyr
            340                 345                 350
Pro Gly Leu Asn Ala Ser Ala Ala Ala Leu Ser Ser Phe Met Asn Pro
        355                 360                 365
Asp Lys Ile Ser Ala Pro Leu Leu Met Pro Gln Arg Leu Pro Ser Pro
    370                 375                 380
Leu Pro Ala His Pro Ser Val Asp Ser Ser Val Leu Leu Gln Ala Leu
385                 390                 395                 400
Lys Pro Ile Pro Pro Leu Asn Leu Glu Thr Lys Asp
                405                 410

<210> 17
<211> 3817
<212> DNA
<213> Homo sapiens
<220>
<221> CDS

<222> (164)..(2665)

<400> 17

tgaattcgtg agagacttga gggaggcgct gcgactgaca agcggctctg cccgggacct 60

tctcgctttc atctagcgct gcactcaatg gaggggcggg caccgcagtg cttaatgctg 120

tcttaactag tgtaggaaaa cggctcaacc caccgctgcc gaa atg aag tat aag 175

Met Lys Tyr Lys

1

aat ctt atg gca agg gcc tta tat gac aat gtc cca gag tgt gcc gag 223

Asn Leu Met Ala Arg Ala Leu Tyr Asp Asn Val Pro Glu Cys Ala Glu

5                   10                  15                  20

gaa ctg gcc ttt cgc aag gga gac atc ctg acc gtc ata gag cag aac 271

Glu Leu Ala Phe Arg Lys Gly Asp Ile Leu Thr Val Ile Glu Gln Asn

                25                  30                  35

aca ggg gga ctg gaa gga tgg tgg ctg tgc tcg tta cac ggt cgg caa 319

Thr Gly Gly Leu Glu Gly Trp Trp Leu Cys Ser Leu His Gly Arg Gln

            40                  45                  50

ggc att gtc cca ggc aac cgg gtg aag ctt ctg att ggt ccc atg cag 367

Gly Ile Val Pro Gly Asn Arg Val Lys Leu Leu Ile Gly Pro Met Gln

        55                  60                  65

gag act gcc tcc agt cac gag cag cct gcc tct gga ctg atg cag cag 415

Glu Thr Ala Ser Ser His Glu Gln Pro Ala Ser Gly Leu Met Gln Gln

    70                  75                  80

acc ttt ggc caa cag aag ctc tat caa gtg cca aac cca cag gct gct 463

Thr Phe Gly Gln Gln Lys Leu Tyr Gln Val Pro Asn Pro Gln Ala Ala

85                  90                  95                  100

ccc cga gac acc atc tac caa gtg cca cct tcc tac caa aat cag gga 511

Pro Arg Asp Thr Ile Tyr Gln Val Pro Pro Ser Tyr Gln Asn Gln Gly
                105                 110                 115
att tac caa gtc ccc act ggc cac ggc acc caa gaa caa gag gta tat 559
Ile Tyr Gln Val Pro Thr Gly His Gly Thr Gln Glu Gln Glu Val Tyr
            120                 125                 130
cag gtg cca cca tca gtg cag aga agc att ggg gga acc agt ggg ccc 607
Gln Val Pro Pro Ser Val Gln Arg Ser Ile Gly Gly Thr Ser Gly Pro
        135                 140                 145
cac gtg ggt aaa aag gtg ata acc ccc gtg agg aca ggc cat ggc tac 655
His Val Gly Lys Lys Val Ile Thr Pro Val Arg Thr Gly His Gly Tyr
    150                 155                 160
gta tac gag tac cca tcc aga tac caa aag gat gtc tat gat atc cct 703
Val Tyr Glu Tyr Pro Ser Arg Tyr Gln Lys Asp Val Tyr Asp Ile Pro
165                 170                 175                 180
cct tct cat acc act caa ggg gta tac gac atc cct ccc tca tca gca 751
Pro Ser His Thr Thr Gln Gly Val Tyr Asp Ile Pro Pro Ser Ser Ala
                185                 190                 195
aaa ggc cct gtg ttt tca gtt cca gtg gga gag ata aaa cct caa ggg 799
Lys Gly Pro Val Phe Ser Val Pro Val Gly Glu Ile Lys Pro Gln Gly
            200                 205                 210
gtg tat gac atc ccg cct aca aaa ggg gta tat gcc att ccg ccc tct 847
Val Tyr Asp Ile Pro Pro Thr Lys Gly Val Tyr Ala Ile Pro Pro Ser
        215                 220                 225
gct tgc cgg gat gaa gca ggg ctt agg gaa aaa gac tat gac ttc ccc 895
Ala Cys Arg Asp Glu Ala Gly Leu Arg Glu Lys Asp Tyr Asp Phe Pro
    230                 235                 240
cct ccc atg aga caa gct gga agg ccg gac ctc aga ccg gag ggg gtt 943

Pro Pro Met Arg Gln Ala Gly Arg Pro Asp Leu Arg Pro Glu Gly Val
245                 250                 255                 260
tat gac att cct cca acc tgc acc aag cca gca ggg aag gac ctt cat 991
Tyr Asp Ile Pro Pro Thr Cys Thr Lys Pro Ala Gly Lys Asp Leu His
                265                 270                 275
gta aaa tac aac tgt gac att cca gga gct gca gaa ccg gtg gct cga 1039
Val Lys Tyr Asn Cys Asp Ile Pro Gly Ala Ala Glu Pro Val Ala Arg
            280                 285                 290
agg cac cag agc ctg tcc ccg aat cac cca ccc ccg caa ctc gga cag 1087
Arg His Gln Ser Leu Ser Pro Asn His Pro Pro Pro Gln Leu Gly Gln
        295                 300                 305
tca gtg ggc tct cag aac gac gca tat gat gtc ccc cga ggc gtt cag 1135
Ser Val Gly Ser Gln Asn Asp Ala Tyr Asp Val Pro Arg Gly Val Gln
    310                 315                 320
ttt ctt gag cca cca gca gaa acc agt gag aaa gca aac ccc cag gaa 1183
Phe Leu Glu Pro Pro Ala Glu Thr Ser Glu Lys Ala Asn Pro Gln Glu
325                 330                 335                 340
agg gat ggt gtt tat gat gtc cct ctg cat aac ccg cca gat gct aaa 1231
Arg Asp Gly Val Tyr Asp Val Pro Leu His Asn Pro Pro Asp Ala Lys
                345                 350                 355
ggc tct cgg gac ttg gtg gat ggg atc aac cga ttg tct ttc tcc agt 1279
Gly Ser Arg Asp Leu Val Asp Gly Ile Asn Arg Leu Ser Phe Ser Ser
            360                 365                 370
aca ggc agc acc cgg agt aac atg tcc acg tct tcc acc tcc tcc aag 1327
Thr Gly Ser Thr Arg Ser Asn Met Ser Thr Ser Ser Thr Ser Ser Lys
        375                 380                 385

gag tcc tca ctg tca gcc tcc cca gct cag gac aaa agg ctc ttc ctg 1375
Glu Ser Ser Leu Ser Ala Ser Pro Ala Gln Asp Lys Arg Leu Phe Leu
    390                 395                 400
gat cca gac aca gct att gag aga ctt cag cgg ctc cag cag gcc ctt 1423
Asp Pro Asp Thr Ala Ile Glu Arg Leu Gln Arg Leu Gln Gln Ala Leu
405                 410                 415                 420
gag atg ggt gtc tcc agc cta atg gca ctg gtc act acc gac tgg cgg 1471
Glu Met Gly Val Ser Ser Leu Met Ala Leu Val Thr Thr Asp Trp Arg
                425                 430                 435
tgt tac gga tat atg gaa aga cac atc aat gaa ata cgc aca gca gtg 1519
Cys Tyr Gly Tyr Met Glu Arg His Ile Asn Glu Ile Arg Thr Ala Val
            440                 445                 450
gac aag gtg gag ctg ttc ctg aag gag tac ctc cac ttt gtc aag gga 1567
Asp Lys Val Glu Leu Phe Leu Lys Glu Tyr Leu His Phe Val Lys Gly
        455                 460                 465
gct gtt gca aat gct gcc tgc ctc ccg gaa ctc atc ctc cac aac aag 1615
Ala Val Ala Asn Ala Ala Cys Leu Pro Glu Leu Ile Leu His Asn Lys
    470                 475                 480
atg aag cgg gag ctg caa cga gtc gaa gac tcc cac cag atc ctg agt 1663
Met Lys Arg Glu Leu Gln Arg Val Glu Asp Ser His Gln Ile Leu Ser
485                 490                 495                 500
caa acc agc cat gac tta aat gag tgc agc tgg tcc ctg aat atc ttg 1711
Gln Thr Ser His Asp Leu Asn Glu Cys Ser Trp Ser Leu Asn Ile Leu
                505                 510                 515
gcc atc aac aag ccc cag aac aag tgt gac gat ctg gac cgg ttt gtg 1759
Ala Ile Asn Lys Pro Gln Asn Lys Cys Asp Asp Leu Asp Arg Phe Val
            520                 525                 530

atg gtg gca aag acg gtg ccc gat gac gcc aag cag ctc acc aca acc 1807
Met Val Ala Lys Thr Val Pro Asp Asp Ala Lys Gln Leu Thr Thr Thr
        535                 540                 545
atc aac acc aac gca gag gcc ctc ttc aga ccc ggc cct ggc agc ttg 1855
Ile Asn Thr Asn Ala Glu Ala Leu Phe Arg Pro Gly Pro Gly Ser Leu
    550                 555                 560
cat ctg aag aat ggg ccg gag agc atc atg aac tca acg gag tac cca 1903
His Leu Lys Asn Gly Pro Glu Ser Ile Met Asn Ser Thr Glu Tyr Pro
565                 570                 575                 580
cac ggt ggc tcc cag gga cag ctg ctg cat cct ggt gac cac aag gcc 1951
His Gly Gly Ser Gln Gly Gln Leu Leu His Pro Gly Asp His Lys Ala
                585                 590                 595
cag gcc cac aac aag gca ctg ccc cca ggc ctg agc aag gag cag gcc 1999
Gln Ala His Asn Lys Ala Leu Pro Pro Gly Leu Ser Lys Glu Gln Ala
            600                 605                 610
cct gac tgt agc agc agt gat ggt tct gag agg agc tgg atg gat gac 2047
Pro Asp Cys Ser Ser Ser Asp Gly Ser Glu Arg Ser Trp Met Asp Asp
        615                 620                 625
tac gat tac gtc cac cta cag ggt aag gag gag ttt gag agg caa cag 2095
Tyr Asp Tyr Val His Leu Gln Gly Lys Glu Glu Phe Glu Arg Gln Gln
    630                 635                 640
aaa gag cta ttg gaa aaa gag aat atc atg aaa cag aac aag atg cag 2143
Lys Glu Leu Leu Glu Lys Glu Asn Ile Met Lys Gln Asn Lys Met Gln
645                 650                 655                 660
ctg gaa cat cat cag ctg agc cag ttc cag ctg ttg gaa caa gag att 2191
Leu Glu His His Gln Leu Ser Gln Phe Gln Leu Leu Glu Gln Glu Ile

                665                 670                 675

aca aag ccc gtg gag aat gac atc tcg aag tgg aag ccc tct cag agc 2239

Thr Lys Pro Val Glu Asn Asp Ile Ser Lys Trp Lys Pro Ser Gln Ser

            680                 685                 690

cta ccc acc aca aac agt ggc gtg agt gct cag gat cgg cag ttg ctg 2287

Leu Pro Thr Thr Asn Ser Gly Val Ser Ala Gln Asp Arg Gln Leu Leu

        695                 700                 705

tgc ttc tac tat gac caa tgt gag acc cat ttc att tcc ctt ctc aac 2335

Cys Phe Tyr Tyr Asp Gln Cys Glu Thr His Phe Ile Ser Leu Leu Asn

    710                 715                 720

gcc att gac gca ctc ttc agt tgt gtc agc tca gcc cag ccc ccg cga 2383

Ala Ile Asp Ala Leu Phe Ser Cys Val Ser Ser Ala Gln Pro Pro Arg

725                 730                 735                 740

atc ttc gtg gca cac agc aag ttt gtc atc ctc agt gca cac aaa ctg 2431

Ile Phe Val Ala His Ser Lys Phe Val Ile Leu Ser Ala His Lys Leu

                745                 750                 755

gtg ttc att gga gac acg ctg aca cgg cag gtg act gcc cag gac att 2479

Val Phe Ile Gly Asp Thr Leu Thr Arg Gln Val Thr Ala Gln Asp Ile

            760                 765                 770

cgc aac aaa gtc atg aac tcc agc aac cag ctc tgc gag cag ctc aag 2527

Arg Asn Lys Val Met Asn Ser Ser Asn Gln Leu Cys Glu Gln Leu Lys

        775                 780                 785

act ata gtc atg gca acc aag atg gcc gcc ctc cat tac ccc agc acc 2575

Thr Ile Val Met Ala Thr Lys Met Ala Ala Leu His Tyr Pro Ser Thr

    790                 795                 800

acg gcc ctg cag gaa atg gtg cac caa gtg aca gac ctt tct aga aat 2623

Thr Ala Leu Gln Glu Met Val His Gln Val Thr Asp Leu Ser Arg Asn

805                    810                    815                    820

gcc cag ctg ttc aag cgc tct ttg ctg gag atg gca acg ttc          2665

Ala Gln Leu Phe Lys Arg Ser Leu Leu Glu Met Ala Thr Phe

                825                    830

tgagaagaaa aaaaagagga aggggactgc gttaacggtt actaaggaaa actggaaata 2725

ctgtctggtt tttgtaaatg ttatctattt ttgtagataa ttttatataa aaatgaaata 2785

ttttaacatt ttatgggtca gacaactttc agaaattcag ggagctggag agggaaatct 2845

ttttttcccc cctgagtgtt cttatgtata cacagaagta tctgagacat aaactgtaca 2905

gaaaacttgt ccacgtcctt ttgtatgccc atgtattcat gtttttgttt gtagatgttt 2965

gtctgatgca tttcattaaa aaaaaaacca tgaattacga agcaccttag taagcacctt 3025

ctaatgctgc attttttttg ttgttgttaa aaacatccag ctggttataa tattgttctc 3085

cacgtccttg tgatgattct gagcctggca ctgggaatct gggaagcata gtttatttgc 3145

aagtgttcac cttccaaatc atgaggcata gcatgactta ttcttgtttt gaaaactctt 3205

ttcaaaactg accatcttaa acacatgatg gccaagtgcc acaaagccct cttgcggaga 3265

catttacgaa tatatatgtg gatccaagtc tcgatagtta ggcgttggag ggaagagaga 3325

ccagagagtt tagaggccag gaccacagtt aggattgggt tgtttcaata ctgagagaca 3385

gctacaataa aaggagagca attgcctccc tggggctgtt caatcttctg catttgtgag 3445

tggttcagtc atgaggtttt ccaaaagatg tttttagagt tgtaaaaacc atatttgcag 3505

caaagattta caaaggcgta tcagactatg attgttcacc aaaatagggg aatggtttga 3565

tccgccagtt gcaagtagag gcctttctga ctcttaatat tcactttggt gctactaccc 3625

ccattacctg aggaactggc caggtccttg atcatggaac tatagagcta ccagacatat 3685

cctgctctct aagggaattt attgctatct tgcaccttct ttaaaactca aaaaacatat 3745

gcagacctga cactcaagag tggctagcta cacagagtcc atctaatttt tgcaacttcc 3805

ccccccgaat tc                                                     3817

<210> 18

<211> 834

<212> PRT

<213> Homo sapiens

<400> 18

Met Lys Tyr Lys Asn Leu Met Ala Arg Ala Leu Tyr Asp Asn Val Pro

1               5                   10                  15

Glu Cys Ala Glu Glu Leu Ala Phe Arg Lys Gly Asp Ile Leu Thr Val

            20                  25                  30

Ile Glu Gln Asn Thr Gly Gly Leu Glu Gly Trp Trp Leu Cys Ser Leu

        35                  40                  45

His Gly Arg Gln Gly Ile Val Pro Gly Asn Arg Val Lys Leu Leu Ile

    50                  55                  60

Gly Pro Met Gln Glu Thr Ala Ser Ser His Glu Gln Pro Ala Ser Gly

65                  70                  75                  80

Leu Met Gln Gln Thr Phe Gly Gln Gln Lys Leu Tyr Gln Val Pro Asn

                85                  90                  95

Pro Gln Ala Ala Pro Arg Asp Thr Ile Tyr Gln Val Pro Pro Ser Tyr

            100                 105                 110

Gln Asn Gln Gly Ile Tyr Gln Val Pro Thr Gly His Gly Thr Gln Glu

        115                 120                 125

Gln Glu Val Tyr Gln Val Pro Pro Ser Val Gln Arg Ser Ile Gly Gly

    130                 135                 140

Thr Ser Gly Pro His Val Gly Lys Lys Val Ile Thr Pro Val Arg Thr

145                 150                 155                 160

Gly His Gly Tyr Val Tyr Glu Tyr Pro Ser Arg Tyr Gln Lys Asp Val

                165                 170                 175

Tyr Asp Ile Pro Pro Ser His Thr Thr Gln Gly Val Tyr Asp Ile Pro

            180                 185                 190
Pro Ser Ser Ala Lys Gly Pro Val Phe Ser Val Pro Val Gly Glu Ile
        195                 200                 205
Lys Pro Gln Gly Val Tyr Asp Ile Pro Pro Thr Lys Gly Val Tyr Ala
    210                 215                 220
Ile Pro Pro Ser Ala Cys Arg Asp Glu Ala Gly Leu Arg Glu Lys Asp
225                 230                 235                 240
Tyr Asp Phe Pro Pro Pro Met Arg Gln Ala Gly Arg Pro Asp Leu Arg
                245                 250                 255
Pro Glu Gly Val Tyr Asp Ile Pro Pro Thr Cys Thr Lys Pro Ala Gly
            260                 265                 270
Lys Asp Leu His Val Lys Tyr Asn Cys Asp Ile Pro Gly Ala Ala Glu
        275                 280                 285
Pro Val Ala Arg Arg His Gln Ser Leu Ser Pro Asn His Pro Pro Pro
    290                 295                 300
Gln Leu Gly Gln Ser Val Gly Ser Gln Asn Asp Ala Tyr Asp Val Pro
305                 310                 315                 320
Arg Gly Val Gln Phe Leu Glu Pro Pro Ala Glu Thr Ser Glu Lys Ala
                325                 330                 335
Asn Pro Gln Glu Arg Asp Gly Val Tyr Asp Val Pro Leu His Asn Pro
            340                 345                 350
Pro Asp Ala Lys Gly Ser Arg Asp Leu Val Asp Gly Ile Asn Arg Leu
        355                 360                 365
Ser Phe Ser Ser Thr Gly Ser Thr Arg Ser Asn Met Ser Thr Ser Ser
    370                 375                 380
Thr Ser Ser Lys Glu Ser Ser Leu Ser Ala Ser Pro Ala Gln Asp Lys
385                 390                 395                 400

Arg Leu Phe Leu Asp Pro Asp Thr Ala Ile Glu Arg Leu Gln Arg Leu
                405                 410                 415
Gln Gln Ala Leu Glu Met Gly Val Ser Ser Leu Met Ala Leu Val Thr
            420                 425                 430
Thr Asp Trp Arg Cys Tyr Gly Tyr Met Glu Arg His Ile Asn Glu Ile
        435                 440                 445
Arg Thr Ala Val Asp Lys Val Glu Leu Phe Leu Lys Glu Tyr Leu His
    450                 455                 460
Phe Val Lys Gly Ala Val Ala Asn Ala Ala Cys Leu Pro Glu Leu Ile
465                 470                 475                 480
Leu His Asn Lys Met Lys Arg Glu Leu Gln Arg Val Glu Asp Ser His
                485                 490                 495
Gln Ile Leu Ser Gln Thr Ser His Asp Leu Asn Glu Cys Ser Trp Ser
            500                 505                 510
Leu Asn Ile Leu Ala Ile Asn Lys Pro Gln Asn Lys Cys Asp Asp Leu
        515                 520                 525
Asp Arg Phe Val Met Val Ala Lys Thr Val Pro Asp Asp Ala Lys Gln
    530                 535                 540
Leu Thr Thr Thr Ile Asn Thr Asn Ala Glu Ala Leu Phe Arg Pro Gly
545                 550                 555                 560
Pro Gly Ser Leu His Leu Lys Asn Gly Pro Glu Ser Ile Met Asn Ser
                565                 570                 575
Thr Glu Tyr Pro His Gly Gly Ser Gln Gly Gln Leu Leu His Pro Gly
            580                 585                 590
Asp His Lys Ala Gln Ala His Asn Lys Ala Leu Pro Pro Gly Leu Ser
        595                 600                 605

Lys Glu Gln Ala Pro Asp Cys Ser Ser Ser Asp Gly Ser Glu Arg Ser
    610                 615                 620
Trp Met Asp Asp Tyr Asp Tyr Val His Leu Gln Gly Lys Glu Glu Phe
625                 630                 635                 640
Glu Arg Gln Gln Lys Glu Leu Leu Glu Lys Glu Asn Ile Met Lys Gln
                645                 650                 655
Asn Lys Met Gln Leu Glu His His Gln Leu Ser Gln Phe Gln Leu Leu
            660                 665                 670
Glu Gln Glu Ile Thr Lys Pro Val Glu Asn Asp Ile Ser Lys Trp Lys
        675                 680                 685
Pro Ser Gln Ser Leu Pro Thr Thr Asn Ser Gly Val Ser Ala Gln Asp
    690                 695                 700
Arg Gln Leu Leu Cys Phe Tyr Tyr Asp Gln Cys Glu Thr His Phe Ile
705                 710                 715                 720
Ser Leu Leu Asn Ala Ile Asp Ala Leu Phe Ser Cys Val Ser Ser Ala
                725                 730                 735
Gln Pro Pro Arg Ile Phe Val Ala His Ser Lys Phe Val Ile Leu Ser
            740                 745                 750
Ala His Lys Leu Val Phe Ile Gly Asp Thr Leu Thr Arg Gln Val Thr
        755                 760                 765
Ala Gln Asp Ile Arg Asn Lys Val Met Asn Ser Ser Asn Gln Leu Cys
    770                 775                 780
Glu Gln Leu Lys Thr Ile Val Met Ala Thr Lys Met Ala Ala Leu His
785                 790                 795                 800
Tyr Pro Ser Thr Thr Ala Leu Gln Glu Met Val His Gln Val Thr Asp
                805                 810                 815
Leu Ser Arg Asn Ala Gln Leu Phe Lys Arg Ser Leu Leu Glu Met Ala

820                    825                    830

Thr Phe

<210> 19

<211> 567

<212> DNA

<213> Homo sapiens

<220>

<221> CDS

<222> (36)..(506)

<400> 19

tgtgacgcct gcagggctgg gacctgacgg tgaag atg ctg gcg ggc aac gaa     53

                                       Met Leu Ala Gly Asn Glu

                                       1               5

ttc cag gtg tcc ctg agc agc tcc atg tcg gtg tca gag ctg aag gcg     101

Phe Gln Val Ser Leu Ser Ser Ser Met Ser Val Ser Glu Leu Lys Ala

            10                  15                  20

cag atc acc cag aac att ggc gtg cac gcc ttc cag cag cgt ctg gct     149

Gln Ile Thr Gln Asn Ile Gly Val His Ala Phe Gln Gln Arg Leu Ala

        25                  30                  35

gtc cac ccg agc ggt gtg gcg ctg cag gac agg gtc ccc ctt gcc agc     197

Val His Pro Ser Gly Val Ala Leu Gln Asp Arg Val Pro Leu Ala Ser

    40                  45                  50

cag ggc ctg ggc cct ggc agc acg gtc ctg ctg gtg gtg gac aaa tgc     245

Gln Gly Leu Gly Pro Gly Ser Thr Val Leu Leu Val Val Asp Lys Cys

55                  60                  65                  70

gac gaa cct ctg agc atc ctg gtg agg aat aac aag ggc cgc agc agc     293

Asp Glu Pro Leu Ser Ile Leu Val Arg Asn Asn Lys Gly Arg Ser Ser
                75                  80                  85
acc tac gag gtg cgg ctg acg cag acc gtg gcc cac ctg aag cag caa 341
Thr Tyr Glu Val Arg Leu Thr Gln Thr Val Ala His Leu Lys Gln Gln
            90                  95                  100
gtg agc ggg ctg gag ggt gtg cag gac gac ctg ttc tgg ctg acc ttc 389
Val Ser Gly Leu Glu Gly Val Gln Asp Asp Leu Phe Trp Leu Thr Phe
        105                 110                 115
gag ggg aag ccc ctg gag gac cag ctc ccg ctg ggg gag tac ggc ctc 437
Glu Gly Lys Pro Leu Glu Asp Gln Leu Pro Leu Gly Glu Tyr Gly Leu
    120                 125                 130
aag ccc ctg agc acc gtg ttc atg aat ctg cgc ctg cgg gga ggc ggc 485
Lys Pro Leu Ser Thr Val Phe Met Asn Leu Arg Leu Arg Gly Gly Gly
135                 140                 145                 150
aca gag cct ggc ggg cgg agc taagggcctc caccagcatc cgagcaggat 536
Thr Glu Pro Gly Gly Arg Ser
                155
caagggccgg aataaaggct gttgtaagag a 567
<210> 20
<211> 157
<212> PRT
<213> Homo sapiens
<400> 20
Met Leu Ala Gly Asn Glu Phe Gln Val Ser Leu Ser Ser Ser Met Ser
1               5                   10                  15
Val Ser Glu Leu Lys Ala Gln Ile Thr Gln Asn Ile Gly Val His Ala

            20                  25                  30
Phe Gln Gln Arg Leu Ala Val His Pro Ser Gly Val Ala Leu Gln Asp
        35                  40                  45
Arg Val Pro Leu Ala Ser Gln Gly Leu Gly Pro Gly Ser Thr Val Leu
    50                  55                  60
Leu Val Val Asp Lys Cys Asp Glu Pro Leu Ser Ile Leu Val Arg Asn
65                  70                  75                  80
Asn Lys Gly Arg Ser Ser Thr Tyr Glu Val Arg Leu Thr Gln Thr Val
                85                  90                  95
Ala His Leu Lys Gln Gln Val Ser Gly Leu Glu Gly Val Gln Asp Asp
            100                 105                 110
Leu Phe Trp Leu Thr Phe Glu Gly Lys Pro Leu Glu Asp Gln Leu Pro
        115                 120                 125
Leu Gly Glu Tyr Gly Leu Lys Pro Leu Ser Thr Val Phe Met Asn Leu
    130                 135                 140
Arg Leu Arg Gly Gly Gly Thr Glu Pro Gly Gly Arg Ser
145                 150                 155
<210> 21
<211> 5095
<212> DNA
<213> Homo sapiens
<220>
<221> CDS
<222> (14).. (2593)
<400> 21
agaggctgcg agc atg ggg ccc tgg ggc tgg aaa ttg cgc tgg acc gtc 49
gcc ttg ctc ctc gcc gcg gcg ggg act gca gtg ggc gac aga tgt gaa 97
aga aac gag ttc cag tgc caa gac ggg aaa tgc atc tcc tac aag tgg 145
gtc tgc gat ggc agc gct gag tgc cag gat ggc tct gat gag tcc cag 193
gag acg tgc ttg tct gtc acc tgc aaa tcc ggg gac ttc agc tgt ggg 241
ggc cgt gtc aac cgc tgc att cct cag ttc tgg agg tgc gat ggc caa 289
gtg gac tgc gac aac ggc tca gac gag caa ggc tgt ccc ccc aag acg 337
tgc tcc cag gac gag ttt cgc tgc cac gat ggg aag tgc atc tct cgg 385
cag ttc gtc tgt gac tca gac cgg gac tgc ttg gac ggc tca gac gag 433
gcc tcc tgc ccg gtg ctc acc tgt ggt ccc gcc agc ttc cag tgc aac 481
agc tcc acc tgc atc ccc cag ctg tgg gcc tgc gac aac gac ccc gac 529
tgc gaa gat ggc tcg gat gag tgg ccg cag cgc tgt agg ggt ctt tac 577
gtg ttc caa ggg gac agt agc ccc tgc tcg gcc ttc gag ttc cac tgc 625
cta agt ggc gag tgc atc cac tcc agc tgg cgc tgt gat ggt ggc ccc 673
gac tgc aag gac aaa tct gac gag gaa aac tgc gct gtg gcc acc tgt 721
cgc cct gac gaa ttc cag tgc tct gat gga aac tgc atc cat ggc agc 769
cgg cag tgt gac cgg gaa tat gac tgc aag gac atg agc gat gaa gtt 817
ggc tgc gtt aat gtg aca ctc tgc gag gga ccc aac aag ttc aag tgt 865
cac agc ggc gaa tgc atc acc ctg gac aaa gtc tgc aac atg gct aga 913
gac tgc cgg gac tgg tca gat gaa ccc atc aaa gag tgc ggg acc aac 961
gaa tgc ttg gac aac aac ggc ggc tgt tcc cac gtc tgc aat gac ctt 1009
aag atc ggc tac gag tgc ctg tgc ccc gac ggc ttc cag ctg gtg gcc 1057
cag cga aga tgc gaa gat atc gat gag tgt cag gat ccc gac acc tgc 1105
agc cag ctc tgc gtg aac ctg gag ggt ggc tac aag tgc cag tgt gag 1153
gaa ggc ttc cag ctg gac ccc cac acg aag gcc tgc aag gct gtg ggc 1201
tcc atc gcc tac ctc ttc ttc acc aac cgg cac gag gtc agg aag atg 1249
acg ctg gac cgg agc gag tac acc agc ctc atc ccc aac ctg agg aac 1297
gtg gtc gct ctg gac acg gag gtg gcc agc aat aga atc tac tgg tct 1345
gac ctg tcc cag aga atg atc tgc agc acc cag ctt gac aga gcc cac 1393
ggc gtc tct tcc tat gac acc gtc atc agc agg gac atc cag gcc ccc 1441
gac ggg ctg gct gtg gac tgg atc cac agc aac atc tac tgg acc gac 1489
tct gtc ctg ggc act gtc tct gtt gcg gat acc aag ggc gtg aag agg 1537
aaa acg tta ttc agg gag aac ggc tcc aag cca agg gcc atc gtg gtg 1585
gat cct gtt cat ggc ttc atg tac tgg act gac tgg gga act ccc gcc 1633
aag atc aag aaa ggg ggc ctg aat ggt gtg gac atc tac tcg ctg gtg 1681
act gaa aac att cag tgg ccc aat ggc atc acc cta gat ctc ctc agt 1729
ggc cgc ctc tac tgg gtt gac tcc aaa ctt cac tcc atc tca agc atc 1777
gat gtc aat ggg ggc aac cgg aag acc atc ttg gag gat gaa aag agg 1825
ctg gcc cac ccc ttc tcc ttg gcc gtc ttt gag gac aaa gta ttt tgg 1873
aca gat atc atc aac gaa gcc att ttc agt gcc aac cgc ctc aca ggt 1921
tcc gat gtc aac ttg ttg gct gaa aac cta ctg tcc cca gag gat atg 1969
gtc ctc ttc cac aac ctc acc cag cca aga gga gtg aac tgg tgt gag 2017
agg acc acc ctg agc aat ggc ggc tgc cag tat ctg tgc ctc cct gcc 2065
ccg cag atc aac ccc cac tcg ccc aag ttt acc tgc gcc tgc ccg gac 2113
ggc atg ctg ctg gcc agg gac atg agg agc tgc ctc aca gag gct gag 2161
gct gca gtg gcc acc cag gag aca tcc acc gtc agg cta aag gtc agc 2209
tcc aca gcc gta agg aca cag cac aca acc acc cgg cct gtt ccc gac 2257
acc tcc cgg ctg cct ggg gcc acc cct ggg ctc acc acg gtg gag ata 2305
gtg aca atg tct cac caa gct ctg ggc gac gtt gct ggc aga gga aat 2353
gag aag aag ccc agt agc gtg agg gct ctg tcc att gtc ctc ccc atc 2401
gtg ctc ctc gtc ttc ctt tgc ctg ggg gtc ttc ctt cta tgg aag aac 2449
tgg cgg ctt aag aac atc aac agc atc aac ttt gac aac ccc gtc tat 2497
cag aag acc aca gag gat gag gtc cac att tgc cac aac cag gac ggc 2545
tac agc tac ccc tcg aga cag atg gtc agt ctg gag gat gac gtg gcg 2593
tgaacatctg cctggagtcc cgcccctgcc cagaaccctt cctgagacct cgccggcctt 2653
gttttattca aagacagaga agaccaaagc attgcctgcc agagctttgt tttatatatt 2713
tattcatctg ggaggcagaa caggcttcgg acagtgccca tgcaatggct tgggttggga 2773
ttttggtttc ttcctttcct gtgaaggata agagaaacag gccccgggcg accaggatga 2833
cacctccatt tctctccagg aagttttgag tttctctcca ccgtgacaca atcctcaaac 2893
atggaagatg aaagggcagg ggatgtcagg cccagagaag caagtggctt tcaacacaca 2953
acagcagatg gcaccaacgg gaccccctgg ccctgcctca tccaccaatc tctaagccaa 3013
acccctaaac tcaggagtca acgtgtttac ctcttctatg caagccttgc tagacagcca 3073
ggttagcctt tgccctgtca cccccgaatc atgacccacc cagtgtcttt cgaggtgggt 3133
ttgtaccttc cttaagccag gaaagggatt catggcgtcg gaaatgatct ggctgaatcc 3193
gtggtggcac cgagaccaaa ctcattcacc aaatgatgcc acttcccaga ggcagagcct 3253
gagtcaccgg tcacccttaa tatttattaa gtgcctgaga cacccggtta ccttggccgt 3313
gaggacacgt ggcctgcacc caggtgtggc tgtcaggaca ccagcctggt gcccatcctc 3373
ccgaccccta cccacttcca ttcccgtggt ctccttgcac tttctcagtt cagagttgta 3433
cactgtgtac atttggcatt tgtgttatta ttttgcactg ttttctgtcg tgtgtgttgg 3493
gatgggatcc caggccaggg aaagcccgtg tcaatgaatg ccggggacag agaggggcag 3553
gttgaccggg acttcaaagc cgtgatcgtg aatatcgaga actgccattg tcgtctttat 3613
gtccgcccac ctagtgcttc cacttctatg caaatgcctc caagccattc acttccccaa 3673
tcttgtcgtt gatgggtatg tgtttaaaac atgcacggtg aggccgggcg cagtggcctc 3733
acgcctgtaa tcccagcact ttgggaggcc gaggcgggtg gatcatgagg tcaggagatc 3793
gagaccatcc tggctaacaa ggtgaaaccc cgtctctact aaaaatacaa aaaattagcc 3853
gggcgcggtg gtgggcacct gtagtcccag ctactcggga ggctgaggca ggagaatggt 3913
gtgaacccgg gaagcggagc ttgcagtgag ccgagattgc gccactgcag tccgcagtct 3973
ggcctgggcg acagagcgag actccgtctc aaaaaaaaca aaacaaaaaa aaaccatgca 4033
tggtgcatca gcagcccatg gcctctggcc aggcatggcg aggctgaggt gggaggatgg 4093
tttgagctca ggcatttgag gctgtcgtga gctatgatta tgccactgct ttccagcctg 4153
ggcaacatag taagacccca tctcttaaaa aatgaatttg gccagacaca ggtgcctcac 4213
gcctgtaatc ccagcacttt gggaggctga gctggatcac ttgagttcag gagttggaga 4273
ccaggcctga gcaacaaagc gagatcccat ctctacaaaa accaaaaagt taaaaatcag 4333
ctgggtatgg tggcacgtgc ctgtgatccc agctacttgg gaggctgagg caggaggatc 4393
gcctgagccc aggaggtgga ggttgcagtg agccatgatc gagccactgc actccagcct 4453
gggcaacaca tgaagaccct atttcagaaa tacaactata aaaaaaataa ataaatcctc 4513
cagtctggat cgtttgacgg gacttcaggt tctttctgaa atcgccgtgt tactgttgca 4573
ctgatgtccg gagagacagt gacagcctcc gtcagactcc cgcgtgaaga tgtcacaagg 4633
gattggcaat tgtccccagg gacaaaacac tgtgtccccc ccagtgcagg gaaccgtgat 4693
aagcctttct ggtttcggag cacgtaaatg cgtccctgta cagatagtgg ggattttttg 4753
ttatgtttgc actttgtata ttggttgaaa ctgttatcac ttatatatat atatacacac 4813
atatatataa aatctattta tttttgcaaa ccctggttgc tgtatttgtt cagtgactat 4873
tctcggggcc ctgtgtaggg ggttattgcc tctgaaatgc ctcttcttta tgtacaaaga 4933
ttatttgcac gaactggact gtgtgcaacg ctttttggga gaatgatgtc cccgttgtat 4993
gtatgagtgg cttctgggag atgggtgtca ctttttaaac cactgtatag aaggtttttg 5053
tagcctgaat gtcttactgt gatcaattaa atttcttaaa tg 5095

<210> 22

<211> 860

<212> PRT

<213> Homo sapiens

<400> 22

<210> 23

<211> 1660

<212> DNA

<213> Homo sapiens

<220>

<221> CDS

<222> (50)..(529)

<400> 23

gcgctctcct cgcaggcaga aactccgctg agcagaactt gccgccaga atg ctc ctc 58

Met Leu Leu

1

ctg ttg ctg agt atc atc gtc ctc cac gtc gcg gtg ctg gtg ctg ctg 106

Leu Leu Leu Ser Ile Ile Val Leu His Val Ala Val Leu Val Leu Leu

    5                   10                  15

ttc gtc tcc acg atc gtc agc caa tgg atc gtg ggc aat gga cac gca 154

Phe Val Ser Thr Ile Val Ser Gln Trp Ile Val Gly Asn Gly His Ala

20                  25                  30                  35

act gat ctc tgg cag aac tgt agc acc tct tcc tca gga aat gtc cac 202

Thr Asp Leu Trp Gln Asn Cys Ser Thr Ser Ser Ser Gly Asn Val His

                40                  45                  50

cac tgt ttc tca tca tca cca aac gaa tgg ctg cag tct gtc cag gcc 250

His Cys Phe Ser Ser Ser Pro Asn Glu Trp Leu Gln Ser Val Gln Ala

            55                  60                  65

acc atg atc ctg tcg atc atc ttc agc att ctg tct ctg ttc ctg ttc 298

Thr Met Ile Leu Ser Ile Ile Phe Ser Ile Leu Ser Leu Phe Leu Phe

        70                  75                  80

ttc tgc caa ctc ttc acc ctc acc aag ggg ggc agg ttt tac atc act 346

Phe Cys Gln Leu Phe Thr Leu Thr Lys Gly Gly Arg Phe Tyr Ile Thr
    85                  90                  95
gga atc ttc caa att ctt gct ggt ctg tgc gtg atg agt gct gcg gcc 394
Gly Ile Phe Gln Ile Leu Ala Gly Leu Cys Val Met Ser Ala Ala Ala
100                 105                 110                 115
atc tac acg gtg agg cac ccg gag tgg cat ctc acc tcg gat tac tcc 442
Ile Tyr Thr Val Arg His Pro Glu Trp His Leu Thr Ser Asp Tyr Ser
                120                 125                 130
tac ggt ttc gcc tac atc ctg gcc tgg gtg gcc ttc ccc ctg gcc ctt 490
Tyr Gly Phe Ala Tyr Ile Leu Ala Trp Val Ala Phe Pro Leu Ala Leu
            135                 140                 145
ctc agc ggt gtc atc tat gtg atc ttg cgg aaa cgc gaa tgaggcgccc 539
Leu Ser Gly Val Ile Tyr Val Ile Leu Arg Lys Arg Glu
        150                 155                 160
agacggtctg tctgaggctc tgagcgtaca tagggaaggg aggaagggaa aacagaaagc 599
agacaaagaa aaaagagcta gcccaaaatc ccaaactcaa accaaaccaa acagaaagca 659
gtggaggtgg gggttgctgt tgattgaaga tgtatataat atctccggtt tataaaacct 719
atttataaca ctttttacat atatgtacat agtattgttt gctttttatg ttgaccatca 779
gcctcgtgtt gagccttaaa gaagtagcta aggaacttta catcctaaca gtataatcca 839
gctcagtatt tttgttttgt tttttgtttg tttgttttgt tttacccaga aataagataa 899
ctccatctcg ccccttccct ttcatctgaa agaagatacc tccctcccag tccacctcat 959
ttagaaaacc aaagtgtggg tagaaacccc aaatgtccaa aagccctttt ctggtgggtg 1019
acccagtgca tccaacagaa acagccgctg cccgaacctc tgtgtgaagc tttacgcgca 1079
cacggacaaa atgcccaaac cggagccctc gaaaaacgcg gcttgtggca ttggcatact 1139
tgccttacag gtggagtatc ttcgtcacac atctaaatga gaaatcagtg acaacaagtc 1199
tttgaaatgg tgctatggat ttaccattcc ttattatcac taatcatcta aacaactcac 1259
tggaaatcca attaacaatt ttataacata agatagaatg gagacctgaa taattctgtg 1319

taatataaat ggtttataac tgcttttgta cctagctagg ctgctattat tactataatg 1379

agtaaatcat aaagccttcg tcactcccac agtttcttta cggtcggagc atcacaacaa 1439

gcgtctagac tccttgggac cgtgagttcc tagagcttgg ctgggtctag gctgttctgt 1499

gcctccaagg actgtctggc aatgacttgt attggccacc aactgtagat gtatatatgg 1559

tgcccttctg atgctaagac tccagacctt ttgtttttgc tttgcatttt ctgatttata 1619

ccaactgtgt ggactaagat gcattaaaat aaacatcaga g 1660

<210> 24

<211> 160

<212> PRT

<213> Homo sapiens

<400> 24

Met Leu Leu Leu Leu Leu Ser Ile Ile Val Leu His Val Ala Val Leu

1               5                   10                  15

Val Leu Leu Phe Val Ser Thr Ile Val Ser Gln Trp Ile Val Gly Asn

            20                  25                  30

Gly His Ala Thr Asp Leu Trp Gln Asn Cys Ser Thr Ser Ser Ser Gly

        35                  40                  45

Asn Val His His Cys Phe Ser Ser Ser Pro Asn Glu Trp Leu Gln Ser

    50                  55                  60

Val Gln Ala Thr Met Ile Leu Ser Ile Ile Phe Ser Ile Leu Ser Leu

65                  70                  75                  80

Phe Leu Phe Phe Cys Gln Leu Phe Thr Leu Thr Lys Gly Gly Arg Phe

                85                  90                  95

Tyr Ile Thr Gly Ile Phe Gln Ile Leu Ala Gly Leu Cys Val Met Ser

            100                 105                 110

Ala Ala Ala Ile Tyr Thr Val Arg His Pro Glu Trp His Leu Thr Ser

        115                 120                 125

Asp Tyr Ser Tyr Gly Phe Ala Tyr Ile Leu Ala Trp Val Ala Phe Pro

    130                 135                 140

Leu Ala Leu Leu Ser Gly Val Ile Tyr Val Ile Leu Arg Lys Arg Glu

145                 150                 155                 160

<210> 25

<211> 3116

<212> DNA

<213> Homo sapiens

<220>

<221> CDS

<222> (36)..(2717)

<400> 25
ggagcccagc aacttctgag gaaagtttgg caccc atg gcg tgg cgg tgc ccc 53
agg atg ggc agg gtc ccg ctg gcc tgg tgc ttg gcg ctg tgc ggc tgg 101
gcg tgc atg gcc ccc agg ggc acg cag gct gaa gaa agt ccc ttc gtg 149
ggc aac cca ggg aat atc aca ggt gcc cgg gga ctc acg ggc acc ctt 197
cgg tgt cag ctc cag gtt cag gga gag ccc ccc gag gta cat tgg ctt 245
cgg gat gga cag atc ctg gag ctc gcg gac agc acc cag acc cag gtg 293
ccc ctg ggt gag gat gaa cag gat gac tgg ata gtg gtc agc cag ctc 341
aga atc acc tcc ctg cag ctt tcc gac acg gga cag tac cag tgt ttg 389
gtg ttt ctg gga cat cag acc ttc gtg tcc cag cct ggc tat gtt ggg 437
ctg gag ggc ttg cct tac ttc ctg gag gag ccc gaa gac agg act gtg 485
gcc gcc aac acc ccc ttc aac ctg agc tgc caa gct cag gga ccc cca 533
gag ccc gtg gac cta ctc tgg ctc cag gat gct gtc ccc ctg gcc acg 581
gct cca ggt cac ggc ccc cag cgc agc ctg cat gtt cca ggg ctg aac 629
aag aca tcc tct ttc tcc tgc gaa gcc cat aac gcc aag ggg gtc acc 677
aca tcc cgc aca gcc acc atc aca gtg ctc ccc cag cag ccc cgt aac 725
ctc cac ctg gtc tcc cgc caa ccc acg gag ctg gag gtg gct tgg act 773
cca ggc ctg agc ggc atc tac ccc ctg acc cac tgc acc ctg cag gct 821
gtg ctg tca gac gat ggg atg ggc atc cag gcg gga gaa cca gac ccc 869
cca gag gag ccc ctc acc tcg caa gca tcc gtg ccc ccc cat cag ctt 917
cgg cta ggc agc ctc cat cct cac ccc cct tat cac atc cgc gtg gca 965
tgc acc agc agc cag ggc ccc tca tcc tgg acc cac tgg ctt cct gtg 1013
gag acg ccg gag gga gtg ccc ctg ggc ccc cct gag aac att agt gct 1061
acg cgg aat ggg agc cag gcc ttc gtg cat tgg caa gag ccc cgg gcg 1109
ccc ctg cag ggt acc ctg tta ggg tac cgg ctg gcg tat caa ggc cag 1157
gac acc cca gag gtg cta atg gac ata ggg cta agg caa gag gtg acc 1205
ctg gag ctg cag ggg gac ggg tct gtg tcc aat ctg aca gtg tgt gtg 1253
gca gcc tac act gct gct ggg gat gga ccc tgg agc ctc cca gta ccc 1301
ctg gag gcc tgg cgc cca ggg gaa gca cag cca gtc cac cag ctg gtg 1349
aag gaa cct tca act cct gcc ttc tcg tgg ccc tgg tgg tat gta ctg 1397
cta gga gca gtc gtg gcc gct gcc tgt gtc ctc atc ttg gct ctc ttc 1445
ctt gtc cac cgg cga aag aag gag acc cgt tat gga gaa gtg ttt gaa 1493
cca aca gtg gaa aga ggt gaa ctg gta gtc agg tac cgc gtg cgc aag 1541
tcc tac agt cgt cgg acc act gaa gct acc ttg aac agc ctg ggc atc 1589
agt gaa gag ctg aag gag aag ctg cgg gat gtg atg gtg gac cgg cac 1637
aag gtg gcc ctg ggg aag act ctg gga gag gga gag ttt gga gct gtg 1685
atg gaa ggc cag ctc aac cag gac gac tcc atc ctc aag gtg gct gtg 1733
aag acg atg aag att gcc atc tgc acg agg tca gag ctg gag gat ttc 1781
ctg agt gaa gcg gtc tgc atg aag gaa ttt gac cat ccc aac gtc atg 1829
agg ctc atc ggt gtc tgt ttc cag ggt tct gaa cga gag agc ttc cca 1877
gca cct gtg gtc atc tta cct ttc atg aaa cat gga gac cta cac agc 1925
ttc ctc ctc tat tcc cgg ctc ggg ggc cag cca gtg tac ctg ccc act    1973
cag atg cta gtg aag ttc atg gca gac atc gcc agt ggc atg gag tat    2021
ctg agt acc aag aga ttc ata cac cgg gac ctg gcg gcc agg aac tgc    2069
atg ctg aat gag aac atg tcc gtg tgt gtg gcg gac ttc ggg ctc tcc    2117
aag aag atc tac aat ggg gac tac tac cgc cag gga cgt atc gcc aag    2165
atg cca gtc aag tgg att gcc att gag agt cta gct gac cgt gtc tac    2213
acc agc aag agc gat gtg tgg tcc ttc ggg gtg aca atg tgg gag att    2261
gcc aca aga ggc caa acc cca tat ccg ggc gtg gag aac agc gag att    2309
tat gac tat ctg cgc cag gga aat cgc ctg aag cag cct gcg gac tgt    2357
ctg gat gga ctg tat gcc ttg atg tcg cgg tgc tgg gag cta aat ccc 2405
cag gac cgg cca agt ttt aca gag ctg cgg gaa gat ttg gag aac aca 2453
ctg aag gcc ttg cct cct gcc cag gag cct gac gaa atc ctc tat gtc 2501
aac atg gat gag ggt gga ggt tat cct gaa ccc cct gga gct gca gga 2549
gga gct gac ccc cca acc cag cca gac cct aag gat tcc tgt agc tgc 2597
ctc act gcg gct gag gtc cat cct gct gga cgc tat gtc ctc tgc cct 2645
tcc aca acc cct agc ccc gct cag cct gct gat agg ggc tcc cca gca 2693
gcc cca ggg cag gag gat ggt gcc tgagacaacc ctccacctgg tactccctct 2747
caggatccaa gctaagcact gccactgggg gaaactccac cttcccactt tcccacccca 2807
cgccttatcc ccacttgcag ccctgtcttc ctacctatcc cacctccatc ccagacaggt 2867
ccctggcctt ctctgtgcag tagcatcacc ttgaaagcag tagcatcacc atctgtaaaa 2927
ggaaggggtt ggattgcaat atctgaagcc ctcccaggtg ttaacattcc aagactctag 2987
agtccaaggt ttaaagagtc tagattcaaa ggttctaggt ttcaaagatg ctgtgagtct 3047
ttggttctaa ggacctgaaa ttccaaagtc tctaattcta ttaaagtgct aaggttctaa 3107
ggcctaaaa 3116

<210> 26
<211> 894
<212> PRT
<213> Homo sapiens
<400> 26
<210> 27
<211> 3781
<212> DNA
<213> Homo sapiens
<220>
<221> CDS
<222> (4)..(2994)
<400> 27
gcc atg gag aac gcg cac acc aag acg gtg gag gag gtg ctg ggc cac 48
ttc ggc gtc aac gag agt acg ggg ctg agc ctg gaa cag gtc aag aag 96
ctt aag gag aga tgg ggc tcc aac gag tta ccg gct gaa gaa gga aaa 144
acc ttg ctg gaa ctt gtg att gag cag ttt gaa gac ttg cta gtt agg 192
att tta tta ctg gca gca tgt ata tct ttt gtt ttg gct tgg ttt gaa 240
gaa ggt gaa gaa aca att aca gcc ttt gta gaa cct ttt gta att tta 288
ctc ata tta gta gcc aat gca att gtg ggt gta tgg cag gaa aga aat 336
gct gaa aat gcc atc gaa gcc ctt aag gaa tat gag cct gaa atg ggc 384
aaa gtg tat cga cag gac aga aag agt gtg cag cgg att aaa gct aaa 432
gac ata gtt cct ggt gat att gta gaa att gct gtt ggt gac aaa gtt 480
cct gct gat ata agg tta act tcc atc aaa tct acc aca cta aga gtt 528
gac cag tca att ctc aca ggt gaa tct gtc tct gtc atc aag cac act 576
gat ccc gtc cct gac cca cga gct gtc aac caa gat aaa aag aac atg 624
ctg ttt tct ggt aca aac att gct gct ggg aaa gct atg gga gtg gtg 672
gta gca act gga gtt aac acc gaa att ggc aag atc cgg gat gaa atg 720
gtg gca aca gaa cag gag aga aca ccc ctt cag caa aaa cta gat gaa 768
ttt ggg gaa cag ctt tcc aaa gtc atc tcc ctt att tgc att gca gtc 816
tgg atc ata aat att ggg cac ttc aat gac ccg gtt cat gga ggg tcc 864
tgg atc aga ggt gct att tac tac ttt aaa att gca gtg gcc ctg gct 912
gta gca gcc att cct gaa ggt ctg cct gca gtc atc acc acc tgc ctg 960
gct ctt gga act cgc aga atg gca aag aaa aat gcc att gtt cga agc 1008
ctc ccg tct gtg gaa acc ctt ggt tgt act tct gtt atc tgc tca gac 1056
aag act ggt aca ctt aca aca aac cag atg tca gtc tgc agg atg ttc 1104
att ctg gac aga gtg gaa ggt gat act tgt tcc ctt aat gag ttt acc 1152
ata act gga tca act tat gca cct att gga gaa gtg cat aaa gat gat 1200
aaa cca gtg aat tgt cac cag tat gat ggt ctg gta gaa tta gca aca 1248
att tgt gct ctt tgt aat gac tct gct ttg gat tac aat gag gca aag 1296
ggt gtg tat gaa aaa gtt gga gaa gct aca gag act gct ctc act tgc 1344
cta gta gag aag atg aat gta ttt gat acc gaa ttg aag ggt ctt tct 1392
aaa ata gaa cgt gca aat gcc tgc aac tca gtc att aaa cag ctg atg 1440
aaa aag gaa ttc act cta gag ttt tca cgt gac aga aag tca atg tcg 1488
gtt tac tgt aca cca aat aaa cca agc agg aca tca atg agc aag atg 1536
ttt gtg aag ggt gct cct gaa ggt gtc att gac agg tgc acc cac att 1584
cga gtt gga agt act aag gtt cct atg acc tct gga gtc aaa cag aag 1632
atc atg tct gtc att cga gag tgg ggt agt ggc agc gac aca ctg cga 1680
tgc ctg gcc ctg gcc act cat gac aac cca ctg aga aga gaa gaa atg 1728
cac ctt gag gac tct gcc aac ttt att aaa tat gag acc aat ctg acc 1776
ttc gtt ggc tgc gtg ggc atg ctg gat cct ccg aga atc gag gtg gcc 1824
tcc tcc gtg aag ctg tgc cgg caa gca ggc atc cgg gtc atc atg atc 1872
act ggg gac aac aag ggc act gct gtg gcc atc tgt cgc cgc atc ggc 1920
atc ttc ggg cag gat gag gac gtg acg tca aaa gct ttc aca ggc cgg 1968
gag ttt gat gaa ctc aac ccc tcc gcc cag cga gac gcc tgc ctg aac 2016
gcc cgc tgt ttt gct cga gtt gaa ccc tcc cac aag tct aaa atc gta 2064
gaa ttt ctt cag tct ttt gat gag att aca gct atg act ggc gat ggc 2112
gtg aac gat gct cct gct ctg aag aaa gcc gag att ggc att gct atg 2160
ggc tct ggc act gcg gtg gct aaa acc gcc tct gag atg gtc ctg gcg 2208
gat gac aac ttc tcc acc att gtg gct gcc gtt gag gag ggg cgg gca 2256
atc tac aac aac atg aaa cag ttc atc cgc tac ctc atc tcg tcc aac 2304
gtc ggg gaa gtt gtc tgt att ttc ctg aca gca gcc ctt gga ttt ccc 2352
gag gct ttg att cct gtt cag ctg ctc tgg gtc aat ctg gtg aca gat 2400
ggc ctg cct gcc act gca ctg ggg ttc aac cct cct gat ctg gac atc 2448
atg aat aaa cct ccc cgg aac cca aag gaa cca ttg atc agc ggg tgg 2496
ctc ttt ttc cgt tac ttg gct att ggc tgt tac gtc ggc gct gct acc 2544
gtg ggt gct gct gca tgg tgg ttc att gct gct gac ggt ggt cca aga 2592
gtg tcc ttc tac cag ctg agt cat ttc cta cag tgt aaa gag gac aac 2640
ccg gac ttt gaa ggc gtg gat tgt gca atc ttt gaa tcc cca tac ccg 2688
atg aca atg gcg ctc tct gtt cta gta act ata gaa atg tgt aac gcc 2736
ctc aac agc ttg tcc gaa aac cag tcc ttg ctg agg atg ccc ccc tgg 2784
gag aac atc tgg ctc gtg ggc tcc atc tgc ctg tcc atg tca ctc cac 2832
ttc ctg atc ctc tat gtc gaa ccc ttg cca ctc atc ttc cag atc aca 2880
ccg ctg aac gtg acc cag tgg ctg atg gtg ctg aaa atc tcc ttg ccc 2928
gtg att ctc atg gat gag acg ctc aag ttt gtg gcc cgc aac tac ctg 2976
gaa cct gca ata ctg gag taaccgcttc ctaaaccatt ttgcagaaat 3024
gtaagggtgt tcggttgcgt gcatgtgcgt ttttagcaac acatctacca accctgtgca 3084
tgactgatgt tggggaaaaa gaaaagtaaa aaacttccca actcactttg tgttatgtgg 3144
aggaaatgtg tattaccaat ggggttgtta gcttttaaat caaaatactg attacagatg 3204
tacaatttag cttaatcaga aagcctctcc agagaagttt ggtttctttg ctgcaagagg 3264
aatgaggctc tgtaacctta tctaagaact tggaagccgt cagccaagtc gccacatttc 3324
tctgcaaaat gtcatagctt atataaatgt acagtattca attgtaatgc atgcttcggt 3384
tgtaagtagc cagatccctc tccagtgaca ttggaacatg ctacttttta attggccctg 3444
tacagtttgc ttatttataa attcattaaa aacactacag gtgttgaatg gttaaaatgt 3504
aggcctccag ttcattttca gttattttct gagtgtgcag acagctattt cgcactgtat 3564
taaatgtaac ttatttaatg aaatcagaag cagtagacag atgttggtgc aatacaaata 3624
ttgtgatgca tttatcttaa taaaatgcta aatgtcaatt tatcactgcg catgtttgac 3684
tttagactgt aaatagagat cagtttgttt ctttctgtgc tggtaacaat gagcgtcgca 3744
cagacatggt ttcaggtaaa taaatctatt ctatgat 3781

<210> 28
<211> 997
<212> PRT
<213> Homo sapiens
<400> 28

<210> 29
<211> 1103
<212> DNA
<213> Homo sapiens
<220>
<221> CDS
<222> (133)..(834)
<400> 29
cttcggtcct gctgtagtgc cttctgcgcc aggcccggtt caatcagcgg ccacaactgt 60
ctagggctca gacaccacca gccaatgagg gagggcacgt ggagccgcgt ctgggctcgc 120
ggctcctgac ca atg ggg aag tgg cat gtg gga ggg cgc cgg ggt tcc ccc 171

Met Gly Lys Trp His Val Gly Gly Arg Arg Gly Ser Pro
1               5                   10
cgc caa tgg gga gct acg gcg cgc ggc cgg gac ttg gag gcg gtg cgg 219
Arg Gln Trp Gly Ala Thr Ala Arg Gly Arg Asp Leu Glu Ala Val Arg
    15                  20                  25
cgc ggc ggg tgc ggt tca gtc ggt cgg cgg cgg cag cgg agg agg agg 267
Arg Gly Gly Cys Gly Ser Val Gly Arg Arg Arg Gln Arg Arg Arg Arg
30                  35                  40                  45
agg agg agg agg atg agg agg atg agg agg atg tgg gcc acg cag ggg 315
Arg Arg Arg Arg Met Arg Arg Met Arg Arg Met Trp Ala Thr Gln Gly
                50                  55                  60
ctg gcg gtg cgc gtg gct ctg agc gtg ctg ccg ggc agc cgg gcg ctg 363
Leu Ala Val Arg Val Ala Leu Ser Val Leu Pro Gly Ser Arg Ala Leu
            65                  70                  75
cgg ccg ggc gac tgc gaa gtt tgt att tct tat ctg gga aga ttt tac 411
Arg Pro Gly Asp Cys Glu Val Cys Ile Ser Tyr Leu Gly Arg Phe Tyr
        80                  85                  90
cag gac ctc aaa gac aga gat gtc aca ttc tca cca gcc act att gaa 459
Gln Asp Leu Lys Asp Arg Asp Val Thr Phe Ser Pro Ala Thr Ile Glu
    95                  100                 105
aac gaa ctt ata aag ttc tgc cgg gaa gca aga ggc aaa gag aat cgg 507
Asn Glu Leu Ile Lys Phe Cys Arg Glu Ala Arg Gly Lys Glu Asn Arg
110                 115                 120                 125
ttg tgc tac tat atc ggg gcc aca gat gat gca gcc acc aaa atc atc 555
Leu Cys Tyr Tyr Ile Gly Ala Thr Asp Asp Ala Ala Thr Lys Ile Ile
                130                 135                 140

aat gag gta tca aag cct ctg gcc cac cac atc cct gtg gag aag atc 603
Asn Glu Val Ser Lys Pro Leu Ala His His Ile Pro Val Glu Lys Ile
            145                 150                 155
tgt gag aag ctt aag aag aag gac agc cag ata tgt gag ctt aag tat 651
Cys Glu Lys Leu Lys Lys Lys Asp Ser Gln Ile Cys Glu Leu Lys Tyr
        160                 165                 170
gac aag cag atc gac ctg agc aca gtg gac ctg aag aag ctc cga gtt 699
Asp Lys Gln Ile Asp Leu Ser Thr Val Asp Leu Lys Lys Leu Arg Val
    175                 180                 185
aaa gag ctg aag aag att ctg gat gac tgg ggg gag aca tgc aaa ggc 747
Lys Glu Leu Lys Lys Ile Leu Asp Asp Trp Gly Glu Thr Cys Lys Gly
190                 195                 200                 205
tgt gca gaa aag tct gac tac atc cgg aag ata aat gaa ctg atg cct 795
Cys Ala Glu Lys Ser Asp Tyr Ile Arg Lys Ile Asn Glu Leu Met Pro
                210                 215                 220
aaa tat gcc ccc aag gca gcc agt gca ccg acc gat ttg tagtctgctc 844
Lys Tyr Ala Pro Lys Ala Ala Ser Ala Pro Thr Asp Leu
            225                 230
aatctctgtt gcacctgagg gggaaaaaac agttcaactg cttactccca aaacagcctt 904
tttgtaattt attttttaag tgggctcctg acaatactgt atcagatgtg aagcctggag 964
ctttcctgat gatgctggcc ctacagtacc cccatgaggg gattcccttc cttctgttgc 1024
tggtgtactc taggacttca aagtgtgtct gggatttttt tattaaagaa aaaaaatttc 1084
tagctgtcaa aaaaaaaaa 1103

<210> 30
<211> 234
<212> PRT

<213> Homo sapiens

<400> 30

Met Gly Lys Trp His Val Gly Gly Arg Arg Gly Ser Pro Arg Gln Trp

1               5                   10                  15

Gly Ala Thr Ala Arg Gly Arg Asp Leu Glu Ala Val Arg Arg Gly Gly

            20                  25                  30

Cys Gly Ser Val Gly Arg Arg Arg Gln Arg Arg Arg Arg Arg Arg Arg

        35                  40                  45

Arg Met Arg Arg Met Arg Arg Met Trp Ala Thr Gln Gly Leu Ala Val

    50                  55                  60

Arg Val Ala Leu Ser Val Leu Pro Gly Ser Arg Ala Leu Arg Pro Gly

65                  70                  75                  80

Asp Cys Glu Val Cys Ile Ser Tyr Leu Gly Arg Phe Tyr Gln Asp Leu

                85                  90                  95

Lys Asp Arg Asp Val Thr Phe Ser Pro Ala Thr Ile Glu Asn Glu Leu

            100                 105                 110

Ile Lys Phe Cys Arg Glu Ala Arg Gly Lys Glu Asn Arg Leu Cys Tyr

        115                 120                 125

Tyr Ile Gly Ala Thr Asp Asp Ala Ala Thr Lys Ile Ile Asn Glu Val

    130                 135                 140

Ser Lys Pro Leu Ala His His Ile Pro Val Glu Lys Ile Cys Glu Lys

145                 150                 155                 160

Leu Lys Lys Lys Asp Ser Gln Ile Cys Glu Leu Lys Tyr Asp Lys Gln

                165                 170                 175

Ile Asp Leu Ser Thr Val Asp Leu Lys Lys Leu Arg Val Lys Glu Leu

            180                 185                 190

Lys Lys Ile Leu Asp Asp Trp Gly Glu Thr Cys Lys Gly Cys Ala Glu

        195                 200                 205
Lys Ser Asp Tyr Ile Arg Lys Ile Asn Glu Leu Met Pro Lys Tyr Ala
    210                 215                 220
Pro Lys Ala Ala Ser Ala Pro Thr Asp Leu
225                 230

<210> 31
<211> 1860
<212> DNA
<213> Homo sapiens
<220>
<221> CDS
<222> (94)..(1266)
<400> 31
cgtgaacggt cgttgcagag attgcgggcg gctgagacgc cgcctgcctg gcacctagga 60
gcgcagcgga gccccgacac cgccgccgcc gcc atg gag tcc gag acc gaa ccc 114
Met Glu Ser Glu Thr Glu Pro
1               5
gag ccc gtc acg ctc ctg gtg aag agc ccc aac cag cgc cac cgc gac 162
Glu Pro Val Thr Leu Leu Val Lys Ser Pro Asn Gln Arg His Arg Asp
        10                  15                  20
ttg gag ctg agt ggc gac cgc ggc tgg agt gtg ggc cac ctc aag gcc 210
Leu Glu Leu Ser Gly Asp Arg Gly Trp Ser Val Gly His Leu Lys Ala
    25                  30                  35
cac ctg agc cgc gtc tac ccc gag cgt ccg cgt cca gag gac cag agg 258
His Leu Ser Arg Val Tyr Pro Glu Arg Pro Arg Pro Glu Asp Gln Arg
40                  45                  50                  55

tta att tat tct ggg aag ctg ttg ttg gat cac caa tgt ctc agg gac 306
Leu Ile Tyr Ser Gly Lys Leu Leu Leu Asp His Gln Cys Leu Arg Asp
                60                  65                  70
ttg ctt cca aag cag gaa aaa cgg cat gtt ttg cat ctg gtg tgc aat 354
Leu Leu Pro Lys Gln Glu Lys Arg His Val Leu His Leu Val Cys Asn
            75                  80                  85
gtg aag agt cct tca aaa atg cca gaa atc aac gcc aag gtg gct gaa 402
Val Lys Ser Pro Ser Lys Met Pro Glu Ile Asn Ala Lys Val Ala Glu
        90                  95                  100
tcc aca gag gag cct gct ggt tct aat cgg gga cag tat cct gag gat 450
Ser Thr Glu Glu Pro Ala Gly Ser Asn Arg Gly Gln Tyr Pro Glu Asp
    105                 110                 115
tcc tca agt gat ggt tta agg caa agg gaa gtt ctt cgg aac ctt tct 498
Ser Ser Ser Asp Gly Leu Arg Gln Arg Glu Val Leu Arg Asn Leu Ser
120                 125                 130                 135
tcc cct gga tgg gaa aac atc tca agg cct gaa gct gcc cag cag gca 546
Ser Pro Gly Trp Glu Asn Ile Ser Arg Pro Glu Ala Ala Gln Gln Ala
                140                 145                 150
ttc caa ggc ctg ggt cct ggt ttc tcc ggt tac aca ccc tat ggg tgg 594
Phe Gln Gly Leu Gly Pro Gly Phe Ser Gly Tyr Thr Pro Tyr Gly Trp
            155                 160                 165
ctt cag ctt tcc tgg ttc cag cag ata tat gca cga cag tac tac atg 642
Leu Gln Leu Ser Trp Phe Gln Gln Ile Tyr Ala Arg Gln Tyr Tyr Met
        170                 175                 180
caa tat tta gca gcc act gct gca tca ggg gct ttt gtt cca cca cca 690
Gln Tyr Leu Ala Ala Thr Ala Ala Ser Gly Ala Phe Val Pro Pro Pro

    185                 190                 195

agt gca caa gag ata cct gtg gtc tct gca cct gct cca gcc cct att 738

Ser Ala Gln Glu Ile Pro Val Val Ser Ala Pro Ala Pro Ala Pro Ile

200                 205                 210                 215

cac aac cag ttt cca gct gaa aac cag cct gcc aat cag aat gct gct 786

His Asn Gln Phe Pro Ala Glu Asn Gln Pro Ala Asn Gln Asn Ala Ala

                220                 225                 230

cct caa gtg gtt gtt aat cct gga gcc aat caa aat ttg cgg atg aat 834

Pro Gln Val Val Val Asn Pro Gly Ala Asn Gln Asn Leu Arg Met Asn

            235                 240                 245

gca caa ggt ggc cct att gtg gaa gaa gat gat gaa ata aat cga gat 882

Ala Gln Gly Gly Pro Ile Val Glu Glu Asp Asp Glu Ile Asn Arg Asp

        250                 255                 260

tgg ttg gat tgg acc tat tca gca gct aca ttt tct gtt ttt ctc agt 930

Trp Leu Asp Trp Thr Tyr Ser Ala Ala Thr Phe Ser Val Phe Leu Ser

    265                 270                 275

atc ctc tac ttc tac tcc tcc ctg agc aga ttc ctc atg gtc atg ggg 978

Ile Leu Tyr Phe Tyr Ser Ser Leu Ser Arg Phe Leu Met Val Met Gly

280                 285                 290                 295

gcc acc gtt gtt atg tac ctg cat cac gtt ggg tgg ttt cca ttt aga 1026

Ala Thr Val Val Met Tyr Leu His His Val Gly Trp Phe Pro Phe Arg

                300                 305                 310

ccg agg ccg gtt cag aac ttc cca aat gat ggt cct cct cct gac gtt 1074

Pro Arg Pro Val Gln Asn Phe Pro Asn Asp Gly Pro Pro Pro Asp Val

            315                 320                 325

gta aat cag gac ccc aac aat aac tta cag gaa ggc act gat cct gaa 1122

Val Asn Gln Asp Pro Asn Asn Asn Leu Gln Glu Gly Thr Asp Pro Glu

        330                 335                 340

act gaa gac ccc aac cac ctc cct cca gac agg gat gta cta gat ggc 1170

Thr Glu Asp Pro Asn His Leu Pro Pro Asp Arg Asp Val Leu Asp Gly

    345                 350                 355

gag cag acc agc ccc tcc ttt atg agc aca gca tgg ctt gtc ttc aag 1218

Glu Gln Thr Ser Pro Ser Phe Met Ser Thr Ala Trp Leu Val Phe Lys

360                 365                 370                 375

act ttc ttt gcc tct ctt ctt cca gaa ggc ccc cca gcc atc gca aac 1266

Thr Phe Phe Ala Ser Leu Leu Pro Glu Gly Pro Pro Ala Ile Ala Asn

                380                 385                 390

tgatggtgtt tgtgctgtag ctgttggagg ctttgacagg aatggactgg atcacctgac 1326

tccagctaga ttgcctctcc tggacatggc aatgatgagt ttttaaaaaa cagtgtggat 1386

gatgatatgc ttttgtgagc aagcaaaagc agaaacgtga agccgtgata caaattggtg 1446

aacaaaaaat gcccaaggct tctcatgtgt ttattctgaa gagctttaat atatactcta 1506

tgtagtttaa taagcactgt acgtagaagg ccttaggtgt tgcatgtcta tgcttgagga 1566

acttttccaa atgtgtgtgt ctgcatgtgt gtttgtacat agaagtcata gatgcagaag 1626

tggttctgct ggtaagattt gattcctgtt ggaatgttta aattacacta agtgtactac 1686

tttatataat caatgaaatt gctagacatg ttttagcagg acttttctag gaaagactta 1746

tgtataattg ctttttaaaa tgcagtgctt tactttaaac taaggggaac tttgcggagg 1806

tgaaaacctt tgctgggttt tctgttcaat aaagttttac tatgaatgac cctg 1860

<210> 32

<211> 391

<212> PRT

<213> Homo sapiens

<400> 32

Met Glu Ser Glu Thr Glu Pro Glu Pro Val Thr Leu Leu Val Lys Ser

1               5                   10                  15
Pro Asn Gln Arg His Arg Asp Leu Glu Leu Ser Gly Asp Arg Gly Trp
            20                  25                  30
Ser Val Gly His Leu Lys Ala His Leu Ser Arg Val Tyr Pro Glu Arg
        35                  40                  45
Pro Arg Pro Glu Asp Gln Arg Leu Ile Tyr Ser Gly Lys Leu Leu Leu
    50                  55                  60
Asp His Gln Cys Leu Arg Asp Leu Leu Pro Lys Gln Glu Lys Arg His
65                  70                  75                  80
Val Leu His Leu Val Cys Asn Val Lys Ser Pro Ser Lys Met Pro Glu
                85                  90                  95
Ile Asn Ala Lys Val Ala Glu Ser Thr Glu Glu Pro Ala Gly Ser Asn
            100                 105                 110
Arg Gly Gln Tyr Pro Glu Asp Ser Ser Ser Asp Gly Leu Arg Gln Arg
        115                 120                 125
Glu Val Leu Arg Asn Leu Ser Ser Pro Gly Trp Glu Asn Ile Ser Arg
    130                 135                 140
Pro Glu Ala Ala Gln Gln Ala Phe Gln Gly Leu Gly Pro Gly Phe Ser
145                 150                 155                 160
Gly Tyr Thr Pro Tyr Gly Trp Leu Gln Leu Ser Trp Phe Gln Gln Ile
                165                 170                 175
Tyr Ala Arg Gln Tyr Tyr Met Gln Tyr Leu Ala Ala Thr Ala Ala Ser
            180                 185                 190
Gly Ala Phe Val Pro Pro Pro Ser Ala Gln Glu Ile Pro Val Val Ser
        195                 200                 205
Ala Pro Ala Pro Ala Pro Ile His Asn Gln Phe Pro Ala Glu Asn Gln

210                 215                 220

Pro Ala Asn Gln Asn Ala Ala Pro Gln Val Val Val Asn Pro Gly Ala

225                 230                 235                 240

Asn Gln Asn Leu Arg Met Asn Ala Gln Gly Gly Pro Ile Val Glu Glu

                245                 250                 255

Asp Asp Glu Ile Asn Arg Asp Trp Leu Asp Trp Thr Tyr Ser Ala Ala

            260                 265                 270

Thr Phe Ser Val Phe Leu Ser Ile Leu Tyr Phe Tyr Ser Ser Leu Ser

        275                 280                 285

Arg Phe Leu Met Val Met Gly Ala Thr Val Val Met Tyr Leu His His

    290                 295                 300

Val Gly Trp Phe Pro Phe Arg Pro Arg Pro Val Gln Asn Phe Pro Asn

305                 310                 315                 320

Asp Gly Pro Pro Pro Asp Val Val Asn Gln Asp Pro Asn Asn Asn Leu

                325                 330                 335

Gln Glu Gly Thr Asp Pro Glu Thr Glu Asp Pro Asn His Leu Pro Pro

            340                 345                 350

Asp Arg Asp Val Leu Asp Gly Glu Gln Thr Ser Pro Ser Phe Met Ser

        355                 360                 365

Thr Ala Trp Leu Val Phe Lys Thr Phe Phe Ala Ser Leu Leu Pro Glu

    370                 375                 380

Gly Pro Pro Ala Ile Ala Asn

385                 390

<210> 33

<211> 4067

<212> DNA

<213> Homo sapiens

<220>

<221> CDS

<222> (812)..(1138)

<400> 33
   cttgaatctt ggggcaggaa ctcagaaaac ttccagcccg ggcagcgcgc gcttggtgca 60
   agactcagga gctagcagcc cgtccccctc cgactctccg gtgccgccgc tgcctgctcc 120
   cgccacccta ggaggcgcgg tgccacccac tactctgtcc tctgcctgtg ctccgtgccc 180
   gaccctatcc cggcggagtc tccccatcct cctttgcttt ccgactgccc aaggcacttt 240
   caatctcaat ctcttctctc tctctctctc tctctgtctc tctctctctc tctctctctc 300
   tctctctctc gcagggtggg gggaagagga ggaggaattc tttccccgcc taacatttca 360
   agggacacaa ttcactccaa gtctcttccc tttccaagcc gcttccgaag tgctcccggt 420
   gcccgcaact cctgatccca acccgcgaga ggagcctctg cgacctcaaa gcctctcttc 480
   cttctccctc gcttccctcc tcctcttgct acctccacct ccaccgccac ctccacctcc 540
   ggcacccacc caccgccgcc gccgccaccg gcagcgcctc ctcctctcct cctcctcctc 600
   ccctcttctc tttttggcag ccgctggacg tccggtgttg atggtggcag cggcggcagc 660
   ctaagcaaca gcagccctcg cagcccgcca gctcgcgctc gccccgccgg cgtccccagc 720
   cctatcacct catctcccga aaggtgctgg gcagctccgg ggcggtcgag gcgaagcggc 780
   tgcagcggcg gtagcggcgg cgggaggcag g atg agc gca cgc ggt gag ggc 832
   Met Ser Ala Arg Gly Glu Gly
   1 5
   gcg ggg cag ccg tcc act tca gcc cag gga caa cct gcc gcc cca gcg 880
   Ala Gly Gln Pro Ser Thr Ser Ala Gln Gly Gln Pro Ala Ala Pro Ala
   10 15 20
   cct cag aag aga gga cgc ggc cgc ccc agg aag cag cag caa gaa cca 928
   Pro Gln Lys Arg Gly Arg Gly Arg Pro Arg Lys Gln Gln Gln Glu Pro
   25 30 35
   acc ggt gag ccc tct cct aag aga ccc agg gga aga ccc aaa ggc agc 976
   Thr Gly Glu Pro Ser Pro Lys Arg Pro Arg Gly Arg Pro Lys Gly Ser
   40 45 50 55
   aaa aac aag agt ccc tct aaa gca gct caa aag aaa gca gaa gcc act 1024
   Lys Asn Lys Ser Pro Ser Lys Ala Ala Gln Lys Lys Ala Glu Ala Thr
   60 65 70
   gga gaa aaa cgg cca aga ggc aga cct agg aaa tgg cca caa caa gtt 1072
   Gly Glu Lys Arg Pro Arg Gly Arg Pro Arg Lys Trp Pro Gln Gln Val
   75 80 85
   gtt cag aag aag cct gct cag gag gaa act gaa gag aca tcc tca caa 1120
   Val Gln Lys Lys Pro Ala Gln Glu Glu Thr Glu Glu Thr Ser Ser Gln
   90 95 100
   gag tct gcc gaa gag gac tagggggcgc aacgttcgat ttctacctca 1168
   Glu Ser Ala Glu Glu Asp
   105
   gcagcagttg gatcttttga agggagaaga cactgcagtg accacttatt ctgtattgcc 1228
   atggtctttc cactttcatc tggggtgggg tggggtgggg tgggggaggg gggggtgggg 1288
   tggggagaaa tcacataacc ttaaaaagga ctatattaat caccttcttt gtaatccctt 1348
   cacagtccca ggtttagtga aaaactgctg taaacacagg ggacacagct taacaatgca 1408
   acttttaatt actgttttct tttttcttaa cctactaata gtttgttgat ctgataagca 1468
   agagtgggcg ggtgagaaaa accgaattgg gtttagtcaa tcactgcact gcatgcaaac 1528
   aagaaacgtg tcacacttgt gacgtcgggc attcatatag gaagaacgcg gtgtgtaaca 1588
   ctgtgtacac ctcaaatacc accccaaccc actccctgta gtgaatcctc tgtttagaac 1648
   accaaagata aggactagat actactttct ctttttcgta taatcttgta gacacttact 1708
   tgatgatttt taacttttta tttctaaatg agacgaaatg ctgatgtatc ctttcattca 1768
   gctaacaaac tagaaaaggt tatgttcatt tttcaaaaag ggaagtaagc aaacaaatat 1828
   tgccaactct tctatttatg gatatcacac atatcagcag gagtaataaa tttactcaca 1888
   gcacttgttt tcaggacaac acttcatttt caggaaatct acttcctaca gagccaaaat 1948
   gccatttagc aataaataac acttgtcagc ctcagagcat ttaaggaaac tagacaagta 2008
   aaattatcct ctttgtaatt taatgaaaag gtacaacaga ataatgcatg atgaactcac 2068
   ctaattatga ggtgggagga gcgaaatcta aatttctttt gctatagtta tacatcaatt 2128
   taaaaagcaa aaaaaaaaag gggggggcaa tctctctctg tgtctttctc tctctctctc 2188
   cctctccctc tctcttttca tgtgtatcag tttccatgaa agacctgaat accacttacc 2248
   tcaaattaag catatgtgtt acttcaagta atacgttttg acataagatg gttgaccaag 2308
   gtgcttttct tcggcttgag ttcaccatct cttcattcaa actgcacttt tagccagaga 2368
   tgcaatatat ccccactact caatactacc tctgaatgtt acaacgaatt tacagtctag 2428
   tacttattac atgctgctat acacaagcaa tgcaagaaaa aaacttactg ggtaggtgat 2488
   tctaatcatc tgcagttctt tttgtacact taattacagt taaagaagca atctccttac 2548
   tgtgtttcag catgactatg tatttttcta tgttttttta attaaaaatt tttaaaatac 2608
   ttgtttcagc ttctctgcta gatttctaca ttaacttgaa aattttttaa ccaagtcgct 2668
   cctaggttct taaggataat tttcctcaat cacactacac atcacacaag atttgactgt 2728
   aatatttaaa tattaccctc caagtctgta cctcaaatga attctttaag gagatggact 2788
   aattgacttg caaagaccta cctccagact tcaaaaggaa tgaacttgtt acttgcagca 2848
   ttcatttgtt ttttcaatgt ttgaaatagt tcaaactgca gctaacccta gtcaaaacta 2908
   tttttgtaaa agacatttga tagaaaggaa cacgttttta catacttttg caaaataagt 2968
   aaataataaa taaaataaag ccaaccttca aagaacttga agctttgtag gtgagatgca 3028
   acaagccctg cttttgcata atgcaatcaa aaatatgtgt ttttaagatt agttgaatat 3088
   aagaaaatgc ttgacaaata ttttcatgta ttttacacaa atgtgatttt tgtaatatgt 3148
   ctcaaccaga tttattttaa acgcttctta tgtagagttt ttatgccttt ctctcctagt 3208
   gagtgtgctg actttttaac atggtattat caactgggcc aggaggtagt ttctcatgac 3268
   ggcttttgtc agtatggctt ttagtactga agccaaatga aactcaaaac catctctctt 3328
   ccagctgctt cagggaggta gtttcaaagg ccacatacct ctctgagact ggcagatcgc 3388
   tcactgttgt gaatcaccaa aggagctatg gagagaatta aaactcaaca ttactgttaa 3448
   ctgtgcgtta aataagcaaa taaacagtgg ctcataaaaa taaaagtcgc attccatatc 3508
   tttggatggg ccttttagaa acctcattgg ccagctcata aaatggaagc aattgctcat 3568
   gttggccaaa catggtgcac cgagtgattt ccatctctgg taaagttaca cttttatttc 3628
   ctgtatgttg tacaatcaaa acacactact acctcttaag tcccagtata cctcattttt 3688
   catactgaaa aaaaaagctt gtggccaatg gaacagtaag aacatcataa aatttttata 3748
   tatatagttt atttttgtgg gagataaatt ttataggact gttctttgct gttgttggtc 3808
   gcagctacat aagactggac atttaacttt tctaccattt ctgcaagtta ggtatgtttg 3868
   caggagaaaa gtatcaagac gtttaactgc agttgacttt ctccctgttc ctttgagtgt 3928
   cttctaactt tattctttgt tctttatgta gaattgctgt ctatgattgt actttgaatc 3988
   gcttgcttgt tgaaaatatt tctctagtgt attatcactg tctgttctgc acaataaaca 4048
   taacagcctc tgtgatccc 4067

<210> 34

<211> 109

<212> PRT

<213> Homo sapiens

<400> 34

Met Ser Ala Arg Gly Glu Gly Ala Gly Gln Pro Ser Thr Ser Ala Gln
1               5                   10                  15

Gly Gln Pro Ala Ala Pro Ala Pro Gln Lys Arg Gly Arg Gly Arg Pro
            20                  25                  30

Arg Lys Gln Gln Gln Glu Pro Thr Gly Glu Pro Ser Pro Lys Arg Pro
        35                  40                  45

Arg Gly Arg Pro Lys Gly Ser Lys Asn Lys Ser Pro Ser Lys Ala Ala
    50                  55                  60

Gln Lys Lys Ala Glu Ala Thr Gly Glu Lys Arg Pro Arg Gly Arg Pro
65                  70                  75                  80

Arg Lys Trp Pro Gln Gln Val Val Gln Lys Lys Pro Ala Gln Glu Glu
                85                  90                  95
Thr Glu Glu Thr Ser Ser Gln Glu Ser Ala Glu Glu Asp
            100                 105

<210> 35
<211> 4228
<212> DNA
<213> Homo sapiens
<220>
<221> CDS
<222> (148)..(1032)
<400> 35
ggcgcagtag cagcgagcag cagagtccgc acgctccggc gaggggcaga agagcgcgag 60
ggagcgcggg gcagcagaag cgagagccga gcgcggaccc agccaggacc cacagccctc 120
cccagctgcc caggaagagc cccagcc atg gaa cac cag ctc ctg tgc tgc gaa 174
Met Glu His Gln Leu Leu Cys Cys Glu
1               5
gtg gaa acc atc cgc cgc gcg tac ccc gat gcc aac ctc ctc aac gac 222
Val Glu Thr Ile Arg Arg Ala Tyr Pro Asp Ala Asn Leu Leu Asn Asp
10                  15                  20                  25
cgg gtg ctg cgg gcc atg ctg aag gcg gag gag acc tgc gcg ccc tcg 270
Arg Val Leu Arg Ala Met Leu Lys Ala Glu Glu Thr Cys Ala Pro Ser
                30                  35                  40
gtg tcc tac ttc aaa tgt gtg cag aag gag gtc ctg ccg tcc atg cgg 318
Val Ser Tyr Phe Lys Cys Val Gln Lys Glu Val Leu Pro Ser Met Arg
            45                  50                  55

aag atc gtc gcc acc tgg atg ctg gag gtc tgc gag gaa cag aag tgc 366
Lys Ile Val Ala Thr Trp Met Leu Glu Val Cys Glu Glu Gln Lys Cys
        60                  65                  70
gag gag gag gtc ttc ccg ctg gcc atg aac tac ctg gac cgc ttc ctg 414
Glu Glu Glu Val Phe Pro Leu Ala Met Asn Tyr Leu Asp Arg Phe Leu
    75                  80                  85
tcg ctg gag ccc gtg aaa aag agc cgc ctg cag ctg ctg ggg gcc act 462
Ser Leu Glu Pro Val Lys Lys Ser Arg Leu Gln Leu Leu Gly Ala Thr
90                  95                  100                 105
tgc atg ttc gtg gcc tct aag atg aag gag acc atc ccc ctg acg gcc 510
Cys Met Phe Val Ala Ser Lys Met Lys Glu Thr Ile Pro Leu Thr Ala
                110                 115                 120
gag aag ctg tgc atc tac acc gac aac tcc atc cgg ccc gag gag ctg 558
Glu Lys Leu Cys Ile Tyr Thr Asp Asn Ser Ile Arg Pro Glu Glu Leu
            125                 130                 135
ctg caa atg gag ctg ctc ctg gtg aac aag ctc aag tgg aac ctg gcc 606
Leu Gln Met Glu Leu Leu Leu Val Asn Lys Leu Lys Trp Asn Leu Ala
        140                 145                 150
gca atg acc ccg cac gat ttc att gaa cac ttc ctc tcc aaa atg cca 654
Ala Met Thr Pro His Asp Phe Ile Glu His Phe Leu Ser Lys Met Pro
    155                 160                 165
gag gcg gag gag aac aaa cag atc atc cgc aaa cac gcg cag acc ttc 702
Glu Ala Glu Glu Asn Lys Gln Ile Ile Arg Lys His Ala Gln Thr Phe
170                 175                 180                 185
gtt gcc ctc tgt gcc aca gat gtg aag ttc att tcc aat ccg ccc tcc 750
Val Ala Leu Cys Ala Thr Asp Val Lys Phe Ile Ser Asn Pro Pro Ser

                190                 195                 200

atg gtg gca gcg ggg agc gtg gtg gcc gca gtg caa ggc ctg aac ctg 798

Met Val Ala Ala Gly Ser Val Val Ala Ala Val Gln Gly Leu Asn Leu

            205                 210                 215

agg agc ccc aac aac ttc ctg tcc tac tac cgc ctc aca cgc ttc ctc 846

Arg Ser Pro Asn Asn Phe Leu Ser Tyr Tyr Arg Leu Thr Arg Phe Leu

        220                 225                 230

tcc aga gtg atc aag tgt gac cca gac tgc ctc cgg gcc tgc cag gag 894

Ser Arg Val Ile Lys Cys Asp Pro Asp Cys Leu Arg Ala Cys Gln Glu

    235                 240                 245

cag atc gaa gcc ctg ctg gag tca agc ctg cgc cag gcc cag cag aac 942

Gln Ile Glu Ala Leu Leu Glu Ser Ser Leu Arg Gln Ala Gln Gln Asn

250                 255                 260                 265

atg gac ccc aag gcc gcc gag gag gag gaa gag gag gag gag gag gtg 990

Met Asp Pro Lys Ala Ala Glu Glu Glu Glu Glu Glu Glu Glu Glu Val

                270                 275                 280

gac ctg gct tgc aca ccc acc gac gtg cgg gac gtg gac atc 1032

Asp Leu Ala Cys Thr Pro Thr Asp Val Arg Asp Val Asp Ile

            285                 290                 295

tgagggcgcc aggcaggcgg gcgccaccgc cacccgcagc gagggcggag ccggccccag 1092

gtgctccact gacagtccct cctctccgga gcattttgat accagaaggg aaagcttcat 1152

tctccttgtt gttggttgtt ttttcctttg ctctttcccc cttccatctc tgacttaagc 1212

aaaagaaaaa gattacccaa aaactgtctt taaaagagag agagagaaaa aaaaaatagt 1272

atttgcataa ccctgagcgg tgggggagga gggttgtgct acagatgata gaggatttta 1332

taccccaata atcaactcgt ttttatatta atgtacttgt ttctctgttg taagaatagg 1392

cattaacaca aaggaggcgt ctcgggagag gattaggttc catcctttac gtgtttaaaa 1452

aaaagcataa aaacatttta aaaacataga aaaattcagc aaaccatttt taaagtagaa 1512

gagggtttta ggtagaaaaa catattcttg tgcttttcct gataaagcac agctgtagtg 1572
gggttctagg catctctgta ctttgcttgc tcatatgcat gtagtcactt tataagtcat 1632
tgtatgttat tatattccgt aggtagatgt gtaacctctt caccttattc atggctgaag 1692
tcacctcttg gttacagtag cgtagcgtgg ccgtgtgcat gtcctttgcg cctgtgacca 1752
ccaccccaac aaaccatcca gtgacaaacc atccagtgga ggtttgtcgg gcaccagcca 1812
gcgtagcagg gtcgggaaag gccacctgtc ccactcctac gatacgctac tataaagaga 1872
agacgaaata gtgacataat atattctatt tttatactct tcctattttt gtagtgacct 1932
gtttatgaga tgctggtttt ctacccaacg gccctgcagc cagctcacgt ccaggttcaa 1992
cccacagcta cttggtttgt gttcttcttc atattctaaa accattccat ttccaagcac 2052
tttcagtcca ataggtgtag gaaatagcgc tgtttttgtt gtgtgtgcag ggagggcagt 2112
tttctaatgg aatggtttgg gaatatccat gtacttgttt gcaagcagga ctttgaggca 2172
agtgtgggcc actgtggtgg cagtggaggt ggggtgtttg ggaggctgcg tgccagtcaa 2232
gaagaaaaag gtttgcattc tcacattgcc aggatgataa gttcctttcc ttttctttaa 2292
agaagttgaa gtttaggaat cctttggtgc caactggtgt ttgaaagtag ggacctcaga 2352
ggtttaccta gagaacaggt ggtttttaag ggttatctta gatgtttcac accggaaggt 2412
ttttaaacac taaaatatat aatttatagt taaggctaaa aagtatattt attgcagagg 2472
atgttcataa ggccagtatg atttataaat gcaatctccc cttgatttaa acacacagat 2532
acacacacac acacacacac acacacaaac cttctgcctt tgatgttaca gatttaatac 2592
agtttatttt taaagataga tccttttata ggtgagaaaa aaacaatctg gaagaaaaaa 2652
accacacaaa gacattgatt cagcctgttt ggcgtttccc agagtcatct gattggacag 2712
gcatgggtgc aaggaaaatt agggtactca acctaagttc ggttccgatg aattcttatc 2772
ccctgcccct tcctttaaaa aacttagtga caaaatagac aatttgcaca tcttggctat 2832
gtaattcttg taatttttat ttaggaagtg ttgaagggag gtggcaagag tgtggaggct 2892
gacgtgtgag ggaggacagg cgggaggagg tgtgaggagg aggctcccga ggggaagggg 2952
cggtgcccac accggggaca ggccgcagct ccatttcttt attgcgctgc taccgttgac 3012
ttccaggcac ggtttggaaa tattcacatc gcttctgtgt atctctttca cattgtttgc 3072

tgctattgga ggatcagttt tttgttttac aatgtcatat actgccatgt actagtttta 3132
gttttctctt agaacattgt attacagatg ccttttttgt agtttttttt ttttttatgt 3192
gatcaatttt gacttaatgt gattactgct ctattccaaa aaggttgctg tttcacaata 3252
cctcatgctt cacttagcca tggtggaccc agcgggcagg ttctgcctgc tttggcgggc 3312
agacacgcgg gcgcgatccc acacaggctg gcgggggccg gccccgaggc cgcgtgcgtg 3372
agaaccgcgc cggtgtcccc agagaccagg ctgtgtccct cttctcttcc ctgcgcctgt 3432
gatgctgggc acttcatctg atcgggggcg tagcatcata gtagttttta cagctgtgtt 3492
atwctttgcg tgtagctatg gaagttgcat aattattatt attattatta taacaagtgt 3552
gtcttacgtg ccaccacggc gttgtacctg taggactctc attcgggatg attggaatag 3612
cttctggaat ttgttcaagt tttgggtatg tttaatctgt tatgtactag tgttctgttt 3672
gttattgttt tgttaattac accataatgc taatttaaag agactccaaa tctcaatgaa 3732
gccagctcac agtgctgtgt gccccggtca cctagcaagc tgccgaacca aaagaatttg 3792
caccccgctg cgggcccacg tggttggggc cctgccctgg cagggtcatc ctgtgctcgg 3852
aggccatctc gggcacaggc ccaccccgcc ccacccctcc agaacacggc tcacgcttac 3912
ctcaaccatc ctggctgcgg cgtctgtctg aaccacgcgg gggccttgag ggacgctttg 3972
tctgtcgtga tggggcaagg gcacaagtcc tggatgttgt gtgtrtcgag aggccaaagg 4032
ctggtggcaa gtgcacgggg cacagcggag tctgtcctgt gacgcgcaag tctgagggtc 4092
tgggcggcgg gcggctgggt ctgtgcattt ctggttgcac cgcggcgctt cccagcacca 4152
acatgtaacc ggcatgtttc cagcagaaga caaaaagaca aacatgaaag tctagaaata 4212
aaactggtaa aacccc 4228

<210> 36

<211> 295

<212> PRT

<213> Homo sapiens

<400> 36

Met Glu His Gln Leu Leu Cys Cys Glu Val Glu Thr Ile Arg Arg Ala

1                   5                   10                  15

Tyr Pro Asp Ala Asn Leu Leu Asn Asp Arg Val Leu Arg Ala Met Leu

            20                  25                  30

Lys Ala Glu Glu Thr Cys Ala Pro Ser Val Ser Tyr Phe Lys Cys Val

        35                  40                  45

Gln Lys Glu Val Leu Pro Ser Met Arg Lys Ile Val Ala Thr Trp Met

    50                  55                  60

Leu Glu Val Cys Glu Glu Gln Lys Cys Glu Glu Glu Val Phe Pro Leu

65                  70                  75                  80

Ala Met Asn Tyr Leu Asp Arg Phe Leu Ser Leu Glu Pro Val Lys Lys

                85                  90                  95

Ser Arg Leu Gln Leu Leu Gly Ala Thr Cys Met Phe Val Ala Ser Lys

            100                 105                 110

Met Lys Glu Thr Ile Pro Leu Thr Ala Glu Lys Leu Cys Ile Tyr Thr

        115                 120                 125

Asp Asn Ser Ile Arg Pro Glu Glu Leu Leu Gln Met Glu Leu Leu Leu

    130                 135                 140

Val Asn Lys Leu Lys Trp Asn Leu Ala Ala Met Thr Pro His Asp Phe

145                 150                 155                 160

Ile Glu His Phe Leu Ser Lys Met Pro Glu Ala Glu Glu Asn Lys Gln

                165                 170                 175

Ile Ile Arg Lys His Ala Gln Thr Phe Val Ala Leu Cys Ala Thr Asp

            180                 185                 190

Val Lys Phe Ile Ser Asn Pro Pro Ser Met Val Ala Ala Gly Ser Val

        195                 200                 205

Val Ala Ala Val Gln Gly Leu Asn Leu Arg Ser Pro Asn Asn Phe Leu

    210                 215                 220

Ser Tyr Tyr Arg Leu Thr Arg Phe Leu Ser Arg Val Ile Lys Cys Asp
225                 230                 235                 240
Pro Asp Cys Leu Arg Ala Cys Gln Glu Gln Ile Glu Ala Leu Leu Glu
                245                 250                 255
Ser Ser Leu Arg Gln Ala Gln Gln Asn Met Asp Pro Lys Ala Ala Glu
            260                 265                 270
Glu Glu Glu Glu Glu Glu Glu Glu Val Asp Leu Ala Cys Thr Pro Thr
        275                 280                 285
Asp Val Arg Asp Val Asp Ile
    290                 295
<210> 37
<211> 5007
<212> DNA
<213> Homo sapiens
<220>
<221> CDS
<222> (436)..(3402)
<400> 37
ggggcgcccg cgggccggag ccggggcggg ggccggggcc taggcgcgcg gacctgcgag 60
cggacccgag aggcggcggc ggcgcagcgg aacggcagag cgggccggag gcggccgagg 120
cgcccggcgc aggcacccgt gcctcccctc tgccaggaac cttggggcct tgtgtgtgac 180
caggacctgg tggcccccgg gcggtggcag agcccctgtc ccaagctgct tcctgccggc 240
acctctgatc aagtgcctag agggatgtgt gtgccagccc tcggtccagt gcccgctcct 300
gagctgactc ctgctgggcc ccgacagctt gccgtgtttc ctgtgcctgt agctccctgg 360
ttggatagct gccgcccggg agaggtgacc cgggcgccct gctagggtga aggcccctgc 420
cctcggcccg ggatc atg aaa ggc ctc ggt gac agc cgc ccc cgc cac ctc 471

Met Lys Gly Leu Gly Asp Ser Arg Pro Arg His Leu
1               5                   10
tcc gac agc cta gac cca ccc cac gag ccc ctg ttt gca ggg acc gac   519
Ser Asp Ser Leu Asp Pro Pro His Glu Pro Leu Phe Ala Gly Thr Asp
        15                  20                  25
cgc aac ccc tac ctg ctg tcg ccc acg gag gcc ttc gcc cgc gag gcc   567
Arg Asn Pro Tyr Leu Leu Ser Pro Thr Glu Ala Phe Ala Arg Glu Ala
    30                  35                  40
cgc ttc ccc ggg cag aac acc ctg cca gga gat ggc ctc ttt ccc ctc   615
Arg Phe Pro Gly Gln Asn Thr Leu Pro Gly Asp Gly Leu Phe Pro Leu
45                  50                  55                  60
aac aac cag ctg ccc ccg ccc agc agc acc ttt ccc cgc atc cac tac   663
Asn Asn Gln Leu Pro Pro Pro Ser Ser Thr Phe Pro Arg Ile His Tyr
                65                  70                  75
aac tcc cac ttc gag gtg cca gag gag agc ccc ttc ccc agc cat gcc   711
Asn Ser His Phe Glu Val Pro Glu Glu Ser Pro Phe Pro Ser His Ala
            80                  85                  90
caa gcc acc aag atc aac cgg ctg ccc gcc aac ctc ctg gac cag ttt   759
Gln Ala Thr Lys Ile Asn Arg Leu Pro Ala Asn Leu Leu Asp Gln Phe
        95                  100                 105
gag aag cag ctg ccc atc cac cgt gat ggc ttc agc acc ctc caa ttt   807
Glu Lys Gln Leu Pro Ile His Arg Asp Gly Phe Ser Thr Leu Gln Phe
    110                 115                 120
ccc cgt ggc gag gcc aag gcc cgt ggt gag agc cct ggc cgc atc cgc   855
Pro Arg Gly Glu Ala Lys Ala Arg Gly Glu Ser Pro Gly Arg Ile Arg
125                 130                 135                 140

cac ctg gtc cac tca gtc cag cgg ctt ttc ttc acc aag gca ccc tca 903
His Leu Val His Ser Val Gln Arg Leu Phe Phe Thr Lys Ala Pro Ser
                145                 150                 155
ctg gag ggc aca gcg ggc aag gtc ggt ggc aat ggc agc aag aag ggt 951
Leu Glu Gly Thr Ala Gly Lys Val Gly Gly Asn Gly Ser Lys Lys Gly
            160                 165                 170
ggc atg gag gac ggc aag ggc cgg agg gcc aaa agc aag gag cgg gcc 999
Gly Met Glu Asp Gly Lys Gly Arg Arg Ala Lys Ser Lys Glu Arg Ala
        175                 180                 185
aag gct ggg gag ccc aaa cgg cgc agc cgc tcc aac atc tca ggc tgg 1047
Lys Ala Gly Glu Pro Lys Arg Arg Ser Arg Ser Asn Ile Ser Gly Trp
    190                 195                 200
tgg agc tcc gat gac aac ttg gac ggc gag gcc ggc gcc ttc cgc agc 1095
Trp Ser Ser Asp Asp Asn Leu Asp Gly Glu Ala Gly Ala Phe Arg Ser
205                 210                 215                 220
agt ggc cca gcc tct ggg ctg atg ata cta ggc cgc cag gca gaa cgc 1143
Ser Gly Pro Ala Ser Gly Leu Met Ile Leu Gly Arg Gln Ala Glu Arg
                225                 230                 235
agc cag cca cgc tac ttc atg cac gcc tac aac acc atc agt ggg cac 1191
Ser Gln Pro Arg Tyr Phe Met His Ala Tyr Asn Thr Ile Ser Gly His
            240                 245                 250
atg ctc aaa acc acc aag aac aac act act gag ctg act gcc cca cca 1239
Met Leu Lys Thr Thr Lys Asn Asn Thr Thr Glu Leu Thr Ala Pro Pro
        255                 260                 265
ccc ccg ccc gca ccc cca gcc acc tgc ccc agc ctt ggg gtg ggc act 1287
Pro Pro Pro Ala Pro Pro Ala Thr Cys Pro Ser Leu Gly Val Gly Thr
    270                 275                 280

gac acc aac tac gtc aaa cgg ggc tcc tgg tcc act ctg acc ctc agc 1335
Asp Thr Asn Tyr Val Lys Arg Gly Ser Trp Ser Thr Leu Thr Leu Ser
285                 290                 295                 300
cac gcc cac gag gtc tgc cag aag acc tca gcc acc ttg gat aag agc 1383
His Ala His Glu Val Cys Gln Lys Thr Ser Ala Thr Leu Asp Lys Ser
                305                 310                 315
ctg ctc aag tcc aaa tcc tgc cac cag ggt cta gcc tac cat tac ctg 1431
Leu Leu Lys Ser Lys Ser Cys His Gln Gly Leu Ala Tyr His Tyr Leu
            320                 325                 330
cag gtg ccc ggc ggc ggc ggc gag tgg agc acc acg ctg ctg tcc cca 1479
Gln Val Pro Gly Gly Gly Gly Glu Trp Ser Thr Thr Leu Leu Ser Pro
        335                 340                 345
cgc gag acg gat gcc gcg gcc gag ggc cct atc ccg tgc cgg cgc atg 1527
Arg Glu Thr Asp Ala Ala Ala Glu Gly Pro Ile Pro Cys Arg Arg Met
    350                 355                 360
cgc agc ggc agc tac atc aag gcc atg ggc gac gag gac agc gac gag 1575
Arg Ser Gly Ser Tyr Ile Lys Ala Met Gly Asp Glu Asp Ser Asp Glu
365                 370                 375                 380
tcc ggc ggc agc ccc aag ccc tca ccc aag acc gcg gcg cgg cgc cag 1623
Ser Gly Gly Ser Pro Lys Pro Ser Pro Lys Thr Ala Ala Arg Arg Gln
                385                 390                 395
agc tat ctg agg gcc acg cag cag tcg ctg gga gag cag agc aac ccc 1671
Ser Tyr Leu Arg Ala Thr Gln Gln Ser Leu Gly Glu Gln Ser Asn Pro
            400                 405                 410
cgc agg agt ctg gac cgc ctg gat tca gtg gac atg ctg ctg ccc tcc 1719
Arg Arg Ser Leu Asp Arg Leu Asp Ser Val Asp Met Leu Leu Pro Ser

        415                 420                 425

aag tgt ccg agc tgg gaa gag gac tac acc ccc gtc agc gac agc ctc 1767

Lys Cys Pro Ser Trp Glu Glu Asp Tyr Thr Pro Val Ser Asp Ser Leu

    430                 435                 440

aac gac tcc agc tgc atc agc cag att ttt gga cag gcc tcc ctg atc 1815

Asn Asp Ser Ser Cys Ile Ser Gln Ile Phe Gly Gln Ala Ser Leu Ile

445                 450                 455                 460

ccc cag ttg ttt ggc cat gag cag cag gta cgg gag gca gag ctg agt 1863

Pro Gln Leu Phe Gly His Glu Gln Gln Val Arg Glu Ala Glu Leu Ser

                465                 470                 475

gac cag tat gag gcg gcc tgc gag tca gcc tgc agt gaa gcg gag tcc 1911

Asp Gln Jyr Glu Ala Ala Cys Glu Ser Ala Cys Ser Glu Ala Glu Ser

480 485 490

aca gcg gca gag acg ctt gac ttg cca ctg ccc agc tac ttc cgc tcc 1959

Thr Ala Ala Glu Thr Leu Asp Leu Pro Leu Pro Ser Tyr Phe Arg Ser

495 500 505

cgc agc cac agc tac ctg cgt gcc atc cag gca ggc tgc tcg cag gag 2007

Arg Ser His Ser Tyr Leu Arg Ala Ile Gln Ala Gly Cys Ser Gln Glu

510 515 520

gag gac agt gtc tcc ctg cag tcc ctc tcc cca ccg ccc agt acc ggc 2055

Glu Asp Ser Val Ser Leu Gln Ser Leu Ser Pro Pro Pro Ser Thr Gly

525 530 535 540

agc ctc agc aat agt cgc acg ctt ccg agt tca tca tgc cta gtg gcg 2103

Ser Leu Ser Asn Ser Arg Thr Leu Pro Ser Ser Ser Cys Leu Val Ala

545 550 555

tat aag aag acc ccg cca ccg gtc cct cca cgc acc act tca aag ccg 2151

Tyr Lys Lys Thr Pro Pro Pro Val Pro Pro Arg Thr Thr Ser Lys Pro

560 565 570
ttc atc tca gtc aca gtc cag agc agt act gag tct gcc cag gac acc 2199
Phe Ile Ser Val Thr Val Gln Ser Ser Thr Glu Ser Ala Gln Asp Thr
575 580 585
tac ctg gac agc cag gac cac aag agc gag gtg act agc cag tcg ggc 2247
Tyr Leu Asp Ser Gln Asp His Lys Ser Glu Val Thr Ser Gln Ser Gly
590 595 600
ctg agc aac tcg tcg gac agc ctg gac agc agt acc cga ccg ccc agc 2295
Leu Ser Asn Ser Ser Asp Ser Leu Asp Ser Ser Thr Arg Pro Pro Ser
605 610 615 620
gtg aca cgg ggt gga gtc gcc cca gcc cct gag gcc cca gag cca ccc 2343
Val Thr Arg Gly Gly Val Ala Pro Ala Pro Glu Ala Pro Glu Pro Pro
625 630 635
cca aaa cat gca gct ctg aaa agt gaa caa ggg acg ctg acc agc tct 2391
Pro Lys His Ala Ala Leu Lys Ser Glu Gln Gly Thr Leu Thr Ser Ser
640 645 650
gag tcc cac ccc gag gcc gcc ccc aaa agg aaa ctg tca tcg ata gga 2439
Glu Ser His Pro Glu Ala Ala Pro Lys Arg Lys Leu Ser Ser Ile Gly
655 660 665
ata caa gag agg act aga agg aac ggt tcc cac ctc tcg gag gac aac 2487
Ile Gln Glu Arg Thr Arg Arg Asn Gly Ser His Leu Ser Glu Asp Asn
670 675 680
gga ccc aaa gcg atc gat gtg atg gca ccc tcc tca gaa agc agc gtc 2535
Gly Pro Lys Ala Ile Asp Val Met Ala Pro Ser Ser Glu Ser Ser Val
685 690 695 700
ccc tct cac agt atg tcc tcc cga cgg gac aca gac tcg gat acc cag 2583

Pro Ser His Ser Met Ser Ser Arg Arg Asp Thr Asp Ser Asp Thr Gln
705 710 715
gat gcc aat gac tca agc tgt aag tca tct gag agg agc ctc ccg gac 2631
Asp Ala Asn Asp Ser Ser Cys Lys Ser Ser Glu Arg Ser Leu Pro Asp
720 725 730
tgt acc cct cac ccc aac tcc atc agc atc gat gcc ggt ccc cgg cag 2679
Cys Thr Pro His Pro Asn Ser Ile Ser Ile Asp Ala Gly Pro Arg Gln
735 740 745
gcc ccc aag att gcc cag atc aag cgc aac ctc tcc tat gga gac aac 2727
Ala Pro Lys Ile Ala Gln Ile Lys Arg Asn Leu Ser Tyr Gly Asp Asn
750 755 760
agc gac cct gcc cta gag gcg tcc tcg ctg ccc cca ccc gac ccc tgg 2775
Ser Asp Pro Ala Leu Glu Ala Ser Ser Leu Pro Pro Pro Asp Pro Trp
765 770 775 780
ctc gag acc tcc tcc agc tcc cca gca gag ccg gca cag cca ggg gcc 2823
Leu Glu Thr Ser Ser Ser Ser Pro Ala Glu Pro Ala Gln Pro Gly Ala
785 790 795
tgc cgc cga gac ggc tac tgg ttc cta aag cta ctg cag gca gaa aca 2871
Cys Arg Arg Asp Gly Tyr Trp Phe Leu Lys Leu Leu Gln Ala Glu Thr
800 805 810
gag cgg ctg gaa ggc tgg tgc tgc cag atg gac aag gag acc aaa gag 2919
Glu Arg Leu Glu Gly Trp Cys Cys Gln Met Asp Lys Glu Thr Lys Glu
815 820 825
aac aac ctc tct gaa gaa gtc tta gga aaa gtc ctc agt gct gtg ggc 2967
Asn Asn Leu Ser Glu Glu Val Leu Gly Lys Val Leu Ser Ala Val Gly
830 835 840
agt gcc cag cta ctg atg tcc cag aaa ttc cag cag ttc cgg ggc ctc 3015

Ser Ala Gln Leu Leu Met Ser Gln Lys Phe Gln Gln Phe Arg Gly Leu
845                 850                 855                 860
tgt gag caa aac ttg aac cct gat gcc aac cca cgc ccc aca gcc cag     3063
Cys Glu Gln Asn Leu Asn Pro Asp Ala Asn Pro Arg Pro Thr Ala Gln
                865                 870                 875
gac ctg gca ggg ttc tgg gac ctg cta cag ctg tcc atc gag gat atc     3111
Asp Leu Ala Gly Phe Trp Asp Leu Leu Gln Leu Ser Ile Glu Asp Ile
            880                 885                 890
agc atg aag ttc gat gaa ctc tac cac ctc aag gcc aac agc tgg cag     3159
Ser Met Lys Phe Asp Glu Leu Tyr His Leu Lys Ala Asn Ser Trp Gln
        895                 900                 905
ctg gtg gag acc ccc gag aag agg aag gaa gag aag aaa cca ccc cct     3207
Leu Val Glu Thr Pro Glu Lys Arg Lys Glu Glu Lys Lys Pro Pro Pro
    910                 915                 920
ccg gtc cca aag aag cca gcc aaa tcc aag ccg gca gtg agc cgc gac     3255
Pro Val Pro Lys Lys Pro Ala Lys Ser Lys Pro Ala Val Ser Arg Asp
925                 930                 935                 940
aag gcc tca gac gcc agc gac aag cag cgc cag gag gcc cgc aag aga     3303
Lys Ala Ser Asp Ala Ser Asp Lys Gln Arg Gln Glu Ala Arg Lys Arg
                945                 950                 955
ctc ctg gcg gcc aag cgg gca gct tct gtg cgg cag aac tca gcc acc     3351
Leu Leu Ala Ala Lys Arg Ala Ala Ser Val Arg Gln Asn Ser Ala Thr
            960                 965                 970
gag agc gca gac agc atc gag att tat gtc ccg gag gcc cag acc agg     3399
Glu Ser Ala Asp Ser Ile Glu Ile Tyr Val Pro Glu Ala Gln Thr Arg
        975                 980                 985

ctc tgagaccatg caggaggaaa gaaacgattt taaatcatta aaaacacaaa 3452
Leu
aactaagtgc gaacggaaca gagttttctc aacctttgct atggttattc tgtctagaga 3512
ccctgagcca actttcaaat tgacgcatac aagggctcac aatttggctt ttttgggtcc 3572
ctcccagctt taggttatga agattttact cacaaaaaaa atcaacaaaa atcacgaaac 3632
tagaaaactt tttttttcct cttgctggcc gtggtggact agatagatgg acgtcggcaa 3692
ctcccggccc agcctccata ctgcggtctt tttactcgtt ctatctgatg agaactcaca 3752
ctagcttgtt tacaagatga cgacagtcca agggcagcct tgggcacctg ccatgtccct 3812
cctttcccca gctatccccg ctctgacctt gattttcatt cttatgtttt tctcttttcc 3872
cttcagagct cacacagtgg tcaccattgt ggcaagcggc tttctgggtc tcagccctct 3932
ctgcggttga gggcccagag gacagagaga tggacatgcg tcccctccct ccccccgcca 3992
agtgctcaca cacaacctca cgcgcacaca cacacacgca gatggaggcg cctcactggg 4052
aggtgccccg ccagccctgg gcagtgtcag gcaggactca ctcaccgctg agcagatgag 4112
agaagtttta gtcttggcgg gtggaaatga gacgaagcca cagttatcac actccagact 4172
cctgcccttt tattttctcc agccccttct tccttcagca aaatctagga ctcccgagtg 4232
gcttccaggg ggccgtcagt cctcagccgc gcctgtgtcc ggtgcccgag gggcgggcgg 4292
cggtgtctgt atgtatgtgt acatatgcac atagacctta gagtgtatag ttaacaaacg 4352
cccatctgct cacccatgcc cacccagcgc cgccgccgct ggctctcggg gcacctggca 4412
ggaggcgggt gtgtgaatag catatatttt tacatgtact atatctaggt gtgtgtacaa 4472
gtgtgtgtaa aaatatatac cttgtgtgta agcagccctt tttttttttg gtctccaccc 4532
ccctcccccc gccccgcact cctaagggcc catctgccca gcctctgagt tttctgttct 4592
attttttttt taaccccaat tatccttctc tctctcctgc ccccgcatcc cactcccagg 4652
gtgtcacgag ccctgagctg caatggcccg ggcctgcagg gcggggtagg ggagggcagg 4712
ggctcagccc cgaagccagc tcagtacctg aggggctgct ctatgctgtg tatgcgcctc 4772
tctggcatcc gagacatcct cttggctggc gcttgctgca ggggggaccc cccccccgtc 4832
cccaggtgaa ccaagggtct gctccggggc ccatttccag cttggccgcc gtctgtgacc 4892
ttgggcaagt cacttgacct ctgtgtgcct caacttcctc ctctgtaaaa cggggacagt 4952

ccctgcccct ccctacctca caggcatgtt gtgagaataa atgaggtaac gtgta 5007

<210> 38

<211> 989

<212> PRT

<213> Homo sapiens

<400> 38

Met Lys Gly Leu Gly Asp Ser Arg Pro Arg His Leu Ser Asp Ser Leu

1               5                   10                  15

Asp Pro Pro His Glu Pro Leu Phe Ala Gly Thr Asp Arg Asn Pro Tyr

            20                  25                  30

Leu Leu Ser Pro Thr Glu Ala Phe Ala Arg Glu Ala Arg Phe Pro Gly

        35                  40                  45

Gln Asn Thr Leu Pro Gly Asp Gly Leu Phe Pro Leu Asn Asn Gln Leu

    50                  55                  60

Pro Pro Pro Ser Ser Thr Phe Pro Arg Ile His Tyr Asn Ser His Phe

65                  70                  75                  80

Glu Val Pro Glu Glu Ser Pro Phe Pro Ser His Ala Gln Ala Thr Lys

                85                  90                  95

Ile Asn Arg Leu Pro Ala Asn Leu Leu Asp Gln Phe Glu Lys Gln Leu

            100                 105                 110

Pro Ile His Arg Asp Gly Phe Ser Thr Leu Gln Phe Pro Arg Gly Glu

        115                 120                 125

Ala Lys Ala Arg Gly Glu Ser Pro Gly Arg Ile Arg His Leu Val His

    130                 135                 140

Ser Val Gln Arg Leu Phe Phe Thr Lys Ala Pro Ser Leu Glu Gly Thr

145                 150                 155                 160

Ala Gly Lys Val Gly Gly Asn Gly Ser Lys Lys Gly Gly Met Glu Asp
                165                 170                 175
Gly Lys Gly Arg Arg Ala Lys Ser Lys Glu Arg Ala Lys Ala Gly Glu
            180                 185                 190
Pro Lys Arg Arg Ser Arg Ser Asn Ile Ser Gly Trp Trp Ser Ser Asp
        195                 200                 205
Asp Asn Leu Asp Gly Glu Ala Gly Ala Phe Arg Ser Ser Gly Pro Ala
    210                 215                 220
Ser Gly Leu Met Ile Leu Gly Arg Gln Ala Glu Arg Ser Gln Pro Arg
225                 230                 235                 240
Tyr Phe Met His Ala Tyr Asn Thr Ile Ser Gly His Met Leu Lys Thr
                245                 250                 255
Thr Lys Asn Asn Thr Thr Glu Leu Thr Ala Pro Pro Pro Pro Pro Ala
            260                 265                 270
Pro Pro Ala Thr Cys Pro Ser Leu Gly Val Gly Thr Asp Thr Asn Tyr
        275                 280                 285
Val Lys Arg Gly Ser Trp Ser Thr Leu Thr Leu Ser His Ala His Glu
    290                 295                 300
Val Cys Gln Lys Thr Ser Ala Thr Leu Asp Lys Ser Leu Leu Lys Ser
305                 310                 315                 320
Lys Ser Cys His Gln Gly Leu Ala Tyr His Tyr Leu Gln Val Pro Gly
                325                 330                 335
Gly Gly Gly Glu Trp Ser Thr Thr Leu Leu Ser Pro Arg Glu Thr Asp
            340                 345                 350
Ala Ala Ala Glu Gly Pro Ile Pro Cys Arg Arg Met Arg Ser Gly Ser
        355                 360                 365

Tyr Ile Lys Ala Met Gly Asp Glu Asp Ser Asp Glu Ser Gly Gly Ser
    370                 375                 380
Pro Lys Pro Ser Pro Lys Thr Ala Ala Arg Arg Gln Ser Tyr Leu Arg
385                 390                 395                 400
Ala Thr Gln Gln Ser Leu Gly Glu Gln Ser Asn Pro Arg Arg Ser Leu
                405                 410                 415
Asp Arg Leu Asp Ser Val Asp Met Leu Leu Pro Ser Lys Cys Pro Ser
            420                 425                 430
Trp Glu Glu Asp Tyr Thr Pro Val Ser Asp Ser Leu Asn Asp Ser Ser
        435                 440                 445
Cys Ile Ser Gln Ile Phe Gly Gln Ala Ser Leu Ile Pro Gln Leu Phe
    450                 455                 460
Gly His Glu Gln Gln Val Arg Glu Ala Glu Leu Ser Asp Gln Tyr Glu
465                 470                 475                 480
Ala Ala Cys Glu Ser Ala Cys Ser Glu Ala Glu Ser Thr Ala Ala Glu
                485                 490                 495
Thr Leu Asp Leu Pro Leu Pro Ser Tyr Phe Arg Ser Arg Ser His Ser
            500                 505                 510
Tyr Leu Arg Ala Ile Gln Ala Gly Cys Ser Gln Glu Glu Asp Ser Val
        515                 520                 525
Ser Leu Gln Ser Leu Ser Pro Pro Pro Ser Thr Gly Ser Leu Ser Asn
    530                 535                 540
Ser Arg Thr Leu Pro Ser Ser Ser Cys Leu Val Ala Tyr Lys Lys Thr
545                 550                 555                 560
Pro Pro Pro Val Pro Pro Arg Thr Thr Ser Lys Pro Phe Ile Ser Val
                565                 570                 575
Thr Val Gln Ser Ser Thr Glu Ser Ala Gln Asp Thr Tyr Leu Asp Ser

            580                 585                 590
Gln Asp His Lys Ser Glu Val Thr Ser Gln Ser Gly Leu Ser Asn Ser
        595                 600                 605
Ser Asp Ser Leu Asp Ser Ser Thr Arg Pro Pro Ser Val Thr Arg Gly
    610                 615                 620
Gly Val Ala Pro Ala Pro Glu Ala Pro Glu Pro Pro Pro Lys His Ala
625                 630                 635                 640
Ala Leu Lys Ser Glu Gln Gly Thr Leu Thr Ser Ser Glu Ser His Pro
                645                 650                 655
Glu Ala Ala Pro Lys Arg Lys Leu Ser Ser Ile Gly Ile Gln Glu Arg
            660                 665                 670
Thr Arg Arg Asn Gly Ser His Leu Ser Glu Asp Asn Gly Pro Lys Ala
        675                 680                 685
Ile Asp Val Met Ala Pro Ser Ser Glu Ser Ser Val Pro Ser His Ser
    690                 695                 700
Met Ser Ser Arg Arg Asp Thr Asp Ser Asp Thr Gln Asp Ala Asn Asp
705                 710                 715                 720
Ser Ser Cys Lys Ser Ser Glu Arg Ser Leu Pro Asp Cys Thr Pro His
                725                 730                 735
Pro Asn Ser Ile Ser Ile Asp Ala Gly Pro Arg Gln Ala Pro Lys Ile
            740                 745                 750
Ala Gln Ile Lys Arg Asn Leu Ser Tyr Gly Asp Asn Ser Asp Pro Ala
        755                 760                 765
Leu Glu Ala Ser Ser Leu Pro Pro Pro Asp Pro Trp Leu Glu Thr Ser
    770                 775                 780
Ser Ser Ser Pro Ala Glu Pro Ala Gln Pro Gly Ala Cys Arg Arg Asp

785                 790                 795                 800

Gly Tyr Trp Phe Leu Lys Leu Leu Gln Ala Glu Thr Glu Arg Leu Glu

                805                 810                 815

Gly Trp Cys Cys Gln Met Asp Lys Glu Thr Lys Glu Asn Asn Leu Ser

            820                 825                 830

Glu Glu Val Leu Gly Lys Val Leu Ser Ala Val Gly Ser Ala Gln Leu

        835                 840                 845

Leu Met Ser Gln Lys Phe Gln Gln Phe Arg Gly Leu Cys Glu Gln Asn

    850                 855                 860

Leu Asn Pro Asp Ala Asn Pro Arg Pro Thr Ala Gln Asp Leu Ala Gly

865                 870                 875                 880

Phe Trp Asp Leu Leu Gln Leu Ser Ile Glu Asp Ile Ser Met Lys Phe

                885                 890                 895

Asp Glu Leu Tyr His Leu Lys Ala Asn Ser Trp Gln Leu Val Glu Thr

            900                 905                 910

Pro Glu Lys Arg Lys Glu Glu Lys Lys Pro Pro Pro Pro Val Pro Lys

        915                 920                 925

Lys Pro Ala Lys Ser Lys Pro Ala Val Ser Arg Asp Lys Ala Ser Asp

    930                 935                 940

Ala Ser Asp Lys Gln Arg Gln Glu Ala Arg Lys Arg Leu Leu Ala Ala

945                 950                 955                 960

Lys Arg Ala Ala Ser Val Arg Gln Asn Ser Ala Thr Glu Ser Ala Asp

                965                 970                 975

Ser Ile Glu Ile Tyr Val Pro Glu Ala Gln Thr Arg Leu

            980                 985

<210> 39

<211> 2522

<212> DNA

<213> Homo sapiens

<220>

<221> CDS

<222> (1)..(1545)

<400> 39

act gct ctc agt gag aag cgc acg ctg gag ggc gag ctg cat gat ctg     48

Thr Ala Leu Ser Glu Lys Arg Thr Leu Glu Gly Glu Leu His Asp Leu

1               5                   10                  15

cgg ggc cag gtg gcc aag ctt gag gca gcc cta ggt gag gcc aag aag     96

Arg Gly Gln Val Ala Lys Leu Glu Ala Ala Leu Gly Glu Ala Lys Lys

            20                  25                  30

caa ctt cag gat gag atg ctg cgg cgg gtg gat gct gag aac agg ctg     144

Gln Leu Gln Asp Glu Met Leu Arg Arg Val Asp Ala Glu Asn Arg Leu

        35                  40                  45

cag acc atg aag gag gaa ctg gac ttc cag aag aac atc tac agt gag     192

Gln Thr Met Lys Glu Glu Leu Asp Phe Gln Lys Asn Ile Tyr Ser Glu

    50                  55                  60

gag ctg cgt gag acc aag cgc cgt cat gag acc cga ctg gtg gag att     240

Glu Leu Arg Glu Thr Lys Arg Arg His Glu Thr Arg Leu Val Glu Ile

65                  70                  75                  80

gac aat ggg aag cag cgt gag ttt gag agc cgg ctg gcg gat gcg ctg     288

Asp Asn Gly Lys Gln Arg Glu Phe Glu Ser Arg Leu Ala Asp Ala Leu

                85                  90                  95

cag gaa ctg cgg gcc cag cat gag gac cag gtg gag cag tat aag aag     336

Gln Glu Leu Arg Ala Gln His Glu Asp Gln Val Glu Gln Tyr Lys Lys

            100                 105                 110
gag ctg gag aag act tat tct gcc aag ctg gac aat gcc agg cag tct 384
Glu Leu Glu Lys Thr Tyr Ser Ala Lys Leu Asp Asn Ala Arg Gln Ser
        115                 120                 125
gct gag agg aac agc aac ctg gtg ggg gct gcc cac gag gag ctg cag 432
Ala Glu Arg Asn Ser Asn Leu Val Gly Ala Ala His Glu Glu Leu Gln
    130                 135                 140
cag tcg cgc atc cgc atc gac agc ctc tct gcc cag ctc agc cag ctc 480
Gln Ser Arg Ile Arg Ile Asp Ser Leu Ser Ala Gln Leu Ser Gln Leu
145                 150                 155                 160
cag aag cag ctg gca gcc aag gag gcg aag ctt cga gac ctg gag gac 528
Gln Lys Gln Leu Ala Ala Lys Glu Ala Lys Leu Arg Asp Leu Glu Asp
                165                 170                 175
tca ctg gcc cgt gag cgg gac acc agc cgg cgg ctg ctg gcg gaa aag 576
Ser Leu Ala Arg Glu Arg Asp Thr Ser Arg Arg Leu Leu Ala Glu Lys
            180                 185                 190
gag cgg gag atg gcc gag atg cgg gca agg atg cag cag cag ctg gac 624
Glu Arg Glu Met Ala Glu Met Arg Ala Arg Met Gln Gln Gln Leu Asp
        195                 200                 205
gag tac cag gag ctt ctg gac atc aag ctg gcc ctg gac atg gag atc 672
Glu Tyr Gln Glu Leu Leu Asp Ile Lys Leu Ala Leu Asp Met Glu Ile
    210                 215                 220
cac gcc tac cgc aag ctc ttg gag ggc gag gag gag agg cta cgc ctg 720
His Ala Tyr Arg Lys Leu Leu Glu Gly Glu Glu Glu Arg Leu Arg Leu
225                 230                 235                 240
tcc ccc agc cct acc tcg cag cgc agc cgt ggc cgt gct tcc tct cac 768

Ser Pro Ser Pro Thr Ser Gln Arg Ser Arg Gly Arg Ala Ser Ser His
                245                 250                 255
tca tcc cag aca cag ggt ggg ggc agc gtc acc aaa aag cgc aaa ctg 816
Ser Ser Gln Thr Gln Gly Gly Gly Ser Val Thr Lys Lys Arg Lys Leu
            260                 265                 270
gag tcc act gag agc cgc agc agc ttc tca cag cac gca cgc act agc 864
Glu Ser Thr Glu Ser Arg Ser Ser Phe Ser Gln His Ala Arg Thr Ser
        275                 280                 285
ggg cgc gtg gcc gtg gag gag gtg gat gag gag ggc aag ttt gtc cgg 912
Gly Arg Val Ala Val Glu Glu Val Asp Glu Glu Gly Lys Phe Val Arg
    290                 295                 300
ctg cgc aac aag tcc aat gag gac cag tcc atg ggc aat tgg cag atc 960
Leu Arg Asn Lys Ser Asn Glu Asp Gln Ser Met Gly Asn Trp Gln Ile
305                 310                 315                 320
aag cgc cag aat gga gat gat ccc ttg ctg act tac cgg ttc cca cca 1008
Lys Arg Gln Asn Gly Asp Asp Pro Leu Leu Thr Tyr Arg Phe Pro Pro
                325                 330                 335
aag ttc acc ctg aag gct ggg cag gtg gtg acg atc tgg gct gca gga 1056
Lys Phe Thr Leu Lys Ala Gly Gln Val Val Thr Ile Trp Ala Ala Gly
            340                 345                 350
gct ggg gcc acc cac agc ccc cct acc gac ctg gtg tgg aag gca cag 1104
Ala Gly Ala Thr His Ser Pro Pro Thr Asp Leu Val Trp Lys Ala Gln
        355                 360                 365
aac acc tgg ggc tgc ggg aac agc ctg cgt acg gct ctc atc aac tcc 1152
Asn Thr Trp Gly Cys Gly Asn Ser Leu Arg Thr Ala Leu Ile Asn Ser
    370                 375                 380
act ggg gaa gaa gtg gcc atg cgc aag ctg gtg cgc tca gtg act gtg 1200

Thr Gly Glu Glu Val Ala Met Arg Lys Leu Val Arg Ser Val Thr Val
385                 390                 395                 400
gtt gag gac gac gag gat gag gat gga gat gac ctg ctc cat cac cac    1248
Val Glu Asp Asp Glu Asp Glu Asp Gly Asp Asp Leu Leu His His His
                405                 410                 415
cac ggc tcc cac tgc agc agc tcg ggg gac ccc gct gag tac aac ctg    1296
His Gly Ser His Cys Ser Ser Ser Gly Asp Pro Ala Glu Tyr Asn Leu
            420                 425                 430
cgc tcg cgc acc gtg ctg tgc ggg acc tgc ggg cag cct gcc gac aag    1344
Arg Ser Arg Thr Val Leu Cys Gly Thr Cys Gly Gln Pro Ala Asp Lys
        435                 440                 445
gca tct gcc agc ggc tca gga gcc cag gtg ggc gga ccc atc tcc tct    1392
Ala Ser Ala Ser Gly Ser Gly Ala Gln Val Gly Gly Pro Ile Ser Ser
    450                 455                 460
ggc tct tct gcc tcc agt gtc acg gtc act cgc agc tac cgc agt gtg    1440
Gly Ser Ser Ala Ser Ser Val Thr Val Thr Arg Ser Tyr Arg Ser Val
465                 470                 475                 480
ggg ggc agt ggg ggt ggc agc ttc ggg gac aat ctg gtc acc cgc tcc    1488
Gly Gly Ser Gly Gly Gly Ser Phe Gly Asp Asn Leu Val Thr Arg Ser
                485                 490                 495
tac ctc ctg ggc aac tcc agc ccc cga acc cag agc ccc cag aac tgc    1536
Tyr Leu Leu Gly Asn Ser Ser Pro Arg Thr Gln Ser Pro Gln Asn Cys
            500                 505                 510
agc atc atg taatctggga cctgccaggc aggggtgggg gtggaggctt            1585
Ser Ile Met
        515

cctgcgtcct cctcacctca tgcccacccc ctgccctgca cgtcatggga gggggcttga 1645

agccaaagaa aaataaccct ttggtttttt tcttctgtat ttttttttct aagagaagtt 1705

attttctaca gtggttttat actgaaggaa aaacacaagc aaaaaaaaaa aaaaagcatc 1765

tatctcatct atctcaatcc taatttctcc tcccttcctt ttccctgctt ccaggaaact 1825

ccacatctgc cttaaaacca aagagggctt cctctagaag ccaagggaaa ggggtgcttt 1885

tatagaggct agcttctgct tttctgccct ggctgctgcc ccaccccggg gaccctgtga 1945

catggtgcct gagaggcagg catagaggct tctccgccag cctcctctgg acggcaggct 2005

cactgccagg ccagcctccg agagggagag agagagagag aggacagctt gagccgggcc 2065

cctggcttgg cctgctgtga ttccactaca cctggctgag gttcctctgc ctgccccgcc 2125

cccagtcccc acccctgccc ccagccccgg ggtgagtcca ttctcccagg taccagctgc 2185

gcttgctttt ctgtatttta tttagacaag agatgggaat gaggtgggag gtggaagaag 2245

ggagaagaaa ggtgagtttg agctgccttc cctagcttta gaccctgggt gggctctgtg 2305

cagtcactgg aggttgaagc caagtggggt gctgggagga gggagaggga ggtcactgga 2365

aaggggagag cctgctgcac ccaccgtgga ggaggaaggc aagagggggt ggaggggtgt 2425

ggcagttggt tttggcaaac gcttaaagag cccttgcctc cccatttccc atctgcaccc 2485

cttctctcct ccccaaatca atacactagt tgtttct 2522

<210> 40

<211> 515

<212> PRT

<213> Homo sapiens

<400> 40

Thr Ala Leu Ser Glu Lys Arg Thr Leu Glu Gly Glu Leu His Asp Leu

1               5                   10                  15

Arg Gly Gln Val Ala Lys Leu Glu Ala Ala Leu Gly Glu Ala Lys Lys

            20                  25                  30

Gln Leu Gln Asp Glu Met Leu Arg Arg Val Asp Ala Glu Asn Arg Leu

        35                  40                  45
Gln Thr Met Lys Glu Glu Leu Asp Phe Gln Lys Asn Ile Tyr Ser Glu
    50                  55                  60
Glu Leu Arg Glu Thr Lys Arg Arg His Glu Thr Arg Leu Val Glu Ile
65                  70                  75                  80
Asp Asn Gly Lys Gln Arg Glu Phe Glu Ser Arg Leu Ala Asp Ala Leu
                85                  90                  95
Gln Glu Leu Arg Ala Gln His Glu Asp Gln Val Glu Gln Tyr Lys Lys
            100                 105                 110
Glu Leu Glu Lys Thr Tyr Ser Ala Lys Leu Asp Asn Ala Arg Gln Ser
        115                 120                 125
Ala Glu Arg Asn Ser Asn Leu Val Gly Ala Ala His Glu Glu Leu Gln
    130                 135                 140
Gln Ser Arg Ile Arg Ile Asp Ser Leu Ser Ala Gln Leu Ser Gln Leu
145                 150                 155                 160
Gln Lys Gln Leu Ala Ala Lys Glu Ala Lys Leu Arg Asp Leu Glu Asp
                165                 170                 175
Ser Leu Ala Arg Glu Arg Asp Thr Ser Arg Arg Leu Leu Ala Glu Lys
            180                 185                 190
Glu Arg Glu Met Ala Glu Met Arg Ala Arg Met Gln Gln Gln Leu Asp
        195                 200                 205
Glu Tyr Gln Glu Leu Leu Asp Ile Lys Leu Ala Leu Asp Met Glu Ile
    210                 215                 220
His Ala Tyr Arg Lys Leu Leu Glu Gly Glu Glu Glu Arg Leu Arg Leu
225                 230                 235                 240
Ser Pro Ser Pro Thr Ser Gln Arg Ser Arg Gly Arg Ala Ser Ser His
                245                 250                 255

Ser Ser Gln Thr Gln Gly Gly Gly Ser Val Thr Lys Lys Arg Lys Leu
            260                 265                 270
Glu Ser Thr Glu Ser Arg Ser Ser Phe Ser Gln His Ala Arg Thr Ser
        275                 280                 285
Gly Arg Val Ala Val Glu Glu Val Asp Glu Glu Gly Lys Phe Val Arg
    290                 295                 300
Leu Arg Asn Lys Ser Asn Glu Asp Gln Ser Met Gly Asn Trp Gln Ile
305                 310                 315                 320
Lys Arg Gln Asn Gly Asp Asp Pro Leu Leu Thr Tyr Arg Phe Pro Pro
                325                 330                 335
Lys Phe Thr Leu Lys Ala Gly Gln Val Val Thr Ile Trp Ala Ala Gly
            340                 345                 350
Ala Gly Ala Thr His Ser Pro Pro Thr Asp Leu Val Trp Lys Ala Gln
        355                 360                 365
Asn Thr Trp Gly Cys Gly Asn Ser Leu Arg Thr Ala Leu Ile Asn Ser
    370                 375                 380
Thr Gly Glu Glu Val Ala Met Arg Lys Leu Val Arg Ser Val Thr Val
385                 390                 395                 400
Val Glu Asp Asp Glu Asp Glu Asp Gly Asp Asp Leu Leu His His His
                405                 410                 415
His Gly Ser His Cys Ser Ser Ser Gly Asp Pro Ala Glu Tyr Asn Leu
            420                 425                 430
Arg Ser Arg Thr Val Leu Cys Gly Thr Cys Gly Gln Pro Ala Asp Lys
        435                 440                 445
Ala Ser Ala Ser Gly Ser Gly Ala Gln Val Gly Gly Pro Ile Ser Ser
    450                 455                 460

Gly Ser Ser Ala Ser Ser Val Thr Val Thr Arg Ser Tyr Arg Ser Val
465                 470                 475                 480
Gly Gly Ser Gly Gly Gly Ser Phe Gly Asp Asn Leu Val Thr Arg Ser
                485                 490                 495
Tyr Leu Leu Gly Asn Ser Ser Pro Arg Thr Gln Ser Pro Gln Asn Cys
            500                 505                 510
Ser Ile Met
        515
<210> 41
<211> 3474
<212> DNA
<213> Homo sapiens
<220>
<221> CDS
<222> (78)..(2813)
<400> 41
   gcgcgccggc ggctcgggca gaggggcggg agctgaggcg ggagcggaca ggctggtggg 60
   cgagcgagag gcgcgga atg gtg gac tac cac gcg gcg aac cag tcg tac    110
   cag tac ggc ccc agc agc gcg gca atg gct tgg cgg cgg ggg agc atg    158
   ggc gac tac atg gcc cag gag gac gac tgg gac cgg gac ctg ctg ctg    206
   gac ccg gcc tgg gag aag cag cag cgc aag acc ttc acg gca tgg agc 254
   aac tcc cac ctg cgg aag gca ggc aca cag atc gag aac att gat gag 302
   gac ttc cga gac ggg ctc aag ctc atg ctg ctc ctg gag gtc ata tca 350
   ggg gag cgg tta cct aag ccg gag cgg ggg aag atg aga gtg cac aaa 398
   atc aac aat gtg aac aaa gcg ctg gac ttt att gcc agc aaa ggg atc 446
   aag ctg gac ttc cat cgg gca gaa gag att gtg gac ggc aac gca aag 494
   atg acc ctg gga atg atc tgg acc atc atc ctt agg ttc gcc atc cag 542
   gac atc tcc gtg gaa gag acc tcg gcc aag gaa ggg ctc ctt ctc tgg 590
   tgc cag aga aag aca gcc cca tat aag aac gtc aat gtg cag aac ttc 638
   cac atc agc tgg aag gat ggt ctt gcc ttc aat gcc ctg atc cac cgg 686
   cac aga cca gag ctg att gag tat gac aag ctg agg aag gac gac cct 734
   gtc acc aac ctg aac aat gcc ttc gaa gtg gct gag aaa tac ctc gac 782
   atc ccc aag atg ctg gat gca gag gac atc gtg aac acg gcc cgg ccc 830
   gac gag aag gcc ata atg acc tat gtg tcc agc ttc tac cat gcc ttt 878
   tca gga gcg cag aag gct gaa act gaa act gcc gcc aac cgg atc tgt 926
   aag gtg ctg gct gtc aac caa gag aac tgc agc acc tcg atg gag gac 974
   tac gag aag ctg gcc agc gac ctc ctg gag tgg atc cgg cgc acc atc 1022
   ccc tgg ctg gag gac cgt gtg ccc caa aag act atc cag gag atg cag 1070
   cag aag ctg gag gac ttc cgc gac tac cgg cgt gtg cac aag ccg ccc 1118
   aag gtg cag gag aag tgc cag ctg gag atc aac ttc aac agc gtg cag 1166
   acc aag ctg cgc ctc agc aac cgg ccc gcc ttc atg ccc tcc gag ggc 1214
   aag atg gtc tcg gac atc aac aat ggc tgg cag cac ttg gag cag gct 1262
   gag aag ggc tac gag gag tgg ctg ctg aat gag att cgc agg ctg gag 1310
   cgg ctc gac cac ctg gca gag aag ttc cgg cag aaa gcc tcc atc cac 1358
   gag gcc tgg act gac ggg aag gaa gcc atg ctg aag cac cgg gac tac 1406
   gag acg gcc aca cta tcg gac atc aaa gcc ctc att cgc aag cac gag 1454
   gcc ttc gag agc gac ctg gct gcg cac cag gac cgc gtg gag cag atc 1502
   gcc gcc tcc gcc cag gag ctc aac gag ctg gat tac tac gac tcc cac 1550
   aat gtc aac acc cgg tgc cag aag atc tgt gac cag tgg gac gcc ctc 1598
   ggc tct ctg aca cat agt cgc agg gaa gcc ctg gag aaa aca gag aag 1646
   cag ctg gag gcc atc atc gac cag ctg cac ctg gaa tac gcc aag ccc 1694
   gcg gcc ccc ttc aac aac tgg atg gag agc gcc atg gag gac ctc cag 1742
   gac atg ttc atc gtc cat acc atc gag gag att gag ggc ctg atc tca 1790
   gcc cat gac cag ttc aag tcc acc ctg ccg gac gcc gat agg gag cgc 1838
   gag gcc atc ctg cat cca caa gga ggc cag agg atc gct gag agc aac 1886
   cac atc aag ctg tcg ggc agc aac ccc tac acc acc gtc acc ccg caa 1934
   atc atc aac tcc aag tgg gag aag gtg cag cag ctg gtg cca aaa cgg 1982
   gac cat gcc ctc ctg gag gag cag agc aag cag cag cag tcc aac gag 2030
   cac ctg cgc cgc cag ttc gcc agc cag gcc aat gtt gtg ggg ccc tgg 2078
   atc cag acc aag atg gag gag atc gcg atc tcc att gag atg aac ggg 2126
   acc ctg gag gac cag ctg agc cac ctg aag cag tat gaa cgc agc atc 2174
   gtg gac tac aag ccc aac ctg gac ctg ctg gag cag cag cac cag ctc 2222
   atc cag gag gcc ctc atc ttc gac aac aag cac acc aac tat acc atg 2270
   gag cac atc cgc gtg ggc tgg gag cag ctg ctc acc acc att gcc cgc 2318
   acc atc aac gag gtg gag aac cag atc ctt acc cgc gac gcc aag ggc 2366
   atc agc cag gag cag atg cag gag ttc cgg gcg tcc ttc aac cac ttc 2414
   gac aag gat cat ggc ggg gcg ctg ggg cga gga gtt caa ggc ctg cct 2462
   cat cag cct ggg cta cga cgt gga gaa cga ccg gca ggt gag gcc gag 2510
   ttc aac cgc atc atg agc ctg gtc gac ccc aac cat agc ggc ctt gtt 2558
   acc ttc caa gcc ttc atc gac ttc atg tcg cgg gag acc acc gac acc 2606
   gac acg gct gac cag gta atc act tcc ttc aag gtc cta gca ggg gac 2654
   aag aac ttc atc aca gct gag gag ctg cgg aga gag ctg ccc ccc gac 2702
   cag gcc gag tac tgc atc gcc cgc atg gcg cca tac cag ggc cct gac 2750
   ggc gtg cgc ggt gcc ctc gac tac aag tcc ttc tcc acg gcc ttg tat 2798
   ggc gag agc gac ctg tgaggcccca gagacctgac ccaacacccc cgacgcctcc 2853
   aggagcctgg cagccccaca gtcccattcc tccactctgt atctatgcaa agcactctct 2913
   ctgcagtctc cggggtgggt gggtgggcag ggagggcctg gggcaggctc tctcctctct 2973
   ctctttgtgg gttggccagg aggttccccc gaccaggttg gggagacttg gggccagcgc 3033
   ttctggtctg gtaaatatgt atgatgtgtt gtgctttttt aaccaaggag gggccagtgg 3093
   attcccacag cacaaccggt cccttccatg ccctgggatg cctcaccaca cccaggtctc 3153
   ttcctttgct ctgaggtccc ttcaaggcct ccccaatcca ggccaaagcc ccatgtgcct 3213
   tgtccaggga actgcctggg ccatgcgagg ggccagcaga gggcgccacc acctgacggc 3273
   tgggacccac ccagcccctc tcccctctct gctccagact cacttgccat tgccaggaga 3333
   tggccccaac aagcaccccg cttttgcagc agaggagctg agttggcaga ccgggccccc 3393
   ctgaaccgca ccccatccca ccagccccgg ccttgctttg tctggcctca cgtgtctcag 3453
   attttctaag aaccaaaaaa a 3474
<210> 42
<211> 912
<212> PRT
<213> Homo sapiens
<400> 42

<210> 43
<211> 8971
<212> DNA
<213> Homo sapiens
<220>
<221> CDS
<222> (110)..(8224)
<400> 43
agcccaaacc gcggccctag ccccggccgc acccccagcc cgcgccagca tgatgaacaa 60
cagcggctac tcagacgccg gcctcggcct gggcgatgag acagacgag atg ccg tcc 118
Met Pro Ser
1
acg gag aag gac ctg gcg gag gac gcg ccg tgg aag aag atc cag cag 166
Thr Glu Lys Asp Leu Ala Glu Asp Ala Pro Trp Lys Lys Ile Gln Gln
    5                   10                  15
aac aca ttc acg cgc tgg tgc aat gag cac ctc aag tgc gtg ggc aag 214
Asn Thr Phe Thr Arg Trp Cys Asn Glu His Leu Lys Cys Val Gly Lys
20                  25                  30                  35
cgc ctg acc gac ctg cag cgc gac ctc agc gac ggg ctc cgg ctc atc 262
Arg Leu Thr Asp Leu Gln Arg Asp Leu Ser Asp Gly Leu Arg Leu Ile
                40                  45                  50
gcg ctg ctc gag gtg ctc agc cag aag cgc atg tac cgc aag ttc cat 310

Ala Leu Leu Glu Val Leu Ser Gln Lys Arg Met Tyr Arg Lys Phe His
            55                  60                  65
ccg cgc ccc aac ttc cgc caa atg aag ctg gag aac gtg tcc gtg gcc 358
Pro Arg Pro Asn Phe Arg Gln Met Lys Leu Glu Asn Val Ser Val Ala
        70                  75                  80
ctc gag ttc ctc gag cgc gag cac atc aag ctc gtg tcc ata gac agc 406
Leu Glu Phe Leu Glu Arg Glu His Ile Lys Leu Val Ser Ile Asp Ser
    85                  90                  95
aag gcc atc gtg gat ggg aac ctg aag ctg atc ctg ggc ctg atc tgg 454
Lys Ala Ile Val Asp Gly Asn Leu Lys Leu Ile Leu Gly Leu Ile Trp
100                 105                 110                 115
acg ctg atc ctg cac tac tcc atc tcc atg ccc atg tgg gag gat gaa 502
Thr Leu Ile Leu His Tyr Ser Ile Ser Met Pro Met Trp Glu Asp Glu
                120                 125                 130
gat gat gag gat gcc cgc aaa cag acg ccc aag cag cgg ctg ctt ggc 550
Asp Asp Glu Asp Ala Arg Lys Gln Thr Pro Lys Gln Arg Leu Leu Gly
            135                 140                 145
tgg atc cag aac aag gtg ccc cag ctg ccc atc acc aac ttc aac cgt 598
Trp Ile Gln Asn Lys Val Pro Gln Leu Pro Ile Thr Asn Phe Asn Arg
        150                 155                 160
gac tgg cag gac ggc aaa gct ctg ggc gcc ctg gtg gac aac tgc gcc 646
Asp Trp Gln Asp Gly Lys Ala Leu Gly Ala Leu Val Asp Asn Cys Ala
    165                 170                 175
ccc ggt ctc tgc ccc gac tgg gag gcc tgg gat ccc aac cag ccc gtg 694
Pro Gly Leu Cys Pro Asp Trp Glu Ala Trp Asp Pro Asn Gln Pro Val
180                 185                 190                 195
gag aac tcc cgg gag gcc atg cag cag gcc gac gac tgg ctt ggg gtg 742

Glu Asn Ser Arg Glu Ala Met Gln Gln Ala Asp Asp Trp Leu Gly Val
                200                 205                 210
ccc cag gtc att gcc cct gag gag att gtg gac ccc aac gtg gat gag 790
Pro Gln Val Ile Ala Pro Glu Glu Ile Val Asp Pro Asn Val Asp Glu
            215                 220                 225
cat tct gtt atg acc tac ctg tcc cag ttc ccc aag gcc aag ctc aaa 838
His Ser Val Met Thr Tyr Leu Ser Gln Phe Pro Lys Ala Lys Leu Lys
        230                 235                 240
cct ggt gcc cct gtt cga tcc aag cag ctg aac ccc aag aaa gcc atc 886
Pro Gly Ala Pro Val Arg Ser Lys Gln Leu Asn Pro Lys Lys Ala Ile
    245                 250                 255
gcc tat ggg cct ggc atc gag cca cag ggc aac acc gtg ctg cag cct 934
Ala Tyr Gly Pro Gly Ile Glu Pro Gln Gly Asn Thr Val Leu Gln Pro
260                 265                 270                 275
gcc cac ttc acc gtg cag acg gtg gac gcg ggc gtg ggc gag gtg ctg 982
Ala His Phe Thr Val Gln Thr Val Asp Ala Gly Val Gly Glu Val Leu
                280                 285                 290
gtc tac atc gag gac cct gaa ggc cac acc gag gag gct aag gtg gtt 1030
Val Tyr Ile Glu Asp Pro Glu Gly His Thr Glu Glu Ala Lys Val Val
            295                 300                 305
ccc aac aat gac aag gat cgc acc tat gct gtc tcc tat gtg ccc aag 1078
Pro Asn Asn Asp Lys Asp Arg Thr Tyr Ala Val Ser Tyr Val Pro Lys
        310                 315                 320
gtc gct ggg tta cac aag gtg acc gtg ctc ttt gct ggc cag aac att 1126
Val Ala Gly Leu His Lys Val Thr Val Leu Phe Ala Gly Gln Asn Ile
    325                 330                 335

gaa cgc agt ccc ttt gag gtg aac gtg ggc atg gcc ctg gga gat gcc 1174
Glu Arg Ser Pro Phe Glu Val Asn Val Gly Met Ala Leu Gly Asp Ala
340                 345                 350                 355
aac aag gtg tca gcc cgt ggc cct ggc ctg gaa cct gtg ggc aat gtg 1222
Asn Lys Val Ser Ala Arg Gly Pro Gly Leu Glu Pro Val Gly Asn Val
                360                 365                 370
gcc aac aaa ccc acc tac ttt gac atc tac act gcg ggg gcc ggc act 1270
Ala Asn Lys Pro Thr Tyr Phe Asp Ile Tyr Thr Ala Gly Ala Gly Thr
            375                 380                 385
ggc gat gtt gct gtg gtg atc gtg gac cca cag ggc cgg cgg gac aca 1318
Gly Asp Val Ala Val Val Ile Val Asp Pro Gln Gly Arg Arg Asp Thr
        390                 395                 400
gtg gag gtg gcc ctg gag gac aag ggt gac agc acg ttc cgc tgc aca 1366
Val Glu Val Ala Leu Glu Asp Lys Gly Asp Ser Thr Phe Arg Cys Thr
    405                 410                 415
tac aga cct gcc atg gag ggg cca cat acc gtg cat gtg gcc ttt gcg 1414
Tyr Arg Pro Ala Met Glu Gly Pro His Thr Val His Val Ala Phe Ala
420                 425                 430                 435
ggt gcc ccc atc acc cgc agt ccc ttc cct gtc cat gtg tcg gaa gcc 1462
Gly Ala Pro Ile Thr Arg Ser Pro Phe Pro Val His Val Ser Glu Ala
                440                 445                 450
tgt aac ccc aac gcc tgc cgc gcc tct ggg cga ggc ctg cag ccc aag 1510
Cys Asn Pro Asn Ala Cys Arg Ala Ser Gly Arg Gly Leu Gln Pro Lys
            455                 460                 465
ggt gtt cgc gtg aaa gag gtg gct gac ttc aag gtg ttt acc aag ggt 1558
Gly Val Arg Val Lys Glu Val Ala Asp Phe Lys Val Phe Thr Lys Gly
        470                 475                 480

gcc ggc agc ggg gag ctc aag gtc acg gtc aag ggg cca aag ggc aca 1606
Ala Gly Ser Gly Glu Leu Lys Val Thr Val Lys Gly Pro Lys Gly Thr
    485                 490                 495
gag gag cca gtg aag gtg cgg gag gct ggg gat ggt gtg ttc gag tgc 1654
Glu Glu Pro Val Lys Val Arg Glu Ala Gly Asp Gly Val Phe Glu Cys
500                 505                 510                 515
gag tac tac ccg gtg gtg cct ggg aag tat gtg gtg acc atc acg tgg 1702
Glu Tyr Tyr Pro Val Val Pro Gly Lys Tyr Val Val Thr Ile Thr Trp
                520                 525                 530
ggc ggc tac gcc atc cct cgc agc ccc ttt gag gta cag gtg agc cca 1750
Gly Gly Tyr Ala Ile Pro Arg Ser Pro Phe Glu Val Gln Val Ser Pro
            535                 540                 545
gag gca gga gtg caa aag gtc cgg gcc tgg ggt cct ggt ttg gag act 1798
Glu Ala Gly Val Gln Lys Val Arg Ala Trp Gly Pro Gly Leu Glu Thr
        550                 555                 560
ggc cag gtg ggc aag tca gcc gat ttt gtg gtg gaa gcc att ggc acc 1846
Gly Gln Val Gly Lys Ser Ala Asp Phe Val Val Glu Ala Ile Gly Thr
    565                 570                 575
gag gtg ggg aca ctg ggc ttc tcc atc gag ggg ccc tca caa gcc aag 1894
Glu Val Gly Thr Leu Gly Phe Ser Ile Glu Gly Pro Ser Gln Ala Lys
580                 585                 590                 595
atc gaa tgt gac gat aag ggg gat ggc tcc tgc gat gtg cgg tac tgg 1942
Ile Glu Cys Asp Asp Lys Gly Asp Gly Ser Cys Asp Val Arg Tyr Trp
                600                 605                 610
ccc acg gag cct ggg gag tac gct gtg cac gtc atc tgt gac gat gag 1990
Pro Thr Glu Pro Gly Glu Tyr Ala Val His Val Ile Cys Asp Asp Glu

            615                 620                 625

gac atc cga gac tca ccc ttc att gcc cac atc ctg ccc gcc cca cct 2038

Asp Ile Arg Asp Ser Pro Phe Ile Ala His Ile Leu Pro Ala Pro Pro

        630                 635                 640

gac tgc ttc cca gat aag gtg aag gcc ttt ggg cct ggc ctg gag cct 2086

Asp Cys Phe Pro Asp Lys Val Lys Ala Phe Gly Pro Gly Leu Glu Pro

    645                 650                 655

acc ggc tgc atc gtg gac aag ccc gct gag ttc acc att gat gct cgt 2134

Thr Gly Cys Ile Val Asp Lys Pro Ala Glu Phe Thr Ile Asp Ala Arg

660                 665                 670                 675

gca gct ggc aag gga gac ctg aag ctc tat gcc cag gac gcc gac ggc 2182

Ala Ala Gly Lys Gly Asp Leu Lys Leu Tyr Ala Gln Asp Ala Asp Gly

                680                 685                 690

tgt ccc atc gac atc aag gtg atc ccc aac ggc aac ggc acc ttc cgc 2230

Cys Pro Ile Asp Ile Lys Val Ile Pro Asn Gly Asn Gly Thr Phe Arg

            695                 700                 705

tgc tcc tac gtg ccc acc aag ccc att aag cac acc atc atc atc tcc 2278

Cys Ser Tyr Val Pro Thr Lys Pro Ile Lys His Thr Ile Ile Ile Ser

        710                 715                 720

tgg gga ggc gta aac gtg ccc aag agc ccc ttc cgg gtg aac gtg ggc 2326

Trp Gly Gly Val Asn Val Pro Lys Ser Pro Phe Arg Val Asn Val Gly

    725                 730                 735

gag ggc agc cac ccc gag cgg gta aag gtg tac ggc ccc gga gtg gag 2374

Glu Gly Ser His Pro Glu Arg Val Lys Val Tyr Gly Pro Gly Val Glu

740                 745                 750                 755

aag aca ggc ctc aag gcc aat gag ccc acc tac ttc acg gtg gac tgc 2422

Lys Thr Gly Leu Lys Ala Asn Glu Pro Thr Tyr Phe Thr Val Asp Cys

                760                 765                 770

agc gag gcg ggg caa ggc gac gtg agc atc ggc atc aag tgc gcc cca 2470

Ser Glu Ala Gly Gln Gly Asp Val Ser Ile Gly Ile Lys Cys Ala Pro

            775                 780                 785

ggc gtg gtg ggc cct gca gag gct gac att gac ttc gac atc atc aag 2518

Gly Val Val Gly Pro Ala Glu Ala Asp Ile Asp Phe Asp Ile Ile Lys

        790                 795                 800

aat gac aac gac acc ttc acc gtc aag tac acg cca cca ggg gcg ggc 2566

Asn Asp Asn Asp Thr Phe Thr Val Lys Tyr Thr Pro Pro Gly Ala Gly

    805                 810                 815

cgc tac acc atc atg gtg ctg ttt gcc aac cag gag atc ccc gcc agc 2614

Arg Tyr Thr Ile Met Val Leu Phe Ala Asn Gln Glu Ile Pro Ala Ser

820                 825                 830                 835

ccc ttc cac atc aag gtg gac cca tcc cac gat gcc agc aaa gtc aag 2662

Pro Phe His Ile Lys Val Asp Pro Ser His Asp Ala Ser Lys Val Lys

                840                 845                 850

gcc gag ggc cct ggg ctg aat cgc aca ggt gtg gaa gtc ggg aag ccc 2710

Ala Glu Gly Pro Gly Leu Asn Arg Thr Gly Val Glu Val Gly Lys Pro

            855                 860                 865

acc cac ttc acg gtg ctg acc aag gga gcc ggc aag gcc aag ctg gat 2758

Thr His Phe Thr Val Leu Thr Lys Gly Ala Gly Lys Ala Lys Leu Asp

        870                 875                 880

gtg cag ttt gca ggg aca gcc aag ggc gag gtt gtg cgg gac ttt gag 2806

Val Gln Phe Ala Gly Thr Ala Lys Gly Glu Val Val Arg Asp Phe Glu

    885                 890                 895

atc ata gac aac cat gac tac tcc tac act gtc aag tac acc gct gtc 2854

Ile Ile Asp Asn His Asp Tyr Ser Tyr Thr Val Lys Tyr Thr Ala Val
900                 905                 910                 915
cag cag ggc aac atg gca gtg aca gtg act tat ggc ggg gac cct gtc 2902
Gln Gln Gly Asn Met Ala Val Thr Val Thr Tyr Gly Gly Asp Pro Val
                920                 925                 930
ccc aag agc ccc ttt gtg gtg aat gtg gca ccc ccg ctg gac ctc agc 2950
Pro Lys Ser Pro Phe Val Val Asn Val Ala Pro Pro Leu Asp Leu Ser
            935                 940                 945
aaa atc aaa gtt cag ggc ctt aat agc aag gtg gct gtg gga cag gaa 2998
Lys Ile Lys Val Gln Gly Leu Asn Ser Lys Val Ala Val Gly Gln Glu
        950                 955                 960
caa gca ttc tct gtg aac aca cga ggg gct ggc ggt cag ggc caa ctg 3046
Gln Ala Phe Ser Val Asn Thr Arg Gly Ala Gly Gly Gln Gly Gln Leu
    965                 970                 975
gat gtg cgg atg act tcg ccc tct cgc cgg ccc atc ccc tgc aag ctg 3094
Asp Val Arg Met Thr Ser Pro Ser Arg Arg Pro Ile Pro Cys Lys Leu
980                 985                 990                 995
gag cca ggc ggt gga gcg gaa gcc cag gct gtg cgc tac atg ccc ccg 3142
Glu Pro Gly Gly Gly Ala Glu Ala Gln Ala Val Arg Tyr Met Pro Pro
                1000                1005                1010
gag gag ggg ccc tac aag gtg gat atc acc tac gat ggt cac ccg gtg 3190
Glu Glu Gly Pro Tyr Lys Val Asp Ile Thr Tyr Asp Gly His Pro Val
            1015                1020                1025
cct ggc agc ccg ttt gct gtg gag ggt gtc ctg ccc cct gat ccc tcc 3238
Pro Gly Ser Pro Phe Ala Val Glu Gly Val Leu Pro Pro Asp Pro Ser
        1030                1035                1040
aag gtc tgt gct tat ggc ccg ggt ctc aag ggt gga ctg gta ggc acc 3286

Lys Val Cys Ala Tyr Gly Pro Gly Leu Lys Gly Gly Leu Val Gly Thr
    1045                1050                1055
ccc gcg cca ttc tcc atc gac acc aag ggg gct ggc aca ggt ggc ctg 3334
Pro Ala Pro Phe Ser Ile Asp Thr Lys Gly Ala Gly Thr Gly Gly Leu
1060                1065                1070                1075
ggg ctg acc gta gag ggc ccc tgc gag gcc aag atc gag tgc cag gac 3382
Gly Leu Thr Val Glu Gly Pro Cys Glu Ala Lys Ile Glu Cys Gln Asp
                1080                1085                1090
aat ggt gat ggc tca tgt gct gtc agc tac ctg ccc acg gag cct ggc 3430
Asn Gly Asp Gly Ser Cys Ala Val Ser Tyr Leu Pro Thr Glu Pro Gly
            1095                1100                1105
gag tac acc atc aac atc ctg ttt gct gag gcc cac atc cct ggc tcg 3478
Glu Tyr Thr Ile Asn Ile Leu Phe Ala Glu Ala His Ile Pro Gly Ser
        1110                1115                1120
ccc ttc aaa gcc acc att cgg cct gtg ttt gac ccg agc aag gtg cgg 3526
Pro Phe Lys Ala Thr Ile Arg Pro Val Phe Asp Pro Ser Lys Val Arg
    1125                1130                1135
gcc agt gga ccg ggc ctg gag cgc ggc aag gtc ggt gag gca gcc acc 3574
Ala Ser Gly Pro Gly Leu Glu Arg Gly Lys Val Gly Glu Ala Ala Thr
1140                1145                1150                1155
ttc act gtg gac tgc tca gag gca ggc gag gcg gag ctg acc att gag 3622
Phe Thr Val Asp Cys Ser Glu Ala Gly Glu Ala Glu Leu Thr Ile Glu
                1160                1165                1170
atc ctg tcg gat gcc ggg gtc aag gcc gag gtg ctg atc cac aac aac 3670
Ile Leu Ser Asp Ala Gly Val Lys Ala Glu Val Leu Ile His Asn Asn
            1175                1180                1185

gcg gat ggc acc tac cac atc acc tac agc cct gcc ttc cct ggc acc 3718
Ala Asp Gly Thr Tyr His Ile Thr Tyr Ser Pro Ala Phe Pro Gly Thr
        1190                1195                1200
tac acc att acc atc aag tat ggc ggg cat ccc gtg ccc aaa ttc ccc 3766
Tyr Thr Ile Thr Ile Lys Tyr Gly Gly His Pro Val Pro Lys Phe Pro
    1205                1210                1215
acc cgt gtc cat gtg cag cct gcg gtc gat acc agt ggc gtc aag gtc 3814
Thr Arg Val His Val Gln Pro Ala Val Asp Thr Ser Gly Val Lys Val
1220                1225                1230                1235
tca ggg cct ggt gtt gag cca cac ggt gtc ctg cgg gag gtg acc act 3862
Ser Gly Pro Gly Val Glu Pro His Gly Val Leu Arg Glu Val Thr Thr
                1240                1245                1250
gag ttc act gtg gat gca aga tcc cta aca gcc aca ggc ggc aac cac 3910
Glu Phe Thr Val Asp Ala Arg Ser Leu Thr Ala Thr Gly Gly Asn His
            1255                1260                1265
gtg acg gct cgt gtg ctc aac ccc tcg ggg gcc aag aca gac acc tat 3958
Val Thr Ala Arg Val Leu Asn Pro Ser Gly Ala Lys Thr Asp Thr Tyr
        1270                1275                1280
gtg aca gac aat ggg gac ggc acc tac cga gtg cag tac acc gcc tac 4006
Val Thr Asp Asn Gly Asp Gly Thr Tyr Arg Val Gln Tyr Thr Ala Tyr
    1285                1290                1295
gag gag ggc gtg cat ctg gtg gag gtc ctg tat gat gag gtc gct gtg 4054
Glu Glu Gly Val His Leu Val Glu Val Leu Tyr Asp Glu Val Ala Val
1300                1305                1310                1315
ccc aag agc ccc ttc cga gtg ggc gtg acc gag ggc tgt gat ccc acc 4102
Pro Lys Ser Pro Phe Arg Val Gly Val Thr Glu Gly Cys Asp Pro Thr
                1320                1325                1330

cgc gtc cga gcc ttc ggg cca ggc ctg gag ggt ggc ttg gtc aac aag 4150
Arg Val Arg Ala Phe Gly Pro Gly Leu Glu Gly Gly Leu Val Asn Lys
            1335                1340                1345
gcc aac cga ttc act gtg gag acc agg gga gcg ggc acc ggg ggc ctt 4198
Ala Asn Arg Phe Thr Val Glu Thr Arg Gly Ala Gly Thr Gly Gly Leu
        1350                1355                1360
ggc cta gcc atc gag ggt ccc tcg gaa gcc aag atg tcc tgc aag gac 4246
Gly Leu Ala Ile Glu Gly Pro Ser Glu Ala Lys Met Ser Cys Lys Asp
    1365                1370                1375
aac aag gat ggt agc tgc acc gtg gag tac atc ccc ttc act cct gga 4294
Asn Lys Asp Gly Ser Cys Thr Val Glu Tyr Ile Pro Phe Thr Pro Gly
1380                1385                1390                1395
gac tat gac gtc aac atc acc ttc ggg ggg cgg ccc atc cca ggg agc 4342
Asp Tyr Asp Val Asn Ile Thr Phe Gly Gly Arg Pro Ile Pro Gly Ser
                1400                1405                1410
ccg ttc cgc gtg cca gtg aag gat gtg gtg gac cct ggg aag gtg aag 4390
Pro Phe Arg Val Pro Val Lys Asp Val Val Asp Pro Gly Lys Val Lys
            1415                1420                1425
tgc tca ggg cca ggg ctg ggg gct ggt gtc agg gcc cgg gtt cct cag 4438
Cys Ser Gly Pro Gly Leu Gly Ala Gly Val Arg Ala Arg Val Pro Gln
        1430                1435                1440
acc ttc aca gtg gac tgc agt caa gct ggc cgg gcg ccc ctg cag gtg 4486
Thr Phe Thr Val Asp Cys Ser Gln Ala Gly Arg Ala Pro Leu Gln Val
    1445                1450                1455
gct gtg ctg ggc ccc aca ggt gtg gcc gag cct gtg gag gtg cgg gac 4534
Ala Val Leu Gly Pro Thr Gly Val Ala Glu Pro Val Glu Val Arg Asp

1460                1465                1470                1475

aat gga gat ggc acc cac act gtc cac tac acc cca gcc act gac ggg    4582

Asn Gly Asp Gly Thr His Thr Val His Tyr Thr Pro Ala Thr Asp Gly

                1480                1485                1490

ccc tac acg gta gcc gtc aag tat gct gac cag gag gtg cca cgc agc    4630

Pro Tyr Thr Val Ala Val Lys Tyr Ala Asp Gln Glu Val Pro Arg Ser

            1495                1500                1505

ccc ttc aag atc aag gtc ctc cca gct cat gat gcc agc aag gtg cgg    4678

Pro Phe Lys Ile Lys Val Leu Pro Ala His Asp Ala Ser Lys Val Arg

        1510                1515                1520

gcc agc ggg cca ggc ctc aac gcc tct ggc atc cct gcc agc ctg cct    4726

Ala Ser Gly Pro Gly Leu Asn Ala Ser Gly Ile Pro Ala Ser Leu Pro

    1525                1530                1535

gtg gag ttc acc atc gac gca cgg gac gcg ggc gag ggg ttg ctc act    4774

Val Glu Phe Thr Ile Asp Ala Arg Asp Ala Gly Glu Gly Leu Leu Thr

1540                1545                1550                1555

gtc cag atc ttg ggc ccc gag ggt aag ccc aag aag gcc aac atc cgg    4822

Val Gln Ile Leu Gly Pro Glu Gly Lys Pro Lys Lys Ala Asn Ile Arg

                1560                1565                1570

gac aat ggg gat ggc acg tac gct gtg tcc tac ctg ccg gac atg agt    4870

Asp Asn Gly Asp Gly Thr Tyr Ala Val Ser Tyr Leu Pro Asp Met Ser

            1575                1580                1585

ggc cgg tac acc atc acc atc aag tat ggc ggt gat gag atc ccc tac    4918

Gly Arg Tyr Thr Ile Thr Ile Lys Tyr Gly Gly Asp Glu Ile Pro Tyr

        1590                1595                1600

tcg ccc ttc cgc atc cat gct ctg ccc act ggg gat gcc agc aag tgc    4966

Ser Pro Phe Arg Ile His Ala Leu Pro Thr Gly Asp Ala Ser Lys Cys

    1605                1610                1615

ctc gtc aca gtg tcc att gga ggc cat ggc ctg ggt gcc tgc ctg ggc 5014

Leu Val Thr Val Ser Ile Gly Gly His Gly Leu Gly Ala Cys Leu Gly

1620                1625                1630                1635

cct cga atc cag att ggg cag gag acg gtg atc acg gtg gat gcc aag 5062

Pro Arg Ile Gln Ile Gly Gln Glu Thr Val Ile Thr Val Asp Ala Lys

                1640                1645                1650

gca gcc ggt gag ggg aag gtg aca tgc acg gtg tcc acg ccg gat ggg 5110

Ala Ala Gly Glu Gly Lys Val Thr Cys Thr Val Ser Thr Pro Asp Gly

            1655                1660                1665

gca gag ctc gat gtg gat gtg gtt gag aac cat gac ggt acc ttt gac 5158

Ala Glu Leu Asp Val Asp Val Val Glu Asn His Asp Gly Thr Phe Asp

        1670                1675                1680

atc tac tac aca gcg ccc gag ccg ggc aag tac gtc atc acc atc cgc 5206

Ile Tyr Tyr Thr Ala Pro Glu Pro Gly Lys Tyr Val Ile Thr Ile Arg

    1685                1690                1695

ttc ggg ggt gag cac atc ccc aac agc ccc ttc cac gtg ctg gcg tgt 5254

Phe Gly Gly Glu His Ile Pro Asn Ser Pro Phe His Val Leu Ala Cys

1700                1705                1710                1715

gac ccc ctg ccg cac gag gag gag ccc tct gaa gtg cca cag ctg cgc 5302

Asp Pro Leu Pro His Glu Glu Glu Pro Ser Glu Val Pro Gln Leu Arg

                1720                1725                1730

cag ccc tac gct cct ccc cgg ccc ggc gcc cgc ccc aca cac tgg gcc 5350

Gln Pro Tyr Ala Pro Pro Arg Pro Gly Ala Arg Pro Thr His Trp Ala

            1735                1740                1745

aca gag gag cca gtg gtg cct gtg gag cca atg gag tcc atg ctg agg 5398

Thr Glu Glu Pro Val Val Pro Val Glu Pro Met Glu Ser Met Leu Arg
        1750                1755                1760
ccc ttc aac ctg gtc atc ccc ttc gcg gtg cag aaa ggg gag ctc aca 5446
Pro Phe Asn Leu Val Ile Pro Phe Ala Val Gln Lys Gly Glu Leu Thr
    1765                1770                1775
gga gag gtg cgg atg ccc tcg ggg aag acg gca cgg ccc aac atc acc 5494
Gly Glu Val Arg Met Pro Ser Gly Lys Thr Ala Arg Pro Asn Ile Thr
1780                1785                1790                1795
gac aac aag gac ggc acc atc acg gtg agg tat gca ccc act gag aaa 5542
Asp Asn Lys Asp Gly Thr Ile Thr Val Arg Tyr Ala Pro Thr Glu Lys
                1800                1805                1810
ggc ctg cac cag atg ggg atc aag tat gac ggc aac cac atc cct ggg 5590
Gly Leu His Gln Met Gly Ile Lys Tyr Asp Gly Asn His Ile Pro Gly
            1815                1820                1825
agc ccc tta cag ttc tat gtg gat gcc atc aac agc cgc cat gtc agt 5638
Ser Pro Leu Gln Phe Tyr Val Asp Ala Ile Asn Ser Arg His Val Ser
        1830                1835                1840
gcc tat ggg cca ggc ctg agc cat ggc atg gtc aac aag cca gcc acc 5686
Ala Tyr Gly Pro Gly Leu Ser His Gly Met Val Asn Lys Pro Ala Thr
    1845                1850                1855
ttc act att gtc acc aaa gat gct gga gaa ggg ggt ctg tca ctg gcc 5734
Phe Thr Ile Val Thr Lys Asp Ala Gly Glu Gly Gly Leu Ser Leu Ala
1860                1865                1870                1875
gtg gag ggc cca tcc aag gca gag atc acc tgt aag gac aac aag gat 5782
Val Glu Gly Pro Ser Lys Ala Glu Ile Thr Cys Lys Asp Asn Lys Asp
                1880                1885                1890
ggc acc tgc acc gtg tcc tat ctg ccg act gcg cct gga gac tac agc 5830

Gly Thr Cys Thr Val Ser Tyr Leu Pro Thr Ala Pro Gly Asp Tyr Ser
            1895                1900                1905
atc atc gtg cgc ttc gat gac aag cac atc ccg ggg agc ccc ttc aca 5878
Ile Ile Val Arg Phe Asp Asp Lys His Ile Pro Gly Ser Pro Phe Thr
        1910                1915                1920
gcc aag atc aca ggt gat gac tcc atg agg acc tca cag ctg aat gtg 5926
Ala Lys Ile Thr Gly Asp Asp Ser Met Arg Thr Ser Gln Leu Asn Val
    1925                1930                1935
ggc acc tcc acg gac gtg tca ctg aag atc acc gag agt gat ctg agc 5974
Gly Thr Ser Thr Asp Val Ser Leu Lys Ile Thr Glu Ser Asp Leu Ser
1940                1945                1950                1955
cag ctg acc gcc agc atc cgt gcc ccc tcg ggc aac gag gag ccc tgc 6022
Gln Leu Thr Ala Ser Ile Arg Ala Pro Ser Gly Asn Glu Glu Pro Cys
                1960                1965                1970
ctg ctg aag cgc ctg ccc aac cgg cac att ggg atc tcc ttc acc ccc 6070
Leu Leu Lys Arg Leu Pro Asn Arg His Ile Gly Ile Ser Phe Thr Pro
            1975                1980                1985
aag gag gtc ggg gag cac gtg gtg agc gtg cgc aag agt ggc aag cat 6118
Lys Glu Val Gly Glu His Val Val Ser Val Arg Lys Ser Gly Lys His
        1990                1995                2000
gtc acc aac agc ccc ttc aag atc ctg gtg ggg cca tct gag atc ggg 6166
Val Thr Asn Ser Pro Phe Lys Ile Leu Val Gly Pro Ser Glu Ile Gly
    2005                2010                2015
gac gcc agc aag gtg cgg gtc tgg ggc aag ggg ctt tcc gag gga cac 6214
Asp Ala Ser Lys Val Arg Val Trp Gly Lys Gly Leu Ser Glu Gly His
2020                2025                2030                2035

aca ttc cag gtg gca gag ttc atc gtg gac act cgc aat gca ggt tat 6262
Thr Phe Gln Val Ala Glu Phe Ile Val Asp Thr Arg Asn Ala Gly Tyr
                2040                2045                2050
ggg ggc ttg ggg ctg agt att gaa ggc cca agc aag gtg gac atc aac 6310
Gly Gly Leu Gly Leu Ser Ile Glu Gly Pro Ser Lys Val Asp Ile Asn
            2055                2060                2065
tgt gag gac atg gag gac ggg aca tgc aaa gtc acc tac tgc ccc acc 6358
Cys Glu Asp Met Glu Asp Gly Thr Cys Lys Val Thr Tyr Cys Pro Thr
        2070                2075                2080
gag ccc ggc acc tac atc atc aac atc aag ttt gct gac aag cac gtg 6406
Glu Pro Gly Thr Tyr Ile Ile Asn Ile Lys Phe Ala Asp Lys His Val
    2085                2090                2095
cct gga agc ccc ttc act gtg aag gtg acc ggc gag ggc cgc atg aag 6454
Pro Gly Ser Pro Phe Thr Val Lys Val Thr Gly Glu Gly Arg Met Lys
2100                2105                2110                2115
gag agc atc acc cgg cgg aga cag gca cct tcc atc gcc acc atc ggc 6502
Glu Ser Ile Thr Arg Arg Arg Gln Ala Pro Ser Ile Ala Thr Ile Gly
                2120                2125                2130
agc acc tgt gac ctc aac ctc aag atc cca gga aac tgg ttc cag atg 6550
Ser Thr Cys Asp Leu Asn Leu Lys Ile Pro Gly Asn Trp Phe Gln Met
            2135                2140                2145
gtg tct gcc cag gag cgc ctg aca cgc acc ttc aca cgc agc agc cac 6598
Val Ser Ala Gln Glu Arg Leu Thr Arg Thr Phe Thr Arg Ser Ser His
        2150                2155                2160
acc tac acc cgc acg gag cgc acg gag atc agc aag acg cgg ggc ggg 6646
Thr Tyr Thr Arg Thr Glu Arg Thr Glu Ile Ser Lys Thr Arg Gly Gly
    2165                2170                2175

gag aca aag ccc gag gtg cgg gtg gag gag tcc acc cag gtc ggc ggg 6694
Glu Thr Lys Pro Glu Val Arg Val Glu Glu Ser Thr Gln Val Gly Gly
2180                2185                2190                2195
gac ccc ttc cct gct gtg ttt ggg gac ttc ctg ggc cgg gag cgc ctg 6742
Asp Pro Phe Pro Ala Val Phe Gly Asp Phe Leu Gly Arg Glu Arg Leu
                2200                2205                2210
gga tcc ttc ggc agc atc acc cgg cag cag gag ggt gag gcc agc tct 6790
Gly Ser Phe Gly Ser Ile Thr Arg Gln Gln Glu Gly Glu Ala Ser Ser
            2215                2220                2225
cag gac atg act gca cag gtg acc agc cca tcg ggc aag gtg gaa gcc 6838
Gln Asp Met Thr Ala Gln Val Thr Ser Pro Ser Gly Lys Val Glu Ala
        2230                2235                2240
gca gag atc gtc gag ggc gag gac agc gcc tac agc gtc cgc ttt gtg 6886
Ala Glu Ile Val Glu Gly Glu Asp Ser Ala Tyr Ser Val Arg Phe Val
    2245                2250                2255
ccc cag gaa atg ggg ccc cat acg gtc gct gtc aag tac cgt ggc cag 6934
Pro Gln Glu Met Gly Pro His Thr Val Ala Val Lys Tyr Arg Gly Gln
2260                2265                2270                2275
cac gtg ccc ggc agc ccc ttt cag ttc act gtg ggg ccg ctg ggt gaa 6982
His Val Pro Gly Ser Pro Phe Gln Phe Thr Val Gly Pro Leu Gly Glu
                2280                2285                2290
ggt ggt gcc cac aag gtg cgg gcc gga cga gca ggg ctg gag cga ggt 7030
Gly Gly Ala His Lys Val Arg Ala Gly Arg Ala Gly Leu Glu Arg Gly
            2295                2300                2305
gtg gcc ggc gtg cca gcc gag ttc agc atc tgg acc cgg gag gct ggc 7078
Val Ala Gly Val Pro Ala Glu Phe Ser Ile Trp Thr Arg Glu Ala Gly

2310                2315                2320

gct ggg ggc ctg tcc att gct gtg gag ggt cct agc aaa gcg gag att    7126

Ala Gly Gly Leu Ser Ile Ala Val Glu Gly Pro Ser Lys Ala Glu Ile

    2325                2330                2335

gca ttt gag gat cgc aaa gat ggc tcc tgc ggc gtc tcc tat gtc gtc    7174

Ala Phe Glu Asp Arg Lys Asp Gly Ser Cys Gly Val Ser Tyr Val Val

2340                2345                2350                2355

cag gaa cca ggt gac tat gag gtc tcc atc aag ttc aat gat gag cac    7222

Gln Glu Pro Gly Asp Tyr Glu Val Ser Ile Lys Phe Asn Asp Glu His

                2360                2365                2370

atc cca gac agc ccc ttt gtg gtg cct gtg gcc tcc ctc tcg gat gac    7270

Ile Pro Asp Ser Pro Phe Val Val Pro Val Ala Ser Leu Ser Asp Asp

            2375                2380                2385

gct cgc cgt ctc act gtc acc agc ctc cag gag acg ggg ctc aag gtg    7318

Ala Arg Arg Leu Thr Val Thr Ser Leu Gln Glu Thr Gly Leu Lys Val

        2390                2395                2400

aac cag cca gcg tcc ttt gcc gtg cag ctg aac ggt gcc cgg ggc gtg    7366

Asn Gln Pro Ala Ser Phe Ala Val Gln Leu Asn Gly Ala Arg Gly Val

    2405                2410                2415

att gat gcc cgg gtg cac aca ccc tcg ggg gct gtg gag gag tgc tac    7414

Ile Asp Ala Arg Val His Thr Pro Ser Gly Ala Val Glu Glu Cys Tyr

2420                2425                2430                2435

gtc tct gag ctg gac agt gac aag cac acc atc cgc ttc atc ccc cac    7462

Val Ser Glu Leu Asp Ser Asp Lys His Thr Ile Arg Phe Ile Pro His

                2440                2445                2450

gag aat ggc gtc cac tcc atc gat gtc aag ttc aac ggt gcc cac atc    7510

Glu Asn Gly Val His Ser Ile Asp Val Lys Phe Asn Gly Ala His Ile

            2455                2460                2465

cct gga agt ccc ttc aag atc cgc gtt ggg gag cag agc cag gct ggg 7558

Pro Gly Ser Pro Phe Lys Ile Arg Val Gly Glu Gln Ser Gln Ala Gly

        2470                2475                2480

gac cca ggc ttg gtg tca gcc tac ggt cct ggg ctc gag gga ggc act 7606

Asp Pro Gly Leu Val Ser Ala Tyr Gly Pro Gly Leu Glu Gly Gly Thr

    2485                2490                2495

acc ggt gtg tca tca gag ttc atc gtg aac acc ctg aat gcc ggc tcg 7654

Thr Gly Val Ser Ser Glu Phe Ile Val Asn Thr Leu Asn Ala Gly Ser

2500                2505                2510                2515

ggg gcc ttg tct gtc acc att gat ggc ccc tcc aag gtg cag ctg gac 7702

Gly Ala Leu Ser Val Thr Ile Asp Gly Pro Ser Lys Val Gln Leu Asp

                2520                2525                2530

tgt cgg gag tgt cct gag ggc cat gtg gtc act tat act ccc atg gcc 7750

Cys Arg Glu Cys Pro Glu Gly His Val Val Thr Tyr Thr Pro Met Ala

            2535                2540                2545

cct ggc aac tac ctc att gcc atc aag tac ggt ggc ccc cag cac atc 7798

Pro Gly Asn Tyr Leu Ile Ala Ile Lys Tyr Gly Gly Pro Gln His Ile

        2550                2555                2560

gtg ggc agc ccc ttc aag gcc aag gtc act ggt ccg agg ctg tcc gga 7846

Val Gly Ser Pro Phe Lys Ala Lys Val Thr Gly Pro Arg Leu Ser Gly

    2565                2570                2575

ggc cac agc ctt cac gaa aca tcc acg gtt ctg gtg gag act gtg acc 7894

Gly His Ser Leu His Glu Thr Ser Thr Val Leu Val Glu Thr Val Thr

2580                2585                2590                2595

aag tcc tcc tca agc cgg ggc tcc agc tac agc tcc atc ccc aag ttc 7942

Lys Ser Ser Ser Ser Arg Gly Ser Ser Tyr Ser Ser Ile Pro Lys Phe
                2600                2605                2610
tcc tca gat gcc agc aag gtg gtg act cgg ggc cct ggg ctg tcc cag 7990
Ser Ser Asp Ala Ser Lys Val Val Thr Arg Gly Pro Gly Leu Ser Gln
            2615                2620                2625
gcc ttc gtg ggc cag aag aac tcc ttc acc gtg gac tgc agc aaa gca 8038
Ala Phe Val Gly Gln Lys Asn Ser Phe Thr Val Asp Cys Ser Lys Ala
        2630                2635                2640
ggc acc aac atg atg atg gtg ggc gtg cac ggc ccc aag acc ccc tgt 8086
Gly Thr Asn Met Met Met Val Gly Val His Gly Pro Lys Thr Pro Cys
    2645                2650                2655
gag gag gtg tac gtg aag cac atg ggg aac cgg gtg tac aat gtc acc 8134
Glu Glu Val Tyr Val Lys His Met Gly Asn Arg Val Tyr Asn Val Thr
2660                2665                2670                2675
tac act gtc aag gag aaa ggg gac tac atc ctc att gtc aag tgg ggt 8182
Tyr Thr Val Lys Glu Lys Gly Asp Tyr Ile Leu Ile Val Lys Trp Gly
                2680                2685                2690
gac gaa agt gtc cct gga agc ccc ttc aaa gtc aag gtc cct 8224
Asp Glu Ser Val Pro Gly Ser Pro Phe Lys Val Lys Val Pro
            2695                2700                2705
tgaatcccaa aagtgcctcc ccagcctcag cccccacctc cagccacaca cacattacac 8284
acacacacac acacacacaa atgtgccaca cccagacacg cacagaatca gacactacaa 8344
acacctgcct tgggggtgaa gtgaaggccc agcctcccca ccccaccgcg ccccaggggt 8404
tggaggacct tgtctgtgtc agacagtgtc cctccctgga atgtgacatg aggccgactg 8464
gggccaggct caggggcaga ggctgggaca caaggggctg gcgagggctg cgaggccagg 8524
gaagccctga gtttctggcg gggctgagca gtgggggagc attgtgttgt gggtgtctgt 8584
gtgtgaggtc accctcaaac tgcaccgccg gccagatacc ctcctgaccc cgaggacttg 8644

gtctggtctc tctggtggct acaaccccag agttttaagg acttggaaag gaagcacaat 8704
cagagaagaa aacagccccc aaccagcagg agcggcctgg cacatggacc ggcctgagcg 8764
atgtgcactc cacccaagcc aggctcccag ggggcctgat ttctctctca ctgtctcttt 8824
ttttaaaatg gttgcacggc tctgccccat ggggggcctt ttttacacac tgcgaggccc 8884
agctttctag ggactttttg cacatgtcat gcagctcagc tgggagctgc ttaggtggaa 8944
aactccaaat aaagtgcgcc tgtcgcc 8971

<210> 44
<211> 2705
<212> PRT
<213> Homo sapiens
<400> 44
Met Pro Ser Thr Glu Lys Asp Leu Ala Glu Asp Ala Pro Trp Lys Lys
1               5                   10                  15
Ile Gln Gln Asn Thr Phe Thr Arg Trp Cys Asn Glu His Leu Lys Cys
            20                  25                  30
Val Gly Lys Arg Leu Thr Asp Leu Gln Arg Asp Leu Ser Asp Gly Leu
        35                  40                  45
Arg Leu Ile Ala Leu Leu Glu Val Leu Ser Gln Lys Arg Met Tyr Arg
    50                  55                  60
Lys Phe His Pro Arg Pro Asn Phe Arg Gln Met Lys Leu Glu Asn Val
65                  70                  75                  80
Ser Val Ala Leu Glu Phe Leu Glu Arg Glu His Ile Lys Leu Val Ser
                85                  90                  95
Ile Asp Ser Lys Ala Ile Val Asp Gly Asn Leu Lys Leu Ile Leu Gly
            100                 105                 110
Leu Ile Trp Thr Leu Ile Leu His Tyr Ser Ile Ser Met Pro Met Trp

        115                 120                 125
Glu Asp Glu Asp Asp Glu Asp Ala Arg Lys Gln Thr Pro Lys Gln Arg
    130                 135                 140
Leu Leu Gly Trp Ile Gln Asn Lys Val Pro Gln Leu Pro Ile Thr Asn
145                 150                 155                 160
Phe Asn Arg Asp Trp Gln Asp Gly Lys Ala Leu Gly Ala Leu Val Asp
                165                 170                 175
Asn Cys Ala Pro Gly Leu Cys Pro Asp Trp Glu Ala Trp Asp Pro Asn
            180                 185                 190
Gln Pro Val Glu Asn Ser Arg Glu Ala Met Gln Gln Ala Asp Asp Trp
        195                 200                 205
Leu Gly Val Pro Gln Val Ile Ala Pro Glu Glu Ile Val Asp Pro Asn
    210                 215                 220
Val Asp Glu His Ser Val Met Thr Tyr Leu Ser Gln Phe Pro Lys Ala
225                 230                 235                 240
Lys Leu Lys Pro Gly Ala Pro Val Arg Ser Lys Gln Leu Asn Pro Lys
                245                 250                 255
Lys Ala Ile Ala Tyr Gly Pro Gly Ile Glu Pro Gln Gly Asn Thr Val
            260                 265                 270
Leu Gln Pro Ala His Phe Thr Val Gln Thr Val Asp Ala Gly Val Gly
        275                 280                 285
Glu Val Leu Val Tyr Ile Glu Asp Pro Glu Gly His Thr Glu Glu Ala
    290                 295                 300
Lys Val Val Pro Asn Asn Asp Lys Asp Arg Thr Tyr Ala Val Ser Tyr
305                 310                 315                 320
Val Pro Lys Val Ala Gly Leu His Lys Val Thr Val Leu Phe Ala Gly

                325                 330                 335
Gln Asn Ile Glu Arg Ser Pro Phe Glu Val Asn Val Gly Met Ala Leu
            340                 345                 350
Gly Asp Ala Asn Lys Val Ser Ala Arg Gly Pro Gly Leu Glu Pro Val
        355                 360                 365
Gly Asn Val Ala Asn Lys Pro Thr Tyr Phe Asp Ile Tyr Thr Ala Gly
    370                 375                 380
Ala Gly Thr Gly Asp Val Ala Val Val Ile Val Asp Pro Gln Gly Arg
385                 390                 395                 400
Arg Asp Thr Val Glu Val Ala Leu Glu Asp Lys Gly Asp Ser Thr Phe
                405                 410                 415
Arg Cys Thr Tyr Arg Pro Ala Met Glu Gly Pro His Thr Val His Val
            420                 425                 430
Ala Phe Ala Gly Ala Pro Ile Thr Arg Ser Pro Phe Pro Val His Val
        435                 440                 445
Ser Glu Ala Cys Asn Pro Asn Ala Cys Arg Ala Ser Gly Arg Gly Leu
    450                 455                 460
Gln Pro Lys Gly Val Arg Val Lys Glu Val Ala Asp Phe Lys Val Phe
465                 470                 475                 480
Thr Lys Gly Ala Gly Ser Gly Glu Leu Lys Val Thr Val Lys Gly Pro
                485                 490                 495
Lys Gly Thr Glu Glu Pro Val Lys Val Arg Glu Ala Gly Asp Gly Val
            500                 505                 510
Phe Glu Cys Glu Tyr Tyr Pro Val Val Pro Gly Lys Tyr Val Val Thr
        515                 520                 525
Ile Thr Trp Gly Gly Tyr Ala Ile Pro Arg Ser Pro Phe Glu Val Gln
    530                 535                 540

Val Ser Pro Glu Ala Gly Val Gln Lys Val Arg Ala Trp Gly Pro Gly
545                 550                 555                 560
Leu Glu Thr Gly Gln Val Gly Lys Ser Ala Asp Phe Val Val Glu Ala
                565                 570                 575
Ile Gly Thr Glu Val Gly Thr Leu Gly Phe Ser Ile Glu Gly Pro Ser
            580                 585                 590
Gln Ala Lys Ile Glu Cys Asp Asp Lys Gly Asp Gly Ser Cys Asp Val
        595                 600                 605
Arg Tyr Trp Pro Thr Glu Pro Gly Glu Tyr Ala Val His Val Ile Cys
    610                 615                 620
Asp Asp Glu Asp Ile Arg Asp Ser Pro Phe Ile Ala His Ile Leu Pro
625                 630                 635                 640
Ala Pro Pro Asp Cys Phe Pro Asp Lys Val Lys Ala Phe Gly Pro Gly
                645                 650                 655
Leu Glu Pro Thr Gly Cys Ile Val Asp Lys Pro Ala Glu Phe Thr Ile
            660                 665                 670
Asp Ala Arg Ala Ala Gly Lys Gly Asp Leu Lys Leu Tyr Ala Gln Asp
        675                 680                 685
Ala Asp Gly Cys Pro Ile Asp Ile Lys Val Ile Pro Asn Gly Asn Gly
    690                 695                 700
Thr Phe Arg Cys Ser Tyr Val Pro Thr Lys Pro Ile Lys His Thr Ile
705                 710                 715                 720
Ile Ile Ser Trp Gly Gly Val Asn Val Pro Lys Ser Pro Phe Arg Val
                725                 730                 735
Asn Val Gly Glu Gly Ser His Pro Glu Arg Val Lys Val Tyr Gly Pro
            740                 745                 750

Gly Val Glu Lys Thr Gly Leu Lys Ala Asn Glu Pro Thr Tyr Phe Thr
        755                 760                 765
Val Asp Cys Ser Glu Ala Gly Gln Gly Asp Val Ser Ile Gly Ile Lys
    770                 775                 780
Cys Ala Pro Gly Val Val Gly Pro Ala Glu Ala Asp Ile Asp Phe Asp
785                 790                 795                 800
Ile Ile Lys Asn Asp Asn Asp Thr Phe Thr Val Lys Tyr Thr Pro Pro
                805                 810                 815
Gly Ala Gly Arg Tyr Thr Ile Met Val Leu Phe Ala Asn Gln Glu Ile
            820                 825                 830
Pro Ala Ser Pro Phe His Ile Lys Val Asp Pro Ser His Asp Ala Ser
        835                 840                 845
Lys Val Lys Ala Glu Gly Pro Gly Leu Asn Arg Thr Gly Val Glu Val
    850                 855                 860
Gly Lys Pro Thr His Phe Thr Val Leu Thr Lys Gly Ala Gly Lys Ala
865                 870                 875                 880
Lys Leu Asp Val Gln Phe Ala Gly Thr Ala Lys Gly Glu Val Val Arg
                885                 890                 895
Asp Phe Glu Ile Ile Asp Asn His Asp Tyr Ser Tyr Thr Val Lys Tyr
            900                 905                 910
Thr Ala Val Gln Gln Gly Asn Met Ala Val Thr Val Thr Tyr Gly Gly
        915                 920                 925
Asp Pro Val Pro Lys Ser Pro Phe Val Val Asn Val Ala Pro Pro Leu
    930                 935                 940
Asp Leu Ser Lys Ile Lys Val Gln Gly Leu Asn Ser Lys Val Ala Val
945                 950                 955                 960
Gly Gln Glu Gln Ala Phe Ser Val Asn Thr Arg Gly Ala Gly Gly Gln

                965                 970                 975
Gly Gln Leu Asp Val Arg Met Thr Ser Pro Ser Arg Arg Pro Ile Pro
            980                 985                 990
Cys Lys Leu Glu Pro Gly Gly Gly Ala Glu Ala Gln Ala Val Arg Tyr
        995                 1000                1005
Met Pro Pro Glu Glu Gly Pro Tyr Lys Val Asp Ile Thr Tyr Asp Gly
    1010                1015                1020
His Pro Val Pro Gly Ser Pro Phe Ala Val Glu Gly Val Leu Pro Pro
1025                1030                1035                1040
Asp Pro Ser Lys Val Cys Ala Tyr Gly Pro Gly Leu Lys Gly Gly Leu
                1045                1050                1055
Val Gly Thr Pro Ala Pro Phe Ser Ile Asp Thr Lys Gly Ala Gly Thr
            1060                1065                1070
Gly Gly Leu Gly Leu Thr Val Glu Gly Pro Cys Glu Ala Lys Ile Glu
        1075                1080                1085
Cys Gln Asp Asn Gly Asp Gly Ser Cys Ala Val Ser Tyr Leu Pro Thr
    1090                1095                1100
Glu Pro Gly Glu Tyr Thr Ile Asn Ile Leu Phe Ala Glu Ala His Ile
1105                1110                1115                1120
Pro Gly Ser Pro Phe Lys Ala Thr Ile Arg Pro Val Phe Asp Pro Ser
                1125                1130                1135
Lys Val Arg Ala Ser Gly Pro Gly Leu Glu Arg Gly Lys Val Gly Glu
            1140                1145                1150
Ala Ala Thr Phe Thr Val Asp Cys Ser Glu Ala Gly Glu Ala Glu Leu
        1155                1160                1165
Thr Ile Glu Ile Leu Ser Asp Ala Gly Val Lys Ala Glu Val Leu Ile

1170                 1175                 1180

His Asn Asn Ala Asp Gly Thr Tyr His Ile Thr Tyr Ser Pro Ala Phe

1185                 1190                 1195                 1200

Pro Gly Thr Tyr Thr Ile Thr Ile Lys Tyr Gly Gly His Pro Val Pro

                 1205                 1210                 1215

Lys Phe Pro Thr Arg Val His Val Gln Pro Ala Val Asp Thr Ser Gly

             1220                 1225                 1230

Val Lys Val Ser Gly Pro Gly Val Glu Pro His Gly Val Leu Arg Glu

         1235                 1240                 1245

Val Thr Thr Glu Phe Thr Val Asp Ala Arg Ser Leu Thr Ala Thr Gly

    1250                 1255                 1260

Gly Asn His Val Thr Ala Arg Val Leu Asn Pro Ser Gly Ala Lys Thr

1265                 1270                 1275                 1280

Asp Thr Tyr Val Thr Asp Asn Gly Asp Gly Thr Tyr Arg Val Gln Tyr

                 1285                 1290                 1295

Thr Ala Tyr Glu Glu Gly Val His Leu Val Glu Val Leu Tyr Asp Glu

             1300                 1305                 1310

Val Ala Val Pro Lys Ser Pro Phe Arg Val Gly Val Thr Glu Gly Cys

         1315                 1320                 1325

Asp Pro Thr Arg Val Arg Ala Phe Gly Pro Gly Leu Glu Gly Gly Leu

    1330                 1335                 1340

Val Asn Lys Ala Asn Arg Phe Thr Val Glu Thr Arg Gly Ala Gly Thr

1345                 1350                 1355                 1360

Gly Gly Leu Gly Leu Ala Ile Glu Gly Pro Ser Glu Ala Lys Met Ser

                 1365                 1370                 1375

Cys Lys Asp Asn Lys Asp Gly Ser Cys Thr Val Glu Tyr Ile Pro Phe

             1380                 1385                 1390

Thr Pro Gly Asp Tyr Asp Val Asn Ile Thr Phe Gly Gly Arg Pro Ile
        1395                1400                1405
Pro Gly Ser Pro Phe Arg Val Pro Val Lys Asp Val Val Asp Pro Gly
    1410                1415                1420
Lys Val Lys Cys Ser Gly Pro Gly Leu Gly Ala Gly Val Arg Ala Arg
1425                1430                1435                1440
Val Pro Gln Thr Phe Thr Val Asp Cys Ser Gln Ala Gly Arg Ala Pro
                1445                1450                1455
Leu Gln Val Ala Val Leu Gly Pro Thr Gly Val Ala Glu Pro Val Glu
            1460                1465                1470
Val Arg Asp Asn Gly Asp Gly Thr His Thr Val His Tyr Thr Pro Ala
-1475 1480 1485
Thr Asp Gly Pro Tyr Thr Val Ala Val Lys Tyr Ala Asp Gln Glu Val
1490 1495 1500
Pro Arg Ser Pro Phe Lys Ile Lys Val Leu Pro Ala His Asp Ala Ser
1505 1510 1515 1520
Lys Val Arg Ala Ser Gly Pro Gly Leu Asn Ala Ser Gly Ile Pro Ala
1525 1530 1535
Ser Leu Pro Val Glu Phe Thr Ile Asp Ala Arg Asp Ala Gly Glu Gly
1540 1545 1550
Leu Leu Thr Val Gln Ile Leu Gly Pro Glu Gly Lys Pro Lys Lys Ala
1555 1560 1565
Asn Ile Arg Asp Asn Gly Asp Gly Thr Tyr Ala Val Ser Tyr Leu Pro
1570 1575 1580
Asp Met Ser Gly Arg Tyr Thr Ile Thr Ile Lys Tyr Gly Gly Asp Glu
1585 1590 1595 1600

Ile Pro Tyr Ser Pro Phe Arg Ile His Ala Leu Pro Thr Gly Asp Ala
1605 1610 1615
Ser Lys Cys Leu Val Thr Val Ser Ile Gly Gly His Gly Leu Gly Ala
1620 1625 1630
Cys Leu Gly Pro Arg Ile Gln Ile Gly Gln Glu Thr Val Ile Thr Val
1635 1640 1645
Asp Ala Lys Ala Ala Gly Glu Gly Lys Val Thr Cys Thr Val Ser Thr
1650 1655 1660
Pro Asp Gly Ala Glu Leu Asp Val Asp Val Val Glu Asn His Asp Gly
1665 1670 1675 1680
Thr Phe Asp Ile Tyr Tyr Thr Ala Pro Glu Pro Gly Lys Tyr Val Ile
1685 1690 1695
Thr Ile Arg Phe Gly Gly Glu His Ile Pro Asn Ser Pro Phe His Val
1700 1705 1710
Leu Ala Cys Asp Pro Leu Pro His Glu Glu Glu Pro Ser Glu Val Pro
1715 1720 1725
Gln Leu Arg Gln Pro Tyr Ala Pro Pro Arg Pro Gly Ala Arg Pro Thr
1730 1735 1740
His Trp Ala Thr Glu Glu Pro Val Val Pro Val Glu Pro Met Glu Ser
1745 1750 1755 1760
Met Leu Arg Pro Phe Asn Leu Val Ile Pro Phe Ala Val Gln Lys Gly
1765 1770 1775
Glu Leu Thr Gly Glu Val Arg Met Pro Ser Gly Lys Thr Ala Arg Pro
1780 1785 1790
Asn Ile Thr Asp Asn Lys Asp Gly Thr Ile Thr Val Arg Tyr Ala Pro
1795 1800 1805
Thr Glu Lys Gly Leu His Gln Met Gly Ile Lys Tyr Asp Gly Asn His

1810                1815                1820

Ile Pro Gly Ser Pro Leu Gln Phe Tyr Val Asp Ala Ile Asn Ser Arg

1825                1830                1835                1840

His Val Ser Ala Tyr Gly Pro Gly Leu Ser His Gly Met Val Asn Lys

                1845                1850                1855

Pro Ala Thr Phe Thr Ile Val Thr Lys Asp Ala Gly Glu Gly Gly Leu

            1860                1865                1870

Ser Leu Ala Val Glu Gly Pro Ser Lys Ala Glu Ile Thr Cys Lys Asp

        1875                1880                1885

Asn Lys Asp Gly Thr Cys Thr Val Ser Tyr Leu Pro Thr Ala Pro Gly

    1890                1895                1900

Asp Tyr Ser Ile Ile Val Arg Phe Asp Asp Lys His Ile Pro Gly Ser

1905                1910                1915                1920

Pro Phe Thr Ala Lys Ile Thr Gly Asp Asp Ser Met Arg Thr Ser Gln

                1925                1930                1935

Leu Asn Val Gly Thr Ser Thr Asp Val Ser Leu Lys Ile Thr Glu Ser

            1940                1945                1950

Asp Leu Ser Gln Leu Thr Ala Ser Ile Arg Ala Pro Ser Gly Asn Glu

        1955                1960                1965

Glu Pro Cys Leu Leu Lys Arg Leu Pro Asn Arg His Ile Gly Ile Ser

    1970                1975                1980

Phe Thr Pro Lys Glu Val Gly Glu His Val Val Ser Val Arg Lys Ser

1985                1990                1995                2000

Gly Lys His Val Thr Asn Ser Pro Phe Lys Ile Leu Val Gly Pro Ser

                2005                2010                2015

Glu Ile Gly Asp Ala Ser Lys Val Arg Val Trp Gly Lys Gly Leu Ser

2020 2025 2030
Glu Gly His Thr Phe Gln Val Ala Glu Phe Ile Val Asp Thr Arg Asn
2035 2040 2045
Ala Gly Tyr Gly Gly Leu Gly Leu Ser Ile Glu Gly Pro Ser Lys Val
2050 2055 2060
Asp Ile Asn Cys Glu Asp Met Glu Asp Gly Thr Cys Lys Val Thr Tyr
2065 2070 2075 2080
Cys Pro Thr Glu Pro Gly Thr Tyr Ile Ile Asn Ile Lys Phe Ala Asp
2085 2090 2095
Lys His Val Pro Gly Ser Pro Phe Thr Val Lys Val Thr Gly Glu Gly
2100 2105 2110
Arg Met Lys Glu Ser Ile Thr Arg Arg Arg Gln Ala Pro Ser Ile Ala
2115 2120 2125
Thr Ile Gly Ser Thr Cys Asp Leu Asn Leu Lys Ile Pro Gly Asn Trp
2130 2135 2140
Phe Gln Met Val Ser Ala Gln Glu Arg Leu Thr Arg Thr Phe Thr Arg
2145 2150 2155 2160
Ser Ser His Thr Tyr Thr Arg Thr Glu Arg Thr Glu Ile Ser Lys Thr
2165 2170 2175
Arg Gly Gly Glu Thr Lys Pro Glu Val Arg Val Glu Glu Ser Thr Gln
2180 2185 2190
Val Gly Gly Asp Pro Phe Pro Ala Val Phe Gly Asp Phe Leu Gly Arg
2195 2200 2205
Glu Arg Leu Gly Ser Phe Gly Ser Ile Thr Arg Gln Gln Glu Gly Glu
2210 2215 2220
Ala Ser Ser Gln Asp Met Thr Ala Gln Val Thr Ser Pro Ser Gly Lys
2225 2230 2235 2240

Val Glu Ala Ala Glu Ile Val Glu Gly Glu Asp Ser Ala Tyr Ser Val
2245 2250 2255
Arg Phe Val Pro Gln Glu Met Gly Pro His Thr Val Ala Val Lys Tyr
2260 2265 2270
Arg Gly Gln His Val Pro Gly Ser Pro Phe Gln Phe Thr Val Gly Pro
2275 2280 2285
Leu Gly Glu Gly Gly Ala His Lys Val Arg Ala Gly Arg Ala Gly Leu
2290 2295 2300
Glu Arg Gly Val Ala Gly Val Pro Ala Glu Phe Ser Ile Trp Thr Arg
2305 2310 2315 2320
Glu Ala Gly Ala Gly Gly Leu Ser Ile Ala Val Glu Gly Pro Ser Lys
2325 2330 2335
Ala Glu Ile Ala Phe Glu Asp Arg Lys Asp Gly Ser Cys Gly Val Ser
2340 2345 2350
Tyr Val Val Gln Glu Pro Gly Asp Tyr Glu Val Ser Ile Lys Phe Asn
2355 2360 2365
Asp Glu His Ile Pro Asp Ser Pro Phe Val Val Pro Val Ala Ser Leu
2370 2375 2380
Ser Asp Asp Ala Arg Arg Leu Thr Val Thr Ser Leu Gln Glu Thr Gly
2385 2390 2395 2400
Leu Lys Val Asn Gln Pro Ala Ser Phe Ala Val Gln Leu Asn Gly Ala
2405 2410 2415
Arg Gly Val Ile Asp Ala Arg Val His Thr Pro Ser Gly Ala Val Glu
2420 2425 2430
Glu Cys Tyr Val Ser Glu Leu Asp Ser Asp Lys His Thr Ile Arg Phe
2435 2440 2445

Ile Pro His Glu Asn Gly Val His Ser Ile Asp Val Lys Phe Asn Gly
2450 2455 2460
Ala His Ile Pro Gly Ser Pro Phe Lys Ile Arg Val Gly Glu Gln Ser
2465 2470 2475 2480
Gln Ala Gly Asp Pro Gly Leu Val Ser Ala Tyr Gly Pro Gly Leu Glu
2485 2490 2495
Gly Gly Thr Thr Gly Val Ser Ser Glu Phe Ile Val Asn Thr Leu Asn
2500 2505 2510
Ala Gly Ser Gly Ala Leu Ser Val Thr Ile Asp Gly Pro Ser Lys Val
2515 2520 2525
Gln Leu Asp Cys Arg Glu Cys Pro Glu Gly His Val Val Thr Tyr Thr
2530 2535 2540
Pro Met Ala Pro Gly Asn Tyr Leu Ile Ala Ile Lys Tyr Gly Gly Pro
2545 2550 2555 2560
Gln His Ile Val Gly Ser Pro Phe Lys Ala Lys Val Thr Gly Pro Arg
2565 2570 2575
Leu Ser Gly Gly His Ser Leu His Glu Thr Ser Thr Val Leu Val Glu
2580 2585 2590
Thr Val Thr Lys Ser Ser Ser Ser Arg Gly Ser Ser Tyr Ser Ser Ile
2595 2600 2605
Pro Lys Phe Ser Ser Asp Ala Ser Lys Val Val Thr Arg Gly Pro Gly
2610 2615 2620
Leu Ser Gln Ala Phe Val Gly Gln Lys Asn Ser Phe Thr Val Asp Cys
2625 2630 2635 2640
Ser Lys Ala Gly Thr Asn Met Met Met Val Gly Val His Gly Pro Lys
2645 2650 2655
Thr Pro Cys Glu Glu Val Tyr Val Lys His Met Gly Asn Arg Val Tyr

2660 2665 2670
Asn Val Thr Tyr Thr Val Lys Glu Lys Gly Asp Tyr Ile Leu Ile Val
2675 2680 2685
Lys Trp Gly Asp Glu Ser Val Pro Gly Ser Pro Phe Lys Val Lys Val
2690 2695 2700
Pro
2705

<210> 45
<211> 2016
<212> DNA
<213> Homo sapiens
<220>
<221> CDS
<222> (210)..(1352)
<400> 45
   ggcacgagga gcagcgcccg cgccctccgc gccttctccg ccgggacctc gagcgaaaga 60
   cgcccgcccg ccgcccagcc ctcgcctccc tgcccaccgg gcccaccgcg ccgccacccc 120
   gaccccgctg cgcacggcct gtccgctgca caccagcttg ttggcgtctt cgtcgccgcg 180
   ctcgccccgg gctactcctg cgcgccaca atg agc tcc cgc atc gcc agg gcg 233
   ctc gcc tta gtc gtc acc ctt ctc cac ttg acc agg ctg gcg ctc tcc 281
   acc tgc ccc gct gcc tgc cac tgc ccc ctg gag gcg ccc aag tgc gcg 329
   ccg gga gtc ggg ctg gtc cgg gac ggc tgc ggc tgc tgt aag gtc tgc 377
   gcc aag cag ctc aac gag gac tgc agc aaa acg cag ccc tgc gac cac 425
   acc aag ggg ctg gaa tgc aac ttc ggc gcc agc tcc acc gct ctg aag 473
   ggg atc tgc aga gct cag tca gag ggc aga ccc tgt gaa tat aac tcc 521
   aga atc tac caa aac ggg gaa agt ttc cag ccc aac tgt aaa cat cag 569
   tgc aca tgt att gat ggc gcc gtg ggc tgc att cct ctg tgt ccc caa 617
   gaa cta tct ctc ccc aac ttg ggc tgt ccc aac cct cgg ctg gtc aaa 665
   gtt acc ggg cag tgc tgc gag gag tgg gtc tgt gac gag gat agt atc 713
   aag gac ccc atg gag gac cag gac ggc ctc ctt ggc aag gag ctg gga 761
   ttc gat gcc tcc gag gtg gag ttg acg aga aac aat gaa ttg att gca 809
   gtt gga aaa ggc agc tca ctg aag cgg ctc cct gtt ttt gga atg gag 857
   cct cgc atc cta tac aac cct tta caa ggc cag aaa tgt att gtt caa 905
   aca act tca tgg tcc cag tgc tca aag acc tgt gga act ggt atc tcc 953
   aca cga gtt acc aat gac aac cct gag tgc cgc ctt gtg aaa gaa acc 1001
   cgg att tgt gag gtg cgg cct tgt gga cag cca gtg tac agc agc ctg 1049
   aaa aag ggc aag aaa tgc agc aag acc aag aaa tcc ccc gaa cca gtc 1097
   agg ttt act tac gct gga tgt ttg agt gtg aag aaa tac cgg ccc aag 1145
   tac tgc ggt tcc tgc gtg gac ggc cga tgc tgc acg ccc cag ctg acc 1193
   agg act gtg aag atg cgg ttc cgc tgc gaa gat ggg gag aca ttt tcc 1241
   aag aac gtc atg atg atc cag tcc tgc aaa tgc aac tac aac tgc ccg 1289
   cat gcc aat gaa gca gcg ttt ccc ttc tac agg ctg ttc aat gac att 1337
   cac aaa ttt agg gac taaatgctac ctgggtttcc agggcacacc tagacaaaca 1392
   agggagaaga gtgtcagaat cagaatcatg gagaaaatgg gcgggggtgg tgtgggtgat 1452
   gggactcatt gtagaaagga agccttgctc attcttgagg agcattaagg tatttcgaaa 1512
   ctgccaaggg tgctggtgcg gatggacact aatgcagcca cgattggaga atactttgct 1572
   tcatagtatt ggagcacatg ttactgcttc attttggagc ttgtggagtt gatgactttc 1632
   tgttttctgt ttgtaaatta tttgctaagc atattttctc taggcttttt tccttttggg 1692
   gttctacagt cgtaaaagag ataataagat tagttggaca gtttaaagct tttattcgtc 1752
   ctttgacaaa agtaaatggg agggcattcc atcccttcct gaagggggac actccatgag 1812
   tgtctgtgag aggcagctat ctgcactcta aactgcaaac agaaatcagg tgttttaaga 1872
   ctgaatgttt tatttatcaa aatgtagctt ttggggaggg aggggaaatg taatactgga 1932
   ataatttgta aatgatttta attttatatt cagtgaaaag atttatttta tggaattaac 1992
   catttaataa agaaatattt acct 2016

<210> 46

<211> 381

<212> PRT

<213> Homo sapiens

<400> 46

<210> 47

<211> 2743

<212> DNA

<213> Homo sapiens

<220>

<221> CDS

<222> (240)..(2387)

<400> 47
gaattccgca gggcgcgggc accggggcgc gggcagggct cggagccacc gcgcaggtcc 60
tagggccgcg gccgggcccc gccacgcgcg cacacgcccc tcgatgactt tcctccgggg 120
cgcgcggcgc tgagcccggg gcgagggctg tcttcccgga gacccgaccc cggcagcgcg 180
gggcggccac ttctcctgtg cctccgcccg ctgctccact ccccgccgcc gccgcgcgg 239
atg cca agc acc agc ttt cca gtc cct tcc aag ttt cca ctt ggc cct 287
gcg gct gcg gtc ttc ggg aga gga gaa act ttg ggg ccc gcg ccg cgc 335
gcc ggc ggc acc atg aag tca gcg gag gaa gaa cac tat ggc tat gca 383
tcc tcc aac gtc agc ccc gcc ctg ccg ctc ccc acg gcg cac tcc acc 431
ctg ccg gcc ccg tgc cac aac ctt cag acc tcc aca ccg ggc atc atc 479
ccg ccg gcg gat cac ccc tcg ggg tac gga gca gct ttg gac ggt ggg 527
ccc gcg ggc tac ttc ctc tcc tcc ggc cac acc agg cct gat ggg gcc 575
cct gcc ctg gag agt cct cgc atc gag ata acc tcg tgc ttg ggc ctg 623
tac cac aac aat aac cag ttt ttc cac gat gtg gag gtg gaa gac gtc 671
ctc cct agc tcc aaa cgg tcc ccc tcc acg gcc acg ctg agt ctg ccc 719
agc ctg gag gcc tac aga gac ccc tcg tgc ctg agc ccg gcc agc agc 767
ctg tcc tcc cgg agc tgc aac tca gag gcc tcc tcc tac gag tcc aac 815
tac tcg tac ccg tac gcg tcc ccc cag acg tcg cca tgg cag tct ccc 863
tgc gtg tct ccc aag acc acg gac ccc gag gag ggc ttt ccc cgc ggg 911
ctg ggg gcc tgc aca ctg ctg ggt tcc ccg cag cac tcc ccc tcc acc 959
tcg ccc cgc gcc agc gtc act gag gag agc tgg ctg ggt gcc cgc tcc 1007
tcc aga ccc gcg tcc cct tgc aac aag agg aag tac agc ctc aac ggc 1055
cgg cag ccg ccc tac tca ccc cac cac tcg ccc acg ccg tcc ccg cac 1103
ggc tcc ccg cgg gtc agc gtg acc gac gac tcg tgg ttg ggc aac acc 1151
acc cag tac acc agc tcg gcc atc gtg gcc gcc atc aac gcg ctg acc 1199
acc gac agc agc ctg gac ctg gga gat ggc gtc cct gtc aag tcc cgc 1247
aag acc acc ctg gag cag ccg ccc tca gtg gcg ctc aag gtg gag ccc 1295
gtc ggg gag gac ctg ggc agc ccc ccg ccc ccg gcc gac ttc gcg ccc 1343
gaa gac tac tcc tct ttc cag cac atc agg aag ggc ggc ttc tgc gac 1391
cag tac ctg gcg gtg ccg cag cac ccc tac cag tgg gcg aag ccc aag 1439
ccc ctg tcc cct acg tcc tac atg agc ccg acc ctg ccc gcc ctg gac 1487
tgg cag ctg ccg tcc cac tca ggc ccg tat gag ctt cgg att gag gtg 1535
cag ccc aag tcc cac cac cga gcc cac tac gag acg gag ggc agc cgg 1583
ggg gcc gtg aag gcg tcg gcc gga gga cac ccc atc gtg cag ctg cat 1631
ggc tac ttg gag aat gag ccg ctg atg ctg cag ctt ttc att ggg acg 1679
gcg gac gac cgc ctg ctg cgc ccg cac gcc ttc tac cag gtg cac cgc 1727
atc aca ggg aag acc gtg tcc acc acc agc cac gag gct atc ctc tcc 1775
aac acc aaa gtc ctg gag atc cca ctc ctg ccg gag aac agc atg cga 1823
gcc gtc att gac tgt gcc gga atc ctg aaa ctc aga aac tcc gac att 1871
gaa ctt cgg aaa gga gag acg gac atc ggg agg aag aac aca cgg gta 1919
cgg ctg gtg ttc cgc gtt cac gtc ccg caa ccc agc ggc cgc acg ctg 1967
tcc ctg cag gtg gcc tcc aac ccc atc gaa tgc tcc cag cgc tca gct 2015
cag gag ctg cct ctg gtg gag aag cag agc acg gac agc tat ccg gtc 2063
gtg ggc ggg aag aag atg gtc ctg tct ggc cac aac ttc ctg cag gac 2111
tcc aag gtc att ttc gtg gag aaa gcc cca gat ggc cac cat gtc tgg 2159
gag atg gaa gcg aaa act gac cgg gac ctg tgc aag ccg aat tct ctg 2207
gtg gtt gag atc ccg cca ttt cgg aat cag agg ata acc agc ccc gtt 2255
cac gtc agt ttc tac gtc tgc aac ggg aag aga aag cga agc cag tac 2303
cag cgt ttc acc tac ctt ccc gcc aac ggt aac gcc atc ttt cta acc 2351
gta agc cgt gaa cat gag cgc gtg ggg tgc ttt ttc taaagacgca 2397
gaaacgacgt cgccgtaaag cagcgtggcg tgttgcacat ttaactgtgt gatgtcccgt 2457
tagtgagacc gagccatcga tgccctgaaa aggaaaggaa aagggaagct tcggatgcat 2517
tttccttgat ccctgttggg ggtggggggc gggggttgca tactcagata gtcacggtta 2577
ttttgcttct tgcgaatgta taacagccaa ggggaaaaca tggctcttct gctccaaaaa 2637
actgaggggg tcctggtgtg catttgcacc ctaaagctgc ttacggtgaa aaggcaaata 2697
ggtatagcta ttttgcaggc acctttagga ataaactttg ctttta 2743

<210> 48
<211> 716
<212> PRT
<213> Homo sapiens
<400> 48
<210> 49
<211> 23[?]3
<212> DNA
<213> Homo sapiens
<220>
<221> CDS
<222> (241)..(1482)
<400> 49
cgccgagacg agcagcggcc gagcgagcgc gggcgcgggc gcaccgaggc gagggaggcg 60
gggaagcccc gccgccgccg ccccgcccgc cccttccccc gccgcccgcc ccctctcccc 120
ccgcccgctc gccgccttcc tccctctgcc ttccttcccc acggccggcc gcctcctcgc 180
ccgcccgccc gcagccgagg agccgaggcc gccgcggccg tggcggcgga gccctcagcc 240
atg gcc tcg ggc gac acc ctc tac atc gcc acg gac ggc tcg gag atg 288

1               5                   10                  15
ccg gcc gag atc gtg gag ctg cac gag atc gag gtg gag acc atc ccg 336


            20                  25                  30
gtg gag acc atc gag acc aca gtg gtg ggc gag gag gag gag gag gac 384

        35                  40                  45
gac gac gac gag gac ggc ggc ggt ggc gac cac ggc ggc ggg ggc ggc 432

    50                  55                  60
cac ggg cac gcc ggc cac cac cac cac cac cat cac cac cac cac cac 480

65                  70                  75                  80
ccg ccc atg atc gct ctg cag ccg ctg gtc acc gac gac ccg acc cag 528

                85                  90                  95
gtg cac cac cac cag gag gtg atc ctg gtg cag acg cgc gag gag gtg 576

            100                 105                 110
gtg ggc ggc gac gac tcg gac ggg ctg cgc gcc gag gac ggc ttc gag 624

        115                 120                 125
gat cag att ctc atc ccg gtg ccc gcg ccg gcc ggc ggc gac gac gac 672

    130                 135                 140
tac att gaa caa acg ctg gtc acc gtg gcg gcg gcc ggc aag agc ggc 720

145                 150                 155                 160
ggc ggc ggc tcg tcg tcg tcg gga ggc ggc cgc gtc aag aag ggc ggc 768


                165                 170                 175
ggc aag aag agc ggc aag aag agt tac ctc agc ggc ggg gcc ggc gcg 816

            180                 185                 190
gcg ggc ggg cgc ggc gcc gac ccg ggc aac aag aag tgg gag cag aag 864

        195                 200                 205
cag gtg cag atc aag acc ctg gag ggc gag ttc tcg gtc acc atg tgg 912

    210                 215                 220
tcc tca gat gaa aaa aaa gat att gac cat gag aca gtg gtt gaa gaa 960

225                 230                 235                 240
cag atc att gga gag aac tca cct cct gat tat tca gaa tat atg aca 1008

                245                 250                 255
gga aag aaa ctt cct cct gga gga ata cct ggc att gac ctc tca gat 1056

            260                 265                 270
ccc aaa caa ctg gca gaa ttt gct aga atg aag cca aga aaa att aaa 1104

        275                 280                 285
gaa gat gat gct cca aga aca ata gct tgc cct cat aaa ggc tgc aca 1152

    290                 295                 300

aag atg ttc agg gat aac tcg gcc atg aga aaa cat ctg cac acc cac 1200

305                 310                 315                 320
ggt ccc aga gtc cac gtc tgt gca gaa tgt ggc aaa gct ttt gtt gag 1248

                325                 330                 335
agt tca aaa cta aaa cga cac caa ctg gtt cat act gga gag aag ccc 1296

            340                 345                 350
ttt cag tgc acg ttc gaa ggc tgt ggg aaa cgc ttt tca ctg gac ttc 1344

        355                 360                 365
aat ttg cgc aca cat gtg cga atc cat acc gga gac agg ccc tat gtg 1392

    370                 375                 380
tgc ccc ttc gat ggt tgt aat aag aag ttt gct cag tca act aac ctg 1440

385                 390                 395                 400
aaa tct cac atc tta aca cat gct aag gcc aaa aac aac cag 1482

                405                 410
tgaaaagaag agagaagacc cttctcgacc acgggaagca tcttccagaa gtgtgattgg 1542
gaataaatat gcctctcctt tgtatattat ttctaggaag aattttaaaa atgaatccta 1602
cacacctaag ggacatgttt tgataaagta gtaaaaatta aaaaaaaaaa actttactaa 1662
gatgacattg ctaagatgct ctatcttgct ctgtaatctc gtttcaaaaa cacagtgttt 1722
ttgtaaagtg tggtcccaac aggaggacaa ttcatgaact tcgcatcaaa agacaattct 1782
ttatacaaca gtgctaaaaa tgggacttct tttcacattc ttataaatat gaagctcacc 1842

tgttgcttac aattttttta attttgtatt ttccaagtgt gcatattgta cacttttttg 1902

gggatatgct tagtaatgct acgtgtgatt tttctggagg ttgataactt tgcttgcagt 1962

agattttctt taaaagaatg ggcagttaca tgcatacttc aaaagtattt tcctgtaaaa 2022

aaaaaaaaag ttatataggt tttgtttgct atcttaattt tggttgtatt ctttgatgtt 2082

aacacatttt gtataattgt atcgtatagc tgtattgaat catgtagtat caaatattag 2142

atgtgattta atagtgttaa tcaatttaaa cccattttag tcactttttt tttccaaaaa 2202

aatactgcca gatgctgatg ttcagtgtaa tttctttgcc tgttcagtta cagaaagtgg 2262

tgctcagttg tagaatgtat tgtacctttt aacacctgat gtgtacatcc catgtaacag 2322

aaagggcaac aataaaatag caatcctaaa g 2353

<210> 50

<211> 414

<212> PRT

<213> Homo sapiens

<400> 50

<210> 51
<211> 1229
<212> DNA
<213> Homo sapiens
<220>
<221> CDS
<222> (222)..(950)
<400> 51
ataacgattt caagagctgc acttaagcat ctagaatttt ctgcgtcaca cctcttgaga 60
gaagagactg gctccaggtc tgactcagtc cactacaagc tagacggtct tcttaaagca 120
ccaacattac ttgagtcttt ggataaaatt gagaaaagag tctacaagta ttgtggactc 180
tacaggaggc aggaggctga caactggcag taaagacaaa g atg tca ggc ctg cgg 236

Met Ser Gly Leu Arg
1               5
ccc ggc act caa gtg gac cct gag att gag ctt ttt gta aag gct gga 284
Pro Gly Thr Gln Val Asp Pro Glu Ile Glu Leu Phe Val Lys Ala Gly
                10                  15                  20
agt gat gga gag agt att gga aac tgt ccc ttt tgc caa cgc ctt ttc 332
Ser Asp Gly Glu Ser Ile Gly Asn Cys Pro Phe Cys Gln Arg Leu Phe
            25                  30                  35
atg atc ctc tgg ctt aaa gga gtt aaa ttt aat gtg aca act gtt gac 380
Met Ile Leu Trp Leu Lys Gly Val Lys Phe Asn Val Thr Thr Val Asp
        40                  45                  50
atg acc aga aag cct gaa gaa cta aag gac tta gcc cca ggt acc aat 428
Met Thr Arg Lys Pro Glu Glu Leu Lys Asp Leu Ala Pro Gly Thr Asn
    55                  60                  65
cct ccg ttc ctg gtg tat aac aag gag ttg aaa aca gac ttc att aaa 476
Pro Pro Phe Leu Val Tyr Asn Lys Glu Leu Lys Thr Asp Phe Ile Lys
70                  75                  80                  85
att gag gag ttt tta gaa caa acc ctg gct cct cca agg tac cct cac 524
Ile Glu Glu Phe Leu Glu Gln Thr Leu Ala Pro Pro Arg Tyr Pro His
                90                  95                  100
ctg agt ccc aag tac aag gag tct ttt gat gtg ggc tgt aac ctc ttt 572
Leu Ser Pro Lys Tyr Lys Glu Ser Phe Asp Val Gly Cys Asn Leu Phe
            105                 110                 115
gcc aag ttt tct gca tac att aag aat aca caa aag gag gca aat aag 620
Ala Lys Phe Ser Ala Tyr Ile Lys Asn Thr Gln Lys Glu Ala Asn Lys
        120                 125                 130
aat ttt gaa aaa tct ctg ctc aaa gaa ttc aag cgt ctg gat gac tac 668

Asn Phe Glu Lys Ser Leu Leu Lys Glu Phe Lys Arg Leu Asp Asp Tyr
    135                 140                 145
tta aac acc cca ctt ctg gat gaa att gat cca gac agt gct ggg gaa 716
Leu Asn Thr Pro Leu Leu Asp Glu Ile Asp Pro Asp Ser Ala Gly Glu
150                 155                 160                 165
ccc cca gtt tcc aga aga cta ttc ttg gat ggg gac cag cta aca ctg 764
Pro Pro Val Ser Arg Arg Leu Phe Leu Asp Gly Asp Gln Leu Thr Leu
                170                 175                 180
gct gat tgt agc ttg tta ccc aag ctg aac att att aaa gtt gct gcc 812
Ala Asp Cys Ser Leu Leu Pro Lys Leu Asn Ile Ile Lys Val Ala Ala
            185                 190                 195
aag aaa tat cgt gac ttt gac att cca gca gaa ttc tca gga gtc tgg 860
Lys Lys Tyr Arg Asp Phe Asp Ile Pro Ala Glu Phe Ser Gly Val Trp
        200                 205                 210
cgt tat ctc cac aat gcc tat gcc cgt gaa gaa ttt acc cac acg tgt 908
Arg Tyr Leu His Asn Ala Tyr Ala Arg Glu Glu Phe Thr His Thr Cys
    215                 220                 225
cct gaa gac aaa gaa att gaa aat act tac gca aat gtg gct 950
Pro Glu Asp Lys Glu Ile Glu Asn Thr Tyr Ala Asn Val Ala
230                 235                 240
taaacagaag agttaggaga gctcttacag gagaaaaggc tatatttgtg atcagatttt 1010
acttattgac atattagaaa ggtttttgca aataagaata tgaaaaatac tgtttcttct 1070
atccaactct cttatgaaaa ggaactctgt attttctatt agccataaat aatctgtcca 1130
ctgtatttta caggtcttca tactttactt taattttctt tatctgtatg gcaaaccact 1190
gcaatcctga atgacatgga aagcatcaca aaaaaaaaa 1229

<210> 52

<211> 243

<212> PRT

<213> Homo sapiens

<400> 52

Met Ser Gly Leu Arg Pro Gly Thr Gln Val Asp Pro Glu Ile Glu Leu

1               5                   10                  15

Phe Val Lys Ala Gly Ser Asp Gly Glu Ser Ile Gly Asn Cys Pro Phe

            20                  25                  30

Cys Gln Arg Leu Phe Met Ile Leu Trp Leu Lys Gly Val Lys Phe Asn

        35                  40                  45

Val Thr Thr Val Asp Met Thr Arg Lys Pro Glu Glu Leu Lys Asp Leu

    50                  55                  60

Ala Pro Gly Thr Asn Pro Pro Phe Leu Val Tyr Asn Lys Glu Leu Lys

65                  70                  75                  80

Thr Asp Phe Ile Lys Ile Glu Glu Phe Leu Glu Gln Thr Leu Ala Pro

                85                  90                  95

Pro Arg Tyr Pro His Leu Ser Pro Lys Tyr Lys Glu Ser Phe Asp Val

            100                 105                 110

Gly Cys Asn Leu Phe Ala Lys Phe Ser Ala Tyr Ile Lys Asn Thr Gln

        115                 120                 125

Lys Glu Ala Asn Lys Asn Phe Glu Lys Ser Leu Leu Lys Glu Phe Lys

    130                 135                 140

Arg Leu Asp Asp Tyr Leu Asn Thr Pro Leu Leu Asp Glu Ile Asp Pro

145                 150                 155                 160

Asp Ser Ala Gly Glu Pro Pro Val Ser Arg Arg Leu Phe Leu Asp Gly

                165                 170                 175

Asp Gln Leu Thr Leu Ala Asp Cys Ser Leu Leu Pro Lys Leu Asn Ile
            180                 185                 190
Ile Lys Val Ala Ala Lys Lys Tyr Arg Asp Phe Asp Ile Pro Ala Glu
        195                 200                 205
Phe Ser Gly Val Trp Arg Tyr Leu His Asn Ala Tyr Ala Arg Glu Glu
    210                 215                 220
Phe Thr His Thr Cys Pro Glu Asp Lys Glu Ile Glu Asn Thr Tyr Ala
225                 230                 235                 240
Asn Val Ala

<210> 53
<211> 4001
<212> DNA
<213> Homo sapiens
<220>
<221> CDS
<222> (2)..(316)
<400> 53
c cca ggt aat cct tat gtc aag gtg aac gtc tac tac ggc aga aag cgc 49
Pro Gly Asn Pro Tyr Val Lys Val Asn Val Tyr Tyr Gly Arg Lys Arg
1               5                   10                  15
att gcc aag aag aaa acc cat gtg aag aag tgc act ttg aac ccc atc 97
Ile Ala Lys Lys Lys Thr His Val Lys Lys Cys Thr Leu Asn Pro Ile
            20                  25                  30
ttc aat gaa tct ttc atc tac gac atc ccc act gac ctc ctg cct gat 145
Phe Asn Glu Ser Phe Ile Tyr Asp Ile Pro Thr Asp Leu Leu Pro Asp
        35                  40                  45

atc agc atc gag ttc ctc gtt atc gac ttc gat cgc acc acc aag aat 193
Ile Ser Ile Glu Phe Leu Val Ile Asp Phe Asp Arg Thr Thr Lys Asn
    50                  55                  60
gag gtg gtg ggg agg ctg atc ctg ggg gca cac agt gtc aca gcc agt 241
Glu Val Val Gly Arg Leu Ile Leu Gly Ala His Ser Val Thr Ala Ser
65                  70                  75                  80
ggt gct gaa cac tgg aga gag gtc tgc gag agc ccc cgc aag cct gtg 289
Gly Ala Glu His Trp Arg Glu Val Cys Glu Ser Pro Arg Lys Pro Val
                85                  90                  95
gcc aag tgg cac agt ctg agc gag tac taatcctgtt cttctctcct 336
Ala Lys Trp His Ser Leu Ser Glu Tyr
            100                 105
ctaatccccg ggggccaagc tggggaggga tgtggagggg aaaaagatga cagagaagtg 396
gactccaaac ctcattttag ttgtagaaga aaatttctta caaaacaaat tccacaaaga 456
acaccctata tgaccacagc tgcagatcag ttcttagcaa tgatgttttt ttttctgctt 516
tgcaaggcgc tagaatcttt tattttactt tatttttttt gaggtggagt ttcgctcttg 576
ttgcccgggc tggagtgcaa tggtgagatc tcaactcact gcaacctctg ccctccaggt 636
tcaagtgatt ctcctccctc agcctcccaa gtagctggga ttacaggcac ccacgagcat 696
gcccggctaa ttttttgtat tttcagtaga gatgggtttc accatgttgg ccaggctggt 756
ctcgaattcc agacctcagg tgatccaccc gcctcggcct cccaaagtgc tgggattaca 816
ggtgcgagcc accgtgcccg gcctctggtt ttgttttgtt tttttttttt aatgggggac 876
aaaagagagg gaaagacccc tataaatcta tatataacaa tgtaaccata tacttgcatg 936
tctaatacaa actgaagaaa ttagcctaac tgccaatatc aagttgcaga ttttaatcca 996
tggaaattgt gttttgtgct gaattgtatt tgctgattac ctgaaattgg cttcttttta 1056
ttgggcttct ctggagaatt tctcccactc cccacctctg cagaagaaaa ttttgctctt 1116
ataaaacctc atgttttcat cattcccatc ttttcttttt attgcctctt atatctctgc 1176
tctttgacct caaggtctag aggtctgcag taagccaaga aacaaaggtg gggtggatga 1236

ggcaaggttt gcaggagaaa gaggaattga gaaatggggt atttttgcta tcagctcttc 1296

tgctatgaag tagtaaaagg cagtctataa ttaactgaca gacctaactg aagcacagag 1356

aatacatcag acttatgcat ccaagacatc agaacttgga ttttatcaaa cttgatgact 1416

tctctaaaag gagctttgga aacttcaaat tcagctatag gatagtacca atgaacacat 1476

ccagctgatc ccaaaagctg ttttcaggta taaggacaag gagaggagac aagtgacgac 1536

agccattccc ctttgcagct atctactgta gtgacagcca tttcttggtt gatgggttgg 1596

aagtcatcag aggtttgaag aattacactg gcctttgttt ttctggaaat gccgaccatg 1656

gagatgcttt agagtcttct caaatagctt agatgttgta atgaggttag ctttgcttca 1716

taaaacaggg gccctcagaa gttctcctta aatttttcaa taaaaattta gctcttaaaa 1776

aaataacagt gtgactgagt gaatgaagat aagttggatt ctttcagagc attcttttcc 1836

tcaaaacgag ctgcataatt cttggaattt atgtcttacc acatggtgga gggatggagg 1896

aactacagga tgcaattctt cttctaccaa tgggcaatag aggttgagag agattcagca 1956

tctttctggg attagaattc aagtctcttt actcctacag cagctgtgtc tccaacgttg 2016

agactttgca gatggcacag actccatgga taataggtaa acttggggcc gggcgcagtg 2076

gctcacgcct gtaatcccag tattttggga ggccgaggtg gaaagatcgc ttgagcccag 2136

gagttcaaga ccagcctggg ctacatgacg aaactccatc tctatcaaaa atacaaaaaa 2196

ttaactaggt gtggtgctgc acgcctgtgg tcccagctat tcaggaggct gaggtgggag 2256

gatcatttga gcccagaggt agaggctgca gtgagccatg atcatgccac tgcactctgg 2316

gctgggtaac agagtgagat cctgtctcaa aaattaatta attaattaat taaaataaac 2376

taggtaaact tggataggca gtagatattt ttgcccacct gaggaggaac tcagtcaagc 2436

tgttgcttaa cagcttgatc cagggcgtga aaggttagtt gagactgaag tgttcacttc 2496

catagaagaa catcactttt aaccttgctt tggcgaaggg agtcggaaag ctgagtctct 2556

atggacgggg gggtgatctt gctttcagtg ttccctcagc ttttgtggat ttaaaaccat 2616

tctgctcccc ctaaaccttt tgtttgattt cagcccatgt tcttgacaat gcagagcaat 2676

tctgagcagt cacaaagcct actctctgtt cttgtccctg ccaaccccca ccccccataa 2736

tctgactcac aacttcacca tcagttgggg tcataccact agtctctgtc ctatacccca 2796

tgaaatgtaa atactgtatc ataagtagaa gaaaataatt tttgttttct aaaaatgcat 2856

tttgagatag tttaatgtaa atctgacagg agcattctga agccccatta ggaaaaaatt 2916

taaatggttc ctcttcatcg ccttaatgtc taaagatcag aaatcgctga gcaaacccgc 2976

ttttgtttcc ttcccagaaa caatgcaaaa caacaggtgg agatagtctg gtctttgccc 3036

tgctgtgtgt gcctctgtag ctcctcctga caaacgtctg ggaaaacagc ctcaccccac 3096

tctcctctct cttccccatt tccttgtagc tttattcctt gcatctttgg gtctactgag 3156

cagtgggtgc tgaggtgaca ggggaggaac cagttgttct gtagcctagg aactgcctca 3216

gtgtctttgc cagaaaaagg caaagaggcg gacagtgcag ggctcctccc tcctacctca 3276

ggcctgatcc atcgtgccct tgactttgcc gtctcaaagt ttcttagctg actttggctt 3336

tcacatttgt tctttccaga gctaactgat aagagtggag gaggaatgcc ttctcctaag 3396

agtcagttga aagaaagaca agagagtcac atcttagctt ttgcacaagg cattcgtggt 3456

caggaatagg ttagggaatg gtcacttctg attttccaac agttgctcct tctctgaaga 3516

gatcttgatt cctttgggaa gacaagaatt tttcttaata acaaaggtcc ctttatgagt 3576

tattccttct ttcagttcat ctcactggag cacagccaag atggacatgt ttatggacag 3636

tgctctagat gtgaaaacag atagaactgg tttgtgggac aggggcagct tgctcaggag 3696

agggaataac gcaggtccct tttcttggaa ggcttgtact atggccatga cagtgacatt 3756

gccctcacca tgatccctct ccaaagtggt tgtctttctt taccttgtgt cttctcttgt 3816

aaaaatgaaa ctcaaaaata aaataaatgt gtcaaatttt gaaaaaaaaa gaaaactgaa 3876

aaagctaaca tgaattgtgt gaaattgcat aatgctgtaa tgctaatcta caatatgtaa 3936

tgctatcttg tatgttgaat ttgttaatgc accacacaag tgcaaaataa agactgattc 3996

acatt 4001

<210> 54

<211> 105

<212> PRT

<213> Homo sapiens

<400> 54

Pro Gly Asn Pro Tyr Val Lys Val Asn Val Tyr Tyr Gly Arg Lys Arg
1               5                   10                  15
Ile Ala Lys Lys Lys Thr His Val Lys Lys Cys Thr Leu Asn Pro Ile
            20                  25                  30
Phe Asn Glu Ser Phe Ile Tyr Asp Ile Pro Thr Asp Leu Leu Pro Asp
        35                  40                  45
Ile Ser Ile Glu Phe Leu Val Ile Asp Phe Asp Arg Thr Thr Lys Asn
    50                  55                  60
Glu Val Val Gly Arg Leu Ile Leu Gly Ala His Ser Val Thr Ala Ser
65                  70                  75                  80
Gly Ala Glu His Trp Arg Glu Val Cys Glu Ser Pro Arg Lys Pro Val
                85                  90                  95
Ala Lys Trp His Ser Leu Ser Glu Tyr
            100                 105

<210> 55
<211> 952
<212> DNA
<213> Homo sapiens
<220>
<221> CDS
<222> (118)..(909)
<400> 55
tgaactctgg atgctgttag cctgagactc aggaagacaa cttctgcagg gtcactccct 60
ggcttctgga ggaaagagaa ggagggcagt gctccagtgg tacagaagtg agacata    117
atg gaa tca ggc ttc acc tcc aag gac acc tat cta agc cat ttt aac   165
Met Glu Ser Gly Phe Thr Ser Lys Asp Thr Tyr Leu Ser His Phe Asn

1                   5                   10                  15

cct cgg gat tac cta gaa aaa tat tac aag ttt ggt tct agg cac tct   213

Pro Arg Asp Tyr Leu Glu Lys Tyr Tyr Lys Phe Gly Ser Arg His Ser

                20                  25                  30

gca gaa agc cag att ctt aag cac ctt ctg aaa aat ctt ttc aag ata   261

Ala Glu Ser Gln Ile Leu Lys His Leu Leu Lys Asn Leu Phe Lys Ile

            35                  40                  45

ttc tgc cta gac ggt gtg aag gga gac ctg ctg att gac atc ggc tct   309

Phe Cys Leu Asp Gly Val Lys Gly Asp Leu Leu Ile Asp Ile Gly Ser

        50                  55                  60

ggc ccc act atc tat cag ctc ctc tct gct tgt gaa tcc ttt aag gag   357

Gly Pro Thr Ile Tyr Gln Leu Leu Ser Ala Cys Glu Ser Phe Lys Glu

65                  70                  75                  80

atc gtc gtc act gac tac tca gac cag aac ctg cag gag ctg gag aag   405

Ile Val Val Thr Asp Tyr Ser Asp Gln Asn Leu Gln Glu Leu Glu Lys

                85                  90                  95

tgg ctg aag aaa gag cca gag gcc ttt gac tgg tcc cca gtg gtg acc   453

Trp Leu Lys Lys Glu Pro Glu Ala Phe Asp Trp Ser Pro Val Val Thr

            100                 105                 110

tat gtg tgt gat ctt gaa ggg aac aga gtc aag ggt cca gag aag gag   501

Tyr Val Cys Asp Leu Glu Gly Asn Arg Val Lys Gly Pro Glu Lys Glu

        115                 120                 125

gag aag ttg aga cag gcg gtc aag cag gtg ctg aag tgt gat gtg act   549

Glu Lys Leu Arg Gln Ala Val Lys Gln Val Leu Lys Cys Asp Val Thr

    130                 135                 140

cag agc cag cca ctg ggg gcc gtc ccc tta ccc ccg gct gac tgc gtg   597

Gln Ser Gln Pro Leu Gly Ala Val Pro Leu Pro Pro Ala Asp Cys Val

145                 150                 155                 160

ctc agc aca ctg tgt ctg gat gcc gcc tgc cca gac ctc ccc acc tac    645

Leu Ser Thr Leu Cys Leu Asp Ala Ala Cys Pro Asp Leu Pro Thr Tyr

                165                 170                 175

tgc agg gcg ctc agg aac ctc ggc agc cta ctg aag cca ggg ggc ttc    693

Cys Arg Ala Leu Arg Asn Leu Gly Ser Leu Leu Lys Pro Gly Gly Phe

            180                 185                 190

ctg gtg atc atg gat gcg ctc aag agc agc tac tac atg att ggt gag    741

Leu Val Ile Met Asp Ala Leu Lys Ser Ser Tyr Tyr Met Ile Gly Glu

        195                 200                 205

cag aag ttc tcc agc ctc ccc ctg ggc cgg gag gca gta gag gct gct    789

Gln Lys Phe Ser Ser Leu Pro Leu Gly Arg Glu Ala Val Glu Ala Ala

    210                 215                 220

gtg aaa gag gct ggc tac aca atc gaa tgg ttt gag gtg atc tcg caa    837

Val Lys Glu Ala Gly Tyr Thr Ile Glu Trp Phe Glu Val Ile Ser Gln

225                 230                 235                 240

agt tat tct tcc acc atg gcc aac aac gaa gga ctt ttc tcc ctg gtg    885

Ser Tyr Ser Ser Thr Met Ala Asn Asn Glu Gly Leu Phe Ser Leu Val

                245                 250                 255

gcg agg aag ctg agc aga ccc ctg tgatgcctgt gacctcaatt aaagcaattc    939

Ala Arg Lys Leu Ser Arg Pro Leu

            260

ctttgacctg tca                                                      952

<210> 56

<211> 264

<212> PRT

<213> Homo sapiens

<400> 56

Met Glu Ser Gly Phe Thr Ser Lys Asp Thr Tyr Leu Ser His Phe Asn

1               5                   10                  15

Pro Arg Asp Tyr Leu Glu Lys Tyr Tyr Lys Phe Gly Ser Arg His Ser

            20                  25                  30

Ala Glu Ser Gln Ile Leu Lys His Leu Leu Lys Asn Leu Phe Lys Ile

        35                  40                  45

Phe Cys Leu Asp Gly Val Lys Gly Asp Leu Leu Ile Asp Ile Gly Ser

    50                  55                  60

Gly Pro Thr Ile Tyr Gln Leu Leu Ser Ala Cys Glu Ser Phe Lys Glu

65                  70                  75                  80

Ile Val Val Thr Asp Tyr Ser Asp Gln Asn Leu Gln Glu Leu Glu Lys

                85                  90                  95

Trp Leu Lys Lys Glu Pro Glu Ala Phe Asp Trp Ser Pro Val Val Thr

            100                 105                 110

Tyr Val Cys Asp Leu Glu Gly Asn Arg Val Lys Gly Pro Glu Lys Glu

        115                 120                 125

Glu Lys Leu Arg Gln Ala Val Lys Gln Val Leu Lys Cys Asp Val Thr

    130                 135                 140

Gln Ser Gln Pro Leu Gly Ala Val Pro Leu Pro Pro Ala Asp Cys Val

145                 150                 155                 160

Leu Ser Thr Leu Cys Leu Asp Ala Ala Cys Pro Asp Leu Pro Thr Tyr

                165                 170                 175

Cys Arg Ala Leu Arg Asn Leu Gly Ser Leu Leu Lys Pro Gly Gly Phe

            180                 185                 190

Leu Val Ile Met Asp Ala Leu Lys Ser Ser Tyr Tyr Met Ile Gly Glu

        195                 200                 205

Gln Lys Phe Ser Ser Leu Pro Leu Gly Arg Glu Ala Val Glu Ala Ala

    210                 215                 220

Val Lys Glu Ala Gly Tyr Thr Ile Glu Trp Phe Glu Val Ile Ser Gln

225                 230                 235                 240

Ser Tyr Ser Ser Thr Met Ala Asn Asn Glu Gly Leu Phe Ser Leu Val

                245                 250                 255

Ala Arg Lys Leu Ser Arg Pro Leu

            260

<210> 57

<211> 2617

<212> DNA

<213> Homo sapiens

<220>

<221> CDS

<222> (94)..(633)

<400> 57

tagaggatcc aagcttacgt acgcgtccgg agaccgcttg tgctggagtc ggagttgtaa 60

cgctccactg actgatagag cgaccggccg acc atg gcg ccc gga gtg gcc cgc 114

Met Ala Pro Gly Val Ala Arg

1               5

ggg ccg acg ccg tac tgg agg ttg cgc ctc ggt ggc gcc gcg ctg ctc 162

Gly Pro Thr Pro Tyr Trp Arg Leu Arg Leu Gly Gly Ala Ala Leu Leu

        10                  15                  20

ctg ctg ctc atc ccg gtg gcc gcc gcg cag gag cct ccc gga gct gct 210

Leu Leu Leu Ile Pro Val Ala Ala Ala Gln Glu Pro Pro Gly Ala Ala
    25                  30                  35
tgt tct cag aac aca aac aaa acc tgt gaa gag tgc ctg aag aac gtc 258
Cys Ser Gln Asn Thr Asn Lys Thr Cys Glu Glu Cys Leu Lys Asn Val
40                  45                  50                  55
tcc tgt ctt tgg tgc aac act aac aag gct tgt ctg gac tac cca gtt 306
Ser Cys Leu Trp Cys Asn Thr Asn Lys Ala Cys Leu Asp Tyr Pro Val
                60                  65                  70
aca agc gtc ttg cca ccg gct tcc ctt tgt aaa ttg agc tct gca cgc 354
Thr Ser Val Leu Pro Pro Ala Ser Leu Cys Lys Leu Ser Ser Ala Arg
            75                  80                  85
tgg gga gtt tgt tgg gtg aac ttt gag gcg ctg atc atc acc atg tcg 402
Trp Gly Val Cys Trp Val Asn Phe Glu Ala Leu Ile Ile Thr Met Ser
        90                  95                  100
gta gtc ggg gga acc ctc ctc ctg ggc att gcc atc tgc tgc tgc tgc 450
Val Val Gly Gly Thr Leu Leu Leu Gly Ile Ala Ile Cys Cys Cys Cys
    105                 110                 115
tgc tgc agg agg aag agg agc cgg aag ccg gac agg agt gag gag aag 498
Cys Cys Arg Arg Lys Arg Ser Arg Lys Pro Asp Arg Ser Glu Glu Lys
120                 125                 130                 135
gcc atg cgt gag cgg gag gag agg cgg ata cgg cag gag gaa cgg aga 546
Ala Met Arg Glu Arg Glu Glu Arg Arg Ile Arg Gln Glu Glu Arg Arg
                140                 145                 150
gca gag atg aag aca aga cat gat gaa atc aga aaa aaa tat ggc ctg 594
Ala Glu Met Lys Thr Arg His Asp Glu Ile Arg Lys Lys Tyr Gly Leu
            155                 160                 165
ttt aaa gaa gaa aac ccg tat gct aga ttt gaa aac aac taaagcgctc 643

Phe Lys Glu Glu Asn Pro Tyr Ala Arg Phe Glu Asn Asn
        170                 175                 180
cagcacatca gtcccgacgc ttcctgtgag gtgcactccg cagcccagcc cagccgggag 703
accacgtggc cattgcggtc tcctgacctt ggccagtgaa cctgccagcc ttccaggaca 763
ggcggccgga gagctgcccc tgaaggacag tcctctcgtc ttgcagactg gtgaccttct 823
attccctgtt catctctgtt tctagattta gtcacttgaa ataagaaatc tttggggttt 883
gggctttttt atactcttct cagtttgtga aacgctaact gcacacgaag ccgcctgacg 943
gcacccagcg ctgtggctgt cattctccca gggcagaacc ctgcgtttct ctctgtccac 1003
taacaagctt cacacgcaac acagggaagt cggtttgact tttgtcatga ggagaactga 1063
ccagccctca tcattcccca taaaaccacg gacagcgtct gtgtgcgcat cttgagtctt 1123
cacacctgtt gactcacacg gcttttgctg atgacacggg gctccagtac acagtctgat 1183
aaggacttaa cgtcctaacc tcaattgtat taaatagcat tggggaatag ctaaaccttt 1243
ttaaaaaaat ttattggatt ttcctccctg cttaaaagat ttcaccagaa aaccttcata 1303
taaaaattca ggcccttttt ggacaatttt taaaatttgt atctttacta gaacatgaga 1363
atctttttcc cttggaagct tgaattataa atgtggtgtt tggcctgcct cagcagcacc 1423
agttgactgc tcgtgtgcca gcggtgtggg gaggacgggg caggacgctg cagctctctc 1483
cagccctgtt ggcatcctca gtgcctgcag gcctctcgct gcctcttggg ctgtctgggg 1543
ggtggccatt tagggatcgt ggggacgggg tccaccccaa gaagaaagaa aggcccgtcc 1603
acaggcccgg ctctggccac gtgccccgga agcaggtgtg tccagagtca gctgagggct 1663
ctccccacac cacccagcag gcgctggtgc tccttctgcc tcatgggacc agtccagctt 1723
ccagccgctc tggctcgagg gtggtctgac cacttccttc tgagtgggct tctctgggag 1783
ctctccagtg gcactgctgg acctgcccac gtttctgtaa aatcaggata cgtggcttta 1843
gtaagcagac caagcgcttc gtggcaggga aagcagcgtg cggggaagtc actgaaaagt 1903
gctgcctaag gaagtttgga aatagtcccc gttccagatt gccttgaatt ttaaaacatt 1963
ttgctttggg aaagtaggtc agcagcacct aagatcaagg atgcgttcca ttttcacact 2023
tcacagtcat gaaaactgag aagactgtct tcagcgtgaa ctaaagttca caggcagatc 2083

actgatccag aacacttcaa gaactcgcca aacagctcga taagcctttt tgactgtgta 2143

catctgtacc gggaataaca ttcctaggct gaaatttcca caaagaatag aacctgtacc 2203

cagttcttca ggctgatttc cctgacctct tgggcatttg tatttgtagt aaagtattgc 2263

agagattcct aagtatttta tagcagccat caaaattgga ctttgtattg tttattcata 2323

aaagacactt ggtaatagac ttcagtgaac tctgtatgaa tgcagtagtg tgtgtgcaaa 2383

atccgcttcc tgagcgtagg gtgctgagct ggcgctaggg ctcggttgtg aaatacagcg 2443

tagtcagccc ttgcgctcag tgtagaaacc cacgtctgta aggtcggtct tcgtccatct 2503

gcttttttct gaaatacact aagagcagcc acaaaactgt aacctcaagg aaaccataaa 2563

gcttggagtg ccttaatttt taaccagttt ccaataaaac ggtttactac ctga 2617

<210> 58

<211> 180

<212> PRT

<213> Homo sapiens

<400> 58

Met Ala Pro Gly Val Ala Arg Gly Pro Thr Pro Tyr Trp Arg Leu Arg

1               5                   10                  15

Leu Gly Gly Ala Ala Leu Leu Leu Leu Leu Ile Pro Val Ala Ala Ala

            20                  25                  30

Gln Glu Pro Pro Gly Ala Ala Cys Ser Gln Asn Thr Asn Lys Thr Cys

        35                  40                  45

Glu Glu Cys Leu Lys Asn Val Ser Cys Leu Trp Cys Asn Thr Asn Lys

    50                  55                  60

Ala Cys Leu Asp Tyr Pro Val Thr Ser Val Leu Pro Pro Ala Ser Leu

65                  70                  75                  80

Cys Lys Leu Ser Ser Ala Arg Trp Gly Val Cys Trp Val Asn Phe Glu

                85                  90                  95

Ala Leu Ile Ile Thr Met Ser Val Val Gly Gly Thr Leu Leu Leu Gly
            100                 105                 110
Ile Ala Ile Cys Cys Cys Cys Cys Cys Arg Arg Lys Arg Ser Arg Lys
        115                 120                 125
Pro Asp Arg Ser Glu Glu Lys Ala Met Arg Glu Arg Glu Glu Arg Arg
    130                 135                 140
Ile Arg Gln Glu Glu Arg Arg Ala Glu Met Lys Thr Arg His Asp Glu
145                 150                 155                 160
Ile Arg Lys Lys Tyr Gly Leu Phe Lys Glu Glu Asn Pro Tyr Ala Arg
                165                 170                 175
Phe Glu Asn Asn
            180
<210> 59
<211> 3217
<212> DNA
<213> Homo sapiens
<220>
<221> CDS
<222> (469)..(1875)
<400> 59
   gtcggtctcc gccagctagg atctcccccg ccgtccgccc ccgcccctcg tctgcctccc 60
   ccgcccgcgc ctccgcccgc gcttcccttt cctgcctcgc gccccactcc ctttcctccc 120
   ctccctgttt cccttcctgt ccttcccagc tcacgctctc tttccctgcc gcctgccttt 180
   cttttttcct ttttttgcat tggcgtcttg gggctgttac acacacgcgc gctgtccatt 240
   gcagcttaca taaaggcggg cgcgattatg caattatatt gttagcgata tttcaagagc 300
   aatggctcgt tttcttagga tttcaacacg aaggcatcat gcatttttga aaaactagta 360
   ttgagaataa taccttgcaa cgtaaagaat gttttttggt atttttacac aatctctact 420
   ttgaccaaac gagtctggac agtttctttt taatggaaaa taggagaa atg gag gaa 477
   Met Glu Glu
   1
   aga atg gaa atg att tct gaa agg cca aaa gag agt atg tat tcc tgg 525
   Arg Met Glu Met Ile Ser Glu Arg Pro Lys Glu Ser Met Tyr Ser Trp
   5 10 15
   aac aaa act gca gag aaa agt gat ttt gaa gct gta gaa gca ctt atg 573
   Asn Lys Thr Ala Glu Lys Ser Asp Phe Glu Ala Val Glu Ala Leu Met
   20 25 30 35
   tca atg agc tgc agt tgg aag tct gat ttt aag aaa tac gtt gaa aac 621
   Ser Met Ser Cys Ser Trp Lys Ser Asp Phe Lys Lys Tyr Val Glu Asn
   40 45 50
   aga cct gtt aca cca gta tct gat ttg tca gag gaa gag aat ctg ctt 669
   Arg Pro Val Thr Pro Val Ser Asp Leu Ser Glu Glu Glu Asn Leu Leu
   55 60 65
   ccg gga aca cct gat ttt cat aca atc cca gca ttt tgt ttg act cca 717
   Pro Gly Thr Pro Asp Phe His Thr Ile Pro Ala Phe Cys Leu Thr Pro
   70 75 80
   cct tac agt cct tct gac ttt gaa ccc tct caa gtg tca aat ctg atg 765
   Pro Tyr Ser Pro Ser Asp Phe Glu Pro Ser Gln Val Ser Asn Leu Met
   85 90 95
   gca cca gcg cca tct act gta cac ttc aag tca ctc tca gat act gcc 813
   Ala Pro Ala Pro Ser Thr Val His Phe Lys Ser Leu Ser Asp Thr Ala
   100 105 110 115
   aaa cct cac att gcc gca cct ttc aaa gag gaa gaa aag agc cca gta 861
   Lys Pro His Ile Ala Ala Pro Phe Lys Glu Glu Glu Lys Ser Pro Val
   120 125 130
   tct gcc ccc aaa ctc ccc aaa gct cag gca aca agt gtg att cgt cat 909
   Ser Ala Pro Lys Leu Pro Lys Ala Gln Ala Thr Ser Val Ile Arg His
   135 140 145
   aca gct gat gcc cag cta tgt aac cac cag acc tgc cca atg aaa gca 957
   Thr Ala Asp Ala Gln Leu Cys Asn His Gln Thr Cys Pro Met Lys Ala
   150 155 160
   gcc agc atc ctc aac tat cag aac aat tct ttt aga aga aga acc cac 1005
   Ala Ser Ile Leu Asn Tyr Gln Asn Asn Ser Phe Arg Arg Arg Thr His
   165 170 175
   cta aat gtt gag gct gca aga aag aac ata cca tgt gcc gct gtg tca 1053
   Leu Asn Val Glu Ala Ala Arg Lys Asn Ile Pro Cys Ala Ala Val Ser
   180 185 190 195
   cca aac aga tcc aaa tgt gag aga aac aca gtg gca gat gtt gat gag 1101
   Pro Asn Arg Ser Lys Cys Glu Arg Asn Thr Val Ala Asp Val Asp Glu
   200 205 210
   aaa gca agt gct gca ctt tat gac ttt tct gtg cct tcc tca gag acg 1149
   Lys Ala Ser Ala Ala Leu Tyr Asp Phe Ser Val Pro Ser Ser Glu Thr
   215 220 225
   gtc atc tgc agg tct cag cca gcc cct gtg tcc cca caa cag aag tca 1197
   Val Ile Cys Arg Ser Gln Pro Ala Pro Val Ser Pro Gln Gln Lys Ser
   230 235 240
   gtg ttg gtc tct cca cct gca gta tct gca ggg gga gtg cca cct atg 1245
   Val Leu Val Ser Pro Pro Ala Val Ser Ala Gly Gly Val Pro Pro Met
   245 250 255
   ccg gtc atc tgc cag atg gtt ccc ctt cct gcc aac aac cct gtt gtg 1293
   Pro Val Ile Cys Gln Met Val Pro Leu Pro Ala Asn Asn Pro Val Val
   260                 265                 270                 275
   aca aca gtc gtt ccc agc act cct ccc agc cag cca cca gct gtt tgc    1341
   Thr Thr Val Val Pro Ser Thr Pro Pro Ser Gln Pro Pro Ala Val Cys
                   280                 285                 290
   ccc cct gtt gtg ttc atg ggc aca caa gtc ccc aaa ggc gct gtc atg    1389
   Pro Pro Val Val Phe Met Gly Thr Gln Val Pro Lys Gly Ala Val Met
               295                 300                 305
   ttt gtg gta ccc cag ccc gtt gtg cag agt tca aag cct ccg gtg gtg    1437
   Phe Val Val Pro Gln Pro Val Val Gln Ser Ser Lys Pro Pro Val Val
           310                 315                 320
   agc ccg aat ggc acc aga ctc tct ccc att gcc cct gct cct ggg ttt    1485
   Ser Pro Asn Gly Thr Arg Leu Ser Pro Ile Ala Pro Ala Pro Gly Phe
       325                 330                 335
   tcc cct tca gca gca aaa gtc act cct cag att gat tca tca agg ata    1533
   Ser Pro Ser Ala Ala Lys Val Thr Pro Gln Ile Asp Ser Ser Arg Ile
   340                 345                 350                 355
   agg agt cac atc tgt agc cac cca gga tgt ggc aag aca tac ttt aaa    1581
   Arg Ser His Ile Cys Ser His Pro Gly Cys Gly Lys Thr Tyr Phe Lys
                   360                 365                 370
   agt tcc cat ctg aag gcc cac acg agg acg cac aca gga gaa aag cct    1629
   Ser Ser His Leu Lys Ala His Thr Arg Thr His Thr Gly Glu Lys Pro
               375                 380                 385
   ttc agc tgt agc tgg aaa ggt tgt gaa agg agg ttt gcc cgt tct gat    1677
   Phe Ser Cys Ser Trp Lys Gly Cys Glu Arg Arg Phe Ala Arg Ser Asp
           390                 395                 400
   gaa ctg tcc aga cac agg cga acc cac acg ggt gag aag aaa ttt gcg    1725
   Glu Leu Ser Arg His Arg Arg Thr His Thr Gly Glu Lys Lys Phe Ala
   405 410 415
   tgc ccc atg tgt gac cgg cgg ttc atg agg agt gac cat ttg acc aag 1773
   Cys Pro Met Cys Asp Arg Arg Phe Met Arg Ser Asp His Leu Thr Lys
   420 425 430 435
   cat gcc cgg cgc cat cta tca gcc aag aag cta cca aac tgg cag atg 1821
   His Ala Arg Arg His Leu Ser Ala Lys Lys Leu Pro Asn Trp Gln Met
   440 445 450
   gaa gtg agc aag cta aat gac att gct cta cct cca acc cct gct ccc 1869
   Glu Val Ser Lys Leu Asn Asp Ile Ala Leu Pro Pro Thr Pro Ala Pro
   455 460 465
   aca cag tgacagaccg gaaagtgaag agtcagaact aactttggtc tcagcgggag 1925
   Thr Gln
   ccagtggtga tgtaaaaatg cttccactgc aagtctgtgg ccccacaacg tggcttaaag 1985
   cagaagcccc acagcctggc acgaaggccc cgtctgggtt aggtgactaa aagggcttcg 2045
   gccacaggca ggtcacagaa aggcaggttt catttcttat cacataagag agatgagaaa 2105
   gcttttattc ctttgaatat tttttgaagg tttcagatga ggtcaacaca ggtagcacag 2165
   attttgaatc tgtgtgcata tttgttactt tactttttgc tgtttatact tgagaccaac 2225
   ttttcaatgt gattcttcta aagcactggt ttcaagaata tggaggctgg aaggaaataa 2285
   acattacggt acagacatgg agatgtaaaa tgagtttgta ttattacaaa tattgtcatc 2345
   tttttctaga gttatcttct ttattattcc tagtctttcc agtcaacatc gtggatgtag 2405
   tgattaaata tatctagaac tatcattttt acactattgt gaatatttgg aattgaacga 2465
   ctgtatattg ctaagagggc caaagaattg gaatcctcct taatttaatt gctttgaagc 2525
   atagctacaa tttgtttttg catttttgtt ttgaaagttt aacaaatgac tgtatctagg 2585
   catttcatta tgctttgaac tttagtttgc ctgcagtttc ttgtgtagat ttgaaaattg 2645
   tataccaacg tgttttctgt agactctaag atacactgca ctttgtttag aaaaaaaact 2705
   gaagatgaaa tatatattgt aaagaaggga tattaagaat cttagataac ttcttgaaaa 2765
   agatggctta tgtcatcagc aaagtacctc catgttatga ggatataatg tgtgcttcat 2825
   tgaattagaa aattagtgac cattattcac aggtggacaa atgttgtcct gttaatttat 2885
   aggagttttt tgggatgtgg aggtagttgg gtagaaaaat tattagaaca ttcacttttg 2945
   ttaacagtat ttctctttta ttctgttata tagtggatga tatacacagt ggcaaaacaa 3005
   aagtacattg cttaaaatat atagtgaaaa atgtcactat atcttcccat ttaacattgt 3065
   ttttgtatat tgggtgtaga tttctgacat caaaacttgg acccttggaa aacaaaagtt 3125
   ttaattaaaa aaaatccttg tgacttacaa tttgcacaat atttcttttg ttgtacttta 3185
   tatcttgttt acaataaaga attccctttg gc 3217

<210> 60
<211> 469
<212> PRT
<213> Homo sapiens
<400> 60
Met Glu Glu Arg Met Glu Met Ile Ser Glu Arg Pro Lys Glu Ser Met
1               5                   10                  15
Tyr Ser Trp Asn Lys Thr Ala Glu Lys Ser Asp Phe Glu Ala Val Glu
            20                  25                  30
Ala Leu Met Ser Met Ser Cys Ser Trp Lys Ser Asp Phe Lys Lys Tyr
        35                  40                  45
Val Glu Asn Arg Pro Val Thr Pro Val Ser Asp Leu Ser Glu Glu Glu
    50                  55                  60
Asn Leu Leu Pro Gly Thr Pro Asp Phe His Thr Ile Pro Ala Phe Cys
65                  70                  75                  80
Leu Thr Pro Pro Tyr Ser Pro Ser Asp Phe Glu Pro Ser Gln Val Ser
                85                  90                  95

Asn Leu Met Ala Pro Ala Pro Ser Thr Val His Phe Lys Ser Leu Ser
            100                 105                 110
Asp Thr Ala Lys Pro His Ile Ala Ala Pro Phe Lys Glu Glu Glu Lys
        115                 120                 125
Ser Pro Val Ser Ala Pro Lys Leu Pro Lys Ala Gln Ala Thr Ser Val
    130                 135                 140
Ile Arg His Thr Ala Asp Ala Gln Leu Cys Asn His Gln Thr Cys Pro
145                 150                 155                 160
Met Lys Ala Ala Ser Ile Leu Asn Tyr Gln Asn Asn Ser Phe Arg Arg
                165                 170                 175
Arg Thr His Leu Asn Val Glu Ala Ala Arg Lys Asn Ile Pro Cys Ala
            180                 185                 190
Ala Val Ser Pro Asn Arg Ser Lys Cys Glu Arg Asn Thr Val Ala Asp
        195                 200                 205
Val Asp Glu Lys Ala Ser Ala Ala Leu Tyr Asp Phe Ser Val Pro Ser
    210                 215                 220
Ser Glu Thr Val Ile Cys Arg Ser Gln Pro Ala Pro Val Ser Pro Gln
225                 230                 235                 240
Gln Lys Ser Val Leu Val Ser Pro Pro Ala Val Ser Ala Gly Gly Val
                245                 250                 255
Pro Pro Met Pro Val Ile Cys Gln Met Val Pro Leu Pro Ala Asn Asn
            260                 265                 270
Pro Val Val Thr Thr Val Val Pro Ser Thr Pro Pro Ser Gln Pro Pro
        275                 280                 285
Ala Val Cys Pro Pro Val Val Phe Met Gly Thr Gln Val Pro Lys Gly
    290                 295                 300
Ala Val Met Phe Val Val Pro Gln Pro Val Val Gln Ser Ser Lys Pro

305                 310                 315                 320

Pro Val Val Ser Pro Asn Gly Thr Arg Leu Ser Pro Ile Ala Pro Ala

                325                 330                 335

Pro Gly Phe Ser Pro Ser Ala Ala Lys Val Thr Pro Gln Ile Asp Ser

            340                 345                 350

Ser Arg Ile Arg Ser His Ile Cys Ser His Pro Gly Cys Gly Lys Thr

        355                 360                 365

Tyr Phe Lys Ser Ser His Leu Lys Ala His Thr Arg Thr His Thr Gly

    370                 375                 380

Glu Lys Pro Phe Ser Cys Ser Trp Lys Gly Cys Glu Arg Arg Phe Ala

385                 390                 395                 400

Arg Ser Asp Glu Leu Ser Arg His Arg Arg Thr His Thr Gly Glu Lys

                405                 410                 415

Lys Phe Ala Cys Pro Met Cys Asp Arg Arg Phe Met Arg Ser Asp His

            420                 425                 430

Leu Thr Lys His Ala Arg Arg His Leu Ser Ala Lys Lys Leu Pro Asn

        435                 440                 445

Trp Gln Met Glu Val Ser Lys Leu Asn Asp Ile Ala Leu Pro Pro Thr

    450                 455                 460

Pro Ala Pro Thr Gln

465

<210> 61

<211> 1428

<212> DNA

<213> Homo sapiens

<220>

<221> CDS

<222> (125)..(868)

<400> 61
   cgagctgata aaggcgccat tttggagggg ccgcgggaga cgtggtgccg ctgcgggctc 60
   gctctgccgt gcgctaggct tggtgggaag gcctgttctc gagtccgcgc ttttcgtcac 120
   cgcc atg tcg gga ggt ggt gtg att cgt ggc ccc gca ggg aac aac gat 169
   Met Ser Gly Gly Gly Val Ile Arg Gly Pro Ala Gly Asn Asn Asp
   1 5 10 15
   tgc cgc atc tac gtg ggt aac tta cct cca gac atc cga acc aag gac 217
   Cys Arg Ile Tyr Val Gly Asn Leu Pro Pro Asp Ile Arg Thr Lys Asp
   20 25 30
   att gag gac gtg ttc tac aaa tac ggc gct atc cgc gac atc gac ctc 265
   Ile Glu Asp Val Phe Tyr Lys Tyr Gly Ala Ile Arg Asp Ile Asp Leu
   35 40 45
   aag aat cgc cgc ggg gga ccg ccc ttc gcc ttc gtt gag ttc gag gac 313
   Lys Asn Arg Arg Gly Gly Pro Pro Phe Ala Phe Val Glu Phe Glu Asp
   50 55 60
   ccg cga gac gcg gaa gac gcg gtg tat ggt cgc gac ggc tat gat tac 361
   Pro Arg Asp Ala Glu Asp Ala Val Tyr Gly Arg Asp Gly Tyr Asp Tyr
   65 70 75
   gat ggg tac cgt ctg cgg gtg gag ttt cct cga agc ggc cgt gga aca 409
   Asp Gly Tyr Arg Leu Arg Val Glu Phe Pro Arg Ser Gly Arg Gly Thr
   80 85 90 95
   ggc cga ggc ggc ggc ggg ggt gga ggt ggc gga gct ccc cga ggt cgc 457
   Gly Arg Gly Gly Gly Gly Gly Gly Gly Gly Gly Ala Pro Arg Gly Arg
   100 105 110
   tat ggc ccc cca tcc agg cgg tct gaa aac aga gtg gtt gtc tct gga 505
   Tyr Gly Pro Pro Ser Arg Arg Ser Glu Asn Arg Val Val Val Ser Gly
   115 120 125
   ctg cct cca agt gga agt tgg cag gat tta aag gat cac atg cgt gaa 553
   Leu Pro Pro Ser Gly Ser Trp Gln Asp Leu Lys Asp His Met Arg Glu
   130 135 140
   gca ggt gat gta tgt tat gct gat gtt tac cga gat ggc act ggt gtc 601
   Ala Gly Asp Val Cys Tyr Ala Asp Val Tyr Arg Asp Gly Thr Gly Val
   145 150 155
   gtg gag ttt gta cgg aaa gaa gat atg acc tat gca gtt cga aaa ctg 649
   Val Glu Phe Val Arg Lys Glu Asp Met Thr Tyr Ala Val Arg Lys Leu
   160 165 170 175
   gat aac act aag ttt aga tct cat gag gga gaa act gcc tac atc cgg 697
   Asp Asn Thr Lys Phe Arg Ser His Glu Gly Glu Thr Ala Tyr Ile Arg
   180 185 190
   gtt aaa gtt gat ggg ccc aga agt cca agt tat gga aga tct cga tct 745
   Val Lys Val Asp Gly Pro Arg Ser Pro Ser Tyr Gly Arg Ser Arg Ser
   195 200 205
   cga agc cgt agt cgt agc aga agc cgt agc aga agc aac agc agg agt 793
   Arg Ser Arg Ser Arg Ser Arg Ser Arg Ser Arg Ser Asn Ser Arg Ser
   210 215 220
   cgc agt tac tcc cca agg aga agc aga gga tca cca cgc tat tct ccc 841
   Arg Ser Tyr Ser Pro Arg Arg Ser Arg Gly Ser Pro Arg Tyr Ser Pro
   225 230 235
   cgt cat agc aga tct cgc tct cgt aca taagatgatt ggtgacactt 888
   Arg His Ser Arg Ser Arg Ser Arg Thr
   240 245
   tttgtagaac ccatgttgta tacagttttc ctttattcag tacaatcttt tcatttttta 948
   attcaaactg ttttgttcag aatgggctaa agtgttgaat tgcattcttg taatatcccc 1008
   ttgctcctaa catctacatt cccttcgtgt ctttgataaa ttgtatttta agtgatgtca 1068
   tagacaggat tgtttaaatt tagttaactc catactcttc agactgtgat attgtgtaaa 1128
   tgtctatctg ccctggtttg tgtgaactgg gatgttgggg gtgtttgtgg ttatcttacc 1188
   tggggaagtt cttatgttta tcttgctttt catgtgtctt tctgtagaca tatctgaaga 1248
   gatggattaa gaatgctttg gattaaggat tgtggagcac atttcaatca ttttaggatt 1308
   gtcaaaagga ggattgagga ggatcagatc aataatggag gcaatggtat gactccaagt 1368
   gctattgtca cagatgaaat tggcagtatt gaccttatac taaaaggcag gggctaaaaa 1428

<210> 62

<211> 248

<212> PRT

<213> Homo sapiens

<400> 62

Met Ser Gly Gly Gly Val Ile Arg Gly Pro Ala Gly Asn Asn Asp Cys

1               5                   10                  15

Arg Ile Tyr Val Gly Asn Leu Pro Pro Asp Ile Arg Thr Lys Asp Ile

            20                  25                  30

Glu Asp Val Phe Tyr Lys Tyr Gly Ala Ile Arg Asp Ile Asp Leu Lys

        35                  40                  45

Asn Arg Arg Gly Gly Pro Pro Phe Ala Phe Val Glu Phe Glu Asp Pro

    50                  55                  60

Arg Asp Ala Glu Asp Ala Val Tyr Gly Arg Asp Gly Tyr Asp Tyr Asp

65                  70                  75                  80

Gly Tyr Arg Leu Arg Val Glu Phe Pro Arg Ser Gly Arg Gly Thr Gly

                85                  90                  95

Arg Gly Gly Gly Gly Gly Gly Gly Gly Gly Ala Pro Arg Gly Arg Tyr

            100                 105                 110

Gly Pro Pro Ser Arg Arg Ser Glu Asn Arg Val Val Val Ser Gly Leu

        115                 120                 125

Pro Pro Ser Gly Ser Trp Gln Asp Leu Lys Asp His Met Arg Glu Ala

    130                 135                 140

Gly Asp Val Cys Tyr Ala Asp Val Tyr Arg Asp Gly Thr Gly Val Val

145                 150                 155                 160

Glu Phe Val Arg Lys Glu Asp Met Thr Tyr Ala Val Arg Lys Leu Asp

                165                 170                 175

Asn Thr Lys Phe Arg Ser His Glu Gly Glu Thr Ala Tyr Ile Arg Val

            180                 185                 190

Lys Val Asp Gly Pro Arg Ser Pro Ser Tyr Gly Arg Ser Arg Ser Arg

        195                 200                 205

Ser Arg Ser Arg Ser Arg Ser Arg Ser Arg Ser Asn Ser Arg Ser Arg

    210                 215                 220

Ser Tyr Ser Pro Arg Arg Ser Arg Gly Ser Pro Arg Tyr Ser Pro Arg

225                 230                 235                 240

His Ser Arg Ser Arg Ser Arg Thr

                245

<210> 63

<211> 3664

<212> DNA

<213> Homo sapiens

<220>

<221> CDS

<222> (195)..(1943)

<400> 63

atggggcctg aaactgtctg ggtctgagct ggggagcgga agccacttgt ccctctccct 60

ccccaggact tctgtgactc ctgggccaca gaggtccaac cagggtaagg gcctggggat 120

accccctgcc tggccccctt gcccaaactg gcaggggggc caggctgggc agcagcccct 180

ctttcacctc aact atg gat ctc ctg ccc ccc aag ccc aag tac aat cca 230

Met Asp Leu Leu Pro Pro Lys Pro Lys Tyr Asn Pro

1               5                   10

ctc cgg aat gag tct ctg tca tcg ctg gag gaa ggg gct tct ggg tcc 278

Leu Arg Asn Glu Ser Leu Ser Ser Leu Glu Glu Gly Ala Ser Gly Ser

        15                  20                  25

acc ccc ccg gag gag ctg cct tcc cca tca gct tca tcc ctg ggg ccc 326

Thr Pro Pro Glu Glu Leu Pro Ser Pro Ser Ala Ser Ser Leu Gly Pro

    30                  35                  40

atc ctg cct cct ctg cct ggg gac gat agt ccc act acc ctg tgc tcc 374

Ile Leu Pro Pro Leu Pro Gly Asp Asp Ser Pro Thr Thr Leu Cys Ser

45                  50                  55                  60

ttc ttc ccc cgg atg agc aac ctg agg ctg gcc aac ccg gct ggg ggg 422

Phe Phe Pro Arg Met Ser Asn Leu Arg Leu Ala Asn Pro Ala Gly Gly

                65                  70                  75

cgc cca ggg tct aag ggg gag cca gga agg gca gct gat gat ggg gag 470

Arg Pro Gly Ser Lys Gly Glu Pro Gly Arg Ala Ala Asp Asp Gly Glu

            80                  85                  90

ggg atc gat ggg gca gcc atg cca gag tca ggc ccc cta ccc ctc ctc 518

Gly Ile Asp Gly Ala Ala Met Pro Glu Ser Gly Pro Leu Pro Leu Leu

        95                  100                 105

cag gac atg aac aag ctg agt gga ggc ggc ggg cgc agg act cgg gtg 566
Gln Asp Met Asn Lys Leu Ser Gly Gly Gly Gly Arg Arg Thr Arg Val
    110                 115                 120
gaa ggg ggc cag ctt ggg ggc gag gag tgg acc cgc cac ggg agc ttt 614
Glu Gly Gly Gln Leu Gly Gly Glu Glu Trp Thr Arg His Gly Ser Phe
125                 130                 135                 140
gtc aat aag ccc acg cgg ggc tgg ctg cat ccc aac gac aaa gtc atg 662
Val Asn Lys Pro Thr Arg Gly Trp Leu His Pro Asn Asp Lys Val Met
                145                 150                 155
gga ccc ggg gtt tcc tac ttg gtt cgg tac atg ggt tgt gtg gag gtc 710
Gly Pro Gly Val Ser Tyr Leu Val Arg Tyr Met Gly Cys Val Glu Val
            160                 165                 170
ctc cag tca atg cgt gcc ctg gac ttc aac acc cgg act cag gtc acc 758
Leu Gln Ser Met Arg Ala Leu Asp Phe Asn Thr Arg Thr Gln Val Thr
        175                 180                 185
agg gag gcc atc agt ctg gtg tgt gag gct gtg ccg ggt gct aag ggg 806
Arg Glu Ala Ile Ser Leu Val Cys Glu Ala Val Pro Gly Ala Lys Gly
    190                 195                 200
gcg aca agg agg aga aag ccc tgt agc cgc ccg ctc agc tct atc ctg 854
Ala Thr Arg Arg Arg Lys Pro Cys Ser Arg Pro Leu Ser Ser Ile Leu
205                 210                 215                 220
ggg agg agt aac ctg aaa ttt gct gga atg cca atc act ctc acc gtc 902
Gly Arg Ser Asn Leu Lys Phe Ala Gly Met Pro Ile Thr Leu Thr Val
                225                 230                 235
tcc acc agc agc ctc aac ctc atg gcc gca gac tgc aaa cag atc atc 950
Ser Thr Ser Ser Leu Asn Leu Met Ala Ala Asp Cys Lys Gln Ile Ile
            240                 245                 250

gcc aac cac cac atg caa tct atc tca ttt gca tcc ggc ggg gat ccg 998
Ala Asn His His Met Gln Ser Ile Ser Phe Ala Ser Gly Gly Asp Pro
        255                 260                 265
gac aca gcc gag tat gtc gcc tat gtt gcc aaa gac cct gtg aat cag 1046
Asp Thr Ala Glu Tyr Val Ala Tyr Val Ala Lys Asp Pro Val Asn Gln
    270                 275                 280
aga gcc tgc cac att ctg gag tgt ccc gaa ggg ctt gcc cag gat gtc 1094
Arg Ala Cys His Ile Leu Glu Cys Pro Glu Gly Leu Ala Gln Asp Val
285                 290                 295                 300
atc agc acc att ggc cag gcc ttc gag ttg cgc ttc aaa caa tac ctc 1142
Ile Ser Thr Ile Gly Gln Ala Phe Glu Leu Arg Phe Lys Gln Tyr Leu
                305                 310                 315
agg aac cca ccc aaa ctg gtc acc cct cat gac agg atg gct ggc ttt 1190
Arg Asn Pro Pro Lys Leu Val Thr Pro His Asp Arg Met Ala Gly Phe
            320                 325                 330
gat ggc tca gca tgg gat gag gag gag gaa gag cca cct gac cat cag 1238
Asp Gly Ser Ala Trp Asp Glu Glu Glu Glu Glu Pro Pro Asp His Gln
        335                 340                 345
tac tat aat gac ttc ccg ggg aag gaa ccc ccc ttg ggg ggg gtg gta 1286
Tyr Tyr Asn Asp Phe Pro Gly Lys Glu Pro Pro Leu Gly Gly Val Val
    350                 355                 360
gac atg agg ctt cgg gaa gga gcc gct cca ggg gct gct cga ccc act 1334
Asp Met Arg Leu Arg Glu Gly Ala Ala Pro Gly Ala Ala Arg Pro Thr
365                 370                 375                 380
gca ccc aat gcc cag acc ccc agc cac ttg gga gct aca ttg cct gta 1382
Ala Pro Asn Ala Gln Thr Pro Ser His Leu Gly Ala Thr Leu Pro Val

                385                 390                 395

gga cag cct gtt ggg gga gat cca gaa gtc cgc aaa cag atg cca cct 1430

Gly Gln Pro Val Gly Gly Asp Pro Glu Val Arg Lys Gln Met Pro Pro

            400                 405                 410

cca cca ccc tgt cca ggc aga gag ctt ttt gat gat ccc tcc tat gtc 1478

Pro Pro Pro Cys Pro Gly Arg Glu Leu Phe Asp Asp Pro Ser Tyr Val

        415                 420                 425

aac gtc cag aac cta gac aag gcc cgg caa gca gtg ggt ggt gct ggg 1526

Asn Val Gln Asn Leu Asp Lys Ala Arg Gln Ala Val Gly Gly Ala Gly

    430                 435                 440

ccc ccc aat cct gct atc aat ggc agt gca ccc cgg gac ctg ttt gac 1574

Pro Pro Asn Pro Ala Ile Asn Gly Ser Ala Pro Arg Asp Leu Phe Asp

445                 450                 455                 460

atg aag ccc ttc gaa gat gct ctt cgg gtg cct cca cct ccc cag tcg 1622



                465                 470                 475

gtg tcc atg gct gag cag ctc cga ggg gag ccc tgg ttc cat ggg aag 1670



            480                 485                 490

ctg agc cgg cgg gag gct gag gca ctg ctg cag ctc aat ggg gac ttc 1718



        495                 500                 505

ttg gta cgg gag agc acg acc aca cct ggc cag tat gtg ctc act ggc 1766



    510                 515                 520

ttg cag agt ggg cag cct aag cat ttg cta ctg gtg gac cct gag ggt 1814



525                 530                 535                 540

gtg gtt cgg act aag gat cac cgc ttt gaa agt gtc agt cac ctt atc 1862



                545                 550                 555

agc tac cac atg gac aat cac ttg ccc atc atc tct gcg ggc agc gaa 1910



            560                 565                 570

ctg tgt cta cag caa cct gtg gag cgg aaa ctg tgatctgccc tagcgctctc 1963



        575                 580

ttccagaaga tgccctccaa tcctttccac cctattccct aactctcggg acctcgtttg 2023

ggagtgttct gtgggcttgg ccttgtgtca gagctgggag tagcatggac tctgggtttc 2083

atatccagct gagtgagagg gtttgagtca aaagcctggg tgagaatcct gcctctcccc 2143

aaacattaat caccaaagta ttaatgtaca gagtggcccc tcacctgggc ctttcctgtg 2203

ccaacctgat gccccttccc caagaaggtg agtgcttgtc atggaaaatg tcctgtggtg 2263

acaggcccag tggaacagtc acccttctgg gcaaggggga acaaatcaca cctctgggct 2323

tcagggtatc ccagacccct ctcaacaccc gcccccccca tgtttaaact ttgtgccttt 2383

gaccatctct taggtctaat gatattttat gcaaacagtt cttggacccc tgaattcttc 2443

aatgacaggg atgccaacac cttcttggct tctgggacct gtgttcttgc tgagcacccct 2503

ctccggtttg ggttgggata acagaggcag gagtggcagc tgtcccctct ccctggggat 2563

atgcaaccct tagagattgc cccagagccc cactcccggc caggcgggag atggacccct 2623

cccttgctca gtgcctcctg gccggggccc ctcaccccaa ggggtctgta tatacatttc 2683

ataaggcctg ccctcccatg ttgcatgcct atgtactctg cgccaaagtg cagcccttcc 2743

tcctgaagcc tctgccctgc ctccctttct gggagggcgg ggtgggggtg actgaatttg 2803

ggcctcttgt acagttaact ctcccaggtg gattttgtgg aggtgagaaa aggggcattg 2863

agactataaa gcagtagaca atccccacat accatctgta gagttggaac tgcattcttt 2923

taaagtttta tatgcatata ttttagggct gctagactta ctttcctatt ttcttttcca 2983

ttgcttattc ttgagcacaa aatgataatc aattattaca tttatacatc acctttttga 3043

cttttccaag cccttttaca gctcttggca ttttcctcgc ctaggcctgt gaggtaactg 3103

ggatcgcacc ttttatacca gagacctgag gcagatgaaa tttatttcca tctaggacta 3163

gaaaaacttg ggtctcttac cgcgagactg agaggcagaa gtcagcccga atgcctgtca 3223

gtttcatgga ggggaaacgc aaaacctgca gttcctgagt accttctaca ggcccggccc 3283

agcctaggcc cggggtggcc acaccacagc aagccggccc cccctctttt ggccttgtgg 3343

ataagggaga gttgaccgtt ttcatcctgg cctccttttg ctgtttggat gtttccacgg 3403

gtctcactta taccaaaggg aaaactcttc attaaagtcc gtatttcttc taaaaaaaaa 3463

aaaaaaaaaa tacatttata catcaccttt ttgacttttc caagcccttt tacagctctt 3523

ggcattttcc tcgcctaggc ctgtgaggta actgggatcg caccttttat accagagacc 3583

tgaggcagat gaaatttatt tccatctagg actagaaaaa cttgggtctc ttaccgcgag 3643

actgagaggc agaagtcagc c 3664

<210> 64

<211> 583

<212> PRT

<213> Homo sapiens

<400> 64

Met Asp Leu Leu Pro Pro Lys Pro Lys Tyr Asn Pro Leu Arg Asn Glu

1               5                   10                  15

Ser Leu Ser Ser Leu Glu Glu Gly Ala Ser Gly Ser Thr Pro Pro Glu

            20                  25                  30

Glu Leu Pro Ser Pro Ser Ala Ser Ser Leu Gly Pro Ile Leu Pro Pro

        35                  40                  45

Leu Pro Gly Asp Asp Ser Pro Thr Thr Leu Cys Ser Phe Phe Pro Arg

    50                  55                  60

Met Ser Asn Leu Arg Leu Ala Asn Pro Ala Gly Gly Arg Pro Gly Ser
65                  70                  75                  80
Lys Gly Glu Pro Gly Arg Ala Ala Asp Asp Gly Glu Gly Ile Asp Gly
                85                  90                  95
Ala Ala Met Pro Glu Ser Gly Pro Leu Pro Leu Leu Gln Asp Met Asn
            100                 105                 110
Lys Leu Ser Gly Gly Gly Gly Arg Arg Thr Arg Val Glu Gly Gly Gln
        115                 120                 125
Leu Gly Gly Glu Glu Trp Thr Arg His Gly Ser Phe Val Asn Lys Pro
    130                 135                 140
Thr Arg Gly Trp Leu His Pro Asn Asp Lys Val Met Gly Pro Gly Val
145                 150                 155                 160
Ser Tyr Leu Val Arg Tyr Met Gly Cys Val Glu Val Leu Gln Ser Met
                165                 170                 175
Arg Ala Leu Asp Phe Asn Thr Arg Thr Gln Val Thr Arg Glu Ala Ile
            180                 185                 190
Ser Leu Val Cys Glu Ala Val Pro Gly Ala Lys Gly Ala Thr Arg Arg
        195                 200                 205
Arg Lys Pro Cys Ser Arg Pro Leu Ser Ser Ile Leu Gly Arg Ser Asn
    210                 215                 220
Leu Lys Phe Ala Gly Met Pro Ile Thr Leu Thr Val Ser Thr Ser Ser
225                 230                 235                 240
Leu Asn Leu Met Ala Ala Asp Cys Lys Gln Ile Ile Ala Asn His His
                245                 250                 255
Met Gln Ser Ile Ser Phe Ala Ser Gly Gly Asp Pro Asp Thr Ala Glu
            260                 265                 270
Tyr Val Ala Tyr Val Ala Lys Asp Pro Val Asn Gln Arg Ala Cys His

        275                 280                 285
Ile Leu Glu Cys Pro Glu Gly Leu Ala Gln Asp Val Ile Ser Thr Ile
    290                 295                 300
Gly Gln Ala Phe Glu Leu Arg Phe Lys Gln Tyr Leu Arg Asn Pro Pro
305                 310                 315                 320
Lys Leu Val Thr Pro His Asp Arg Met Ala Gly Phe Asp Gly Ser Ala
                325                 330                 335
Trp Asp Glu Glu Glu Glu Glu Pro Pro Asp His Gln Tyr Tyr Asn Asp
            340                 345                 350
Phe Pro Gly Lys Glu Pro Pro Leu Gly Gly Val Val Asp Met Arg Leu
        355                 360                 365
Arg Glu Gly Ala Ala Pro Gly Ala Ala Arg Pro Thr Ala Pro Asn Ala
    370                 375                 380
Gln Thr Pro Ser His Leu Gly Ala Thr Leu Pro Val Gly Gln Pro Val
385                 390                 395                 400
Gly Gly Asp Pro Glu Val Arg Lys Gln Met Pro Pro Pro Pro Pro Cys
                405                 410                 415
Pro Gly Arg Glu Leu Phe Asp Asp Pro Ser Tyr Val Asn Val Gln Asn
            420                 425                 430
Leu Asp Lys Ala Arg Gln Ala Val Gly Gly Ala Gly Pro Pro Asn Pro
        435                 440                 445
Ala Ile Asn Gly Ser Ala Pro Arg Asp Leu Phe Asp Met Lys Pro Phe
    450                 455                 460
Glu Asp Ala Leu Arg Val Pro Pro Pro Pro Gln Ser Val Ser Met Ala
465                 470                 475                 480
Glu Gln Leu Arg Gly Glu Pro Trp Phe His Gly Lys Leu Ser Arg Arg

                485                 490                 495
Glu Ala Glu Ala Leu Leu Gln Leu Asn Gly Asp Phe Leu Val Arg Glu
            500                 505                 510
Ser Thr Thr Thr Pro Gly Gln Tyr Val Leu Thr Gly Leu Gln Ser Gly
        515                 520                 525
Gln Pro Lys His Leu Leu Leu Val Asp Pro Glu Gly Val Val Arg Thr
    530                 535                 540
Lys Asp His Arg Phe Glu Ser Val Ser His Leu Ile Ser Tyr His Met
545                 550                 555                 560
Asp Asn His Leu Pro Ile Ile Ser Ala Gly Ser Glu Leu Cys Leu Gln
                565                 570                 575
Gln Pro Val Glu Arg Lys Leu
            580

<210> 65
<211> 2493
<212> DNA
<213> Homo sapiens
<220>
<221> CDS
<222> (41)..(1237)
<400> 65
actgcgactc gagacagcgg cccggcagga cagctccaga atg aaa atg cgg ttc 55
Met Lys Met Arg Phe
1               5
ttg ggg ttg gtg gtc tgt ttg gtt ctc tgg ccc ctg cat tct gag ggg 103
Leu Gly Leu Val Val Cys Leu Val Leu Trp Pro Leu His Ser Glu Gly

                10                  15                  20
tct gga ggg aaa ctg aca gct gtg gat cct gaa aca aac atg aat gtg 151
Ser Gly Gly Lys Leu Thr Ala Val Asp Pro Glu Thr Asn Met Asn Val
            25                  30                  35
agt gaa att atc tct tac tgg gga ttc cct agt gag gaa tac cta gtt 199
Ser Glu Ile Ile Ser Tyr Trp Gly Phe Pro Ser Glu Glu Tyr Leu Val
        40                  45                  50
gag aca gaa gat gga tat att ctg tgc ctt aac cga att cct cat ggg 247
Glu Thr Glu Asp Gly Tyr Ile Leu Cys Leu Asn Arg Ile Pro His Gly
    55                  60                  65
agg aag aac cat tct gac aaa ggt ccc aaa cca gtt gtc ttc ctg caa 295
Arg Lys Asn His Ser Asp Lys Gly Pro Lys Pro Val Val Phe Leu Gln
70                  75                  80                  85
cat ggc ttg ctg gca gat tct agt aac tgg gtc aca aac ctt gcc aac 343
His Gly Leu Leu Ala Asp Ser Ser Asn Trp Val Thr Asn Leu Ala Asn
                90                  95                  100
agc agc ctg ggc ttc att ctt gct gat gct ggt ttt gac gtg tgg atg 391
Ser Ser Leu Gly Phe Ile Leu Ala Asp Ala Gly Phe Asp Val Trp Met
            105                 110                 115
ggc aac agc aga gga aat acc tgg tct cgg aaa cat aag aca ctc tca 439
Gly Asn Ser Arg Gly Asn Thr Trp Ser Arg Lys His Lys Thr Leu Ser
        120                 125                 130
gtt tct cag gat gaa ttc tgg gct ttc agt tat gat gag atg gca aaa 487
Val Ser Gln Asp Glu Phe Trp Ala Phe Ser Tyr Asp Glu Met Ala Lys
    135                 140                 145
tat gac cta cca gct tcc att aac ttc att ctg aat aaa act ggc caa 535
Tyr Asp Leu Pro Ala Ser Ile Asn Phe Ile Leu Asn Lys Thr Gly Gln

150                 155                 160                 165

gaa caa gtg tat tat gtg ggt cat tct caa ggc acc act ata ggt ttt    583

Glu Gln Val Tyr Tyr Val Gly His Ser Gln Gly Thr Thr Ile Gly Phe

                170                 175                 180

ata gca ttt tca cag atc cct gag ctg gct aaa agg att aaa atg ttt    631

Ile Ala Phe Ser Gln Ile Pro Glu Leu Ala Lys Arg Ile Lys Met Phe

            185                 190                 195

ttt gcc ctg ggt cct gtg gct tcc gtc gcc ttc tgt act agc cct atg    679

Phe Ala Leu Gly Pro Val Ala Ser Val Ala Phe Cys Thr Ser Pro Met

        200                 205                 210

gcc aaa tta gga cga tta cca gat cat ctc att aag gac tta ttt gga    727

Ala Lys Leu Gly Arg Leu Pro Asp His Leu Ile Lys Asp Leu Phe Gly

    215                 220                 225

gac aaa gaa ttt ctt ccc cag agt gcg ttt ttg aag tgg ctg ggt acc    775

Asp Lys Glu Phe Leu Pro Gln Ser Ala Phe Leu Lys Trp Leu Gly Thr

230                 235                 240                 245

cac gtt tgc act cat gtc ata ctg aag gag ctc tgt gga aat ctc tgt    823

His Val Cys Thr His Val Ile Leu Lys Glu Leu Cys Gly Asn Leu Cys

                250                 255                 260

ttt ctt ctg tgt gga ttt aat gag aga aat tta aat atg tct aga gtg    871

Phe Leu Leu Cys Gly Phe Asn Glu Arg Asn Leu Asn Met Ser Arg Val

            265                 270                 275

gat gta tat aca aca cat tct cct gct gga act tct gtg caa aac atg    919

Asp Val Tyr Thr Thr His Ser Pro Ala Gly Thr Ser Val Gln Asn Met

        280                 285                 290

tta cac tgg agc cag gct gtt aaa ttc caa aag ttt caa gcc ttt gac    967

Leu His Trp Ser Gln Ala Val Lys Phe Gln Lys Phe Gln Ala Phe Asp
    295                 300                 305
tgg gga agc agt gcc aag aat tat ttt cat tac aac cag agt tat cct 1015
Trp Gly Ser Ser Ala Lys Asn Tyr Phe His Tyr Asn Gln Ser Tyr Pro
310                 315                 320                 325
ccc aca tac aat gtg aag gac atg ctt gtg ccg act gca gtc tgg agc 1063
Pro Thr Tyr Asn Val Lys Asp Met Leu Val Pro Thr Ala Val Trp Ser
                330                 335                 340
ggg ggt cac gac tgg ctt gca gat gtc tac gac gtc aat atc tta ctg 1111
Gly Gly His Asp Trp Leu Ala Asp Val Tyr Asp Val Asn Ile Leu Leu
            345                 350                 355
act cag atc acc aac ttg gtg ttc cat gag agc att ccg gaa tgg gag 1159
Thr Gln Ile Thr Asn Leu Val Phe His Glu Ser Ile Pro Glu Trp Glu
        360                 365                 370
cat ctt gac ttc att tgg ggc ctg gat gcc cct tgg agg ctt tat aat 1207
His Leu Asp Phe Ile Trp Gly Leu Asp Ala Pro Trp Arg Leu Tyr Asn
    375                 380                 385
aaa att att aat cta atg agg aaa tat cag tgaaagctgg acttgagctg 1257
Lys Ile Ile Asn Leu Met Arg Lys Tyr Gln
390                 395
tgtaccacca agtcaatgat tatgtcatgt gaaaatgtgt ttgcttcatt tctgtaaaac 1317
acttgttttt ctttcccagg tcttttgttt ttttatatcc aagaaaatga taactttgaa 1377
gatgcccagt tcactctagt ttcaattaga aacatactag ctattttttc tttaattagg 1437
gctggaatag gaagccagtg tctcaaccat agtattgtct ctttaagtct tttaaatatc 1497
actgatgtgt aaaaaggtca ttatatccat tctgttttta aaatttaaaa tatattgact 1557
ttttgccctt cataggacaa agtaatatat gtgttggaat tttaaaattg tgttgtcatt 1617
ggtaaatctg tcactgactt aagcgaggta taaaagtacg cagttttcat gtccttgcct 1677

taaagagctc tctagtctaa cggtcttgta gttagagatc taaatgacat tttatcatgt 1737
tttcctgcag caggtgcata gtcaaatcca gaaatatcac agctgtgcca gtaataagga 1797
tgctaacaat taattttatc aaacctaact gtgacagctg tgatttgaca cgttttaatt 1857
gctcaggtta aatgaaatag ttttccggcg tcttcaaaaa caaattgcac tgataaaaca 1917
aaaacaaaag tatgttttaa atgctttgaa gactgataca ctcaaccatc tatattcatg 1977
agctctcaat ttcatggcag gccatagttc tacttatctg agaagcaaat ccctgtggag 2037
actataccac tattttttct gagattaatg tactcttgga gcccgctact gtcgttattg 2097
atcacatctg tgtgaagcca aagccccgtg gttgcccatg agaagtgtcc ttgttcattt 2157
tcacccaaat gaagtgtgaa cgtgatgttt tcggatgcaa actcagctca gggattcatt 2217
ttgtgtctta gttttatatg catccttatt tttaatacac ctgcttcacg tccctatgtt 2277
gggaagtcca tatttgtctg cttttcttgc agcatcattt ccttacaata ctgtccggtg 2337
gacaaaatga caattgatat gtttttctga tataattact ttagctgcac taacagtaca 2397
atgcttgtta atggttaata taggcagggc gaatactact ttgtaacttt taaagtctta 2457
aacttttcaa taaaattgag tgagacttat aggccc 2493

<210> 66
<211> 399
<212> PRT
<213> Homo sapiens
<400> 66
Met Lys Met Arg Phe Leu Gly Leu Val Val Cys Leu Val Leu Trp Pro
1               5                   10                  15
Leu His Ser Glu Gly Ser Gly Gly Lys Leu Thr Ala Val Asp Pro Glu
            20                  25                  30
Thr Asn Met Asn Val Ser Glu Ile Ile Ser Tyr Trp Gly Phe Pro Ser
        35                  40                  45
Glu Glu Tyr Leu Val Glu Thr Glu Asp Gly Tyr Ile Leu Cys Leu Asn

    50                  55                  60

Arg Ile Pro His Gly Arg Lys Asn His Ser Asp Lys Gly Pro Lys Pro

65                  70                  75                  80

Val Val Phe Leu Gln His Gly Leu Leu Ala Asp Ser Ser Asn Trp Val

                85                  90                  95

Thr Asn Leu Ala Asn Ser Ser Leu Gly Phe Ile Leu Ala Asp Ala Gly

            100                 105                 110

Phe Asp Val Trp Met Gly Asn Ser Arg Gly Asn Thr Trp Ser Arg Lys

        115                 120                 125

His Lys Thr Leu Ser Val Ser Gln Asp Glu Phe Trp Ala Phe Ser Tyr

    130                 135                 140

Asp Glu Met Ala Lys Tyr Asp Leu Pro Ala Ser Ile Asn Phe Ile Leu

145                 150                 155                 160

Asn Lys Thr Gly Gln Glu Gln Val Tyr Tyr Val Gly His Ser Gln Gly

                165                 170                 175

Thr Thr Ile Gly Phe Ile Ala Phe Ser Gln Ile Pro Glu Leu Ala Lys

            180                 185                 190

Arg Ile Lys Met Phe Phe Ala Leu Gly Pro Val Ala Ser Val Ala Phe

        195                 200                 205

Cys Thr Ser Pro Met Ala Lys Leu Gly Arg Leu Pro Asp His Leu Ile

    210                 215                 220

Lys Asp Leu Phe Gly Asp Lys Glu Phe Leu Pro Gln Ser Ala Phe Leu

225                 230                 235                 240

Lys Trp Leu Gly Thr His Val Cys Thr His Val Ile Leu Lys Glu Leu

                245                 250                 255

Cys Gly Asn Leu Cys Phe Leu Leu Cys Gly Phe Asn Glu Arg Asn Leu

260                 265                 270

Asn Met Ser Arg Val Asp Val Tyr Thr Thr His Ser Pro Ala Gly Thr

        275                 280                 285

Ser Val Gln Asn Met Leu His Trp Ser Gln Ala Val Lys Phe Gln Lys

    290                 295                 300

Phe Gln Ala Phe Asp Trp Gly Ser Ser Ala Lys Asn Tyr Phe His Tyr

305                 310                 315                 320

Asn Gln Ser Tyr Pro Pro Thr Tyr Asn Val Lys Asp Met Leu Val Pro

                325                 330                 335

Thr Ala Val Trp Ser Gly Gly His Asp Trp Leu Ala Asp Val Tyr Asp

            340                 345                 350

Val Asn Ile Leu Leu Thr Gln Ile Thr Asn Leu Val Phe His Glu Ser

        355                 360                 365

Ile Pro Glu Trp Glu His Leu Asp Phe Ile Trp Gly Leu Asp Ala Pro

    370                 375                 380

Trp Arg Leu Tyr Asn Lys Ile Ile Asn Leu Met Arg Lys Tyr Gln

385                 390                 395

<210> 67

<211> 1633

<212> DNA

<213> Homo sapiens

<220>

<221> CDS

<222> (323)..(1177)

<400> 67

aacttaatgt ttttgcattg gactttgagt taagattatt ttttaaatcc tgaggactag 60

cattaattga cagctgaccc aggtgctaca cagaagtgga ttcagtgaat ctaggaagac 120

agcagcagac aggattccag gaaccagtgt ttgatgaagc taggactgag gagcaagcga 180

gcaagcagca gttcgtggaa tcctgtctgc tgctgtcttc ctggtttagg agccgacggg 240

cgctcgcagg ctcagcgcgc gctgcccgcg gcaggacccg gccgcctccg ccgccgccgc 300

cgcccctaag cctcccgaag cc atg gcc ggg ctc ggc cac ccc gcc gcc ttc 352

Met Ala Gly Leu Gly His Pro Ala Ala Phe

1               5                   10

ggc cgg gcc acc cac gcc gtg gtg cgg gcg cta ccc gag tcg ctc ggc 400

Gly Arg Ala Thr His Ala Val Val Arg Ala Leu Pro Glu Ser Leu Gly

                15                  20                  25

cag cac gcg ctg aga agc gcc aag ggc gag gag gtg gac gtc gcc cgc 448

Gln His Ala Leu Arg Ser Ala Lys Gly Glu Glu Val Asp Val Ala Arg

            30                  35                  40

gcg gaa cgg cag cac cag ctc tac gtg ggc gtg ctg ggc agc aag ctg 496

Ala Glu Arg Gln His Gln Leu Tyr Val Gly Val Leu Gly Ser Lys Leu

        45                  50                  55

ggg ctg cag gtg gtg gag ctg ccg gcc gac gag agc ctt ccg gac tgc 544

Gly Leu Gln Val Val Glu Leu Pro Ala Asp Glu Ser Leu Pro Asp Cys

    60                  65                  70

gtc ttc gtg gag gac gtg gcc gtg gtg tgc gag gag acg gcc ctc atc 592

Val Phe Val Glu Asp Val Ala Val Val Cys Glu Glu Thr Ala Leu Ile

75                  80                  85                  90

acc cga ccc ggg gcg ccg agc cgg agg aag gag gtt gac atg atg aaa 640

Thr Arg Pro Gly Ala Pro Ser Arg Arg Lys Glu Val Asp Met Met Lys

                95                  100                 105

gaa gca tta gaa aaa ctt cag ctc aat ata gta gag atg aaa gat gaa 688

Glu Ala Leu Glu Lys Leu Gln Leu Asn Ile Val Glu Met Lys Asp Glu

            110                 115                 120
aat gca act tta gat ggc gga gat gtt tta ttc aca ggc aga gaa ttt 736
Asn Ala Thr Leu Asp Gly Gly Asp Val Leu Phe Thr Gly Arg Glu Phe
        125                 130                 135
ttt gtg ggc ctt tcc aaa agg aca aat caa cga ggt gct gaa atc ttg 784
Phe Val Gly Leu Ser Lys Arg Thr Asn Gln Arg Gly Ala Glu Ile Leu
    140                 145                 150
gct gat act ttt aag gac tat gca gtc tcc aca gtg cca gtg gca gat 832
Ala Asp Thr Phe Lys Asp Tyr Ala Val Ser Thr Val Pro Val Ala Asp
155                 160                 165                 170
ggg ttg cat ttg aag agt ttc tgc agc atg gct ggg cct aac ctg atc 880
Gly Leu His Leu Lys Ser Phe Cys Ser Met Ala Gly Pro Asn Leu Ile
                175                 180                 185
gca att ggg tct agt gaa tct gca cag aag gcc ctt aag atc atg caa 928
Ala Ile Gly Ser Ser Glu Ser Ala Gln Lys Ala Leu Lys Ile Met Gln
            190                 195                 200
cag atg agt gac cac cgc tac gac aaa ctc act gtg cct gat gac ata 976
Gln Met Ser Asp His Arg Tyr Asp Lys Leu Thr Val Pro Asp Asp Ile
        205                 210                 215
gca gca aac tgt ata tat cta aat atc ccc aac aaa ggg cac gtc ttg 1024
Ala Ala Asn Cys Ile Tyr Leu Asn Ile Pro Asn Lys Gly His Val Leu
    220                 225                 230
ctg cac cga acc ccg gaa gag tat cca gaa agt gca aag gtt tat gag 1072
Leu His Arg Thr Pro Glu Glu Tyr Pro Glu Ser Ala Lys Val Tyr Glu
235                 240                 245                 250
aaa ctg aag gac cat atg ctg atc ccc gtg agc atg tct gaa ctg gaa 1120

Lys Leu Lys Asp His Met Leu Ile Pro Val Ser Met Ser Glu Leu Glu
                255                 260                 265
aag gtg gat ggg ctg ctc acc tgc tgc tca gtt tta att aac aag aaa 1168
Lys Val Asp Gly Leu Leu Thr Cys Cys Ser Val Leu Ile Asn Lys Lys
            270                 275                 280
gta gac tcc tgagctgcag agtccccccc ggtagccggc aagaccgcac 1217
Val Asp Ser
        285
aggcaaggcc gatgactctg tgcccactcc tgttgttttc cttgacaatc tactgtgcca 1277
ctgtgctact aactcttgtt tacaaaattt gattctaagt tgaattgctt cattcaacac 1337
ccccaccctc cctccccrcg aggtggtacc taagctgtgg atttgctaaa tgaattaagc 1397
aacctagaag atacagagct aatgaattat caaaatgtga ttaatcccag taaggaaaca 1457
ctcatttagt gtctgtattt ttggtgtnaa aattatttag ttgccagtat attctgaaga 1517
atgtcttctt gatcagtcag ataagcttgc tttttttttt tttttttcat gaatcatgtt 1577
tggttcctgt gaaagtccct ggtccaggga tcctcctcct ttctctttta cttctg 1633
<210> 68
<211> 285
<212> PRT
<213> Homo sapiens
<400> 68
Met Ala Gly Leu Gly His Pro Ala Ala Phe Gly Arg Ala Thr His Ala
1               5                   10                  15
Val Val Arg Ala Leu Pro Glu Ser Leu Gly Gln His Ala Leu Arg Ser
            20                  25                  30
Ala Lys Gly Glu Glu Val Asp Val Ala Arg Ala Glu Arg Gln His Gln
        35                  40                  45

Leu Tyr Val Gly Val Leu Gly Ser Lys Leu Gly Leu Gln Val Val Glu
    50                  55                  60
Leu Pro Ala Asp Glu Ser Leu Pro Asp Cys Val Phe Val Glu Asp Val
65                  70                  75                  80
Ala Val Val Cys Glu Glu Thr Ala Leu Ile Thr Arg Pro Gly Ala Pro
                85                  90                  95
Ser Arg Arg Lys Glu Val Asp Met Met Lys Glu Ala Leu Glu Lys Leu
            100                 105                 110
Gln Leu Asn Ile Val Glu Met Lys Asp Glu Asn Ala Thr Leu Asp Gly
        115                 120                 125
Gly Asp Val Leu Phe Thr Gly Arg Glu Phe Phe Val Gly Leu Ser Lys
    130                 135                 140
Arg Thr Asn Gln Arg Gly Ala Glu Ile Leu Ala Asp Thr Phe Lys Asp
145                 150                 155                 160
Tyr Ala Val Ser Thr Val Pro Val Ala Asp Gly Leu His Leu Lys Ser
                165                 170                 175
Phe Cys Ser Met Ala Gly Pro Asn Leu Ile Ala Ile Gly Ser Ser Glu
            180                 185                 190
Ser Ala Gln Lys Ala Leu Lys Ile Met Gln Gln Met Ser Asp His Arg
        195                 200                 205
Tyr Asp Lys Leu Thr Val Pro Asp Asp Ile Ala Ala Asn Cys Ile Tyr
    210                 215                 220
Leu Asn Ile Pro Asn Lys Gly His Val Leu Leu His Arg Thr Pro Glu
225                 230                 235                 240
Glu Tyr Pro Glu Ser Ala Lys Val Tyr Glu Lys Leu Lys Asp His Met
                245                 250                 255
Leu Ile Pro Val Ser Met Ser Glu Leu Glu Lys Val Asp Gly Leu Leu

            260                 265                 270

Thr Cys Cys Ser Val Leu Ile Asn Lys Lys Val Asp Ser

        275                 280                 285

<210> 69

<211> 1779

<212> DNA

<213> Homo sapiens

<220>

<221> CDS

<222> (147)..(1421)

<400> 69

aagcgcctgt ctgaacctct gccagtcctg gagactggtg ccctgagctc caaccagcgg 60

gcctcatcta caccctcacc accgcaactt ctcacccgag caagaagcag ctcccagaga 120

gaaagaacgt tcccacctgc ctagcc atg gga gag gac gct gca cag gcc gaa 173

Met Gly Glu Asp Ala Ala Gln Ala Glu

1               5

aag ttc cag cac cct ggg tct gac atg cgg cag gaa aag ccc tcg agc 221

Lys Phe Gln His Pro Gly Ser Asp Met Arg Gln Glu Lys Pro Ser Ser

10                  15                  20                  25

ccc agc ccg atg cct tcc tcc aca cca agc ccc agc ctg aac cta ggg 269

Pro Ser Pro Met Pro Ser Ser Thr Pro Ser Pro Ser Leu Asn Leu Gly

                30                  35                  40

aac aca gag gag gcc atc cgg gac aac tca cag gtg aac gca gtc acg 317

Asn Thr Glu Glu Ala Ile Arg Asp Asn Ser Gln Val Asn Ala Val Thr

            45                  50                  55

gtg ctc acg ctc ctg gac aag ctg gtg aac atg cta gac gct gtg cag 365

Val Leu Thr Leu Leu Asp Lys Leu Val Asn Met Leu Asp Ala Val Gln
        60                  65                  70
gag aac cag cac aag atg gag cag cga cag atc agt ttg gag ggc tcc 413
Glu Asn Gln His Lys Met Glu Gln Arg Gln Ile Ser Leu Glu Gly Ser
    75                  80                  85
gtg aag ggc atc cag aat gac ctc acc aag ctc tcc aag tac cag gcc 461
Val Lys Gly Ile Gln Asn Asp Leu Thr Lys Leu Ser Lys Tyr Gln Ala
90                  95                  100                 105
tcc acc agc aac acg gtg agc aag ctg ctg gag aag tcc cgc aag gtc 509
Ser Thr Ser Asn Thr Val Ser Lys Leu Leu Glu Lys Ser Arg Lys Val
                110                 115                 120
agc gcc cac acg cgc gcg gtc aaa gag cgc atg gat agg cag tgc gca 557
Ser Ala His Thr Arg Ala Val Lys Glu Arg Met Asp Arg Gln Cys Ala
            125                 130                 135
cag gtg aag cgg ctg gag aac aac cac gcc cag ctc ctc cga cgc aac 605
Gln Val Lys Arg Leu Glu Asn Asn His Ala Gln Leu Leu Arg Arg Asn
        140                 145                 150
cat ttc aaa gtg ctc atc ttc cag gag gaa aat gag atc cct gcc agc 653
His Phe Lys Val Leu Ile Phe Gln Glu Glu Asn Glu Ile Pro Ala Ser
    155                 160                 165
gtg ttt gtg aaa cag ccc gtt tcc ggt gcc gtg gaa ggg aag gag gag 701
Val Phe Val Lys Gln Pro Val Ser Gly Ala Val Glu Gly Lys Glu Glu
170                 175                 180                 185
ctt ccg gat gaa aac aaa tcc ctg gag gaa acc ctg cac acc gtg gac 749
Leu Pro Asp Glu Asn Lys Ser Leu Glu Glu Thr Leu His Thr Val Asp
                190                 195                 200

ctc tcc tca gat gat gat ttg ccc cac gat gag gag gcc ctg gaa gac 797
Leu Ser Ser Asp Asp Asp Leu Pro His Asp Glu Glu Ala Leu Glu Asp
            205                 210                 215
agt gcc gag gaa aag gtg gaa gaa agt agg gca gag aaa ata aaa aga 845
Ser Ala Glu Glu Lys Val Glu Glu Ser Arg Ala Glu Lys Ile Lys Arg
        220                 225                 230
tcc agc ctg aag aaa gtg gat agc ctc aag aaa gca ttt tct cgc cag 893
Ser Ser Leu Lys Lys Val Asp Ser Leu Lys Lys Ala Phe Ser Arg Gln
    235                 240                 245
aac atc gag aaa aag atg aac aag ctg ggg aca aag atc gta tct gta 941
Asn Ile Glu Lys Lys Met Asn Lys Leu Gly Thr Lys Ile Val Ser Val
250                 255                 260                 265
gag agg aga gag aag att aag aaa tct ctc acg tca aat cac cag aaa 989
Glu Arg Arg Glu Lys Ile Lys Lys Ser Leu Thr Ser Asn His Gln Lys
                270                 275                 280
ata tcc tca gga aaa agc tcc ccc ttc aag gtt tct ccc ctc act ttc 1037
Ile Ser Ser Gly Lys Ser Ser Pro Phe Lys Val Ser Pro Leu Thr Phe
            285                 290                 295
ggg cgg aag aaa gtc cga gag gga gaa agc cat gca gaa aat gag acc 1085
Gly Arg Lys Lys Val Arg Glu Gly Glu Ser His Ala Glu Asn Glu Thr
        300                 305                 310
aag tca gaa gac ctg cct agc agt gag cag atg cca aat gac cag gaa 1133
Lys Ser Glu Asp Leu Pro Ser Ser Glu Gln Met Pro Asn Asp Gln Glu
    315                 320                 325
gag gag tcc ttt gca gag ggt cat tcc gaa gcg tcc ctc gcc agc gct 1181
Glu Glu Ser Phe Ala Glu Gly His Ser Glu Ala Ser Leu Ala Ser Ala
330                 335                 340                 345

ctg gtg gaa ggg gaa att gca gag gag gct gct gag aag gcg acc tcc 1229
Leu Val Glu Gly Glu Ile Ala Glu Glu Ala Ala Glu Lys Ala Thr Ser
                350                 355                 360
agg ggg agt aac tcg ggg atg gac agc aac atc gac ttg act att gtg 1277
Arg Gly Ser Asn Ser Gly Met Asp Ser Asn Ile Asp Leu Thr Ile Val
            365                 370                 375
gaa gat gaa gag gag gag tca gtg gcc ctg gaa cag gca cag aag gta 1325
Glu Asp Glu Glu Glu Glu Ser Val Ala Leu Glu Gln Ala Gln Lys Val
        380                 385                 390
cgc tat gag ggt agc tac gcg cta aca tcc gag gag gcg gag cgc tcc 1373
Arg Tyr Glu Gly Ser Tyr Ala Leu Thr Ser Glu Glu Ala Glu Arg Ser
    395                 400                 405
gat ggg gac ccc gtg cag ccc gcc gtg ctc cag gtg cac cag acc tcc 1421
Asp Gly Asp Pro Val Gln Pro Ala Val Leu Gln Val His Gln Thr Ser
410                 415                 420                 425
tgagcttaga gccaccgtgc catcctgtgc tgtgctcaag cgggcagcca gggctgaaga 1481
acaaactctt gcacatctcc agcacgactc acccactcct gcgttcctgt ccaggcagta 1541
atcattgacc atatagtcat agtaagacac acgagaccag gctttaccat gaaagcgacc 1601
tgtcacggac tccactttta atttgctctt aggttctatc tctgtagaat gtctccaaga 1661
ttgaagaaga aactgagcag ttgaaaaatg ctaatctctt tgacttagtc agaaaaaaac 1721
agaggataat taagatacta gtcatgaaaa gtgattcatt cttttttgtc attccatc 1779

<210> 70
<211> 425
<212> PRT
<213> Homo sapiens
<400> 70

Met Gly Glu Asp Ala Ala Gln Ala Glu Lys Phe Gln His Pro Gly Ser
1               5                   10                  15
Asp Met Arg Gln Glu Lys Pro Ser Ser Pro Ser Pro Met Pro Ser Ser
            20                  25                  30
Thr Pro Ser Pro Ser Leu Asn Leu Gly Asn Thr Glu Glu Ala Ile Arg
        35                  40                  45
Asp Asn Ser Gln Val Asn Ala Val Thr Val Leu Thr Leu Leu Asp Lys
    50                  55                  60
Leu Val Asn Met Leu Asp Ala Val Gln Glu Asn Gln His Lys Met Glu
65                  70                  75                  80
Gln Arg Gln Ile Ser Leu Glu Gly Ser Val Lys Gly Ile Gln Asn Asp
                85                  90                  95
Leu Thr Lys Leu Ser Lys Tyr Gln Ala Ser Thr Ser Asn Thr Val Ser
            100                 105                 110
Lys Leu Leu Glu Lys Ser Arg Lys Val Ser Ala His Thr Arg Ala Val
        115                 120                 125
Lys Glu Arg Met Asp Arg Gln Cys Ala Gln Val Lys Arg Leu Glu Asn
    130                 135                 140
Asn His Ala Gln Leu Leu Arg Arg Asn His Phe Lys Val Leu Ile Phe
145                 150                 155                 160
Gln Glu Glu Asn Glu Ile Pro Ala Ser Val Phe Val Lys Gln Pro Val
                165                 170                 175
Ser Gly Ala Val Glu Gly Lys Glu Glu Leu Pro Asp Glu Asn Lys Ser
            180                 185                 190
Leu Glu Glu Thr Leu His Thr Val Asp Leu Ser Ser Asp Asp Asp Leu
        195                 200                 205

Pro His Asp Glu Glu Ala Leu Glu Asp Ser Ala Glu Glu Lys Val Glu
    210                 215                 220
Glu Ser Arg Ala Glu Lys Ile Lys Arg Ser Ser Leu Lys Lys Val Asp
225                 230                 235                 240
Ser Leu Lys Lys Ala Phe Ser Arg Gln Asn Ile Glu Lys Lys Met Asn
                245                 250                 255
Lys Leu Gly Thr Lys Ile Val Ser Val Glu Arg Arg Glu Lys Ile Lys
            260                 265                 270
Lys Ser Leu Thr Ser Asn His Gln Lys Ile Ser Ser Gly Lys Ser Ser
        275                 280                 285
Pro Phe Lys Val Ser Pro Leu Thr Phe Gly Arg Lys Lys Val Arg Glu
    290                 295                 300
Gly Glu Ser His Ala Glu Asn Glu Thr Lys Ser Glu Asp Leu Pro Ser
305                 310                 315                 320
Ser Glu Gln Met Pro Asn Asp Gln Glu Glu Glu Ser Phe Ala Glu Gly
                325                 330                 335
His Ser Glu Ala Ser Leu Ala Ser Ala Leu Val Glu Gly Glu Ile Ala
            340                 345                 350
Glu Glu Ala Ala Glu Lys Ala Thr Ser Arg Gly Ser Asn Ser Gly Met
        355                 360                 365
Asp Ser Asn Ile Asp Leu Thr Ile Val Glu Asp Glu Glu Glu Glu Ser
    370                 375                 380
Val Ala Leu Glu Gln Ala Gln Lys Val Arg Tyr Glu Gly Ser Tyr Ala
385                 390                 395                 400
Leu Thr Ser Glu Glu Ala Glu Arg Ser Asp Gly Asp Pro Val Gln Pro
                405                 410                 415
Ala Val Leu Gln Val His Gln Thr Ser

            420                 425

<210> 71

<211> 2638

<212> DNA

<213> Homo sapiens

<220>

<221> CDS

<222> (288)..(1844)

<400> 71

gaggaaaggg gaaatgcggc ccgctcccca ctcagtgcca ctctgtgcca ctccgtgcca 60

ggccctgagg gcacccggtt gctgcttcct tccgtctttc cccaaggact atcagagatg 120

ccagcgtgac ccctgacacg tgtgtgcagc agcctgcagc tgccccaagc catggctgaa 180

cactgactcc cagctgtggg cttcaccatt acagactccc cagggcttca aagacttctc 240

agcttcgagc atggcttttg gctgtcaggg cagctgtaca atagtgg atg ttt gag 296

Met Phe Glu

1

acg gag gca gat gag aag agg gag atg gcc ttg gag gaa ggg aag ggg 344

Thr Glu Ala Asp Glu Lys Arg Glu Met Ala Leu Glu Glu Gly Lys Gly

    5                   10                  15

cct ggt gcc gag gat tcc cca ccc agc aag gag ccc tct cct ggc cag 392

Pro Gly Ala Glu Asp Ser Pro Pro Ser Lys Glu Pro Ser Pro Gly Gln

20                  25                  30                  35

gag ctt cct cca gga caa gac ctt cca ccc aac aag gac tcc cct tct 440

Glu Leu Pro Pro Gly Gln Asp Leu Pro Pro Asn Lys Asp Ser Pro Ser

                40                  45                  50

ggg cag gaa ccc gct ccc agc caa gaa cca ctg tcc agc aaa gac tca 488

Gly Gln Glu Pro Ala Pro Ser Gln Glu Pro Leu Ser Ser Lys Asp Ser
            55                  60                  65
gct acc tct gaa gga tcc cct cca ggc cca gat gct ccg ccc agc aag 536
Ala Thr Ser Glu Gly Ser Pro Pro Gly Pro Asp Ala Pro Pro Ser Lys
        70                  75                  80
gat gtg cca cca tgc cag gaa ccc cct cca gcc caa gac ctc tca ccc 584
Asp Val Pro Pro Cys Gln Glu Pro Pro Pro Ala Gln Asp Leu Ser Pro
    85                  90                  95
tgc cag gac cta cct gct ggt caa gaa ccc ctg cct cac cag gac cct 632
Cys Gln Asp Leu Pro Ala Gly Gln Glu Pro Leu Pro His Gln Asp Pro
100                 105                 110                 115
cta ctc acc aaa gac ctc cct gcc atc cag gaa tcc ccc acc cgg gac 680
Leu Leu Thr Lys Asp Leu Pro Ala Ile Gln Glu Ser Pro Thr Arg Asp
                120                 125                 130
ctt cca ccc tgt caa gat ctg cct cct agc cag gtc tcc ctg cca gcc 728
Leu Pro Pro Cys Gln Asp Leu Pro Pro Ser Gln Val Ser Leu Pro Ala
            135                 140                 145
aag gcc ctt act gag gac acc atg agc tcc ggg gac cta cta gca gct 776
Lys Ala Leu Thr Glu Asp Thr Met Ser Ser Gly Asp Leu Leu Ala Ala
        150                 155                 160
act ggg gac cca cct gcg gcc ccc agg cca gcc ttc gtg atc cct gag 824
Thr Gly Asp Pro Pro Ala Ala Pro Arg Pro Ala Phe Val Ile Pro Glu
    165                 170                 175
gtc cgg ctg gat agc acc tac agc cag aag gca ggg gca gag cag ggc 872
Val Arg Leu Asp Ser Thr Tyr Ser Gln Lys Ala Gly Ala Glu Gln Gly
180                 185                 190                 195

tgc tcg gga gat gag gag gat gca gaa gag gcc gag gag gtg gag gag 920
Cys Ser Gly Asp Glu Glu Asp Ala Glu Glu Ala Glu Glu Val Glu Glu
                200                 205                 210
ggg gag gaa ggg gag gag gac gag gat gag gac acc agc gat gac aac 968
Gly Glu Glu Gly Glu Glu Asp Glu Asp Glu Asp Thr Ser Asp Asp Asn
            215                 220                 225
tac gga gag cgc agt gag gcc aag cgc agc agc atg atc gag acg ggc 1016
Tyr Gly Glu Arg Ser Glu Ala Lys Arg Ser Ser Met Ile Glu Thr Gly
        230                 235                 240
cag ggg gct gag ggt ggc ctc tca ctg cgt gtg cag aac tcg ctg cgg 1064
Gln Gly Ala Glu Gly Gly Leu Ser Leu Arg Val Gln Asn Ser Leu Arg
    245                 250                 255
cgc cgg acg cac agc gag ggc agc ctg ctg cag gag ccc cga ggg ccc 1112
Arg Arg Thr His Ser Glu Gly Ser Leu Leu Gln Glu Pro Arg Gly Pro
260                 265                 270                 275
tgc ttt gcc tcc gac acc acc ttg cac tgc tca gac ggt gag ggc gcc 1160
Cys Phe Ala Ser Asp Thr Thr Leu His Cys Ser Asp Gly Glu Gly Ala
                280                 285                 290
gcc tcc acc tgg ggc atg cct tcg ccc agc acc ctc aag aaa gag ctg 1208
Ala Ser Thr Trp Gly Met Pro Ser Pro Ser Thr Leu Lys Lys Glu Leu
            295                 300                 305
ggc cgc aat ggt ggc tcc atg cac cac ctt tcc ctc ttc ttc aca gga 1256
Gly Arg Asn Gly Gly Ser Met His His Leu Ser Leu Phe Phe Thr Gly
        310                 315                 320
cac agg aag atg agc ggg gct gac acc gtt ggg gat gat gac gaa gcc 1304
His Arg Lys Met Ser Gly Ala Asp Thr Val Gly Asp Asp Asp Glu Ala
    325                 330                 335

tcc cgg aag aga aag agc aaa aac cta gcc aag gac atg aag aac aag 1352
Ser Arg Lys Arg Lys Ser Lys Asn Leu Ala Lys Asp Met Lys Asn Lys
340                 345                 350                 355
ctg ggg atc ttc aga cgg cgg aat gag tcc cct gga gcc cct ccc gcg 1400
Leu Gly Ile Phe Arg Arg Arg Asn Glu Ser Pro Gly Ala Pro Pro Ala
                360                 365                 370
ggc aag gca gac aaa atg atg aag tca ttc aag ccc acc tca gag gaa 1448
Gly Lys Ala Asp Lys Met Met Lys Ser Phe Lys Pro Thr Ser Glu Glu
            375                 380                 385
gcc ctc aag tgg ggc gag tcc ttg gag aag ctg ctg gtt cac aaa tac 1496
Ala Leu Lys Trp Gly Glu Ser Leu Glu Lys Leu Leu Val His Lys Tyr
        390                 395                 400
ggg tta gca gtg ttc caa gcc ttc ctt cgc act gag ttc agt gag gag 1544
Gly Leu Ala Val Phe Gln Ala Phe Leu Arg Thr Glu Phe Ser Glu Glu
    405                 410                 415
aat ctg gag ttc tgg ttg gct tgt gag gac ttc aag aag gtc aag tca 1592
Asn Leu Glu Phe Trp Leu Ala Cys Glu Asp Phe Lys Lys Val Lys Ser
420                 425                 430                 435
cag tcc aag atg gca tcc aag gcc aag aag atc ttt gct gaa tac atc 1640
Gln Ser Lys Met Ala Ser Lys Ala Lys Lys Ile Phe Ala Glu Tyr Ile
                440                 445                 450
gcg atc cag gca tgc aag gag gtc aac ctg gac tcc tac acg cgg gag 1688
Ala Ile Gln Ala Cys Lys Glu Val Asn Leu Asp Ser Tyr Thr Arg Glu
            455                 460                 465
cac acc aag gac aac ctg cag agc gtc acg cgg ggc tgc ttc gac ctg 1736
His Thr Lys Asp Asn Leu Gln Ser Val Thr Arg Gly Cys Phe Asp Leu

        470                 475                 480

gca cag aag cgc atc ttc ggg ctc atg gaa aag gac tcg tac cct cgc 1784

Ala Gln Lys Arg Ile Phe Gly Leu Met Glu Lys Asp Ser Tyr Pro Arg

    485                 490                 495

ttt ctc cgt tct gac ctc tac ctg gac ctt att aac cag aag aag atg 1832

Phe Leu Arg Ser Asp Leu Tyr Leu Asp Leu Ile Asn Gln Lys Lys Met

500                 505                 510                 515

agt ccc ccg ctt taggggccac tggagtcgag ctcagcgttc acaccaggcg 1884

Ser Pro Pro Leu

ggctgggtcc cctgcccacc tgcctccctg ccccctgtga cggagggggc aagcaagccc 1944

ccagaggccg tgtctctgga cagacggata gacatacgga agcgaggcct ggaccaagag 2004

aggcccaggc tactggagga gtagaaggat gggccccgtg gggtccccac tgccccggta 2064

cgagggggcc caagaccctg gcaggtcagg ggccctggcc aagccagatc tggagctgct 2124

gctccctgct gcggagaccg cggaggcttc gcgttgacca agttccttaa agaactggct 2184

gatggggcag gaggtccagg cctgggctct cgggccctcc tagagggcca ttggagcttg 2244

cagctcagac cccccacttg agttttattt atttaaatag tagttggatg cttggcacgt 2304

cgtcctgtaa taggaaaccc ttgcctcatc agttttcctg atttacaagt gcaatatttt 2364

agccaatgcc ttgggagaag ctgccatgca aaggtggaca ccattctcca gcttcagggg 2424

atatgctcgt cccgggcacc ggtggcaggc agctggcctt ctggactaag gcagcctggg 2484

gggacactgc agtctggcta cacacagaga tctggcaccc cctgggtgga gtgtccctcg 2544

ggggctttgg gaaagcatgg caccctcaga ccacacagta gccaagttct ggagcaaata 2604

aaaggcctgt gttatttctt gttcttgaaa aaaa 2638

<210> 72

<211> 519

<212> PRT

<213> Homo sapiens

<400> 72

Met Phe Glu Thr Glu Ala Asp Glu Lys Arg Glu Met Ala Leu Glu Glu

1               5                   10                  15

Gly Lys Gly Pro Gly Ala Glu Asp Ser Pro Pro Ser Lys Glu Pro Ser

            20                  25                  30

Pro Gly Gln Glu Leu Pro Pro Gly Gln Asp Leu Pro Pro Asn Lys Asp

        35                  40                  45

Ser Pro Ser Gly Gln Glu Pro Ala Pro Ser Gln Glu Pro Leu Ser Ser

    50                  55                  60

Lys Asp Ser Ala Thr Ser Glu Gly Ser Pro Pro Gly Pro Asp Ala Pro

65                  70                  75                  80

Pro Ser Lys Asp Val Pro Pro Cys Gln Glu Pro Pro Pro Ala Gln Asp

                85                  90                  95

Leu Ser Pro Cys Gln Asp Leu Pro Ala Gly Gln Glu Pro Leu Pro His

            100                 105                 110

Gln Asp Pro Leu Leu Thr Lys Asp Leu Pro Ala Ile Gln Glu Ser Pro

        115                 120                 125

Thr Arg Asp Leu Pro Pro Cys Gln Asp Leu Pro Pro Ser Gln Val Ser

    130                 135                 140

Leu Pro Ala Lys Ala Leu Thr Glu Asp Thr Met Ser Ser Gly Asp Leu

145                 150                 155                 160

Leu Ala Ala Thr Gly Asp Pro Pro Ala Ala Pro Arg Pro Ala Phe Val

                165                 170                 175

Ile Pro Glu Val Arg Leu Asp Ser Thr Tyr Ser Gln Lys Ala Gly Ala

            180                 185                 190

Glu Gln Gly Cys Ser Gly Asp Glu Glu Asp Ala Glu Glu Ala Glu Glu

        195                 200                 205

Val Glu Glu Gly Glu Glu Gly Glu Glu Asp Glu Asp Glu Asp Thr Ser
    210                 215                 220
Asp Asp Asn Tyr Gly Glu Arg Ser Glu Ala Lys Arg Ser Ser Met Ile
225                 230                 235                 240
Glu Thr Gly Gln Gly Ala Glu Gly Gly Leu Ser Leu Arg Val Gln Asn
                245                 250                 255
Ser Leu Arg Arg Arg Thr His Ser Glu Gly Ser Leu Leu Gln Glu Pro
            260                 265                 270
Arg Gly Pro Cys Phe Ala Ser Asp Thr Thr Leu His Cys Ser Asp Gly
        275                 280                 285
Glu Gly Ala Ala Ser Thr Trp Gly Met Pro Ser Pro Ser Thr Leu Lys
    290                 295                 300
Lys Glu Leu Gly Arg Asn Gly Gly Ser Met His His Leu Ser Leu Phe
305                 310                 315                 320
Phe Thr Gly His Arg Lys Met Ser Gly Ala Asp Thr Val Gly Asp Asp
                325                 330                 335
Asp Glu Ala Ser Arg Lys Arg Lys Ser Lys Asn Leu Ala Lys Asp Met
            340                 345                 350
Lys Asn Lys Leu Gly Ile Phe Arg Arg Arg Asn Glu Ser Pro Gly Ala
        355                 360                 365
Pro Pro Ala Gly Lys Ala Asp Lys Met Met Lys Ser Phe Lys Pro Thr
    370                 375                 380
Ser Glu Glu Ala Leu Lys Trp Gly Glu Ser Leu Glu Lys Leu Leu Val
385                 390                 395                 400
His Lys Tyr Gly Leu Ala Val Phe Gln Ala Phe Leu Arg Thr Glu Phe
                405                 410                 415

Ser Glu Glu Asn Leu Glu Phe Trp Leu Ala Cys Glu Asp Phe Lys Lys
            420                 425                 430
Val Lys Ser Gln Ser Lys Met Ala Ser Lys Ala Lys Lys Ile Phe Ala
        435                 440                 445
Glu Tyr Ile Ala Ile Gln Ala Cys Lys Glu Val Asn Leu Asp Ser Tyr
    450                 455                 460
Thr Arg Glu His Thr Lys Asp Asn Leu Gln Ser Val Thr Arg Gly Cys
465                 470                 475                 480
Phe Asp Leu Ala Gln Lys Arg Ile Phe Gly Leu Met Glu Lys Asp Ser
                485                 490                 495
Tyr Pro Arg Phe Leu Arg Ser Asp Leu Tyr Leu Asp Leu Ile Asn Gln
            500                 505                 510
Lys Lys Met Ser Pro Pro Leu
        515

<210> 73
<211> 1901
<212> DNA
<213> Homo sapiens
<220>
<221> CDS
<222> (250)..(1206)
<400> 73
aaaaaacagc agggttagct tgtccctccc ctccctcttc agcttcccag acactgattc 60
tggaatgaaa attcacctgc ctctgagttg gctcctaatg ggggtgggag tgttacttcg 120
gttcccaggt tggaagatta tctcacccgg ccccagctat ataagctgac cggtgtggag 180
gggcccagca gggccaactc cagggattcc ttccacgaca gaaaaacata caagactcct 240

tcagccaac atg atg gta ctg aaa gta gag gaa ctg gtc act gga aag aag 291
Met Met Val Leu Lys Val Glu Glu Leu Val Thr Gly Lys Lys
1               5                   10
aat ggc aat ggg gag gca ggg gaa ttc ctt cct gag gat ttc aga gat 339
Asn Gly Asn Gly Glu Ala Gly Glu Phe Leu Pro Glu Asp Phe Arg Asp
15                  20                  25                  30
gga gag tat gaa gct gct gtt act tta gag aag cag gag gat ctg aag 387
Gly Glu Tyr Glu Ala Ala Val Thr Leu Glu Lys Gln Glu Asp Leu Lys
                35                  40                  45
aca ctt cta gcc cac cct gtg acc ctg ggg gag caa cag tgg aaa agc 435
Thr Leu Leu Ala His Pro Val Thr Leu Gly Glu Gln Gln Trp Lys Ser
            50                  55                  60
gag aaa caa cga gag gca gag ctc cca aag aaa aaa cta gaa caa aga 483
Glu Lys Gln Arg Glu Ala Glu Leu Pro Lys Lys Lys Leu Glu Gln Arg
        65                  70                  75
tcc aag ctt gaa aat tta gaa gac ctt gaa ata atc att caa ctg aag 531
Ser Lys Leu Glu Asn Leu Glu Asp Leu Glu Ile Ile Ile Gln Leu Lys
    80                  85                  90
aaa agg aaa aaa tac agg aaa act aaa gtt cca gtt gta aag gaa cca 579
Lys Arg Lys Lys Tyr Arg Lys Thr Lys Val Pro Val Val Lys Glu Pro
95                  100                 105                 110
gaa cct gaa atc att acg gaa cct gtg gat gtg cct acg ttt ctg aag 627
Glu Pro Glu Ile Ile Thr Glu Pro Val Asp Val Pro Thr Phe Leu Lys
                115                 120                 125
gct gct ctg gag aat aaa ctg cca gta gta gaa aaa ttc ttg tca gac 675
Ala Ala Leu Glu Asn Lys Leu Pro Val Val Glu Lys Phe Leu Ser Asp
            130                 135                 140

aag aac aat cca gat gtt tgt gat gag tat aaa cgg aca gct ctt cat 723
Lys Asn Asn Pro Asp Val Cys Asp Glu Tyr Lys Arg Thr Ala Leu His
        145                 150                 155
aga gca tgc ttg gaa gga cat ttg gca att gtg gag aag tta atg gaa 771
Arg Ala Cys Leu Glu Gly His Leu Ala Ile Val Glu Lys Leu Met Glu
    160                 165                 170
gct gga gcc cag atc gaa ttc cgt gat atg ctt gaa tcc aca gcc atc 819
Ala Gly Ala Gln Ile Glu Phe Arg Asp Met Leu Glu Ser Thr Ala Ile
175                 180                 185                 190
cac tgg gca agc cgt gga gga aac ctg gat gtt tta aaa ttg ttg ctg 867
His Trp Ala Ser Arg Gly Gly Asn Leu Asp Val Leu Lys Leu Leu Leu
                195                 200                 205
aat aaa gga gca aaa att agc gcc cga gat aag ttg ctc agc aca gcg 915
Asn Lys Gly Ala Lys Ile Ser Ala Arg Asp Lys Leu Leu Ser Thr Ala
            210                 215                 220
ctg cat gtg gcg gtg agg act ggc cac tat gag tgc gcg gag cat ctt 963
Leu His Val Ala Val Arg Thr Gly His Tyr Glu Cys Ala Glu His Leu
        225                 230                 235
atc gcc tgt gag gca gac ctc aac gcc aaa gac aga gaa gga gat acc 1011
Ile Ala Cys Glu Ala Asp Leu Asn Ala Lys Asp Arg Glu Gly Asp Thr
    240                 245                 250
ccg ttg cat gat gcg gtg aga ctg aac cgc tat aag atg atc cga ctc 1059
Pro Leu His Asp Ala Val Arg Leu Asn Arg Tyr Lys Met Ile Arg Leu
255                 260                 265                 270
ctg att atg tat ggc gcg gat ctc aac atc aag aac tgt gct ggg aag 1107
Leu Ile Met Tyr Gly Ala Asp Leu Asn Ile Lys Asn Cys Ala Gly Lys

                275                 280                 285

acg ccg atg gat ctg gtg cta cac tgg cag aat gga acc aaa gca ata 1155

Thr Pro Met Asp Leu Val Leu His Trp Gln Asn Gly Thr Lys Ala Ile

            290                 295                 300

ttc gac agc ctc aga gag aac tcc tac aag acc tct cgc ata gct aca 1203

Phe Asp Ser Leu Arg Glu Asn Ser Tyr Lys Thr Ser Arg Ile Ala Thr

        305                 310                 315

ttc tgaggcaaac gacagactct taatcagtaa atgttcactg gcattttgaa 1256

Phe

ggcatggccc aggagaagag acactagcca taaaatctag tttctattta tcaacgtgtt 1316

gtgaagatgt acctaatgaa gttttgagaa agcacagggt tataggtgtt taaatttcct 1376

ttagtgaaac tcttatttat ttttatgtat tcctgtttat ttatttactg ccacgctact 1436

gatattcaga ccttcatgat catccatctg gtgagcagag cttcatttgt atataacact 1496

ttcagagcct tcccacccat aggtagttct taaaccaggt gaaagagcaa agttcaagtg 1556

cctacttatg tgtcattcgc tcatgtaaga gtttttaaga gagggctgat tatcacagcc 1616

ctcttttctc ctgaattttt aatgcagaag tttgaatgaa gcaagggaag gcatgtaggg 1676

acaggaaagg aaacaatgga aggaaagtga ttctgtgaaa aggacagtga agccagctat 1736

tttaccccca ggctggattt tttttttttt tttttttttt ttttttttta ccgagtacac 1796

agagtaccca agtgaagaga acgtcatgag tgtaagtgca aatcagtgga aggagcggca 1856

aactgggaca tgcagaattg aatttgctca aaaaaaaaaa aaaaa 1901

<210> 74

<211> 319

<212> PRT

<213> Homo sapiens

<400> 74

Met Met Val Leu Lys Val Glu Glu Leu Val Thr Gly Lys Lys Asn Gly

1                   5                   10                  15

Asn Gly Glu Ala Gly Glu Phe Leu Pro Glu Asp Phe Arg Asp Gly Glu

                20                  25                  30

Tyr Glu Ala Ala Val Thr Leu Glu Lys Gln Glu Asp Leu Lys Thr Leu

            35                  40                  45

Leu Ala His Pro Val Thr Leu Gly Glu Gln Gln Trp Lys Ser Glu Lys

        50                  55                  60

Gln Arg Glu Ala Glu Leu Pro Lys Lys Lys Leu Glu Gln Arg Ser Lys

65                  70                  75                  80

Leu Glu Asn Leu Glu Asp Leu Glu Ile Ile Ile Gln Leu Lys Lys Arg

                85                  90                  95

Lys Lys Tyr Arg Lys Thr Lys Val Pro Val Val Lys Glu Pro Glu Pro

            100                 105                 110

Glu Ile Ile Thr Glu Pro Val Asp Val Pro Thr Phe Leu Lys Ala Ala

        115                 120                 125

Leu Glu Asn Lys Leu Pro Val Val Glu Lys Phe Leu Ser Asp Lys Asn

    130                 135                 140

Asn Pro Asp Val Cys Asp Glu Tyr Lys Arg Thr Ala Leu His Arg Ala

145                 150                 155                 160

Cys Leu Glu Gly His Leu Ala Ile Val Glu Lys Leu Met Glu Ala Gly

                165                 170                 175

Ala Gln Ile Glu Phe Arg Asp Met Leu Glu Ser Thr Ala Ile His Trp

            180                 185                 190

Ala Ser Arg Gly Gly Asn Leu Asp Val Leu Lys Leu Leu Leu Asn Lys

        195                 200                 205

Gly Ala Lys Ile Ser Ala Arg Asp Lys Leu Leu Ser Thr Ala Leu His

    210                 215                 220

Val Ala Val Arg Thr Gly His Tyr Glu Cys Ala Glu His Leu Ile Ala
225                 230                 235                 240
Cys Glu Ala Asp Leu Asn Ala Lys Asp Arg Glu Gly Asp Thr Pro Leu
                245                 250                 255
His Asp Ala Val Arg Leu Asn Arg Tyr Lys Met Ile Arg Leu Leu Ile
            260                 265                 270
Met Tyr Gly Ala Asp Leu Asn Ile Lys Asn Cys Ala Gly Lys Thr Pro
        275                 280                 285
Met Asp Leu Val Leu His Trp Gln Asn Gly Thr Lys Ala Ile Phe Asp
    290                 295                 300
Ser Leu Arg Glu Asn Ser Tyr Lys Thr Ser Arg Ile Ala Thr Phe
305                 310                 315
<210> 75
<211> 5613
<212> DNA
<213> Homo sapiens
<220>
<221> CDS
<222> (118)..(5475)
<400> 75
cccggagcag ggcgagagct cgcgtcgccg gaaaggaaga cgggaagaaa gggcaggcgg 60
ctcggcgggc gtcttctcca ctcctctgcc gcgtccccgt ggctgcaggg agccggc 117
atg ggg ctt ctc cag ttg cta gct ttc agt ttc tta gcc ctg tgc aga 165
Met Gly Leu Leu Gln Leu Leu Ala Phe Ser Phe Leu Ala Leu Cys Arg
1               5                   10                  15
gcc cga gtg cgc gct cag gaa ccc gag ttc agc tac ggc tgc gca gaa 213

Ala Arg Val Arg Ala Gln Glu Pro Glu Phe Ser Tyr Gly Cys Ala Glu
            20                  25                  30
ggc agc tgc tat ccc gcc acg ggc gac ctt ctc atc ggc cga gca cag 261
Gly Ser Cys Tyr Pro Ala Thr Gly Asp Leu Leu Ile Gly Arg Ala Gln
        35                  40                  45
aag ctt tcg gtg acc tcg acg tgc ggg ctg cac aag ccc gaa ccc tac 309
Lys Leu Ser Val Thr Ser Thr Cys Gly Leu His Lys Pro Glu Pro Tyr
    50                  55                  60
tgt atc gtc agc cac ttg cag gag gac aaa aaa tgc ttc ata tgc aat 357
Cys Ile Val Ser His Leu Gln Glu Asp Lys Lys Cys Phe Ile Cys Asn
65                  70                  75                  80
tcc caa gat cct tat cat gag acc ctg aat cct gac agc cat ctc att 405
Ser Gln Asp Pro Tyr His Glu Thr Leu Asn Pro Asp Ser His Leu Ile
                85                  90                  95
gaa aat gtg gtc act aca ttt gct cca aac cgc ctt aag att tgg tgg 453
Glu Asn Val Val Thr Thr Phe Ala Pro Asn Arg Leu Lys Ile Trp Trp
            100                 105                 110
caa tct gaa aat ggt gtg gaa aat gta act atc caa ctg gat ttg gaa 501
Gln Ser Glu Asn Gly Val Glu Asn Val Thr Ile Gln Leu Asp Leu Glu
        115                 120                 125
gca gaa ttc cat ttt act cat ctc ata atg act ttc aag aca ttc cgt 549
Ala Glu Phe His Phe Thr His Leu Ile Met Thr Phe Lys Thr Phe Arg
    130                 135                 140
cca gct gct atg ctg ata gaa cga tcg tcc gac ttt ggg aaa acc tgg 597
Pro Ala Ala Met Leu Ile Glu Arg Ser Ser Asp Phe Gly Lys Thr Trp
145                 150                 155                 160

ggt gtg tat aga tac ttc gcc tat gac tgt gag gcc tcg ttt cca ggc 645
Gly Val Tyr Arg Tyr Phe Ala Tyr Asp Cys Glu Ala Ser Phe Pro Gly
                165                 170                 175
att tca act ggc ccc atg aaa aaa gtc gat gac ata att tgt gat tct 693
Ile Ser Thr Gly Pro Met Lys Lys Val Asp Asp Ile Ile Cys Asp Ser
            180                 185                 190
cga tat tct gac att gaa ccc tca act gaa gga gag gtg ata ttt cgt 741
Arg Tyr Ser Asp Ile Glu Pro Ser Thr Glu Gly Glu Val Ile Phe Arg
        195                 200                 205
gct tta gat cct gct ttc aaa ata gaa gat cct tat agc cca agg ata 789
Ala Leu Asp Pro Ala Phe Lys Ile Glu Asp Pro Tyr Ser Pro Arg Ile
    210                 215                 220
cag aat tta tta aaa att acc aac ttg aga atc aag ttt gtg aaa ctg 837
Gln Asn Leu Leu Lys Ile Thr Asn Leu Arg Ile Lys Phe Val Lys Leu
225                 230                 235                 240
cat act ttg gga gat aac ctt ctg gat tcc agg atg gaa atc aga gaa 885
His Thr Leu Gly Asp Asn Leu Leu Asp Ser Arg Met Glu Ile Arg Glu
                245                 250                 255
aag tat tat tat gca gtt tat gat atg gtg gtt cga gga aat tgc ttc 933
Lys Tyr Tyr Tyr Ala Val Tyr Asp Met Val Val Arg Gly Asn Cys Phe
            260                 265                 270
tgc tat ggt cat gcc agc gaa tgt gcc cct gtg gat gga ttc aat gaa 981
Cys Tyr Gly His Ala Ser Glu Cys Ala Pro Val Asp Gly Phe Asn Glu
        275                 280                 285
gaa gtg gaa gga atg gtt cac gga cac tgc atg tgc agg cat aac acc 1029
Glu Val Glu Gly Met Val His Gly His Cys Met Cys Arg His Asn Thr
    290                 295                 300

aag ggc tta aac tgt gaa ctc tgc atg gat ttc tac cat gat tta cct 1077
Lys Gly Leu Asn Cys Glu Leu Cys Met Asp Phe Tyr His Asp Leu Pro
305                 310                 315                 320
tgg aga cct gct gaa ggc cga aac agc aac gcc tgt aaa aaa tgt aac 1125
Trp Arg Pro Ala Glu Gly Arg Asn Ser Asn Ala Cys Lys Lys Cys Asn
                325                 330                 335
tgc aat gaa cat tcc atc tct tgt cac ttt gac atg gct gtt tac ctg 1173
Cys Asn Glu His Ser Ile Ser Cys His Phe Asp Met Ala Val Tyr Leu
            340                 345                 350
gcc acg ggg aac gtc agc gga ggc gtg tgt gat gac tgt cag cac aac 1221
Ala Thr Gly Asn Val Ser Gly Gly Val Cys Asp Asp Cys Gln His Asn
        355                 360                 365
acc atg ggg cgc aac tgt gag cag tgc aag ccg ttt tac tac cag cac 1269
Thr Met Gly Arg Asn Cys Glu Gln Cys Lys Pro Phe Tyr Tyr Gln His
    370                 375                 380
cca gag agg gac atc cga gat cct aat ttc tgt gaa cga tgt acg tgt 1317
Pro Glu Arg Asp Ile Arg Asp Pro Asn Phe Cys Glu Arg Cys Thr Cys
385                 390                 395                 400
gac cca gct ggc tct caa aat gag gga att tgt gac agc tat act gat 1365
Asp Pro Ala Gly Ser Gln Asn Glu Gly Ile Cys Asp Ser Tyr Thr Asp
                405                 410                 415
ttt tct act ggt ctc att gct ggc cag tgt cgg tgt aaa tta aat gtg 1413
Phe Ser Thr Gly Leu Ile Ala Gly Gln Cys Arg Cys Lys Leu Asn Val
            420                 425                 430
gaa gga gaa cat tgt gat gtt tgc aaa gaa ggc ttc tat gat tta agc 1461
Glu Gly Glu His Cys Asp Val Cys Lys Glu Gly Phe Tyr Asp Leu Ser

        435                 440                 445
agt gaa gat cca ttt ggt tgt aaa tct tgt gct tgc aat cct ctg gga 1509
Ser Glu Asp Pro Phe Gly Cys Lys Ser Cys Ala Cys Asn Pro Leu Gly
    450                 455                 460
aca att cct gga ggg aat cct tgt gat tcc gag aca ggt cac tgc tac 1557
Thr Ile Pro Gly Gly Asn Pro Cys Asp Ser Glu Thr Gly His Cys Tyr
465                 470                 475                 480
tgc aag cgt ctg gtg aca gga cag cat tgt gac cag tgc ctg cca gag 1605
Cys Lys Arg Leu Val Thr Gly Gln His Cys Asp Gln Cys Leu Pro Glu
                485                 490                 495
cac tgg ggc tta agc aat gat ttg gat gga tgt cga cca tgt gac tgt 1653
His Trp Gly Leu Ser Asn Asp Leu Asp Gly Cys Arg Pro Cys Asp Cys
            500                 505                 510
gac ctt ggg gga gcc tta aac aac agt tgc ttt gcg gag tca ggc cag 1701
Asp Leu Gly Gly Ala Leu Asn Asn Ser Cys Phe Ala Glu Ser Gly Gln
        515                 520                 525
tgc tca tgc cgg cct cac atg att gga cgt cag tgc aac gaa gtg gaa 1749
Cys Ser Cys Arg Pro His Met Ile Gly Arg Gln Cys Asn Glu Val Glu
    530                 535                 540
cct ggt tac tac ttt gcc acc ctg gat cac tac ctc tat gaa gcg gag 1797
Pro Gly Tyr Tyr Phe Ala Thr Leu Asp His Tyr Leu Tyr Glu Ala Glu
545                 550                 555                 560
gaa gcc aac ttg ggg cct ggg gtt agc ata gtg gag cgg caa tat atc 1845
Glu Ala Asn Leu Gly Pro Gly Val Ser Ile Val Glu Arg Gln Tyr Ile
                565                 570                 575
cag gac cgg att ccc tcc tgg act gga gcc ggc ttc gtc cga gtg cct 1893
Gln Asp Arg Ile Pro Ser Trp Thr Gly Ala Gly Phe Val Arg Val Pro

            580                 585                 590

gaa ggg gct tat ttg gag ttt ttc att gac aac ata cca tat tcc atg 1941

Glu Gly Ala Tyr Leu Glu Phe Phe Ile Asp Asn Ile Pro Tyr Ser Met

        595                 600                 605

gag tac gac atc cta att cgc tac gag cca cag cta ccc gac cac tgg 1989

Glu Tyr Asp Ile Leu Ile Arg Tyr Glu Pro Gln Leu Pro Asp His Trp

    610                 615                 620

gaa aaa gct gtc atc aca gtg cag cga cct gga agg att cca acc agc 2037

Glu Lys Ala Val Ile Thr Val Gln Arg Pro Gly Arg Ile Pro Thr Ser

625                 630                 635                 640

agc cga tgt ggt aat acc atc ccc gat gat gac aac cag gtg gtg tca 2085

Ser Arg Cys Gly Asn Thr Ile Pro Asp Asp Asp Asn Gln Val Val Ser

                645                 650                 655

tta tca cca ggc tca aga tat gtc gtc ctt cct cgg ccg gtg tgc ttt 2133

Leu Ser Pro Gly Ser Arg Tyr Val Val Leu Pro Arg Pro Val Cys Phe

            660                 665                 670

gag aag gga aca aac tac acg gtg agg ttg gag ctg cct cag tac acc 2181

Glu Lys Gly Thr Asn Tyr Thr Val Arg Leu Glu Leu Pro Gln Tyr Thr

        675                 680                 685

tcc tct gat agc gac gtg gag agc ccc tac acg ctg atc gat tct ctt 2229

Ser Ser Asp Ser Asp Val Glu Ser Pro Tyr Thr Leu Ile Asp Ser Leu

    690                 695                 700

gtt ctc atg cca tac tgt aaa tca ctg gac atc ttc acc gtg gga ggt 2277

Val Leu Met Pro Tyr Cys Lys Ser Leu Asp Ile Phe Thr Val Gly Gly

705                 710                 715                 720

tca gga gat ggg gtg gtc acc aac agt gcc tgg gaa acc ttt cag aga 2325

Ser Gly Asp Gly Val Val Thr Asn Ser Ala Trp Glu Thr Phe Gln Arg
                725                 730                 735
tac cga tgt cta gag aac agc aga agc gtt gtg aaa aca ccg atg aca 2373
Tyr Arg Cys Leu Glu Asn Ser Arg Ser Val Val Lys Thr Pro Met Thr
            740                 745                 750
gat gtt tgc aga aac atc atc ttt agc att tct gcc ctg tta cac cag 2421
Asp Val Cys Arg Asn Ile Ile Phe Ser Ile Ser Ala Leu Leu His Gln
        755                 760                 765
aca ggc ctg gct tgt gaa tgc gac cct cag ggt tcg tta agt tcc gtg 2469
Thr Gly Leu Ala Cys Glu Cys Asp Pro Gln Gly Ser Leu Ser Ser Val
    770                 775                 780
tgt gat ccc aac gga ggc cag tgc cag tgc cgg ccc aac gtg gtt gga 2517
Cys Asp Pro Asn Gly Gly Gln Cys Gln Cys Arg Pro Asn Val Val Gly
785                 790                 795                 800
aga acc tgc aac aga tgt gca cct gga act ttt ggc ttt ggc ccc agt 2565
Arg Thr Cys Asn Arg Cys Ala Pro Gly Thr Phe Gly Phe Gly Pro Ser
                805                 810                 815
gga tgc aaa cct tgt gag tgc cat ctg caa gga tct gtc aat gcc ttc 2613
Gly Cys Lys Pro Cys Glu Cys His Leu Gln Gly Ser Val Asn Ala Phe
            820                 825                 830
tgc aat ccc gtc act ggc cag tgc cac tgt ttc cag gga gtg tat gct 2661
Cys Asn Pro Val Thr Gly Gln Cys His Cys Phe Gln Gly Val Tyr Ala
        835                 840                 845
cgg cag tgt gat cgg tgc tta cct ggg cac tgg ggc ttt cca agt tgc 2709
Arg Gln Cys Asp Arg Cys Leu Pro Gly His Trp Gly Phe Pro Ser Cys
    850                 855                 860
cag ccc tgc cag tgc aat ggc cac gcc gat gac tgc gac cca gtg act 2757

Gln Pro Cys Gln Cys Asn Gly His Ala Asp Asp Cys Asp Pro Val Thr
865                 870                 875                 880
ggg gag tgc ttg aac tgc cag gac tac acc atg ggt cat aac tgt gaa 2805
Gly Glu Cys Leu Asn Cys Gln Asp Tyr Thr Met Gly His Asn Cys Glu
                885                 890                 895
agg tgc ttg gct ggt tac tat ggc gac ccc atc att ggg tca ggt gat 2853
Arg Cys Leu Ala Gly Tyr Tyr Gly Asp Pro Ile Ile Gly Ser Gly Asp
            900                 905                 910
cac tgc cgc cct tgc cct tgc cca gat ggt ccc gac agt gga cgc cag 2901
His Cys Arg Pro Cys Pro Cys Pro Asp Gly Pro Asp Ser Gly Arg Gln
        915                 920                 925
ttt gcc agg agc tgc tac caa gat cct gtt act tta cag ctt gcc tgt 2949
Phe Ala Arg Ser Cys Tyr Gln Asp Pro Val Thr Leu Gln Leu Ala Cys
    930                 935                 940
gtt tgt gat cct gga tac att ggt tcc aga tgt gac gac tgt gcc tca 2997
Val Cys Asp Pro Gly Tyr Ile Gly Ser Arg Cys Asp Asp Cys Ala Ser
945                 950                 955                 960
gga tac ttt ggc aat cca tca gaa gtt ggg ggg tcg tgt cag cct tgc 3045
Gly Tyr Phe Gly Asn Pro Ser Glu Val Gly Gly Ser Cys Gln Pro Cys
                965                 970                 975
cag tgt cac aac aac att gac acg aca gac cca gaa gcc tgt gac aag 3093
Gln Cys His Asn Asn Ile Asp Thr Thr Asp Pro Glu Ala Cys Asp Lys
            980                 985                 990
gag act ggg agg tgt ctc aag tgc ctg tac cac acg gaa ggg gaa cac 3141
Glu Thr Gly Arg Cys Leu Lys Cys Leu Tyr His Thr Glu Gly Glu His
        995                 1000                1005

tgt cag ttc tgc cgg ttt gga tac tat ggt gat gcc ctc cgg cag gac 3189
Cys Gln Phe Cys Arg Phe Gly Tyr Tyr Gly Asp Ala Leu Arg Gln Asp
    1010                1015                1020
tgt cga aag tgt gtc tgt aat tac ctg ggc acc gtg caa gag cac tgt 3237
Cys Arg Lys Cys Val Cys Asn Tyr Leu Gly Thr Val Gln Glu His Cys
1025                1030                1035                1040
aac ggc tct gac tgc cag tgc gac aaa gcc act ggt cag tgc ttg tgt 3285
Asn Gly Ser Asp Cys Gln Cys Asp Lys Ala Thr Gly Gln Cys Leu Cys
                1045                1050                1055
ctt cct aat gtg atc ggg cag aac tgt gac cgc tgt gcg ccc aat acc 3333
Leu Pro Asn Val Ile Gly Gln Asn Cys Asp Arg Cys Ala Pro Asn Thr
            1060                1065                1070
tgg cag ctg gcc agt ggc act ggc tgt gac cca tgc aac tgc aat gct 3381
Trp Gln Leu Ala Ser Gly Thr Gly Cys Asp Pro Cys Asn Cys Asn Ala
        1075                1080                1085
gct cat tcc ttc ggg cca tct tgc aat gag ttc acg ggg cag tgc cag 3429
Ala His Ser Phe Gly Pro Ser Cys Asn Glu Phe Thr Gly Gln Cys Gln
    1090                1095                1100
tgc atg cct ggg ttt gga ggc cgc acc tgc agc gag tgc cag gaa ctc 3477
Cys Met Pro Gly Phe Gly Gly Arg Thr Cys Ser Glu Cys Gln Glu Leu
1105                1110                1115                1120
ttc tgg gga gac ccc gac gtg gag tgc cga gcc tgt gac tgt gac ccc 3525
Phe Trp Gly Asp Pro Asp Val Glu Cys Arg Ala Cys Asp Cys Asp Pro
                1125                1130                1135
agg ggc att gag acg cca cag tgt gac cag tcc acg ggc cag tgt gtc 3573
Arg Gly Ile Glu Thr Pro Gln Cys Asp Gln Ser Thr Gly Gln Cys Val
            1140                1145                1150

tgc gtt gag ggt gtt gag ggt cca cgc tgt gac aag tgc acg cga ggg 3621
Cys Val Glu Gly Val Glu Gly Pro Arg Cys Asp Lys Cys Thr Arg Gly
        1155                1160                1165
tac tcg ggg gtc ttc cct gac tgc aca ccc tgc cac cag tgc ttt gct 3669
Tyr Ser Gly Val Phe Pro Asp Cys Thr Pro Cys His Gln Cys Phe Ala
    1170                1175                1180
ctc tgg gat gtg atc att gcc gag ctg acc aac agg aca cac aga ttc 3717
Leu Trp Asp Val Ile Ile Ala Glu Leu Thr Asn Arg Thr His Arg Phe
1185                1190                1195                1200
ctg gag aaa gcc aag gcc ttg aag atc agt ggt gtg atc ggg cct tac 3765
Leu Glu Lys Ala Lys Ala Leu Lys Ile Ser Gly Val Ile Gly Pro Tyr
                1205                1210                1215
cgt gag act gtg gac tcg gtg gag agg aaa gtc agc gag ata aaa gac 3813
Arg Glu Thr Val Asp Ser Val Glu Arg Lys Val Ser Glu Ile Lys Asp
            1220                1225                1230
atc ctg gcg cag agc ccc gca gca gag cca ctg aaa aac att ggg aat 3861
Ile Leu Ala Gln Ser Pro Ala Ala Glu Pro Leu Lys Asn Ile Gly Asn
        1235                1240                1245
ctc ttt gag gaa gca gag aaa ctg att aaa gat gtt aca gaa atg atg 3909
Leu Phe Glu Glu Ala Glu Lys Leu Ile Lys Asp Val Thr Glu Met Met
    1250                1255                1260
gct caa gta gaa gtg aaa tta tct gac aca act tcc caa agc aac agc 3957
Ala Gln Val Glu Val Lys Leu Ser Asp Thr Thr Ser Gln Ser Asn Ser
1265                1270                1275                1280
aca gcc aaa gaa ctg gat tct cta cag aca gaa gcc gaa agc cta gac 4005
Thr Ala Lys Glu Leu Asp Ser Leu Gln Thr Glu Ala Glu Ser Leu Asp

1285                    1290                    1295

aac act gtg aaa gaa ctt gct gaa caa ctg gaa ttt atc aaa aac tca    4053
Asn Thr Val Lys Glu Leu Ala Glu Gln Leu Glu Phe Ile Lys Asn Ser

        1300                    1305                    1310

gat att cgg ggt gcc ttg gat agc att acc aag tat ttc cag atg tct    4101
Asp Ile Arg Gly Ala Leu Asp Ser Ile Thr Lys Tyr Phe Gln Met Ser

    1315                    1320                    1325

ctt gag gca gag gag agg gtg aat gcc tcc acc aca gaa ccc aac agc    4149
Leu Glu Ala Glu Glu Arg Val Asn Ala Ser Thr Thr Glu Pro Asn Ser

1330                    1335                    1340

act gtg gag cag tca gcc ctc atg aga gac aga gta gaa gac gtg atg    4197
Thr Val Glu Gln Ser Ala Leu Met Arg Asp Arg Val Glu Asp Val Met

1345                    1350                    1355                    1360

atg gag cga gaa tcc cag ttc aag gaa aaa caa gag gag cag gct cgc    4245
Met Glu Arg Glu Ser Gln Phe Lys Glu Lys Gln Glu Glu Gln Ala Arg

                1365                    1370                    1375

ctc ctt gat gaa ctg gca ggc aag cta caa agc cta gac ctt tca gcc    4293
Leu Leu Asp Glu Leu Ala Gly Lys Leu Gln Ser Leu Asp Leu Ser Ala

            1380                    1385                    1390

gct gcc gaa atg acc tgt gga aca ccc cca ggg gcc tcc tgt tcc gag    4341
Ala Ala Glu Met Thr Cys Gly Thr Pro Pro Gly Ala Ser Cys Ser Glu

        1395                    1400                    1405

act gaa tgt ggc ggg cca aac tgc aga act gac gaa gga gag agg aag    4389
Thr Glu Cys Gly Gly Pro Asn Cys Arg Thr Asp Glu Gly Glu Arg Lys

    1410                    1415                    1420

tgt ggg ggg cct ggc tgt ggt ggt ctg gtt act gtt gca cac aac gcc    4437
Cys Gly Gly Pro Gly Cys Gly Gly Leu Val Thr Val Ala His Asn Ala

1425                1430                1435                1440

tgg cag aaa gcc atg gac ttg gac caa gat gtc ctg agt gcc ctg gct    4485

Trp Gln Lys Ala Met Asp Leu Asp Gln Asp Val Leu Ser Ala Leu Ala

                1445                1450                1455

gaa gtg gaa cag ctc tcc aag atg gtc tct gaa gca aaa ctg agg gca    4533

Glu Val Glu Gln Leu Ser Lys Met Val Ser Glu Ala Lys Leu Arg Ala

            1460                1465                1470

gat gag gca aaa caa agt gct gaa gac att ctg ttg aag aca aat gct    4581

Asp Glu Ala Lys Gln Ser Ala Glu Asp Ile Leu Leu Lys Thr Asn Ala

        1475                1480                1485

acc aaa gaa aaa atg gac aag agc aat gag gag ctg aga aat cta atc    4629

Thr Lys Glu Lys Met Asp Lys Ser Asn Glu Glu Leu Arg Asn Leu Ile

    1490                1495                1500

aag caa atc aga aac ttt ttg acc cag gat agt gct gat ttg gac agc    4677

Lys Gln Ile Arg Asn Phe Leu Thr Gln Asp Ser Ala Asp Leu Asp Ser

1505                1510                1515                1520

att gaa gca gtt gct aat gaa gta ttg aaa atg gag atg cct agc acc    4725

Ile Glu Ala Val Ala Asn Glu Val Leu Lys Met Glu Met Pro Ser Thr

                1525                1530                1535

cca cag cag tta cag aac ttg aca gaa gat ata cgt gaa cga gtt gaa    4773

Pro Gln Gln Leu Gln Asn Leu Thr Glu Asp Ile Arg Glu Arg Val Glu

            1540                1545                1550

agc ctt tct caa gta gag gtt att ctt cag cat agt gct gct gac att    4821

Ser Leu Ser Gln Val Glu Val Ile Leu Gln His Ser Ala Ala Asp Ile

        1555                1560                1565

gcc aga gct gag atg ttg tta gaa gaa gct aaa aga gca agc aaa agt    4869

Ala Arg Ala Glu Met Leu Leu Glu Glu Ala Lys Arg Ala Ser Lys Ser
    1570                1575                1580
gca aca gat gtt aaa gtc act gca gat atg gta aag gaa gct ctg gaa 4917
Ala Thr Asp Val Lys Val Thr Ala Asp Met Val Lys Glu Ala Leu Glu
1585                1590                1595                1600
gaa gca gaa aag gcc cag gtc gca gca gag aag gca att aaa caa gca 4965
Glu Ala Glu Lys Ala Gln Val Ala Ala Glu Lys Ala Ile Lys Gln Ala
                1605                1610                1615
gat gaa gac att caa gga acc cag aac ctg tta act tcg att gag tct 5013
Asp Glu Asp Ile Gln Gly Thr Gln Asn Leu Leu Thr Ser Ile Glu Ser
            1620                1625                1630
gaa aca gca gct tct gag gaa acc ttg ttc aac gcg tcc cag cgc atc 5061
Glu Thr Ala Ala Ser Glu Glu Thr Leu Phe Asn Ala Ser Gln Arg Ile
        1635                1640                1645
agc gag tta gag agg aat gtg gaa gaa ctt aag cgg aaa gct gcc caa 5109
Ser Glu Leu Glu Arg Asn Val Glu Glu Leu Lys Arg Lys Ala Ala Gln
    1650                1655                1660
aac tcc ggg gag gca gaa tat att gaa aaa gta gta tat act gtg aag 5157
Asn Ser Gly Glu Ala Glu Tyr Ile Glu Lys Val Val Tyr Thr Val Lys
1665                1670                1675                1680
caa agt gca gaa gat gtt aag aag act tta gat ggt gaa ctt gat gaa 5205
Gln Ser Ala Glu Asp Val Lys Lys Thr Leu Asp Gly Glu Leu Asp Glu
                1685                1690                1695
aag tat aaa aaa gta gaa aat tta att gcc aaa aaa act gaa gag tca 5253
Lys Tyr Lys Lys Val Glu Asn Leu Ile Ala Lys Lys Thr Glu Glu Ser
            1700                1705                1710
gct gat gcc aga agg aaa gcc gaa atg cta caa aat gaa gca aaa act 5301

Ala Asp Ala Arg Arg Lys Ala Glu Met Leu Gln Asn Glu Ala Lys Thr
        1715                1720                1725
ctt tta gct caa gca aat agc aag ctg caa ctg ctc aaa gat tta gaa 5349
Leu Leu Ala Gln Ala Asn Ser Lys Leu Gln Leu Leu Lys Asp Leu Glu
    1730                1735                1740
aga aaa tat gaa gac aat caa aga tac tta gaa gat aaa gct caa gaa 5397
Arg Lys Tyr Glu Asp Asn Gln Arg Tyr Leu Glu Asp Lys Ala Gln Glu
1745                1750                1755                1760
tta gca aga ctg gaa gga gaa gtc cgt tca ctc cta aag gat ata agc 5445
Leu Ala Arg Leu Glu Gly Glu Val Arg Ser Leu Leu Lys Asp Ile Ser
                1765                1770                1775
cag aaa gtt gct gtg tat agc aca tgc ttg taacagagga gaataaaaaa 5495
Gln Lys Val Ala Val Tyr Ser Thr Cys Leu
            1780                1785
tggctgaggt gaacaaggta aaacaactac attttaaaaa ctgacttaat gctcttcaaa 5555
ataaaacatc acctatttaa tgtttttaat cacattttgt atgagttaaa taaagccc 5613

<210> 76
<211> 1786
<212> PRT
<213> Homo sapiens
<400> 76
Met Gly Leu Leu Gln Leu Leu Ala Phe Ser Phe Leu Ala Leu Cys Arg
1               5                   10                  15
Ala Arg Val Arg Ala Gln Glu Pro Glu Phe Ser Tyr Gly Cys Ala Glu
            20                  25                  30
Gly Ser Cys Tyr Pro Ala Thr Gly Asp Leu Leu Ile Gly Arg Ala Gln

        35                  40                  45
Lys Leu Ser Val Thr Ser Thr Cys Gly Leu His Lys Pro Glu Pro Tyr
    50                  55                  60
Cys Ile Val Ser His Leu Gln Glu Asp Lys Lys Cys Phe Ile Cys Asn
65                  70                  75                  80
Ser Gln Asp Pro Tyr His Glu Thr Leu Asn Pro Asp Ser His Leu Ile
                85                  90                  95
Glu Asn Val Val Thr Thr Phe Ala Pro Asn Arg Leu Lys Ile Trp Trp
            100                 105                 110
Gln Ser Glu Asn Gly Val Glu Asn Val Thr Ile Gln Leu Asp Leu Glu
        115                 120                 125
Ala Glu Phe His Phe Thr His Leu Ile Met Thr Phe Lys Thr Phe Arg
    130                 135                 140
Pro Ala Ala Met Leu Ile Glu Arg Ser Ser Asp Phe Gly Lys Thr Trp
145                 150                 155                 160
Gly Val Tyr Arg Tyr Phe Ala Tyr Asp Cys Glu Ala Ser Phe Pro Gly
                165                 170                 175
Ile Ser Thr Gly Pro Met Lys Lys Val Asp Asp Ile Ile Cys Asp Ser
            180                 185                 190
Arg Tyr Ser Asp Ile Glu Pro Ser Thr Glu Gly Glu Val Ile Phe Arg
        195                 200                 205
Ala Leu Asp Pro Ala Phe Lys Ile Glu Asp Pro Tyr Ser Pro Arg Ile
    210                 215                 220
Gln Asn Leu Leu Lys Ile Thr Asn Leu Arg Ile Lys Phe Val Lys Leu
225                 230                 235                 240
His Thr Leu Gly Asp Asn Leu Leu Asp Ser Arg Met Glu Ile Arg Glu

                245                 250                 255
Lys Tyr Tyr Tyr Ala Val Tyr Asp Met Val Val Arg Gly Asn Cys Phe
            260                 265                 270
Cys Tyr Gly His Ala Ser Glu Cys Ala Pro Val Asp Gly Phe Asn Glu
        275                 280                 285
Glu Val Glu Gly Met Val His Gly His Cys Met Cys Arg His Asn Thr
    290                 295                 300
Lys Gly Leu Asn Cys Glu Leu Cys Met Asp Phe Tyr His Asp Leu Pro
305                 310                 315                 320
Trp Arg Pro Ala Glu Gly Arg Asn Ser Asn Ala Cys Lys Lys Cys Asn
                325                 330                 335
Cys Asn Glu His Ser Ile Ser Cys His Phe Asp Met Ala Val Tyr Leu
            340                 345                 350
Ala Thr Gly Asn Val Ser Gly Gly Val Cys Asp Asp Cys Gln His Asn
        355                 360                 365
Thr Met Gly Arg Asn Cys Glu Gln Cys Lys Pro Phe Tyr Tyr Gln His
    370                 375                 380
Pro Glu Arg Asp Ile Arg Asp Pro Asn Phe Cys Glu Arg Cys Thr Cys
385                 390                 395                 400
Asp Pro Ala Gly Ser Gln Asn Glu Gly Ile Cys Asp Ser Tyr Thr Asp
                405                 410                 415
Phe Ser Thr Gly Leu Ile Ala Gly Gln Cys Arg Cys Lys Leu Asn Val
            420                 425                 430
Glu Gly Glu His Cys Asp Val Cys Lys Glu Gly Phe Tyr Asp Leu Ser
        435                 440                 445
Ser Glu Asp Pro Phe Gly Cys Lys Ser Cys Ala Cys Asn Pro Leu Gly
    450                 455                 460

Thr Ile Pro Gly Gly Asn Pro Cys Asp Ser Glu Thr Gly His Cys Tyr
465                 470                 475                 480
Cys Lys Arg Leu Val Thr Gly Gln His Cys Asp Gln Cys Leu Pro Glu
                485                 490                 495
His Trp Gly Leu Ser Asn Asp Leu Asp Gly Cys Arg Pro Cys Asp Cys
            500                 505                 510
Asp Leu Gly Gly Ala Leu Asn Asn Ser Cys Phe Ala Glu Ser Gly Gln
        515                 520                 525
Cys Ser Cys Arg Pro His Met Ile Gly Arg Gln Cys Asn Glu Val Glu
    530                 535                 540
Pro Gly Tyr Tyr Phe Ala Thr Leu Asp His Tyr Leu Tyr Glu Ala Glu
545                 550                 555                 560
Glu Ala Asn Leu Gly Pro Gly Val Ser Ile Val Glu Arg Gln Tyr Ile
                565                 570                 575
Gln Asp Arg Ile Pro Ser Trp Thr Gly Ala Gly Phe Val Arg Val Pro
            580                 585                 590
Glu Gly Ala Tyr Leu Glu Phe Phe Ile Asp Asn Ile Pro Tyr Ser Met
        595                 600                 605
Glu Tyr Asp Ile Leu Ile Arg Tyr Glu Pro Gln Leu Pro Asp His Trp
    610                 615                 620
Glu Lys Ala Val Ile Thr Val Gln Arg Pro Gly Arg Ile Pro Thr Ser
625                 630                 635                 640
Ser Arg Cys Gly Asn Thr Ile Pro Asp Asp Asp Asn Gln Val Val Ser
                645                 650                 655
Leu Ser Pro Gly Ser Arg Tyr Val Val Leu Pro Arg Pro Val Cys Phe
            660                 665                 670

Glu Lys Gly Thr Asn Tyr Thr Val Arg Leu Glu Leu Pro Gln Tyr Thr
        675                 680                 685
Ser Ser Asp Ser Asp Val Glu Ser Pro Tyr Thr Leu Ile Asp Ser Leu
    690                 695                 700
Val Leu Met Pro Tyr Cys Lys Ser Leu Asp Ile Phe Thr Val Gly Gly
705                 710                 715                 720
Ser Gly Asp Gly Val Val Thr Asn Ser Ala Trp Glu Thr Phe Gln Arg
                725                 730                 735
Tyr Arg Cys Leu Glu Asn Ser Arg Ser Val Val Lys Thr Pro Met Thr
            740                 745                 750
Asp Val Cys Arg Asn Ile Ile Phe Ser Ile Ser Ala Leu Leu His Gln
        755                 760                 765
Thr Gly Leu Ala Cys Glu Cys Asp Pro Gln Gly Ser Leu Ser Ser Val
    770                 775                 780
Cys Asp Pro Asn Gly Gly Gln Cys Gln Cys Arg Pro Asn Val Val Gly
785                 790                 795                 800
Arg Thr Cys Asn Arg Cys Ala Pro Gly Thr Phe Gly Phe Gly Pro Ser
                805                 810                 815
Gly Cys Lys Pro Cys Glu Cys His Leu Gln Gly Ser Val Asn Ala Phe
            820                 825                 830
Cys Asn Pro Val Thr Gly Gln Cys His Cys Phe Gln Gly Val Tyr Ala
        835                 840                 845
Arg Gln Cys Asp Arg Cys Leu Pro Gly His Trp Gly Phe Pro Ser Cys
    850                 855                 860
Gln Pro Cys Gln Cys Asn Gly His Ala Asp Asp Cys Asp Pro Val Thr
865                 870                 875                 880
Gly Glu Cys Leu Asn Cys Gln Asp Tyr Thr Met Gly His Asn Cys Glu

                885                 890                 895
Arg Cys Leu Ala Gly Tyr Tyr Gly Asp Pro Ile Ile Gly Ser Gly Asp
            900                 905                 910
His Cys Arg Pro Cys Pro Cys Pro Asp Gly Pro Asp Ser Gly Arg Gln
        915                 920                 925
Phe Ala Arg Ser Cys Tyr Gln Asp Pro Val Thr Leu Gln Leu Ala Cys
    930                 935                 940
Val Cys Asp Pro Gly Tyr Ile Gly Ser Arg Cys Asp Asp Cys Ala Ser
945                 950                 955                 960
Gly Tyr Phe Gly Asn Pro Ser Glu Val Gly Gly Ser Cys Gln Pro Cys
                965                 970                 975
Gln Cys His Asn Asn Ile Asp Thr Thr Asp Pro Glu Ala Cys Asp Lys
            980                 985                 990
Glu Thr Gly Arg Cys Leu Lys Cys Leu Tyr His Thr Glu Gly Glu His
        995                 1000                1005
Cys Gln Phe Cys Arg Phe Gly Tyr Tyr Gly Asp Ala Leu Arg Gln Asp
    1010                1015                1020
Cys Arg Lys Cys Val Cys Asn Tyr Leu Gly Thr Val Gln Glu His Cys
1025                1030                1035                1040
Asn Gly Ser Asp Cys Gln Cys Asp Lys Ala Thr Gly Gln Cys Leu Cys
                1045                1050                1055
Leu Pro Asn Val Ile Gly Gln Asn Cys Asp Arg Cys Ala Pro Asn Thr
            1060                1065                1070
Trp Gln Leu Ala Ser Gly Thr Gly Cys Asp Pro Cys Asn Cys Asn Ala
        1075                1080                1085
Ala His Ser Phe Gly Pro Ser Cys Asn Glu Phe Thr Gly Gln Cys Gln

1090                1095                1100

Cys Met Pro Gly Phe Gly Gly Arg Thr Cys Ser Glu Cys Gln Glu Leu

1105                1110                1115                1120

Phe Trp Gly Asp Pro Asp Val Glu Cys Arg Ala Cys Asp Cys Asp Pro

                1125                1130                1135

Arg Gly Ile Glu Thr Pro Gln Cys Asp Gln Ser Thr Gly Gln Cys Val

            1140                1145                1150

Cys Val Glu Gly Val Glu Gly Pro Arg Cys Asp Lys Cys Thr Arg Gly

        1155                1160                1165

Tyr Ser Gly Val Phe Pro Asp Cys Thr Pro Cys His Gln Cys Phe Ala

    1170                1175                1180

Leu Trp Asp Val Ile Ile Ala Glu Leu Thr Asn Arg Thr His Arg Phe

1185                1190                1195                1200

Leu Glu Lys Ala Lys Ala Leu Lys Ile Ser Gly Val Ile Gly Pro Tyr

                1205                1210                1215

Arg Glu Thr Val Asp Ser Val Glu Arg Lys Val Ser Glu Ile Lys Asp

            1220                1225                1230

Ile Leu Ala Gln Ser Pro Ala Ala Glu Pro Leu Lys Asn Ile Gly Asn

        1235                1240                1245

Leu Phe Glu Glu Ala Glu Lys Leu Ile Lys Asp Val Thr Glu Met Met

    1250                1255                1260

Ala Gln Val Glu Val Lys Leu Ser Asp Thr Thr Ser Gln Ser Asn Ser

1265                1270                1275                1280

Thr Ala Lys Glu Leu Asp Ser Leu Gln Thr Glu Ala Glu Ser Leu Asp

                1285                1290                1295

Asn Thr Val Lys Glu Leu Ala Glu Gln Leu Glu Phe Ile Lys Asn Ser

            1300                1305                1310

Asp Ile Arg Gly Ala Leu Asp Ser Ile Thr Lys Tyr Phe Gln Met Ser
        1315                1320                1325
Leu Glu Ala Glu Glu Arg Val Asn Ala Ser Thr Thr Glu Pro Asn Ser
    1330                1335                1340
Thr Val Glu Gln Ser Ala Leu Met Arg Asp Arg Val Glu Asp Val Met
1345                1350                1355                1360
Met Glu Arg Glu Ser Gln Phe Lys Glu Lys Gln Glu Glu Gln Ala Arg
                1365                1370                1375
Leu Leu Asp Glu Leu Ala Gly Lys Leu Gln Ser Leu Asp Leu Ser Ala
            1380                1385                1390
Ala Ala Glu Met Thr Cys Gly Thr Pro Pro Gly Ala Ser Cys Ser Glu
        1395                1400                1405
Thr Glu Cys Gly Gly Pro Asn Cys Arg Thr Asp Glu Gly Glu Arg Lys
    1410                1415                1420
Cys Gly Gly Pro Gly Cys Gly Gly Leu Val Thr Val Ala His Asn Ala
1425                1430                1435                1440
Trp Gln Lys Ala Met Asp Leu Asp Gln Asp Val Leu Ser Ala Leu Ala
                1445                1450                1455
Glu Val Glu Gln Leu Ser Lys Met Val Ser Glu Ala Lys Leu Arg Ala
            1460                1465                1470
Asp Glu Ala Lys Gln Ser Ala Glu Asp Ile Leu Leu Lys Thr Asn Ala
        1475                1480                1485
Thr Lys Glu Lys Met Asp Lys Ser Asn Glu Glu Leu Arg Asn Leu Ile
    1490                1495                1500
Lys Gln Ile Arg Asn Phe Leu Thr Gln Asp Ser Ala Asp Leu Asp Ser
1505                1510                1515                1520

Ile Glu Ala Val Ala Asn Glu Val Leu Lys Met Glu Met Pro Ser Thr
                1525                1530                1535
Pro Gln Gln Leu Gln Asn Leu Thr Glu Asp Ile Arg Glu Arg Val Glu
            1540                1545                1550
Ser Leu Ser Gln Val Glu Val Ile Leu Gln His Ser Ala Ala Asp Ile
        1555                1560                1565
Ala Arg Ala Glu Met Leu Leu Glu Glu Ala Lys Arg Ala Ser Lys Ser
    1570                1575                1580
Ala Thr Asp Val Lys Val Thr Ala Asp Met Val Lys Glu Ala Leu Glu
1585                1590                1595                1600
Glu Ala Glu Lys Ala Gln Val Ala Ala Glu Lys Ala Ile Lys Gln Ala
                1605                1610                1615
Asp Glu Asp Ile Gln Gly Thr Gln Asn Leu Leu Thr Ser Ile Glu Ser
            1620                1625                1630
Glu Thr Ala Ala Ser Glu Glu Thr Leu Phe Asn Ala Ser Gln Arg Ile
        1635                1640                1645
Ser Glu Leu Glu Arg Asn Val Glu Glu Leu Lys Arg Lys Ala Ala Gln
    1650                1655                1660
Asn Ser Gly Glu Ala Glu Tyr Ile Glu Lys Val Val Tyr Thr Val Lys
1665                1670                1675                1680
Gln Ser Ala Glu Asp Val Lys Lys Thr Leu Asp Gly Glu Leu Asp Glu
                1685                1690                1695
Lys Tyr Lys Lys Val Glu Asn Leu Ile Ala Lys Lys Thr Glu Glu Ser
            1700                1705                1710
Ala Asp Ala Arg Arg Lys Ala Glu Met Leu Gln Asn Glu Ala Lys Thr
        1715                1720                1725
Leu Leu Ala Gln Ala Asn Ser Lys Leu Gln Leu Leu Lys Asp Leu Glu

1730                1735                1740
Arg Lys Tyr Glu Asp Asn Gln Arg Tyr Leu Glu Asp Lys Ala Gln Glu
1745                1750                1755                1760
Leu Ala Arg Leu Glu Gly Glu Val Arg Ser Leu Leu Lys Asp Ile Ser
                1765                1770                1775
Gln Lys Val Ala Val Tyr Ser Thr Cys Leu
            1780                1785

<210> 77
<211> 585
<212> DNA
<213> Homo sapiens
<220>
<221> CDS
<222> (27)..(335)
<400> 77
ctgagactga cctgcaggac gaaacc atg aag agc ctg atc ctt ctt gcc atc   53
                             Met Lys Ser Leu Ile Leu Leu Ala Ile
                             1               5
ctg gcc gcc tta gcg gta gta act ttg tgt tat gaa tca cat gaa agc   101
Leu Ala Ala Leu Ala Val Val Thr Leu Cys Tyr Glu Ser His Glu Ser
10                  15                  20                  25
atg gaa tct tat gaa ctt aat ccc ttc att aac agg aga aat gca aat   149
Met Glu Ser Tyr Glu Leu Asn Pro Phe Ile Asn Arg Arg Asn Ala Asn
                30                  35                  40
acc ttc ata tcc cct cag cag aga tgg aga gct aaa gtc caa gag agg   197
Thr Phe Ile Ser Pro Gln Gln Arg Trp Arg Ala Lys Val Gln Glu Arg

            45                  50                  55
atc cga gaa cgc tct aag cct gtc cac gag ctc aat agg gaa gcc tgt 245
Ile Arg Glu Arg Ser Lys Pro Val His Glu Leu Asn Arg Glu Ala Cys
        60                  65                  70
gat gac tac aga ctt tgc gaa cgc tac gcc atg gtt tat gga tac aat 293
Asp Asp Tyr Arg Leu Cys Glu Arg Tyr Ala Met Val Tyr Gly Tyr Asn
    75                  80                  85
gct gcc tat aat cgc tac ttc agg aag cgc cga ggg acc aaa 335
Ala Ala Tyr Asn Arg Tyr Phe Arg Lys Arg Arg Gly Thr Lys
90                  95                  100
tgagactgag ggaagaaaaa aaatctcttt ttttctggag gctggcacct gattttgtat 395
ccccctgtag cagcattact gaaatacata ggcttatata caatgcttct ttcctgtata 455
ttctcttgtc tggctgcacc cctttttccc gcccccagat tgataagtaa tgaaagtgca 515
ctgcagtgag ggtcaaagga gagtcaacat atgtgattgt tccataataa acttctggtg 575
tgatactttc 585
<210> 78
<211> 103
<212> PRT
<213> Homo sapiens
<400> 78
Met Lys Ser Leu Ile Leu Leu Ala Ile Leu Ala Ala Leu Ala Val Val
1               5                   10                  15
Thr Leu Cys Tyr Glu Ser His Glu Ser Met Glu Ser Tyr Glu Leu Asn
            20                  25                  30
Pro Phe Ile Asn Arg Arg Asn Ala Asn Thr Phe Ile Ser Pro Gln Gln
        35                  40                  45

Arg Trp Arg Ala Lys Val Gln Glu Arg Ile Arg Glu Arg Ser Lys Pro
    50                  55                  60
Val His Glu Leu Asn Arg Glu Ala Cys Asp Asp Tyr Arg Leu Cys Glu
65                  70                  75                  80
Arg Tyr Ala Met Val Tyr Gly Tyr Asn Ala Ala Tyr Asn Arg Tyr Phe
                85                  90                  95
Arg Lys Arg Arg Gly Thr Lys
            100
<210> 79
<211> 1775
<212> DNA
<213> Homo sapiens
<220>
<221> CDS
<222> (6)..(1148)
<400> 79
cagca atg cat ctc ctt gcg att ctg ttt tgt gct ctc tgg tct gca gtg 50
Met His Leu Leu Ala Ile Leu Phe Cys Ala Leu Trp Ser Ala Val
1               5                   10                  15
ttg gcc gag aac tcg gat gat tat gat ctc atg tat gtg aat ttg gac 98
Leu Ala Glu Asn Ser Asp Asp Tyr Asp Leu Met Tyr Val Asn Leu Asp
                20                  25                  30
aac gaa ata gac aat gga ctc cat ccc act gag gac ccc acg ccg tgc 146
Asn Glu Ile Asp Asn Gly Leu His Pro Thr Glu Asp Pro Thr Pro Cys
            35                  40                  45
gcc tgc ggt cag gag cac tcg gaa tgg gac aag ctc ttc atc atg ctg 194

Ala Cys Gly Gln Glu His Ser Glu Trp Asp Lys Leu Phe Ile Met Leu
        50                  55                  60
gag aac tcg cag atg aga gag cgc atg ctg ctg caa gcc acg gac gac 242
Glu Asn Ser Gln Met Arg Glu Arg Met Leu Leu Gln Ala Thr Asp Asp
    65                  70                  75
gtc ctg cgg ggc gag ctg cag agg ctg cgg gag gag ctg ggc cgg ctc 290
Val Leu Arg Gly Glu Leu Gln Arg Leu Arg Glu Glu Leu Gly Arg Leu
80                  85                  90                  95
gcg gaa agc ctg gcg agg ccg tgc gcg ccg ggg gct ccc gca gag gcc 338
Ala Glu Ser Leu Ala Arg Pro Cys Ala Pro Gly Ala Pro Ala Glu Ala
                100                 105                 110
agg ctg acc agt gct ctg gac gag ctg ctg cag gcg acc cgc gac gcg 386
Arg Leu Thr Ser Ala Leu Asp Glu Leu Leu Gln Ala Thr Arg Asp Ala
            115                 120                 125
ggc cgc agg ctg gcg cgt atg gag ggc gcg gag gcg cag cgc cca gag 434
Gly Arg Arg Leu Ala Arg Met Glu Gly Ala Glu Ala Gln Arg Pro Glu
        130                 135                 140
gag gcg ggg cgc gcc ctg gcc gcg gtg cta gag gag ctg cgg cag acg 482
Glu Ala Gly Arg Ala Leu Ala Ala Val Leu Glu Glu Leu Arg Gln Thr
    145                 150                 155
cga gcc gac ctg cac gcg gtg cag ggc tgg gct gcc cgg agc tgg ctg 530
Arg Ala Asp Leu His Ala Val Gln Gly Trp Ala Ala Arg Ser Trp Leu
160                 165                 170                 175
ccg gca ggt tgt gaa aca gct att tta ttc cca atg cgt tcc aag aag 578
Pro Ala Gly Cys Glu Thr Ala Ile Leu Phe Pro Met Arg Ser Lys Lys
                180                 185                 190

att ttt gga agc gtg cat cca gtg aga cca atg agg ctt gag tct ttt 626
Ile Phe Gly Ser Val His Pro Val Arg Pro Met Arg Leu Glu Ser Phe
            195                 200                 205
agt gcc tgc att tgg gtc aaa gcc aca gat gta tta aac aaa acc atc 674
Ser Ala Cys Ile Trp Val Lys Ala Thr Asp Val Leu Asn Lys Thr Ile
        210                 215                 220
ctg ttt tcc tat ggc aca aag agg aat cca tat gaa atc cag ctg tat 722
Leu Phe Ser Tyr Gly Thr Lys Arg Asn Pro Tyr Glu Ile Gln Leu Tyr
    225                 230                 235
ctc agc tac caa tcc ata gtg ttt gtg gtg ggt gga gag gag aac aaa 770
Leu Ser Tyr Gln Ser Ile Val Phe Val Val Gly Gly Glu Glu Asn Lys
240                 245                 250                 255
ctg gtt gct gaa gcc atg gtt tcc ctg gga agg tgg acc cac ctg tgc 818
Leu Val Ala Glu Ala Met Val Ser Leu Gly Arg Trp Thr His Leu Cys
                260                 265                 270
ggc acc tgg aat tca gag gaa ggg ctc aca tcc ttg tgg gta aat ggt 866
Gly Thr Trp Asn Ser Glu Glu Gly Leu Thr Ser Leu Trp Val Asn Gly
            275                 280                 285
gaa ctg gcg gct acc act gtt gag atg gcc aca ggt cac att gtt cct 914
Glu Leu Ala Ala Thr Thr Val Glu Met Ala Thr Gly His Ile Val Pro
        290                 295                 300
gag gga gga atc ctg cag att ggc caa gaa aag aat ggc tgc tgt gtg 962
Glu Gly Gly Ile Leu Gln Ile Gly Gln Glu Lys Asn Gly Cys Cys Val
    305                 310                 315
ggt ggt ggc ttt gat gaa aca tta gcc ttc tct ggg aga ctc aca ggc 1010
Gly Gly Gly Phe Asp Glu Thr Leu Ala Phe Ser Gly Arg Leu Thr Gly
320                 325                 330                 335

ttc aat atc tgg gat agt gtt ctt agc aat gaa gag ata aga gag acc 1058
Phe Asn Ile Trp Asp Ser Val Leu Ser Asn Glu Glu Ile Arg Glu Thr
                340                 345                 350
gga gga gca gag tct tgt cac atc cgg ggg aat att gtt ggg tgg gga 1106
Gly Gly Ala Glu Ser Cys His Ile Arg Gly Asn Ile Val Gly Trp Gly
            355                 360                 365
gtc aca gag atc cag cca cat gga gga gct cag tat gtt tca 1148
Val Thr Glu Ile Gln Pro His Gly Gly Ala Gln Tyr Val Ser
        370                 375                 380
taaatgttgt gaaactccac ttgaagccaa agaaagaaac tcacacttaa aacacatgcc 1208
agttgggaag gtctgaaaac tcagtgcata ataggaacac ttgagactaa tgaaagagag 1268
agttgagacc aatctttatt tgtactggcc aaatactgaa taaacagttg aaggaaagac 1328
attggaaaaa gcttttgagg ataatgttac tagactttat gccatggtgc tttcagttta 1388
atgctgtgtc tctgtcagat aaactctcaa ataattaaaa aggactgtat tgttgaacag 1448
agggacaatt gttttacttt tctttggtta attttgtttt ggccagagat gaattttaca 1508
ttggaagaat aacaaaataa gatttgttgt ccattgttca ttgttattgg tatgtacctt 1568
attacaaaaa aaatgatgaa aacatattta tactacaagg tgacttaaca actataaatg 1628
tagtttatgt gttataatcg aatgtcacgt ttttgagaag atagtcatat aagttatatt 1688
gcaaaaggga tttgtattaa tttaagacta tttttgtaaa gctctactgt aaataaaata 1748
ttttataaaa ctaaaaaaaa aaaaaaa 1775

<210> 80
<211> 381
<212> PRT
<213> Homo sapiens
<400> 80
Met His Leu Leu Ala Ile Leu Phe Cys Ala Leu Trp Ser Ala Val Leu

1                   5                   10                  15
Ala Glu Asn Ser Asp Asp Tyr Asp Leu Met Tyr Val Asn Leu Asp Asn
                20                  25                  30
Glu Ile Asp Asn Gly Leu His Pro Thr Glu Asp Pro Thr Pro Cys Ala
            35                  40                  45
Cys Gly Gln Glu His Ser Glu Trp Asp Lys Leu Phe Ile Met Leu Glu
        50                  55                  60
Asn Ser Gln Met Arg Glu Arg Met Leu Leu Gln Ala Thr Asp Asp Val
65                  70                  75                  80
Leu Arg Gly Glu Leu Gln Arg Leu Arg Glu Glu Leu Gly Arg Leu Ala
                85                  90                  95
Glu Ser Leu Ala Arg Pro Cys Ala Pro Gly Ala Pro Ala Glu Ala Arg
            100                 105                 110
Leu Thr Ser Ala Leu Asp Glu Leu Leu Gln Ala Thr Arg Asp Ala Gly
        115                 120                 125
Arg Arg Leu Ala Arg Met Glu Gly Ala Glu Ala Gln Arg Pro Glu Glu
    130                 135                 140
Ala Gly Arg Ala Leu Ala Ala Val Leu Glu Glu Leu Arg Gln Thr Arg
145                 150                 155                 160
Ala Asp Leu His Ala Val Gln Gly Trp Ala Ala Arg Ser Trp Leu Pro
                165                 170                 175
Ala Gly Cys Glu Thr Ala Ile Leu Phe Pro Met Arg Ser Lys Lys Ile
            180                 185                 190
Phe Gly Ser Val His Pro Val Arg Pro Met Arg Leu Glu Ser Phe Ser
        195                 200                 205
Ala Cys Ile Trp Val Lys Ala Thr Asp Val Leu Asn Lys Thr Ile Leu

210                 215                 220

Phe Ser Tyr Gly Thr Lys Arg Asn Pro Tyr Glu Ile Gln Leu Tyr Leu

225                 230                 235                 240

Ser Tyr Gln Ser Ile Val Phe Val Val Gly Gly Glu Glu Asn Lys Leu

                245                 250                 255

Val Ala Glu Ala Met Val Ser Leu Gly Arg Trp Thr His Leu Cys Gly

            260                 265                 270

Thr Trp Asn Ser Glu Glu Gly Leu Thr Ser Leu Trp Val Asn Gly Glu

        275                 280                 285

Leu Ala Ala Thr Thr Val Glu Met Ala Thr Gly His Ile Val Pro Glu

    290                 295                 300

Gly Gly Ile Leu Gln Ile Gly Gln Glu Lys Asn Gly Cys Cys Val Gly

305                 310                 315                 320

Gly Gly Phe Asp Glu Thr Leu Ala Phe Ser Gly Arg Leu Thr Gly Phe

                325                 330                 335

Asn Ile Trp Asp Ser Val Leu Ser Asn Glu Glu Ile Arg Glu Thr Gly

            340                 345                 350

Gly Ala Glu Ser Cys His Ile Arg Gly Asn Ile Val Gly Trp Gly Val

        355                 360                 365

Thr Glu Ile Gln Pro His Gly Gly Ala Gln Tyr Val Ser

    370                 375                 380

<210> 81

<211> 2312

<212> DNA

<213> Homo sapiens

<220>

<221> CDS
<222> (146)..(1192)
<400> 81
tccagtgacg gagccgcccg gccgacagcc ccgagacgac agcccggcgc gtcccggtcc 60
ccacctccga ccaccgccag cgctccaggc cccgcgctcc ccgctcgccg ccaccgcgcc 120
ctccgctccg cccgcagtgc caacc atg acc gcc gcc agt atg ggc ccc gtc 172
Met Thr Ala Ala Ser Met Gly Pro Val
1               5
cgc gtc gcc ttc gtg gtc ctc ctc gcc ctc tgc agc cgg ccg gcc gtc 220
Arg Val Ala Phe Val Val Leu Leu Ala Leu Cys Ser Arg Pro Ala Val
10                  15                  20                  25
ggc cag aac tgc agc ggg ccg tgc cgg tgc ccg gac gag ccg gcg ccg 268
Gly Gln Asn Cys Ser Gly Pro Cys Arg Cys Pro Asp Glu Pro Ala Pro
                30                  35                  40
cgc tgc ccg gcg ggc gtg agc ctc gtg ctg gac ggc tgc ggc tgc tgc 316
Arg Cys Pro Ala Gly Val Ser Leu Val Leu Asp Gly Cys Gly Cys Cys
            45                  50                  55
cgc gtc tgc gcc aag cag ctg ggc gag ctg tgc acc gag cgc gac ccc 364
Arg Val Cys Ala Lys Gln Leu Gly Glu Leu Cys Thr Glu Arg Asp Pro
        60                  65                  70
tgc gac ccg cac aag ggc ctc ttc tgt gac ttc ggc tcc ccg gcc aac 412
Cys Asp Pro His Lys Gly Leu Phe Cys Asp Phe Gly Ser Pro Ala Asn
    75                  80                  85
cgc aag atc ggc gtg tgc acc gcc aaa gat ggt gct ccc tgc atc ttc 460
Arg Lys Ile Gly Val Cys Thr Ala Lys Asp Gly Ala Pro Cys Ile Phe
90                  95                  100                 105
ggt ggt acg gtg tac cgc agc gga gag tcc ttc cag agc agc tgc aag 508

Gly Gly Thr Val Tyr Arg Ser Gly Glu Ser Phe Gln Ser Ser Cys Lys
                110                 115                 120
tac cag tgc acg tgc ctg gac ggg gcg gtg ggc tgc atg ccc ctg tgc 556
Tyr Gln Cys Thr Cys Leu Asp Gly Ala Val Gly Cys Met Pro Leu Cys
            125                 130                 135
agc atg gac gtt cgt ctg ccc agc cct gac tgc ccc ttc ccg agg agg 604
Ser Met Asp Val Arg Leu Pro Ser Pro Asp Cys Pro Phe Pro Arg Arg
        140                 145                 150
gtc aag ctg ccc ggg aaa tgc tgc gag gag tgg gtg tgt gac gag ccc 652
Val Lys Leu Pro Gly Lys Cys Cys Glu Glu Trp Val Cys Asp Glu Pro
    155                 160                 165
aag gac caa acc gtg gtt ggg cct gcc ctc gcg gct tac cga ctg gaa 700
Lys Asp Gln Thr Val Val Gly Pro Ala Leu Ala Ala Tyr Arg Leu Glu
170                 175                 180                 185
gac acg ttt ggc cca gac cca act atg att aga gcc aac tgc ctg gtc 748
Asp Thr Phe Gly Pro Asp Pro Thr Met Ile Arg Ala Asn Cys Leu Val
                190                 195                 200
cag acc aca gag tgg agc gcc tgt tcc aag acc tgt ggg atg ggc atc 796
Gln Thr Thr Glu Trp Ser Ala Cys Ser Lys Thr Cys Gly Met Gly Ile
            205                 210                 215
tcc acc cgg gtt acc aat gac aac gcc tcc tgc agg cta gag aag cag 844
Ser Thr Arg Val Thr Asn Asp Asn Ala Ser Cys Arg Leu Glu Lys Gln
        220                 225                 230
agc cgc ctg tgc atg gtc agg cct tgc gaa gct gac ctg gaa gag aac 892
Ser Arg Leu Cys Met Val Arg Pro Cys Glu Ala Asp Leu Glu Glu Asn
    235                 240                 245

att aag aag ggc aaa aag tgc atc cgt act ccc aaa atc tcc aag cct 940
Ile Lys Lys Gly Lys Lys Cys Ile Arg Thr Pro Lys Ile Ser Lys Pro
250                 255                 260                 265
atc aag ttt gag ctt tct ggc tgc acc agc atg aag aca tac cga gct 988
Ile Lys Phe Glu Leu Ser Gly Cys Thr Ser Met Lys Thr Tyr Arg Ala
                270                 275                 280
aaa ttc tgt gga gta tgt acc gac ggc cga tgc tgc acc ccc cac aga 1036
Lys Phe Cys Gly Val Cys Thr Asp Gly Arg Cys Cys Thr Pro His Arg
            285                 290                 295
acc acc acc ctg ccg gtg gag ttc aag tgc cct gac ggc gag gtc atg 1084
Thr Thr Thr Leu Pro Val Glu Phe Lys Cys Pro Asp Gly Glu Val Met
        300                 305                 310
aag aag aac atg atg ttc atc aag acc tgt gcc tgc cat tac aac tgt 1132
Lys Lys Asn Met Met Phe Ile Lys Thr Cys Ala Cys His Tyr Asn Cys
    315                 320                 325
ccc gga gac aat gac atc ttt gaa tcg ctg tac tac agg aag atg tac 1180
Pro Gly Asp Asn Asp Ile Phe Glu Ser Leu Tyr Tyr Arg Lys Met Tyr
330                 335                 340                 345
gga gac atg gca tgaagccaga gagtgagaga cattaactca ttagactgga 1232
Gly Asp Met Ala
acttgaactg attcacatct catttttccg taaaaatgat ttcagtagca caagttattt 1292
aaatctgttt ttctaactgg gggaaaagat tcccacccaa ttcaaaacat tgtgccatgt 1352
caaacaaata gtctatcttc cccagacact ggtttgaaga atgttaagac ttgacagtgg 1412
aactacatta gtacacagca ccagaatgta tattaaggtg tggctttagg agcagtggga 1472
gggtaccagc agaaaggtta gtatcatcag atagctctta tacgagtaat atgcctgcta 1532
tttgaagtgt aattgagaag gaaaatttta gcgtgctcac tgacctgcct gtagccccag 1592
tgacagctag gatgtgcatt ctccagccat caagagactg agtcaagttg ttccttaagt 1652

cagaacagca gactcagctc tgacattctg attcgaatga cactgttcag gaatcggaat 1712

cctgtcgatt agactggaca gcttgtggca agtgaatttc ctgtaacaag ccagattttt 1772

taaaatttat attgtaaata ttgtgtgtgt gtgtgtgtgt gtatatatat atatatatgt 1832

acagttatct aagttaattt aaagttgttt gtgccttttt atttttgttt ttaatgcttt 1892

gatatttcaa tgttagcctc aatttctgaa caccataggt agaatgtaaa gcttgtctga 1952

tcgttcaaag catgaaatgg atacttatat ggaaattctc tcagatagaa tgacagtccg 2012

tcaaaacaga ttgtttgcaa aggggaggca tcagtgtcct tggcaggctg atttctaggt 2072

aggaaatgtg gtagctcacg ctcactttta atgaacaaat ggcctttatt aaaaactgag 2132

tgactctata tagctgatca gttttttcac ctggaagcat ttgtttctac tttgatatga 2192

ctgtttttcg gacagtttat ttgttgagag tgtgaccaaa agttacatgt ttgcaccttt 2252

ctagttgaaa ataaagtata ttttttctaa aaaaaaaaaa aaacgacagc aacggaattc 2312

<210> 82

<211> 349

<212> PRT

<213> Homo sapiens

<400> 82

Met Thr Ala Ala Ser Met Gly Pro Val Arg Val Ala Phe Val Val Leu

1               5                   10                  15

Leu Ala Leu Cys Ser Arg Pro Ala Val Gly Gln Asn Cys Ser Gly Pro

            20                  25                  30

Cys Arg Cys Pro Asp Glu Pro Ala Pro Arg Cys Pro Ala Gly Val Ser

        35                  40                  45

Leu Val Leu Asp Gly Cys Gly Cys Cys Arg Val Cys Ala Lys Gln Leu

    50                  55                  60

Gly Glu Leu Cys Thr Glu Arg Asp Pro Cys Asp Pro His Lys Gly Leu

65                  70                  75                  80

Phe Cys Asp Phe Gly Ser Pro Ala Asn Arg Lys Ile Gly Val Cys Thr
                85                  90                  95
Ala Lys Asp Gly Ala Pro Cys Ile Phe Gly Gly Thr Val Tyr Arg Ser
            100                 105                 110
Gly Glu Ser Phe Gln Ser Ser Cys Lys Tyr Gln Cys Thr Cys Leu Asp
        115                 120                 125
Gly Ala Val Gly Cys Met Pro Leu Cys Ser Met Asp Val Arg Leu Pro
    130                 135                 140
Ser Pro Asp Cys Pro Phe Pro Arg Arg Val Lys Leu Pro Gly Lys Cys
145                 150                 155                 160
Cys Glu Glu Trp Val Cys Asp Glu Pro Lys Asp Gln Thr Val Val Gly
                165                 170                 175
Pro Ala Leu Ala Ala Tyr Arg Leu Glu Asp Thr Phe Gly Pro Asp Pro
            180                 185                 190
Thr Met Ile Arg Ala Asn Cys Leu Val Gln Thr Thr Glu Trp Ser Ala
        195                 200                 205
Cys Ser Lys Thr Cys Gly Met Gly Ile Ser Thr Arg Val Thr Asn Asp
    210                 215                 220
Asn Ala Ser Cys Arg Leu Glu Lys Gln Ser Arg Leu Cys Met Val Arg
225                 230                 235                 240
Pro Cys Glu Ala Asp Leu Glu Glu Asn Ile Lys Lys Gly Lys Lys Cys
                245                 250                 255
Ile Arg Thr Pro Lys Ile Ser Lys Pro Ile Lys Phe Glu Leu Ser Gly
            260                 265                 270
Cys Thr Ser Met Lys Thr Tyr Arg Ala Lys Phe Cys Gly Val Cys Thr
        275                 280                 285

Asp Gly Arg Cys Cys Thr Pro His Arg Thr Thr Thr Leu Pro Val Glu
    290                 295                 300
Phe Lys Cys Pro Asp Gly Glu Val Met Lys Lys Asn Met Met Phe Ile
305                 310                 315                 320
Lys Thr Cys Ala Cys His Tyr Asn Cys Pro Gly Asp Asn Asp Ile Phe
                325                 330                 335
Glu Ser Leu Tyr Tyr Arg Lys Met Tyr Gly Asp Met Ala
            340                 345
<210> 83
<211> 2954
<212> DNA
<213> Homo sapiens
<220>
<221> CDS
<222> (173)..(1525)
<400> 83
   gaattcccaa acgtgcacag gggagtgagg gcagggcgct cgcagggggc acgcagggag 60
   ggcccagggc gccagggagg ccgcgccggg ctaatccgaa ggggctgcga ggtcaggctg 120
   taaccgggtc aatgtgtgga atattggggg gctcggctgc agacttggcc aa atg gac 178
   Met Asp
   1
   ggg act att aag gag gct ctg tcg gtg gtg agc gac gac cag tcc ctc 226
   Gly Thr Ile Lys Glu Ala Leu Ser Val Val Ser Asp Asp Gln Ser Leu
   5 10 15
   ttt gac tca gcg tac gga gcg gca gcc cat ctc ccc aag gcc gac atg 274
   Phe Asp Ser Ala Tyr Gly Ala Ala Ala His Leu Pro Lys Ala Asp Met
   20                       25                       30
   act gcc tcg ggg agt cct gac tac ggg cag ccc cac aag atc aac ccc   322
   Thr Ala Ser Gly Ser Pro Asp Tyr Gly Gln Pro His Lys Ile Asn Pro
   35                       40                       45                       50
   ctc cca cca cag cag gag tgg atc aat cag cca gtg agg gtc aac gtc   370
   Leu Pro Pro Gln Gln Glu Trp Ile Asn Gln Pro Val Arg Val Asn Val
                       55                       60                       65
   aag cgg gag tat gac cac atg aat gga tcc agg gag tct ccg gtg gac   418
   Lys Arg Glu Tyr Asp His Met Asn Gly Ser Arg Glu Ser Pro Val Asp
                  70                       75                       80
   tgc agc gtt agc aaa tgc agc aag ctg gtg ggc gga ggc gag tcc aac   466
   Cys Ser Val Ser Lys Cys Ser Lys Leu Val Gly Gly Gly Glu Ser Asn
             85                       90                       95
   ccc atg aac tac aac agc tat atg gac gag aag aat ggc ccc cct cct   514
   Pro Met Asn Tyr Asn Ser Tyr Met Asp Glu Lys Asn Gly Pro Pro Pro
        100                      105                      110
   ccc aac atg acc acc aac gag agg aga gtc atc gtc ccc gca gac ccc   562
   Pro Asn Met Thr Thr Asn Glu Arg Arg Val Ile Val Pro Ala Asp Pro
   115                      120                      125                      130
   aca ctg tgg aca cag gag cat gtg agg caa tgg ctg gag tgg gcc ata   610
   Thr Leu Trp Thr Gln Glu His Val Arg Gln Trp Leu Glu Trp Ala Ile
                       135                      140                      145
   aag gag tac agc ttg atg gag atc gac aca tcc ttt ttc cag aac atg   658
   Lys Glu Tyr Ser Leu Met Glu Ile Asp Thr Ser Phe Phe Gln Asn Met
                  150                      155                      160
   gat ggc aag gaa ctg tgt aaa atg aac aag gag gac ttc ctc cgc gcc   706
   Asp Gly Lys Glu Leu Cys Lys Met Asn Lys Glu Asp Phe Leu Arg Ala
   165 170 175
   acc acc ctc tac aac acg gaa gtg ctg ttg tca cac ctc agt tac ctc 754
   Thr Thr Leu Tyr Asn Thr Glu Val Leu Leu Ser His Leu Ser Tyr Leu
   180 185 190
   agg gaa agt tca ctg ctg gcc tat aat aca acc tcc cac acc gac caa 802
   Arg Glu Ser Ser Leu Leu Ala Tyr Asn Thr Thr Ser His Thr Asp Gln
   195 200 205 210
   tcc tca cga ttg agt gtc aaa gaa gac cct tct tat gac tca gtc aga 850
   Ser Ser Arg Leu Ser Val Lys Glu Asp Pro Ser Tyr Asp Ser Val Arg
   215 220 225
   aga gga gct tgg ggc aat aac atg aat tct ggc ctc aac aaa agt cct 898
   Arg Gly Ala Trp Gly Asn Asn Met Asn Ser Gly Leu Asn Lys Ser Pro
   230 235 240
   ccc ctt gga ggg gca caa acg atc agt aag aat aca gag caa cgg ccc 946
   Pro Leu Gly Gly Ala Gln Thr Ile Ser Lys Asn Thr Glu Gln Arg Pro
   245 250 255
   cag cca gat ccg tat cag atc ctg ggc ccg acc agc agt cgc cta gcc 994
   Gln Pro Asp Pro Tyr Gln Ile Leu Gly Pro Thr Ser Ser Arg Leu Ala
   260 265 270
   aac cct gga agc ggg cag atc cag ctg tgg caa ttc ctc ctg gag ctg 1042
   Asn Pro Gly Ser Gly Gln Ile Gln Leu Trp Gln Phe Leu Leu Glu Leu
   275 280 285 290
   ctc tcc gac agc gcc aac gcc agc tgt atc acc tgg gag ggg acc aac 1090
   Leu Ser Asp Ser Ala Asn Ala Ser Cys Ile Thr Trp Glu Gly Thr Asn
   295 300 305
   ggg gag ttc aaa atg acg gac ccc gat gag gtg gcc agg cgc tgg ggc 1138
   Gly Glu Phe Lys Met Thr Asp Pro Asp Glu Val Ala Arg Arg Trp Gly
   310 315 320
   gag cgg aaa agc aag ccc aac atg aat tac gac aag ctg agc cgg gcc 1186
   Glu Arg Lys Ser Lys Pro Asn Met Asn Tyr Asp Lys Leu Ser Arg Ala
   325 330 335
   ctc cgt tat tac tat gat aaa aac att atg acc aaa gtg cac ggc aaa 1234
   Leu Arg Tyr Tyr Tyr Asp Lys Asn Ile Met Thr Lys Val His Gly Lys
   340 345 350
   aga tat gct tac aaa ttt gac ttc cac ggc att gcc cag gct ctg cag 1282
   Arg Tyr Ala Tyr Lys Phe Asp Phe His Gly Ile Ala Gln Ala Leu Gln
   355 360 365 370
   cca cat ccg acc gag tcg tcc atg tac aag tac cct tct gac atc tcc 1330
   Pro His Pro Thr Glu Ser Ser Met Tyr Lys Tyr Pro Ser Asp Ile Ser
   375 380 385
   tac atg cct tcc caa cat gcc cac cag cag aag gtg aac ttt gtc cct 1378
   Tyr Met Pro Ser Gln His Ala His Gln Gln Lys Val Asn Phe Val Pro
   390 395 400
   ccc cat cca tcc tcc atg cct gtc act tcc tcc agc ttc ttt gga gcc 1426
   Pro His Pro Ser Ser Met Pro Val Thr Ser Ser Ser Phe Phe Gly Ala
   405 410 415
   gca tca caa tac tgg acc tcc acg ggg gga atc tac ccc aac ccc aac 1474
   Ala Ser Gln Tyr Trp Thr Ser Thr Gly Gly Ile Tyr Pro Asn Pro Asn
   420 425 430
   gtc ccc cgc cat cct aac acc cac gtg cct tca cac tta ggc agc tac 1522
   Val Pro Arg His Pro Asn Thr His Val Pro Ser His Leu Gly Ser Tyr
   435 440 445 450
   tac tagaagctta ctcatcagtg gccttctagc tgaagcccat cctgcacact 1575
   Tyr
   tactggatgc tttggactca acaggacata tgtggccttg aagggaagac aaaactggat 1635
   gttctttctt gttggataga acctttgtat ttgttcttta aaaatatttt ttttaatgtt 1695
   ggtaactttt gcttcctcta cctgaacaaa gagatgaata attccatggg ccagtatgcc 1755
   agtttgaatt ctcagtctcc tagcatcttg tgagttgcat attaagatta ctggaatggt 1815
   taagtcatgg ttctgagaaa gaagctgtac gttttcttta tgtttttatg accaaagcag 1875
   tttcttgtca atacacgggg ttcagtatga cacagaatca tggacttaac ccgtcatgtt 1935
   ctggtttgag atttagtgac aaatagaggt gggaagctta taatctaatt ttaggaggac 1995
   caaattcagc ggatggcaac tggaacattg attgtaaggc cagtgaagtt ttcacccaac 2055
   tggaatttga tggaaagaag gtttgtgtgt ttaagacgcc aagggcattg cagaatccct 2115
   ctcagtggac agtatgcact cagctgacca ctctctctag aaatagtcaa gatatgaact 2175
   aagaaatttt aatgcaaata catacattcc tgaaagacgg ggaattaaat tactaatttt 2235
   ttttttttaa atgatgacag tggtcccaga acttggaaaa gttgtaggga tttctaaact 2295
   caagcagatt cgcaagtgct gtgcgcttgt cagaccatca gaccagggcc aaccaatcag 2355
   aaggcaactt actgtataaa ttatgcagag ttattttcct atatctcaca gtattaaaaa 2415
   ataaataatt aaaaattaag aataaataaa cgagttgacc tcggtcacaa aagcagtttt 2475
   actatcgaat caatcgctgt tatttttttt taatgtaatt tgtacatctt ttttcaatct 2535
   gtacatttgg gctgtcttgt atgtttttat gctccttttt aaaaagcata atatgcctat 2595
   agctgaaaag gaaacagggc tgtttaagtc actgacttat gagaaagcaa agcactggta 2655
   cagttattta acaggcatac acaagcaggg aaaagataat ccatttagat ctttaatgct 2715
   ttggaaatgc gtgtaacagt actgcaataa tcacagctct gggaaaaaca acgaaacttt 2775
   cccttgtgga gaggagggat tttcctgctc tatataagca acatattttt agacattaaa 2835
   atatatataa ttttgcaggt aattgttgac ttttttaact atattaagtg ttaagctgac 2895
   aactgtcaaa gaagaccatg ttgtaaaata atttgactaa ataaatggtt ccttctctc 2954

<210> 84

<211> 451

<212> PRT

<213> Homo sapiens

<400> 84

Met Asp Gly Thr Ile Lys Glu Ala Leu Ser Val Val Ser Asp Asp Gln

1               5                   10                  15

Ser Leu Phe Asp Ser Ala Tyr Gly Ala Ala Ala His Leu Pro Lys Ala

            20                  25                  30

Asp Met Thr Ala Ser Gly Ser Pro Asp Tyr Gly Gln Pro His Lys Ile

        35                  40                  45

Asn Pro Leu Pro Pro Gln Gln Glu Trp Ile Asn Gln Pro Val Arg Val

    50                  55                  60

Asn Val Lys Arg Glu Tyr Asp His Met Asn Gly Ser Arg Glu Ser Pro

65                  70                  75                  80

Val Asp Cys Ser Val Ser Lys Cys Ser Lys Leu Val Gly Gly Gly Glu

                85                  90                  95

Ser Asn Pro Met Asn Tyr Asn Ser Tyr Met Asp Glu Lys Asn Gly Pro

            100                 105                 110

Pro Pro Pro Asn Met Thr Thr Asn Glu Arg Arg Val Ile Val Pro Ala

        115                 120                 125

Asp Pro Thr Leu Trp Thr Gln Glu His Val Arg Gln Trp Leu Glu Trp

    130                 135                 140

Ala Ile Lys Glu Tyr Ser Leu Met Glu Ile Asp Thr Ser Phe Phe Gln

145                 150                 155                 160

Asn Met Asp Gly Lys Glu Leu Cys Lys Met Asn Lys Glu Asp Phe Leu

                165                 170                 175

Arg Ala Thr Thr Leu Tyr Asn Thr Glu Val Leu Leu Ser His Leu Ser

            180                 185                 190
Tyr Leu Arg Glu Ser Ser Leu Leu Ala Tyr Asn Thr Thr Ser His Thr
        195                 200                 205
Asp Gln Ser Ser Arg Leu Ser Val Lys Glu Asp Pro Ser Tyr Asp Ser
    210                 215                 220
Val Arg Arg Gly Ala Trp Gly Asn Asn Met Asn Ser Gly Leu Asn Lys
225                 230                 235                 240
Ser Pro Pro Leu Gly Gly Ala Gln Thr Ile Ser Lys Asn Thr Glu Gln
                245                 250                 255
Arg Pro Gln Pro Asp Pro Tyr Gln Ile Leu Gly Pro Thr Ser Ser Arg
            260                 265                 270
Leu Ala Asn Pro Gly Ser Gly Gln Ile Gln Leu Trp Gln Phe Leu Leu
        275                 280                 285
Glu Leu Leu Ser Asp Ser Ala Asn Ala Ser Cys Ile Thr Trp Glu Gly
    290                 295                 300
Thr Asn Gly Glu Phe Lys Met Thr Asp Pro Asp Glu Val Ala Arg Arg
305                 310                 315                 320
Trp Gly Glu Arg Lys Ser Lys Pro Asn Met Asn Tyr Asp Lys Leu Ser
                325                 330                 335
Arg Ala Leu Arg Tyr Tyr Tyr Asp Lys Asn Ile Met Thr Lys Val His
            340                 345                 350
Gly Lys Arg Tyr Ala Tyr Lys Phe Asp Phe His Gly Ile Ala Gln Ala
        355                 360                 365
Leu Gln Pro His Pro Thr Glu Ser Ser Met Tyr Lys Tyr Pro Ser Asp
    370                 375                 380
Ile Ser Tyr Met Pro Ser Gln His Ala His Gln Gln Lys Val Asn Phe
385                 390                 395                 400

Val Pro Pro His Pro Ser Ser Met Pro Val Thr Ser Ser Ser Phe Phe
                405                 410                 415
Gly Ala Ala Ser Gln Tyr Trp Thr Ser Thr Gly Gly Ile Tyr Pro Asn
            420                 425                 430
Pro Asn Val Pro Arg His Pro Asn Thr His Val Pro Ser His Leu Gly
        435                 440                 445
Ser Tyr Tyr
    450
<210> 85
<211> 1817
<212> DNA
<213> Homo sapiens
<220>
<221> CDS
<222> (2)..(361)
<400> 85
   g cag cag gat ggg gag ggc cat acc cag gac acg gag ctc gtg gag acc 49
   Gln Gln Asp Gly Glu Gly His Thr Gln Asp Thr Glu Leu Val Glu Thr
   1 5 10 15
   agg cct gca ggg gat gga acc ttc cag aag tgg gca gct gtg gtg gtg 97
   Arg Pro Ala Gly Asp Gly Thr Phe Gln Lys Trp Ala Ala Val Val Val
   20 25 30
   cct tct gga gag gag cag aga tac acg tgc cat gtg cag cat gag ggg 145
   Pro Ser Gly Glu Glu Gln Arg Tyr Thr Cys His Val Gln His Glu Gly
   35 40 45
   cta ccc gag ccc gtc acc ctg aga tgg aag ccg gct tcc cag ccc acc 193
   Leu Pro Glu Pro Val Thr Leu Arg Trp Lys Pro Ala Ser Gln Pro Thr
   50 55 60
   atc ccc atc gtg ggc atc att gct ggc ctg gtt ctc ctt gga tct gtg 241
   Ile Pro Ile Val Gly Ile Ile Ala Gly Leu Val Leu Leu Gly Ser Val
   65 70 75 80
   gtc tct gga gct gtg gtt gct gct gtg ata tgg agg aag aag agc tca 289
   Val Ser Gly Ala Val Val Ala Ala Val Ile Trp Arg Lys Lys Ser Ser
   85 90 95
   ggt gga aaa gga ggg agc tac tct aag gct gag tgg agc gac agt gcc 337
   Gly Gly Lys Gly Gly Ser Tyr Ser Lys Ala Glu Trp Ser Asp Ser Ala
   100 105 110
   cag ggg tct gag tct cac agc ttg taaagcctga gacagctgcc ttgtgtgcga 391
   Gln Gly Ser Glu Ser His Ser Leu
   115 120
   ctgagatgca cagctgcctt gtgtgcgact gagatgcagg atttcctcac gcctccccta 451
   tgtgtcttag ggactctggc ttctcttttt tgcaagggcc tctgaatctg tctgtgtccc 511
   tgttagcaca atgtgaggag gtagagaaac agtccacctc tgtgtctacc atgaccccct 571
   tcctcacact gacctgtgtt ccttccctgt tctcttttct attaaaaata agaacctggg 631
   cagagtgcgg cagctcatgc ctgtaatccc agcacttagg gaggccgagg agggcagatc 691
   acgaggtcag gagatcgaaa ccatcctggc taacacggtg aaaccccgtc tctactaaaa 751
   aatacaaaaa attagctggg cgcagaggca cgggcctgta gtcccagcta ctcaggaggc 811
   ggaggcagga gaatggcgtc aacccgggag gcggaggttg cagtgagcca ggattgtgcg 871
   actgcatcca gcctgggtga cagggtgaaa cgccatctca aaaaataaaa attgaaaaat 931
   aaaaaaagaa cctggatctc aatttaattt ttcatattct tgcaatgaaa tggacttgag 991
   gaagctaaga tcatagctag aaatacagat aattccacag cacatctcta gcaaatttag 1051
   cctattccta ttctctagcc tattccttac cacctgtaat cttgaccata taccttggag 1111
   ttgaatattg ttttcatact gctgtggttt gaatgttccc tccaacactc atgttgagac 1171
   ttaatcccta atgtggcaat actgaaaggt ggggcctttg agatgtgatt ggatcgtaag 1231
   gctgtgcctt cattcatggg ttaatggatt aatgggttat cacaggaatg ggactggtgg 1291
   ctttataaga agaggaaaag agaactgagc tagcatgccc agcccacaga gagcctccac 1351
   tagagtgatg ctaagtggaa atgtgaggtg cagctgccac agagggcccc caccagggaa 1411
   atgtctagtg tctagtggat ccaggccaca ggagagagtg ccttgtggag cgctgggagc 1471
   aggaccctga accaccacca gaccccagaa ctgtggagtc agtggcagca tgcagcgccc 1531
   cttgggaaag ctttagcacc agcctgcaac ccattcgacg agccacgtag gctgcaccca 1591
   gcaaagccac aggcacgggg ctacctgagc cttgggggcc caatccctgc tccagtgtgt 1651
   ccgtgaggca gcacacgaag tcaaaagaga ttattctctt cccacagata ccttttctct 1711
   cccatgaccc tttaacagca tctgcttcat tcccctcacc ttcccaggct gatctgaggt 1771
   aaactttgaa gtaaaataaa agctgtgttt gagcatcaaa aaaaaa 1817

<210> 86
<211> 120
<212> PRT
<213> Homo sapiens
<400> 86
Gln Gln Asp Gly Glu Gly His Thr Gln Asp Thr Glu Leu Val Glu Thr
1               5                   10                  15
Arg Pro Ala Gly Asp Gly Thr Phe Gln Lys Trp Ala Ala Val Val Val
            20                  25                  30
Pro Ser Gly Glu Glu Gln Arg Tyr Thr Cys His Val Gln His Glu Gly
        35                  40                  45
Leu Pro Glu Pro Val Thr Leu Arg Trp Lys Pro Ala Ser Gln Pro Thr
    50                  55                  60
Ile Pro Ile Val Gly Ile Ile Ala Gly Leu Val Leu Leu Gly Ser Val

65                    70                    75                    80

Val Ser Gly Ala Val Val Ala Ala Val Ile Trp Arg Lys Lys Ser Ser

                85                    90                    95

Gly Gly Lys Gly Gly Ser Tyr Ser Lys Ala Glu Trp Ser Asp Ser Ala

            100                   105                   110

Gln Gly Ser Glu Ser His Ser Leu

        115                   120

<210> 87

<211> 2876

<212> DNA

<213> Homo sapiens

<220>

<221> CDS

<222> (76).. (1281)

<400> 87

gaattcctgc agctcagcag ccgccgccag agcaggacga accgccaatc gcaaggcacc 60

tctgagaact tcagg atg cag atg tct cca gcc ctc acc tgc cta gtc ctg  111

                 

                 1               5                   10

ggc ctg gcc ctt gtc ttt ggt gaa ggg tct gct gtg cac cat ccc cca   159



        15                  20                  25

tcc tac gtg gcc cac ctg gcc tca gac ttc ggg gtg agg gtg ttt cag   207



    30                  35                  40

cag gtg gcg cag gcc tcc aag gac cgc aac gtg gtt ttc tca ccc tat   255


45                  50                  55                  60
ggg gtg gcc tcg gtg ttg gcc atg ctc cag ctg aca aca gga gga gaa    303

                65                  70                  75
acc cag cag cag att caa gca gct atg gga ttc aag att gat gac aag    351

            80                  85                  90
ggc atg gcc ccc gcc ctc cgg cat ctg tac aag gag ctc atg ggg cca    399

        95                  100                 105
tgg aac aag gat gag atc agc acc aca gac gcg atc ttc gtc cag cgg    447

    110                 115                 120
gat ctg aag ctg gtc cag ggc ttc atg ccc cac ttc ttc agg ctg ttc    495

125                 130                 135                 140
cgg agc acg gtc aag caa gtg gac ttt tca gag gtg gag aga gcc aga    543

                145                 150                 155
ttc atc atc aat gac tgg gtg aag aca cac aca aaa ggt atg atc agc    591

            160                 165                 170
aac ttg ctt ggg aaa gga gcc gtg gac cag ctg aca cgg ctg gtg ctg    639

        175                 180                 185
gtg aat gcc ctc tac ttc aac ggc cag tgg aag act ccc ttc ccc gac    687


    190                 195                 200
tcc agc acc cac cgc cgc ctc ttc cac aaa tca gac ggc agc act gtc 735

205                 210                 215                 220
tct gtg ccc atg atg gct cag acc aac aag ttc aac tat act gag ttc 783

                225                 230                 235
acc acg ccc gat ggc cat tac tac gac atc ctg gaa ctg ccc tac cac 831

            240                 245                 250
ggg gac acc ctc agc atg ttc att gct gcc cct tat gaa aaa gag gtg 879

        255                 260                 265
cct ctc tct gcc ctc acc aac att ctg agt gcc cag ctc atc agc cac 927

    270                 275                 280
tgg aaa ggc aac atg acc agg ctg ccc cgc ctc ctg gtt ctg ccc aag 975

285                 290                 295                 300
ttc tcc ctg gag act gaa gtc gac ctc agg aag ccc cta gag aac ctg 1023

                305                 310                 315
gga atg acc gac atg ttc aga cag ttt cag gct gac ttc acg agt ctt 1071

            320                 325                 330

tca gac caa gag cct ctc cac gtc gcg cag gcg ctg cag aaa gtg aag 1119

        335                 340                 345
atc gag gtg aac gag agt ggc acg gtg gcc tcc tca tcc aca gct gtc 1167

    350                 355                 360
ata gtc tca gcc cgc atg gcc ccc gag gag atc atc atg gac aga ccc 1215

365                 370                 375                 380
ttc ctc ttt gtg gtc cgg cac aac ccc aca gga aca gtc ctt ttc atg 1263

                385                 390                 395
ggc caa gtg atg gaa ccc tgaccctggg gaaagacgcc ttcatctggg 1311

            400
acaaaactgg agatgcatcg ggaaagaaga aactccgaag aaaagaattt tagtgttaat 1371
gactctttct gaaggaagag aagacatttg ccttttgtta aaagatggta aaccagatct 1431
gtctccaaga ccttggcctc tccttggagg acctttaggt caaactccct agtctccacc 1491
tgagaccctg ggagagaagt ttgaagcaca actcccttaa ggtctccaaa ccagacggtg 1551
acgcctgcgg gaccatctgg ggcacctgct tccacccgtc tctctgccca ctcgggtctg 1611
cagacctggt tcccactgag gccctttgca ggatggaact acggggctta caggagcttt 1671
tgtgtgcctg gtagaaacta tttctgttcc agtcacattg ccatcactct tgtactgcct 1731
gccaccgcgg aggaggctgg tgacaggcca aaggccagtg gaagaaacac cctttcatct 1791
cagagtccac tgtggcactg gccacccctc cccagtacag gggtgctgca ggtggcagag 1851
tgaatgtccc ccatcatgtg gcccaactct cctggcctgg ccatctccct ccccagaaac 1911
agtgtgcatg ggttattttg gagtgtaggt gacttgttta ctcattgaag cagatttctg 1971
cttcctttta tttttatagg aatagaggaa gaaatgtcag atgcgtgccc agctcttcac 2031

cccccaatct cttggtgggg aggggtgtac ctaaatattt atcatatcct tgcccttgag 2091
tgcttgttag agagaaagag aactactaag gaaaataata ttatttaaac tcgctcctag 2151
tgtttctttg tggtctgtgt caccgtatct caggaagtcc agccacttga ctggcacaca 2211
cccctccgga catccagcgt gacggagccc acactgccac cttgtggccg cctgagaccc 2271
tcgcgccccc cgcgcccccc gcgcccctct ttttcccctt gatggaaatt gaccatacaa 2331
tttcatcctc cttcagggga tcaaaaggac ggagtggggg gacagagact cagatgagga 2391
cagagtggtt tccaatgtgt tcaatagatt taggagcaga aatgcaaggg gctgcatgac 2451
ctaccaggac agaactttcc ccaattacag ggtgactcac agccgcattg gtgactcact 2511
tcaatgtgtc atttccggct gctgtgtgtg agcagtggac acgtgagggg ggggtgggtg 2571
agagagacag gcagctcgga ttcaactacc ttagataata tttctgaaaa cctaccagcc 2631
agagggtagg gcacaaagat ggatgtaatg cactttggga ggccaaggcg ggaggattgc 2691
ttgagcccag gagttcaaga ccagcctggg caacatacca agacccccgt ctctttaaaa 2751
atatatatat tttaaatata cttaaatata tatttctaat atctttaaat atatatatat 2811
attttaaaga ccaatttatg ggagaattgc acacagatgt gaaatgaatg taatctaata 2871
gaagc 2876

<210> 88

<211> 402

<212> PRT

<213> Homo sapiens

<400> 88

<210> 89

<211> 1412

<212> DNA

<213> Homo sapiens

<220>

<221> CDS

<222> (52)..(1341)

<400> 89

cggggtcgtc cgcaaagcct gagtcctgtc ctttctctct ccccggacag c atg agc 57

Met Ser

1

ttc acc act cgc tcc acc ttc tcc acc aac tac cgg tcc ctg ggc tct 105

Phe Thr Thr Arg Ser Thr Phe Ser Thr Asn Tyr Arg Ser Leu Gly Ser

        5                   10                  15

gtc cag gcg ccc agc tac ggc gcc cgg ccg gtc agc agc gcg gcc agc 153

Val Gln Ala Pro Ser Tyr Gly Ala Arg Pro Val Ser Ser Ala Ala Ser

    20                  25                  30

gtc tat gca ggc gct ggg ggc tct ggt tcc cgg atc tcc gtg tcc cgc 201

Val Tyr Ala Gly Ala Gly Gly Ser Gly Ser Arg Ile Ser Val Ser Arg

35                  40                  45                  50

tcc acc agc ttc agg ggc ggc atg ggg tcc ggg ggc ctg gcc acc ggg 249

Ser Thr Ser Phe Arg Gly Gly Met Gly Ser Gly Gly Leu Ala Thr Gly

                55                  60                  65

ata gcc ggg ggt ctg gca gga atg gga ggc atc cag aac gag aag gag 297

Ile Ala Gly Gly Leu Ala Gly Met Gly Gly Ile Gln Asn Glu Lys Glu

            70                  75                  80

acc atg caa agc ctg aac gac cgc ctg gcc tct tac ctg gac aga gtg 345

Thr Met Gln Ser Leu Asn Asp Arg Leu Ala Ser Tyr Leu Asp Arg Val

        85                  90                  95

agg agc ctg gag acc gag aac cgg agg ctg gag agc aaa atc cgg gag 393

Arg Ser Leu Glu Thr Glu Asn Arg Arg Leu Glu Ser Lys Ile Arg Glu

    100                 105                 110

cac ttg gag aag aag gga ccc cag gtc aga gac tgg agc cat tac ttc 441

His Leu Glu Lys Lys Gly Pro Gln Val Arg Asp Trp Ser His Tyr Phe

115                 120                 125                 130

aag atc atc gag gac ctg agg gct cag atc ttc gca aat act gtg gac    489

Lys Ile Ile Glu Asp Leu Arg Ala Gln Ile Phe Ala Asn Thr Val Asp

                135                 140                 145

aat gcc cgc atc gtt ctg cag att gac aat gcc cgt ctt gct gct gat    537

Asn Ala Arg Ile Val Leu Gln Ile Asp Asn Ala Arg Leu Ala Ala Asp

            150                 155                 160

gac ttt aga gtc aag tat gag aca gag ctg gcc atg cgc cag tct gtg    585

Asp Phe Arg Val Lys Tyr Glu Thr Glu Leu Ala Met Arg Gln Ser Val

        165                 170                 175

gag aac gac atc cat ggg ctc cgc aag gtc att gat gac acc aat atc    633

Glu Asn Asp Ile His Gly Leu Arg Lys Val Ile Asp Asp Thr Asn Ile

    180                 185                 190

aca cga ctg cag ctg gag aca gag atc gag gct ctc aag gag gag ctg    681

Thr Arg Leu Gln Leu Glu Thr Glu Ile Glu Ala Leu Lys Glu Glu Leu

195                 200                 205                 210

ctc ttc atg aag aag aac cac gaa gag gaa gta aaa ggc cta caa gcc    729

Leu Phe Met Lys Lys Asn His Glu Glu Glu Val Lys Gly Leu Gln Ala

                215                 220                 225

cag att gcc agc tct ggg ttg acc gtg gag gta gat gcc ccc aaa tct    777

Gln Ile Ala Ser Ser Gly Leu Thr Val Glu Val Asp Ala Pro Lys Ser

            230                 235                 240

cag gac ctc gcc aag atc atg gca gac atc cgg gcc caa tat gac gag    825

Gln Asp Leu Ala Lys Ile Met Ala Asp Ile Arg Ala Gln Tyr Asp Glu

        245                 250                 255

ctg gct cgg aag aac cga gag gag cta gac aag tac tgg tct cag cag    873

Leu Ala Arg Lys Asn Arg Glu Glu Leu Asp Lys Tyr Trp Ser Gln Gln
    260                 265                 270
att gag gag agc acc aca gtg gtc acc aca cag tct gct gag gtt gga 921
Ile Glu Glu Ser Thr Thr Val Val Thr Thr Gln Ser Ala Glu Val Gly
275                 280                 285                 290
gct gct gag acg acg ctc aca gag ctg aga cgt aca gtc cag tcc ttg 969
Ala Ala Glu Thr Thr Leu Thr Glu Leu Arg Arg Thr Val Gln Ser Leu
                295                 300                 305
gag atc gac ctg gac tcc atg aga aat ctg aag gcc agc ttg gag aac 1017
Glu Ile Asp Leu Asp Ser Met Arg Asn Leu Lys Ala Ser Leu Glu Asn
            310                 315                 320
agc ctg agg gag gtg gag gcc cgc tac gcc cta cag atg gag cag ctc 1065
Ser Leu Arg Glu Val Glu Ala Arg Tyr Ala Leu Gln Met Glu Gln Leu
        325                 330                 335
aac ggg atc ctg ctg cac ctt gag tca gag ctg gca cag acc cgg gca 1113
Asn Gly Ile Leu Leu His Leu Glu Ser Glu Leu Ala Gln Thr Arg Ala
    340                 345                 350
gag gga cag cgc cag gcc cag gag tat gag gcc ctg ctg aac atc aag 1161
Glu Gly Gln Arg Gln Ala Gln Glu Tyr Glu Ala Leu Leu Asn Ile Lys
355                 360                 365                 370
gtc aag ctg gag gct gag atc gcc acc tac cgc cgc ctg ctg gaa gat 1209
Val Lys Leu Glu Ala Glu Ile Ala Thr Tyr Arg Arg Leu Leu Glu Asp
                375                 380                 385
ggc gag gac ttt aat ctt ggt gat gcc ttg gac agc agc aac tcc atg 1257
Gly Glu Asp Phe Asn Leu Gly Asp Ala Leu Asp Ser Ser Asn Ser Met
            390                 395                 400
caa acc atc caa aag acc acc acc cgc cgg ata gtg gat ggc aaa gtg 1305

Gln Thr Ile Gln Lys Thr Thr Thr Arg Arg Ile Val Asp Gly Lys Val
        405                 410                 415
gtg tct gag acc aat gac acc aaa gtt ctg agg cat taagccagca 1351
Val Ser Glu Thr Asn Asp Thr Lys Val Leu Arg His
    420                 425                 430
gaagcagggt accctttggg gagcaggagg ccaataaaaa gttcagagtt cattggatgt 1411
c 1412
<210> 90
<211> 430
<212> PRT
<213> Homo sapiens
<400> 90
Met Ser Phe Thr Thr Arg Ser Thr Phe Ser Thr Asn Tyr Arg Ser Leu
1               5                   10                  15
Gly Ser Val Gln Ala Pro Ser Tyr Gly Ala Arg Pro Val Ser Ser Ala
            20                  25                  30
Ala Ser Val Tyr Ala Gly Ala Gly Gly Ser Gly Ser Arg Ile Ser Val
        35                  40                  45
Ser Arg Ser Thr Ser Phe Arg Gly Gly Met Gly Ser Gly Gly Leu Ala
    50                  55                  60
Thr Gly Ile Ala Gly Gly Leu Ala Gly Met Gly Gly Ile Gln Asn Glu
65                  70                  75                  80
Lys Glu Thr Met Gln Ser Leu Asn Asp Arg Leu Ala Ser Tyr Leu Asp
                85                  90                  95
Arg Val Arg Ser Leu Glu Thr Glu Asn Arg Arg Leu Glu Ser Lys Ile
            100                 105                 110

Arg Glu His Leu Glu Lys Lys Gly Pro Gln Val Arg Asp Trp Ser His
        115                 120                 125
Tyr Phe Lys Ile Ile Glu Asp Leu Arg Ala Gln Ile Phe Ala Asn Thr
    130                 135                 140
Val Asp Asn Ala Arg Ile Val Leu Gln Ile Asp Asn Ala Arg Leu Ala
145                 150                 155                 160
Ala Asp Asp Phe Arg Val Lys Tyr Glu Thr Glu Leu Ala Met Arg Gln
                165                 170                 175
Ser Val Glu Asn Asp Ile His Gly Leu Arg Lys Val Ile Asp Asp Thr
            180                 185                 190
Asn Ile Thr Arg Leu Gln Leu Glu Thr Glu Ile Glu Ala Leu Lys Glu
        195                 200                 205
Glu Leu Leu Phe Met Lys Lys Asn His Glu Glu Glu Val Lys Gly Leu
    210                 215                 220
Gln Ala Gln Ile Ala Ser Ser Gly Leu Thr Val Glu Val Asp Ala Pro
225                 230                 235                 240
Lys Ser Gln Asp Leu Ala Lys Ile Met Ala Asp Ile Arg Ala Gln Tyr
                245                 250                 255
Asp Glu Leu Ala Arg Lys Asn Arg Glu Glu Leu Asp Lys Tyr Trp Ser
            260                 265                 270
Gln Gln Ile Glu Glu Ser Thr Thr Val Val Thr Thr Gln Ser Ala Glu
        275                 280                 285
Val Gly Ala Ala Glu Thr Thr Leu Thr Glu Leu Arg Arg Thr Val Gln
    290                 295                 300
Ser Leu Glu Ile Asp Leu Asp Ser Met Arg Asn Leu Lys Ala Ser Leu
305                 310                 315                 320

Glu Asn Ser Leu Arg Glu Val Glu Ala Arg Tyr Ala Leu Gln Met Glu
                325                 330                 335
Gln Leu Asn Gly Ile Leu Leu His Leu Glu Ser Glu Leu Ala Gln Thr
            340                 345                 350
Arg Ala Glu Gly Gln Arg Gln Ala Gln Glu Tyr Glu Ala Leu Leu Asn
        355                 360                 365
Ile Lys Val Lys Leu Glu Ala Glu Ile Ala Thr Tyr Arg Arg Leu Leu
    370                 375                 380
Glu Asp Gly Glu Asp Phe Asn Leu Gly Asp Ala Leu Asp Ser Ser Asn
385                 390                 395                 400
Ser Met Gln Thr Ile Gln Lys Thr Thr Thr Arg Arg Ile Val Asp Gly
                405                 410                 415
Lys Val Val Ser Glu Thr Asn Asp Thr Lys Val Leu Arg His
            420                 425                 430

<210> 91
<211> 1040
<212> DNA
<213> Homo sapiens
<220>
<221> CDS
<222> (171)..(968)
<400> 91
gggcagacga tgctgaagat gctctccttt aagctgctgc tgctggccgt ggctctgggc 60
ttctttgaag gagatgctaa gtttggggaa agaaacgaag ggagcggaca aggaggagaa 120
ggtgcctgaa tgggaacccc ccgaagcgcc tgaaaaggag agacaggagg atg atg 176
Met Met

tcc cag ctg gag ctg ctg agt ggg gga gag atg ctg tgc ggt ggc ttc 224
Ser Gln Leu Glu Leu Leu Ser Gly Gly Glu Met Leu Cys Gly Gly Phe
        5                   10                  15
tac cct cgg ctg tcc tgc tgc ctg cgg agt gac agc ccg ggg cta ggg 272
Tyr Pro Arg Leu Ser Cys Cys Leu Arg Ser Asp Ser Pro Gly Leu Gly
    20                  25                  30
cgc ctg gag aat aag ata ttt tct gtt acc aac aac aca gaa tgt ggg 320
Arg Leu Glu Asn Lys Ile Phe Ser Val Thr Asn Asn Thr Glu Cys Gly
35                  40                  45                  50
aag tta ctg gag gaa atc aaa tgt gca ctt tgc tct cca cat tct caa 368
Lys Leu Leu Glu Glu Ile Lys Cys Ala Leu Cys Ser Pro His Ser Gln
                55                  60                  65
agc ctg ttc cac tca cct gag aga gaa gtc ttg gaa aga gac cta gta 416
Ser Leu Phe His Ser Pro Glu Arg Glu Val Leu Glu Arg Asp Leu Val
            70                  75                  80
ctt cct ctg ctc tgc aaa gac tat tgc aaa gaa ttc ttt tac act tgc 464
Leu Pro Leu Leu Cys Lys Asp Tyr Cys Lys Glu Phe Phe Tyr Thr Cys
        85                  90                  95
cga ggc cat att cca ggt ttc ctt caa aca act gcg gat gag ttt tgc 512
Arg Gly His Ile Pro Gly Phe Leu Gln Thr Thr Ala Asp Glu Phe Cys
    100                 105                 110
ttt tac tat gca aga aaa gat ggt ggg ttg tgc ttt cca gat ttt cca 560
Phe Tyr Tyr Ala Arg Lys Asp Gly Gly Leu Cys Phe Pro Asp Phe Pro
115                 120                 125                 130
aga aaa caa gtc aga gga cca gca tct aac tac ttg gac cag atg gaa 608
Arg Lys Gln Val Arg Gly Pro Ala Ser Asn Tyr Leu Asp Gln Met Glu

                135                 140                 145

gaa tat gac aaa gtg gaa gag atc agc aga aag cac aaa cac aac tgc 656

Glu Tyr Asp Lys Val Glu Glu Ile Ser Arg Lys His Lys His Asn Cys

            150                 155                 160

ttc tgt att cag gag gtt gtg agt ggg ctg cgg cag ccc gtt ggt gcc 704

Phe Cys Ile Gln Glu Val Val Ser Gly Leu Arg Gln Pro Val Gly Ala

        165                 170                 175

ctg cat agt ggg gat ggc tcg caa cgt ctc ttc att ctg gaa aaa gaa 752

Leu His Ser Gly Asp Gly Ser Gln Arg Leu Phe Ile Leu Glu Lys Glu

    180                 185                 190

ggt tat gtg aag ata ctt acc cct gaa gga gaa att ttc aag gag cct 800

Gly Tyr Val Lys Ile Leu Thr Pro Glu Gly Glu Ile Phe Lys Glu Pro

195                 200                 205                 210

tat ttg gac att cac aaa ctt gtt caa agt gga ata aag gtt ggc ttt 848

Tyr Leu Asp Ile His Lys Leu Val Gln Ser Gly Ile Lys Val Gly Phe

                215                 220                 225

tta aat ttt att tat ttt tgt gct ggc tac gtt aat ttt att tta gtg 896

Leu Asn Phe Ile Tyr Phe Cys Ala Gly Tyr Val Asn Phe Ile Leu Val

            230                 235                 240

tta cct tcc tca ctg aag gta ttt ctt tgt aat aaa aga aag aat ctt 944

Leu Pro Ser Ser Leu Lys Val Phe Leu Cys Asn Lys Arg Lys Asn Leu

        245                 250                 255

gca gga gaa aat aag ggg gca aca taagaaacaa taattatggc acctgaatta 998

Ala Gly Glu Asn Lys Gly Ala Thr

    260                 265

ggacagtgac attaaakgtt ggctktttaw attttaaaaa aa 1040

<210> 92

<211> 266

<212> PRT

<213> Homo sapiens

<400> 92

Met Met Ser Gln Leu Glu Leu Leu Ser Gly Gly Glu Met Leu Cys Gly

1               5                   10                  15

Gly Phe Tyr Pro Arg Leu Ser Cys Cys Leu Arg Ser Asp Ser Pro Gly

            20                  25                  30

Leu Gly Arg Leu Glu Asn Lys Ile Phe Ser Val Thr Asn Asn Thr Glu

        35                  40                  45

Cys Gly Lys Leu Leu Glu Glu Ile Lys Cys Ala Leu Cys Ser Pro His

    50                  55                  60

Ser Gln Ser Leu Phe His Ser Pro Glu Arg Glu Val Leu Glu Arg Asp

65                  70                  75                  80

Leu Val Leu Pro Leu Leu Cys Lys Asp Tyr Cys Lys Glu Phe Phe Tyr

                85                  90                  95

Thr Cys Arg Gly His Ile Pro Gly Phe Leu Gln Thr Thr Ala Asp Glu

            100                 105                 110

Phe Cys Phe Tyr Tyr Ala Arg Lys Asp Gly Gly Leu Cys Phe Pro Asp

        115                 120                 125

Phe Pro Arg Lys Gln Val Arg Gly Pro Ala Ser Asn Tyr Leu Asp Gln

    130                 135                 140

Met Glu Glu Tyr Asp Lys Val Glu Glu Ile Ser Arg Lys His Lys His

145                 150                 155                 160

Asn Cys Phe Cys Ile Gln Glu Val Val Ser Gly Leu Arg Gln Pro Val

                165                 170                 175

Gly Ala Leu His Ser Gly Asp Gly Ser Gln Arg Leu Phe Ile Leu Glu

            180                 185                 190

Lys Glu Gly Tyr Val Lys Ile Leu Thr Pro Glu Gly Glu Ile Phe Lys

        195                 200                 205

Glu Pro Tyr Leu Asp Ile His Lys Leu Val Gln Ser Gly Ile Lys Val

    210                 215                 220

Gly Phe Leu Asn Phe Ile Tyr Phe Cys Ala Gly Tyr Val Asn Phe Ile

225                 230                 235                 240

Leu Val Leu Pro Ser Ser Leu Lys Val Phe Leu Cys Asn Lys Arg Lys

                245                 250                 255

Asn Leu Ala Gly Glu Asn Lys Gly Ala Thr

            260                 265

<210> 93

<211> 1639

<212> DNA

<213> Homo sapiens

<220>

<221> CDS

<222> (75)..(371)

<400> 93

agcagagcac acaagcttct aggacaagag ccaggaagaa accaccggaa ggaaccatct 60

cactgtgtgt aaac atg act tcc aag ctg gcc gtg gct ctc ttg gca gcc 110

Met Thr Ser Lys Leu Ala Val Ala Leu Leu Ala Ala

1               5                   10

ttc ctg att tct gca gct ctg tgt gaa ggt gca gtt ttg cca agg agt 158

Phe Leu Ile Ser Ala Ala Leu Cys Glu Gly Ala Val Leu Pro Arg Ser
        15                  20                  25
gct aaa gaa ctt aga tgt cag tgc ata aag aca tac tcc aaa cct ttc 206
Ala Lys Glu Leu Arg Cys Gln Cys Ile Lys Thr Tyr Ser Lys Pro Phe
    30                  35                  40
cac ccc aaa ttt atc aaa gaa ctg aga gtg att gag agt gga cca cac 254
His Pro Lys Phe Ile Lys Glu Leu Arg Val Ile Glu Ser Gly Pro His
45                  50                  55                  60
tgc gcc aac aca gaa att att gta aag ctt tct gat gga aga gag ctc 302
Cys Ala Asn Thr Glu Ile Ile Val Lys Leu Ser Asp Gly Arg Glu Leu
                65                  70                  75
tgt ctg gac ccc aag gaa aac tgg gtg cag agg gtt gtg gag aag ttt 350
Cys Leu Asp Pro Lys Glu Asn Trp Val Gln Arg Val Val Glu Lys Phe
            80                  85                  90
ttg aag agg gct gag aat tca taaaaaaatt cattctctgt ggtatccaag 401
Leu Lys Arg Ala Glu Asn Ser
        95
aatcagtgaa gatgccagtg aaacttcaag caaatctact tcaacacttc atgtattgtg 461
tgggtctgtt gtagggttgc cagatgcaat acaagattcc tggttaaatt tgaatttcag 521
taaacaatga atagtttttc attgtaccat gaaatatcca gaacatactt atatgtaaag 581
tattatttat ttgaatctac aaaaaacaac aaataatttt taaatataag gatttcccta 641
gatattgcac gggagaatat acaaatagca aaattgagcc aagggccaag agaatatccg 701
aactttaatt tcaggaattg aatgggtttg ctagaatgtg atatttgaag catcacataa 761
aaatgatggg acaataaatt ttgccataaa gtcaaattta gctggaaatc ctggattttt 821
ttctgttaaa tctggcaacc ctagtctgct agccaggatc cacaagtcct tgttccactg 881
tgccttggtt tctcctttat ttctaagtgg aaaaagtatt agccaccatc ttacctcaca 941
gtgatgttgt gaggacatgt ggaagcactt taagtttttt catcataaca taaattattt 1001

tcaagtgtaa cttattaacc tatttattat ttatgtattt atttaagcat caaatatttg 1061
tgcaagaatt tggaaaaata gaagatgaat cattgattga atagttataa agatgttata 1121
gtaaatttat tttattttag atattaaatg atgttttatt agataaattt caatcagggt 1181
ttttagatta aacaaagaaa caattgggta cccagttaaa ttttcatttc agataaacaa 1241
caaataattt tttagtataa gtacattatt gtttatctga aagttttaat tgaactaaca 1301
atcctagttt gatactccca gtcttgtcat tgccagctgt gttggtagtg ctgtgttgaa 1361
ttacggaata atgagttaga actattaaaa cagccaaaac tccacagtca atattagtaa 1421
tttcttgctg gttgaaactt gtttattatg tacaaataga ttcttataat attatttaaa 1481
tgactgcatt tttaaataca aggctttata tttttaactt taagatgttt ttatgtgctc 1541
tccaaatttt ttttactgtt tctgattgta tggaaatata aaagtaaata tgaaacattt 1601
aaaatataat ttgttgtcaa agtaaaaaaa aaaaaaaa 1639

<210> 94
<211> 99
<212> PRT
<213> Homo sapiens
<400> 94
Met Thr Ser Lys Leu Ala Val Ala Leu Leu Ala Ala Phe Leu Ile Ser
Ala Ala Leu Cys Glu Gly Ala Val Leu Pro Arg Ser Ala Lys Glu Leu
1 5 10 15
Arg Cys Gln Cys Ile Lys Thr Tyr Ser Lys Pro Phe His Pro Lys Phe
20 25 30
Ile Lys Glu Leu Arg Val Ile Glu Ser Gly Pro His Cys Ala Asn Thr
35 40 45
Glu Ile Ile Val Lys Leu Ser Asp Gly Arg Glu Leu Cys Leu Asp Pro
50 55 60
Lys Glu Asn Trp Val Gln Arg Val Val Glu Lys Phe Leu Lys Arg Ala

65                  70                  75                  80

Glu Asn Ser

<210> 95

<211> 3293

<212> DNA

<213> Homo sapiens

<220>

<221> CDS

<222> (8)..(1945)

<400> 95

gggaagc atg ggg ctt ccc agg ctg gtc tgc gcc ttc ttg ctc gcc gcc    49

        Met Gly Leu Pro Arg Leu Val Cys Ala Phe Leu Leu Ala Ala

          1               5                  10

tgc tgc tgc tgt cct cgc gtc gcg ggt gtg ccc gga gag gct gag cag    97

Cys Cys Cys Cys Pro Arg Val Ala Gly Val Pro Gly Glu Ala Glu Gln

 15                  20                  25                  30

cct gcg cct gag ctg gtg gag gtg gaa gtg ggc agc aca gcc ctt ctg    145

Pro Ala Pro Glu Leu Val Glu Val Glu Val Gly Ser Thr Ala Leu Leu

                 35                  40                  45

aag tgc ggc ctc tcc cag tcc caa ggc aac ctc agc cat gtc gac tgg    193

Lys Cys Gly Leu Ser Gln Ser Gln Gly Asn Leu Ser His Val Asp Trp

             50                  55                  60

ttt tct gtc cac aag gag aag cgg acg ctc atc ttc cgt gtg cgc cag    241

Phe Ser Val His Lys Glu Lys Arg Thr Leu Ile Phe Arg Val Arg Gln

         65                  70                  75

ggc cag ggc cag agc gaa cct ggg gag tac gag cag cgg ctc agc ctc    289

Gly Gln Gly Gln Ser Glu Pro Gly Glu Tyr Glu Gln Arg Leu Ser Leu
    80                  85                  90
cag gac aga ggg gct act ctg gcc ctg act caa gtc acc ccc caa gac 337
Gln Asp Arg Gly Ala Thr Leu Ala Leu Thr Gln Val Thr Pro Gln Asp
95                  100                 105                 110
gag cgc atc ttc ttg tgc cag ggc aag cgc cct cgg tcc cag gag tac 385
Glu Arg Ile Phe Leu Cys Gln Gly Lys Arg Pro Arg Ser Gln Glu Tyr
                115                 120                 125
cgc atc cag ctc cgc gtc tac aaa gct ccg gag gag cca aac atc cag 433
Arg Ile Gln Leu Arg Val Tyr Lys Ala Pro Glu Glu Pro Asn Ile Gln
            130                 135                 140
gtc aac ccc ctg ggc atc cct gtg aac agt aag gag cct gag gag gtc 481
Val Asn Pro Leu Gly Ile Pro Val Asn Ser Lys Glu Pro Glu Glu Val
        145                 150                 155
gct acc tgt gta ggg agg aac ggg tac ccc att cct caa gtc atc tgg 529
Ala Thr Cys Val Gly Arg Asn Gly Tyr Pro Ile Pro Gln Val Ile Trp
    160                 165                 170
tac aag aat ggc cgg cct ctg aag gag gag aag aac cgg gtc cac att 577
Tyr Lys Asn Gly Arg Pro Leu Lys Glu Glu Lys Asn Arg Val His Ile
175                 180                 185                 190
cag tcg tcc cag act gtg gag tcg agt ggt ttg tac acc ttg cag agt 625
Gln Ser Ser Gln Thr Val Glu Ser Ser Gly Leu Tyr Thr Leu Gln Ser
                195                 200                 205
att ctg aag gca cag ctg gtt aaa gaa gac aaa gat gcc cag ttt tac 673
Ile Leu Lys Ala Gln Leu Val Lys Glu Asp Lys Asp Ala Gln Phe Tyr
            210                 215                 220

tgt gag ctc aac tac cgg ctg ccc agt ggg aac cac atg aag gag tcc 721
Cys Glu Leu Asn Tyr Arg Leu Pro Ser Gly Asn His Met Lys Glu Ser
        225                 230                 235
agg gaa gtc acc gtc cct gtt ttc tac ccg aca gaa aaa gtg tgg ctg 769
Arg Glu Val Thr Val Pro Val Phe Tyr Pro Thr Glu Lys Val Trp Leu
    240                 245                 250
gaa gtg gag ccc gtg gga atg ctg aag gaa ggg gac cgc gtg gaa atc 817
Glu Val Glu Pro Val Gly Met Leu Lys Glu Gly Asp Arg Val Glu Ile
255                 260                 265                 270
agg tgt ttg gct gat ggc aac cct cca cca cac ttc agc atc agc aag 865
Arg Cys Leu Ala Asp Gly Asn Pro Pro Pro His Phe Ser Ile Ser Lys
                275                 280                 285
cag aac ccc agc acc agg gag gca gag gaa gag aca acc aac gac aac 913
Gln Asn Pro Ser Thr Arg Glu Ala Glu Glu Glu Thr Thr Asn Asp Asn
            290                 295                 300
ggg gtc ctg gtg ctg gag cct gcc cgg aag gaa cac agt ggg cgc tat 961
Gly Val Leu Val Leu Glu Pro Ala Arg Lys Glu His Ser Gly Arg Tyr
        305                 310                 315
gaa tgt cag gcc tgg aac ttg gac acc atg ata tcg ctg ctg agt gaa 1009
Glu Cys Gln Ala Trp Asn Leu Asp Thr Met Ile Ser Leu Leu Ser Glu
    320                 325                 330
cca cag gaa cta ctg gtg aac tat gtg tct gac gtc cga gtg agt ccc 1057
Pro Gln Glu Leu Leu Val Asn Tyr Val Ser Asp Val Arg Val Ser Pro
335                 340                 345                 350
gca gcc cct gag aga cag gaa ggc agc agc ctc acc ctg acc tgt gag 1105
Ala Ala Pro Glu Arg Gln Glu Gly Ser Ser Leu Thr Leu Thr Cys Glu
                355                 360                 365

gca gag agt agc cag gac ctc gag ttc cag tgg ctg aga gaa gag aca 1153
Ala Glu Ser Ser Gln Asp Leu Glu Phe Gln Trp Leu Arg Glu Glu Thr
            370                 375                 380
gac cag gtg ctg gaa agg ggg cct gtg ctt cag ttg cat gac ctg aaa 1201
Asp Gln Val Leu Glu Arg Gly Pro Val Leu Gln Leu His Asp Leu Lys
        385                 390                 395
cgg gag gca gga ggc ggc tat cgc tgc gtg gcg tct gtg ccc agc ata 1249
Arg Glu Ala Gly Gly Gly Tyr Arg Cys Val Ala Ser Val Pro Ser Ile
    400                 405                 410
ccc ggc ctg aac cgc aca cag ctg gtc aag ctg gcc att ttt ggc ccc 1297
Pro Gly Leu Asn Arg Thr Gln Leu Val Lys Leu Ala Ile Phe Gly Pro
415                 420                 425                 430
cct tgg atg gca ttc aag gag agg aag gtg tgg gtg aaa gag aat atg 1345
Pro Trp Met Ala Phe Lys Glu Arg Lys Val Trp Val Lys Glu Asn Met
                435                 440                 445
gtg ttg aat ctg tct tgt gaa gcg tca ggg cac ccc cgg ccc acc atc 1393
Val Leu Asn Leu Ser Cys Glu Ala Ser Gly His Pro Arg Pro Thr Ile
            450                 455                 460
tcc tgg aac gtc aac ggc acg gca agt gaa caa gac caa gat cca cag 1441
Ser Trp Asn Val Asn Gly Thr Ala Ser Glu Gln Asp Gln Asp Pro Gln
        465                 470                 475
cga gtc ctg agc acc ctg aat gtc ctc gtg acc ccg gag ctg ttg gag 1489
Arg Val Leu Ser Thr Leu Asn Val Leu Val Thr Pro Glu Leu Leu Glu
    480                 485                 490
aca ggt gtt gaa tgc acg gcc tcc aac gac ctg ggc aaa aac acc agc 1537
Thr Gly Val Glu Cys Thr Ala Ser Asn Asp Leu Gly Lys Asn Thr Ser

495                    500                    505                    510

atc ctc ttc ctg gag ctg gtc aat tta acc acc ctc aca cca gac tcc    1585

Ile Leu Phe Leu Glu Leu Val Asn Leu Thr Thr Leu Thr Pro Asp Ser

                515                    520                    525

aac aca acc act ggc ctc agc act tcc act gcc agt cct cat acc aga    1633

Asn Thr Thr Thr Gly Leu Ser Thr Ser Thr Ala Ser Pro His Thr Arg

            530                    535                    540

gcc aac agc acc tcc aca gag aga aag ctg ccg gag ccg gag agc cgg    1681

Ala Asn Ser Thr Ser Thr Glu Arg Lys Leu Pro Glu Pro Glu Ser Arg

        545                    550                    555

ggc gtg gtc atc gtg gct gtg att gtg tgc atc ctg gtc ctg gcg gtg    1729

Gly Val Val Ile Val Ala Val Ile Val Cys Ile Leu Val Leu Ala Val

    560                    565                    570

ctg ggc gct gtc ctc tat ttc ctc tat aag aag ggc aag ctg ccg tgc    1777

Leu Gly Ala Val Leu Tyr Phe Leu Tyr Lys Lys Gly Lys Leu Pro Cys

575                    580                    585                    590

agg cgc tca ggg aag cag gag atc acg ctg ccc ccg tct cgt aag acc    1825

Arg Arg Ser Gly Lys Gln Glu Ile Thr Leu Pro Pro Ser Arg Lys Thr

                595                    600                    605

gaa ctt gta gtt gaa gtt aag tca gat aag ctc cca gaa gag atg ggc    1873

Glu Leu Val Val Glu Val Lys Ser Asp Lys Leu Pro Glu Glu Met Gly

            610                    615                    620

ctc ctg cag ggc agc agc ggt gac aag agg gct ccg gga gac cag gga    1921

Leu Leu Gln Gly Ser Ser Gly Asp Lys Arg Ala Pro Gly Asp Gln Gly

        625                    630                    635

gag aaa tac atc gat ctg agg cat tagccccgaa tcacttcagc tcccttccct    1975

Glu Lys Tyr Ile Asp Leu Arg His

640                645

gcctggacca ttcccagctc cctgctcact cttctctcag ccaaagctca aagggactag 2035

agagaagcct cctgctcccc tcgcctgcac accccctttc agagggccac tgggttagga 2095

cctgaggacc tcacttggcc ctgcaaggcc cgcttttcag ggaccagtcc accaccatct 2155

cctccacgtt gagtgaagct catcccaagc aaggagcccc agtctcccga gcgggtagga 2215

gagtttcttg cagaacgtgt tttttcttta cacacattat gctgtaaata cgctcgtcct 2275

gccagcagct gagctgggta gcctctctga gctggtttcc tgccccaaag gctggcattc 2335

caccatccag gtgcaccact gaagtgagga cacaccggag ccaggcgcct gctcatgttg 2395

aagtgcgctg ttcacacccg ctccggagag caccccagca gcatccagaa gcagctgcag 2455

tgcaagcttg catgcctgcg tgttgctgca ccaccctcct gtctgcctct tcaaagtctc 2515

ctgtgacatt ttttctttgg tcagaggcca ggaactgtgt cattccttaa agatacgtgc 2575

cggggccagg tgtggctcac gcctgtaatc ccagcacttt gggaggccga ggcggcggat 2635

cacaaagtca gacgagacca tcctggctaa cacggtgaaa ccctgtctct actaaaaata 2695

caaaaaaaaa ttagctaggc gtagtggttg gcacctatag tcccagctac tcggaaggct 2755

gaagcaggag aatggtatga atccaggagg tggagcttgc agtgagccga gaccgtgcca 2815

ctgcactcca gcctgggcaa cacagcgaga ctccgtctcg aggaaaaaaa aaatcgtgct 2875

cgtagcagct ggctctgttt cgagtcaggt gaattagcct caatccccgt gttcacttgc 2935

tcccatagcc ctcttgatgg atcacgtaaa actgaaaggc agcggggagc agacaaagat 2995

gaggtctaca ctgtccttca tggggattaa agctatggtt atattagcac caaacttcta 3055

caaaccaagc tcaggaccca accctagaag ggcccaaatg agagaatggt acttagggat 3115

ggcaaaacgg gcctggctag agcttcgggt gtgtgtgtct gtctgtgtat gcatacatat 3175

gtgtgtatat atggttttgt caggtgtgta aatttgcaaa ttgtttcctt tatatatgta 3235

tgtatatata tatatgaaaa tatatatata tatgaaaaat aaagcttaat tgtcccag   3293

<210> 96

<211> 646

<212> PRT

<213> Homo sapiens

<400> 96

Met Gly Leu Pro Arg Leu Val Cys Ala Phe Leu Leu Ala Ala Cys Cys

1               5                   10                  15

Cys Cys Pro Arg Val Ala Gly Val Pro Gly Glu Ala Glu Gln Pro Ala

            20                  25                  30

Pro Glu Leu Val Glu Val Glu Val Gly Ser Thr Ala Leu Leu Lys Cys

        35                  40                  45

Gly Leu Ser Gln Ser Gln Gly Asn Leu Ser His Val Asp Trp Phe Ser

    50                  55                  60

Val His Lys Glu Lys Arg Thr Leu Ile Phe Arg Val Arg Gln Gly Gln

65                  70                  75                  80

Gly Gln Ser Glu Pro Gly Glu Tyr Glu Gln Arg Leu Ser Leu Gln Asp

                85                  90                  95

Arg Gly Ala Thr Leu Ala Leu Thr Gln Val Thr Pro Gln Asp Glu Arg

            100                 105                 110

Ile Phe Leu Cys Gln Gly Lys Arg Pro Arg Ser Gln Glu Tyr Arg Ile

        115                 120                 125

Gln Leu Arg Val Tyr Lys Ala Pro Glu Glu Pro Asn Ile Gln Val Asn

    130                 135                 140

Pro Leu Gly Ile Pro Val Asn Ser Lys Glu Pro Glu Glu Val Ala Thr

145                 150                 155                 160

Cys Val Gly Arg Asn Gly Tyr Pro Ile Pro Gln Val Ile Trp Tyr Lys

                165                 170                 175

Asn Gly Arg Pro Leu Lys Glu Glu Lys Asn Arg Val His Ile Gln Ser

            180                 185                 190

Ser Gln Thr Val Glu Ser Ser Gly Leu Tyr Thr Leu Gln Ser Ile Leu
        195                 200                 205
Lys Ala Gln Leu Val Lys Glu Asp Lys Asp Ala Gln Phe Tyr Cys Glu
    210                 215                 220
Leu Asn Tyr Arg Leu Pro Ser Gly Asn His Met Lys Glu Ser Arg Glu
225                 230                 235                 240
Val Thr Val Pro Val Phe Tyr Pro Thr Glu Lys Val Trp Leu Glu Val
                245                 250                 255
Glu Pro Val Gly Met Leu Lys Glu Gly Asp Arg Val Glu Ile Arg Cys
            260                 265                 270
Leu Ala Asp Gly Asn Pro Pro Pro His Phe Ser Ile Ser Lys Gln Asn
        275                 280                 285
Pro Ser Thr Arg Glu Ala Glu Glu Glu Thr Thr Asn Asp Asn Gly Val
    290                 295                 300
Leu Val Leu Glu Pro Ala Arg Lys Glu His Ser Gly Arg Tyr Glu Cys
305                 310                 315                 320
Gln Ala Trp Asn Leu Asp Thr Met Ile Ser Leu Leu Ser Glu Pro Gln
                325                 330                 335
Glu Leu Leu Val Asn Tyr Val Ser Asp Val Arg Val Ser Pro Ala Ala
            340                 345                 350
Pro Glu Arg Gln Glu Gly Ser Ser Leu Thr Leu Thr Cys Glu Ala Glu
        355                 360                 365
Ser Ser Gln Asp Leu Glu Phe Gln Trp Leu Arg Glu Glu Thr Asp Gln
    370                 375                 380
Val Leu Glu Arg Gly Pro Val Leu Gln Leu His Asp Leu Lys Arg Glu
385                 390                 395                 400
Ala Gly Gly Gly Tyr Arg Cys Val Ala Ser Val Pro Ser Ile Pro Gly

                405                 410                 415
Leu Asn Arg Thr Gln Leu Val Lys Leu Ala Ile Phe Gly Pro Pro Trp
            420                 425                 430
Met Ala Phe Lys Glu Arg Lys Val Trp Val Lys Glu Asn Met Val Leu
        435                 440                 445
Asn Leu Ser Cys Glu Ala Ser Gly His Pro Arg Pro Thr Ile Ser Trp
    450                 455                 460
Asn Val Asn Gly Thr Ala Ser Glu Gln Asp Gln Asp Pro Gln Arg Val
465                 470                 475                 480
Leu Ser Thr Leu Asn Val Leu Val Thr Pro Glu Leu Leu Glu Thr Gly
                485                 490                 495
Val Glu Cys Thr Ala Ser Asn Asp Leu Gly Lys Asn Thr Ser Ile Leu
            500                 505                 510
Phe Leu Glu Leu Val Asn Leu Thr Thr Leu Thr Pro Asp Ser Asn Thr
        515                 520                 525
Thr Thr Gly Leu Ser Thr Ser Thr Ala Ser Pro His Thr Arg Ala Asn
    530                 535                 540
Ser Thr Ser Thr Glu Arg Lys Leu Pro Glu Pro Glu Ser Arg Gly Val
545                 550                 555                 560
Val Ile Val Ala Val Ile Val Cys Ile Leu Val Leu Ala Val Leu Gly
                565                 570                 575
Ala Val Leu Tyr Phe Leu Tyr Lys Lys Gly Lys Leu Pro Cys Arg Arg
            580                 585                 590
Ser Gly Lys Gln Glu Ile Thr Leu Pro Pro Ser Arg Lys Thr Glu Leu
        595                 600                 605
Val Val Glu Val Lys Ser Asp Lys Leu Pro Glu Glu Met Gly Leu Leu

610                 615                 620

Gln Gly Ser Ser Gly Asp Lys Arg Ala Pro Gly Asp Gln Gly Glu Lys

625                 630                 635                 640

Tyr Ile Asp Leu Arg His

                645

<210> 97

<211> 1642

<212> DNA

<213> Homo sapiens

<220>

<221> CDS

<222> (158)..(1279)

<400> 97
gaatcggcgg tcccgcaggt cccggatgtt gcggacagta tgaggcaagc gcagggggac 60
ggggaccagc agctgtcgcc gccgctctca gggtgaagag ggaacagaaa tctttgcccc 120
ctgactttgg aaatctcgtt taaccttcaa actggcg atg tca agg gtt cca agt 175
cct cca cct ccg gca gaa atg tcg agt ggc ccc gta gct gag agt tgg 223
tgc tac aca cag atc aag gta gtg aaa ttc tcc tac atg tgg acc atc 271
aat aac ttt agc ttt tgc cgg gag gaa atg ggt gaa gtc att aaa agt 319
tct aca ttt tca tca gga gca aat gat aaa ctg aaa tgg tgt ttg cga 367
gta aac ccc aaa ggg tta gat gaa gaa agc aaa gat tac ctg tca ctt 415
tac ctg tta ctg gtc agc tgt cca aag agt gaa gtt cgg gca aaa ttc 463
aaa ttc tcc atc ctg aat gcc aag gga gaa gaa acc aaa gct atg gag 511
agt caa cgg gca tat agg ttt gtg caa ggc aaa gac tgg gga ttc aag 559
aaa ttc atc cgt aga gat ttt ctt ttg gat gag gcc aac ggg ctt ctc 607
cct gat gac aag ctt acc ctc ttc tgc gag gtg agt gtt gtg caa gat 655
tct gtc aac att tct ggc cag aat acc atg aac atg gta aag gtt cct 703
gag tgc cgg ctg gca gat gag tta gga gga ctg tgg gag aat tcc cgg 751
ttc aca gac tgc tgc ttg tgt gtt gcc ggc cag gaa ttc cag gct cac 799
aag gct atc tta gca gct cgt tct ccg gtt ttt agt gcc atg ttt gaa 847
cat gaa atg gag gag agc aaa aag aat cga gtt gaa atc aat gat gtg 895
gag cct gaa gtt ttt aag gaa atg atg tgc ttc att tac acg ggg aag 943
gct cca aac ctc gac aaa atg gct gat gat ttg ctg gca gct gct gac 991
aag tat gcc ctg gag cgc tta aag gtc atg tgt gag gat gcc ctc tgc 1039
agt aac ctg tcc gtg gag aac gct gca gaa att ctc atc ctg gcc gac 1087
ctc cac agt gca gat cag ttg aaa act cag gca gtg gat ttc atc aac 1135
tat cat gct tcg gat gtc ttg gag acc tct ggg tgg aag tca atg gtg 1183
gtg tca cat ccc cac ttg gtg gct gag gca tac cgc tct ctg gct tca 1231
gca cag tgc cct ttt ctg gga ccc cca cgc aaa cgc ctg aag caa tcc 1279
taagatcctg cttgttgtaa gactccgttt aatttccaga agcagcagcc actgttgctg 1339
ccactgacca ccaggtagac agcgcaatct gtggagcttt tactctgttg tgaggggaag 1399
agactgcatt gtggccccag acttttaaaa cagcactaaa taacttgggg gaaacggggg 1459
gagggaaaat gaaatgaaaa ccctgttgct gcgtcactgt gttccctttg gcctgtctga 1519
gtttgatact gtggggattc agtttaggcg ctggcccgag gatatcccag cggtggtact 1579
tcggagacac ctgtctgcat ctgactgagc agaacaaatc gtcaggtgcc tggagcaaaa 1639
agg 1642
<210> 98
<211> 374
<212> PRT
<213> Homo sapiens
<400> 98

<210> 99

<211> 5722

<212> DNA

<213> Homo sapiens

<220>

<221> CDS

<222> (112)..(3621)

<400> 99
   ggacgcacag gcattccccg cgcccctcca gccctcgccg ccctcgccac cgctcccggc 60
   cgccgcgctc cggtacacac aggatccctg ctgggcacca acagctccac c atg ggg 117
   Met Gly
   1
   ctg gcc tgg gga cta ggc gtc ctg ttc ctg atg cat gtg tgt ggc acc 165
   Leu Ala Trp Gly Leu Gly Val Leu Phe Leu Met His Val Cys Gly Thr
   5 10 15
   aac cgc att cca gag tct ggc gga gac aac agc gtg ttt gac atc ttt 213
   Asn Arg Ile Pro Glu Ser Gly Gly Asp Asn Ser Val Phe Asp Ile Phe
   20 25 30
   gaa ctc acc ggg gcc gcc cgc aag ggg tct ggg cgc cga ctg gtg aag 261
   Glu Leu Thr Gly Ala Ala Arg Lys Gly Ser Gly Arg Arg Leu Val Lys
   35 40 45 50
   ggc ccc gac cct tcc agc cca gct ttc cgc atc gag gat gcc aac ctg 309
   Gly Pro Asp Pro Ser Ser Pro Ala Phe Arg Ile Glu Asp Ala Asn Leu
   55 60 65
   atc ccc cct gtg cct gat gac aag ttc caa gac ctg gtg gat gct gtg 357
   Ile Pro Pro Val Pro Asp Asp Lys Phe Gln Asp Leu Val Asp Ala Val
   70 75 80
   cgg gca gaa aag ggt ttc ctc ctt ctg gca tcc ctg agg cag atg aag 405
   Arg Ala Glu Lys Gly Phe Leu Leu Leu Ala Ser Leu Arg Gln Met Lys
   85 90 95
   aag acc cgg ggc acg ctg ctg gcc ctg gag cgg aaa gac cac tct ggc 453
   Lys Thr Arg Gly Thr Leu Leu Ala Leu Glu Arg Lys Asp His Ser Gly
   100 105 110
   cag gtc ttc agc gtg gtg tcc aat ggc aag gcg ggc acc ctg gac ctc 501
   Gln Val Phe Ser Val Val Ser Asn Gly Lys Ala Gly Thr Leu Asp Leu
   115 120 125 130
   agc ctg acc gtc caa gga aag cag cac gtg gtg tct gtg gaa gaa gct 549
   Ser Leu Thr Val Gln Gly Lys Gln His Val Val Ser Val Glu Glu Ala
   135 140 145
   ctc ctg gca acc ggc cag tgg aag agc atc acc ctg ttt gtg cag gaa 597
   Leu Leu Ala Thr Gly Gln Trp Lys Ser Ile Thr Leu Phe Val Gln Glu
   150 155 160
   gac agg gcc cag ctg tac atc gac tgt gaa aag atg gag aat gct gag 645
   Asp Arg Ala Gln Leu Tyr Ile Asp Cys Glu Lys Met Glu Asn Ala Glu
   165 170 175
   ttg gac gtc ccc atc caa agc gtc ttc acc aga gac ctg gcc agc atc 693
   Leu Asp Val Pro Ile Gln Ser Val Phe Thr Arg Asp Leu Ala Ser Ile
   180 185 190
   gcc aga ctc cgc atc gca aag ggg ggc gtc aat gac aat ttc cag ggg 741
   Ala Arg Leu Arg Ile Ala Lys Gly Gly Val Asn Asp Asn Phe Gln Gly
   195 200 205 210
   gtg ctg cag aat gtg agg ttt gtc ttt gga acc aca cca gaa gac atc 789
   Val Leu Gln Asn Val Arg Phe Val Phe Gly Thr Thr Pro Glu Asp Ile
   215 220 225
   ctc agg aac aaa ggc tgc tcc agc tct acc agt gtc ctc ctc acc ctt 837
   Leu Arg Asn Lys Gly Cys Ser Ser Ser Thr Ser Val Leu Leu Thr Leu
   230 235 240
   gac aac aac gtg gtg aat ggt tcc agc cct gcc atc cgc act aac tac 885
   Asp Asn Asn Val Val Asn Gly Ser Ser Pro Ala Ile Arg Thr Asn Tyr
   245 250 255
   att ggc cac aag aca aag gac ttg caa gcc atc tgc ggc atc tcc tgt 933
   Ile Gly His Lys Thr Lys Asp Leu Gln Ala Ile Cys Gly Ile Ser Cys
   260 265 270
   gat gag ctg tcc agc atg gtc ctg gaa ctc agg ggc ctg cgc acc att 981
   Asp Glu Leu Ser Ser Met Val Leu Glu Leu Arg Gly Leu Arg Thr Ile
   275 280 285 290
   gtg acc acg ctg cag gac agc atc cgc aaa gtg act gaa gag aac aaa 1029
   Val Thr Thr Leu Gln Asp Ser Ile Arg Lys Val Thr Glu Glu Asn Lys
   295 300 305
   gag ttg gcc aat gag ctg agg cgg cct ccc cta tgc tat cac aac gga 1077
   Glu Leu Ala Asn Glu Leu Arg Arg Pro Pro Leu Cys Tyr His Asn Gly
   310 315 320
   gtt cag tac aga aat aac gag gaa tgg act gtt gat agc tgc act gag 1125
   Val Gln Tyr Arg Asn Asn Glu Glu Trp Thr Val Asp Ser Cys Thr Glu
   325 330 335
   tgt cac tgt cag aac tca gtt acc atc tgc aaa aag gtg tcc tgc ccc 1173
   Cys His Cys Gln Asn Ser Val Thr Ile Cys Lys Lys Val Ser Cys Pro
   340 345 350
   atc atg ccc tgc tcc aat gcc aca gtt cct gat gga gaa tgc tgt cct 1221
   Ile Met Pro Cys Ser Asn Ala Thr Val Pro Asp Gly Glu Cys Cys Pro
   355 360 365 370
   cgc tgt tgg ccc agc gac tct gcg gac gat ggc tgg tct cca tgg tcc 1269
   Arg Cys Trp Pro Ser Asp Ser Ala Asp Asp Gly Trp Ser Pro Trp Ser
   375 380 385
   gag tgg acc tcc tgt tct acg agc tgt ggc aat gga att cag cag cgc 1317
   Glu Trp Thr Ser Cys Ser Thr Ser Cys Gly Asn Gly Ile Gln Gln Arg
   390 395 400
   ggc cgc tcc tgc gat agc ctc aac aac cga tgt gag ggc tcc tcg gtc 1365
   Gly Arg Ser Cys Asp Ser Leu Asn Asn Arg Cys Glu Gly Ser Ser Val
   405 410 415
   cag aca cgg acc tgc cac att cag gag tgt gac aaa aga ttt aaa cag 1413
   Gln Thr Arg Thr Cys His Ile Gln Glu Cys Asp Lys Arg Phe Lys Gln
   420 425 430
   gat ggt ggc tgg agc cac tgg tcc ccg tgg tca tct tgt tct gtg aca 1461
   Asp Gly Gly Trp Ser His Trp Ser Pro Trp Ser Ser Cys Ser Val Thr
   435 440 445 450
   tgt ggt gat ggt gtg atc aca agg atc cgg ctc tgc aac tct ccc agc 1509
   Cys Gly Asp Gly Val Ile Thr Arg Ile Arg Leu Cys Asn Ser Pro Ser
   455 460 465
   ccc cag atg aat ggg aaa ccc tgt gaa ggc gaa gcg cgg gag acc aaa 1557
   Pro Gln Met Asn Gly Lys Pro Cys Glu Gly Glu Ala Arg Glu Thr Lys
   470 475 480
   gcc tgc aag aaa gac gcc tgc ccc atc aat gga ggc tgg ggt cct tgg 1605
   Ala Cys Lys Lys Asp Ala Cys Pro Ile Asn Gly Gly Trp Gly Pro Trp
   485 490 495
   tca cca tgg gac atc tgt tct gtc acc tgt gga gga ggg gta cag aaa 1653
   Ser Pro Trp Asp Ile Cys Ser Val Thr Cys Gly Gly Gly Val Gln Lys
   500 505 510
   cgt agt cgt ctc tgc aac aac ccc gca ccc cag ttt gga ggc aag gac 1701
   Arg Ser Arg Leu Cys Asn Asn Pro Ala Pro Gln Phe Gly Gly Lys Asp
   515 520 525 530
   tgc gtt ggt gat gta aca gaa aac cag atc tgc aac aag cag gac tgt 1749
   Cys Val Gly Asp Val Thr Glu Asn Gln Ile Cys Asn Lys Gln Asp Cys
   535 540 545
   cca att gat gga tgc ctg tcc aat ccc tgc ttt gcc ggc gtg aag tgt 1797
   Pro Ile Asp Gly Cys Leu Ser Asn Pro Cys Phe Ala Gly Val Lys Cys
   550 555 560
   act agc tac cct gat ggc agc tgg aaa tgt ggt gct tgt ccc cct ggt 1845
   Thr Ser Tyr Pro Asp Gly Ser Trp Lys Cys Gly Ala Cys Pro Pro Gly
   565 570 575
   tac agt gga aat ggc atc cag tgc aca gat gtt gat gag tgc aaa gaa 1893
   Tyr Ser Gly Asn Gly Ile Gln Cys Thr Asp Val Asp Glu Cys Lys Glu
   580 585 590
   gtg cct gat gcc tgc ttc aac cac aat gga gag cac cgg tgt gag aac 1941
   Val Pro Asp Ala Cys Phe Asn His Asn Gly Glu His Arg Cys Glu Asn
   595 600 605 610
   acg gac ccc ggc tac aac tgc ctg ccc tgc ccc cca cgc ttc acc ggc 1989
   Thr Asp Pro Gly Tyr Asn Cys Leu Pro Cys Pro Pro Arg Phe Thr Gly
   615 620 625
   tca cag ccc ttc ggc cag ggt gtc gaa cat gcc acg gcc aac aaa cag 2037
   Ser Gln Pro Phe Gly Gln Gly Val Glu His Ala Thr Ala Asn Lys Gln
   630 635 640
   gtg tgc aag ccc cgt aac ccc tgc acg gat ggg acc cac gac tgc aac 2085
   Val Cys Lys Pro Arg Asn Pro Cys Thr Asp Gly Thr His Asp Cys Asn
   645 650 655
   aag aac gcc aag tgc aac tac ctg ggc cac tat agc gac ccc atg tac 2133
   Lys Asn Ala Lys Cys Asn Tyr Leu Gly His Tyr Ser Asp Pro Met Tyr
   660 665 670
   cgc tgc gag tgc aag cct ggc tac gct ggc aat ggc atc atc tgc ggg 2181
   Arg Cys Glu Cys Lys Pro Gly Tyr Ala Gly Asn Gly Ile Ile Cys Gly
   675 680 685 690
   gag gac aca gac ctg gat ggc tgg ccc aat gag aac ctg gtg tgc gtg 2229
   Glu Asp Thr Asp Leu Asp Gly Trp Pro Asn Glu Asn Leu Val Cys Val
   695 700 705
   gcc aat gcg act tac cac tgc aaa aag gat aat tgc ccc aac ctt ccc 2277
   Ala Asn Ala Thr Tyr His Cys Lys Lys Asp Asn Cys Pro Asn Leu Pro
   710 715 720
   aac tca ggg cag gaa gac tat gac aag gat gga att ggt gat gcc tgt 2325
   Asn Ser Gly Gln Glu Asp Tyr Asp Lys Asp Gly Ile Gly Asp Ala Cys
   725 730 735
   gat gat gac gat gac aat gat aaa att cca gat gac agg gac aac tgt 2373
   Asp Asp Asp Asp Asp Asn Asp Lys Ile Pro Asp Asp Arg Asp Asn Cys
   740 745 750
   cca ttc cat tac aac cca gct cag tat gac tat gac aga gat gat gtg 2421
   Pro Phe His Tyr Asn Pro Ala Gln Tyr Asp Tyr Asp Arg Asp Asp Val
   755 760 765 770
   gga gac cgc tgt gac aac tgt ccc tac aac cac aac cca gat cag gca 2469
   Gly Asp Arg Cys Asp Asn Cys Pro Tyr Asn His Asn Pro Asp Gln Ala
   775 780 785
   gac aca gac aac aat ggg gaa gga gac gcc tgt gct gca gac att gat 2517
   Asp Thr Asp Asn Asn Gly Glu Gly Asp Ala Cys Ala Ala Asp Ile Asp
   790 795 800
   gga gac ggt atc ctc aat gaa cgg gac aac tgc cag tac gtc tac aat 2565
   Gly Asp Gly Ile Leu Asn Glu Arg Asp Asn Cys Gln Tyr Val Tyr Asn
   805 810 815
   gtg gac cag aga gac act gat atg gat ggg gtt gga gat cag tgt gac 2613
   Val Asp Gln Arg Asp Thr Asp Met Asp Gly Val Gly Asp Gln Cys Asp
   820 825 830
   aat tgc ccc ttg gaa cac aat ccg gat cag ctg gac tct gac tca gac 2661
   Asn Cys Pro Leu Glu His Asn Pro Asp Gln Leu Asp Ser Asp Ser Asp
   835 840 845 850
   cgc att gga gat acc tgt gac aac aat cag gat att gat gaa gat ggc 2709
   Arg Ile Gly Asp Thr Cys Asp Asn Asn Gln Asp Ile Asp Glu Asp Gly
   855 860 865
   cac cag aac aat ctg gac aac tgt ccc tat gtg ccc aat gcc aac cag 2757
   His Gln Asn Asn Leu Asp Asn Cys Pro Tyr Val Pro Asn Ala Asn Gln
   870 875 880
   gct gac cat gac aaa gat ggc aag gga gat gcc tgt gac cac gat gat 2805
   Ala Asp His Asp Lys Asp Gly Lys Gly Asp Ala Cys Asp His Asp Asp
   885 890 895
   gac aac gat ggc att cct gat gac aag gac aac tgc aga ctc gtg ccc 2853
   Asp Asn Asp Gly Ile Pro Asp Asp Lys Asp Asn Cys Arg Leu Val Pro
   900 905 910
   aat ccc gac cag aag gac tct gac ggc gat ggt cga ggt gat gcc tgc 2901
   Asn Pro Asp Gln Lys Asp Ser Asp Gly Asp Gly Arg Gly Asp Ala Cys
   915 920 925 930
   aaa gat gat ttt gac cat gac agt gtg cca gac atc gat gac atc tgt 2949
   Lys Asp Asp Phe Asp His Asp Ser Val Pro Asp Ile Asp Asp Ile Cys
   935 940 945
   cct gag aat gtt gac atc agt gag acc gat ttc cgc cga ttc cag atg 2997
   Pro Glu Asn Val Asp Ile Ser Glu Thr Asp Phe Arg Arg Phe Gln Met
   950 955 960
   att cct ctg gac ccc aaa ggg aca tcc caa aat gac cct aac tgg gtt 3045
   Ile Pro Leu Asp Pro Lys Gly Thr Ser Gln Asn Asp Pro Asn Trp Val
   965 970 975
   gta cgc cat cag ggt aaa gaa ctc gtc cag act gtc aac tgt gat cct 3093
   Val Arg His Gln Gly Lys Glu Leu Val Gln Thr Val Asn Cys Asp Pro
   980 985 990
   gga ctc gct gta ggt tat gat gag ttt aat gct gtg gac ttc agt ggc 3141
   Gly Leu Ala Val Gly Tyr Asp Glu Phe Asn Ala Val Asp Phe Ser Gly
   995 1000 1005 1010
   acc ttc ttc atc aac acc gaa agg gac gat gac tat gct gga ttt gtc 3189
   Thr Phe Phe Ile Asn Thr Glu Arg Asp Asp Asp Tyr Ala Gly Phe Val
   1015 1020 1025
   ttt ggc tac cag tcc agc agc cgc ttt tat gtt gtg atg tgg aag caa 3237
   Phe Gly Tyr Gln Ser Ser Ser Arg Phe Tyr Val Val Met Trp Lys Gln
   1030 1035 1040
   gtc acc cag tcc tac tgg gac acc aac ccc acg agg gct cag gga tac 3285
   Val Thr Gln Ser Tyr Trp Asp Thr Asn Pro Thr Arg Ala Gln Gly Tyr
   1045 1050 1055
   tcg ggc ctt tct gtg aaa gtt gta aac tcc acc aca ggg cct ggc gag 3333
   Ser Gly Leu Ser Val Lys Val Val Asn Ser Thr Thr Gly Pro Gly Glu
   1060 1065 1070
   cac ctg cgg aac gcc ctg tgg cac aca gga aac acc cct ggc cag gtg 3381
   His Leu Arg Asn Ala Leu Trp His Thr Gly Asn Thr Pro Gly Gln Val
   1075 1080 1085 1090
   cgc acc ctg tgg cat gac cct cgt cac ata ggc tgg aaa gat ttc acc 3429
   Arg Thr Leu Trp His Asp Pro Arg His Ile Gly Trp Lys Asp Phe Thr
   1095 1100 1105
   gcc tac aga tgg cgt ctc agc cac agg cca aag acg ggt ttc att aga 3477
   Ala Tyr Arg Trp Arg Leu Ser His Arg Pro Lys Thr Gly Phe Ile Arg
   1110 1115 1120
   gtg gtg atg tat gaa ggg aag aaa atc atg gct gac tca gga ccc atc 3525
   Val Val Met Tyr Glu Gly Lys Lys Ile Met Ala Asp Ser Gly Pro Ile
   1125 1130 1135
   tat gat aaa acc tat gct ggt ggt aga cta ggg ttg ttt gtc ttc tct 3573
   Tyr Asp Lys Thr Tyr Ala Gly Gly Arg Leu Gly Leu Phe Val Phe Ser
   1140 1145 1150
   caa gaa atg gtg ttc ttc tct gac ctg aaa tac gaa tgt aga gat ccc 3621
   Gln Glu Met Val Phe Phe Ser Asp Leu Lys Tyr Glu Cys Arg Asp Pro
   1155 1160 1165 1170
   taatcatcaa attgttgatt gaaagactga tcataaacca atgctggtat tgcaccttct 3681
   ggaactatgg gcttgagaaa acccccagga tcacttctcc ttggcttcct tcttttctgt 3741
   gcttgcatca gtgtggactc ctagaacgtg cgacctgcct caagaaaatg cagttttcaa 3801
   aaacagactc atcagcattc agcctccaat gaataagaca tcttccaagc atataaacaa 3861
   ttgctttggt ttccttttga aaaagcatct acttgcttca gttgggaagg tgcccattcc 3921
   actctgcctt tgtcacagag cagggtgcta ttgtgaggcc atctctgagc agtggactca 3981
   aaagcatttt caggcatgtc agagaaggga ggactcacta gaattagcaa acaaaaccac 4041
   cctgacatcc tccttcagga acacggggag cagaggccaa agcactaagg ggagggcgca 4101
   tacccgagac gattgtatga agaaaatatg gaggaactgt tacatgttcg gtactaagtc 4161
   attttcaggg gattgaaaga ctattgctgg atttcatgat gctgactggc gttagctgat 4221
   taacccatgt aaataggcac ttaaatagaa gcaggaaagg gagacaaaga ctggcttctg 4281
   gacttcctcc ctgatcccca cccttactca tcaccttgca gtggccagaa ttagggaatc 4341
   agaatcaaac cagtgtaagg cagtgctggc tgccattgcc tggtcacatt gaaattggtg 4401
   gcttcattct agatgtagct tgtgcagatg tagcaggaaa ataggaaaac ctaccatctc 4461
   agtgagcacc agctgcctcc caaaggaggg gcagccgtgc ttatattttt atggttacaa 4521
   tggcacaaaa ttattatcaa cctaactaaa acattccttt tctctttttt ccgtaattac 4581
   taggtagttt tctaattctc tcttttggaa gtatgatttt tttaaagtct ttacgatgta 4641
   aaatatttat tttttactta ttctggaaga tctggctgaa ggattattca tggaacagga 4701
   agaagcgtaa agactatcca tgtcatcttt gttgagagtc ttcgtgactg taagattgta 4761
   aatacagatt atttattaac tctgttctgc ctggaaattt aggcttcata cggaaagtgt 4821
   ttgagagcaa gtagttgaca tttatcagca aatctcttgc aagaacagca caaggaaaat 4881
   cagtctaata agctgctctg ccccttgtgc tcagagtgga tgttatggga ttcctttttt 4941
   ctctgtttta tcttttcaag tggaattagt tggttatcca tttgcaaatg ttttaaattg 5001
   caaagaaagc catgaggtct tcaatactgt tttaccccat cccttgtgca tatttccagg 5061
   gagaaggaaa gcatatacac tttttctttt catttttcca aaagagaaaa aaatgacaaa 5121
   aggtgaaact tacatacaaa tattacctca tttgttgtgt gactgagtaa agaatttttg 5181
   gatcaagcgg aaagagttta agtgtctaac aaacttaaag ctactgtagt acctaaaaag 5241
   tcagtgttgt acatagcata aaaactctgc agagaagtat tcccaataag gaaatagcat 5301
   tgaaatgtta aatacaattt ctgaaagtta tgtttttttt ctatcatctg gtataccatt 5361
   gctttatttt tataaattat tttctcattg ccattggaat agaatattca gattgtgtag 5421
   atatgctatt taaataattt atcaggaaat actgcctgta gagttagtat ttctattttt 5481
   atataatgtt tgcacactga attgaagaat tgttggtttt ttcttttttt tgtttttttt 5541
   tttttttttt ttttttttgc ttttgacctc ccatttttac tatttgccaa tacctttttc 5601
   taggaatgtg cttttttttg tacacatttt tatccatttt acattctaaa gcagtgtaag 5661
   ttgtatatta ctgtttctta tgtacaagga acaacaataa atcatatgga aatttatatt 5721
   t 5722

<210> 100
<211> 1170
<212> PRT
<213> Homo sapiens
<400> 100
Met Gly Leu Ala Trp Gly Leu Gly Val Leu Phe Leu Met His Val Cys
1               5                   10                  15
Gly Thr Asn Arg Ile Pro Glu Ser Gly Gly Asp Asn Ser Val Phe Asp

            20                  25                  30
Ile Phe Glu Leu Thr Gly Ala Ala Arg Lys Gly Ser Gly Arg Arg Leu
        35                  40                  45
Val Lys Gly Pro Asp Pro Ser Ser Pro Ala Phe Arg Ile Glu Asp Ala
    50                  55                  60
Asn Leu Ile Pro Pro Val Pro Asp Asp Lys Phe Gln Asp Leu Val Asp
65                  70                  75                  80
Ala Val Arg Ala Glu Lys Gly Phe Leu Leu Leu Ala Ser Leu Arg Gln
                85                  90                  95
Met Lys Lys Thr Arg Gly Thr Leu Leu Ala Leu Glu Arg Lys Asp His
            100                 105                 110
Ser Gly Gln Val Phe Ser Val Val Ser Asn Gly Lys Ala Gly Thr Leu
        115                 120                 125
Asp Leu Ser Leu Thr Val Gln Gly Lys Gln His Val Val Ser Val Glu
    130                 135                 140
Glu Ala Leu Leu Ala Thr Gly Gln Trp Lys Ser Ile Thr Leu Phe Val
145                 150                 155                 160
Gln Glu Asp Arg Ala Gln Leu Tyr Ile Asp Cys Glu Lys Met Glu Asn
                165                 170                 175
Ala Glu Leu Asp Val Pro Ile Gln Ser Val Phe Thr Arg Asp Leu Ala
            180                 185                 190
Ser Ile Ala Arg Leu Arg Ile Ala Lys Gly Gly Val Asn Asp Asn Phe
        195                 200                 205
Gln Gly Val Leu Gln Asn Val Arg Phe Val Phe Gly Thr Thr Pro Glu
    210                 215                 220
Asp Ile Leu Arg Asn Lys Gly Cys Ser Ser Ser Thr Ser Val Leu Leu
225                 230                 235                 240

Thr Leu Asp Asn Asn Val Val Asn Gly Ser Ser Pro Ala Ile Arg Thr
                245                 250                 255
Asn Tyr Ile Gly His Lys Thr Lys Asp Leu Gln Ala Ile Cys Gly Ile
            260                 265                 270
Ser Cys Asp Glu Leu Ser Ser Met Val Leu Glu Leu Arg Gly Leu Arg
        275                 280                 285
Thr Ile Val Thr Thr Leu Gln Asp Ser Ile Arg Lys Val Thr Glu Glu
    290                 295                 300
Asn Lys Glu Leu Ala Asn Glu Leu Arg Arg Pro Pro Leu Cys Tyr His
305                 310                 315                 320
Asn Gly Val Gln Tyr Arg Asn Asn Glu Glu Trp Thr Val Asp Ser Cys
                325                 330                 335
Thr Glu Cys His Cys Gln Asn Ser Val Thr Ile Cys Lys Lys Val Ser
            340                 345                 350
Cys Pro Ile Met Pro Cys Ser Asn Ala Thr Val Pro Asp Gly Glu Cys
        355                 360                 365
Cys Pro Arg Cys Trp Pro Ser Asp Ser Ala Asp Asp Gly Trp Ser Pro
    370                 375                 380
Trp Ser Glu Trp Thr Ser Cys Ser Thr Ser Cys Gly Asn Gly Ile Gln
385                 390                 395                 400
Gln Arg Gly Arg Ser Cys Asp Ser Leu Asn Asn Arg Cys Glu Gly Ser
                405                 410                 415
Ser Val Gln Thr Arg Thr Cys His Ile Gln Glu Cys Asp Lys Arg Phe
            420                 425                 430
Lys Gln Asp Gly Gly Trp Ser His Trp Ser Pro Trp Ser Ser Cys Ser
        435                 440                 445

Val Thr Cys Gly Asp Gly Val Ile Thr Arg Ile Arg Leu Cys Asn Ser
    450                 455                 460
Pro Ser Pro Gln Met Asn Gly Lys Pro Cys Glu Gly Glu Ala Arg Glu
465                 470                 475                 480
Thr Lys Ala Cys Lys Lys Asp Ala Cys Pro Ile Asn Gly Gly Trp Gly
                485                 490                 495
Pro Trp Ser Pro Trp Asp Ile Cys Ser Val Thr Cys Gly Gly Gly Val
            500                 505                 510
Gln Lys Arg Ser Arg Leu Cys Asn Asn Pro Ala Pro Gln Phe Gly Gly
        515                 520                 525
Lys Asp Cys Val Gly Asp Val Thr Glu Asn Gln Ile Cys Asn Lys Gln
    530                 535                 540
Asp Cys Pro Ile Asp Gly Cys Leu Ser Asn Pro Cys Phe Ala Gly Val
545                 550                 555                 560
Lys Cys Thr Ser Tyr Pro Asp Gly Ser Trp Lys Cys Gly Ala Cys Pro
                565                 570                 575
Pro Gly Tyr Ser Gly Asn Gly Ile Gln Cys Thr Asp Val Asp Glu Cys
            580                 585                 590
Lys Glu Val Pro Asp Ala Cys Phe Asn His Asn Gly Glu His Arg Cys
        595                 600                 605
Glu Asn Thr Asp Pro Gly Tyr Asn Cys Leu Pro Cys Pro Pro Arg Phe
    610                 615                 620
Thr Gly Ser Gln Pro Phe Gly Gln Gly Val Glu His Ala Thr Ala Asn
625                 630                 635                 640
Lys Gln Val Cys Lys Pro Arg Asn Pro Cys Thr Asp Gly Thr His Asp
                645                 650                 655
Cys Asn Lys Asn Ala Lys Cys Asn Tyr Leu Gly His Tyr Ser Asp Pro

            660                 665                 670
Met Tyr Arg Cys Glu Cys Lys Pro Gly Tyr Ala Gly Asn Gly Ile Ile
        675                 680                 685
Cys Gly Glu Asp Thr Asp Leu Asp Gly Trp Pro Asn Glu Asn Leu Val
    690                 695                 700
Cys Val Ala Asn Ala Thr Tyr His Cys Lys Lys Asp Asn Cys Pro Asn
705                 710                 715                 720
Leu Pro Asn Ser Gly Gln Glu Asp Tyr Asp Lys Asp Gly Ile Gly Asp
                725                 730                 735
Ala Cys Asp Asp Asp Asp Asp Asn Asp Lys Ile Pro Asp Asp Arg Asp
            740                 745                 750
Asn Cys Pro Phe His Tyr Asn Pro Ala Gln Tyr Asp Tyr Asp Arg Asp
        755                 760                 765
Asp Val Gly Asp Arg Cys Asp Asn Cys Pro Tyr Asn His Asn Pro Asp
    770                 775                 780
Gln Ala Asp Thr Asp Asn Asn Gly Glu Gly Asp Ala Cys Ala Ala Asp
785                 790                 795                 800
Ile Asp Gly Asp Gly Ile Leu Asn Glu Arg Asp Asn Cys Gln Tyr Val
                805                 810                 815
Tyr Asn Val Asp Gln Arg Asp Thr Asp Met Asp Gly Val Gly Asp Gln
            820                 825                 830
Cys Asp Asn Cys Pro Leu Glu His Asn Pro Asp Gln Leu Asp Ser Asp
        835                 840                 845
Ser Asp Arg Ile Gly Asp Thr Cys Asp Asn Asn Gln Asp Ile Asp Glu
    850                 855                 860
Asp Gly His Gln Asn Asn Leu Asp Asn Cys Pro Tyr Val Pro Asn Ala

865                 870                 875                 880
Asn Gln Ala Asp His Asp Lys Asp Gly Lys Gly Asp Ala Cys Asp His
                885                 890                 895
Asp Asp Asp Asn Asp Gly Ile Pro Asp Asp Lys Asp Asn Cys Arg Leu
            900                 905                 910
Val Pro Asn Pro Asp Gln Lys Asp Ser Asp Gly Asp Gly Arg Gly Asp
        915                 920                 925
Ala Cys Lys Asp Asp Phe Asp His Asp Ser Val Pro Asp Ile Asp Asp
    930                 935                 940
Ile Cys Pro Glu Asn Val Asp Ile Ser Glu Thr Asp Phe Arg Arg Phe
945                 950                 955                 960
Gln Met Ile Pro Leu Asp Pro Lys Gly Thr Ser Gln Asn Asp Pro Asn
                965                 970                 975
Trp Val Val Arg His Gln Gly Lys Glu Leu Val Gln Thr Val Asn Cys
            980                 985                 990
Asp Pro Gly Leu Ala Val Gly Tyr Asp Glu Phe Asn Ala Val Asp Phe
        995                 1000                1005
Ser Gly Thr Phe Phe Ile Asn Thr Glu Arg Asp Asp Asp Tyr Ala Gly
    1010                1015                1020
Phe Val Phe Gly Tyr Gln Ser Ser Ser Arg Phe Tyr Val Val Met Trp
1025                1030                1035                1040
Lys Gln Val Thr Gln Ser Tyr Trp Asp Thr Asn Pro Thr Arg Ala Gln
                1045                1050                1055
Gly Tyr Ser Gly Leu Ser Val Lys Val Val Asn Ser Thr Thr Gly Pro
            1060                1065                1070
Gly Glu His Leu Arg Asn Ala Leu Trp His Thr Gly Asn Thr Pro Gly
        1075                1080                1085

Gln Val Arg Thr Leu Trp His Asp Pro Arg His Ile Gly Trp Lys Asp
    1090                1095                1100
Phe Thr Ala Tyr Arg Trp Arg Leu Ser His Arg Pro Lys Thr Gly Phe
1105                1110                1115                1120
Ile Arg Val Val Met Tyr Glu Gly Lys Lys Ile Met Ala Asp Ser Gly
                1125                1130                1135
Pro Ile Tyr Asp Lys Thr Tyr Ala Gly Gly Arg Leu Gly Leu Phe Val
            1140                1145                1150
Phe Ser Gln Glu Met Val Phe Phe Ser Asp Leu Lys Tyr Glu Cys Arg
        1155                1160                1165
Asp Pro
    1170
<210> 101
<211> 838
<212> DNA
<213> Homo sapiens
<220>
<221> CDS
<222> (35)..(568)
<400> 101
   gaattccgga gttttcatcc agccacgggc cagc atg tct ggg ggc aaa tac gta 55
   gac tcg gag gga cat ctc tac acc gtt ccc atc cgg gaa cag ggc aac 103
   atc tac aag ccc aac aac aag gcc atg gca gac gag ctg agc gag aag 151
   caa gtg tac gac gcg cac acc aag gag atc gac ctg gtc aac cgc gac 199
   cct aaa cac ctc aac gat gac gtg gtc aag att gac ttt gaa gat gtg 247
   att gca gaa cca gaa ggg aca cac agt ttt cac ggc att tgg aag gcc 295
   agc ttc acc acc ttc act gtg acg aaa tac tgg ttt tac cgc ttg ctg 343
   tct gcc ctc ttt ggc atc ccg atg gca ctc atc tgg ggc att tac ttc 391
   gcc att ctc tct ttc ctg cac atc tgg gca gtt gta cca tgc att aag 439
   agc ttc ctg att gag att cag tgc acc agc cgt gtc tat tcc atc tac 487
   gtc cac acc gtc tgt gac cca ctc ttt gaa gct gtt ggg aaa ata ttc 535
   agc aat gtc cgc atc aac ttg cag aaa gaa ata taaatgacat ttcaaggata 588
   gaagtatacc tgattttttt tccttttaat tttcctggtg ccaatttcaa gttccaagtt 648
   gctaatacag caacgaattt atgaattgaa ttatcttggt tgaaaataaa aagatcactt 708
   tctcagtttt cataagtatt atgtctcttc tgagctattt catctatttt tggcagtctg 768
   aatttttaaa acccatttat atttctttcc ttaccttttt atttgcatgt ggatcaacca 828
   tcgctttatt 838

<210> 102

<211> 178

<212> PRT

<213> Homo sapiens

<400> 102

<210> 103

<211> 2269

<212> DNA

<213> Homo sapiens

<220>

<221> CDS

<222> (1)..(444)

<400> 103

ccg ccc gcc acc agc tac gcc ccg tcc gac gtg ccc tcg ggg gtc gcg 48

Pro Pro Ala Thr Ser Tyr Ala Pro Ser Asp Val Pro Ser Gly Val Ala

1               5                   10                  15

ctg ttc ctc acc atc cct ttc gcc ttc ttc ctg ccc gag ctg ata ttt 96

Leu Phe Leu Thr Ile Pro Phe Ala Phe Phe Leu Pro Glu Leu Ile Phe

            20                  25                  30

ggg ttc ttg gtc tgg acc atg gta gcc gcc acc cac ata gta tac ccc 144

Gly Phe Leu Val Trp Thr Met Val Ala Ala Thr His Ile Val Tyr Pro
        35                  40                  45
ttg ctg caa gga tgg gtg atg tat gtc tcg ctc acc tcg ttt ctc atc 192
Leu Leu Gln Gly Trp Val Met Tyr Val Ser Leu Thr Ser Phe Leu Ile
    50                  55                  60
tcc ttg atg ttc ctg ttg tct tac ttg ttt gga ttt tac aaa aga ttt 240
Ser Leu Met Phe Leu Leu Ser Tyr Leu Phe Gly Phe Tyr Lys Arg Phe
65                  70                  75                  80
gaa tcc tgg aga gtt ctg gac agc ctg tac cac ggg acc act ggc atc 288
Glu Ser Trp Arg Val Leu Asp Ser Leu Tyr His Gly Thr Thr Gly Ile
                85                  90                  95
ctg tac atg agc gct gcc gtc cta caa gta cat gcc acg att gtt tct 336
Leu Tyr Met Ser Ala Ala Val Leu Gln Val His Ala Thr Ile Val Ser
            100                 105                 110
gag aaa ctg ctg gac cca aga att tac tac att aat tcg gca gcc tcg 384
Glu Lys Leu Leu Asp Pro Arg Ile Tyr Tyr Ile Asn Ser Ala Ala Ser
        115                 120                 125
ttc ttc gcc ttc atc gcc acg ctg ctc tac att ctc cat gcc ttc agc 432
Phe Phe Ala Phe Ile Ala Thr Leu Leu Tyr Ile Leu His Ala Phe Ser
    130                 135                 140
atc tat tac cac tgatgcacag gcgccaggcc aagggggaaa tgctctttga 484
Ile Tyr Tyr His
145
aagctccaat tattggtccc caaaagcagc ttccaacgtt tgccatctgg atgacaaacg 544
gaagatccac taaaacgtcc acgggattaa cagaacgtcc ttgcagactg agcgatgaca 604
ccacactttg tttggacatt taaattcact ctgctgaata ggaggaagct tttctttttc 664
ctgggaaaac aactgtctct tggaattatc tgaccatgaa cttgctcttc tagacaactc 724

acatcaaagc cctcactcca ctaatggaga atcctagccc cactaatgcc aagtctgttt 784
ggggattttg cctcagctat gggcttccct agagtaggtc taggggaata ctcagtctga 844
tctttttttt gtttgtttta ttttgttttt tttgagacgg agtctcgctc ttcctccaag 904
gctggagtgc agtgacgcga tctccactca ctgcaggctc cgcctcccgg gttcccgcca 964
ttctcctgcc tcagcctccc gagtagccgg gactacaggc gcccaccacc atgcccggct 1024
aatttagttg tatttttagt agagatgggg tttcaccgta ttagccagga tggtctcgat 1084
ctcctgacct cgtgatccgc ccgcctcggc ctcccaaagt gctgggatta caggcgtgag 1144
ccaccgtgcc cggcctgatt ctcttaaaat tgaagaggtg ctgccaaggc cttcagatct 1204
aacgcagatg catagacctt gttcctggta cttgttcagc ctgtgctggg gagccgtggt 1264
cccgagttcc ctgggaggct gacagggtca agccaccctg cccaccaccc tcccacttcc 1324
cctccccttt cctctccagc attaggattc aagggaaatc tgcatgaagc caattttgag 1384
ggtagacgtg tggggaaaat aaatcattat acagtaagac ctggggcttg aggggtgggg 1444
aatggggagg gaagggcata gcctgctcct ccatgagtct gacatctcgg aaactgagca 1504
gctgccggac gcctgggtca ggaatccaag accccacctc ttaaggactg gttcctcaga 1564
aagcaccctc agggaaaaag gtgaaaacat tacatccgtg gattctcctg ccacaaccgc 1624
attggaagaa aaggctgccg caacatctca gcgaggagtg aaggacccat gtcccaggaa 1684
ccgcgctgcg ccacctgcac tcacccccct cacattctct taagcacccg gtggccctcc 1744
gaggctggcg gaatggtggt gcccacgggg ttgggcaagg gctcaccagg acctcaacgg 1804
gcaaagttgt gcacactaaa atatcaaatc aaggtgcttg gttttaaagt aaatgttttt 1864
ctaaagaaag ctgtgttctt ctgttgaccc agacgaatag ggcacagccc tgtaactgca 1924
cgtgccttct gtcattggga atgaaataaa ttattacgag aaaggacttt gtcctaactg 1984
gtttgaggcc ttacagtttt gtatctacat ttttcccctc ctggggtttg cggggacagg 2044
gacagaacta caggagtcat gggaaagaaa attctggctt cactactgct cactgctcac 2104
tttctgatca ctctgatact tttttttttt tttttttttt gcaacctgat accttgaaaa 2164
gcttctatgt gtctctcctt ttgttgcctg gcagctgtct aggatgatca ctgattacta 2224
tttactaagt agccacatgc aaataaaagt tgtttggtaa aatgg 2269

<210> 104

<211> 148

<212> PRT

<213> Homo sapiens

<400> 104

Pro Pro Ala Thr Ser Tyr Ala Pro Ser Asp Val Pro Ser Gly Val Ala

1               5                   10                  15

Leu Phe Leu Thr Ile Pro Phe Ala Phe Phe Leu Pro Glu Leu Ile Phe

            20                  25                  30

Gly Phe Leu Val Trp Thr Met Val Ala Ala Thr His Ile Val Tyr Pro

        35                  40                  45

Leu Leu Gln Gly Trp Val Met Tyr Val Ser Leu Thr Ser Phe Leu Ile

    50                  55                  60

Ser Leu Met Phe Leu Leu Ser Tyr Leu Phe Gly Phe Tyr Lys Arg Phe

65                  70                  75                  80

Glu Ser Trp Arg Val Leu Asp Ser Leu Tyr His Gly Thr Thr Gly Ile

                85                  90                  95

Leu Tyr Met Ser Ala Ala Val Leu Gln Val His Ala Thr Ile Val Ser

            100                 105                 110

Glu Lys Leu Leu Asp Pro Arg Ile Tyr Tyr Ile Asn Ser Ala Ala Ser

        115                 120                 125

Phe Phe Ala Phe Ile Ala Thr Leu Leu Tyr Ile Leu His Ala Phe Ser

    130                 135                 140

Ile Tyr Tyr His

145

<210> 105

<211> 2899

<212> DNA

<213> Homo sapiens

<220>

<221> CDS

<222> (91)..(2196)

<400> 105

gatctgaatt cggtcccagc tagagctcca gcgcccgctc aggccccact cgaccctctc 60

gggcctcggc tacttggact gcggcggaat atg gcg gct ccg atg act ccc gcg 114

Met Ala Ala Pro Met Thr Pro Ala

1               5

gct cgg ccc gag gac tac gag gcg gcg ctc aat gcc gcc ctg gct gac 162

Ala Arg Pro Glu Asp Tyr Glu Ala Ala Leu Asn Ala Ala Leu Ala Asp

    10                  15                  20

gtg ccc gaa ctg gcc aga ctc ctg gag atc gac ccg tac ttg aag ccc 210

Val Pro Glu Leu Ala Arg Leu Leu Glu Ile Asp Pro Tyr Leu Lys Pro

25                  30                  35                  40

tac gcc gtg gac ttc cag cgc agg tat aag cag ttt agc caa att ttg 258

Tyr Ala Val Asp Phe Gln Arg Arg Tyr Lys Gln Phe Ser Gln Ile Leu

                45                  50                  55

aag aac att gga gaa aat gaa ggt ggt att gat aag ttt tcc aga ggc 306

Lys Asn Ile Gly Glu Asn Glu Gly Gly Ile Asp Lys Phe Ser Arg Gly

            60                  65                  70

tat gaa tca ttt ggc gtc cac aga tgt gct gat ggt ggt tta tac tcc 354

Tyr Glu Ser Phe Gly Val His Arg Cys Ala Asp Gly Gly Leu Tyr Ser

        75                  80                  85

aaa gaa tgg gcc ccg gga gca gaa gga gtt ttt ctt act gga gat ttt 402
Lys Glu Trp Ala Pro Gly Ala Glu Gly Val Phe Leu Thr Gly Asp Phe
    90                  95                  100
aat ggt tgg aat cca ttt tcg tac cca tac aaa aaa ctg gat tat gga 450
Asn Gly Trp Asn Pro Phe Ser Tyr Pro Tyr Lys Lys Leu Asp Tyr Gly
105                 110                 115                 120
aaa tgg gag ctg tat atc cca cca aag cag aat aaa tct gta ctc gtg 498
Lys Trp Glu Leu Tyr Ile Pro Pro Lys Gln Asn Lys Ser Val Leu Val
                125                 130                 135
cct cat gga tcc aaa tta aag gta gtt att act agt aaa agc gga gag 546
Pro His Gly Ser Lys Leu Lys Val Val Ile Thr Ser Lys Ser Gly Glu
            140                 145                 150
atc ttg tat cgt att tca ccg tgg gca aag tat gtg gtt cgt gaa ggt 594
Ile Leu Tyr Arg Ile Ser Pro Trp Ala Lys Tyr Val Val Arg Glu Gly
        155                 160                 165
gat aat gtg aat tat gat tgg ata cac tgg gat cca gaa cac tca tat 642
Asp Asn Val Asn Tyr Asp Trp Ile His Trp Asp Pro Glu His Ser Tyr
    170                 175                 180
gag ttt aag cat tcc aga cca aag aag cca cgg agt cta aga att tat 690
Glu Phe Lys His Ser Arg Pro Lys Lys Pro Arg Ser Leu Arg Ile Tyr
185                 190                 195                 200
gaa tct cat gtg gga att tct tcc cat gaa gga aaa gta gct tct tat 738
Glu Ser His Val Gly Ile Ser Ser His Glu Gly Lys Val Ala Ser Tyr
                205                 210                 215
aaa cat ttt aca tgc aat gta cta cca aga atc aaa ggc ctt gga tac 786
Lys His Phe Thr Cys Asn Val Leu Pro Arg Ile Lys Gly Leu Gly Tyr
            220                 225                 230

aac tgc att cag ttg atg gca atc atg gag cat gct tac tat gcc agc 834

Asn Cys Ile Gln Leu Met Ala Ile Met Glu His Ala Tyr Tyr Ala Ser

        235                 240                 245

ttt ggt tac caa atc aca agc ttc ttt gca gct tcc agc cgt tat gga 882

Phe Gly Tyr Gln Ile Thr Ser Phe Phe Ala Ala Ser Ser Arg Tyr Gly

    250                 255                 260

aca cct gaa gag cta caa gaa ctg gta gac aca gct cat tcc atg ggt 930

Thr Pro Glu Glu Leu Gln Glu Leu Val Asp Thr Ala His Ser Met Gly

265                 270                 275                 280

atc ata gtc ctc tta gat gtg gta cac agc cat gct tca aaa aat tca 978

Ile Ile Val Leu Leu Asp Val Val His Ser His Ala Ser Lys Asn Ser

                285                 290                 295

gca gat gga ttg aat atg ttt gat ggg aca gat tcc tgt tat ttt cat 1026

Ala Asp Gly Leu Asn Met Phe Asp Gly Thr Asp Ser Cys Tyr Phe His

            300                 305                 310

tct gga cct aga ggg act cat gat ctt tgg gat agc aga ttg ttt gcc 1074

Ser Gly Pro Arg Gly Thr His Asp Leu Trp Asp Ser Arg Leu Phe Ala

        315                 320                 325

tac tcc agc tgg gaa gtt tta aga ttc ctt ctg tca aac ata aga tgg 1122

Tyr Ser Ser Trp Glu Val Leu Arg Phe Leu Leu Ser Asn Ile Arg Trp

    330                 335                 340

tgg ttg gaa gaa tat cgc ttt gat gga ttt cgt ttt gat ggt gtt acg 1170

Trp Leu Glu Glu Tyr Arg Phe Asp Gly Phe Arg Phe Asp Gly Val Thr

345                 350                 355                 360

tcc atg ctt tat cat cac cat gga gtg ggt caa ggt ttc tca ggt gat 1218

Ser Met Leu Tyr His His His Gly Val Gly Gln Gly Phe Ser Gly Asp

                365                 370                 375

tac agt gaa tat ttc gga cta caa gta gat gaa gat gcc ttg act tac 1266

Tyr Ser Glu Tyr Phe Gly Leu Gln Val Asp Glu Asp Ala Leu Thr Tyr

            380                 385                 390

ctc atg ttg gca aat cat ttg gtt cac acg ctg tgt ccc gat tct ata 1314

Leu Met Leu Ala Asn His Leu Val His Thr Leu Cys Pro Asp Ser Ile

        395                 400                 405

aca ata gct gag gat gta tca gga atg cca gct ctg tgc tct cca att 1362

Thr Ile Ala Glu Asp Val Ser Gly Met Pro Ala Leu Cys Ser Pro Ile

    410                 415                 420

tcc cag gga ggg ggt ggt ttt gac tat cga cta gcc atg gca att cca 1410

Ser Gln Gly Gly Gly Gly Phe Asp Tyr Arg Leu Ala Met Ala Ile Pro

425                 430                 435                 440

gat aag tgg att cag cta ctt aaa gag ttt aaa gat gaa gac tgg aac 1458

Asp Lys Trp Ile Gln Leu Leu Lys Glu Phe Lys Asp Glu Asp Trp Asn

                445                 450                 455

atg ggc gat ata gta tac acg ctc aca aac agg cgc tac ctt gaa aag 1506

Met Gly Asp Ile Val Tyr Thr Leu Thr Asn Arg Arg Tyr Leu Glu Lys

            460                 465                 470

tgc att gct tat gca gag agc cat gat cag gca ttg gtt ggg gat aag 1554

Cys Ile Ala Tyr Ala Glu Ser His Asp Gln Ala Leu Val Gly Asp Lys

        475                 480                 485

tcg ctg gca ttt tgg ttg atg gat gcc gaa atg tat aca aac atg agt 1602

Ser Leu Ala Phe Trp Leu Met Asp Ala Glu Met Tyr Thr Asn Met Ser

    490                 495                 500

gtc ctg act cct ttt act cca gtt att gat cgt gga ata cag ctt cat 1650

Val Leu Thr Pro Phe Thr Pro Val Ile Asp Arg Gly Ile Gln Leu His

505                 510                 515                 520

aaa atg att cga ctc att acg cat ggg ctt ggt gga gaa ggc tat ctc 1698

Lys Met Ile Arg Leu Ile Thr His Gly Leu Gly Gly Glu Gly Tyr Leu

                525                 530                 535

aat ttc atg ggt aat gaa ttt ggg cat cct gaa tgg tta gac ttc cca 1746

Asn Phe Met Gly Asn Glu Phe Gly His Pro Glu Trp Leu Asp Phe Pro

            540                 545                 550

aga aaa gga aat aat gag agt tac cat tat gcc agg cgg cag ttt cat 1794

Arg Lys Gly Asn Asn Glu Ser Tyr His Tyr Ala Arg Arg Gln Phe His

        555                 560                 565

tta act gac gac gac ctt ctt cgc tac aag ttc cta aat aat ttt gac 1842

Leu Thr Asp Asp Asp Leu Leu Arg Tyr Lys Phe Leu Asn Asn Phe Asp

    570                 575                 580

agg gat atg aat aga ttg gaa gaa aga tat ggt tgg ctt gca gct cca 1890

Arg Asp Met Asn Arg Leu Glu Glu Arg Tyr Gly Trp Leu Ala Ala Pro

585                 590                 595                 600

cag gcc tac gtg agt gaa aaa cat gaa ggc aat aag atc att gct ttt 1938

Gln Ala Tyr Val Ser Glu Lys His Glu Gly Asn Lys Ile Ile Ala Phe

                605                 610                 615

gaa aga gca ggt ctt ctt ttc att ttc aac ttc cat cca agc aag agc 1986

Glu Arg Ala Gly Leu Leu Phe Ile Phe Asn Phe His Pro Ser Lys Ser

            620                 625                 630

tac act gac tac cga gtt gga aca gca ttg cca ggg aaa ttc aaa att 2034

Tyr Thr Asp Tyr Arg Val Gly Thr Ala Leu Pro Gly Lys Phe Lys Ile

        635                 640                 645

gtg cta gat tca gat gca gcg gaa tat gga ggg cat cag aga ctg gac 2082

Val Leu Asp Ser Asp Ala Ala Glu Tyr Gly Gly His Gln Arg Leu Asp
    650                 655                 660
cac agc act gac ttt ttt tct gag gct ttt gaa cat aat ggg cgt ccc 2130
His Ser Thr Asp Phe Phe Ser Glu Ala Phe Glu His Asn Gly Arg Pro
665                 670                 675                 680
tat tct ctt ttg gtg tac att cca agc aga gtg gcc ctc atc ctt cag 2178
Tyr Ser Leu Leu Val Tyr Ile Pro Ser Arg Val Ala Leu Ile Leu Gln
                685                 690                 695
aat gtg gat ctg ccg aat tgaagaggcc tgatttcagc tccaccagat 2226
Asn Val Asp Leu Pro Asn
            700
gcagatttgt gttttgtttt cttgttatca ctgtcacaca gcttataaca tgtatgcttt 2286
tcagaataca gttgtctagc caagccatca agtgtctgaa attcaatatt ggtttatgca 2346
aatacagcaa acttttattt aagtagatag gagaatatgt ttaaaatatt aggaatccta 2406
gaccatattt tcaagtcatc ttagcagcta ggattctcaa atggaagtgt tatatataat 2466
atgttaaaaa cattttgctt tcctggctaa ttatttgatc cttttaaatc caaatttgaa 2526
tcatttgtca tgtatgatta tttctgttaa atgtacacag tatttaagat ggatatttgg 2586
tggctctatt tgttctgata tcttttggtc taaattatga ggtaccaaga ttgtttcttt 2646
gtttcttttt ttcaaattgt gtttagaaat actgtaataa atatgcagta gtgatataaa 2706
gaattatatc caaggtaata taaaagccat tacgtatgaa ctcatccgtg tctcattttg 2766
tgttttattt tgtgatctct tgtccactaa gtatcttgtt aaatgccagt atctcagtct 2826
ttctgaagcc ctgaaatggt aattgtagca tttcagaaaa tgtctttcat ttcaatcaat 2886
aaaaagcttt tgt 2899

<210> 106
<211> 702
<212> PRT

<213> Homo sapiens

<400> 106

Met Ala Ala Pro Met Thr Pro Ala Ala Arg Pro Glu Asp Tyr Glu Ala

1               5                   10                  15

Ala Leu Asn Ala Ala Leu Ala Asp Val Pro Glu Leu Ala Arg Leu Leu

            20                  25                  30

Glu Ile Asp Pro Tyr Leu Lys Pro Tyr Ala Val Asp Phe Gln Arg Arg

        35                  40                  45

Tyr Lys Gln Phe Ser Gln Ile Leu Lys Asn Ile Gly Glu Asn Glu Gly

    50                  55                  60

Gly Ile Asp Lys Phe Ser Arg Gly Tyr Glu Ser Phe Gly Val His Arg

65                  70                  75                  80

Cys Ala Asp Gly Gly Leu Tyr Ser Lys Glu Trp Ala Pro Gly Ala Glu

                85                  90                  95

Gly Val Phe Leu Thr Gly Asp Phe Asn Gly Trp Asn Pro Phe Ser Tyr

            100                 105                 110

Pro Tyr Lys Lys Leu Asp Tyr Gly Lys Trp Glu Leu Tyr Ile Pro Pro

        115                 120                 125

Lys Gln Asn Lys Ser Val Leu Val Pro His Gly Ser Lys Leu Lys Val

    130                 135                 140

Val Ile Thr Ser Lys Ser Gly Glu Ile Leu Tyr Arg Ile Ser Pro Trp

145                 150                 155                 160

Ala Lys Tyr Val Val Arg Glu Gly Asp Asn Val Asn Tyr Asp Trp Ile

                165                 170                 175

His Trp Asp Pro Glu His Ser Tyr Glu Phe Lys His Ser Arg Pro Lys

            180                 185                 190

Lys Pro Arg Ser Leu Arg Ile Tyr Glu Ser His Val Gly Ile Ser Ser

        195                 200                 205
His Glu Gly Lys Val Ala Ser Tyr Lys His Phe Thr Cys Asn Val Leu
    210                 215                 220
Pro Arg Ile Lys Gly Leu Gly Tyr Asn Cys Ile Gln Leu Met Ala Ile
225                 230                 235                 240
Met Glu His Ala Tyr Tyr Ala Ser Phe Gly Tyr Gln Ile Thr Ser Phe
                245                 250                 255
Phe Ala Ala Ser Ser Arg Tyr Gly Thr Pro Glu Glu Leu Gln Glu Leu
            260                 265                 270
Val Asp Thr Ala His Ser Met Gly Ile Ile Val Leu Leu Asp Val Val
        275                 280                 285
His Ser His Ala Ser Lys Asn Ser Ala Asp Gly Leu Asn Met Phe Asp
    290                 295                 300
Gly Thr Asp Ser Cys Tyr Phe His Ser Gly Pro Arg Gly Thr His Asp
305                 310                 315                 320
Leu Trp Asp Ser Arg Leu Phe Ala Tyr Ser Ser Trp Glu Val Leu Arg
                325                 330                 335
Phe Leu Leu Ser Asn Ile Arg Trp Trp Leu Glu Glu Tyr Arg Phe Asp
            340                 345                 350
Gly Phe Arg Phe Asp Gly Val Thr Ser Met Leu Tyr His His His Gly
        355                 360                 365
Val Gly Gln Gly Phe Ser Gly Asp Tyr Ser Glu Tyr Phe Gly Leu Gln
    370                 375                 380
Val Asp Glu Asp Ala Leu Thr Tyr Leu Met Leu Ala Asn His Leu Val
385                 390                 395                 400
His Thr Leu Cys Pro Asp Ser Ile Thr Ile Ala Glu Asp Val Ser Gly

                405                 410                 415
Met Pro Ala Leu Cys Ser Pro Ile Ser Gln Gly Gly Gly Gly Phe Asp
            420                 425                 430
Tyr Arg Leu Ala Met Ala Ile Pro Asp Lys Trp Ile Gln Leu Leu Lys
        435                 440                 445
Glu Phe Lys Asp Glu Asp Trp Asn Met Gly Asp Ile Val Tyr Thr Leu
    450                 455                 460
Thr Asn Arg Arg Tyr Leu Glu Lys Cys Ile Ala Tyr Ala Glu Ser His
465                 470                 475                 480
Asp Gln Ala Leu Val Gly Asp Lys Ser Leu Ala Phe Trp Leu Met Asp
                485                 490                 495
Ala Glu Met Tyr Thr Asn Met Ser Val Leu Thr Pro Phe Thr Pro Val
            500                 505                 510
Ile Asp Arg Gly Ile Gln Leu His Lys Met Ile Arg Leu Ile Thr His
        515                 520                 525
Gly Leu Gly Gly Glu Gly Tyr Leu Asn Phe Met Gly Asn Glu Phe Gly
    530                 535                 540
His Pro Glu Trp Leu Asp Phe Pro Arg Lys Gly Asn Asn Glu Ser Tyr
545                 550                 555                 560
His Tyr Ala Arg Arg Gln Phe His Leu Thr Asp Asp Asp Leu Leu Arg
                565                 570                 575
Tyr Lys Phe Leu Asn Asn Phe Asp Arg Asp Met Asn Arg Leu Glu Glu
            580                 585                 590
Arg Tyr Gly Trp Leu Ala Ala Pro Gln Ala Tyr Val Ser Glu Lys His
        595                 600                 605
Glu Gly Asn Lys Ile Ile Ala Phe Glu Arg Ala Gly Leu Leu Phe Ile
    610                 615                 620

Phe Asn Phe His Pro Ser Lys Ser Tyr Thr Asp Tyr Arg Val Gly Thr
625                 630                 635                 640
Ala Leu Pro Gly Lys Phe Lys Ile Val Leu Asp Ser Asp Ala Ala Glu
                645                 650                 655
Tyr Gly Gly His Gln Arg Leu Asp His Ser Thr Asp Phe Phe Ser Glu
            660                 665                 670
Ala Phe Glu His Asn Gly Arg Pro Tyr Ser Leu Leu Val Tyr Ile Pro
        675                 680                 685
Ser Arg Val Ala Leu Ile Leu Gln Asn Val Asp Leu Pro Asn
    690                 695                 700
<210> 107
<211> 790
<212> DNA
<213> Homo sapiens
<220>
<221> CDS
<222> (78)..(626)
<400> 107
actgccccaa ggcccccgcc gccgctccag cgccgcgcag ccaccgccgc cgccgccgcc 60
tctccttagt cgccgcc atg acg acc gcg tcc acc tcg cag gtg cgc cag   110
                   Met Thr Thr Ala Ser Thr Ser Gln Val Arg Gln
                   1               5                   10
aac tac cac cag gac tca gag gcc gcc atc aac cgc cag atc aac ctg   158
Asn Tyr His Gln Asp Ser Glu Ala Ala Ile Asn Arg Gln Ile Asn Leu
            15                  20                  25
gag ctc tac gcc tcc tac gtt tac ctg tcc atg tct tac tac ttt gac   206

Glu Leu Tyr Ala Ser Tyr Val Tyr Leu Ser Met Ser Tyr Tyr Phe Asp
        30                  35                  40
cgc gat gat gtg gct ttg aag aac ttt gcc aaa tac ttt ctt cac caa 254
Arg Asp Asp Val Ala Leu Lys Asn Phe Ala Lys Tyr Phe Leu His Gln
    45                  50                  55
tct cat gag gag agg gaa cat gct gag aaa ctg atg aag ctg cag aac 302
Ser His Glu Glu Arg Glu His Ala Glu Lys Leu Met Lys Leu Gln Asn
60                  65                  70                  75
caa cga ggt ggc cga atc ttc ctt cag gat atc aag aaa cca gac tgt 350
Gln Arg Gly Gly Arg Ile Phe Leu Gln Asp Ile Lys Lys Pro Asp Cys
                80                  85                  90
gat gac tgg gag agc ggg ctg aat gca atg gag tgt gca tta cat ttg 398
Asp Asp Trp Glu Ser Gly Leu Asn Ala Met Glu Cys Ala Leu His Leu
            95                  100                 105
gaa aaa aat gtg aat cag tca cta ctg gaa ctg cac aaa ctg gcc act 446
Glu Lys Asn Val Asn Gln Ser Leu Leu Glu Leu His Lys Leu Ala Thr
        110                 115                 120
gac aaa aat gac ccc cat ttg tgt gac ttc att gag aca cat tac ctg 494
Asp Lys Asn Asp Pro His Leu Cys Asp Phe Ile Glu Thr His Tyr Leu
    125                 130                 135
aat gag cag gtg aaa gcc atc aaa gaa ttg ggt gac cac gtg acc aac 542
Asn Glu Gln Val Lys Ala Ile Lys Glu Leu Gly Asp His Val Thr Asn
140                 145                 150                 155
ttg cgc aag atg gga gcg ccc gaa tct ggc ttg gcg gaa tat ctc ttt 590
Leu Arg Lys Met Gly Ala Pro Glu Ser Gly Leu Ala Glu Tyr Leu Phe
                160                 165                 170

gac aag cac acc ctg gga gac agt gat aat gaa agc taagcctcgg 636
Asp Lys His Thr Leu Gly Asp Ser Asp Asn Glu Ser
            175                 180
gctaatttcc ccatagccgt ggggtgactt ccctggtcac caaggcagtg catgcatgtt 696
ggggtttcct ttaccttttc tataagttgt accaaaacat ccacttaagt tctttgattt 756
gtaccattcc ttcaaataaa gaaatttggt accc 790
<210> 108
<211> 183
<212> PRT
<213> Homo sapiens
<400> 108
Met Thr Thr Ala Ser Thr Ser Gln Val Arg Gln Asn Tyr His Gln Asp
1               5                   10                  15
Ser Glu Ala Ala Ile Asn Arg Gln Ile Asn Leu Glu Leu Tyr Ala Ser
            20                  25                  30
Tyr Val Tyr Leu Ser Met Ser Tyr Tyr Phe Asp Arg Asp Asp Val Ala
        35                  40                  45
Leu Lys Asn Phe Ala Lys Tyr Phe Leu His Gln Ser His Glu Glu Arg
    50                  55                  60
Glu His Ala Glu Lys Leu Met Lys Leu Gln Asn Gln Arg Gly Gly Arg
65                  70                  75                  80
Ile Phe Leu Gln Asp Ile Lys Lys Pro Asp Cys Asp Asp Trp Glu Ser
                85                  90                  95
Gly Leu Asn Ala Met Glu Cys Ala Leu His Leu Glu Lys Asn Val Asn
            100                 105                 110
Gln Ser Leu Leu Glu Leu His Lys Leu Ala Thr Asp Lys Asn Asp Pro

115                 120                 125

His Leu Cys Asp Phe Ile Glu Thr His Tyr Leu Asn Glu Gln Val Lys

    130                 135                 140

Ala Ile Lys Glu Leu Gly Asp His Val Thr Asn Leu Arg Lys Met Gly

145                 150                 155                 160

Ala Pro Glu Ser Gly Leu Ala Glu Tyr Leu Phe Asp Lys His Thr Leu

                165                 170                 175

Gly Asp Ser Asp Asn Glu Ser

            180

<210> 109

<211> 3460

<212> DNA

<213> Homo sapiens

<220>

<221> CDS

<222> (256)..(1857)

<400> 109

ccctaccgcc cccaattccg ccctgccccc gccgcggcgg cgctagccgc cactgaggga 60

ccgaccctat aaaggccgct ccgcgagggg tgcgcagcat tcggcagagg gcgcttcgac 120

gggctgggct gtgcgcctgc gcagtgtggg tcgctcccga ttccctgccc cggccggccc 180

cgcctcggct ccgcaccctc gccccgctct cagccgccgc tctgccccgc agcagccagc 240

cccgtgtccg gcagt atg ttc agc tgg gtc agc aag gat gcc cgc cgc aag 291

                 Met Phe Ser Trp Val Ser Lys Asp Ala Arg Arg Lys

                 1               5                   10

aag gag ccg gag ctc ttc cag acg gtg gcc gag ggg ctg cgg cag ctg 339

Lys Glu Pro Glu Leu Phe Gln Thr Val Ala Glu Gly Leu Arg Gln Leu

        15                  20                  25
tac gcg cag aag ctg cta ccc ctg gag gag cac tac cgc ttc cac gag 387
Tyr Ala Gln Lys Leu Leu Pro Leu Glu Glu His Tyr Arg Phe His Glu
    30                  35                  40
ttc cac tcg ccc gcr ctg gag gac gct gac ttc gac aac aag cct atg 435
Phe His Ser Pro Xaa Leu Glu Asp Ala Asp Phe Asp Asn Lys Pro Met
45                  50                  55                  60
gtg ctc ctc gtg rgg cag tac agc acg ggc aag acc acc ttc atc cga 483
Val Leu Leu Val Xaa Gln Tyr Ser Thr Gly Lys Thr Thr Phe Ile Arg
                65                  70                  75
cac ctg atc gag cag gac ttc ccg ggg atg cgc atc ggg ccc gag ccc 531
His Leu Ile Glu Gln Asp Phe Pro Gly Met Arg Ile Gly Pro Glu Pro
            80                  85                  90
acc acc gac tcc ttc atc gcc gtc atg cac ggc ccc act gag ggc gtg 579
Thr Thr Asp Ser Phe Ile Ala Val Met His Gly Pro Thr Glu Gly Val
        95                  100                 105
gtg ccg ggc aac gcg ctc gtg gtg gac ccg cgg cgc ccc ttc cgc aag 627
Val Pro Gly Asn Ala Leu Val Val Asp Pro Arg Arg Pro Phe Arg Lys
    110                 115                 120
ctc aac gcg ttt ggc aac gct ttc ctc aac agg ttc atg tgt gcc cag 675
Leu Asn Ala Phe Gly Asn Ala Phe Leu Asn Arg Phe Met Cys Ala Gln
125                 130                 135                 140
ctg ccc aac ccc gtc ctg gac agc atc agc atc atc gac acc ccc ggg 723
Leu Pro Asn Pro Val Leu Asp Ser Ile Ser Ile Ile Asp Thr Pro Gly
                145                 150                 155
atc ctg tct gga gag aag cag cgg atc agc aga ggc tat gac ttt gca 771
Ile Leu Ser Gly Glu Lys Gln Arg Ile Ser Arg Gly Tyr Asp Phe Ala

            160                 165                 170

gcc gtc ctg gag tgg ttc gcg gag cgt gtg gac cgc atc atc ctg ctc 819

Ala Val Leu Glu Trp Phe Ala Glu Arg Val Asp Arg Ile Ile Leu Leu

        175                 180                 185

ttc gac gcc cac aag ctg gac atc tcc gat gag ttc tcg gaa gtg atc 867

Phe Asp Ala His Lys Leu Asp Ile Ser Asp Glu Phe Ser Glu Val Ile

    190                 195                 200

aag gct ctg aag aac cat gag gac aag atc cgc gtg gtg ctg aac aag 915

Lys Ala Leu Lys Asn His Glu Asp Lys Ile Arg Val Val Leu Asn Lys

205                 210                 215                 220

gca gac cag atc gag acg cag cag ctg atg cgg gtg tac ggg gcc ctc 963

Ala Asp Gln Ile Glu Thr Gln Gln Leu Met Arg Val Tyr Gly Ala Leu

                225                 230                 235

atg tgg tcc ctg ggc aag atc atc aac acc ccc gag gtg gtc agg gtc 1011

Met Trp Ser Leu Gly Lys Ile Ile Asn Thr Pro Glu Val Val Arg Val

            240                 245                 250

tac atc ggc tcc ttc tgg tcc cac ccg ctc ctc atc ccc gac aac cgc 1059

Tyr Ile Gly Ser Phe Trp Ser His Pro Leu Leu Ile Pro Asp Asn Arg

        255                 260                 265

aag ctc ttt gag gcc gag gag cag gac ctc ttc aag gac atc cag tca 1107

Lys Leu Phe Glu Ala Glu Glu Gln Asp Leu Phe Lys Asp Ile Gln Ser

    270                 275                 280

ctg ccc cga aac gcc gcc ctc agg aag ctc aat gac ctg atc aag cgg 1155

Leu Pro Arg Asn Ala Ala Leu Arg Lys Leu Asn Asp Leu Ile Lys Arg

285                 290                 295                 300

gca cgg ctg gcc aag gtt cac gcc tac atc atc agc tcc ctc aag aaa 1203

Ala Arg Leu Ala Lys Val His Ala Tyr Ile Ile Ser Ser Leu Lys Lys
                305                 310                 315
gag atg ccc aat gtc ttt ggt aaa gag agc aaa aag aaa gag ctg gtg 1251
Glu Met Pro Asn Val Phe Gly Lys Glu Ser Lys Lys Lys Glu Leu Val
            320                 325                 330
aac aac ctg gga gag atc tac cag aag att gag cgc gag cac cag atc 1299
Asn Asn Leu Gly Glu Ile Tyr Gln Lys Ile Glu Arg Glu His Gln Ile
        335                 340                 345
tcc cct ggg gac ttc ccg agc ctc cgc aag atg cag gaa ctc ctg cag 1347
Ser Pro Gly Asp Phe Pro Ser Leu Arg Lys Met Gln Glu Leu Leu Gln
    350                 355                 360
acc cag gac ttc agc aag ttc cag gcg ctg aag ccc aag ctg ctg gac 1395
Thr Gln Asp Phe Ser Lys Phe Gln Ala Leu Lys Pro Lys Leu Leu Asp
365                 370                 375                 380
acg gtg gat gac atg ctg gcc aac gac atc gcg cgg ctg atg gtg atg 1443
Thr Val Asp Asp Met Leu Ala Asn Asp Ile Ala Arg Leu Met Val Met
                385                 390                 395
gtg cgg cag gag gag tcc ctg atg cct tcc cag gtg gtc aag ggc ggc 1491
Val Arg Gln Glu Glu Ser Leu Met Pro Ser Gln Val Val Lys Gly Gly
            400                 405                 410
gcc ttt gac ggc acc atg aac ggg ccg ttc ggg cac ggc tac ggc gag 1539
Ala Phe Asp Gly Thr Met Asn Gly Pro Phe Gly His Gly Tyr Gly Glu
        415                 420                 425
ggg gcc ggc gag ggc atc cac gac gtg gag tgg gtg gtg ggc aag gac 1587
Gly Ala Gly Glu Gly Ile His Asp Val Glu Trp Val Val Gly Lys Asp
    430                 435                 440
aag ccc acc tac gac gag atc ttc tac acg ctg tcc cct gtc aac ggc 1635

Lys Pro Thr Tyr Asp Glu Ile Phe Tyr Thr Leu Ser Pro Val Asn Gly
445                 450                 455                 460
aag atc acg ggc gcc aac gcc aag aag gag atg gtg aag tcc aag ctc 1683
Lys Ile Thr Gly Ala Asn Ala Lys Lys Glu Met Val Lys Ser Lys Leu
                465                 470                 475
ccc aac acc gtg cta ggg aag atc tgg aag ctg gcc gac gtg gac aag 1731
Pro Asn Thr Val Leu Gly Lys Ile Trp Lys Leu Ala Asp Val Asp Lys
            480                 485                 490
gac ggg ctg ctg gac gac gag gag ttc gcg ctg gcc aac cac ctc atc 1779
Asp Gly Leu Leu Asp Asp Glu Glu Phe Ala Leu Ala Asn His Leu Ile
        495                 500                 505
aag gtc aag ctg gag ggc cac gag ctg ccc gcc gac ctg ccc ccg cac 1827
Lys Val Lys Leu Glu Gly His Glu Leu Pro Ala Asp Leu Pro Pro His
    510                 515                 520
ctg gtg ccg ccc tcc aag cgc aga cat gag tgatggcgcc cggccccgca 1877
Leu Val Pro Pro Ser Lys Arg Arg His Glu
525                 530
cctgccattt gcacgcccgg ccgggaggca gagacggggg gaggggaagc ctcaccattt 1937
ctcaaggtcc ataaagactg agcggatgtt tcctcgcctc tcgaaaagga aaaccaccat 1997
ctttctttta aggctgttcc tgggcctggc gggggaggca ggggtgagag gatggaattg 2057
tgtgcacaag aactgtggct attttaatat ataacgttag aggctgcgtt ctttgtcgcc 2117
gcctcccctg tgtgccagcc ctgtgtgcac ggcctctgcc ccccggcctt gtgctgtggc 2177
tggagctgga cagtgcagtg actgcgaccg tgggggagcc aggtcgccct tttggcagct 2237
gctaggctga ggctgcatgg acaggaacac caggcaccct ccgtgtgctt ctgagctgag 2297
gttgcttcac gggaccgtgg cttccttcct cacctggctc tgcctccccc gtgctctcgg 2357
gcgaagtggg ttcttgtgcc ttcccctccc gggcccaggc tccccgtgcg cgggccctgc 2417

cctttcctcc cgcgccccac cggctccgac gcgcaacccc gctcagcagt cacagaagca 2477
gggcccagcc accttggtct ttttttggga gttcagggga gtaggagaat gtcttccaga 2537
aaaatacata agctagtttc tgttctgtaa agtgatatct ttcatacttg accaaagttc 2597
ccaataactt cccaaccact gttcaaaagc tgtgattttt gtctcccctt cccaccctcc 2657
agccaaggag cagccctgcc caggggaatt taggtgtggg tacccgggga gcaccccgtt 2717
cctggacccc agtgttgcat ttcctggctg aggaagggtg gtcatcccag ctcctgccct 2777
accctctcac ttaactggag ctttgggacg caccctccac agtgggaggt ggtggtgggg 2837
tggcggtggc ggggcctcac gacagcttgg tgctggtaag aggaagcccg tggttctggc 2897
taggctctca tgtccagaca gcggggacca ggggaaaacc cagccccttc tgtaatcccc 2957
cttcatttcc taccttcctt cctcctctgt ttagcaaagg agggcagctc acttggatgt 3017
ccttacaacg cccctggccc caggttgagc aataagaaac cagaaccttg cggcccagtg 3077
gcccgggcca gttcaggccg cctccccctc ctctgcctgg ggccattgag ccccagcctc 3137
caggggcccg ggtgcgtttg caagccagtg gccactgtcc gggctgtgat ggcaccaagg 3197
caggtggagc accaggtacc acacagctgg gcttcccacc aggctttccc gcgggggtct 3257
cagggagctt ctccccagcg ctgctcggag tctgcaggaa ctggccttgt tctccttagc 3317
ccgtcactcc atacagtatt aggtgaggat ggatgcgggc gctgtccttg ccgggaagtc 3377
actgttgaag ttgcagtggc ttgttcacac ctgtgggaag agaagtgaag actttctcct 3437
tgcattaaaa agtctgaact gtg 3460

<210> 110

<211> 534

<212> PRT

<213> Homo sapiens

<400> 110

Met Phe Ser Trp Val Ser Lys Asp Ala Arg Arg Lys Lys Glu Pro Glu
1               5                   10                  15

Leu Phe Gln Thr Val Ala Glu Gly Leu Arg Gln Leu Tyr Ala Gln Lys

            20                  25                  30
Leu Leu Pro Leu Glu Glu His Tyr Arg Phe His Glu Phe His Ser Pro
        35                  40                  45
Xaa Leu Glu Asp Ala Asp Phe Asp Asn Lys Pro Met Val Leu Leu Val
    50                  55                  60
Xaa Gln Tyr Ser Thr Gly Lys Thr Thr Phe Ile Arg His Leu Ile Glu
65                  70                  75                  80
Gln Asp Phe Pro Gly Met Arg Ile Gly Pro Glu Pro Thr Thr Asp Ser
                85                  90                  95
Phe Ile Ala Val Met His Gly Pro Thr Glu Gly Val Val Pro Gly Asn
            100                 105                 110
Ala Leu Val Val Asp Pro Arg Arg Pro Phe Arg Lys Leu Asn Ala Phe
        115                 120                 125
Gly Asn Ala Phe Leu Asn Arg Phe Met Cys Ala Gln Leu Pro Asn Pro
    130                 135                 140
Val Leu Asp Ser Ile Ser Ile Ile Asp Thr Pro Gly Ile Leu Ser Gly
145                 150                 155                 160
Glu Lys Gln Arg Ile Ser Arg Gly Tyr Asp Phe Ala Ala Val Leu Glu
                165                 170                 175
Trp Phe Ala Glu Arg Val Asp Arg Ile Ile Leu Leu Phe Asp Ala His
            180                 185                 190
Lys Leu Asp Ile Ser Asp Glu Phe Ser Glu Val Ile Lys Ala Leu Lys
        195                 200                 205
Asn His Glu Asp Lys Ile Arg Val Val Leu Asn Lys Ala Asp Gln Ile
    210                 215                 220
Glu Thr Gln Gln Leu Met Arg Val Tyr Gly Ala Leu Met Trp Ser Leu
225                 230                 235                 240

Gly Lys Ile Ile Asn Thr Pro Glu Val Val Arg Val Tyr Ile Gly Ser
                245                 250                 255
Phe Trp Ser His Pro Leu Leu Ile Pro Asp Asn Arg Lys Leu Phe Glu
            260                 265                 270
Ala Glu Glu Gln Asp Leu Phe Lys Asp Ile Gln Ser Leu Pro Arg Asn
        275                 280                 285
Ala Ala Leu Arg Lys Leu Asn Asp Leu Ile Lys Arg Ala Arg Leu Ala
    290                 295                 300
Lys Val His Ala Tyr Ile Ile Ser Ser Leu Lys Lys Glu Met Pro Asn
305                 310                 315                 320
Val Phe Gly Lys Glu Ser Lys Lys Lys Glu Leu Val Asn Asn Leu Gly
                325                 330                 335
Glu Ile Tyr Gln Lys Ile Glu Arg Glu His Gln Ile Ser Pro Gly Asp
            340                 345                 350
Phe Pro Ser Leu Arg Lys Met Gln Glu Leu Leu Gln Thr Gln Asp Phe
        355                 360                 365
Ser Lys Phe Gln Ala Leu Lys Pro Lys Leu Leu Asp Thr Val Asp Asp
    370                 375                 380
Met Leu Ala Asn Asp Ile Ala Arg Leu Met Val Met Val Arg Gln Glu
385                 390                 395                 400
Glu Ser Leu Met Pro Ser Gln Val Val Lys Gly Gly Ala Phe Asp Gly
                405                 410                 415
Thr Met Asn Gly Pro Phe Gly His Gly Tyr Gly Glu Gly Ala Gly Glu
            420                 425                 430
Gly Ile His Asp Val Glu Trp Val Val Gly Lys Asp Lys Pro Thr Tyr
        435                 440                 445

Asp Glu Ile Phe Tyr Thr Leu Ser Pro Val Asn Gly Lys Ile Thr Gly
    450                 455                 460
Ala Asn Ala Lys Lys Glu Met Val Lys Ser Lys Leu Pro Asn Thr Val
465                 470                 475                 480
Leu Gly Lys Ile Trp Lys Leu Ala Asp Val Asp Lys Asp Gly Leu Leu
                485                 490                 495
Asp Asp Glu Glu Phe Ala Leu Ala Asn His Leu Ile Lys Val Lys Leu
            500                 505                 510
Glu Gly His Glu Leu Pro Ala Asp Leu Pro Pro His Leu Val Pro Pro
        515                 520                 525
Ser Lys Arg Arg His Glu
    530
<210> 111
<211> 1622
<212> DNA
<213> Homo sapiens
<220>
<221> CDS
<222> (89)..(724)
<400> 111
ggtcggtgtg cttgtgaccc tgcctttgtg tggctgtcac cggtgggact ggcggggact 60
gtgtgattaa cctccatttc agctaatc atg gga gag att aaa gtc tct cct 112
Met Gly Glu Ile Lys Val Ser Pro
1               5
gat tat aac tgg ttt aga ggt aca gtt ccc ctt aaa aag att att gtg 160
Asp Tyr Asn Trp Phe Arg Gly Thr Val Pro Leu Lys Lys Ile Ile Val

    10                  15                  20
gat gat gat gac agt aag ata tgg tcg ctc tat gac gcg ggc ccc cga 208
Asp Asp Asp Asp Ser Lys Ile Trp Ser Leu Tyr Asp Ala Gly Pro Arg
25                  30                  35                  40
agt atc agg tgt cct ctc ata ttc ctg ccc cct gtc agt gga act gca 256
Ser Ile Arg Cys Pro Leu Ile Phe Leu Pro Pro Val Ser Gly Thr Ala
                45                  50                  55
gat gtc ttt ttc cgg cag att ttg gct ctg act gga tgg ggt tac cgg 304
Asp Val Phe Phe Arg Gln Ile Leu Ala Leu Thr Gly Trp Gly Tyr Arg
            60                  65                  70
gtt atc gct ttg cag tat cca gtt tat tgg gac cat ctc gag ttc tgt 352
Val Ile Ala Leu Gln Tyr Pro Val Tyr Trp Asp His Leu Glu Phe Cys
        75                  80                  85
gat gga ttc aga aaa ctt tta gac cat tta caa ttg gat aaa gtt cat 400
Asp Gly Phe Arg Lys Leu Leu Asp His Leu Gln Leu Asp Lys Val His
    90                  95                  100
ctt ttt ggc gct tct ttg gga ggc ttt ttg gcc cag aaa ttt gct gaa 448
Leu Phe Gly Ala Ser Leu Gly Gly Phe Leu Ala Gln Lys Phe Ala Glu
105                 110                 115                 120
tac act cac aaa tct cct aga gtc cat tcc cta atc ctc tgc aat tcc 496
Tyr Thr His Lys Ser Pro Arg Val His Ser Leu Ile Leu Cys Asn Ser
                125                 130                 135
ttc agt gac acc tct atc ttc aac caa act tgg act gca aac agc ttt 544
Phe Ser Asp Thr Ser Ile Phe Asn Gln Thr Trp Thr Ala Asn Ser Phe
            140                 145                 150
tgg ctg atg cct gca ttt atg ctc aaa aaa ata gtt ctt gga aat ttt 592
Trp Leu Met Pro Ala Phe Met Leu Lys Lys Ile Val Leu Gly Asn Phe

        155                 160                 165

tca tct ggc ccg gtg gac cct atg atg gct gat gcc att gat ttc atg 640

Ser Ser Gly Pro Val Asp Pro Met Met Ala Asp Ala Ile Asp Phe Met

    170                 175                 180

gta gac agg cta gaa agt ttg ggt cag agt gaa ctg gct tca aga ctt 688

Val Asp Arg Leu Glu Ser Leu Gly Gln Ser Glu Leu Ala Ser Arg Leu

185                 190                 195                 200

acc ttg aaa ttg tca aaa ttc tta tgt gga acc tca taaaattcgg 734

Thr Leu Lys Leu Ser Lys Phe Leu Cys Gly Thr Ser

                205                 210

ggacatacct gtaactatta tggatgtgtt tgatcagagt gcgctttcaa ctgaagctaa 794

agaagaaatg tacaagctgt atccctaatg ccccgaagag gctcatctga aaacaggagg 854

caatttccca tacctgtgca gaagtgcaga ggtcaatctt tatgtacaga tacatttgtc 914

kgcaattccw kggrggaccm aatacgcggc ctttgaccca tcaatggtca gtgccgagga 974

gcttgaggtg cagaaaggca gccttggcat cagccaggag gagcagtagt gtgtctctcg 1034

ctgtcaatga tgagttgacc cggtgtgttc ttgtatagtc agtgggcatc agcacccgtt 1094

cagccggcct tttccttcag gttcgtcagg ctcaccggtt ctcactgtgt ctgggaagta 1154

ggactgatgg tcatcttcat gacaggcggc atctccacta agcctgtgta actgttccct 1214

ctttggtttt cttagctttt gaatttgaag aagtactttt gaagactccc attttaagaa 1274

ccgtgcaaat tttgctacca aaagtcttca ccactgtgtt cttaagtgaa tgttaatttc 1334

tgaggtttgg gactttgtgg tggttttttt cttcttttct tttccattct tctttctttc 1394

tttttatgtt gtttgctgta aatgctgcac atccagattg catatcagga cattggttat 1454

tttatgcttt cttggatata accatgatca gagtgccatg gccactaccc cactgtttgc 1514

tctcctgcaa atcaactgct tttaatttac acttaaacaa attgttttga gtgttagcta 1574

ctgcctttct agatattagt catttggaat aaaaattcaa tttcactg 1622

<210> 112

<211> 212

<212> PRT

<213> Homo sapiens

<400> 112
   Met Gly Glu Ile Lys Val Ser Pro Asp Tyr Asn Trp Phe Arg Gly Thr
   1               5                   10                  15
   Val Pro Leu Lys Lys Ile Ile Val Asp Asp Asp Asp Ser Lys Ile Trp
               20                  25                  30
   Ser Leu Tyr Asp Ala Gly Pro Arg Ser Ile Arg Cys Pro Leu Ile Phe
           35                  40                  45
   Leu Pro Pro Val Ser Gly Thr Ala Asp Val Phe Phe Arg Gln Ile Leu
       50                  55                  60
   Ala Leu Thr Gly Trp Gly Tyr Arg Val Ile Ala Leu Gln Tyr Pro Val
   65                  70                  75                  80
   Tyr Trp Asp His Leu Glu Phe Cys Asp Gly Phe Arg Lys Leu Leu Asp
                   85                  90                  95
   His Leu Gln Leu Asp Lys Val His Leu Phe Gly Ala Ser Leu Gly Gly
               100                 105                 110
   Phe Leu Ala Gln Lys Phe Ala Glu Tyr Thr His Lys Ser Pro Arg Val
           115                 120                 125
   His Ser Leu Ile Leu Cys Asn Ser Phe Ser Asp Thr Ser Ile Phe Asn
       130                 135                 140
   Gln Thr Trp Thr Ala Asn Ser Phe Trp Leu Met Pro Ala Phe Met Leu
   145                 150                 155                 160
   Lys Lys Ile Val Leu Gly Asn Phe Ser Ser Gly Pro Val Asp Pro Met
                   165                 170                 175
   Met Ala Asp Ala Ile Asp Phe Met Val Asp Arg Leu Glu Ser Leu Gly
   180 185 190
   Gln Ser Glu Leu Ala Ser Arg Leu Thr Leu Lys Leu Ser Lys Phe Leu
   195 200 205
   Cys Gly Thr Ser
   210
<210> 113
<211> 2391
<212> DNA
<213> Homo sapiens
<220>
<221> CDS
<222> (1)..(360)
<220>
<221> unsure
<222> (49)
<223> g or t
<220>
<221> unsure
<222> (51)
<223> a or t
<220>
<221> unsure
<222> (54)
<223> t or c
<220>

<221> unsure
<222> (55)
<223> g or t
<220>
<221> unsure
<222> (56)
<223> g or t
<220>
<221> unsure
<222> (91)
<223> g or c
<220>
<221> unsure
<222> (101)
<223> t or c
<220>
<221> unsure
<222> (103)
<223> g or t
<220>
<221> unsure
<222> (325)
<223> t or c
<220>
<221> unsure
<222> (425)
<223> g or t

<220>

<221> unsure

<222> (688)

<223> g or c

<220>

<221> unsure

<222> (1459)

<223> g or t

<220>

<221> unsure

<222> (1705)

<223> g or t

<400> 113

gac ccg ttc cat gca gtt gta tac ata gtg ttc atg ctg ggc tcc tgt 48

Asp Pro Phe His Ala Val Val Tyr Ile Val Phe Met Leu Gly Ser Cys

1               5                   10                  15

kcw tty kkc tcc aaa acg tgg att gag gtc tca ggt tcc tct scc aaa 96

Xaa Xaa Xaa Ser Lys Thr Trp Ile Glu Val Ser Gly Ser Ser Xaa Lys

            20                  25                  30

gat gyt kca aag cag ctg aag gag cag cag atg gtg atg aga ggc cac 144

Asp Xaa Xaa Lys Gln Leu Lys Glu Gln Gln Met Val Met Arg Gly His

        35                  40                  45

cga gag acc tcc atg gtc cat gaa ctc aac cgg tac atc ccc aca gcc 192

Arg Glu Thr Ser Met Val His Glu Leu Asn Arg Tyr Ile Pro Thr Ala

    50                  55                  60

gcg gcc ttt ggt ggg ctg tgc atc ggg gcc ctc tcg gtc ctg gct gac 240

Ala Ala Phe Gly Gly Leu Cys Ile Gly Ala Leu Ser Val Leu Ala Asp
65                  70                  75                  80
ttc cta ggc gcc att ggg tct gga acc ggg atc ctg ctc gca gtc aca    288
Phe Leu Gly Ala Ile Gly Ser Gly Thr Gly Ile Leu Leu Ala Val Thr
                85                  90                  95
atc atc tac cag tac ttt gag atc ttc gtt aag gag yaa agc gag gtt    336
Ile Ile Tyr Gln Tyr Phe Glu Ile Phe Val Lys Glu Xaa Ser Glu Val
            100                 105                 110
ggc agc atg ggg gcc ctg ctc ttc tgagcccgtc tcccggacag gttgaggaag    390
Gly Ser Met Gly Ala Leu Leu Phe
        115                 120
ctgctccaga agcgcctcgg aaggggagct ctcakcatgg cgcgtgctgc tgcggcatat 450
ggacttttaa taatgtgttt tgaatttcgt attctttcat tccactgtgt aaagtgctag 510
acattttcca atttaaaatt ttgcttttta tcctggcact ggcaaaaaga actgtgaaag 570
tgaaatttta ttcagccgac tgccagagaa gtgggaatgg tataggattg tccccaagtg 630
tccatgtaac ttttgtttta acctttgcac cttctcagtg ctgtatgcgg ctgcagcstc 690
ctcacctgtt tccccacaaa gggaatttct cactctggtt ggaagcacaa acacctggaa 750
atgtctacgt ttcattttgg caagtarggt gtgaagcctg ggagcagatc atgtatttcc 810
cggagacgtg ggaccttgct ggcatgtctc cttcacaatc aggcgtggga atatctggct 870
taggactgtt tctctctaag acaccattgt tttcccttat tttaaaagtg atttttttaa 930
ggacagaact tcttccaaaa gagagggatg gctttcccag aagacactcc tggccatctg 990
tggatttgtc tgtgcaccta ttggctcttc tagctgactc ttctggttgg gcttagagtc 1050
tgcctgtttc tgctagctcc gtgtttagtc cacttgggtc atcagctctg ccaagctgag 1110
cctggccaag ctaggtggac agacccttgc agtgatgtcc gtttgtccag attctgccag 1170
tcatcactgg acacgtctcc tcggcagctg ccctagcaag gggagacatt gtggtagcta 1230
tcagacatgg acagaaactg acttagtgct cacaagcccc tacacctttc tgggctgaag 1290
atcacccagc tgtgttcaga attttcttac tgtgcttagg actgcacgca agtragcaga 1350

caccaccgac ttcctttctg cgtcaccagt gtcgtcagca gagagaggac agcacaggct 1410
caaggttggt agtgaagtca ggttcggggt gcatgggctg tggtggtgkt gatcagttgc 1470
tccagtgttt gaaataagaa gactcatgtt tatgtctgga ataagttctg tttgtgctga 1530
caggtggcct aggtcctgga gatgagcacc ctctctctgg cctttaggga gtcccctctt 1590
aggacaggca ctgcccagca gcaagggcag cagagttggg tgctaagatc ctgaggagct 1650
cgaggtttcg agctggcttt agacattggt gggaccaagg atgttttgca ggatkccctg 1710
atcctaagaa gggggcctgg gggtgcgtgc agcctgtcgg ggagacccca ctctgacagt 1770
gggcacacgg cagcctgcaa agcacagggc caccgccaca gcccggcaga ggggcacact 1830
ctggagacct tgctggcagt gctagccagg aaacagagtg accaagggac aagaagggac 1890
ttgcctaaag ccacccagca actcagcagc agaaccaaga tgggccccag gctcctccat 1950
atggcccagg gcttaccacc ctatcacacg tggcctttgt cctagaccca gtcctgagca 2010
ggggagaggc tcttgagacc tgatgccctc ctacccacat ggttctccca ctgccctgtc 2070
tgctctgctg ctacagaggg gcagggcctc ccccagccca cgcttaggaa tgcttggcct 2130
ctggcaggca ggcagctgta cccaagctgg tgggcagggg gctggaaggc accaggcctc 2190
aggaggagcc ccatagtccc gcctgcagcc tgtaaccatc ggctgggccc tgcaaggccc 2250
acactcacgc cctgtgggtg atggtcacgg tgggtgggtg ggggctgacc ccagcttcca 2310
ggggactgtc actgtggacg ccaaaatggc ataactgaga taaggtgaat aagtgacaaa 2370
taaagccagt tttttacaag g 2391

<210> 114

<211> 120

<212> PRT

<213> Homo sapiens

<220>

<221> unsure

<222> (17)

<223> unknown

<220>

<221> unsure

<222> (18)

<223> unknown

<220>

<221> unsure

<222> (19)

<223> unknown

<220>

<221> unsure

<222> (31)

<223> unknown

<220>

<221> unsure

<222> (109)

<223> unknown

<400> 114

Asp Pro Phe His Ala Val Val Tyr Ile Val Phe Met Leu Gly Ser Cys

1               5                   10                  15

Xaa Xaa Xaa Ser Lys Thr Trp Ile Glu Val Ser Gly Ser Ser Xaa Lys

            20                  25                  30

Asp Xaa Xaa Lys Gln Leu Lys Glu Gln Gln Met Val Met Arg Gly His

        35                  40                  45

Arg Glu Thr Ser Met Val His Glu Leu Asn Arg Tyr Ile Pro Thr Ala

    50                  55                  60

Ala Ala Phe Gly Gly Leu Cys Ile Gly Ala Leu Ser Val Leu Ala Asp

65                    70                    75                    80

Phe Leu Gly Ala Ile Gly Ser Gly Thr Gly Ile Leu Leu Ala Val Thr

                85                    90                    95

Ile Ile Tyr Gln Tyr Phe Glu Ile Phe Val Lys Glu Xaa Ser Glu Val

            100                   105                   110

Gly Ser Met Gly Ala Leu Leu Phe

        115                   120

<210> 115

<211> 599

<212> DNA

<213> Homo sapiens

<400> 115

gggtatggaa ttggctgagg atcaaacgta tgtaggtgaa aggataccag gatgttgcta 60

aaggtgaggg acagtttggg tttgggactt accggggtga tgttagatct ggaaccccca 120

agtgaggctg gagggagtta aggtcagtat ggaagatagg gttgggacag ggtgctttgg 180

aatgaaagag tgaccttaga gggctccttg ggcctcagga atgctcctgc tgctgtgaag 240

atgagaaggt gctcttactc agttaatgat gagtgactat atttaccaaa gcccctacct 300

gctgctgggt cccttgtagc acaggagact tgggctaagg gcccctccca gggaagggac 360

accatcaggc ctctggctga ggcagtagca tagaggatcc atttctacct gcatttccca 420

gaggactagc aggaggcagc cttgagaaac cggcagttcc caaagccagc gcctggctgt 480

tctctcattg tcactgccct ctccccaacc tctcctctaa cccactagag attgcctgtg 540

tcctgcctct tgcctcttgt agaatgcagc tctggccctc aataaatgct tcctgcatt  599

<210> 116

<211> 364

<212> DNA

<213> Homo sapiens

<220>

<221> unsure

<222> (134)

<223> a, c, g or t

<220>

<221> unsure

<222> (135)

<223> a, c, g or t

<220>

<221> unsure

<222> (179)

<223> g or a

<400> 116

ttcgatcaca tagttcctca ttcccaccga agtgcatgaa atggcagtag aaatcactat 60

acagtgcttc caggggtgca ttggtgggaa tgagaatagt gatgaagtag aaatgtctgc 120

cacagttcca gganngggta ggtagcagtg tgtgtgttat gtgccactga ccctgaaara 180

tgtgccatag cccaagccaa ttgaaattga tcagggggcc aggcatggtg gctcatgcct 240

gtaatcccag caccttggga agctgaggtg ggaggattgc ttgaaaccag gagttcaaga 300

ccagcctgtg caacatagca aaaccccatc tctacaaaga ttaaaaataa aaaattagct 360

gggc 364

<210> 117

<211> 852

<212> DNA

<213> Homo sapiens

<220>

<221> CDS

<222> (26)..(217)

<220>

<221> unsure

<222> (37)

<223> a, c, g or t

<400> 117

gttcagtttc aagtttacaa gaggc atg gat gga gtn gtg acg ttc ttg aca 52

Met Asp Gly Xaa Val Thr Phe Leu Thr

1               5

agc tgg gct aac ctt tcc cga act tgt ttc ccg gag gca agg tgc tcg 100

Ser Trp Ala Asn Leu Ser Arg Thr Cys Phe Pro Glu Ala Arg Cys Ser

10                  15                  20                  25

gtg acc cag cgc atc tta acc ttg ggt ctc cta ggc tcg agg cta ggg 148

Val Thr Gln Arg Ile Leu Thr Leu Gly Leu Leu Gly Ser Arg Leu Gly

                30                  35                  40

cat tac gtt tcg tgg aac caa agc agc caa ttg cat agc aag tat ttt 196

His Tyr Val Ser Trp Asn Gln Ser Ser Gln Leu His Ser Lys Tyr Phe

            45                  50                  55

cct gca ttc caa tta aat gct taagaaaaag cagcatccta taaaattgtg 247

Pro Ala Phe Gln Leu Asn Ala

        60

atcataaaca tccatttccc tcagcttttg tgagtgcctt gacttacagc caacatcact 307

gtttaactca gtctgtttaa aaacaaactt ttctggtggt tgataacaga gagttgctcc 367

ctgagccatc agggtcctgg gagctggaag tgaaagggtt attaacattc tacctttatg 427

cagctgttgg ctgaccagaa taaactccct gctgagttca agctttgaat ggaatggatg 487

caaatgatgt tgtttccatt agagcaggtg ctcacagcat tctgattggc ctgagcagac 547

cgaggctatg gctgttggga caagcttagc atcctggaca tcttgtcaaa gaacctcact 607

cacccctctg gcctctacag ccctcagagg agagaaaacc aattctccaa caaacaggtc 667

tctccaacat ggtggtgctg gcaggcttag gtttagaaaa tcctgactgt taaaggcgtt 727

tgaatacatc acattcctat gcaaatgttt ttaatctcca gtttaatgta gtttattttt 787

cctatatgta aagtattttt atacggcttg tatcatgata gtttagcaat aaaacagttg 847

gaagc 852

<210> 118

<211> 64

<212> PRT

<213> Homo sapiens

<220>

<221> unsure

<222> (4)

<223> unknown

<400> 118

Met Asp Gly Xaa Val Thr Phe Leu Thr Ser Trp Ala Asn Leu Ser Arg

1               5                   10                  15

Thr Cys Phe Pro Glu Ala Arg Cys Ser Val Thr Gln Arg Ile Leu Thr

            20                  25                  30

Leu Gly Leu Leu Gly Ser Arg Leu Gly His Tyr Val Ser Trp Asn Gln

        35                  40                  45

Ser Ser Gln Leu His Ser Lys Tyr Phe Pro Ala Phe Gln Leu Asn Ala

    50                  55                  60

<210> 119

<211> 1156

<212> DNA

<213> Homo sapiens

<220>

<221> CDS

<222> (524)..(1105)

<220>

<221> unsure

<222> (10)

<223> a or t

<400> 119
   cggcaactgw gaggaagcaa agggaaaaaa actccattaa aaagcccagc tttcctccat 60
   gttagatgtg acttggaaaa tgagaaagat ttagcaaaat tccaccgtat cttttgccag 120
   gctagagaca gggagagcag agtaaaaccc tcaggctgct gaaatttcta ggctgttagg 180
   aagcccctcg aattctgtga aaatgagggt ttcttaactc acactgagag cggaaagggg 240
   cagacccttt tcataactcc ctcaagtgtg tgttaccttt ctttaccagc atggtaagca 300
   acaggacata tcccagcctc ggacatgtct gtatgatcca aggtacccaa agtcagacag 360
   agtaaactca agcctggcac tggctttctg ccgcttcatg tgctttggaa aaagcaggag 420
   aagcaatagc agcaggagtc cccagcagct ggagccgcaa gaatgaactg caaagaggga 480
   actgacagca gctgcggctg cagggggcaa cgacgagaag aag atg ttg aag tgt 535
   Met Leu Lys Cys
   1
   gtg gtg gtg ggg gac ggt gcc gtg ggg aaa acc tgc ctg ctg atg agc 583
   Val Val Val Gly Asp Gly Ala Val Gly Lys Thr Cys Leu Leu Met Ser
   5 10 15 20
   tac gcc aac gac gcc ttc cca gag gaa tac gtg ccc act gtg ttt gac 631
   Tyr Ala Asn Asp Ala Phe Pro Glu Glu Tyr Val Pro Thr Val Phe Asp
   25 30 35
   cac tat gca gtt act gtg act gtg gga ggc aag caa cac ttg ctc gga 679
   His Tyr Ala Val Thr Val Thr Val Gly Gly Lys Gln His Leu Leu Gly
   40 45 50
   ctg tat gac acc gcg gga cag gag gac tac aac cag ctg agg cca ctc 727
   Leu Tyr Asp Thr Ala Gly Gln Glu Asp Tyr Asn Gln Leu Arg Pro Leu
   55 60 65
   tcc tac ccc aac acg gat gtg ttt ttg atc tgc ttc tct gtc gta aac 775
   Ser Tyr Pro Asn Thr Asp Val Phe Leu Ile Cys Phe Ser Val Val Asn
   70 75 80
   cct gcc tct tac cac aat gtc cag gag gaa tgg gtc ccc gag ctc aag 823
   Pro Ala Ser Tyr His Asn Val Gln Glu Glu Trp Val Pro Glu Leu Lys
   85 90 95 100
   gac tgc atg cct cac gtg cct tat gtc ctc ata ggg acc cag att gat 871
   Asp Cys Met Pro His Val Pro Tyr Val Leu Ile Gly Thr Gln Ile Asp
   105 110 115
   ctc cgt gat gac cca aaa acc ttg gcc cgt ttg ctg tat atg aaa gag 919
   Leu Arg Asp Asp Pro Lys Thr Leu Ala Arg Leu Leu Tyr Met Lys Glu
   120 125 130
   aaa cct ctc act tac gag cat ggt gtg aag ctc gca aaa gcg atc gga 967
   Lys Pro Leu Thr Tyr Glu His Gly Val Lys Leu Ala Lys Ala Ile Gly
   135 140 145
   gca cag tgc tac ttg gaa tgt tca gct ctg act cag aaa ggt ctc aaa 1015
   Ala Gln Cys Tyr Leu Glu Cys Ser Ala Leu Thr Gln Lys Gly Leu Lys
   150 155 160
   gcg gtt ttt gat gaa gca atc ctc acc att ttc cac ccc aag aaa aag 1063
   Ala Val Phe Asp Glu Ala Ile Leu Thr Ile Phe His Pro Lys Lys Lys
   165 170 175 180
   aag aaa cgc tgt tct gag ggt cac agc tgc tgt tca att atc 1105
   Lys Lys Arg Cys Ser Glu Gly His Ser Cys Cys Ser Ile Ile
   185 190
   tgaggttgtc tgggacctgc ctccacccca tccagggatg agaatggcag c 1156

<210> 120

<211> 194

<212> PRT

<213> Homo sapiens

<400> 120

Met Leu Lys Cys Val Val Val Gly Asp Gly Ala Val Gly Lys Thr Cys

1               5                   10                  15

Leu Leu Met Ser Tyr Ala Asn Asp Ala Phe Pro Glu Glu Tyr Val Pro

            20                  25                  30

Thr Val Phe Asp His Tyr Ala Val Thr Val Thr Val Gly Gly Lys Gln

        35                  40                  45

His Leu Leu Gly Leu Tyr Asp Thr Ala Gly Gln Glu Asp Tyr Asn Gln

    50                  55                  60

Leu Arg Pro Leu Ser Tyr Pro Asn Thr Asp Val Phe Leu Ile Cys Phe

65                  70                  75                  80

Ser Val Val Asn Pro Ala Ser Tyr His Asn Val Gln Glu Glu Trp Val
                85                  90                  95
Pro Glu Leu Lys Asp Cys Met Pro His Val Pro Tyr Val Leu Ile Gly
            100                 105                 110
Thr Gln Ile Asp Leu Arg Asp Asp Pro Lys Thr Leu Ala Arg Leu Leu
        115                 120                 125
Tyr Met Lys Glu Lys Pro Leu Thr Tyr Glu His Gly Val Lys Leu Ala
    130                 135                 140
Lys Ala Ile Gly Ala Gln Cys Tyr Leu Glu Cys Ser Ala Leu Thr Gln
145                 150                 155                 160
Lys Gly Leu Lys Ala Val Phe Asp Glu Ala Ile Leu Thr Ile Phe His
                165                 170                 175
Pro Lys Lys Lys Lys Lys Arg Cys Ser Glu Gly His Ser Cys Cys Ser
            180                 185                 190
Ile Ile
<210> 121
<211> 1732
<212> DNA
<213> Homo sapiens
<220>
<221> CDS
<222> (2)..(259)
<220>
<221> unsure
<222> (28)
<223> a, c, g or t

<220>

<221> unsure

<222> (388)

<223> g or a

<220>

<221> unsure

<222> (631)

<223> a or t

<220>

<221> unsure

<222> (637)

<223> g or a

<220>

<221> unsure

<222> (638)

<223> g or a

<220>

<221> unsure

<222> (639)

<223> g or a

<400> 121

g gac att gag tcc aag gag cag gtg cgn acc ctc acg ggc cac gtg ggc 49

Asp Ile Glu Ser Lys Glu Gln Val Xaa Thr Leu Thr Gly His Val Gly

1               5                   10                  15

acc gtg tat gcc ctg gcg gtc atc tcg acg cca gac cag acc aaa gtc 97

Thr Val Tyr Ala Leu Ala Val Ile Ser Thr Pro Asp Gln Thr Lys Val

            20                  25                  30
ttc agt gca tcc tac gac cgg tcc ctc agg gtc tgg agt atg gac aac 145
Phe Ser Ala Ser Tyr Asp Arg Ser Leu Arg Val Trp Ser Met Asp Asn
        35                  40                  45
atg atc tgc acg cag acc ctg ctg cgt cac cag ggc agt gtc acc gcg 193
Met Ile Cys Thr Gln Thr Leu Leu Arg His Gln Gly Ser Val Thr Ala
    50                  55                  60
ctg gct gtg tcc cgg ggc cga ctc ttc tca ggg gct gtg gat agc act 241
Leu Ala Val Ser Arg Gly Arg Leu Phe Ser Gly Ala Val Asp Ser Thr
65                  70                  75                  80
gtg aag gtt tgg act tgc taacaggatc caggccaggc tgtggtttcc 289
Val Lys Val Trp Thr Cys
                85
cctgaaccag ccctggacct ttctgagcca ggctggccac atggggtggt ctcggggttt 349
ctgcctgccc cgtgggcata ggtggacagg ctctggcarg ccggcagtgc cctccccgtc 409
ccatgctcgg cgagcctccc tctactcggc actgtccttg ctgcccagcc cctctctggg 469
tgccaggtac gacgcttgcc ccggcccacc ctccatcccc accctccatc cccaccctag 529
atggagcgag ggccttttta ctcacctttt ctaccgtttt tagactgtat gtagatttgg 589
ttacctcctg gttgaaataa atgctccaca gactgtgaaa awaaaaarrr acaamtcctc 649
gggacaaggg ggctgtgtgt ggccttgagg ttggtgtgca caggcactgg ctgctgtgag 709
tgggggggca tggggcagtt tcctttggtg gaccccagga cttcggccca ctccggggct 769
cccctccctg ctaggaggca actcgtcaca cccaagctgc tggcctccag tcccatctcc 829
cccaacacat gtgcccccaa aaagtgagcc aggcacctct gtttcctgct gtttattgac 889
agccgacgga gcgccttgcc cagacctccc ctgcccacct gctggagccc agcctgtgcc 949
gccctctgag gagaggcctg gggggacagc tgggcacgtc cactcgcagg gaaacacggg 1009
gtgagacagc aggaaggggc cctgcacgcc gggacgccac ctccgccagc cgcctccacc 1069
cgccccacac cacaatcgct ggttttcggc attttttaaa tttttttttt aagaaacgtc 1129

aaagttgtgc ccaacactgt ggatcagcaa acacgataga ggagaccagt cagtacttct 1189
tggagggggc aggaggagag aggaaaaggg agggcgagaa tgaccacaca acacagcctt 1249
ggaccatgag cagaagcgtc cgtgggaact ccactggggt ggatgggctg cctgcacagc 1309
ccctggagag ggggccaggc acaccctcag agggagctgc aagcccgtgg cctggcctgc 1369
tacatgccct gcttccacgt ggctgccacg ctgacacacc cacattcacc aaacccaccc 1429
gcgccctggg acgcagccac gccaggagga ggacacggcc gccgagagca aggcacaacc 1489
tcgagttctt ggggcgcaga gaacttagga gagaagcacg gaggagcccc cggcagagca 1549
cccgcccccg ggccccagcc ttccacctgt gctagcagcc tggggcctcc actctggccg 1609
gaggaaggac cgcaggcaga cagcctgggc ctctaacagc ttttgtccgg agctagactt 1669
cgtgtccttt cagttggtaa atggttttct atagaatcaa taatatttct ttctttaaat 1729
ata 1732

<210> 122
<211> 86
<212> PRT
<213> Homo sapiens
<220>
<221> unsure
<222> (9)
<223> unknown
<400> 122
Asp Ile Glu Ser Lys Glu Gln Val Xaa Thr Leu Thr Gly His Val Gly
1               5                   10                  15
Thr Val Tyr Ala Leu Ala Val Ile Ser Thr Pro Asp Gln Thr Lys Val
            20                  25                  30
Phe Ser Ala Ser Tyr Asp Arg Ser Leu Arg Val Trp Ser Met Asp Asn
        35                  40                  45

Met Ile Cys Thr Gln Thr Leu Leu Arg His Gln Gly Ser Val Thr Ala
    50                  55                  60
Leu Ala Val Ser Arg Gly Arg Leu Phe Ser Gly Ala Val Asp Ser Thr
65                  70                  75                  80
Val Lys Val Trp Thr Cys
                85

<210> 123
<211> 603
<212> DNA
<213> Homo sapiens
<220>
<221> CDS
<222> (168)..(350)
<220>
<221> unsure
<222> (343)
<223> g or a
<220>
<221> unsure
<222> (422)
<223> t or c
<220>
<221> unsure
<222> (457)
<223> g or c
<400> 123

gttctttccc ccaccctgcc acaccctggg agaaaaaact agactttggc ttcagaaagc 60
acagatgtga cccaggctta ctaaagagac aactccacag ccctgggaac acactcttga 120
gccaaacttg gttgaagact aggtcttccc tggcaagttc cggaaga atg gac tta 176
Met Asp Leu
1
ctg act ttt atc aac tct tct cac tgc caa ggc caa cag cat ctg agg 224
Leu Thr Phe Ile Asn Ser Ser His Cys Gln Gly Gln Gln His Leu Arg
    5                   10                  15
tat agc ttt ttg gga gta cct gct ttc ttg cct cct gga gga tat ttt 272
Tyr Ser Phe Leu Gly Val Pro Ala Phe Leu Pro Pro Gly Gly Tyr Phe
20                  25                  30                  35
ctg tcc tgg ggc ttc atg gcc cct ctc ttc cct gtt aca cat tgc tgt 320
Leu Ser Trp Gly Phe Met Ala Pro Leu Phe Pro Val Thr His Cys Cys
                40                  45                  50
gct tca gag cct ttg cag ctg cra cct agt tgaatccaca taggsttcct 370
Ala Ser Glu Pro Leu Gln Leu Xaa Pro Ser
            55                  60
tccacacggt gggaaggatc ttgctgcttt cactcacagg accagggagt tyttcaatca 430
ggaggtgggt ttttgttccc ttcaggsctt tggcaacatc tagagacagt tttgattgcc 490
acgcctggag tgggatgtgt gtgctactgg catctagtgg ctgctaaaca tcctacactg 550
cataggatag tccccactac ccccagccaa gaattatctg actccagggg tca 603
<210> 124
<211> 61
<212> PRT
<213> Homo sapiens
<220>

<221> unsure

<222> (59)

<223> unknown

<400> 124

Met Asp Leu Leu Thr Phe Ile Asn Ser Ser His Cys Gln Gly Gln Gln

1               5                   10                  15

His Leu Arg Tyr Ser Phe Leu Gly Val Pro Ala Phe Leu Pro Pro Gly

            20                  25                  30

Gly Tyr Phe Leu Ser Trp Gly Phe Met Ala Pro Leu Phe Pro Val Thr

        35                  40                  45

His Cys Cys Ala Ser Glu Pro Leu Gln Leu Xaa Pro Ser

    50                  55                  60

<210> 125

<211> 1289

<212> DNA

<213> Homo sapiens

<220>

<221> CDS

<222> (775)..(1017)

<220>

<221> unsure

<222> (200)

<223> g or a

<400> 125

tatgcgagcc aatattgaga gaggaatttt gctgaggttt tctctgaggt ttttttgatg 60

ctttatagga aactattttt taaaaaaagc catttcccac ccaaggacac agtggatgtg 120

ttttccctga ctccagcagg gcaaggaatg taaccgagag gttgtgtggg ctgggctctg 180

gtgccctctt ccctggcccr gaacacctct cctcctgatt cccttggcac cttgtctttc 240

tgtctgttta cctgtctccc tgcctgccca tctgcatctt ttgcagccca ctctgacttc 300

catctggggg ctgagaccac ccttgcctgc ccccttcttt ctgccttaag aatgtccttt 360

taggctgggc atggttgtca cgcctgtaac cccagcactt tgggaggcgg agacgggcag 420

ataacctgag gtcaggattt cgagaccaac ctgacctaca tggagaaact ccgcctctag 480

taaaaataca aaattagccg ggcatggtgg tgcacgcctc taatcccagc tactcgggag 540

gctgaggcag gagaatcact tgaacccggg aagtggaggt tgcagtgagc caagagtaca 600

ccactgcact ccagcctggg caacagagcg agactccgtc ttaaaaaaaa aaaaaaaaag 660

aacgcccttt tactgtcctc atcatcccag tttgaggcag tgctggagtg gggaaggccg 720

tcttagacca tagaggttgg aagacgctga gagatcatcc agcccagccc cttg atg 777

Met

1

tta cag agc aga aga cag atg ccc aaa cag gag aag gca ctt gcc cac 825

Leu Gln Ser Arg Arg Gln Met Pro Lys Gln Glu Lys Ala Leu Ala His

            5                   10                  15

ggt cat acg gca ggt tgc cac aaa acc aag atg gca gcc ctt cct cag 873

Gly His Thr Ala Gly Cys His Lys Thr Lys Met Ala Ala Leu Pro Gln

        20                  25                  30

cgt gcc tca ctg cca ctc cca gag cca ggg agc ccc ata aaa ccc aca 921

Arg Ala Ser Leu Pro Leu Pro Glu Pro Gly Ser Pro Ile Lys Pro Thr

    35                  40                  45

tca tgt ctt aag agt ata tct ggc tcc ttg acc agc aat cgg ccc tgg 969

Ser Cys Leu Lys Ser Ile Ser Gly Ser Leu Thr Ser Asn Arg Pro Trp

50                  55                  60                  65

gag cca cca ggt ggg aaa agc gcc tct gcc aga gtc cag gcc ttg gga 1017

Glu Pro Pro Gly Gly Lys Ser Ala Ser Ala Arg Val Gln Ala Leu Gly
                70                  75                  80
tgacagacag cttgcccgca cactcgggcc ccactcaagg atgtagggcc ttttctggcc 1077
cctgacccct ccctgggcat tgggagcgtg gggacggggc tggccttggg aggagcggca 1137
ggggcatcac ctccttctgc tgcttctccc tgctcctacc ctcaagggcc tgggggctgc 1197
ccagctgcct ctatgccctt ctgggggtct cagcccactg ctgacacttc tgcaatccag 1257
agaaacacta aataaagcaa tacgtgtttg cc 1289
<210> 126
<211> 81
<212> PRT
<213> Homo sapiens
<400> 126
Met Leu Gln Ser Arg Arg Gln Met Pro Lys Gln Glu Lys Ala Leu Ala
1               5                   10                  15
His Gly His Thr Ala Gly Cys His Lys Thr Lys Met Ala Ala Leu Pro
            20                  25                  30
Gln Arg Ala Ser Leu Pro Leu Pro Glu Pro Gly Ser Pro Ile Lys Pro
        35                  40                  45
Thr Ser Cys Leu Lys Ser Ile Ser Gly Ser Leu Thr Ser Asn Arg Pro
    50                  55                  60
Trp Glu Pro Pro Gly Gly Lys Ser Ala Ser Ala Arg Val Gln Ala Leu
65                  70                  75                  80
Gly
<210> 127
<211> 1085
<212> DNA

<213> Homo sapiens

<220>

<221> CDS

<222> (734)..(886)

<220>

<221> unsure

<222> (276)

<223> g or t

<400> 127
   ttttgagaca gagttttgct ctcattgccc aggctggagt gcggtggtgc tatttcagct 60
   caccacaacc tctgcctcct gggttcaagc gattctcctg ccttagcctc ccgaatagct 120
   ggaattacag gcacgcacca ccatgcctga ctaattttgt atttttagta gacatggggt 180
   ttctccatgt tggtcaggct ggtctcaaac tcccaccttc aggtgatccg cccacctcgg 240
   cctcctgagg tggctgagat tacaggcgtg caactkgtgc cagcttgcta attttcacag 300
   aagttgatgg caattcttca catgtaaaca gtgccagtgc acagaacctt tatatatttt 360
   ttgaagccag tactgtgctc tgcatataac aaagctgctt caaggatgag acctttttct 420
   aaaagcatgt aatgtgagaa gccggcctgc cttattttct tttttctttt ttaatgatta 480
   aaaatagttt gtggcaaggc acggtggctc aggcctgtaa ttctagcact ttgggaggcc 540
   gaggcaggag gattacttga gcctacaagt ttgatgggcc agcatgcaca gcatagcaag 600
   actgcatctc tacagagagt aaaaaaaatt acccgagtgt ggtgatgtgc atctgtaatc 660
   tcagctactt gggaggctga ggtgagagga tcacttgagc ttgggtgagg tgaggctgca 720
   gtgagtcctg atc atg ctg ctg cac tca atc ttg gac aac aga gca aga 769
   Met Leu Leu His Ser Ile Leu Asp Asn Arg Ala Arg
   1 5 10
   ccc tgt ctc aaa aaa aaa aaa aat ata tat ata tat ata tat tat ttt 817
   Pro Cys Leu Lys Lys Lys Lys Asn Ile Tyr Ile Tyr Ile Tyr Tyr Phe
   15 20 25
   tat gag gtg aag tgc atc aaa ctt ggg aaa gat ttg agg agg ctg gga 865
   Tyr Glu Val Lys Cys Ile Lys Leu Gly Lys Asp Leu Arg Arg Leu Gly
   30 35 40
   acc tcc tgg aaa acc act cct tgaagaaaga tatgagagac atttagaagt 916
   Thr Ser Trp Lys Thr Thr Pro
   45 50
   gattcctgct ttcagaagga ggtggattca aatacatcaa aagtcccttc ctctgctaag 976
   tgtttatagt tcaatgaata atttcaatat ttgtatgtgt tcttgtcatt ttattttttt 1036
   ctgaaaaact tccaaaaatt tgaaaataaa attacagcct tttcttctt 1085
<210> 128
<211> 51
<212> PRT
<213> Homo sapiens
<400> 128
Met Leu Leu His Ser Ile Leu Asp Asn Arg Ala Arg Pro Cys Leu Lys
1               5                   10                  15
Lys Lys Lys Asn Ile Tyr Ile Tyr Ile Tyr Tyr Phe Tyr Glu Val Lys
            20                  25                  30
Cys Ile Lys Leu Gly Lys Asp Leu Arg Arg Leu Gly Thr Ser Trp Lys
        35                  40                  45
Thr Thr Pro
    50
<210> 129
<211> 1544

<212> DNA

<213> Homo sapiens

<220>

<221> unsure

<222> (1076)

<223> g or a

<400> 129

gctttgaact tactcaggaa agccagcccc cataatattg tattaccaaa cagtatcgct 60

ttgttaggaa ggatctggaa taatcttgaa gggaagtcag agttttctcc ctgcctatta 120

acaaaaaccc aattttgttc atattgaagc atgaaataaa tgagagcaag gtagggccaa 180

attaactctt gtggacagtc cctaaaagtc cagttctaca tttgtgaaaa ttgtggtgcc 240

atgaattaag atggatgact ggaaaaaggt gttggagaaa gagttaaaga tgaggaagag 300

atatttttag tatatgaagt tatccaggga cttgatattc ataattcagt gctgtggaaa 360

tgaaaaaaat gattgaagag gtggaacgga aatgacctta gggggaaaaa aaaggaccaa 420

agaagtctga ttaaaagttg aaatcagtat ttctgaattc aaattgcttg aatttccaaa 480

atagtcagta aaggatctaa tagaaccaga attatttggg tgaattctgc aggttttatg 540

ggcttgtcac aacgtgaagg gctggaatgt atattaccaa atgggaattt ccattgtagg 600

tttttgctag tcccaccccc attttagcct aatttggctt aaacgcagta tggggagaat 660

tgttcccatt ccatgtgttc tgaattcagc tcatctccca gcatatagat atatcctcct 720

ttaactccga ccagaaccct tcttcctgtg gcactcccca cccatagacc ttcagatcat 780

ctcccacacc ctggatctca ctctcctctt agtaacagag acactcctga ggttggactt 840

ccttgctttt ctctacttcc aaatcacaat ttcttacaac caagctttgt gctcccgagt 900

aagcagggat gtactagggg aatgtaaaac tgcaaactta aaaacctgca tcttcttgaa 960

gcatcagttt tacttaccaa atggtttaga gtcataagat gacctatttt tatataaaag 1020

ttatattata gaataaaatg ttcatacgca tagactgtta agataaaaaa ataggraatc 1080

ttgcaaggta attcttattt gcaagtgggt tatgtgttca ctctcctcta cctttatggt 1140

attttggtgt tcacttacga agcatacaac tagaaccata tccaagcaga ctctgggttg 1200

ctgttaaccc agggcctaga cttctagtgc ctctgaggca gaaccaaagg agcctgcact 1260

gggggaaatc cctttttcctg cctgcctgtc tgcctgtgac ctgtgtacgt attacaggct 1320

ttaggaccag ctgattgtta tgcttgcagg atggttttga aacagaaaca atacttgttt 1380

actgtaggaa tcctatttat attatttttc agtcctgtga atgctgtgaa aagatttatt 1440

cctttgaggc caggaagctc ccaggcatat atgcttctag gttaggattg tcctgactca 1500

ctaaagatgc caggatattg gggctgaggg gagtttgagg tgtt 1544

<210> 130

<211> 508

<212> DNA

<213> Homo sapiens

<400> 130

tgaaatcagg gctggagctt tacttaggat tcacatggcc tcctaggaac catgggacaa 60

atgggaaaca ggttatcggg ggattcatga agtcagtgag agtaattgct tcttttttgc 120

gggtgaactg aatgtatttc ttcaccaaat cttgatgtta acaattaaaa agaagaaatg 180

acatgcaagt aggtcttagc agaaaaatgc aggctgggca tgagtcatgt tgttaccctc 240

ccacatgctc ctacaatcca cagagatgcc tgtctgcagg ttcttgaagt tattgttagt 300

atttggtatc tcaaattttt cgtcactgtt cacatgccac tttctctgtg cacagtggta 360

tcctcatttg cttttaacc tacactgagg agtctttgtc aggttgcact gattttccaa 420

ttctgcagta atgagtaagc tcacggcatg gggaagaaga cagtcagtcc aatgaagttc 480

tctaaattat tttaacattg cctttgaa 508

<210> 131

<211> 1204

<212> DNA

<213> Homo sapiens

<220>

<221> unsure

<222> (50)

<223> g or t

<220>

<221> unsure

<222> (54)

<223> g or c

<220>

<221> unsure

<222> (300)

<223> g or c

<220>

<221> unsure

<222> (407)

<223> g or a

<220>

<221> unsure

<222> (415)

<223> a, c, g or t
<220>
<221> unsure
<222> (417)
<223> g or c

<220>
<221> unsure
<222> (419)
<223> t or c
<220>
<221> unsure
<222> (430)
<223> a or t
<220>
<221> unsure
<222> (448)
<223> t or c
<220>
<221> unsure
<222> (449)
<223> g or t
<220>
<221> unsure
<222> (472)
<223> a, c, g or t
<400> 131
   ggcccctggc aatttgcctc aagatggggg tttgaaaata actttacctk actsaaggag 60
   tgtctggagc acctcctagt ctaagtctgc aagctccagt tcttgcctaa aaccatgcca 120
   gtggccaccc ttgggctcag acagctctgg gccttttgac cacaagccag cccctcgccc 180
   tctctgtggc atagtcttct ctgccccagg actgcagggc ggcttcctcc aaggcttcca 240
   aggctcaaaa gaaatttggc tccatccaag aaggctccag ctcccctact ggcccctggs 300
   ttcaggccca cacccctggg ccagggccag agagtgtgtc tcaggagaat tcaatgggct 360
   ctagagagac acacagaaag tttgggcatt tgggaaattt tcaaggrtgt atgtntsgyt 420
   cacgtatggw gcaggttgtc ctggtccykg ggtgcaggga agtgggctgc anggaagtgg 480
   attggagggg agcttgagga atataaggag cgggggtgga gactcaggct atggacaagg 540
   acagccccaa ggttgggaag acctggcctt agtcgtcctc agcctagggg cagggcagtg 600
   aagaaagctc tccccgctcc tgctgtaatg acccagagta gcctccccag gccggcatct 660
   tatgtgtgtc ttccaccatc ctcatggtgg cacttttcta ggcctgtctc ccagcattgt 720
   gcaaggctcg gaagagaacc agggaagtga aactgggtga aaacagaaag ctcaatggat 780
   gggctaggtt ccccagatca ttagggcaga gtttgcacgt cctctggtca ctggaatcca 840
   cccagcccac gaatcatctc cctcttgaag gattttattt ctactgggtt ttggaacaaa 900
   ctcctgctga gaccccacag ccagaaactg aaagcagcag ctccccaaag cctggaaaat 960
   ccctaagaga aggcctgggg caggaagtgg agtgacaggg gacaggtaga gagaaggggg 1020
   cccaatggcc agggagtgaa ggaggtggcg ttgctgagag cagtctgcac atgcttctgt 1080
   ctgagtgcag gaaggtgttc cagggtcgaa attacacttc tcgtacctgg agacgctgtt 1140
   tgtgggagca ctgggctcat gcctggcaca caataggtct gcaataaacc atggttaaat 1200
   cctg 1204

<210> 132
<211> 508
<212> DNA
<213> Homo sapiens
<220>
<221> unsure
<222> (223)
<223> a, c, g or t
<220>
<221> unsure
<222> (237)

<223> a, c, g or t

<220>

<221> unsure

<222> (380)

<223> a, c, g or t

<220>

<221> unsure

<222> (468)

<223> a, c, g or t

<400> 132

cccgggagaa tcacttcagc ctgggagatc aaggctgcag tgagctctga acgcaccact 60

acactccagc ctgggtgaca gagtgagatc ctgtctcaaa aaaagagaaa gaaaaccttt 120

gagattcttc catttttaga gctgagagag cacttgtgaa acacacacac atgcacaaac 180

atataaacat gcatacaggc atgcacatgc acacacaaat acncatacac acacacnagc 240

acacacacac caccaccacc atcatcagag gaacttacag aaaaggggac atttatagat 300

tcctaggaat atgccaaagc ttttcaaagc ctctatggac agctcattcc ttaacttttc 360

ctctttaaaa tcttttttan cttcttattt gccccagcca ctatcactgc ctcaggcagc 420

tgcaacgtta aacaattgcc actgattact ttcaacaaat aacctcanag aaaaggctgt 480

gtgtattgaa tgggtatcaa gtcacgtc 508

<210> 133

<211> 484

<212> DNA

<213> Homo sapiens

<220>

<221> unsure

<222> (313)

<223> a, c, g or t

<220>

<221> unsure

<222> (336)

<223> g or c

<220>

<221> unsure

<222> (401)

<223> g or c

<400> 133

gtcgactcga gcggccgcgg accgtttttt ttttttttct ctcctctgcc ccttctaata 60

tcttggagag ggatggagac tgaagagtga gtttggtcct ccacttgatc caggttctta 120

tttttgtttt ctacttcaaa gcgagaactt ggtactgtga ctttgataag aattgacttc 180

aggcccagca agatccctca tgcctgtaat cccagcactt tgggggccca aggcaggagg 240

attgcgtgag cccaggagtt cgagacccac ctgggcaaca tagggacctt gtctctacaa 300

aaaataaaat tanctgggtt tgatggtgca catcastggt cctggctact cgggaggcca 360

aggtgggagg aatgcttgag gatcggaggt caaggctgca stgagccaat attgtgccac 420

tgcattccag cctgggcaac agagtgagac actgtcttaa aaaaaaaaaa aaaaaacggt 480

ccgc 484

<210> 134
<211> 605
<212> DNA
<213> Homo sapiens
<400> 134
gctgcatgtc taaaattaca tggagttagt gtctattctt tttcccttt tgcagcaact 60

tacacagcat ttttaacacc tttttttct agtttttttg ttcggttttg ttttccatca 120

ggaatttgag ttctctctaa cccagcttac tgtgggacat aggaaaactc agtagaaata 180

cctttggtga tcttgttgag tttaagtctg atcttgatct taaactcagt aagccactat 240

ctgcaatttt gtacattata tagtattttg aagatatgga accttatgaa aaaaaaatag 300

caaattagtt ctttttcccc cagaggggaa agttatgttc tgcaaatagt gtgtgtctta 360

ttttactgtt gaacagcaat tgctatttat ttttttattg cctagaactt caacatgttg 420

tataggaatc ctgtagtgcc actagttaaa tgccgaattc tcatctggat gttaccatca 480

aacatcagta cacttgtcat ttcacatgtg tttaatgtga cagtttttca gtactgtatg 540

tgttaatttc tacttttttt aatatttaaa attgctttta aataaacata ttctcagttg 600

atccc 605

<210> 135
<211> 1786
<212> DNA
<213> Homo sapiens
<220>
<221> CDS
<222> (2)..(151)
<220>
<221> unsure
<222> (689)
<223> g or a
<400> 135
g gca cga ggg tcc tct gca tgg ggt cag gtg ctt ctg tgc ttg ctg tcc 49
tac ctc tct cca cag cag ggc tct caa aac cat ttt gat ccc cca ttg 97
gca gag ggt tcc cct ctt tac aga gtt cag tca tta aaa gca tgg atc 145
agc tgt taatctcatt ggaggaggga actgtttcct gcattcattc atctgggaac 201
cttcttgagt agccactgtc tgccagccac tgctctagag atgggaaaac agcacggaac 261
aaaaccaagg tctttcttcc agcgaattta tatccttcag gaagctggtt cctgccacca 321
acttagcagg caacagttct cctcccctag tgggcacagg gtaccagttt tgtaggaaaa 381
gtggtccagc aaaggaagaa agcagaccaa cccagctgcc ttaccttatt ctggggccat 441
tcccccagcg atgagagctg ctcttgtttc tactgccacc atctcttctg gctgcacttc 501
acctgctgct tgagcttctg accttccttc agttccacca aatgaggaca ggaaatagca 561
gtcaagaccc ctgggccctg ctgagcgtga aacaggaagg caatgggatg agttgctggg 621
acggaagaat gggcctgggg cagaacaaat agggagcatt tgaaagcttc tggctgataa 681
atctccargg tgcatcccgg ttgccacgcc tgcccccatt aacctgctcc tggtaaatac 741
tgatccagca gctgctccag gagaggccgt cttttttttc ccagccacgc tgtgtctttg 801
catgagactc cttggggcct gggcacagag agaaaagaat tgagactcag gaggctcagt 861
gggtgagaaa atgcaaagtg gcttcacaga cacagggctg tgggagcaga tcgacgggga 921
acttgggaga tgaacttcag ggccttccga cgccttgtct caggaacatg ctttgagaaa 981
aatggtagca tcctttccat aactcagtct ctcttcccta gtttccctga agtgtgacgt 1041
tttagtatct ggagctcagt gatccccatg aatgagggat aaagtttcac tcttggtatt 1101
ttctaactag tgctagggaa agtcctgaga cacgatcaca gccactgctt ggcatacagg 1161
gcctccaccc aataagcaaa ctggagattc ctcagcctct cgtggacacc cacatctcat 1221
tcttctcaca gcagagaagc tctcccttca gcctgagctg tcttctttct gctgcagtgc 1281
agcctgctcc ctcctaccct ggcctcaagg aaggtgggaa acatcttctg catttcaaag 1341
tcctcacttt gacttatttg gccttcatct tggcatggaa ggtggcaggc agaatggaaa 1401
tacctccccc caaacaaaac agatattctt gcgtgtgtaa gggcagaagg gacaagctct 1461
ctatcccatg agactagggg ccggagccca cctgcctttc cccacaactt ttcctgctca 1521
aacccactcc tcttgacaca ctggaatctg tattatatat atttttaaga aaatacaatg 1581
atggttgtct ggttttgttg tttttacagg tgttgtggaa taaaaactgt aagaaaatta 1641
agtatttaaa atgttccaat aaagtggggt tttttgttat tctaatatat tattgtgtac 1701
ctattgtaaa tatgaaacac tcctattttg caagctgagg acacaatttg tactgttgtt 1761
atatataaat aaagtttact gaatt 1786

<210> 136
<211> 50
<212> PRT
<213> Homo sapiens
<400> 136

<210> 137
<211> 835
<212> DNA

<213> Homo sapiens

<220>

<221> CDS

<222> (535)..(729)

<400> 137
gaaaaatctc attatttcaa aatgatagac atataccaaa aacaagtcta taatgtgagt 60
agttactaaa atttacacat cttaaaagtg tgtaaatgct taaatttcag aattaccatc 120
agaacctcaa ttgacattcc tttgaatagg ctaataagtg acaaataaga ttaataagat 180
ttttcaaaat cgccaggact ggtgaatata aatgatgatt gaactggaat aatattgggg 240
accaaatcaa atgaatgatt aaattatgaa gctcatatcc ttttgaaggt agttgcaaag 300
agacatttca aaactgccct aggccattgc agcatcctta gatgggacgc ataatcatta 360
ccttaaagca tcaccactca ttttgaccat atagatttta ttatgttagt ttaaaaggtc 420
aatcagcctc atgactttat agttatgtct tgtatttaaa aacatttttt atacatttgg 480
ttatgttgat aaaccaaaaa catttgatta ataaaatatc tatttgaata aatt atg 537
agc tat cct ttc aaa cag cta ttg gca agt ttt aaa ccc aaa ata tat 585
aca cat agt tct gta ata aaa ctg ttt gac ttc tca agt aac atg act 633
tcc tta ttt ctg aac agt act ggt tac ttt caa aat gaa ttt tta ttg 681
aga ttt tcc att aac tat ttt ttt caa aga ctc aaa ttt tgt acc aag 729
taaatccagg ctttatgtac aaacatgttg tttgttttat ttggggctgg gggaggtata 789
tgatgagcag acttctcgga attcataata aattttctaa aagcct 835
<210> 138
<211> 65
<212> PRT
<213> Homo sapiens
<400> 138
<210> 139
<211> 626
<212> DNA
<213> Homo sapiens
<220>
<221> CDS
<222> (1).. (201)

<220>

<221> unsure

<222> (348)

<223> t or c

<220>

<221> unsure

<222> (353)

<223> a or t

<220>

<221> unsure

<222> (358)

<223> a or t

<220>

<221> unsure

<222> (363)

<223> a or t

<220>

<221> unsure

<222> (368)

<223> g or a

<400> 139

tgt ttc agt gtg act gtc ttg tta gag gtg aag ttt atc cag ggt aac 48

Cys Phe Ser Val Thr Val Leu Leu Glu Val Lys Phe Ile Gln Gly Asn

1               5                   10                  15

ttg ctc act aac tat tcc ttt tta tgg cct ggg gtt aaa ggg agc atg 96

Leu Leu Thr Asn Tyr Ser Phe Leu Trp Pro Gly Val Lys Gly Ser Met
            20                  25                  30
gct cac act ggt gaa aat aag gaa ggc ctg gtc tta tct tgt att aat 144
Ala His Thr Gly Glu Asn Lys Glu Gly Leu Val Leu Ser Cys Ile Asn
        35                  40                  45
aat act ggc tgc att cca cca gcc aga gat ttc tat ctg cga aga cct 192
Asn Thr Gly Cys Ile Pro Pro Ala Arg Asp Phe Tyr Leu Arg Arg Pro
    50                  55                  60
atg aaa cac tgaagagaaa tgtaggcaga aggaaatggc cacatatcac 241
Met Lys His
65
aagttctatt atatattctt ttgtaaatac atattgtata ttacttggat gttttcttat 301
atcatttact gtctttttga gttaatgtca gtttttactc tctcaaytta cwatgtwaca 361
twgtaartaa cataatgtcc tttattattt atatttaagc atctaacata tagagttgtt 421
ttcatataag tttaagataa atgtcaaaaa tatatgttct tttgtttttc tttgctttaa 481
aattatgtat ctttcctttt tcttttttt aagaataatt tattgttcag gagaaagaat 541
gtatatgtaa ctgaaactat ctgaagaatg cacattgaag gccgtgaggt actgataaac 601
taaagaattt attattcaaa atact 626
<210> 140
<211> 67
<212> PRT
<213> Homo sapiens
<400> 140
Cys Phe Ser Val Thr Val Leu Leu Glu Val Lys Phe Ile Gln Gly Asn
1               5                   10                  15
Leu Leu Thr Asn Tyr Ser Phe Leu Trp Pro Gly Val Lys Gly Ser Met

20                   25                   30
Ala His Thr Gly Glu Asn Lys Glu Gly Leu Val Leu Ser Cys Ile Asn
        35                   40                   45
Asn Thr Gly Cys Ile Pro Pro Ala Arg Asp Phe Tyr Leu Arg Arg Pro
    50                   55                   60
Met Lys His
65
<210> 141
<211> 525
<212> DNA
<213> Homo sapiens
<220>
<221> CDS
<222> (103).. (525)
<400> 141
aagaaatgga ggactcagaa ccaaggattt ccaagtgatt tcttccaaag cacaggaatc 60
tcactctgtt aaagctggtc tgttctaact gagatgacag tc atg tcc ctt tcc    114
                                                Met Ser Leu Ser
                                                 1
agg gac ctc aag gac gac ttt cac agt gac acg gta ctc tcc atc tta   162
Arg Asp Leu Lys Asp Asp Phe His Ser Asp Thr Val Leu Ser Ile Leu
 5                   10                   15                   20
aat gag cag cgc att cgg ggc att tta tgc gat gtc act atc att gtg   210
Asn Glu Gln Arg Ile Arg Gly Ile Leu Cys Asp Val Thr Ile Ile Val
                 25                   30                   35
gaa gat acc aaa ttt aaa gcc cat agc aat gtt ctg gca gct tca agc   258

Glu Asp Thr Lys Phe Lys Ala His Ser Asn Val Leu Ala Ala Ser Ser
            40                  45                  50
ctg tat ttt aaa aat atc ttt tgg agc cat aca atc tgt att tcc agc 306
Leu Tyr Phe Lys Asn Ile Phe Trp Ser His Thr Ile Cys Ile Ser Ser
        55                  60                  65
cac gtc ctg gag ctg gac gat ctc aaa gct gaa gtg ttt act gaa ata 354
His Val Leu Glu Leu Asp Asp Leu Lys Ala Glu Val Phe Thr Glu Ile
    70                  75                  80
ctt aat tat atc tac agt tcc aca gtc gtt gtc aag aga cag gaa aca 402
Leu Asn Tyr Ile Tyr Ser Ser Thr Val Val Val Lys Arg Gln Glu Thr
85                  90                  95                  100
gtc act gat ctc gca gct gca gga aaa aag ctg gga ata tcg ttc ttg 450
Val Thr Asp Leu Ala Ala Ala Gly Lys Lys Leu Gly Ile Ser Phe Leu
                105                 110                 115
gaa gac ctt act gat cgc aac ttc tca aat tcc ccg ggt ccc tat gta 498
Glu Asp Leu Thr Asp Arg Asn Phe Ser Asn Ser Pro Gly Pro Tyr Val
            120                 125                 130
ttc tgt att act gaa aag gga gtg gtt 525
Phe Cys Ile Thr Glu Lys Gly Val Val
        135                 140

<210> 142
<211> 141
<212> PRT
<213> Homo sapiens
<400> 142
Met Ser Leu Ser Arg Asp Leu Lys Asp Asp Phe His Ser Asp Thr Val

1               5                   10                  15

Leu Ser Ile Leu Asn Glu Gln Arg Ile Arg Gly Ile Leu Cys Asp Val

            20                  25                  30

Thr Ile Ile Val Glu Asp Thr Lys Phe Lys Ala His Ser Asn Val Leu

        35                  40                  45

Ala Ala Ser Ser Leu Tyr Phe Lys Asn Ile Phe Trp Ser His Thr Ile

    50                  55                  60

Cys Ile Ser Ser His Val Leu Glu Leu Asp Asp Leu Lys Ala Glu Val

65                  70                  75                  80

Phe Thr Glu Ile Leu Asn Tyr Ile Tyr Ser Ser Thr Val Val Val Lys

                85                  90                  95

Arg Gln Glu Thr Val Thr Asp Leu Ala Ala Ala Gly Lys Lys Leu Gly

            100                 105                 110

Ile Ser Phe Leu Glu Asp Leu Thr Asp Arg Asn Phe Ser Asn Ser Pro

        115                 120                 125

Gly Pro Tyr Val Phe Cys Ile Thr Glu Lys Gly Val Val

    130                 135                 140

<210> 143

<211> 1827

<212> DNA

<213> Homo sapiens

<220>

<221> CDS

<222> (138)..(1307)

<400> 143

gagacttggg ctggagccgc cctgggtgtc agcggctcgg ctcccgcgca cgctccggcc 60

gtcgcgcagc ctcggcacct gcaggtccgt gcgtcccgcg gctggcgccc ctgactccgt 120

cccggccagg gagggcc atg att tcc ctc ccg ggg ccc ctg gtg acc aac 170

Met Ile Ser Leu Pro Gly Pro Leu Val Thr Asn

1               5                   10

ttg ctg cgg ttt ttg ttc ctg ggg ctg agt gcc ctc gcg ccc ccc tcg 218

Leu Leu Arg Phe Leu Phe Leu Gly Leu Ser Ala Leu Ala Pro Pro Ser

            15                  20                  25

cgg gcc cag ctg caa ctg cac ttg ccc gcc aac cgg ttg cag gcg gtg 266

Arg Ala Gln Leu Gln Leu His Leu Pro Ala Asn Arg Leu Gln Ala Val

        30                  35                  40

gag gga ggg gaa gtg gtg ctt cca gcg tgg tac acc ttg cac ggg gag 314

Glu Gly Gly Glu Val Val Leu Pro Ala Trp Tyr Thr Leu His Gly Glu

    45                  50                  55

gtg tct tca tcc cag cca tgg gag gtg ccc ttt gtg atg tgg ttc ttc 362

Val Ser Ser Ser Gln Pro Trp Glu Val Pro Phe Val Met Trp Phe Phe

60                  65                  70                  75

aaa cag aaa gaa aag gag gat cag gtg ttg tcc tac atc aat ggg gtc 410

Lys Gln Lys Glu Lys Glu Asp Gln Val Leu Ser Tyr Ile Asn Gly Val

                80                  85                  90

aca aca agc aaa cct gga gta tcc ttg gtc tac tcc atg ccc tcc cgg 458

Thr Thr Ser Lys Pro Gly Val Ser Leu Val Tyr Ser Met Pro Ser Arg

            95                  100                 105

aac ctg tcc ctg cgg ctg gag ggt ctc cag gag aaa gac tct ggc ccc 506

Asn Leu Ser Leu Arg Leu Glu Gly Leu Gln Glu Lys Asp Ser Gly Pro

        110                 115                 120

tac agc tgc tcc gtg aat gtg caa gac aaa caa ggc aaa tct agg ggc 554

Tyr Ser Cys Ser Val Asn Val Gln Asp Lys Gln Gly Lys Ser Arg Gly

    125                 130                 135

cac agc atc aaa acc tta gaa ctc aat gta ctg gtt cct cca gct cct 602

His Ser Ile Lys Thr Leu Glu Leu Asn Val Leu Val Pro Pro Ala Pro

140                 145                 150                 155

cca tcc tgc cgt ctc cag ggt gtg ccc cat gtg ggg gca aac gtg acc 650

Pro Ser Cys Arg Leu Gln Gly Val Pro His Val Gly Ala Asn Val Thr

                160                 165                 170

ctg agc tgc cag tct cca agg agt aag cct gct gtc caa tac cag tgg 698

Leu Ser Cys Gln Ser Pro Arg Ser Lys Pro Ala Val Gln Tyr Gln Trp

            175                 180                 185

gat cgg cag ctt cca tcc ttc cag act ttc ttt gca cca gca tta gat 746

Asp Arg Gln Leu Pro Ser Phe Gln Thr Phe Phe Ala Pro Ala Leu Asp

        190                 195                 200

gtc atc cgt ggg tct tta agc ctc acc aac ctt tcg tct tcc atg gct 794

Val Ile Arg Gly Ser Leu Ser Leu Thr Asn Leu Ser Ser Ser Met Ala

    205                 210                 215

gga gtc tat gtc tgc aag gcc cac aat gag gtg ggc act gcc caa tgt 842

Gly Val Tyr Val Cys Lys Ala His Asn Glu Val Gly Thr Ala Gln Cys

220                 225                 230                 235

aat gtg acg ctg gaa gtg agc aca ggg cct gga gct gca gtg gtt gct 890

Asn Val Thr Leu Glu Val Ser Thr Gly Pro Gly Ala Ala Val Val Ala

                240                 245                 250

gga gct gtt gtg ggt acc ctg gtt gga ctg ggg ttg ctg gct ggg ctg 938

Gly Ala Val Val Gly Thr Leu Val Gly Leu Gly Leu Leu Ala Gly Leu

            255                 260                 265

gtc ctc ttg tac cac cgc cgg ggc aag gcc ctg gag gag cca gcc aat 986

Val Leu Leu Tyr His Arg Arg Gly Lys Ala Leu Glu Glu Pro Ala Asn
        270                 275                 280
gat atc aag gag gat gcc att gct ccc cgg acc ctg ccc tgg ccc aag 1034
Asp Ile Lys Glu Asp Ala Ile Ala Pro Arg Thr Leu Pro Trp Pro Lys
    285                 290                 295
agc tca gac aca atc tcc aag aat ggg acc ctt tcc tct gtc acc tcc 1082
Ser Ser Asp Thr Ile Ser Lys Asn Gly Thr Leu Ser Ser Val Thr Ser
300                 305                 310                 315
gca cga gcc ctc tgg cca ccc cat ggc cct ccc agg cct ggt gca ttg 1130
Ala Arg Ala Leu Trp Pro Pro His Gly Pro Pro Arg Pro Gly Ala Leu
                320                 325                 330
acc ccc acg ccc agt ctc tcc agc cag gcc ctg ccc tca cca aga ctg 1178
Thr Pro Thr Pro Ser Leu Ser Ser Gln Ala Leu Pro Ser Pro Arg Leu
            335                 340                 345
ccc acg aca gat ggg gcc cac cct caa cca ata tcc ccc atc cct ggt 1226
Pro Thr Thr Asp Gly Ala His Pro Gln Pro Ile Ser Pro Ile Pro Gly
        350                 355                 360
ggg gtt tct tcc tct ggc ttg agc cgc atg ggt gct gtg cct gtg atg 1274
Gly Val Ser Ser Ser Gly Leu Ser Arg Met Gly Ala Val Pro Val Met
    365                 370                 375
gtg cct gcc cag agt caa gct ggc tct ctg gta tgatgacccc accactcatt 1327
Val Pro Ala Gln Ser Gln Ala Gly Ser Leu Val
380                 385                 390
ggctaaagga tttggggtct ctccttccta taagggtcac ctctagcaca gaggcctgag 1387
tcatgggaaa gagtcacact cctgaccctt agtactctgc ccccacctct ctttactgtg 1447
ggaaaaccat ctcagtaaga cctaagtgtc caggagacag aaggagaaga ggaagtggat 1507
ctggaattgg gaggagcctc cacccacccc tgactcctcc ttatgaagcc agctgctgaa 1567

attagctact caccaagagt gaggggcaga gacttccagt cactgagtct cccaggcccc 1627
cttgatctgt accccacccc tatctaacac caccccttggc tcccactcca gctccctgta 1687
ttgatataac ctgtcaggct ggcttggtta ggttttactg gggcagagga tagggaatct 1747
cttattaaaa ctaacatgaa atatgtgttg ttttcatttg caaatttaaa taaagataca 1807
taatgtttgt atgagataag 1827

<210> 144
<211> 390
<212> PRT
<213> Homo sapiens
<400> 144
Met Ile Ser Leu Pro Gly Pro Leu Val Thr Asn Leu Leu Arg Phe Leu
1               5                   10                  15
Phe Leu Gly Leu Ser Ala Leu Ala Pro Pro Ser Arg Ala Gln Leu Gln
            20                  25                  30
Leu His Leu Pro Ala Asn Arg Leu Gln Ala Val Glu Gly Gly Glu Val
        35                  40                  45
Val Leu Pro Ala Trp Tyr Thr Leu His Gly Glu Val Ser Ser Ser Gln
    50                  55                  60
Pro Trp Glu Val Pro Phe Val Met Trp Phe Phe Lys Gln Lys Glu Lys
65                  70                  75                  80
Glu Asp Gln Val Leu Ser Tyr Ile Asn Gly Val Thr Thr Ser Lys Pro
                85                  90                  95
Gly Val Ser Leu Val Tyr Ser Met Pro Ser Arg Asn Leu Ser Leu Arg
            100                 105                 110
Leu Glu Gly Leu Gln Glu Lys Asp Ser Gly Pro Tyr Ser Cys Ser Val
        115                 120                 125

Asn Val Gln Asp Lys Gln Gly Lys Ser Arg Gly His Ser Ile Lys Thr
    130                 135                 140
Leu Glu Leu Asn Val Leu Val Pro Pro Ala Pro Pro Ser Cys Arg Leu
145                 150                 155                 160
Gln Gly Val Pro His Val Gly Ala Asn Val Thr Leu Ser Cys Gln Ser
                165                 170                 175
Pro Arg Ser Lys Pro Ala Val Gln Tyr Gln Trp Asp Arg Gln Leu Pro
            180                 185                 190
Ser Phe Gln Thr Phe Phe Ala Pro Ala Leu Asp Val Ile Arg Gly Ser
        195                 200                 205
Leu Ser Leu Thr Asn Leu Ser Ser Ser Met Ala Gly Val Tyr Val Cys
    210                 215                 220
Lys Ala His Asn Glu Val Gly Thr Ala Gln Cys Asn Val Thr Leu Glu
225                 230                 235                 240
Val Ser Thr Gly Pro Gly Ala Ala Val Val Ala Gly Ala Val Val Gly
                245                 250                 255
Thr Leu Val Gly Leu Gly Leu Leu Ala Gly Leu Val Leu Leu Tyr His
            260                 265                 270
Arg Arg Gly Lys Ala Leu Glu Glu Pro Ala Asn Asp Ile Lys Glu Asp
        275                 280                 285
Ala Ile Ala Pro Arg Thr Leu Pro Trp Pro Lys Ser Ser Asp Thr Ile
    290                 295                 300
Ser Lys Asn Gly Thr Leu Ser Ser Val Thr Ser Ala Arg Ala Leu Trp
305                 310                 315                 320
Pro Pro His Gly Pro Pro Arg Pro Gly Ala Leu Thr Pro Thr Pro Ser
                325                 330                 335

Leu Ser Ser Gln Ala Leu Pro Ser Pro Arg Leu Pro Thr Thr Asp Gly
            340                 345                 350
Ala His Pro Gln Pro Ile Ser Pro Ile Pro Gly Gly Val Ser Ser Ser
        355                 360                 365
Gly Leu Ser Arg Met Gly Ala Val Pro Val Met Val Pro Ala Gln Ser
    370                 375                 380
Gln Ala Gly Ser Leu Val
385                 390
<210> 145
<211> 3466
<212> DNA
<213> Homo sapiens
<220>
<221> CDS
<222> (84)..(2726)
<400> 145
tgcgggaagc gatgtagtag ctgccaggct gtcccccgcc ctgcccggcc cgagccccgc 60
gggccgccgc cgccaccgcc gcc atg aag aag cag ttc aac cgc atg aag cag 113
Met Lys Lys Gln Phe Asn Arg Met Lys Gln
1               5                   10
ctg gct aac cag acc gtg ggc aga gct gag aaa aca gaa gtc ctt agt 161
Leu Ala Asn Gln Thr Val Gly Arg Ala Glu Lys Thr Glu Val Leu Ser
                15                  20                  25
gaa gat cta tta cag att gag aga cgc ctg gac acg gtg cgg tca ata 209
Glu Asp Leu Leu Gln Ile Glu Arg Arg Leu Asp Thr Val Arg Ser Ile
            30                  35                  40

tgc cac cat tcc cat aag cgc ttg gtg gca tgt ttc cag ggc cag cat 257
Cys His His Ser His Lys Arg Leu Val Ala Cys Phe Gln Gly Gln His
        45                  50                  55
ggc acc gat gcc gag agg aga cac aaa aaa ctg cct ctg aca gct ctt 305
Gly Thr Asp Ala Glu Arg Arg His Lys Lys Leu Pro Leu Thr Ala Leu
    60                  65                  70
gct caa aat atg caa gaa gca tcg act cag ctg gaa gac tct ctc ctg 353
Ala Gln Asn Met Gln Glu Ala Ser Thr Gln Leu Glu Asp Ser Leu Leu
75                  80                  85                  90
ggg aag atg ctg gag acg tgt gga gat gct gag aat cag ctg gct ctc 401
Gly Lys Met Leu Glu Thr Cys Gly Asp Ala Glu Asn Gln Leu Ala Leu
                95                  100                 105
gag ctc tcc cag cac gaa gtc ttt gtt gag aag gag atc gtg gac cct 449
Glu Leu Ser Gln His Glu Val Phe Val Glu Lys Glu Ile Val Asp Pro
            110                 115                 120
ctg tac ggc ata gct gag gtg gag att ccc aac atc cag aag cag agg 497
Leu Tyr Gly Ile Ala Glu Val Glu Ile Pro Asn Ile Gln Lys Gln Arg
        125                 130                 135
aag cag ctt gca aga ttg gtg tta gac tgg gat tca gtc aga gcc agg 545
Lys Gln Leu Ala Arg Leu Val Leu Asp Trp Asp Ser Val Arg Ala Arg
    140                 145                 150
tgg aac caa gct cac aaa tcc tca gga acc aac ttt cag ggg ctt cca 593
Trp Asn Gln Ala His Lys Ser Ser Gly Thr Asn Phe Gln Gly Leu Pro
155                 160                 165                 170
tca aaa ata gat act cta aag gaa gag atg gat gaa gct gga aat aaa 641
Ser Lys Ile Asp Thr Leu Lys Glu Glu Met Asp Glu Ala Gly Asn Lys
                175                 180                 185

gta gaa cag tgc aag gat caa ctt gca gca gac atg tac aac ttt atg 689
Val Glu Gln Cys Lys Asp Gln Leu Ala Ala Asp Met Tyr Asn Phe Met
            190                 195                 200
gcc aaa gaa ggg gag tat ggc aaa ttc ttt gtt acg tta tta gaa gcc 737
Ala Lys Glu Gly Glu Tyr Gly Lys Phe Phe Val Thr Leu Leu Glu Ala
        205                 210                 215
caa gca gat tac cat aga aaa gca tta gca gtc tta gaa aag acc ctc 785
Gln Ala Asp Tyr His Arg Lys Ala Leu Ala Val Leu Glu Lys Thr Leu
    220                 225                 230
ccc gaa atg cga gcc cat caa gat aag tgg gcg gaa aaa cca gcc ttt 833
Pro Glu Met Arg Ala His Gln Asp Lys Trp Ala Glu Lys Pro Ala Phe
235                 240                 245                 250
ggg act ccc cta gaa gaa cac ctg aag agg agc ggg cgc gag att gcg 881
Gly Thr Pro Leu Glu Glu His Leu Lys Arg Ser Gly Arg Glu Ile Ala
                255                 260                 265
ctg ccc att gaa gcc tgt gtc atg ctg ctt ctg gag aca ggc atg aag 929
Leu Pro Ile Glu Ala Cys Val Met Leu Leu Leu Glu Thr Gly Met Lys
            270                 275                 280
gag gag ggc ctt ttc cga att ggg gct ggg gcc tcc aag tta aag aag 977
Glu Glu Gly Leu Phe Arg Ile Gly Ala Gly Ala Ser Lys Leu Lys Lys
        285                 290                 295
ctg aaa gct gct ttg gac tgt tct act tct cac ctg gat gag ttc tat 1025
Leu Lys Ala Ala Leu Asp Cys Ser Thr Ser His Leu Asp Glu Phe Tyr
    300                 305                 310
tca gac ccc cat gct gta gca ggt gct tta aaa tcc tat tta cgg gaa 1073
Ser Asp Pro His Ala Val Ala Gly Ala Leu Lys Ser Tyr Leu Arg Glu

315                 320                 325                 330

ttg cct gaa cct ttg atg act ttt aat ctg tat gaa gaa tgg aca caa 1121

Leu Pro Glu Pro Leu Met Thr Phe Asn Leu Tyr Glu Glu Trp Thr Gln

                335                 340                 345

gtt gca agt gtg cag gat caa gac aaa aaa ctt caa gac ttg tgg aga 1169

Val Ala Ser Val Gln Asp Gln Asp Lys Lys Leu Gln Asp Leu Trp Arg

            350                 355                 360

aca tgt cag aag ttg cca cca caa aat ttt gtt aac ttt aga tat ttg 1217

Thr Cys Gln Lys Leu Pro Pro Gln Asn Phe Val Asn Phe Arg Tyr Leu

        365                 370                 375

atc aag ttc ctt gca aag ctt gct cag acc agc gat gtg aat aaa atg 1265

Ile Lys Phe Leu Ala Lys Leu Ala Gln Thr Ser Asp Val Asn Lys Met

    380                 385                 390

act ccc agc aac att gcg att gtg tta ggc cct aac ttg tta tgg gcc 1313

Thr Pro Ser Asn Ile Ala Ile Val Leu Gly Pro Asn Leu Leu Trp Ala

395                 400                 405                 410

aga aat gaa gga aca ctt gct gaa atg gca gca gcc aca tcc gtc cat 1361

Arg Asn Glu Gly Thr Leu Ala Glu Met Ala Ala Ala Thr Ser Val His

                415                 420                 425

gtg gtt gca gtg att gaa ccc atc att cag cat gcc gac tgg ttc ttc 1409

Val Val Ala Val Ile Glu Pro Ile Ile Gln His Ala Asp Trp Phe Phe

            430                 435                 440

cct gaa gag gtg gaa ttt aat gta tca gaa gca ttt gta cct ctc acc 1457

Pro Glu Glu Val Glu Phe Asn Val Ser Glu Ala Phe Val Pro Leu Thr

        445                 450                 455

acc ccg agt tct aat cac tca ttc cac act gga aac gac tct gac tcg 1505

Thr Pro Ser Ser Asn His Ser Phe His Thr Gly Asn Asp Ser Asp Ser

    460                 465                 470

ggg acc ctg gag agg aag cgg cct gct agc atg gcg gtg atg gaa gga 1553

Gly Thr Leu Glu Arg Lys Arg Pro Ala Ser Met Ala Val Met Glu Gly

475                 480                 485                 490

gac ttg gtg aag aag gaa agc ttt ggt gtg aag ctt atg gac ttc cag 1601

Asp Leu Val Lys Lys Glu Ser Phe Gly Val Lys Leu Met Asp Phe Gln

                495                 500                 505

gcc cac cgg cgg ggt ggc act cta aat aga aag cac ata tcc ccc gct 1649

Ala His Arg Arg Gly Gly Thr Leu Asn Arg Lys His Ile Ser Pro Ala

            510                 515                 520

ttc cag ccg cca ctt ccg ccc aca gat ggc agc acc gtg gtg ccc gct 1697

Phe Gln Pro Pro Leu Pro Pro Thr Asp Gly Ser Thr Val Val Pro Ala

        525                 530                 535

ggc cca gag ccc cct ccc cag agc tct agg gct gaa agc agc tct ggg 1745

Gly Pro Glu Pro Pro Pro Gln Ser Ser Arg Ala Glu Ser Ser Ser Gly

    540                 545                 550

ggt ggg act gtc ccc tct tcc gcg ggc ata ctg gag cag ggg ccg agc 1793

Gly Gly Thr Val Pro Ser Ser Ala Gly Ile Leu Glu Gln Gly Pro Ser

555                 560                 565                 570

cca ggc gac ggc agt cct ccc aaa ccg aag gac cct gta tct gca gct 1841

Pro Gly Asp Gly Ser Pro Pro Lys Pro Lys Asp Pro Val Ser Ala Ala

                575                 580                 585

gtg cca gca cca ggg aga aac aac agt cag ata gca tct ggc caa aat 1889

Val Pro Ala Pro Gly Arg Asn Asn Ser Gln Ile Ala Ser Gly Gln Asn

            590                 595                 600

cag ccc cag gca gct gct ggc tcc cac cag ctc tcc atg ggc caa cct 1937

Gln Pro Gln Ala Ala Ala Gly Ser His Gln Leu Ser Met Gly Gln Pro
        605                 610                 615
cac aat gct gca ggg ccc agc ccg cat aca ctg cgc cga gct gtt aaa 1985
His Asn Ala Ala Gly Pro Ser Pro His Thr Leu Arg Arg Ala Val Lys
    620                 625                 630
aaa ccc gct cca gca ccc ccg aaa ccg ggc aac cca cct cct ggc cac 2033
Lys Pro Ala Pro Ala Pro Pro Lys Pro Gly Asn Pro Pro Pro Gly His
635                 640                 645                 650
ccc ggg ggc cag agt tct tca gga aca tct cag cat cca ccc agt ctg 2081
Pro Gly Gly Gln Ser Ser Ser Gly Thr Ser Gln His Pro Pro Ser Leu
                655                 660                 665
tca cca aag cca ccc acc cga agc ccc tct cct ccc acc cag cac acg 2129
Ser Pro Lys Pro Pro Thr Arg Ser Pro Ser Pro Pro Thr Gln His Thr
            670                 675                 680
ggc cag cct cca ggc cag ccc tcc gcc ccc tcc cag ctc tca gca ccc 2177
Gly Gln Pro Pro Gly Gln Pro Ser Ala Pro Ser Gln Leu Ser Ala Pro
        685                 690                 695
cgg agg tac tcc agc agc ttg tct cca atc caa gct ccc aat cac cca 2225
Arg Arg Tyr Ser Ser Ser Leu Ser Pro Ile Gln Ala Pro Asn His Pro
    700                 705                 710
ccg ccg cag ccc cct acg cag gcc acg cca ctg atg cac acc aaa ccc 2273
Pro Pro Gln Pro Pro Thr Gln Ala Thr Pro Leu Met His Thr Lys Pro
715                 720                 725                 730
aat agc cag ggc cct ccc aac ccc atg gca ttg ccc agt gag cat gga 2321
Asn Ser Gln Gly Pro Pro Asn Pro Met Ala Leu Pro Ser Glu His Gly
                735                 740                 745
ctt gag cag cca tct cac acc cct ccc cag act cca acg ccc ccc agt 2369

Leu Glu Gln Pro Ser His Thr Pro Pro Gln Thr Pro Thr Pro Pro Ser
            750                 755                 760
act ccg ccc cta gga aaa cag aac ccc agt ctg cca gct cct cag acc 2417
Thr Pro Pro Leu Gly Lys Gln Asn Pro Ser Leu Pro Ala Pro Gln Thr
        765                 770                 775
ctg gca ggg ggt aac cct gaa act gca cag cca cat gct gga acc tta 2465
Leu Ala Gly Gly Asn Pro Glu Thr Ala Gln Pro His Ala Gly Thr Leu
    780                 785                 790
ccg aga ccg aga cca gta cca aag cca agg aac cgg ccc agc gtg ccc 2513
Pro Arg Pro Arg Pro Val Pro Lys Pro Arg Asn Arg Pro Ser Val Pro
795                 800                 805                 810
cca ccc ccc caa cct cct ggt gtc cac tca gct ggg gac agc agc ctc 2561
Pro Pro Pro Gln Pro Pro Gly Val His Ser Ala Gly Asp Ser Ser Leu
                815                 820                 825
acc aac aca gca cca aca gct tcc aag ata gta aca gac tcc aat tcc 2609
Thr Asn Thr Ala Pro Thr Ala Ser Lys Ile Val Thr Asp Ser Asn Ser
            830                 835                 840
agg gtt tca gaa ccg cat cgc agc atc ttt cct gaa atg cac tca gac 2657
Arg Val Ser Glu Pro His Arg Ser Ile Phe Pro Glu Met His Ser Asp
        845                 850                 855
tca gcc agc aaa gac gtg cct ggc cgc atc ctg ctg gat ata gac aat 2705
Ser Ala Ser Lys Asp Val Pro Gly Arg Ile Leu Leu Asp Ile Asp Asn
    860                 865                 870
gat acc gag agc act gcc ctg tgaagaaagc cctttcccag ccctccacca 2756
Asp Thr Glu Ser Thr Ala Leu
875                 880

cttccaccct ggcgagtgga gcaggggcag gcgaacctct ttctttgcag accgaacagt 2816

gaaaagcttt cagtggagga caaaggaggg cctcactgtg cgggacctgg ccttctgcac 2876

ggcccaagga gaacctggag gccaccacta aagctgaatg acctgtgtct tgaagaagtt 2936

ggctttcttt acatggaagg gaaatcatgc caaaaaaatc caaaacaaag aagtacctgg 2996

agtggagaga gtattcctgc tgaaacgcgc ataggaagct tttgtccctg ctgttaatgc 3056

gggcagcacc tacagcaact tggaatgagt aagaagcagt gcgttaacta tctatttaat 3116

aaaatgcgct cattatgcaa gtcgcctact ctctgctacc tggacgttca ttcttatgta 3176

ttaggaggga ggctgcgctc cttcagactt gctgcagaat cattttgtat catgtatggt 3236

ctgtgtctcc ccagtcccct cagaaccatg cccatggatg gtgactgctg gctctgtcac 3296

ctcatcaaac tggatgtgac ccatgccgcc tcgttggatt gtcggaatgt agacagaaat 3356

gtactgttct tttttttttt tttaaacaat gtaattgcta cttgataagg accgaacatt 3416

attctagttt catgtttaat ttgaattaaa tatattctgt ggtttatatg 3466

<210> 146

<211> 881

<212> PRT

<213> Homo sapiens

<400> 146

Met Lys Lys Gln Phe Asn Arg Met Lys Gln Leu Ala Asn Gln Thr Val

1               5                   10                  15

Gly Arg Ala Glu Lys Thr Glu Val Leu Ser Glu Asp Leu Leu Gln Ile

            20                  25                  30

Glu Arg Arg Leu Asp Thr Val Arg Ser Ile Cys His His Ser His Lys

        35                  40                  45

Arg Leu Val Ala Cys Phe Gln Gly Gln His Gly Thr Asp Ala Glu Arg

    50                  55                  60

Arg His Lys Lys Leu Pro Leu Thr Ala Leu Ala Gln Asn Met Gln Glu

65                  70                  75                  80
Ala Ser Thr Gln Leu Glu Asp Ser Leu Leu Gly Lys Met Leu Glu Thr
                85                  90                  95
Cys Gly Asp Ala Glu Asn Gln Leu Ala Leu Glu Leu Ser Gln His Glu
            100                 105                 110
Val Phe Val Glu Lys Glu Ile Val Asp Pro Leu Tyr Gly Ile Ala Glu
        115                 120                 125
Val Glu Ile Pro Asn Ile Gln Lys Gln Arg Lys Gln Leu Ala Arg Leu
    130                 135                 140
Val Leu Asp Trp Asp Ser Val Arg Ala Arg Trp Asn Gln Ala His Lys
145                 150                 155                 160
Ser Ser Gly Thr Asn Phe Gln Gly Leu Pro Ser Lys Ile Asp Thr Leu
                165                 170                 175
Lys Glu Glu Met Asp Glu Ala Gly Asn Lys Val Glu Gln Cys Lys Asp
            180                 185                 190
Gln Leu Ala Ala Asp Met Tyr Asn Phe Met Ala Lys Glu Gly Glu Tyr
        195                 200                 205
Gly Lys Phe Phe Val Thr Leu Leu Glu Ala Gln Ala Asp Tyr His Arg
    210                 215                 220
Lys Ala Leu Ala Val Leu Glu Lys Thr Leu Pro Glu Met Arg Ala His
225                 230                 235                 240
Gln Asp Lys Trp Ala Glu Lys Pro Ala Phe Gly Thr Pro Leu Glu Glu
                245                 250                 255
His Leu Lys Arg Ser Gly Arg Glu Ile Ala Leu Pro Ile Glu Ala Cys
            260                 265                 270
Val Met Leu Leu Leu Glu Thr Gly Met Lys Glu Glu Gly Leu Phe Arg
        275                 280                 285

Ile Gly Ala Gly Ala Ser Lys Leu Lys Lys Leu Lys Ala Ala Leu Asp
    290                 295                 300
Cys Ser Thr Ser His Leu Asp Glu Phe Tyr Ser Asp Pro His Ala Val
305                 310                 315                 320
Ala Gly Ala Leu Lys Ser Tyr Leu Arg Glu Leu Pro Glu Pro Leu Met
                325                 330                 335
Thr Phe Asn Leu Tyr Glu Glu Trp Thr Gln Val Ala Ser Val Gln Asp
            340                 345                 350
Gln Asp Lys Lys Leu Gln Asp Leu Trp Arg Thr Cys Gln Lys Leu Pro
        355                 360                 365
Pro Gln Asn Phe Val Asn Phe Arg Tyr Leu Ile Lys Phe Leu Ala Lys
    370                 375                 380
Leu Ala Gln Thr Ser Asp Val Asn Lys Met Thr Pro Ser Asn Ile Ala
385                 390                 395                 400
Ile Val Leu Gly Pro Asn Leu Leu Trp Ala Arg Asn Glu Gly Thr Leu
                405                 410                 415
Ala Glu Met Ala Ala Ala Thr Ser Val His Val Val Ala Val Ile Glu
            420                 425                 430
Pro Ile Ile Gln His Ala Asp Trp Phe Phe Pro Glu Glu Val Glu Phe
        435                 440                 445
Asn Val Ser Glu Ala Phe Val Pro Leu Thr Thr Pro Ser Ser Asn His
    450                 455                 460
Ser Phe His Thr Gly Asn Asp Ser Asp Ser Gly Thr Leu Glu Arg Lys
465                 470                 475                 480
Arg Pro Ala Ser Met Ala Val Met Glu Gly Asp Leu Val Lys Lys Glu
                485                 490                 495

Ser Phe Gly Val Lys Leu Met Asp Phe Gln Ala His Arg Arg Gly Gly
            500                 505                 510
Thr Leu Asn Arg Lys His Ile Ser Pro Ala Phe Gln Pro Pro Leu Pro
        515                 520                 525
Pro Thr Asp Gly Ser Thr Val Val Pro Ala Gly Pro Glu Pro Pro Pro
    530                 535                 540
Gln Ser Ser Arg Ala Glu Ser Ser Ser Gly Gly Gly Thr Val Pro Ser
545                 550                 555                 560
Ser Ala Gly Ile Leu Glu Gln Gly Pro Ser Pro Gly Asp Gly Ser Pro
                565                 570                 575
Pro Lys Pro Lys Asp Pro Val Ser Ala Ala Val Pro Ala Pro Gly Arg
            580                 585                 590
Asn Asn Ser Gln Ile Ala Ser Gly Gln Asn Gln Pro Gln Ala Ala Ala
        595                 600                 605
Gly Ser His Gln Leu Ser Met Gly Gln Pro His Asn Ala Ala Gly Pro
    610                 615                 620
Ser Pro His Thr Leu Arg Arg Ala Val Lys Lys Pro Ala Pro Ala Pro
625                 630                 635                 640
Pro Lys Pro Gly Asn Pro Pro Pro Gly His Pro Gly Gly Gln Ser Ser
                645                 650                 655
Ser Gly Thr Ser Gln His Pro Pro Ser Leu Ser Pro Lys Pro Pro Thr
            660                 665                 670
Arg Ser Pro Ser Pro Pro Thr Gln His Thr Gly Gln Pro Pro Gly Gln
        675                 680                 685
Pro Ser Ala Pro Ser Gln Leu Ser Ala Pro Arg Arg Tyr Ser Ser Ser
    690                 695                 700
Leu Ser Pro Ile Gln Ala Pro Asn His Pro Pro Pro Gln Pro Pro Thr

705                 710                 715                 720

Gln Ala Thr Pro Leu Met His Thr Lys Pro Asn Ser Gln Gly Pro Pro

                725                 730                 735

Asn Pro Met Ala Leu Pro Ser Glu His Gly Leu Glu Gln Pro Ser His

            740                 745                 750

Thr Pro Pro Gln Thr Pro Thr Pro Pro Ser Thr Pro Pro Leu Gly Lys

        755                 760                 765

Gln Asn Pro Ser Leu Pro Ala Pro Gln Thr Leu Ala Gly Gly Asn Pro

    770                 775                 780

Glu Thr Ala Gln Pro His Ala Gly Thr Leu Pro Arg Pro Arg Pro Val

785                 790                 795                 800

Pro Lys Pro Arg Asn Arg Pro Ser Val Pro Pro Pro Pro Gln Pro Pro

                805                 810                 815

Gly Val His Ser Ala Gly Asp Ser Ser Leu Thr Asn Thr Ala Pro Thr

            820                 825                 830

Ala Ser Lys Ile Val Thr Asp Ser Asn Ser Arg Val Ser Glu Pro His

        835                 840                 845

Arg Ser Ile Phe Pro Glu Met His Ser Asp Ser Ala Ser Lys Asp Val

    850                 855                 860

Pro Gly Arg Ile Leu Leu Asp Ile Asp Asn Asp Thr Glu Ser Thr Ala

865                 870                 875                 880

Leu

<210> 147

<211> 3021

<212> DNA

<213> Homo sapiens

<220>

<221> CDS

<222> (140)..(1105)

<400> 147

gagctgccgc tgtcgtcttt gcttcagccg cagtcgccac tggctgcctg aggtgctctt 60

acagcctgtt ccaagtgtgg cttaatccgt ctccaccacc agatctttct ccgtggattc 120

ctctgctaag accgctgcc atg cca gtg acg gta acc cgc acc acc atc aca 172

Met Pro Val Thr Val Thr Arg Thr Thr Ile Thr

1               5                   10

acc acc acg acg tca tct tcg ggc ctg ggg tcc ccc atg atc gtg ggg 220

Thr Thr Thr Thr Ser Ser Ser Gly Leu Gly Ser Pro Met Ile Val Gly

            15                  20                  25

tcc cct cgg gcc ctg aca cag ccc ctg ggt ctc ctt cgc ctg ctg cag 268

Ser Pro Arg Ala Leu Thr Gln Pro Leu Gly Leu Leu Arg Leu Leu Gln

        30                  35                  40

ctg gtg tct acc tgc gtg gcc ttc tcg ctg gtg gct agc gtg ggc gcc 316

Leu Val Ser Thr Cys Val Ala Phe Ser Leu Val Ala Ser Val Gly Ala

    45                  50                  55

tgg acg ggg tcc atg ggc aac tgg tcc atg ttc acc tgg tgc ttc tgc 364

Trp Thr Gly Ser Met Gly Asn Trp Ser Met Phe Thr Trp Cys Phe Cys

60                  65                  70                  75

ttc tcc gtg acc ctg atc atc ctc atc gtg gag ctg tgc ggg ctc cag 412

Phe Ser Val Thr Leu Ile Ile Leu Ile Val Glu Leu Cys Gly Leu Gln

                80                  85                  90

gcc cgc ttc ccc ctg tct tgg cgc aac ttc ccc atc acc ttc gcc tgc 460

Ala Arg Phe Pro Leu Ser Trp Arg Asn Phe Pro Ile Thr Phe Ala Cys

            95                  100                 105
tat gcg ggc ctc ttc tgc ctc tcg gcc tcc atc atc tac ccc acc acc 508
Tyr Ala Gly Leu Phe Cys Leu Ser Ala Ser Ile Ile Tyr Pro Thr Thr
        110                 115                 120
tat gtc cag ttc ctg tcc cac ggc cgt tcg cgg gac cac gcc atc gcc 556
Tyr Val Gln Phe Leu Ser His Gly Arg Ser Arg Asp His Ala Ile Ala
    125                 130                 135
gcc acc ttc ttc tcc tgc atc gcg tgt gtg gct tac gcc acc gaa gtg 604
Ala Thr Phe Phe Ser Cys Ile Ala Cys Val Ala Tyr Ala Thr Glu Val
140                 145                 150                 155
gcc tgg acc cgg gcc cgg ccc ggc gag atc act ggc tat atg gcc acc 652
Ala Trp Thr Arg Ala Arg Pro Gly Glu Ile Thr Gly Tyr Met Ala Thr
                160                 165                 170
gta ccc ggg ctg ctg aag gtg ctg gag acc ttc gtt gcc tgc atc atc 700
Val Pro Gly Leu Leu Lys Val Leu Glu Thr Phe Val Ala Cys Ile Ile
            175                 180                 185
ttc gcg ttc atc agc gac ccc aac ctg tac cag cac cag ccg gcc ctg 748
Phe Ala Phe Ile Ser Asp Pro Asn Leu Tyr Gln His Gln Pro Ala Leu
        190                 195                 200
gag tgg tgc gtg gcg gtg tac gcc atc tgc ttc atc cta gcg gcc atc 796
Glu Trp Cys Val Ala Val Tyr Ala Ile Cys Phe Ile Leu Ala Ala Ile
    205                 210                 215
gcc atc ctg ctg aac ctg ggg gag tgc acc aac gtg cta ccc atc ccc 844
Ala Ile Leu Leu Asn Leu Gly Glu Cys Thr Asn Val Leu Pro Ile Pro
220                 225                 230                 235
ttc ccc agc ttc ctg tcg ggg ctg gcc ttg ctg tct gtc ctc ctc tat 892
Phe Pro Ser Phe Leu Ser Gly Leu Ala Leu Leu Ser Val Leu Leu Tyr

                240                 245                 250

gcc acc gcc ctt gtt ctc tgg ccc ctc tac cag ttc gat gag aag tat 940

Ala Thr Ala Leu Val Leu Trp Pro Leu Tyr Gln Phe Asp Glu Lys Tyr

            255                 260                 265

ggc ggc cag cct cgg cgc tcg aga gat gta agc tgc agc cgc agc cat 988

Gly Gly Gln Pro Arg Arg Ser Arg Asp Val Ser Cys Ser Arg Ser His

        270                 275                 280

gcc tac tac gtg tgt gcc tgg gac cgc cga ctg gct gtg gcc atc ctg 1036

Ala Tyr Tyr Val Cys Ala Trp Asp Arg Arg Leu Ala Val Ala Ile Leu

    285                 290                 295

acg gcc atc aac cta ctg gcg tat gtg gct gac ctg gtg cac tct gcc 1084

Thr Ala Ile Asn Leu Leu Ala Tyr Val Ala Asp Leu Val His Ser Ala

300                 305                 310                 315

cac ctg gtt ttt gtc aag gtc taagactctc ccaagaggct cccgttccct 1135

His Leu Val Phe Val Lys Val

                320

ctccaacctc tttgttcttc ttgcccgagt tttctttatg gagtacttct ttcctccgcc 1195

tttcctctgt tttcctcttc ctgtctcccc tccctcccac ctttttcttt ccttcccaat 1255

tccttgcact ctaaccagtt cttggatgca tcttcttcct tccctttcct cttgctgttt 1315

ccttcctgtg ttgttttgtt gcccacatcc tgttttcacc cctgagctgt ttctcttttt 1375

cttttctttc tttttttttt ttttttaaga cggattctca ctctgtggcc caggctggag 1435

cgcagtggtg cgatctcgac tcactgcaac ccccgcctcc tgggttcaag cgattctcct 1495

gccccagcct cccaagtagc tgggaggaca ggtgtgagct gccgcaccca gcctgtttct 1555

ctttttccac tcttcttttt tctcatctct tttctgggtt gcctgtcggc tttcttatct 1615

gcctgttttg caagcacctt ctcctgtgtc cttgggagcc ctgagacttc tttctctcct 1675

tgcctccacc cacctccaaa ggtgctgagc tcacatccac accccttgca gccgtccatg 1735

ccacagcccc ccaaggggcc ccattgccaa agcatgcctg cccaccctcg ctgtgcctta 1795
gtcagtgtgt acgtgtgtgt gtgtgtgtgt ttggggggtg gggggtgggt agctggggat 1855
tgggccctct ttctcccagt ggaggaaggt gtgcagtgta cttccccttt aaattaaaaa 1915
acatatatat atatatattt ggaggtcagt aatttccaat gggcgggagg cattaagcac 1975
cgaccctggg tccctaggcc ccgcctggca ctcagccttg ccagagattg gctccagaat 2035
ttttgccagg cttacagaac acccactgcc tagaggccat cttaaaggaa gcaggggctg 2095
gatgcctttc atcccaacta ttctctgtgg tatgaaaaag aaaaaaaaaa aaaaagaagg 2155
agtcggggcc gggcgtggtg gctcacgcct gtaatcccag cactttggga gaccaagtca 2215
ggcaatcatc tgaagtcagg agttcaagac cagcctggcc aacatggtga aagcatgtct 2275
ctattaaaaa tacaaaaatt agccgggcgt ggtggcgggc gcctgtaatc ccaggtattt 2335
ggggggactg agacaggaga atcccttcaa cccgggaggt ggaggttgca gtaagtcaag 2395
atggcaccac tgtgctccag cctgggggac agagcgagac tccatctcaa aaaaaaaagg 2455
gaatcggacg aagaaccaca ggatgttgaa gacaactgtc tgaagtattt gtgagggaca 2515
gcgatgtggc cctctgtgtt aagaataacg tgtcctgctt tggcagagag aagaaaatag 2575
ccactgcccg ctttcaaggc aagatcgacc ttttctgttt tgttttgttt ttctttcttt 2635
ttcctggcca tgaggacaaa aattactgag tggcccttaa agagggaagt ttgttttcag 2695
ctgttctctt ttgcccgtag gtgggagggt ggggattgct gcgtcctagc tagaggaatg 2755
gctttgcttg aatgtgtagt gcacacgcac gggtgtttct gtgtgctagt tgcttcttgc 2815
tgctgcttcc tgcttgtctg ggactcacat acataacgtg atatatatat atatatataa 2875
atgtataaat atatatttta ttttttttta aatccttgga gcttctggtt cctatcagtt 2935
cctgttgtta atcgtagaac cgttgtccct tcccccattc ccgtatccat catgttcttt 2995
ttcttttaaa tatcaatata aaaggt 3021

<210> 148

<211> 322

<212> PRT

<213> Homo sapiens

<400> 148

Met Pro Val Thr Val Thr Arg Thr Thr Ile Thr Thr Thr Thr Thr Ser

1               5                   10                  15

Ser Ser Gly Leu Gly Ser Pro Met Ile Val Gly Ser Pro Arg Ala Leu

            20                  25                  30

Thr Gln Pro Leu Gly Leu Leu Arg Leu Leu Gln Leu Val Ser Thr Cys

        35                  40                  45

Val Ala Phe Ser Leu Val Ala Ser Val Gly Ala Trp Thr Gly Ser Met

    50                  55                  60

Gly Asn Trp Ser Met Phe Thr Trp Cys Phe Cys Phe Ser Val Thr Leu

65                  70                  75                  80

Ile Ile Leu Ile Val Glu Leu Cys Gly Leu Gln Ala Arg Phe Pro Leu

                85                  90                  95

Ser Trp Arg Asn Phe Pro Ile Thr Phe Ala Cys Tyr Ala Gly Leu Phe

            100                 105                 110

Cys Leu Ser Ala Ser Ile Ile Tyr Pro Thr Thr Tyr Val Gln Phe Leu

        115                 120                 125

Ser His Gly Arg Ser Arg Asp His Ala Ile Ala Ala Thr Phe Phe Ser

    130                 135                 140

Cys Ile Ala Cys Val Ala Tyr Ala Thr Glu Val Ala Trp Thr Arg Ala

145                 150                 155                 160

Arg Pro Gly Glu Ile Thr Gly Tyr Met Ala Thr Val Pro Gly Leu Leu

                165                 170                 175

Lys Val Leu Glu Thr Phe Val Ala Cys Ile Ile Phe Ala Phe Ile Ser

            180                 185                 190

Asp Pro Asn Leu Tyr Gln His Gln Pro Ala Leu Glu Trp Cys Val Ala

        195                 200                 205

Val Tyr Ala Ile Cys Phe Ile Leu Ala Ala Ile Ala Ile Leu Leu Asn
    210                 215                 220
Leu Gly Glu Cys Thr Asn Val Leu Pro Ile Pro Phe Pro Ser Phe Leu
225                 230                 235                 240
Ser Gly Leu Ala Leu Leu Ser Val Leu Leu Tyr Ala Thr Ala Leu Val
                245                 250                 255
Leu Trp Pro Leu Tyr Gln Phe Asp Glu Lys Tyr Gly Gly Gln Pro Arg
            260                 265                 270
Arg Ser Arg Asp Val Ser Cys Ser Arg Ser His Ala Tyr Tyr Val Cys
        275                 280                 285
Ala Trp Asp Arg Arg Leu Ala Val Ala Ile Leu Thr Ala Ile Asn Leu
    290                 295                 300
Leu Ala Tyr Val Ala Asp Leu Val His Ser Ala His Leu Val Phe Val
305                 310                 315                 320
Lys Val
<210> 149
<211> 4409
<212> DNA
<213> Homo sapiens
<220>
<221> CDS
<222> (39)..(2027)
<400> 149
ggtgtcagga tcgcagaaag tatgtccctt ctctcacc atg agc tgg ctc tcc agt 56
Met Ser Trp Leu Ser Ser
1               5

tcc cag gga gtg gta cta aca gcc tac cac ccc agc ggc aag gac cag 104
Ser Gln Gly Val Val Leu Thr Ala Tyr His Pro Ser Gly Lys Asp Gln
            10                  15                  20
gcc gtc ggg aac agc cat gca aag gca ggg gag gaa gcc acc tcg agt 152
Ala Val Gly Asn Ser His Ala Lys Ala Gly Glu Glu Ala Thr Ser Ser
        25                  30                  35
cgc aga tat ggc cag tac act atg aac cag gaa agc acc acc atc aaa 200
Arg Arg Tyr Gly Gln Tyr Thr Met Asn Gln Glu Ser Thr Thr Ile Lys
    40                  45                  50
gtt atg gag aag cct cca ttt gat cga tca att tcc cag gat tct ttg 248
Val Met Glu Lys Pro Pro Phe Asp Arg Ser Ile Ser Gln Asp Ser Leu
55                  60                  65                  70
gat gaa cta tct atg gaa gac tat tgg ata gaa cta gaa aac atc aag 296
Asp Glu Leu Ser Met Glu Asp Tyr Trp Ile Glu Leu Glu Asn Ile Lys
                75                  80                  85
aaa tct agt gaa aac agc caa gaa gat caa gag gtg gtt gtt gtc aaa 344
Lys Ser Ser Glu Asn Ser Gln Glu Asp Gln Glu Val Val Val Val Lys
            90                  95                  100
gag cct gat gag gga gaa ttg gaa gaa gag tgg ctt aaa gag gcc ggt 392
Glu Pro Asp Glu Gly Glu Leu Glu Glu Glu Trp Leu Lys Glu Ala Gly
        105                 110                 115
tta tcc aat ctc ttc gga gag tct gct gga gat cca cag gaa agc att 440
Leu Ser Asn Leu Phe Gly Glu Ser Ala Gly Asp Pro Gln Glu Ser Ile
    120                 125                 130
gtg ttt tta tca aca ttg acg cgg acc cag gca gca gca gtt cag aag 488
Val Phe Leu Ser Thr Leu Thr Arg Thr Gln Ala Ala Ala Val Gln Lys

135                 140                 145                 150
cga gta gag acg gtc tcc cag acc ttg agg aaa aaa aac aaa cag tac 536
Arg Val Glu Thr Val Ser Gln Thr Leu Arg Lys Lys Asn Lys Gln Tyr
                155                 160                 165
cag att cct gac gtc aga gac ata ttt gct caa cag aga gaa tca aaa 584
Gln Ile Pro Asp Val Arg Asp Ile Phe Ala Gln Gln Arg Glu Ser Lys
            170                 175                 180
gaa aca gct cca ggt ggc act gaa tcg cag tca ctt aga aca aat gaa 632
Glu Thr Ala Pro Gly Gly Thr Glu Ser Gln Ser Leu Arg Thr Asn Glu
        185                 190                 195
aac aaa tac caa gga aga gat gac gag gca tct aac ctt gtt ggt gaa 680
Asn Lys Tyr Gln Gly Arg Asp Asp Glu Ala Ser Asn Leu Val Gly Glu
    200                 205                 210
gag aag ctg atc cca cct gag gag acg cct gcc cct gaa aca gac atc 728
Glu Lys Leu Ile Pro Pro Glu Glu Thr Pro Ala Pro Glu Thr Asp Ile
215                 220                 225                 230
aac ctg gag gta tca ttt gcc gag caa gca ctc aat cag aaa gag agc 776
Asn Leu Glu Val Ser Phe Ala Glu Gln Ala Leu Asn Gln Lys Glu Ser
                235                 240                 245
tcc aag gag aaa atc cag aag agc aaa ggc gat gat gcc aca tta cct 824
Ser Lys Glu Lys Ile Gln Lys Ser Lys Gly Asp Asp Ala Thr Leu Pro
            250                 255                 260
agt ttc aga ttg cca aaa gac aaa acg ggt acc aca agg att ggt gac 872
Ser Phe Arg Leu Pro Lys Asp Lys Thr Gly Thr Thr Arg Ile Gly Asp
        265                 270                 275
ctc gca ccc cag gac atg aag aaa gtt tgc cat tta gcc cta att gag 920
Leu Ala Pro Gln Asp Met Lys Lys Val Cys His Leu Ala Leu Ile Glu

    280                 285                 290

ctg act gcc ctc tat gat gta ttg ggt att gag ctg aaa caa caa aaa 968

Leu Thr Ala Leu Tyr Asp Val Leu Gly Ile Glu Leu Lys Gln Gln Lys

295                 300                 305                 310

gct gtg aaa atc aaa aca aaa gat tct ggt ctt ttt tgc gtt cca ttg 1016

Ala Val Lys Ile Lys Thr Lys Asp Ser Gly Leu Phe Cys Val Pro Leu

                315                 320                 325

aca gcg cta tta gaa caa gat cag agg aaa gta cca gga atg cga ata 1064

Thr Ala Leu Leu Glu Gln Asp Gln Arg Lys Val Pro Gly Met Arg Ile

            330                 335                 340

ccc ttg atc ttt caa aaa ctg att tct cga att gaa gag aga ggt ttg 1112

Pro Leu Ile Phe Gln Lys Leu Ile Ser Arg Ile Glu Glu Arg Gly Leu

        345                 350                 355

gaa aca gaa ggc ctc tta cgg atc cct gga gct gcc att aga atc aag 1160

Glu Thr Glu Gly Leu Leu Arg Ile Pro Gly Ala Ala Ile Arg Ile Lys

    360                 365                 370

aat ctt tgc caa gaa cta gaa gca aag ttt tat gaa ggg act ttt aat 1208

Asn Leu Cys Gln Glu Leu Glu Ala Lys Phe Tyr Glu Gly Thr Phe Asn

375                 380                 385                 390

tgg gaa agt gtc aaa cag cat gat gcc gcc agc ctg ctg aag ctc ttc 1256

Trp Glu Ser Val Lys Gln His Asp Ala Ala Ser Leu Leu Lys Leu Phe

                395                 400                 405

att cgg gag ttg ccc cag cca ctg ctc agt gtg gag tat ctc aaa gcc 1304

Ile Arg Glu Leu Pro Gln Pro Leu Leu Ser Val Glu Tyr Leu Lys Ala

            410                 415                 420

ttt cag gct gtc cag aat ctt cca acc aag aag cag caa cta cag gct 1352

Phe Gln Ala Val Gln Asn Leu Pro Thr Lys Lys Gln Gln Leu Gln Ala
        425                 430                 435
ttg aac ctt ctt ggc atc ctc cta cct gat gca aac agg gac aca ctg 1400
Leu Asn Leu Leu Gly Ile Leu Leu Pro Asp Ala Asn Arg Asp Thr Leu
    440                 445                 450
aag gcc ctt ctt gaa ttt ctc caa aga gta ata gat aat aaa gaa aaa 1448
Lys Ala Leu Leu Glu Phe Leu Gln Arg Val Ile Asp Asn Lys Glu Lys
455                 460                 465                 470
aat aaa atg aca gtc atg aat gta gca atg gtc atg gcc ccg aat ctc 1496
Asn Lys Met Thr Val Met Asn Val Ala Met Val Met Ala Pro Asn Leu
                475                 480                 485
ttt atg tgt cat gca ttg gga ttg aag tcc agt gaa cag cga gaa ttt 1544
Phe Met Cys His Ala Leu Gly Leu Lys Ser Ser Glu Gln Arg Glu Phe
            490                 495                 500
gta atg gca gct ggg aca gca aat acc atg cac tta ttg att aag tac 1592
Val Met Ala Ala Gly Thr Ala Asn Thr Met His Leu Leu Ile Lys Tyr
        505                 510                 515
caa aaa ctt ctg tgg aca att ccc aag ttt att gta aac caa gtg agg 1640
Gln Lys Leu Leu Trp Thr Ile Pro Lys Phe Ile Val Asn Gln Val Arg
    520                 525                 530
aag caa aac acg gaa aat cat aaa aag gat aaa aga gcc atg aag aaa 1688
Lys Gln Asn Thr Glu Asn His Lys Lys Asp Lys Arg Ala Met Lys Lys
535                 540                 545                 550
ttg ctg aag aaa atg gct tat gac cga gaa aaa tat gaa aag caa gat 1736
Leu Leu Lys Lys Met Ala Tyr Asp Arg Glu Lys Tyr Glu Lys Gln Asp
                555                 560                 565
aag agt aca aat gat gct gac gtt cct cag gga gtg att cga gtg caa 1784

Lys Ser Thr Asn Asp Ala Asp Val Pro Gln Gly Val Ile Arg Val Gln

            570                 575                 580

gct ccc cat ctt tcg aaa gtt tcc atg gca ata cag cta act gaa gaa 1832

Ala Pro His Leu Ser Lys Val Ser Met Ala Ile Gln Leu Thr Glu Glu

        585                 590                 595

cta aaa gcc agt gat gta ctt gcc agg ttt ctc agc caa gaa agt ggg 1880

Leu Lys Ala Ser Asp Val Leu Ala Arg Phe Leu Ser Gln Glu Ser Gly

    600                 605                 610

gtt gcc cag act ctc aag aaa gga gaa gtt ttt ttg tat gaa att gga 1928

Val Ala Gln Thr Leu Lys Lys Gly Glu Val Phe Leu Tyr Glu Ile Gly

615                 620                 625                 630

gga aat att ggg gaa cgc tgc ctt gat gat gac act tac atg aag gat 1976

Gly Asn Ile Gly Glu Arg Cys Leu Asp Asp Asp Thr Tyr Met Lys Asp

                635                 640                 645

tta tat cag ctt aac cca aat gct gag tgg gtt ata aag tca aag cca 2024

Leu Tyr Gln Leu Asn Pro Asn Ala Glu Trp Val Ile Lys Ser Lys Pro

            650                 655                 660

ttg tagaagactt aacaagctgc agataaccat gtggacttct gtcataattc 2077

Leu

ttgctgagtc aagagtgtaa ataaaagaaa tggcaggact catattattc agttgtaccc 2137

aagtatttaa aaatgactct cttaagcctt aaaaagtcat agatttgtgc tgctgccaga 2197

attatattaa ttattattaa tgttattatt agaaaaaaaa tttctggagt gagagtaaag 2257

aggcttaatt agtttgtggg cagttttcat atgctctgtg aaatgtgtcc agatgtgaca 2317

tagttttttt tttttttaat atgtggaaat gtcttctctt cccattcttt tctcctaaaa 2377

tcatatatac tgtaatatat gctctctcac ctctattacc tcctcacatc taccctttcc 2437

cagttaggtt tgctttttga ccaaaaagat aacaaatacc aggtatggca agttgtgaag 2497

acagcacatt aaaacatacc taatttcaca gtattcctgt cacgacagaa tgttagtatt 2557
catctctttg aatcatttgc tcaaataata acattccacc ttttcctgct gtatcacagg 2617
aagtgatttg catttttttt cagttcatct gacttatgtt cacagaaccg tatcagcgac 2677
caagaaaata ggactgtcag aagctgccag ttattactga accattaaat acttatatac 2737
taagaataaa taaaatatac ccatgtgaaa taataattgg attatggata acaagagagt 2797
gaaagccaaa gcactttctg tctactgtac tcttctaaat ggaattttaa aagtcatagc 2857
tggctttacg tgttgtcatt attagcatta taaatatgca tgatagtata atccagtaat 2917
ggttgaagaa tgtattttac ttaaagaggg attttttttt ttaagtcctg aataagtcta 2977
ctggaagaat tattcttctg ggtgaaaagc ttttgtttgt gttcttattt taaataatcg 3037
gagtcaattt attaaaatgt tcttgaaagt actattccca gggattttaa tgcacaaacc 3097
atattgtgac aagagatgag cctctgtact gtaaataaga aatgaagtag agaaatgtta 3157
aatattttat gagtttagaa tatagtaaat aaaaggtgat gtaaatgaat gctgcacaaa 3217
cggtgttcat gatactttta gtagtacttt aggaaaaact acacattctc agaagctctt 3277
gatgtctcta atgaaggggg ggaatgctgt taatgagaac agtcataaat ttttagcata 3337
taattacaag aacagcctgt ggatatgatc acttaaatga ttttgtggtg attcgtgcca 3397
ttgctttttt atttaaaaga aaattttgta attaaatgcc tttttctaaa ttatcttctc 3457
ttggaatcat tacttttaat cctatgtgtt tatgagtatt tttgcttttt ttttattaat 3517
attgagaaat ggactttttt gttattaaaa gtcacctcta ttttctattt tctttgtaat 3577
ttttaaagta ggaagatgtc agagatgtaa atatgttttc gtctttagtt tttttccttt 3637
tacaaatttt tattcttcag gattttcaaa atacagttta gtctgtttct ttgacaatat 3697
gtattaattt cccaattagc aaaatggtac ttattagtgg gttgaaaaca attaataata 3757
taaaagaaaa attaagtgct taaaacattt taggagtata caacttcaaa aaaaaagata 3817
gcagtgagga taatgattta agtaaaaggt tgtctgaagc atatgccaac taaatttcgc 3877
aacgtttgct acctacctga aaaggagggt caggagggag acacaacata tttttgatca 3937
tgaaaaagta tcttaatttt aaaaaagtga aaatgccatt ttattttgaa tcccttttag 3997
aactcacgac ccaagttcat caatgttgaa taatatcacg tttaaataac aaaaaaatat 4057
ggactttaaa aaatctcaaa ttttttagag acagggtctt gctctgtttt ccagattgga 4117

gtgcagcagt gcattcgcgg ctaactgcag cctcaagcac tggggctcaa gcaaacctcc 4177

tgcctcagcc tcgtgagtag ctaggaccac aggtgcatgc caccatgcct ggctctaaag 4237

agaaaaaaaa cttgatacca tagagccttg aatataaata tcctgatgtt aacctactgc 4297

ttttgctgtg attttttttc cttagtgagt tttaaatctc aggctagatt tttatttgtt 4357

tttctgtgtg tgtatgagac aaaataaaaa taaatatatt tgccttgagt tt 4409

<210> 150

<211> 663

<212> PRT

<213> Homo sapiens

<400> 150

Met Ser Trp Leu Ser Ser Ser Gln Gly Val Val Leu Thr Ala Tyr His

1               5                   10                  15

Pro Ser Gly Lys Asp Gln Ala Val Gly Asn Ser His Ala Lys Ala Gly

            20                  25                  30

Glu Glu Ala Thr Ser Ser Arg Arg Tyr Gly Gln Tyr Thr Met Asn Gln

        35                  40                  45

Glu Ser Thr Thr Ile Lys Val Met Glu Lys Pro Pro Phe Asp Arg Ser

    50                  55                  60

Ile Ser Gln Asp Ser Leu Asp Glu Leu Ser Met Glu Asp Tyr Trp Ile

65                  70                  75                  80

Glu Leu Glu Asn Ile Lys Lys Ser Ser Glu Asn Ser Gln Glu Asp Gln

                85                  90                  95

Glu Val Val Val Val Lys Glu Pro Asp Glu Gly Glu Leu Glu Glu Glu

            100                 105                 110

Trp Leu Lys Glu Ala Gly Leu Ser Asn Leu Phe Gly Glu Ser Ala Gly

        115                 120                 125

Asp Pro Gln Glu Ser Ile Val Phe Leu Ser Thr Leu Thr Arg Thr Gln
    130                 135                 140
Ala Ala Ala Val Gln Lys Arg Val Glu Thr Val Ser Gln Thr Leu Arg
145                 150                 155                 160
Lys Lys Asn Lys Gln Tyr Gln Ile Pro Asp Val Arg Asp Ile Phe Ala
                165                 170                 175
Gln Gln Arg Glu Ser Lys Glu Thr Ala Pro Gly Gly Thr Glu Ser Gln
            180                 185                 190
Ser Leu Arg Thr Asn Glu Asn Lys Tyr Gln Gly Arg Asp Asp Glu Ala
        195                 200                 205
Ser Asn Leu Val Gly Glu Glu Lys Leu Ile Pro Pro Glu Glu Thr Pro
    210                 215                 220
Ala Pro Glu Thr Asp Ile Asn Leu Glu Val Ser Phe Ala Glu Gln Ala
225                 230                 235                 240
Leu Asn Gln Lys Glu Ser Ser Lys Glu Lys Ile Gln Lys Ser Lys Gly
                245                 250                 255
Asp Asp Ala Thr Leu Pro Ser Phe Arg Leu Pro Lys Asp Lys Thr Gly
            260                 265                 270
Thr Thr Arg Ile Gly Asp Leu Ala Pro Gln Asp Met Lys Lys Val Cys
        275                 280                 285
His Leu Ala Leu Ile Glu Leu Thr Ala Leu Tyr Asp Val Leu Gly Ile
    290                 295                 300
Glu Leu Lys Gln Gln Lys Ala Val Lys Ile Lys Thr Lys Asp Ser Gly
305                 310                 315                 320
Leu Phe Cys Val Pro Leu Thr Ala Leu Leu Glu Gln Asp Gln Arg Lys
                325                 330                 335

Val Pro Gly Met Arg Ile Pro Leu Ile Phe Gln Lys Leu Ile Ser Arg
            340                 345                 350
Ile Glu Glu Arg Gly Leu Glu Thr Glu Gly Leu Leu Arg Ile Pro Gly
        355                 360                 365
Ala Ala Ile Arg Ile Lys Asn Leu Cys Gln Glu Leu Glu Ala Lys Phe
    370                 375                 380
Tyr Glu Gly Thr Phe Asn Trp Glu Ser Val Lys Gln His Asp Ala Ala
385                 390                 395                 400
Ser Leu Leu Lys Leu Phe Ile Arg Glu Leu Pro Gln Pro Leu Leu Ser
                405                 410                 415
Val Glu Tyr Leu Lys Ala Phe Gln Ala Val Gln Asn Leu Pro Thr Lys
            420                 425                 430
Lys Gln Gln Leu Gln Ala Leu Asn Leu Leu Gly Ile Leu Leu Pro Asp
        435                 440                 445
Ala Asn Arg Asp Thr Leu Lys Ala Leu Leu Glu Phe Leu Gln Arg Val
    450                 455                 460
Ile Asp Asn Lys Glu Lys Asn Lys Met Thr Val Met Asn Val Ala Met
465                 470                 475                 480
Val Met Ala Pro Asn Leu Phe Met Cys His Ala Leu Gly Leu Lys Ser
                485                 490                 495
Ser Glu Gln Arg Glu Phe Val Met Ala Ala Gly Thr Ala Asn Thr Met
            500                 505                 510
His Leu Leu Ile Lys Tyr Gln Lys Leu Leu Trp Thr Ile Pro Lys Phe
        515                 520                 525
Ile Val Asn Gln Val Arg Lys Gln Asn Thr Glu Asn His Lys Lys Asp
    530                 535                 540
Lys Arg Ala Met Lys Lys Leu Leu Lys Lys Met Ala Tyr Asp Arg Glu

545                    550                    555                    560

Lys Tyr Glu Lys Gln Asp Lys Ser Thr Asn Asp Ala Asp Val Pro Gln

                565                    570                    575

Gly Val Ile Arg Val Gln Ala Pro His Leu Ser Lys Val Ser Met Ala

            580                    585                    590

Ile Gln Leu Thr Glu Glu Leu Lys Ala Ser Asp Val Leu Ala Arg Phe

        595                    600                    605

Leu Ser Gln Glu Ser Gly Val Ala Gln Thr Leu Lys Lys Gly Glu Val

    610                    615                    620

Phe Leu Tyr Glu Ile Gly Gly Asn Ile Gly Glu Arg Cys Leu Asp Asp

625                    630                    635                    640

Asp Thr Tyr Met Lys Asp Leu Tyr Gln Leu Asn Pro Asn Ala Glu Trp

                645                    650                    655

Val Ile Lys Ser Lys Pro Leu

            660

<210> 151

<211> 4490

<212> DNA

<213> Homo sapiens

<220>

<221> CDS

<222> (3)..(2591)

<400> 151

aa atg ggc tac atc ccc tcc tcc tat gtg cag ccc ttg aac tac cgg     47

   Met Gly Tyr Ile Pro Ser Ser Tyr Val Gln Pro Leu Asn Tyr Arg

   1               5                   10                  15

aac tca aca ctg agt gac agc ggt atg att gat aat ctt cca gac agc 95
Asn Ser Thr Leu Ser Asp Ser Gly Met Ile Asp Asn Leu Pro Asp Ser
                20                  25                  30
cca gac gag gta gcc aag gag ctg gag ctg ctc ggg gga tgg aca gat 143
Pro Asp Glu Val Ala Lys Glu Leu Glu Leu Leu Gly Gly Trp Thr Asp
            35                  40                  45
gac aaa aaa gta cca ggc aga atg tac agt aat aac cct ttc tgg aat 191
Asp Lys Lys Val Pro Gly Arg Met Tyr Ser Asn Asn Pro Phe Trp Asn
        50                  55                  60
ggg gtc cag acc aat cca ttt ctg aat ggg aac gtg ccc gtc atg ccc 239
Gly Val Gln Thr Asn Pro Phe Leu Asn Gly Asn Val Pro Val Met Pro
    65                  70                  75
agc ctg gat gag ctg aat ccc aaa agt act gtg gat ttg ctc ctt ttt 287
Ser Leu Asp Glu Leu Asn Pro Lys Ser Thr Val Asp Leu Leu Leu Phe
80                  85                  90                  95
gac gca ggt aca tcc tcc ttc acc gaa tcc agc tca gcc acc acg aat 335
Asp Ala Gly Thr Ser Ser Phe Thr Glu Ser Ser Ser Ala Thr Thr Asn
                100                 105                 110
agc act ggc aac atc ttc gat gag ctt cca gtc aca aac ggg ctc cac 383
Ser Thr Gly Asn Ile Phe Asp Glu Leu Pro Val Thr Asn Gly Leu His
            115                 120                 125
gca gag ccg ccg gtc agg cgg gac aac ccc ttc ttc aga agc aag cgc 431
Ala Glu Pro Pro Val Arg Arg Asp Asn Pro Phe Phe Arg Ser Lys Arg
        130                 135                 140
tcc tac agt ctc tcg gaa ctc tcc gtc ctc caa gcc aag tcc gac gct 479
Ser Tyr Ser Leu Ser Glu Leu Ser Val Leu Gln Ala Lys Ser Asp Ala

    145                 150                 155

ccc aca tcg tcg agt ttc ttc acc ggc ttg aaa tca cct gcc ccc gag 527

Pro Thr Ser Ser Ser Phe Phe Thr Gly Leu Lys Ser Pro Ala Pro Glu

160                 165                 170                 175

caa ttt cag agc cgg gag gat ttt cga act gcc tgg cta aac cac agg 575

Gln Phe Gln Ser Arg Glu Asp Phe Arg Thr Ala Trp Leu Asn His Arg

                180                 185                 190

aag ctg gcc cgg tct tgc cac gac ctg gac ttg ctt ggc caa agc cct 623

Lys Leu Ala Arg Ser Cys His Asp Leu Asp Leu Leu Gly Gln Ser Pro

            195                 200                 205

ggt tgg ggc cag acc caa gcc gtg gag aca aac atc gtg tgc aag ctg 671

Gly Trp Gly Gln Thr Gln Ala Val Glu Thr Asn Ile Val Cys Lys Leu

        210                 215                 220

gat agc tcc ggg ggt gct gtc cag ctt cct gac acc agc atc agc atc 719

Asp Ser Ser Gly Gly Ala Val Gln Leu Pro Asp Thr Ser Ile Ser Ile

    225                 230                 235

cac gtg ccc gag ggc cac gtc gcc cct ggg gag acc cag cag atc tcc 767

His Val Pro Glu Gly His Val Ala Pro Gly Glu Thr Gln Gln Ile Ser

240                 245                 250                 255

atg aaa gcc ctg ctg gac ccc ccg ctg gag ctc aac agt gac agg tcc 815

Met Lys Ala Leu Leu Asp Pro Pro Leu Glu Leu Asn Ser Asp Arg Ser

                260                 265                 270

tgc agc atc agc cct gtg ctg gag gtc aag ctg agc aac ctg gag gtg 863

Cys Ser Ile Ser Pro Val Leu Glu Val Lys Leu Ser Asn Leu Glu Val

            275                 280                 285

aaa acc tct atc atc ttg gag atg aaa gtg tca gcc gag ata aaa aat 911

Lys Thr Ser Ile Ile Leu Glu Met Lys Val Ser Ala Glu Ile Lys Asn

        290                 295                 300

gac ctt ttt agc aaa agc aca gtg ggc ctc cag tgc ctg agg agc gac 959

Asp Leu Phe Ser Lys Ser Thr Val Gly Leu Gln Cys Leu Arg Ser Asp

    305                 310                 315

tcg aag gaa ggg cca tat gtc tcc gtc ccg ctc aac tgc agc tgt ggg 1007

Ser Lys Glu Gly Pro Tyr Val Ser Val Pro Leu Asn Cys Ser Cys Gly

320                 325                 330                 335

gac acg gtc cag gca cag ctg cac aac ctg gag ccc tgt atg tac gtg 1055

Asp Thr Val Gln Ala Gln Leu His Asn Leu Glu Pro Cys Met Tyr Val

                340                 345                 350

gct gtc gtg gcc cat ggc cca agc atc ctc tac cct tcc acc gtg tgg 1103

Ala Val Val Ala His Gly Pro Ser Ile Leu Tyr Pro Ser Thr Val Trp

            355                 360                 365

gac ttc atc aat aaa aaa gtc aca gtg ggt ctc tac ggc cct aaa cac 1151

Asp Phe Ile Asn Lys Lys Val Thr Val Gly Leu Tyr Gly Pro Lys His

        370                 375                 380

atc cac cca tcc ttc aag acg gta gtg acc att ttt ggg cat gac tgt 1199

Ile His Pro Ser Phe Lys Thr Val Val Thr Ile Phe Gly His Asp Cys

    385                 390                 395

gcc cca aag acg ctc ctg gtc agc gag gtc aca cgc cag gca ccc aac 1247

Ala Pro Lys Thr Leu Leu Val Ser Glu Val Thr Arg Gln Ala Pro Asn

400                 405                 410                 415

cct gcc ccg gtg gcc ctg cag ctg tgg ggg aag cac cag ttc gtt ttg 1295

Pro Ala Pro Val Ala Leu Gln Leu Trp Gly Lys His Gln Phe Val Leu

                420                 425                 430

tcc agg ccc cag gat ctc aag gtc tgt atg ttt tcc aat atg acg aat 1343

Ser Arg Pro Gln Asp Leu Lys Val Cys Met Phe Ser Asn Met Thr Asn
            435                 440                 445
tac gag gtc aaa gcc agc gag cag gcc aaa gtg gtg cga gga ttc cag 1391
Tyr Glu Val Lys Ala Ser Glu Gln Ala Lys Val Val Arg Gly Phe Gln
        450                 455                 460
ctg aag ctg ggc aag gtg agc cgc ctg atc ttc ccc atc acc tcc cag 1439
Leu Lys Leu Gly Lys Val Ser Arg Leu Ile Phe Pro Ile Thr Ser Gln
    465                 470                 475
aac ccc aac gag ctc tct gac ttc acg ctg cgg gtt cag gtg aag gac 1487
Asn Pro Asn Glu Leu Ser Asp Phe Thr Leu Arg Val Gln Val Lys Asp
480                 485                 490                 495
gac cag gag gcc atc ctc acc cag ttt tgt gtc cag act cct cag cca 1535
Asp Gln Glu Ala Ile Leu Thr Gln Phe Cys Val Gln Thr Pro Gln Pro
                500                 505                 510
ccc cct aaa agt gcc atc aag cct tcc ggg caa agg agg ttt ctc aag 1583
Pro Pro Lys Ser Ala Ile Lys Pro Ser Gly Gln Arg Arg Phe Leu Lys
            515                 520                 525
aag aac gaa gtc ggg aaa atc atc ctg tcc ccg ttt gcc acc act aca 1631
Lys Asn Glu Val Gly Lys Ile Ile Leu Ser Pro Phe Ala Thr Thr Thr
        530                 535                 540
aag tac ccg act ttc cag gac cgc ccg gtg tcc agc ctc aag ttt ggt 1679
Lys Tyr Pro Thr Phe Gln Asp Arg Pro Val Ser Ser Leu Lys Phe Gly
    545                 550                 555
aag ttg ctc aag act gtg gtg cgg cag aac aag aac cac tac ctg ctg 1727
Lys Leu Leu Lys Thr Val Val Arg Gln Asn Lys Asn His Tyr Leu Leu
560                 565                 570                 575
gag tac aag aag ggc gac ggg atc gcc ctg ctc agc gag gag cgg gtc 1775

Glu Tyr Lys Lys Gly Asp Gly Ile Ala Leu Leu Ser Glu Glu Arg Val
                580                 585                 590
agg ctc cgg ggc cag ctg tgg acc aag gag tgg tac atc ggc tac tac 1823
Arg Leu Arg Gly Gln Leu Trp Thr Lys Glu Trp Tyr Ile Gly Tyr Tyr
            595                 600                 605
cag ggc agg gtg ggc ctc gtg cac acc aag aac gtg ctg gtg gtc ggc 1871
Gln Gly Arg Val Gly Leu Val His Thr Lys Asn Val Leu Val Val Gly
        610                 615                 620
agg gcc cgg ccc agc ctg tgc tcg ggc ccc gag ctg agc acc tcg gtg 1919
Arg Ala Arg Pro Ser Leu Cys Ser Gly Pro Glu Leu Ser Thr Ser Val
    625                 630                 635
ctg ctg gag cag atc ctg cgg ccc tgc aaa ttc ctc acg tac atc tat 1967
Leu Leu Glu Gln Ile Leu Arg Pro Cys Lys Phe Leu Thr Tyr Ile Tyr
640                 645                 650                 655
gcc tcc gtg agg acc ctg ctc atg gag aac atc agc agc tgg cgc tcc 2015
Ala Ser Val Arg Thr Leu Leu Met Glu Asn Ile Ser Ser Trp Arg Ser
                660                 665                 670
ttc gct gac gcc ctg ggc tac gtg aac ctg ccg ctc acc ttt ttc tgc 2063
Phe Ala Asp Ala Leu Gly Tyr Val Asn Leu Pro Leu Thr Phe Phe Cys
            675                 680                 685
cgg gca gag ctg gat agt gag ccc gag cgg gtg gcg tcc gtc ctg gaa 2111
Arg Ala Glu Leu Asp Ser Glu Pro Glu Arg Val Ala Ser Val Leu Glu
        690                 695                 700
aag ctg aag gag gac tgt aac aac act gag aac aaa gaa cgg aag tcc 2159
Lys Leu Lys Glu Asp Cys Asn Asn Thr Glu Asn Lys Glu Arg Lys Ser
    705                 710                 715

ttc cag aag gag ctt gtg atg gcc cta ctg aag atg gac tgc cag ggc 2207
Phe Gln Lys Glu Leu Val Met Ala Leu Leu Lys Met Asp Cys Gln Gly
720                 725                 730                 735
ctg gtg gtc aga ctc atc cag gac ttt gtg ctc ctg acc acg gct gta 2255
Leu Val Val Arg Leu Ile Gln Asp Phe Val Leu Leu Thr Thr Ala Val
                740                 745                 750
gag gtg gcc cag cgc tgg cgg gag ctg gct gag aag ctg gcc aag gtc 2303
Glu Val Ala Gln Arg Trp Arg Glu Leu Ala Glu Lys Leu Ala Lys Val
            755                 760                 765
tcc aag cag cag atg gac gcc tac gag tct ccc cac cgg gac agg aac 2351
Ser Lys Gln Gln Met Asp Ala Tyr Glu Ser Pro His Arg Asp Arg Asn
        770                 775                 780
ggg gtt gtg gac agc gag gcc atg tgg aag cct gcg tat gac ttc tta 2399
Gly Val Val Asp Ser Glu Ala Met Trp Lys Pro Ala Tyr Asp Phe Leu
    785                 790                 795
ctc acc tgg agc cat cag atc ggg gac agc tac cgg gat gtc atc cag 2447
Leu Thr Trp Ser His Gln Ile Gly Asp Ser Tyr Arg Asp Val Ile Gln
800                 805                 810                 815
gag ctg cac ctg ggc ctg gac aag atg aaa aac ccc atc acc aag cgc 2495
Glu Leu His Leu Gly Leu Asp Lys Met Lys Asn Pro Ile Thr Lys Arg
                820                 825                 830
tgg aag cac ctc act ggg act ctg atc ttg gtg aac tcc ctg gac gtt 2543
Trp Lys His Leu Thr Gly Thr Leu Ile Leu Val Asn Ser Leu Asp Val
            835                 840                 845
ctg aga gca gcc gcc ttc agc cct gcg gac cag gac gac ttc gtg att 2591
Leu Arg Ala Ala Ala Phe Ser Pro Ala Asp Gln Asp Asp Phe Val Ile
        850                 855                 860

tgaatgggtc ccctcccctc ctgctgctct ggagtgcaag ccctcttctg ccctgcgtgc 2651
cctgctgtca ccgcggagct gaagagggag gaaggggcgg ctgctcagac agatttaggg 2711
cccgccagct aggctacacc catcatgcgc cgccctcctc catcgaggga gaggcctgaa 2771
gggactgcct actgcagctc gttgccaatc acatagcttt ctatttgtta agtataaatt 2831
taaatttaaa atcacttttt taacgaatgg ggggaaggga tctatgagaa aggtggtatc 2891
taattttttt atggaccata aaggtttaaa agaaaatagg ggcacagggt gttgaggttt 2951
ttatgttgtt atagaccttt ttaaattatg ttagagatgt atataggtat ttaaaggtca 3011
ctgggagcat ttctgattcc cggccacact ttgcatttca acactcagcc cggaaagatg 3071
ctcgttcggt tgttggacct ctttcactcc ctgcgtgtaa gaaggtgaat cacgtgggaa 3131
aaagtggctt ttcagtaaac gggtacagct cattctttct gagaaggccc caggtcctgc 3191
tccctcctcg gatttgattg tcttccgtgc tttgcctcac tcgtagtaaa tgaccatcca 3251
tagaatatgt gaatctttgg tgagcttcag tgggcagagt gaagtcccgc attagcattt 3311
aggtgccctg agctgtttct gccaatagat tagaaagcag ccatgagttg acagtcttta 3371
gggcccctgc cagtgtgcaa ttagtcattg acaagaacaa tgccatttga gagtgaggtg 3431
gtccctgctg ctacgaggcc attgtactgt tttttccttg aggtcaaagc agtgcttccc 3491
atagagtttg ctgcctcttc tgtggacagg aagaaaactt catgaccgaa tcagagcctt 3551
ggtggccact gactctcgtg cttattgcag atgctgtggt tggcctcaca agcaacgcct 3611
tatgctgatg tgcagaggtg ccagctgcca tttgccaaac tctgcatttc atttcatcta 3671
aggcttaacc cctcttcctt cctggtgtac ctgtgtctcc tcggaaggaa gtcatagttt 3731
agatgaaacc attttttgta caatgtaaag atcatctgag caagatgagc attttgtaaa 3791
aatgaaaatg tgactcacat aaaatcagga acttggcaca gtgttgcatt aataacttta 3851
gggtgcagac atgctgtgtg aatctcacaa tgcgtcgtag atgtcgcgtg ttggaaggga 3911
gcaggaggaa ggactgatac tggcaaatca gtagagtgag gtgatcctta gcaacgtgcc 3971
aggacacttc ctgtgtgcct gcagttgtca gggaccattt gggatcccga atctcattct 4031
ctaaaactgc tttcttgaaa catgttactt ccttagtata atcaatgtat actcccttac 4091
tggcctgaaa cgttgtatag ctacttattc agatactgaa gaccaacgga ctgaaaaaaa 4151

gaacaaacat tagctatttt atgctgcaag aaccaggaca cacaattcgc caatcatccc 4211

accatataac cttcgattgt gcttctcaac tccaccccat aatttctccc agagatcatc 4271

tatcaccttt tccccaaaga agaaacaaaa ccagttgcac cttaaaccat ggatattttt 4331

tcctcagggg ctttaaatag tttcctatgc aacgtgtctt gtagcacaaa taaaattcta 4391

caaaagttgc agtaaatttt atttggatat tttaacctgt taagtgtgtg tgtgttttct 4451

gtacccaacc agactttaaa taaaacaaac atgaaacct 4490

<210> 152

<211> 863

<212> PRT

<213> Homo sapiens

<400> 152

Met Gly Tyr Ile Pro Ser Ser Tyr Val Gln Pro Leu Asn Tyr Arg Asn

1               5                   10                  15

Ser Thr Leu Ser Asp Ser Gly Met Ile Asp Asn Leu Pro Asp Ser Pro

            20                  25                  30

Asp Glu Val Ala Lys Glu Leu Glu Leu Leu Gly Gly Trp Thr Asp Asp

        35                  40                  45

Lys Lys Val Pro Gly Arg Met Tyr Ser Asn Asn Pro Phe Trp Asn Gly

    50                  55                  60

Val Gln Thr Asn Pro Phe Leu Asn Gly Asn Val Pro Val Met Pro Ser

65                  70                  75                  80

Leu Asp Glu Leu Asn Pro Lys Ser Thr Val Asp Leu Leu Leu Phe Asp

                85                  90                  95

Ala Gly Thr Ser Ser Phe Thr Glu Ser Ser Ser Ala Thr Thr Asn Ser

            100                 105                 110

Thr Gly Asn Ile Phe Asp Glu Leu Pro Val Thr Asn Gly Leu His Ala

        115                 120                 125

Glu Pro Pro Val Arg Arg Asp Asn Pro Phe Phe Arg Ser Lys Arg Ser

    130                 135                 140

Tyr Ser Leu Ser Glu Leu Ser Val Leu Gln Ala Lys Ser Asp Ala Pro

145                 150                 155                 160

Thr Ser Ser Ser Phe Phe Thr Gly Leu Lys Ser Pro Ala Pro Glu Gln

                165                 170                 175

Phe Gln Ser Arg Glu Asp Phe Arg Thr Ala Trp Leu Asn His Arg Lys

            180                 185                 190

Leu Ala Arg Ser Cys His Asp Leu Asp Leu Leu Gly Gln Ser Pro Gly

        195                 200                 205

Trp Gly Gln Thr Gln Ala Val Glu Thr Asn Ile Val Cys Lys Leu Asp

    210                 215                 220

Ser Ser Gly Gly Ala Val Gln Leu Pro Asp Thr Ser Ile Ser Ile His

225                 230                 235                 240

Val Pro Glu Gly His Val Ala Pro Gly Glu Thr Gln Gln Ile Ser Met

                245                 250                 255

Lys Ala Leu Leu Asp Pro Pro Leu Glu Leu Asn Ser Asp Arg Ser Cys

            260                 265                 270

Ser Ile Ser Pro Val Leu Glu Val Lys Leu Ser Asn Leu Glu Val Lys

        275                 280                 285

Thr Ser Ile Ile Leu Glu Met Lys Val Ser Ala Glu Ile Lys Asn Asp

    290                 295                 300

Leu Phe Ser Lys Ser Thr Val Gly Leu Gln Cys Leu Arg Ser Asp Ser

305                 310                 315                 320

Lys Glu Gly Pro Tyr Val Ser Val Pro Leu Asn Cys Ser Cys Gly Asp

                325                 330                 335

Thr Val Gln Ala Gln Leu His Asn Leu Glu Pro Cys Met Tyr Val Ala
            340                 345                 350
Val Val Ala His Gly Pro Ser Ile Leu Tyr Pro Ser Thr Val Trp Asp
        355                 360                 365
Phe Ile Asn Lys Lys Val Thr Val Gly Leu Tyr Gly Pro Lys His Ile
    370                 375                 380
His Pro Ser Phe Lys Thr Val Val Thr Ile Phe Gly His Asp Cys Ala
385                 390                 395                 400
Pro Lys Thr Leu Leu Val Ser Glu Val Thr Arg Gln Ala Pro Asn Pro
                405                 410                 415
Ala Pro Val Ala Leu Gln Leu Trp Gly Lys His Gln Phe Val Leu Ser
            420                 425                 430
Arg Pro Gln Asp Leu Lys Val Cys Met Phe Ser Asn Met Thr Asn Tyr
        435                 440                 445
Glu Val Lys Ala Ser Glu Gln Ala Lys Val Val Arg Gly Phe Gln Leu
    450                 455                 460
Lys Leu Gly Lys Val Ser Arg Leu Ile Phe Pro Ile Thr Ser Gln Asn
465                 470                 475                 480
Pro Asn Glu Leu Ser Asp Phe Thr Leu Arg Val Gln Val Lys Asp Asp
                485                 490                 495
Gln Glu Ala Ile Leu Thr Gln Phe Cys Val Gln Thr Pro Gln Pro Pro
            500                 505                 510
Pro Lys Ser Ala Ile Lys Pro Ser Gly Gln Arg Arg Phe Leu Lys Lys
        515                 520                 525
Asn Glu Val Gly Lys Ile Ile Leu Ser Pro Phe Ala Thr Thr Thr Lys
    530                 535                 540

Tyr Pro Thr Phe Gln Asp Arg Pro Val Ser Ser Leu Lys Phe Gly Lys
545                 550                 555                 560
Leu Leu Lys Thr Val Val Arg Gln Asn Lys Asn His Tyr Leu Leu Glu
                565                 570                 575
Tyr Lys Lys Gly Asp Gly Ile Ala Leu Leu Ser Glu Glu Arg Val Arg
            580                 585                 590
Leu Arg Gly Gln Leu Trp Thr Lys Glu Trp Tyr Ile Gly Tyr Tyr Gln
        595                 600                 605
Gly Arg Val Gly Leu Val His Thr Lys Asn Val Leu Val Val Gly Arg
    610                 615                 620
Ala Arg Pro Ser Leu Cys Ser Gly Pro Glu Leu Ser Thr Ser Val Leu
625                 630                 635                 640
Leu Glu Gln Ile Leu Arg Pro Cys Lys Phe Leu Thr Tyr Ile Tyr Ala
                645                 650                 655
Ser Val Arg Thr Leu Leu Met Glu Asn Ile Ser Ser Trp Arg Ser Phe
            660                 665                 670
Ala Asp Ala Leu Gly Tyr Val Asn Leu Pro Leu Thr Phe Phe Cys Arg
        675                 680                 685
Ala Glu Leu Asp Ser Glu Pro Glu Arg Val Ala Ser Val Leu Glu Lys
    690                 695                 700
Leu Lys Glu Asp Cys Asn Asn Thr Glu Asn Lys Glu Arg Lys Ser Phe
705                 710                 715                 720
Gln Lys Glu Leu Val Met Ala Leu Leu Lys Met Asp Cys Gln Gly Leu
                725                 730                 735
Val Val Arg Leu Ile Gln Asp Phe Val Leu Leu Thr Thr Ala Val Glu
            740                 745                 750
Val Ala Gln Arg Trp Arg Glu Leu Ala Glu Lys Leu Ala Lys Val Ser

        755                 760                 765

Lys Gln Gln Met Asp Ala Tyr Glu Ser Pro His Arg Asp Arg Asn Gly

    770                 775                 780

Val Val Asp Ser Glu Ala Met Trp Lys Pro Ala Tyr Asp Phe Leu Leu

785                 790                 795                 800

Thr Trp Ser His Gln Ile Gly Asp Ser Tyr Arg Asp Val Ile Gln Glu

                805                 810                 815

Leu His Leu Gly Leu Asp Lys Met Lys Asn Pro Ile Thr Lys Arg Trp

            820                 825                 830

Lys His Leu Thr Gly Thr Leu Ile Leu Val Asn Ser Leu Asp Val Leu

        835                 840                 845

Arg Ala Ala Ala Phe Ser Pro Ala Asp Gln Asp Asp Phe Val Ile

    850                 855                 860

<210> 153

<211> 2194

<212> DNA

<213> Homo sapiens

<220>

<221> CDS

<222> (133)..(1125)

<400> 153

ggtgcggcgc tggtcccctc agagggttcc tgctgctgcc ggtgccttgg accctccccc 60

tcgcttctcg ttctactgcc ccaggagccc ggcgggtccg ggactcccgt ccgtgccggt 120

gcgggcgccg gc atg tgg ctg tgg gag gac cag ggc ggc ctc ctg ggc cct 171

Met Trp Leu Trp Glu Asp Gln Gly Gly Leu Leu Gly Pro

1               5                   10

ttc tcc ttc ctg ctg cta gtg ctg ctg ctg gtg acg cgg agc ccg gtc 219
Phe Ser Phe Leu Leu Leu Val Leu Leu Leu Val Thr Arg Ser Pro Val
    15                  20                  25
aat gcc tgc ctc ctc acc ggc agc ctc ttc gtt cta ctg cgc gtc ttc 267
Asn Ala Cys Leu Leu Thr Gly Ser Leu Phe Val Leu Leu Arg Val Phe
30                  35                  40                  45
agc ttt gag ccg gtg ccc tct tgc agg gcc ctg cag gtg ctc aag ccc 315
Ser Phe Glu Pro Val Pro Ser Cys Arg Ala Leu Gln Val Leu Lys Pro
                50                  55                  60
cgg gac cgc att tct gcc atc gcc cac cgt ggc ggc agc cac gac gcg 363
Arg Asp Arg Ile Ser Ala Ile Ala His Arg Gly Gly Ser His Asp Ala
            65                  70                  75
ccc gag aac acg ctg gcg gcc att cgg cag gca gct aag aat gga gca 411
Pro Glu Asn Thr Leu Ala Ala Ile Arg Gln Ala Ala Lys Asn Gly Ala
        80                  85                  90
aca ggc gtg gag ttg gac att gag ttt act tct gac ggg att cct gtc 459
Thr Gly Val Glu Leu Asp Ile Glu Phe Thr Ser Asp Gly Ile Pro Val
    95                  100                 105
tta atg cac gat aac aca gta gat agg acg act gat ggg act ggg cga 507
Leu Met His Asp Asn Thr Val Asp Arg Thr Thr Asp Gly Thr Gly Arg
110                 115                 120                 125
ttg tgt gat ttg aca ttt gaa caa att agg aag ctg aat cct gca gca 555
Leu Cys Asp Leu Thr Phe Glu Gln Ile Arg Lys Leu Asn Pro Ala Ala
                130                 135                 140
aac cac aga ctc agg aat gat ttc cct gat gaa aag atc cct acc cta 603
Asn His Arg Leu Arg Asn Asp Phe Pro Asp Glu Lys Ile Pro Thr Leu

            145                 150                 155
agg gaa gct gtt gca gag tgc cta aac cat aac ctc aca atc ttc ttt 651
Arg Glu Ala Val Ala Glu Cys Leu Asn His Asn Leu Thr Ile Phe Phe
        160                 165                 170
gat gtc aaa ggc cat gca cac aag gct act gag gct cta aag aaa atg 699
Asp Val Lys Gly His Ala His Lys Ala Thr Glu Ala Leu Lys Lys Met
    175                 180                 185
tat atg gaa ttt cct caa ctg tat aat aat agt gtg gtc tgt tct ttc 747
Tyr Met Glu Phe Pro Gln Leu Tyr Asn Asn Ser Val Val Cys Ser Phe
190                 195                 200                 205
ttg cca gaa gtt atc tac aag atg aga caa aca gat cgg gat gta ata 795
Leu Pro Glu Val Ile Tyr Lys Met Arg Gln Thr Asp Arg Asp Val Ile
                210                 215                 220
aca gca tta act cac aga cct tgg agc cta agc cat aca gga gat ggg 843
Thr Ala Leu Thr His Arg Pro Trp Ser Leu Ser His Thr Gly Asp Gly
            225                 230                 235
aaa cca cgc tat gat act ttc tgg aaa cat ttt ata ttt gtt atg atg 891
Lys Pro Arg Tyr Asp Thr Phe Trp Lys His Phe Ile Phe Val Met Met
        240                 245                 250
gac att ttg ctc gat tgg agc atg cat aat atc ttg tgg tac ctg tgt 939
Asp Ile Leu Leu Asp Trp Ser Met His Asn Ile Leu Trp Tyr Leu Cys
    255                 260                 265
gga att tca gct ttc ctc atg caa aag gat ttt gta tcc ccg gcc tac 987
Gly Ile Ser Ala Phe Leu Met Gln Lys Asp Phe Val Ser Pro Ala Tyr
270                 275                 280                 285
ttg aag aag tgg tca gct aaa gga atc cag gtt gtt ggt tgg act gtt 1035
Leu Lys Lys Trp Ser Ala Lys Gly Ile Gln Val Val Gly Trp Thr Val

                290                 295                 300

aat acc ttt gat gaa aag agt tac tac gaa tcc cat ctt ggt tcc agc 1083

Asn Thr Phe Asp Glu Lys Ser Tyr Tyr Glu Ser His Leu Gly Ser Ser

            305                 310                 315

tat atc act gac agc atg gta gaa gac tgc gaa cct cac ttc 1125

Tyr Ile Thr Asp Ser Met Val Glu Asp Cys Glu Pro His Phe

        320                 325                 330

tagactttca cggtgggacg aaacgggttc agaaactgcc aggggcctca tacagggata 1185

tcaaaatacc ctttgtgcta gcccaggccc tggggaatca ggtgactcac acaaatgcaa 1245

tagttggtca ctgcattttt acctgaacca aagctaaacc cggtgttgcc accatgcacc 1305

atggcatgcc agagttcaac actgttgctc ttgaaaatct gggtctgaaa aaacgcacaa 1365

gagcccctgc cctgccctag ctgaggcaca cagggagacc cagtgaggat aagcacagat 1425

tgaattgtac agtttgcaga tgcagatgta aatgcatggg acatgcatga taactcagag 1485

ttgacatttt aaaacttgcc acacttattt caaatatttg tactcagcta tgttaacatg 1545

tactgtagac atcaaacttg tggccatact aataaaatta ttaaaaggag cactaaagga 1605

aaactgtgtg ccaagcatca tatcctaagg catacggaat ttggggaagc caccatgcaa 1665

tccagtgagg cttcagtgta cagcaaccaa aatggtaggg aggtcttgaa gccaatgagg 1725

gatttatagc atcttgaata gagagctgca aaccaccagg gggcagagtt gcatttttcc 1785

aggcttttta ggaagctctg caacagatgt gatctgatca taggcaatta gaactggaag 1845

aaacttccaa aaatatctag gtttgtcctc attttacaaa tgaggaaact aaactctgtg 1905

gaagggaagg ggttgcctca aaagtcacag cttagctggg cacagtggct catgccgata 1965

atcccagcaa ttcagaaagc tgaggcagga ggattacttg aggccagact gggcaatata 2025

gcaagacccc atctctaaaa aattaggcat ggtggtgcat gcctgtattc ccagctactc 2085

aggaggttga ggtgggagga tcacttgagc ccagaagttc aaggctgcaa tgagccatga 2145

ttacaccacg gcactacaac cttggtggca cagtgagaac ctgactctt 2194

<210> 154

<211> 331

<212> PRT

<213> Homo sapiens

<400> 154

Met Trp Leu Trp Glu Asp Gln Gly Gly Leu Leu Gly Pro Phe Ser Phe
1               5                   10                  15

Leu Leu Leu Val Leu Leu Leu Val Thr Arg Ser Pro Val Asn Ala Cys
            20                  25                  30

Leu Leu Thr Gly Ser Leu Phe Val Leu Leu Arg Val Phe Ser Phe Glu
        35                  40                  45

Pro Val Pro Ser Cys Arg Ala Leu Gln Val Leu Lys Pro Arg Asp Arg
    50                  55                  60

Ile Ser Ala Ile Ala His Arg Gly Gly Ser His Asp Ala Pro Glu Asn
65                  70                  75                  80

Thr Leu Ala Ala Ile Arg Gln Ala Ala Lys Asn Gly Ala Thr Gly Val
                85                  90                  95

Glu Leu Asp Ile Glu Phe Thr Ser Asp Gly Ile Pro Val Leu Met His
            100                 105                 110

Asp Asn Thr Val Asp Arg Thr Thr Asp Gly Thr Gly Arg Leu Cys Asp
        115                 120                 125

Leu Thr Phe Glu Gln Ile Arg Lys Leu Asn Pro Ala Ala Asn His Arg
    130                 135                 140

Leu Arg Asn Asp Phe Pro Asp Glu Lys Ile Pro Thr Leu Arg Glu Ala
145                 150                 155                 160

Val Ala Glu Cys Leu Asn His Asn Leu Thr Ile Phe Phe Asp Val Lys
                165                 170                 175

Gly His Ala His Lys Ala Thr Glu Ala Leu Lys Lys Met Tyr Met Glu
            180                 185                 190
Phe Pro Gln Leu Tyr Asn Asn Ser Val Val Cys Ser Phe Leu Pro Glu
        195                 200                 205
Val Ile Tyr Lys Met Arg Gln Thr Asp Arg Asp Val Ile Thr Ala Leu
    210                 215                 220
Thr His Arg Pro Trp Ser Leu Ser His Thr Gly Asp Gly Lys Pro Arg
225                 230                 235                 240
Tyr Asp Thr Phe Trp Lys His Phe Ile Phe Val Met Met Asp Ile Leu
                245                 250                 255
Leu Asp Trp Ser Met His Asn Ile Leu Trp Tyr Leu Cys Gly Ile Ser
            260                 265                 270
Ala Phe Leu Met Gln Lys Asp Phe Val Ser Pro Ala Tyr Leu Lys Lys
        275                 280                 285
Trp Ser Ala Lys Gly Ile Gln Val Val Gly Trp Thr Val Asn Thr Phe
    290                 295                 300
Asp Glu Lys Ser Tyr Tyr Glu Ser His Leu Gly Ser Ser Tyr Ile Thr
305                 310                 315                 320
Asp Ser Met Val Glu Asp Cys Glu Pro His Phe
                325                 330

<210> 155
<211> 3377
<212> DNA
<213> Homo sapiens
<220>
<221> CDS

<222> (44)..(1666)

<400> 155

gcctctgcac ccctactgct tctgctttga aggcggaggc tcc atg ttg tcc cct 55

Met Leu Ser Pro

1

cag cga gtg gga gca gct gcc tca aga gga gca gat gat gcc atg gag 103

Gln Arg Val Gly Ala Ala Ala Ser Arg Gly Ala Asp Asp Ala Met Glu

5                   10                  15                  20

agc agc aag cct ggt cca gtg cag gtt gtt ttg gtt cag aaa gat caa 151

Ser Ser Lys Pro Gly Pro Val Gln Val Val Leu Val Gln Lys Asp Gln

                25                  30                  35

cat tcc ttt gag cta gat gag aaa gcc ttg gcc agc atc ctc ttg cag 199

His Ser Phe Glu Leu Asp Glu Lys Ala Leu Ala Ser Ile Leu Leu Gln

            40                  45                  50

gac cac atc cga gat ctt gat gtg gtg gtg gtt tca gtg gct ggt gcc 247

Asp His Ile Arg Asp Leu Asp Val Val Val Val Ser Val Ala Gly Ala

        55                  60                  65

ttc cga aag ggc aag tcc ttc att ctg gat ttt atg cta cga tac tta 295

Phe Arg Lys Gly Lys Ser Phe Ile Leu Asp Phe Met Leu Arg Tyr Leu

    70                  75                  80

tat tct cag aag gaa agt ggc cat tca aat tgg ttg ggt gac cca gaa 343

Tyr Ser Gln Lys Glu Ser Gly His Ser Asn Trp Leu Gly Asp Pro Glu

85                  90                  95                  100

gaa ccg tta aca gga ttt tcc tgg aga ggg gga tct gat cca gaa acc 391

Glu Pro Leu Thr Gly Phe Ser Trp Arg Gly Gly Ser Asp Pro Glu Thr

                105                 110                 115

act ggg att caa atc tgg agt gaa gtt ttc act gtg gag aag cca ggt 439

Thr Gly Ile Gln Ile Trp Ser Glu Val Phe Thr Val Glu Lys Pro Gly
            120                 125                 130
ggg aag aag gtt gca gtt gtt ctg atg gat acc cag ggg gca ttt gac 487
Gly Lys Lys Val Ala Val Val Leu Met Asp Thr Gln Gly Ala Phe Asp
        135                 140                 145
agc cag tca act gtg aaa gac tgt gct acc atc ttt gct cta agc act 535
Ser Gln Ser Thr Val Lys Asp Cys Ala Thr Ile Phe Ala Leu Ser Thr
    150                 155                 160
atg act agt tct gtt cag att tat aat tta tct cag aac att caa gaa 583
Met Thr Ser Ser Val Gln Ile Tyr Asn Leu Ser Gln Asn Ile Gln Glu
165                 170                 175                 180
gat gat ctt caa cag ctg cag ctc ttc aca gaa tac ggt cgt ctg gca 631
Asp Asp Leu Gln Gln Leu Gln Leu Phe Thr Glu Tyr Gly Arg Leu Ala
                185                 190                 195
atg gat gaa att ttc caa aag cct ttc cag aca ctg atg ttt ttg gtt 679
Met Asp Glu Ile Phe Gln Lys Pro Phe Gln Thr Leu Met Phe Leu Val
            200                 205                 210
aga gat tgg agt ttc cct tat gaa tat agc tat gga ctc caa gga gga 727
Arg Asp Trp Ser Phe Pro Tyr Glu Tyr Ser Tyr Gly Leu Gln Gly Gly
        215                 220                 225
atg gca ttt ttg gat aag cgt tta cag gtg aag gaa cat caa cat gaa 775
Met Ala Phe Leu Asp Lys Arg Leu Gln Val Lys Glu His Gln His Glu
    230                 235                 240
gaa att cag aat gtt cga aat cac att cac tca tgt ttc tcc gat gtc 823
Glu Ile Gln Asn Val Arg Asn His Ile His Ser Cys Phe Ser Asp Val
245                 250                 255                 260

acc tgc ttt ctc tta cca cat cca gga ctc cag gtg gcc aca agc cct 871
Thr Cys Phe Leu Leu Pro His Pro Gly Leu Gln Val Ala Thr Ser Pro
                265                 270                 275
gac ttt gat ggg aaa tta aaa gat att gct ggt gaa ttc aaa gag cag 919
Asp Phe Asp Gly Lys Leu Lys Asp Ile Ala Gly Glu Phe Lys Glu Gln
            280                 285                 290
tta cag gca ctg ata ccg tat gta tta aac cca tct aag tta atg gaa 967
Leu Gln Ala Leu Ile Pro Tyr Val Leu Asn Pro Ser Lys Leu Met Glu
        295                 300                 305
aag gag atc aat ggc tca aag gtc acc tgt cgg gga cta ctg gag tat 1015
Lys Glu Ile Asn Gly Ser Lys Val Thr Cys Arg Gly Leu Leu Glu Tyr
    310                 315                 320
ttt aag gca tat att aaa att tat caa gga gaa gat ctg cct cac ccc 1063
Phe Lys Ala Tyr Ile Lys Ile Tyr Gln Gly Glu Asp Leu Pro His Pro
325                 330                 335                 340
aag tcc atg ctt cag gcc act gct gaa gcc aac aac tta gca gct gca 1111
Lys Ser Met Leu Gln Ala Thr Ala Glu Ala Asn Asn Leu Ala Ala Ala
                345                 350                 355
gcc tct gcc aag gac att tat tat aac aac atg gaa gag gtt tgt ggg 1159
Ala Ser Ala Lys Asp Ile Tyr Tyr Asn Asn Met Glu Glu Val Cys Gly
            360                 365                 370
gga gag aaa cct tat ttg tct cca gac att cta gag gag aag cac tgt 1207
Gly Glu Lys Pro Tyr Leu Ser Pro Asp Ile Leu Glu Glu Lys His Cys
        375                 380                 385
gaa ttc aaa caa ctt gct ctg gac cat ttt aag aag acc aag aag atg 1255
Glu Phe Lys Gln Leu Ala Leu Asp His Phe Lys Lys Thr Lys Lys Met
    390                 395                 400

ggt ggg aag gat ttc agc ttt cgt tac cag cag gag ctg gag gag gaa 1303
Gly Gly Lys Asp Phe Ser Phe Arg Tyr Gln Gln Glu Leu Glu Glu Glu
405                 410                 415                 420
atc aag gaa tta tat gag aac ttc tgc aag cac aat ggt agc aag aac 1351
Ile Lys Glu Leu Tyr Glu Asn Phe Cys Lys His Asn Gly Ser Lys Asn
                425                 430                 435
gtc ttc agc acc ttc cga acc cct gca gtg ctg ttc acg ggc att gta 1399
Val Phe Ser Thr Phe Arg Thr Pro Ala Val Leu Phe Thr Gly Ile Val
            440                 445                 450
gct ttg tac ata gcc tca ggc ctc act ggc ttc ata ggt ctt gag gtt 1447
Ala Leu Tyr Ile Ala Ser Gly Leu Thr Gly Phe Ile Gly Leu Glu Val
        455                 460                 465
gta gcc cag ttg ttc aac tgt atg gtt gga cta ctg tta ata gca ctc 1495
Val Ala Gln Leu Phe Asn Cys Met Val Gly Leu Leu Leu Ile Ala Leu
    470                 475                 480
ctc acc tgg ggc tac atc agg tat tct ggt caa tat cgt gag ctg ggc 1543
Leu Thr Trp Gly Tyr Ile Arg Tyr Ser Gly Gln Tyr Arg Glu Leu Gly
485                 490                 495                 500
gga gct att gat ttt ggt gcc gca tat gtg ttg gag cag gct tct tct 1591
Gly Ala Ile Asp Phe Gly Ala Ala Tyr Val Leu Glu Gln Ala Ser Ser
                505                 510                 515
cat atc ggt aat tcc act cag gcc act gtg agg gat gca gtt gtt gga 1639
His Ile Gly Asn Ser Thr Gln Ala Thr Val Arg Asp Ala Val Val Gly
            520                 525                 530
aga cca tcc atg gat aaa aaa gct caa tagcatctta acgtgaagat 1686
Arg Pro Ser Met Asp Lys Lys Ala Gln

        535                 540

caaacaagaa cacaacaagc ccctactgat ttctgggttt ctgccacggc cacaggttca 1746

tatccagagg aatggcagat ctgagacgat ccaggaagag ctaaaacatg gccctgtaat 1806

aaatgagcag acctctcctg tggtttcaaa ttattaaaca cacttccatt tctcttggaa 1866

gcatttcttt tccttgctgt tatagatgca agcctgtgtc tattttcata ttactctgct 1926

ttgtgcactt tatggaggag gaagctagag gaaaaatgga aatgcagctt ttaagttctt 1986

tatgtgccac ttagtgcctt ttaagattga ttccatggtt ttgcacacac gatggggagg 2046

ggatggagga taacctcatg aaaggtgcca ttttcgggtg aaacttgaca tttcttttat 2106

actttacttt tgagaaggat tctttttttt ttgagttgga gtctcgctct gtcaccaggc 2166

ttgagtgcag tggtgtgatc ttggctcact gcaacctctg cctgccgggt tcaagcagtt 2226

ctcctgcctc agcctcccaa gtagctggga ctacaggtgt acgccaccat acccagctaa 2286

tttttttgta tttttagtag agatggggtt tcaccatgtt ggccaggatg gtcttggtct 2346

cttgaccttg tgatccgcct gcccgccttg gcctcccaaa gtgctgggat tacaggcgtg 2406

agccaccgtg cccggccaag aaggattcct ttttaaaaag tttacagaac ttggagaaac 2466

ttcagaacta aagactaact gaaaatgatg tcattacact caaaaaaaat ttacaatagg 2526

gaatcctgtt gccacatagt gtggaaaaat catgtcatat ttaaatatac catactctga 2586

aatgtgaggt ttttacccag taggctgaca gttttgttgc aacttgctct attttttttt 2646

ttttttggtc cctatagctt ctttctagaa aaagaggcaa acgtgccttg aaaagccaga 2706

gtggctcata ataaaaggaa tgcgctagat acttcaagaa aaaagctaag tttaaatgaa 2766

ccatgtgacc tctgataagt cacttgaact tgtgccttag tcaggttcac tgtaggttta 2826

catatgtatg tatgttttac acaactcttg taattgtcat ttgaggggtt cacttcctcc 2886

tcccaccccc tgggagcggc cctgcgctgt cactgacatc tcattaaaaa aaaaaaaaaa 2946

tttgctctca aggtgtttga ggctttaatg caacccttta gcccttggtt ctttttggtg 3006

caagaattct ggctgtttac ctcagactca gacccctgaa atgttgccaa attcttcaaa 3066

taactgtttg gggggtgggg ggagatgaaa gagagtcgcg ttttgtttac agttaaagac 3126

atccaatatc ttaaaaagga gttttccttt agaaacacac acacccttcc tcttgctcaa 3186

aagatctcac tccatgatac tgtgtaaaat atttttgcac tgttgtgaag tatttttgac 3246

ttttttctgt acataactgt gttctcagag ctgaatgttt atatcttttg ctgtgcaaaa 3306
gaaacatgta aaatgttgtt cagttgtata tacagaaatg tgtataaaac attttgttat 3366
tttttaaaag t 3377

<210> 156
<211> 541
<212> PRT
<213> Homo sapiens
<400> 156
Met Leu Ser Pro Gln Arg Val Gly Ala Ala Ala Ser Arg Gly Ala Asp
1               5                   10                  15
Asp Ala Met Glu Ser Ser Lys Pro Gly Pro Val Gln Val Val Leu Val
            20                  25                  30
Gln Lys Asp Gln His Ser Phe Glu Leu Asp Glu Lys Ala Leu Ala Ser
        35                  40                  45
Ile Leu Leu Gln Asp His Ile Arg Asp Leu Asp Val Val Val Val Ser
    50                  55                  60
Val Ala Gly Ala Phe Arg Lys Gly Lys Ser Phe Ile Leu Asp Phe Met
65                  70                  75                  80
Leu Arg Tyr Leu Tyr Ser Gln Lys Glu Ser Gly His Ser Asn Trp Leu
                85                  90                  95
Gly Asp Pro Glu Glu Pro Leu Thr Gly Phe Ser Trp Arg Gly Gly Ser
            100                 105                 110
Asp Pro Glu Thr Thr Gly Ile Gln Ile Trp Ser Glu Val Phe Thr Val
        115                 120                 125
Glu Lys Pro Gly Gly Lys Lys Val Ala Val Val Leu Met Asp Thr Gln
    130                 135                 140

Gly Ala Phe Asp Ser Gln Ser Thr Val Lys Asp Cys Ala Thr Ile Phe
145                 150                 155                 160
Ala Leu Ser Thr Met Thr Ser Ser Val Gln Ile Tyr Asn Leu Ser Gln
                165                 170                 175
Asn Ile Gln Glu Asp Asp Leu Gln Gln Leu Gln Leu Phe Thr Glu Tyr
            180                 185                 190
Gly Arg Leu Ala Met Asp Glu Ile Phe Gln Lys Pro Phe Gln Thr Leu
        195                 200                 205
Met Phe Leu Val Arg Asp Trp Ser Phe Pro Tyr Glu Tyr Ser Tyr Gly
    210                 215                 220
Leu Gln Gly Gly Met Ala Phe Leu Asp Lys Arg Leu Gln Val Lys Glu
225                 230                 235                 240
His Gln His Glu Glu Ile Gln Asn Val Arg Asn His Ile His Ser Cys
                245                 250                 255
Phe Ser Asp Val Thr Cys Phe Leu Leu Pro His Pro Gly Leu Gln Val
            260                 265                 270
Ala Thr Ser Pro Asp Phe Asp Gly Lys Leu Lys Asp Ile Ala Gly Glu
        275                 280                 285
Phe Lys Glu Gln Leu Gln Ala Leu Ile Pro Tyr Val Leu Asn Pro Ser
    290                 295                 300
Lys Leu Met Glu Lys Glu Ile Asn Gly Ser Lys Val Thr Cys Arg Gly
305                 310                 315                 320
Leu Leu Glu Tyr Phe Lys Ala Tyr Ile Lys Ile Tyr Gln Gly Glu Asp
                325                 330                 335
Leu Pro His Pro Lys Ser Met Leu Gln Ala Thr Ala Glu Ala Asn Asn
            340                 345                 350

Leu Ala Ala Ala Ala Ser Ala Lys Asp Ile Tyr Tyr Asn Asn Met Glu
        355                 360                 365
Glu Val Cys Gly Gly Glu Lys Pro Tyr Leu Ser Pro Asp Ile Leu Glu
    370                 375                 380
Glu Lys His Cys Glu Phe Lys Gln Leu Ala Leu Asp His Phe Lys Lys
385                 390                 395                 400
Thr Lys Lys Met Gly Gly Lys Asp Phe Ser Phe Arg Tyr Gln Gln Glu
                405                 410                 415
Leu Glu Glu Glu Ile Lys Glu Leu Tyr Glu Asn Phe Cys Lys His Asn
            420                 425                 430
Gly Ser Lys Asn Val Phe Ser Thr Phe Arg Thr Pro Ala Val Leu Phe
        435                 440                 445
Thr Gly Ile Val Ala Leu Tyr Ile Ala Ser Gly Leu Thr Gly Phe Ile
    450                 455                 460
Gly Leu Glu Val Val Ala Gln Leu Phe Asn Cys Met Val Gly Leu Leu
465                 470                 475                 480
Leu Ile Ala Leu Leu Thr Trp Gly Tyr Ile Arg Tyr Ser Gly Gln Tyr
                485                 490                 495
Arg Glu Leu Gly Gly Ala Ile Asp Phe Gly Ala Ala Tyr Val Leu Glu
            500                 505                 510
Gln Ala Ser Ser His Ile Gly Asn Ser Thr Gln Ala Thr Val Arg Asp
        515                 520                 525
Ala Val Val Gly Arg Pro Ser Met Asp Lys Lys Ala Gln
    530                 535                 540
<210> 157
<211> 2172

<212> DNA

<213> Homo sapiens

<220>

<221> CDS

<222> (45)..(563)

<400> 157
   ggaacacggc acccgcactg cgcgtcatgg tgcaggcctg gtat atg gac gac gcc 56
   ccg ggc gac ccg cgg caa ccc cac cgc ccc gac ccc ggc cgc cca gtg 104
   ggc ctg gag cag ctg cgg cgg ctc ggg gtg ctc tac tgg aag ctg gat 152
   gct gac aaa tat gag aat gat cca gaa tta gaa aag atc cga aga gag 200
   agg aac tac tcc tgg atg gac atc ata acc ata tgc aaa gat aaa cta 248
   cca aat tat gaa gaa aag att aag atg ttc tac gag gag cat ttg cac 296
   ttg gac gat gag atc cgc tac atc ctg gat ggc agt ggg tac ttc gat 344
   gtg agg gac aag gag gac cag tgg atc cgg atc ttc atg gag aag gga 392
   gac atg gtg acg ctc ccc gcg ggg atc tat cac cgc ttc acg gtg gac 440
   gag aag aac tac acg aag gcc atg cgg ctg ttt gtg gga gaa ccg gtg 488
   tgg aca gcg tac aac cgg ccc gct gac cat ttt gaa gcc cgc ggg cag 536
   tac gtg aaa ttt ctg gca cag acc gcc tagcagtgct gcctgggaac 583
   taacacgtgc ctcgtaaagg tccccaatgt aatgactgag cagaaaatca atcactttct 643
   ctttgctttt agaggatagc cttgaggcta gattatcttt cctttgtaag attatttgat 703
   cagaatattt tgtaatgaaa ggatctagaa agcaacttgg aagtgtaaag agtcaccttc 763
   attttctgta actcaatcaa gactggtggg tccatggccc tgtgttagtt catgcattca 823
   gttgagtccc aaatgaaagt ttcatctccc gaaatgcagt tccttagatg cccatctgga 883
   cgtgatgccg cgcctgccgt gtaagaaggt gcaatcctag ataacacagc tagccagata 943
   gaagacactt ttttctccaa aatgatgcct tggggtgggg agtggtaggg ggaagagctc 1003
   ccaccctaag gggcacacac tgagttgctt atgccacttc cttgttcaaa ataaagtaac 1063
   tgccttaatc ttatactcat ggcttggagt taccttatat tcaggtatat gtgatatttt 1123
   gcctggtttg ttaaaattgc cccatttaga ttccttctat aattgttctt atagataagt 1183
   aatttatata tgagctgtgt tagtattttt tcagtgtgag atctctggat tctttcacaa 1243
   taaagctgtt gaattttaac aggagtatta gtacataaat tttctactca acaattccga 1303
   gataggatta tgcctagttt gtcatatcac agaaaaactc caagttaact tcatgttttg 1363
   gaagggcagg tcgtttttaa agtatttctt tttttaactg gatgaaaaat cttcatgtta 1423
   ggattaattt tcttaatcac ctccacactg tacagaggaa actcaagcct taaatgttta 1483
   agtaaactct gtctcagttt taggattaaa atacccaccg gtggtgtgat gatgccatat 1543
   accgcagggc ttgcttctgt caagtgtgac tctatctcag taattaaaat aagtgctgat 1603
   ctactgattt tttttaatgg attcatttct aaatgggcat tataaataga gcttgttcat 1663
   ttttaagaac gaaacattca tatgataaac tatcgcttta aattgccttt cttgcttcat 1723
   ataacttttc cctgtcagga tccttagtgt ttgaaactcc tcgtgcgggg ctggcctcct 1783
   gcggactcta gtttcgcctc cttgatgtgg cgcctgggat ttcttcactt cagagctgta 1843
   tttttacagg caagagtaag ttcctgggca cagtggctca tgcctgtaat ctcagctact 1903
   caggaggcta aggtgggagg attcttagag cctgggaggt cgaggctgca gtgagctgtg 1963
   attgtggcca ctgcactcca gcctgggtga cagagcgaga ctctgtctca aaaaagaaga 2023
   aagagtaaga gctgaggcat ataatagaat tctgctaaag cacttaaggt gaaatcacat 2083
   tttcttttcc caggatgttg ctcacatctt tcgtttttat tgaggtgtca tttatgtaca 2143
   ataaaatgta ctcattttca gtgtttttg 2172

<210> 158

<211> 173

<212> PRT

<213> Homo sapiens

<400> 158

<210> 159

<211> 20

<212> DNA

<220>

<223> Description of the artificial sequence:an artificially synthesized primer
sequence

<400> 159

ggaagtgtta cttctgctct 20

<210> 160

<211> 50

<212> DNA

<220>

<223> Description of the artificial sequence:an artificially synthesized primer
sequence

<400> 160

gagagagaga gagagagaga actagtctcg agtttttttt tttttttttt 50

<210> 161

<211> 41

<212> DNA

<220>

<223> Description of the artificial sequence:an artificially synthesized primer
sequence

<400> 161

gagagagaga gagagagcgg ccgcactagt cccccccccc c 41

<210> 162
<211> 30
<212> RNA
<213> Artificial Sequence
<220>
<223> Description of the artificial sequence: an artificially synthesized oligo-
cap linker sequence
<400> 162
agcaucgagu cggccuuguu ggccuacugg 30

<210> 163
<211> 42
<212> DNA
<213> Artificial Sequence
<220>
<223> Description of the artificial sequence: an artificially synthesized
oligo(dT)primer sequence
<400> 163
gcggctgaag acggcctatg tggccttttt tttttttttt tt 42

<210> 164
<211> 21
<212> DNA
<213> Artificial Sequence
<220>
<223> Description of the artificial sequence: an artificially synthesized primer
sequence
<400> 164
agcatcgagt cggccttgtt g                                                21

<210> 165
<211> 21
<212> DNA
<213> Artificial Sequence
<220>
<223> Description of the artificial sequence: an artificially synthesized primer
sequence
<400> 165
gcggctgaag acggcctatg t                                                21

<210> 166
<211> 30
<212> DNA
<213> Artificial Sequence
<220>
<223> Description of the artificial sequence: an artificially synthesized primer
sequence
<400> 166
actttattgt catagtttag atctattttg                                       30

<210> 167
<211> 30
<212> DNA
<213> Artificial Sequence
<220>
<223> Description of the artificial sequence: an artificially synthesized primer
sequence
<400> 167
ataatcctta aaaactccat ttccacccct                                       30

<210> 168
<211> 1536
<212> DNA
<213> Homo sapiens
<220>
<221> CDS
<222>(139)..(1062)
<400> 168
GTGCTCCGCC GCCCGCCCCG ACCCGGGCCC AGCCGCCTCC ACGGCCCGCG CTCGTACTGG      60
AGCGAAGAGC GGCCTCCTGA GGGAGGGGAA GGGACGTGGG GGCGGCCACG GCAGGATTAA     120
CCTCCATTTC AGCTAATC ATG GGA GAG ATT AAA GTC TCT CCT GAT TAT AAC       171
TGG TTT AGA GGT ACA GTT CCC CTT AAA AAG ATT ATT GTG GAT GAT GAT 219
GAC AGT AAG ATA TGG TCG CTC TAT GAC GCG GGC CCC CGA AGT ATC AGG 267
TGT CCT CTC ATA TTC CTG CCC CCT GTC AGT GGA ACT GCA GAT GTC TTT 315
TTC CGG CAG ATT TTG GCT CTG ACT GGA TGG GGT TAC CGG GTT ATC GCT 363
TTG CAG TAT CCA GTT TAT TGG GAC CAT CTC GAG TTC TGT GAT GGA TTC 411
AGA AAA CTT TTA GAC CAT TTA CAA TTG GAT AAA GTT CAT CTT TTT GGC 459
GCT TCT TTG GGA GGC TTT TTG GCC CAG AAA TTT GCT GAA TAT ACT CAC 507
AAA TCT CCT AGA GTC CAT TCC CTA ATC CTC TGC AAT TCC TTC AGT GAC 555
ACC TCT ATC TTC AAC CAA ACT TGG ACT GCA AAC AGC TTT TGG CTG ATG 603
CCT GCA TTT ATG CTC AAA AAA ATA GTT CTT GGA AAT TTT TCA TCT GGC 651
CCG GTG GAC CCT ATG ATG GCT GAT GCC ATT GAT TTC ATG GTA GAC AGG 699
CTA GAA AGT TTG GGT CAG AGT GAA CTG GCT TCA AGA CTT ACC TTG AAT 747
TGT CAA AAT TCT TAT GTG GTA CCT CAT AAA ATT CGG GAC ATA CCT GTA 795
ACT ATT ATG GAT GTG TTT GAT CAG AGT GCG CTT TCA ACT GAA GCT AAA     843
GAA GAA ATG TAC AAG CTG TAT CCT AAT GCC CGA AGA GCT CAT CTG AAA     891
ACA GGA GGC AAT TTC CCA TAC CTG TGC AGA AGT GCA GAG GTC AAT CTT     939
TAT GTA CAG ATA CAT TTG CTG CAA TTC CAT GGA ACC AAA TAC GCG GCC     987
ATT GAC CCA TCA ATG GTC AGT GCC GAG GAG CTT GAG GTG CAG AAA GGC     1035
AGC CTT GGC ATC AGC CAG GAG GAG CAG TAGTGTGTCT CTCGCTGTCA ATGATGA     1089
GTTGACCCGG TGTGTTCTTG TATAGTCAGT GGCATCAGCA CCCGTCAGCC GGCCTTTTCC     1149
TTCAGGTTCG TCAGGCTCAC CGGTTCTCAC TGTGTCTGGG AAGTAGGACT GATGGTCATC     1209
TTCATGACAG GCGGCATCTC CACTAAGCCT GTGTAACTGT TCCCTCTTTG GTTTTCTTAG     1269
CTTTTGAATT TGAAGAAGTA CTTTTGAAGA CTCCCATTTT AAGAACCGTG CAGATTTTGC     1329
TACCAAAAGT CTTCACCACT GTGTTCTTAA GTGAATGTTA ATTTCTGAGG TTTGGGACTT     1389
TGTGGTGGTT TTTTTCTTCT TTTCTTTTCC ATTCTTCTTT CTTTCTTTTT ATGTTGTTTG     1449
CTGTAAATGC TGCACATCCA GATTGCATAT CAGGACATTG GTTATTTTAT GCTTTCTTGG     1509
ATATAACCAT GATCAGAGTG CCATGGC     1536

<210> 169
<211> 308
<212> PRT
<213> Homo sapiens
<400> 169

<210> 170
<211> 2560
<212> DNA
<213> Homo sapiens
<220>
<221> CDS
(202).. (1002)
<400> 170
CTGGCCTACT GGGGCTCCAG CCGTGTCCTG AGGAGCTGGA CCAGCCACAT CCCCTGGGGC      60
TGCAGTTGAA GCAGAACCAA GTGGCCATCC CGGCGTTAGA CCGTAGGTTC CTGGTCCCGG     120
AGTGGTCGGA GCCCGCCAGT GGGCAGGCAG CTCTTGCTCA CAGGCCGCGG TGCCCAGGCC     180
GCTGGCTCTC CGCAGGGCGG A ATG GCG CTG CAA GTG GAG CTG GTA CCC ACC     231
                        Met Ala Leu Gln Val Glu Leu Val Pro Thr
                        1               5                   10

GGG GAG ATC ATC CGC GTG GTT CAT CCC CAC AGG CCC TGC AAG CTT GCC     279
Gly Glu Ile Ile Arg Val Val His Pro His Arg Pro Cys Lys Leu Ala
                15                  20                  25

CTG GGC AGT GAC GGG GTT CGG GTG ACC ATG GAG AGT GCG CTC ACC GCC     327
Leu Gly Ser Asp Gly Val Arg Val Thr Met Glu Ser Ala Leu Thr Ala
            30                  35                  40

CGT GAC CGG GTG GGG GTG CAG GAT TTC GTG CTG CTG GAG AAC TTC ACC     375
Arg Asp Arg Val Gly Val Gln Asp Phe Val Leu Leu Glu Asn Phe Thr
        45                  50                  55

AGC GAG GCC GCC TTC ATC GGG AAC CTG CGG CGG CGA TTT CGG GAG AAT 423
Ser Glu Ala Ala Phe Ile Gly Asn Leu Arg Arg Arg Phe Arg Glu Asn
    60                  65                  70

CTC ATC TAC ACC TAC ATT GGC CCC GTC CTG GTC TCT GTC AAT CCC TAC 471
Leu Ile Tyr Thr Tyr Ile Gly Pro Val Leu Val Ser Val Asn Pro Tyr
75                  80                  85                  90

CGG GAC CTG CAG ATC TAC AGC CGG CAG CAT ATG GAG CGT TAC CGT GGC 519
Arg Asp Leu Gln Ile Tyr Ser Arg Gln His Met Glu Arg Tyr Arg Gly
                95                  100                 105

GTC AGC TTC TAT GAA GTG CCC CCT CAC CTG TTT GCC GTG GCG GAC ACT 567
Val Ser Phe Tyr Glu Val Pro Pro His Leu Phe Ala Val Ala Asp Thr
            110                 115                 120

GTG TAC CGA GCA CTG CGC ACG GAG CGT CGG GAC CAG GCT GTG ATG ATC 615
Val Tyr Arg Ala Leu Arg Thr Glu Arg Arg Asp Gln Ala Val Met Ile
        125                 130                 135

TCT GGG GAG AGC GGG GCA GGC AAG ACC GAG GCC ACC AAG AGG CTG CTG 663
Ser Gly Glu Ser Gly Ala Gly Lys Thr Glu Ala Thr Lys Arg Leu Leu
    140                 145                 150

CAG TTC TAT GCA GAG ACC TGC CCA GCC CCC GAG CGC GGA GGT GCC GTG 711
Gln Phe Tyr Ala Glu Thr Cys Pro Ala Pro Glu Arg Gly Gly Ala Val
155                 160                 165                 170

CGG GAC CGG CTG CTA CAG AGC AAC CCG GTG CTG GAG GCC TTT GGA AAT 759
Arg Asp Arg Leu Leu Gln Ser Asn Pro Val Leu Glu Ala Phe Gly Asn
                175                 180                 185

GCC AAG ACC CTC CGG AAC GAT AAC TCC AGC AGG TTC GGG AAG TAC ATG 807
Ala Lys Thr Leu Arg Asn Asp Asn Ser Ser Arg Phe Gly Lys Tyr Met
            190                 195                 200

GAT GTG CAG TTT GAC TTC AAG GGT GCC CCC GTG GGT GGC CAC ATC CTC 855
Asp Val Gln Phe Asp Phe Lys Gly Ala Pro Val Gly Gly His Ile Leu
        205                 210                 215

AGT TAC CCC CTG GAA AAG TCA CGA GTG GTG CAC CAG AAT CAT GGG GAG 903
Ser Tyr Pro Leu Glu Lys Ser Arg Val Val His Gln Asn His Gly Glu
    220                 225                 230

CGG AAC TTC ACA TCT TCT ACC AGC TGC TGG AGG GGG GCG AGG AGG AGA 951
Arg Asn Phe Thr Ser Ser Thr Ser Cys Trp Arg Gly Ala Arg Arg Arg
235                 240                 245                 250

CTC TTC GCA GGC TGG GCT TGG AAC GGA ACC CCC AGA GCT ATC TGT ACC 999
Leu Phe Ala Gly Trp Ala Trp Asn Gly Thr Pro Arg Ala Ile Cys Thr
                255                 260                 265

TGG TGAAGGCCAG TGTGCCAAAG TCTTCTTCAT CAACGACAAG AGTGACTGGA AGGTCG 1058

Trp

TCAGGAAGGC TCTGACAGTC ATTGATTTCA CCGAGGATGA AGTGGAGGAC CTGCTGAGCA 1118
TCGTGGCCAG CGTCCTTCAT TTGGGCAACA TCCACTTTGC TGCCAACGAG GAGAGCAATG 1178
CCCAGGTCAC CACCGAGAAC CAGCTCAAGT ATCTGAGCCC ATTCAGTATG CGGTGCCTGT 1238
TGTGAAATAC GACCGCAAGG GCTACAAGCC TCGCTCCCGG CAGCTGCTGC TCACGCCCAA 1298
CGCCGTCGTC ATCGTGGAGG ACGCCAAAGT CAAGCAGAGG ATTGATTACG CCAACCTGAC 1358
CGGAATCTCT GTCAGCAGCC TGAGCGACAG TCTTTTTGTG CTTCATGTAC AGCGTGCGGA 1418
CAATAAGCAA AAGGGAGATG TGGTGCTGCA GAGTGACCAC GTGATTGAGA CGCTGACCAA 1478
GACAGCCCTC AGTGCCAACC GCGTGAACAG CATCAACATC AACCAGGGCA GCATCACGTT 1538
TGCAGGGGGC CCCGGCAGGG ATGGCACCAT TGACTTCACA CCCGGCTCGG AGCTGCTCAT 1598
CACCAAGGCC AAGAACGGGC ACCTGGCTGT GGTCGCCCCA CGGCTGAATT CTCGGTGATA 1658
AAGGCGCCCA CTGGACCCTC CCAACGCCCA ATGCTTTGCT TTTCTCCTCC TCCCCTTCCC 1718
AGTTACCAAA GACTCGAACT TCCAGACAGG GACCCAGGGA CACCCCGAAG CCCACCTGCA 1778
ATCTCCCACC TCCTGCCCAT CCCTCTCTTG AGGGAGCAGC AGGGGCCAGG AGCTACCCCA 1838
GGAGTGGGCC AGGCCGGGCC ACAGCAATAG GAAAGCCAGG GCCAGAGCGA GCCATGCCAG 1898
CCCTACTGCC GATGCCAAAT ATTTGAGAGA AGGGAACTTT TGCTGAGGTT TTCTCTGAGG 1958
TTTTTTTTGA TGCTTTATAG GAAACTATTT TTTAAAAAAA GCCATTTCCC ACCCAAGGAC 2018
ACAGTGGATG TGTTTTCCCT GACTCCAGCA GGGCAAGGAA ATGTAGCCGA GAGGTTGTGT 2078
GGGCTGGGCT CTGGTGCCCT CTTCCCTGGC CAGGACACCT CTCCTCCTGA TTCCCTTGGC 2138
ACCTTGTCTT TCTGTCTGTT TACCTGTCTC CCTGCCTGCC CATCTGCATC TTTTGCAGCC 2198
CACTCTGACT TCCATCTGGG GGCTGAGACC ACCCTTGCCT GCCCCCTTCT TTCTGCCTTA 2258
AGAATGTCCT TTTAGGCTGG GCATGGTGGC TCACGCCTGT AACCCCAGCA CTTTGGGAGG 2318
CGGAGACGGG CAGATAACCT GAGGTCAGGA TTTCGAGACC AACCTGACCT ACATGGAGAA 2378
ACTCCGCCTC TGGTAAGGAT ACAAAATTAG CCGGGCATGG TGGTGCACGC CTCTAATCCC 2438
AGCTGCTCGG GAGGCTGAGG CAGGAGAATC ACTTGAACCC GGGAAGTGGA GGTTGCAGTG 2498
AGCCAAGAGT ACACCACTGC ACTCCAGCCT GGGCAACAGA GCGAGACTCC GTCTTAAAAA 2558
AA 2560

<210> 171
<211> 267
<212> PRT
<213> Homo sapiens
<400> 171
Met Ala Leu Gln Val Glu Leu Val Pro Thr Gly Glu Ile Ile Arg Val
1               5                   10                  15
Val His Pro His Arg Pro Cys Lys Leu Ala Leu Gly Ser Asp Gly Val
            20                  25                  30
Arg Val Thr Met Glu Ser Ala Leu Thr Ala Arg Asp Arg Val Gly Val
        35                  40                  45
Gln Asp Phe Val Leu Leu Glu Asn Phe Thr Ser Glu Ala Ala Phe Ile
    50                  55                  60
Gly Asn Leu Arg Arg Arg Phe Arg Glu Asn Leu Ile Tyr Thr Tyr Ile
65                  70                  75                  80
Gly Pro Val Leu Val Ser Val Asn Pro Tyr Arg Asp Leu Gln Ile Tyr
                85                  90                  95
Ser Arg Gln His Met Glu Arg Tyr Arg Gly Val Ser Phe Tyr Glu Val
            100                 105                 110
Pro Pro His Leu Phe Ala Val Ala Asp Thr Val Tyr Arg Ala Leu Arg
        115                 120                 125
Thr Glu Arg Arg Asp Gln Ala Val Met Ile Ser Gly Glu Ser Gly Ala
    130                 135                 140
Gly Lys Thr Glu Ala Thr Lys Arg Leu Leu Gln Phe Tyr Ala Glu Thr

145                 150                 155                 160
Cys Pro Ala Pro Glu Arg Gly Gly Ala Val Arg Asp Arg Leu Leu Gln
                165                 170                 175
Ser Asn Pro Val Leu Glu Ala Phe Gly Asn Ala Lys Thr Leu Arg Asn
            180                 185                 190
Asp Asn Ser Ser Arg Phe Gly Lys Tyr Met Asp Val Gln Phe Asp Phe
        195                 200                 205
Lys Gly Ala Pro Val Gly Gly His Ile Leu Ser Tyr Pro Leu Glu Lys
    210                 215                 220
Ser Arg Val Val His Gln Asn His Gly Glu Arg Asn Phe Thr Ser Ser
225                 230                 235                 240
Thr Ser Cys Trp Arg Gly Ala Arg Arg Arg Leu Phe Ala Gly Trp Ala
                245                 250                 255
Trp Asn Gly Thr Pro Arg Ala Ile Cys Thr Trp
            260                 265

<210> 172
<211> 2650
<212> DNA
<213> Homo sapiens
<220>
<221> CDS
<222>(116)..(1216)
<400> 172
CTTTTGCTGC TGCGCCCGGG CAGGGGCTGC CGCGGCCCCA GGTCCCGCTT CGAGACGCGG      60
CGCGGTCCAG GCGGGAGGCG ACTCCCTAGG AAGGGACCCG GGGCGGGAGG AGGAA ATG      118
                                                             
                                                              1

AGG CCG CGC GGA AGG AAG GCG GCG AGC CCC GGG GCC CCG AGG CCT TGG      166

            5                   10                  15

CCG CGT CAC AGC ACC CAC ATG GCC TCT GGA GTG GGC GCG GCC TTC GAG      214

        20                  25                  30

GAA CTG CCT CAC GAC GGC ACG TGT GAC GAG TGC GAG CCC GAC GAG GCT      262

    35                  40                  45

CCG GGG GCC GAG GAA GTG TGC CGA GAA TGC GGC TTC TGC TAC TGC CGC      310

50                  55                  60                  65

CGC CAT GCC GAG GCG CAC AGG CAG AAG TTC CTC AGT CAC CAT CTG GCC      358

                70                  75                  80

GAA TAC GTC CAC GGC TCC CAG GCC TGG ACC CCG CCA GCT GAC GGA GAG      406

            85                  90                  95

GGG GCG GGG AAG GAA GAA GCG GAG GTC AAG GTG GAG CAG GAG AGG GAG 454

        100                 105                 110

ATA GAA AGC GAG GCA GGG GAA GAG AGT GAG TCG GAG GAA GAG AGC GAG 502

    115                 120                 125

TCA GAG GAA GAG AGC GAG ACA GAG GAA GAG AGT GAG GAT GAG AGC GAT 550

130                 135                 140                 145

GAG GAG AGT GAA GAA GAC AGC GAG GAA GAA ATG GAG GAT GAG CAA GAA 598

                150                 155                 160

AGC GAG GCC GAA GAA GAC AAC CAA GAA GAA GGG GAA TCC GAG GCG GAG 646

            165                 170                 175

GGA GAA ACT GAG GCA GAA AGT GAA TTT GAC CCA GAA ATA GAA ATG GAA 694

        180                 185                 190

GCA GAG AGA GTG GCC AAG AGG AAG TGT CCG GAC CAT GGG CTT GAT TTG 742

    195                 200                 205

AGT ACC TAT TGC CAG GAA GAT AGG CAG CTC ATC TGT GTC CTG TGT CCA 790

210                 215                 220                 225

GTC ATT GGG GCT CAC CAG GGC CAC CAA CTC TCC ACC CTA GAC GAA GCC 838

                230                 235                 240

TTT GAA GAA TTA AGA AGC AAA GAC TCA GGT GGA CTG AAG GCC GCT ATG 886

            245                 250                 255

ATC GAA TTG GTG GAA AGG TTG AAG TTC AAG AGC TCA GAC CCT AAA GTA 934

        260                 265                 270

ACT CGG GAC CAA ATG AAG ATG TTT ATA CAG CAG GAA TTT AAG AAA GTT 982

    275                 280                 285

CAG AAA GTG ATT GCT GAT GAG GAG CAG AAG GCC CTT CAT CTA GTG GAC 1030

290                 295                 300                 305

ATC CAA GAG GCA ATG GCC ACA GCT CAT GTG ACT GAG ATA CTG GCA GAC 1078


                310                 315                 320

ATC CAA TCC CAC ATG GAT AGG TTG ATG ACT CAG ATG GCC CAA GCC AAG 1126

            325                 330                 335

GAA CAA CTT GAT ACC TCT AAT GAA TCA GCT GAG CCA AAG GCA GAG GGC 1174

        340                 345                 350

GAT GAG GAA GGA CCC AGT GGT GCC AGT GAA GAA GAG GAC ACA TGAAGGCTT 1225

    355                 360                 365

GCTACCCCCA GTGGAAAATC ATCCCCTCCC CTTGTGTGTA TGTGACAGCG TGTATGTAAC 1285
GGCTTCTGAT TTCTGTGAAA GCTGCTCAGC AACAAACGTA CTTCCACCAG ATGTGTCCCC 1345
AGATCCACAG CAGGCACATA TCTCTCCAAG GGATGACCAG TTTTATGCTT ACTGTGTGCT 1405
TCTCATCCCC TGGTTGTGGT AGGTCAAGGA AAAGAGCCCC TTTGATCCAC CAGGAGCAAT 1465
TAAGAAAGGT CCTTCAGGTA ATCCCTCAAT GGCTGCTTTG AACTTACTCA GGAAAGCCAG 1525
CCCCCATAAT ATTGTATTAC CAAACAGTAT CGCTTTGTTA GGAAGGATCT GGAATAATCT 1585
TGAAGGGAAG TCAGAGTTTT CTCCCTGCCT ATTAACAAAA ACCCAATTTT GTTCATATTG 1645
AAGCATGAAA TAAATGAGAG CAAGGTAGGG CCAAATTAAC TCTTGTGGAC AGTCCCTAAA 1705
AGTCCAGTTC TACATTTGTG AAAATTGTGG TGCCATGAAT TAAGATGGAT GACTGGAAAA 1765
AGGTGTTGGA GAAAGAGTTA AAGATGAGGA AGAGATATTT TTAGTATATG AAGTTATCCA 1825
GGACTTGATA TTCATAATTC AGTGCTGTGG AAATGAAAAA AATGATTGAA GAGGTGGAAC 1885
GGAAATGACC TTAGGGGGAA AAAAAAGGAC CAAAGAAGTC TGATTAAAAG TTGAAATCAG 1945
TATTTCTGAA TTCAAATTGC TTGAATTTCC AAAATAGTCA GTAAAGGATC TAATAGAACC 2005
AGAATTATTT GGGTGAATTC TGCAGGTTTT ATGGGCTTGT CACAACGTGA AGGGCTGGAA 2065
TGTATATTAC CAAATGGGAA TTTCCATTGT AGGTTTTTGC TAGTCCCACC CCCATTTTAG 2125
CCTAATTTGG CTTAAACGCA GTATGGGGAG AATTGTTCCC ATTCCATGTG TTCTGAATTC 2185
AGCTCATCTC CCAGCATATA GATATATCCT CCTTTAACTC CGACCAGAAC CCTTCTTCCT 2245
GTGGCACTCC CCACCCATAG ACCTTCAGAT CATCTCCCAC ACCCTGGATC TCACTCTCCT 2305
CTTAGTAACA GAGACACTCC TGAGGTTGGA CTTCCTTGCT TTTCTCTACT TCCAAATCAC 2365
AATTTCTTAC AACCAAGCTT TGTGCTCCCG AGTAAGCAGG GATGTACTAG GGGAATGTAA 2425
AACTGCAAAC TTAAAAACCT GCATCTTCTT GAAGCATCAG TTTTACTTAC CAAATGGTTT 2485
AGAGTCATAA GATGACCTAT TTTTATATAA AAGTTATATT ATAGAATAAA ATGTTCATAC 2545
GCATAGACTG TTAAG 2560

<210> 173
<211> 367
<212> PRT
<213> Homo sapiens
173

1               5                   10                  15

            20                  25                  30

        35                  40                  45

    50                  55                  60


65                  70                  75                  80

                85                  90                  95

            100                 105                 110

        115                 120                 125

    130                 135                 140

145                 150                 155                 160

                165                 170                 175

            180                 185                 190

        195                 200                 205

    210                 215                 220

225                 230                 235                 240

                245                 250                 255

            260                 265                 270

        275                 280                 285

    290                 295                 300

305                 310                 315                 320

                325                 330                 335

            340                 345                 350

        355                 360                 365

<210> 174
<211> 41
<212> DNA
<213> Artificial Sequence
<220>
<223> Description of the artificial sequence:a synthetic DNA

<400> 174
ttaagcttgc caccatgagc aaccccagcg ccccaccacc a                41

<210> 175
<211> 29
<212> DNA
<213> Artificial Sequence
<220>

<223> Description of the artificial sequence:a synthetic DNA

<400> 175
gtatcgattt aattgcgatc ccccatcag                                29

176
<211> 24
<212> DNA
<213> Artificial Sequence
<220>
<223> Description of the artificial sequence:a synthetic DNA

<400> 176
cacctactgtatgacaccacattc                                       24

<210> 177
<211> 24
<212> DNA
<213> Artificial Sequence
<220>
<223> Description of the artificial sequence:a synthetic DNA

<400> 177
gagatgctgttccatgctggcctg                                       24

<210> 178
<211> 24
<212> DNA
<213> Artificial Sequence
<220>
<223> Description of the artificial sequence:a synthetic DNA

<400> 178
ggaaagctctccgtggctaacaag                                       24

<210> 179
<211> 24
<212> DNA
<213> Artificial Sequence
<220>
<223> Description of the artificial sequence:a synthetic DNA

<400> 179
catagtccttgacaagggtcacag                                       24

<210> 180
<211> 22
<212> DNA
<213> Artificial Sequence
<220>
<223> Description of the artificial sequence:a synthetic DNA

<400> 180

cccatcaccatcttccaggagc 22

<210> 181
<211> 26
<212> DNA
<213> Artificial Sequence
<220>
<223> Description of the artificial sequence:a synthetic DNA

<400> 181
ttcaccaccttcttgatgtcatcata 26

## Claims

1. A DNA having a nucleotide sequence selected from the nucleotide sequences represented by SEQ ID NO:143, 145, 147, 149, 151, 153, 155, I57, 168, 170 and 172.

2. A shear stress-responsive DNA capable of hybridizing with a DNA having the nucleotide sequences represented by SEQ ID NO:143, 145, 149, 151, 153, 155, 157, 168, 170 or 172 under stringent conditions.

3. A shear stress-responsive DNA capable of hybridizing with a DNA having the nucleotide sequence represented by SEQ ID NO:147 under stringent conditions, and having not less than 90% homology with the DNA.

4. A DNA having the same sequence as 5 to 60 consecutive bases in a nucleotide sequence selected from the nucleotide sequences represented by SEQ ID NO:143, 145, 149, 153, 155, 157, 168, 170 and 172, or a DNA having a sequence complementary to the DNA.

5. A method for detecting an mRNA for a shear stress-responsive gene using a DNA according to any one of claims 1 to 4.

6. A diagnostic agent for vascular diseases caused by arteriosclerosis which contains a DNA according to any one of claims 1 to 4.

7. A method for detecting a gene causative of arteriosclerosis using a DNA according to any one of claims 1 to 4.

8. A method for screening an agent for regulating the transcription or translation of a shear stress-responsive gene using a DNA according to any one of claims 1 to 4.

9. A method for screening a therapeutic agent for vascular diseases caused by arteriosclerosis using a DNA according to any one of claims 1 to 4.

10. A therapeutic agent for vascular diseases caused by arteriosclerosis which contains a DNA according to any one of claims 1 to 4.

11. A recombinant virus vector containing a DNA according to any one of claims I to 4.

12. A recombinant virus vector containing an RNA comprising a sequence homologous with the sense strand of a DNA according to any one of claims 1 to 4.

13. A DNA having a nucleotide sequence selected from the nucleotide sequences represented by SEQ ID NO: 111, 113, 115, 116, 117, 119, 121, 123, 125, 127, 129, 130, 131, 132, 133, 134, 135, 137, 139 and 141.

14. A shear stress-responsive DNA capable of hybridizing with the DNA according to claim 13 under stringent conditions.

15. A DNA having the same sequence as 5 to 60 consecutive bases in a nucleotide sequence selected from the nucleotide sequences represented by SEQ ID NO:111, 113, 115, 116, 117, 119, 121, 123, 125, 127, 129, 130, 131, 132, 133, 134, 135, 137, 139 and 141, or a DNA having a sequence complementary to the DNA.

16. A diagnostic agent for vascular diseases caused by arteriosclerosis which contains a DNA according to any one of claims 13 to 15.

17. A method for detecting a gene causative of arteriosclerosis using a DNA according to any one of claims 13 to 15.

18. A method for screening an agent for regulating the transcription or translation of a shear stress-responsive gene using a DNA according to any one of claims 13 to 15.

19. A method for screening a therapeutic agent for vascular diseases caused by arteriosclerosis using a DNA according to any one of claims 13 to 15.

20. A method for detecting an mRNA for a shear stress-responsive gene using a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103,105, 107 and 109.

21. A method for identifying the apoptosis sensitivity of cells by detecting the endogenous transcription level of a DNA having the nucleotide sequence represented by SEQ ID NO:7 using a DNA having the nucleotide sequence represented by SEQ ID NO:7 or a DNA having the same sequence as 5 to 60 consecutive bases in the nucleotide sequence represented by SEQ ID NO:7:

22. A method for suppressing or promoting the apoptosis of cells by regulating the endogenous transcription or translation of a DNA having the nucleotide sequence represented by SEQ ID NO:7 using a DNA having the nucleotide sequence represented by SEQ ID NO:7 or a DNA having the same sequence as 5 to 60 consecutive bases in the nucleotide sequence represented by SEQ ID NO:7, or an antisense DNA having a nucleotide sequence complementary to the nucleotide sequence of each of these DNAs.

23. A diagnostic agent for vascular diseases caused by arteriosclerosis which contains a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.

24. An agent for identifying the apoptosis sensitivity of cells which contains a DNA having the nucleotide sequence represented by SEQ ID NO:7, or a DNA having the same sequence as 5 to 60 consecutive bases in the nucleotide sequence represented by SEQ ID NO:7.

25. A method for screening an agent for regulating the transcription or translation of a shear stress-responsive gene using a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.

26. A method for screening a therapeutic agent for vascular diseases caused by arteriosclerosis using a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.

27. A method for screening an agent for suppressing or promoting the apoptosis of cells by regulating the endogenous transcription or translation of a DNA having the nucleotide sequence represented by SEQ ID NO:7 using a DNA having the nucleotide sequence represented by SEQ ID NO:7 or a DNA having the same sequence as 5 to 60 consecutive bases in the nucleotide sequence represented by SEQ ID NO:7.

28. A therapeutic agent for vascular diseases caused by arteriosclerosis which contains a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.

29. An agent for suppressing or promoting the apoptosis of cells which contains a DNA having the nucleotide sequence represented by SEQ ID NO:7 or a DNA having the same sequence as 5 to 60 consecutive bases in the nucleotide sequence represented by SEQ ID NO:7, or an antisense DNA having a nucleotide sequence complementary to the nucleotide sequence of each of these DNAs.

30. A recombinant virus vector containing a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.

31. A recombinant virus vector containing an RNA comprising a sequence homologous with the sense strand of a DNA having a nucleotide sequence selected from the nucleotide sequences represented by SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107 and 109.

32. A therapeutic agent for vascular diseases caused by arteriosclerosis which contains a recombinant virus vector according to claim 30 or 31.

33. A method for suppressing the apoptosis of cells using a recombinant virus vector containing a DNA having the nucleotide sequence represented by SEQ ID NO:7, or a recombinant virus vector containing an RNA comprising a sequence homologous with the sense strand of a DNA having the nucleotide sequence represented by SEQ ID NO:7.

34. A method for screening an agent for suppressing or promoting the apoptosis of cells using a recombinant virus vector containing a DNA having the nucleotide sequence represented by SEQ ID NO:7, or a recombinant virus vector containing an RNA comprising a sequence homologous with the sense strand of a DNA having the nucleotide sequence represented by SEQ ID NO:7.

35. A protein having an amino acid sequence selected from the amino acid sequences represented by SEQ ID NO:144, 146, 148, 150, 152, 154, 156, 158, 169, 171 and 173.

36. A protein comprising an amino acid sequence in which one or more amino acids are deleted, replaced or added as compared with the amino acid sequence possessed by the protein according to claim 35, and having an activity participating in the formation of an arteriosclerotic lesion.

37. A DNA encoding a protein according to claim 35 or 36.

38. A recombinant DNA obtained by inserting a DNA according to any one of claims 1-4 and 37 into a vector.

39. A transformant obtained by introducing the recombinant DNA according to claim 38 into a host cell.

40. A process for the preparation of a protein which comprises culturing the transformant according to claim 39 in a culture medium, causing a protein according to claim 35 or 36 to be produced and accumulated in the culture medium, and harvesting the protein from the resulting culture.

41. A method for screening a therapeutic agent for vascular diseases caused by arteriosclerosis which comprises culturing the transformant according to claim 39 in a culture medium and using the resulting culture for the screening.

42. A method for screening a therapeutic agent for vascular diseases caused by arteriosclerosis using a protein according to claim 35 or 36.

43. A recombinant virus vector capable of producing a protein according to claim 35 or 36.

44. A therapeutic agent for vascular diseases caused by arteriosclerosis which contains the recombinant virus vector of claim 43.

45. An antibody capable of recognizing a protein according to claim 35 or 36.

46. A method for detecting a protein according to claim 35 or 36 immunologically. using the antibody according to claim 45.

47. A method for screening a therapeutic agent for vascular diseases caused by arteriosclerosis using the antibody according to claim 45.

48. A method for screening an agent for regulating the transcription or translation of a shear stress-responsive gene using the antibody according to claim 45.

49. A diagnostic agent for vascular diseases caused by arteriosclerosis which contains the antibody according to claim 45.

50. A therapeutic agent for vascular diseases caused by arteriosclerosis which contains the antibody according to claim 45.

51. A drug delivery method which comprises combining the antibody of claim 45 with a radioactive isotope, a protein or a low-molecular-weight agent, and delivering the resulting conjugated antibody to an arteriosclerotic lesion.

52. An antibody capable of recognizing a protein having an amino acid sequence represented by SEQ ID NO: 112, 114, 118, 120, 122, 124, 126, 128, 136, 138, 140 and 142.

53. A method for screening a therapeutic agent for vascular diseases caused by arteriosclerosis using the antibody according to claim 52.

54. A method for screening an agent for suppressing the transcription or translation of a shear stress-responsive gene using the antibody according to claim 52.

55. A diagnostic agent for vascular diseases caused by arteriosclerosis which contains the antibody according to claim 52.

56. A therapeutic agent for vascular diseases caused by arteriosclerosis which contains the antibody according to claim 52.

57. A drug delivery method which comprises combining the antibody of claim 52 with a radioactive isotope, a protein or a low-molecular-weight agent, and delivering the resulting conjugated antibody to an arteriosclerotic lesion.

58. A method for screening an agent capable of binding specifically to a protein having the amino acid sequence represented by SEQ ID NO:8 and effective for suppressing or promoting the apoptosis of cells, using a protein having the amino acid sequence represented by SEQ ID NO:8.

59. A method for screening an agent for suppressing or promoting the apoptosis of cells which comprises inserting a DNA having the nucleotide sequence represented by SEQ ID NO:7 or a DNA encoding a protein having the amino acid sequence represented by SEQ ID NO:8, into a vector; introducing the resulting recombinant DNA into a host cell; culturing the resulting transformant in a culture medium; and using the resulting culture for the screening.

60. A recombinant virus vector capable of producing a protein having an amino acid sequence selected from the amino acid sequences represented by SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108 and 110.

61. A therapeutic agent for vascular diseases caused by arteriosclerosis which contains the recombinant virus vector according to claim 60.

62. A method for suppressing the apoptosis of cells using a recombinant virus vector capable of producing a protein having the amino acid sequence represented by SEQ ID NO:8.

63. An agent for suppressing the apoptosis of cells which contains a recombinant virus vector capable of producing a protein having the amino acid sequence represented by SEQ ID NO:8.

64. A method for screening a therapeutic agent for vascular diseases caused by arteriosclerosis using an antibody capable of recognizing a protein having the amino acid sequence represented by SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108 or 110.

65. A method for screening an agent for suppressing or promoting the transcription or translation of a shear stress-responsive gene using an antibody capable of recognizing a protein having the amino acid sequence represented by SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108 or 110.

66. A method for regulating the apoptosis of cells using an antibody capable of recognizing a protein having the amino acid sequence represented by SEQ ID NO:8.

67. A method for screening an agent for regulating the apoptosis of cells using an antibody capable of recognizing a protein having the amino acid sequence represented by SEQ ID NO:8.

68. A method for identifying the apoptosis sensitivity of cells by detecting the expression level of a protein having the amino acid sequence represented by SEQ ID NO:8 using an antibody capable of recognizing a protein having the amino acid sequence represented by SEQ ID NO:8.

69. A method according to any one of claims 21, 22, 27, 33, 34, 58, 59, 62, 66, 67 and 68 wherein the cells are vascular endothelial cells.

70. A diagnostic agent for vascular diseases caused by arteriosclerosis which contains an antibody capable of recognizing a protein having the amino acid sequence represented by SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108 or 110.

71. An agent for identifying the apoptosis sensitivity of cells which contains an antibody capable of recognizing a protein having the amino acid sequence represented by SEQ ID NO:8.

72. A therapeutic agent for vascular diseases caused by arteriosclerosis which contains an antibody capable of recognizing a protein having the amino acid sequence represented by SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108 or 110.

73. An agent for regulating the apoptosis of cells which comprises an antibody capable of recognizing a protein having the amino acid sequence represented by SEQ ID NO:8.

74. An agent for suppressing or promoting the apoptosis of cells which is obtained by a method according to any one of claims 27, 34, 58, 59 and 67.

75. An agent according to any one of claims 24, 29, 63, 71, 73 and 74 wherein the cells are vascular endothelial cells.

76. A drug delivery method which comprises combining an antibody capable of recognizing a protein having the amino acid sequence represented by SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108 or 110, with a radioactive isotope, a protein or a low-molecular-weight agent, and delivering the resulting conjugated antibody to an arteriosclerotic lesion.
